# EUROPEAN PATENT APPLICATION

(11) **EP 1 481 685 A1**
(43) Date of publication of application: **01.12.2004**
(21) Application number: 03012388.9
(22) Date of filing: 30.05.2003
(51) Int. Cl.: A61K 38/17, C07K 14/47, G01N 33/50, A61P 29/00

(54) **Use of PAM**

(71) Applicant: Aventis Pharma Deutschland GmbH, 65929 Frankfurt am Main (DE)
(72) Inventor: Michaelis, Martin, Dr., 60325 Frankfurt (DE); Geisslinger, Gerd, Prof. Dr., 65812 Bad Soden (DE); Scholich, Klaus, Dr., 63303 Dreieich (DE); Tegeder, Irmgard, Dr., 60598 Frankfurt (DE)

(57) **Abstract**

Use of PAM or functional fragments or derivatives thereof for the preparation of pharmaceutical compounds.

## Description

The invention concerns the use of PAM (Protein Associated with Myc). Other aspects of the invention concern a method for screening pharmaceuticals and methods for the treatment of pain.

Pain is a complex subjective sensation reflecting real or potential tissue damage and the affective response to it. Acute pain is a physiological signal indicating a potential or actual injury. Chronic pain can either be somatogenetic (organic) or psychogenic. Chronic pain is frequently accompanied or followed by vegetative signs, which often result in depression.

Somatogenetic pain may be of nociceptive origin, inflammatory or neuropathic. Nociceptive pain is judged to be commensurate with ongoing activation of somatic or visceral pain-sensitive nerve fibers. Neuropathic pain results from dysfunction in the nervous system; it is believed to be sustained by aberrant somatosensory processes in the peripheral nervous system, the CNS, or both. (For an overview of pain mechanisms, see for example Scholz and Woolf, 2002; Julius and Basbaum, 2001, Woolf and Mannion, 1999; Wood, J.D., 2000; Woolf and Salter, 2000.)

Chronic pain results in individual suffering and social economic costs of tremendous extent. Existing pharmacological pain therapies are widely unsatisfying both in terms of efficacy and of safety.

Up to now, two classes of analgesics are mainly employed for the treatment of pain: Non-opioid analgesics, mostly acetaminophen and NSAIDS (non-steroidal antiinflammatory drugs) and opioid (narcotic) agonists (wherein "opioid" is a generic term for natural or synthetic substances that bind to specific opioid receptors in the CNS, producing an agonist action). Unfortunately both analgesic classes, opioids and non-opioids, have several unwanted side effects. The most serious side effects of opioids are the possibility of inhibition of the respiratory system and after long-term treatment the possibility of addiction (Schaible H.G., Vanegas H., 2000). NSAIDs, a major class of non-opioids, on the other hand, can induce a variety of gastrointestinal complications such as ulcers and bleeding, but also kidney damage (Schaible H.G., Vanegas H., 2000). It has been estimated that in the U.S.A. about 16.000 patients die every year because of severe gastro-intestinal complications caused by conventional NSAIDs.

In light of the severe drawbacks connected with state of the art pain treatments, there is a great need for novel classes of pain modulating drugs. Especially in light of the vast gap between the fast advancing understanding of the neurobiology of pain and the unmet clinical need to provide effective treatments without the drawbacks of state of the art treatments, efforts need to be directed to the discovery of new targets for novel classes of analgesics. Thus, it is the object of the present invention to provide a new means for the development and provision of a new class of pain modulating drugs.

This object is solved by the use of PAM or functional fragments or derivatives thereof for the preparation of pharmaceutical compounds that modulate pain.

The invention is based on findings of the inventors that demonstrate for the first time the implication of PAM in nociceptive processing and its ability to decrease pain. The term functional refers to the ability of PAM to lower intracellular cAMP levels or to interact with AC; more preferably it refers to its ability to inhibit AC activity and even more preferably to its ability to decrease pain.

A fragment of PAM can be any polypeptide or polynucleotide that is shorter than the corresponding wild type, e.g. shorter than homo sapiens (hs) PAM according to one of the sequences according the accession numbers ACC 39928 (SEQ ID No. 1), NP_055872, NM_015057, AF075587 (SEQ ID No.2) or shorter than a polynucleotide from position 24679861 to position 24962245 of the sequence according to accession number NT_024524.11 (SEQ ID No.3).

A derivative of PAM or of a PAM fragment can be any modification of the amino acid or nucleotide sequence having PAM function or any other kind of modification, such as a chemical or biological modification e.g. leading to the stabilization of the polypeptide or polynucleotide (such as phosphoorothioate modifications), or enabling a specific targeting of the polypeptide or polynucleotide to certain cells or facilitating its entry into or uptake by cells (such as cell-permeant phosphopeptides orthe coupling to cell-permeant peptide vectors, e.g. based on the antennapedia/penetratin, TAT, and signal-peptide based sequences; or coupling to parts of ligands, for specific transporters or importers).

PAM or fragments or derivatives thereof may either be used in the form of a polypeptide or a polynucleotide. Useful are suitable modifications or additives for ensuring or facilitating its targeting to the site of need and its entering the cell. On the other hand, a local application, such as an intraspinal application using suitable catheters, etc. or the like is possible for ensuring its targeting to the spinal cord. Other useful additives include salts, buffers or the like for its stabilization, etc.

The PAM polynucleotide or fragment or derivative thereof can e.g. be put into a suitable vector that ensures its intracellular expression and, preferably also its targeting into the cell. A cell type specific expression can be ensured using appropriate promoters/enhancers of neuron-specific genes which are known in the art The use of PAM oligonucleotides is also possible.

The present invention is based on studies of the inventors, that demonstrate for the first time the surprising implication of PAM in sensitisation mechanisms within the spinal cord and dorsal root ganglia (DRGs).

PAM (Protein Associated with Myc) is a giant protein of 510 kDa. The protein, genomic and coding polynucleotide sequences of PAM are known in the state of the art and are, e. g. publicly available from the NCBI (National Centre for Biotechnology Information; National Library of Medicine, Building 38A, Bethesda, MD 20894, USA; www.ncbi.nhm.nih.gov) data base under the accession numbers AAC39928 (coding sequence; SEQ ID No.1), AF075587 (protein sequence; SEQ ID No.2). Human PAM is located on Chromosome 13q22; its genomic sequence is publicly available under NT_024524.11 (Start: position 24679861; Stop: position 24962245; SEQ ID No.3). Alternatively, the protein and coding sequence are publicly available under KIAA0916 protein Accession NP_055872 (protein sequence) and NM_015057 (coding sequence).

For rat PAM, the following EST-clone coding sequences are publicly available:
AW921303 (corresponds to bp 960-1394 of hs cDNA; SEQ ID No.4)
AW918711 (corresponds to bp 8188-8632 of hs cDNA; SEQ ID No.5)
BQ201485 (corresponds to bp 8966-9633 of hs cDNA; SEQ ID No.6)
BE112881 (corresponds to bp 10311-10830 of hs cDNA; SEQ ID No.7)
AW441131 (corresponds to bp 13569-14152 of hs cDNA; SEQ ID No.8)
BF409872 (corresponds to bp 13569-14807 of hs cDNA; SEQ ID No.9).

PAM was originally identified by its ability to interact specifically with the transcriptional activating domain in the N-Terminus of Myc (Guo Q., et al., 1998). PAM has recently been described as a powerful inhibitor of AC activity (Scholich K., Pierre S., Patel T.B.: Protein associated with myc (PAM) is a potent inhibitor of adenylyl cyclase. J. Biol. Chem. 2001, Dec 14;276(50):47583-9.), but there has been no evidence of its function in nociceptive processing and sensitisation, so far.

Rather, PAM is believed to be playing a role in presynaptic growth regulation: PAM mRNA has been known to be highly expressed in specific anatomical regions, including hippocampus, dentate gyrus and cerebellum. Both PAM and Myc expression in the brain of adult rats and mice is confined to the maturing Purkinje cells in the cerebellum and granule and pyramidal cells in the hippocampus (Ruppert C., et al., 1986; Yang H. et al., 2002). None of these cell types, however is known to be involved in pain processing and sensitisation.

PAM homologues in *Drosophila* (highwire) and C. *elegans* (rpm-1) have been shown to play a crucial role in presynaptic terminal organization (Zhen et al., 2000), the regulation of synaptic growth (Wan et al.,2000), synaptogenesis, and neurite growth and targeting (Schaefer et al., 2000). These findings led to the assumption that highwire, rpm-1 and their mammalian homolog PAM might act as negative regulators of synaptic growth (Chang et al., 2000; Jin Y. 2002). Accordingly, a dramatic increase in PAM expression in the cerebellum, hippocampus and dentate gyrus was found during the major synaptogenic period in these structures (Yang et al., 2002).

During brain development in rodents, PAM expression is turned on shortly after birth, up-regulated during the first two weeks, and, thereafter, PAM expression remains elevated during adulthood (Yang et al., 2002). So far, nothing has been known about the expression and regulation of PAM in the spinal cord and DRGs and its function in sensitisation mechanisms and regulation of pain.

Previously, it has been demonstrated that human PAM is a potent regulator of cyclic AMP (cAMP)-signaling and inhibits the enzyme activity of several adenylyl cyclase (AC; E.C.4.6.1.1 ) isoforms at nanomolar concentrations (Scholich et al. 2001).

The ubiquitous cyclic AMP (cAMP) second messenger system is one of different signal transduction mechanisms translating extracellular stimuli to intracellular signals and responses. Upon extracellular stimulation, G-protein coupled receptors (GPCRs) modulate plasma-membrane bound enzymes or ion channels via trimeric GTP-binding regulatory proteins (G-proteins). One of the enzymes modulated in its activity by GPCRs is the adenylyl cyclase (AC), a cAMP generating enzyme. Thus, the incoming extracellular stimuli influence the intracellular concentration of the intracellular mediator cyclic AMP. A rise in cAMP levels affects the cell by stimulating protein kinase A (PKA), which phosphorylates specific intracellular target proteins and thereby alters their activity.

Each type of cell has characteristic sets of GPCRs, enzymes modulated by those GPCRs, specific subsets of adenylyl cyclase (AC) and target proteins, that, acting together with more unspecific or generally occurring players (such as the ubiquitous cAMP), enable each cell to make its own distinctive response to incoming extracellular signals. It is for example known that the cyclic AMP (cAMP)-second messenger plays a major role in the regulation of synaptic plasticity (Bailey et al., 1996; Xia et al., 1997; Brandon et al., 1997); on the other hand it is involved in metabolic processes and cellular proliferation. Thus, the role of the ubiquitous cAMP messenger system and its different components varies according to different specializations of different tissue and cell types.

A further aspect of the invention regards the use of PAM or functional fragments or derivatives thereof for the modulation of pain. This modulation is preferably a lessening or prevention or total inhibition.

Moreover, the use of PAM or functional fragments of derivatives thereof for identifying compounds that modulate pain is encompassed by present invention. The modulating compounds are preferably PAM activating compounds. More preferably they have the ability to prevent, lessen or abolish pain.

The compounds can for example be identified by their ability to
a) activate or enhance PAM function (i.e. its ability to lower intracellular cAMP levels, to interact with other factors like AC, especially with AC, to inhibit AC or its ability to lower the pain perception) or
b) activate PAM on RNA level (i.e. by activation of PAM transcription or transcript stabilisation) or
c) activate PAM on protein level (i.e. by activation of PAM translation or its posttranslational processing; by modulation of PAM posttranslational modification or by activation of its stabilisation or inhibition of its degradation).

In this aspect, PAM (or its fragment or derivatives thereof) can either be used as a polypeptide or oligo- or polynucleotide.

Another aspect of present invention regards a method of lessening pain comprising administering a sufficient amount of PAM or a functional fragment or derivative thereof to an individual.

Administration should suitably be performed in a way that allows for targeting of PAM to the site of action (DRG or spinal cord), e.g. by systemical administration of PAM derivatives or formulations targeting themselves to site of need or local (e.g. intraspinal) application of PAM or its fragments or derivatives thereof.

Another aspect of the invention concerns a method of screening for pharmaceuticals useful for modulating and/or preventing pain, comprising the steps
a. Providing two samples
b. contacting one sample containing PAM or a functional fragment or derivative thereof with a compound,
c. determining the PAM activity in the presence of compound,
d. determining the PAM activity in the absence of compound, and
e. comparing the PAM activity according to c) with that according to d).

PAM can be derived from any sequence available that allows for its specific purpose according to the different aspects of the present invention. Preferably, PAM is human PAM.

For the different aspects of present invention it is also preferred, if PAM is an isolated polypeptide or a oligo- or polynucleotide,. isolated in the context of the different aspects of present invention means at least partially purified from a natural source or recombinant PAM (which can, of course, also be purified or partially purified).

An assay is any type of analytical method to monitor a biological process. For the use in drug screening, the assay needs to be reproducible and is preferably also scalable and robust. The assay is preferably suitable for high throughput screening of chemical substances for their ability of modulating (preferably diminishing) and / or preventing pain. The type of assay depends e.g. on the type of PAM used (either polypeptide or polynucleotide) and the "read out", i.e. the way in which PAM activity is determined (see below).

Different types of such assays are commonly known in the state of the art and commercially available from commercial suppliers. Suitable assays encompass radioisotopic or fluorescent assays, for example fluorescence polarization assays (such as those offered commercially by Panvera, Perkin-Elmer life sciences (e.g. LANCE) or Packard Bioscience (e.g. HTRF or ALPHAscreen™)) for measuring the interaction of a labeled member with a non-labeled member (e.g. PAM or fragments thereof could be labeled and their interaction with AC could be monitored).

More examples include cell based assays, wherein a cell line stably (inducibly or not; chromosomal or episomal) or transiently expresses a recombinant protein of interest. These assays comprise e.g. reporter gene assays, wherein the regulation of a certain promotor or a signal transduction pathway of a member of a signal transduction cascade is measured according to the activity of a reporter enzyme, the expression of which is under the control of said certain promotor. For this type of assay, a recombinant cell line has to be constructed containing the reporter gene under the control of a defined promotor that is to be investigated itself or that is regulated by the signaling cascade under investigation. Suitable reporter enzymes are commonly known within the state of the art and comprise firefly luciferase, renilla luciferase (e.g. commercially available by Packard reagents), ß-Galactosidase. Suitable cell lines depend on the aim of the assay but comprise mostly cell lines that are easy to transfect and easy to cultivate, such as, e.g. HeLA, COS, CHO, NIH-3T3, etc.

Assays for measuring the intracellular ion level comprise e.g. FLIPR (fluorometric imaging plate reader, commercially available from Molecular Devices) assays, wherein an argon laser light source combined with a cooled CCD camera allows for parallel measurements in 384 well plates transient ion signals (such as Ca²⁺, etc) within cells (e.g. neuronal cells or other cells (e.g. cells recombinantly or naturally expressing certain ion channels). FLIPR assays allow e.g. for monitoring of intracellular calcium using certain fluorochromes, such as Fluo-3, Fluo-4, or monitoring intracellular pH using BCECF or BCPCF pr specific FLIPR assay kits, or detecting membrane potential changes using e.g. DiBAC or specific FLIPR assay kits, or monitoring of membrane polarization. For the monitoring of other intracellular ions, e.g. zinc or sodium, other dyes known in the state of the art can be used. Other types of assays and other types of read outs are commonly known to persons with skills in the art.

For the measurement of cAMP levels, e.g. AlphaScreen, fluorescence polarization or HTRF technology is suitable.

For the determination of ion channel activity (which control e.g. intracellular ion concentrations and can thus be employed for measurement of intracellular ion concentrations) e.g. membrane potential sensitive assays and dyes can be used such as DiBAC or Molecular Devices' membrane potential assay kit on FLIPR technology; mitochondrial membrane polarization measuring JC-1 dye with FLIPR technology; ion sensitive dyes such as Fluo-3, Fluo-4 or Molecular Devices calcium assay kit for intracellular calcium concentration measurement; sodium sensitive dye e.g. from Molecular Probes for measurement of intracellular sodium; assays based on patch-clamping or atomic adsorption spectroscopy-based Rubidium ion efflux measurement for determining of intracellular potassium concentrations, and so on. Further automatical devices and analytical methods for detecting certain changes and states within cells are known to the person of skill in the art and comprise, e.g. the Acumen detector (flureescence-based laser scanning reader that allows for 3dimensional reconstitution of distribution of suitably labeled objects) by ACUMEN bioscience.

The PAM polypeptide is preferably a polypeptide that comprises or consists of the sequence according to SEQ ID No 2 or is encoded by a polynucleotide comprising or consisting of the sequence according to SEQ ID No 1 or 3.

The PAM polynucleotide is preferably a polynucleotide comprising or consisting of the sequence according to SEQ ID No 1 or 3 or a polynucleotide comprising or consisting of a sequence that is able to hybridize with the above polynucleotides under stringent conditions.

Stringency describes reaction conditions that influence the specificity of hybridisation or annealing of two single stranded nucleic acid molecules. Stringency, and thus specificity of a reaction depends, inter alia, of the temperature and buffer-conditions used for a reaction: Stringency, and thus specificity, can e.g. be increased by increasing the reaction temperature and/or lowering the ion strength of the reaction-buffer. Conditions of low stringence (and thus low reaction and hybridisation specificity) exist for example, if a hybridisation is performed at room temperature in 2xSSC-solution. Conditions of high stringency comprise e.g. a hybridisation reaction at 68°C in 0,1xSSC and 0,1% SDS solution.

Hybridisation under conditions of stringency within the different aspects of present invention is preferably understood to be:
1) Hybridising a labelled probe with a nucleic acid sample to be analysed at 65°C, or in the case of oligonucleotide probes, at 5°C below the annealing or melting temperature of the duplex consisting of oligonucleotide and sample (annealing and melting temperature are in the following understood to be synonyms) over night in 50mM Tris pH 7,5, 1 M Nacl, 1% SDS, 10% Dextran Sulfate, 0,5 mg/ml denatured salmon or hering sperm DNA.
2) Washing for 10 minutes in 2xSSC at room temperature.
3) Washing for 30 minutes in 1xSSC/0,1%SDS at 65°C (or in the case of oligonucleotides: 5°C below the annealing temperature).
4) Washing for 30 minutes in 0,1xSSC/0,1%SDS at 65°C (or in the case of oligonucleotides: 5°C below the annealing temperature).

Oligonucleotides for the use as hybridisation probes are polynucleotide and preferably DNA-fragments having a length of 15 to 30, preferably 20 nucleotides. The annealing temperature is determined according to the formula Tm=2x (number of A+T) + 4x (number of G+C)°C.

For preparing a 2xSSC or a 0,1xSSC (or any other kind of SSC dilution), e.g. a 20x SSC solution is diluted accordingly. 20xSSC consists of 3M NaCl/0,3 M Na-Citrate x2H₂O.

Before performing a hybridisation reaction, the polynucleotides are, if wanted after performing electrophoretic separation (then: Southern Blot (DNA) or Northern Blot (RNA)) or without electrophoretic separation (then: slot or dot Blot), transferred to a suitable membrane, e.g. a nylon or nitrocellulose membrane. Hybridisation is performed using a suitably labelled probe. Suitable labelling techniques are e.g. radioactive labelling or labelling using fluorescence dyes. The probe is a single stranded polyribo- or polydesoxyribonucleotide being single stranded naturally or being usually double stranded and having been made single stranded by denaturation. This probe binds to the DNA or RNA sample (which is also in single stranded state) by means of base pairing.

The PAM fragments are preferably fragments comprised within the above sequences ID No. 1, 2 or 3 and the derivatives are preferably derived from the above sequences ID No. 1, 2 or 3 or from fragments thereof.

The functional fragments or derivatives thereof are preferably capable of inhibiting adenylyl cyclase (AC) activity, more preferably that of AC Type I, V or VI.

According to a preferred embodiment of the different aspects of present invention, the functional fragments or derivatives thereof comprise or consist of amino acids 400 to 1400, preferably 446 to 1062, 499 to 1065 or 1028 to 1231, and more preferably 1000 to 1300 and even more preferably 1000 to 1100 and even more preferably 1028 to 1065 of the human PAM sequence, preferably of the human PAM sequence according to SEQ ID No. 2, or if they are encoded by the respective polynucleotide fragments, especially if comprised within the sequences according to SEQ ID No.2 or 3.

If the functional fragments or derivatives thereof are polynucleotides, it is preferred, if they comprise or consist of polynucleotides encoding the above polypeptide fragments. More specifically, it is preferred if they comprise or consist of positions 1482 to 3332 (encoding amino acids 446 to 1062) or 1641 to 3341 (encoding amino acids 498 to 1066) or 3228 to 3839 (encoding amino acids 1038 to 1231) of the human PAM cds. It is even more preferred, if the human PAM cds from which the fragments are derived has the sequence according to SEQ ID No.2.

According to one preferred embodiment of present method for identifying pain modulating compounds, a cell expressing, PAM, preferably recombinant PAM is used.

The cell can be any type of cell, e.g. a eucaryotic or prokaryotic single cell organism (such as bacteria, e.g. e. coli, or yeast, e.g. s. pombe or s. cerevisiae) or cell lines derived from multicellular organisms (such as HeLa, COS, NIH-3T3, CHO, etc), wherein mammalian cell lines are preferred.

According to another preferred embodiment, a modified cell, having a lower PAM activity as compared to its unmodified state, is used. This way, it can be tested, if the chemical compounds to be tested for their ability of modulating (preferably diminishing) and / or preventing pain, are able to enhance or restore the lowered or totally abolished PAM activity.

The modification can be any type of modification (stable or transient, preferably stable), that leads to a decrease of PAM activity (i.e. its ability to lower intracellular CAMP levels, to interact with other factors like AC, especially with AC, to inhibit AC or its ability to lower the pain perception), PAM transcript steady state level (i.e. by activation of PAM transcription or transcript stabilisation) or PAM protein steady state level (i.e. by activation of PAM translation or its posttranslational processing; by modulation of PAM posttranslational modification or by activation of its stabilisation or by inhibition of its degradation). This can for example be achieved by using dominant negative mutants of PAM, antisense oligonucleotides, RNAi constructs of PAM, by generating functional or genomic PAM knock outs (which can e.g. be inducible) or other suitable techniques known within the state of the art. For an overview of the above techniques, see for example: Current protocols in Molecular biology (2000) J.G. Seidman, Chapter 23, Supplemtent 52, John Wiley and Sons, Inc.; Gene Targeting: a practical approach (1995), Editor: A.L. Joyner, IRL Press; Genetic Manipulation of Receptor Expression and Function, 2000; Antisense Therapeutics, 1996; Scherr et al., 2003.
According to a preferred embodiment, a PAM knock-out cell is used. Suitable cell lines for the generation of knock-outs are well known in the state of the art and comprise e.g Current protocols in Molecular Biology (2000) J.G. Seidman, Chapter 23, Supplement 52, John Wiley and Sons, Inc; or Gene Targeting a practical approach. (1995) Ed. A.L. Joyner, IRL Press.

The PAM activity can either be determined directly, e.g. by its ability (or the ability of its fragments and derivatives) to interact with AC or inactivate AC, or it can be determined indirectly, e.g. by its ability (or the ability of its fragments and derivatives) to lower intracellular cAMP levels, to modulate ion concentrations within the neurons or its ability to modulate, especially decrease pain perception. Suitable techniques for measuring the above parameters are well known in the state of the art (see also above): The cAMP levels can e.g. be measured by HTRF or ALPHAscreen™, the ion concentrations can e.g. be estimated by patch clamping or suitable dyes, the pain perception can e.g. be measured by means of the formalin test or tests of mechanical or thermal hyperalgesia, or the hot plate test etc.

Another aspect of present invention concerns a method of identifying a compound that modulates pain comprising
a) Selecting a compound that modulates the activity of PAM as a test compound, and
b) Administering said test compound to a subject to determine whether the pain is modulated.

The subject can be any subject with the ability of perceiving pain, preferably it is a mammal, either a non-human mammal or a human (i.e. within a patient study).

In the following, the invention is illustrated in more detail by means of examples and figures. However, the examples are not meant to limit the scope of the invention.

### Examples: Investigation of PAM expression pattern and function

### 1. Preparation of animal sections:

Wild type Sprague Dawley rats were purchased from Charles River Wiga GmbH (Sulzfeld, Germany). The animals had free access to food and water prior to the experiments. They were maintained in climate- and light-controlled rooms (24 ± 0.5°C). Each animal was used at one occasion only. In all experiments the ethics guidelines for investigations in conscious animals were obeyed, and the procedures were approved by the local Ethics Committee. After killing, adult rats were fixed by perfusion with 4% paraformaldehyde in 0.1 M phosphate buffered saline (PBS, pH 7.2) for one hour. Tissues were cryostat-sectioned in the horizontal plane at a thickness of 14-16 µm. Sections were mounted on Superfrost Plus Slides (Fisher Scientific Co., Pittsburgh, PA) and stored at -80°C until use.

### 2. Preparation of riboprobes:

The riboprobes were generated as described previously (Yang et al., 2002). Antisense and sense riboprobes of rat PAM were obtained with T7 and T3 polymerases, after linearizing the plasmid with Hind III (antisense) and BamHI (sense), respectively (see Yang et al., 2002). In vitro transcription was performed in the presence of [35S] UTP-αS (ICN, Irvine CA), linearized PAM cDNA, NTP at 37°C for 1 hour according to the manufacturer's recommendation (Promega, Madison, WI). The RNA transcripts were purified using RNA Probe Purification Kit (Pequlab, Erlangen, Germany).

### 3. In situ hybridization:

In situ hybridization was performed as described earlier (Yang et al. 2002): Sections were fixed in 4% paraformadehyde in 0.1 M phosphate-buffered saline (pH 7.2), pretreated with 0.25% acetic anhydride and 0.1 M triethanolamine, rinsed with 0.2x SSC and dehydrated with serially increasing concentrations of alcohol. Sections were prehybridized with prehybridization solution (50% deionized formamide, 0.6 M sodium chloride, 10 mM Tris-HCl (pH 7.6), 50 mM EDTA, 0.025% sodium pyrophosphate, 0.02% Ficoll, 0.02% BSA, 0.02% polyvinyl pyrrolidone, 10 mM DTT, and heat-denatured, heterologous nucleic acids (0.005% yeast tRNA, type X, 0.05% yeast total RNA, typeI, 0.05% salmon testes DNA, type III)) for 2 hours at room temperature; hybridized with riboprobes in hybridization solution (2.5×106 cpm/section), 50% deionized formamide and 50% hybridization buffer containing 0.6 M sodium chloride, 10 mM Tris-HCl (pH 7.6), 50 mM EDTA, 0.025% sodium pyrophosphate, 0.02% Ficoll, 0.02 % BSA, 0.02% polyvinyl pyrrolidone, heat-denatured, heterologous nucleic acids (0.005% yeast tRNA, type X, 0.005% yeast total RNA, type I, 0.05% salmon testes DNA, type III), 100 mM DTT, 0.0005% polyadenylic acid, 10% dextran sulfate) at 50°C overnight. Sections were rinsed with 2x, 1x, 0.5x SSC at RT (room temperature). After digestion in 20 µg/ml RNase A (Sigma, St. Louis, MO), sections were washed in 1 x RNase buffer, 2x, 1 x, 0.5x SSC at room temperature, and in 0.1x SSC overnight at 45°C. Sections were dehydrated with serially increasing concentrations of alcohol, exposed to Kodak Biomax MR film (Kodak, Rochester, NY) for 3-7 days at -80°C.

### 4. Antibody generation and Immunofluorescence staining:

Antisera were raised commercially in rabbits using peptides consisting of amino acid residues 135-153 and 4601-4614 of human PAM corresponding to the SEQ ID No.1, respectively (BioTrend, Cologne, Germany). The antiserum was commercially produced by BioTrend, Cologne, Germany, according to standard procedures. To monitor the distribution of PAM in spinal cord and DRG slices, the slices were permeablized in 0.1 % Triton X-100 for 5 minutes. The slices were blocked for 1 hour in 3% BSA in PBS and then incubated for 1 hour with anti-PAM antiserum (1:50 dilution). This was followed by incubation with FITC-labeled goat anti-rabbit antibody in PBS containing 3% BSA. The slices were then washed with PBS and mounted using fluoromount™.

### 5. RT-PCR:

Total RNA from rat spinal cords and DRGs was isolated by guanidinium isothiocyanate/phenol/chloroform extraction (Chomczynski and Sacchi 1987). 2 µg of total RNA were annealed with 0.6 µM of each of oligo (dT) primer and reverse-transcribed using reverse transcriptase (Promega, Madison, Wl) for 30 minutes at 37 °C. The cDNA was then immediately used for amplification. Oligonucleotide primers used for the amplification of rat GADPH were 5'-GAAGGGTGGGGCCAAAAG-3' (sense; SEQ ID No.10) and 5'-GGATGCAGGGATGATGTTCT-3' (antisense; SEQ ID No.11; Trajkovic et al. 2000). Primers for the amplification of AC isoforms were chosen as published by Bek et al. (Bek et al. 2001). Primers for rat PAM were 5'-GGTGGTGAAGCTCGCTGTGATGCT-3' (sense; SEQ ID No.12) and 5'-CGTGTGAGCATTTCTGCACAC TCC-3' (antisense; SEQ ID No.13). The PCR product corresponds to the human PAM cDNA nucleotides 13692-14064. The corresponding rat sequence was derived from the EST clone AW441131 (SEQ ID No.8). For semiquantitative PCR, SAWDAY DNA Polymerase (Peqlab, Erlangen, Germany) was used. After an initial denaturation step at 95 °C for 5 minutes, 30 cycles were performed with 1 minute at 95°C, 30 seconds at 55°C, and 10 seconds at 72°C, followed by a final 10-minute extension step at 72°C. Quantitative PCR was performed using the TaqMan™ system and reagents (Applied Biosystems, Weiterstadt, Germany) according to the instructions of the manufacturer.

### 6. Purification of full length PAM:

PAM purification was performed with some modifications as published previously (Scholich et al. 2001 ). Shortly, HeLa cells were grown in DMEM medium with 10% fetal bovine serum and 1% penicillin/streptomycin. Confluent cells of forty 150 mm dishes were harvested with 1x PBS, 1 mM EDTA and pelleted for 5 minutes at 400 x g. The cells were resuspended in TED buffer (50 mM Tris-HCl, pH 8.0, 1 mM EDTA, 1 mM DTT) containing 125 mM NaCl, 20 µg/ml of aprotinin, 20 µg/ml leupeptin, 1 mM benzamidine, 5 µg/ml soybean trypsin inhibitor and lysed by 2x 5 seconds of sonication. The homogenate was centrifuged at 27000 x g for 30 min at 4 °C, and the supernatant was loaded on a Q-Sepharose XK16 column (Amersham, Pharmacia, Piscataway, NJ) and eluted with a gradient of 150-350 mM NaCl in TED according to instructions of the manufacturer. The fractions were analyzed by Western blotting according to standard procedures; positive fractions were pooled and the NaCl concentration adjusted to 1 M. The protein was then loaded on a Phenyl-Sepharose XK16 column (Amersham, Pharmacia, Piscataway, NJ) and washed with 300 mM NaCl in TED according to instructions of the manufacturer. The flow through and wash fractions contained PAM. They were pooled and the buffer exchanged for the aforementioned TED buffer containing 100 mM NaCl using Centricon 50 (Amicon, Beverly, MA) according to the manufacturer's instructions. The protein was then loaded on a MonoS 5/5 FPLC column (Amersham, Pharmacia, Piscataway, NJ) and washed with the loading buffer (100 mM NaCl in TED) according to instructions of the manufacturer. The flow through was collected and applied to a Mono Q 5/5 FPLC column (Amersham, Pharmacia, Piscataway, NJ). The protein was eluted with a gradient from 150-400 mM NaCl in TED. Positive fractions were pooled and the buffer exchanged for 50 mM Tris-HCl, pH 8.0, 1 mM DTT using Centricon 50 (Amicon, Beverly, MA) and stored at -80°C. The stored PAM was used within 3 weeks.

### 7. Expression and purification of recombinant Gsα:

The hexahistidyl tagged constitutively active Q213L mutant of Gsα (Gsα^{*}) was expressed and purified as described in Graziano et al., 1991. To ensure maximal activation of the Gsα^{*}, the G protein was incubated with 1 µM GTP_{γ}S in the presence of MgCl2 (25 mM) for 30 minutes prior to use in AC activity assays.

### 8. Adenylyl Cyclase Activity Assays (AC activity assays):

Spinal cords were lysed in 25 mM Hepes, pH 7.4, 1 mM EGTA and cell membranes were prepared as described by Kassis and Fishman. Aliquots were stored at -80°C until use. AC activity assays were performed in a volume of 100 µl for 15 min at room temperature in the presence of 100 µM MgCl₂ as previously described (Patel et al., 2002). Gsα^{*} (80 nM) or forskolin (100 µM) were used to stimulate AC enzyme activity in membranes (10 µg protein).

### 9. Spinal delivery of PAM antisense and sense oligonucleotides:

Rats were anesthetized with ketamine (60 mg/kg i.p.) and midazolam (0.5-1 mg/kg i.p.). The skin was incised above the vertebral column from vertebrae Th13 up to L3. Muscle tissue around L2-3 was cleared away. The processus spinosus of L3 was removed and a laminectomy was done at L2. Polyethylene catheters (ID 0.28 mm, OD 0.61 mm) were then inserted into the peridural space so that the tip of the catheter reached Th9-10. The catheter was fixed with cyanacrylate glue, externalized in the neck region, and the skin was sutured.

### 10. Infusion of PAM oligonucleotides:

The sequences of the oligodeoxynucleotides (ODNs) were chosen from the rat PAM sequence as follows. Sense: 5'-GACTGGTTTAGCAATGGC-3'(SEQ ID No.14), antisense: 5'-GCCATTGCTAAACCAGTC-3' (SEQ ID No.15), and antisense ODN harboring three mutations (3M-as; mutations are underlined): 5'- GCAATTGCTAAATCAGTA -3'(SEQ ID No.16). Three days after surgery, rats were placed into a "freely moving system" (CMA, Stockholm, Sweden) and antisense (n=5) or sense (n=5) oligonucleotides (2.5 mg/ml in artificial cerebrospinal fluid) were infused through the catheter at a flow rate of 0.05-0.1 µl/min for 100 hours using a microinfusion pump (CMA, Stockholm, Sweden).

### 11. Formalin test:

Within 15 min after stopping the infusion, the formalin test was performed. 50 µl of a 5% formaldehyde solution was injected subcutaneously (s.c.) into the dorsal surface of one hind paw. Flinches were counted in one-minute intervals up to 60 min starting right after formalin injection. Flinches of 5 min intervals were summarized as mean flinches per minute. To compare the nociceptive behavior between groups, the sum of flinches during the two phases of the one-hour observation period were submitted to the Students t-test. α was set at 0.05.

At the end of the formalin test, the rats were killed, the lumbar spinal cord and dorsal root ganglia (DRGs) were excised, snap frozen in liquid nitrogen and stored at -80°C until further analysis. To determine PAM expression, spinal cord slices were analyzed immunhistochemically using the above anti PAM antibodies.

### 12. Zymosan-evoked inflammation:

For induction of an inflammation, 2.5 mg zymosan A (Sigma, St. Louis, MO) suspended in 30µl 0.03 M phosphate buffered saline (PBS, pH 7.5) was injected subcutaneously into the midplantar region of the right hindpaw. Such intraplantar zymosan injection is known to induce a reliable model of thermal and mechanical hyperalgesia rats (Meller and Gebhart 1997). Rats were killed by cardiac puncture under deep isoflurane anesthesia 24-96 hours after the Zymosan injection. The lumbar spinal cord and dorsal root ganglia (DRGs) were excised, snap frozen in liquid nitrogen and stored at -80°C until further analysis.

### 13 Results:

The above experiments of the inventors using RT-PCR, immunhistochemistry and in situ hybridisation, demonstrate for the first time that PAM is expressed in sensory neurons of the spinal cord as well as in dorsal root ganglia (DRGs) of adult rats. PAM mRNA was detected by RT-PCR at similar levels in the spinal cord and dorsal root ganglia throughout development (E14-adult). PAM-expression is up-regulated 24-48 hours after zymosan treatment of rats as shown by western blot and RT-PCR.

The major adenylyl cyclase isoforms, which are expressed in the spinal cord and DRGs, are AC type 5 and 6 and AC type 4 and 6, respectively. No major changes in AC isoform expression were observed after zymosan treatment in spinal cord. Hence, Gαs stimulated AC activity in membrane preparations from spinal cord and DRG was inhibited by PAM. Consequently, it was found that treatment with antisense but not sense oligonucleotides against PAM increased formalin induced paw flinching in adult rats. Accordingly cAMP accumulation in the spinal cord of rats treated with antisense oligonucleotides to PAM was elevated as compared to control rats.

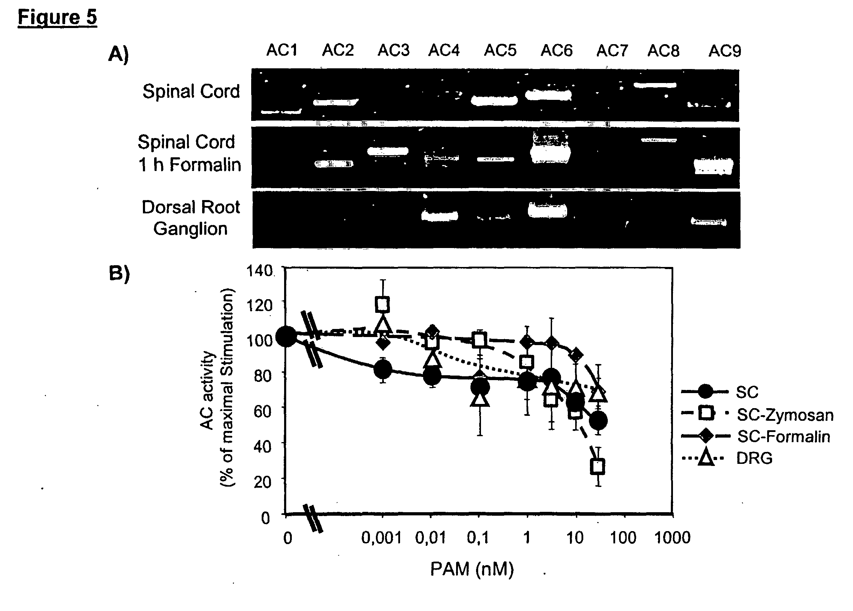

Addition of purified PAM to spinal cord lysates resulted in an inhibition of Gαs-stimulated AC activity of spinal cord lysates from control and zymosan treated animals (Fig. 5b). At 30 nM Gαs-stimulated AC activity was decreased by 50% in spinal cord lysates of control animals and 70% in lysates derived from rats treated with zymosan for 96 hours.

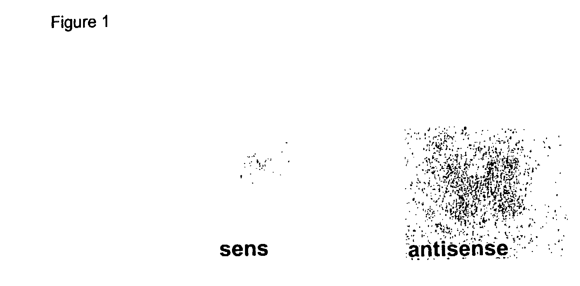

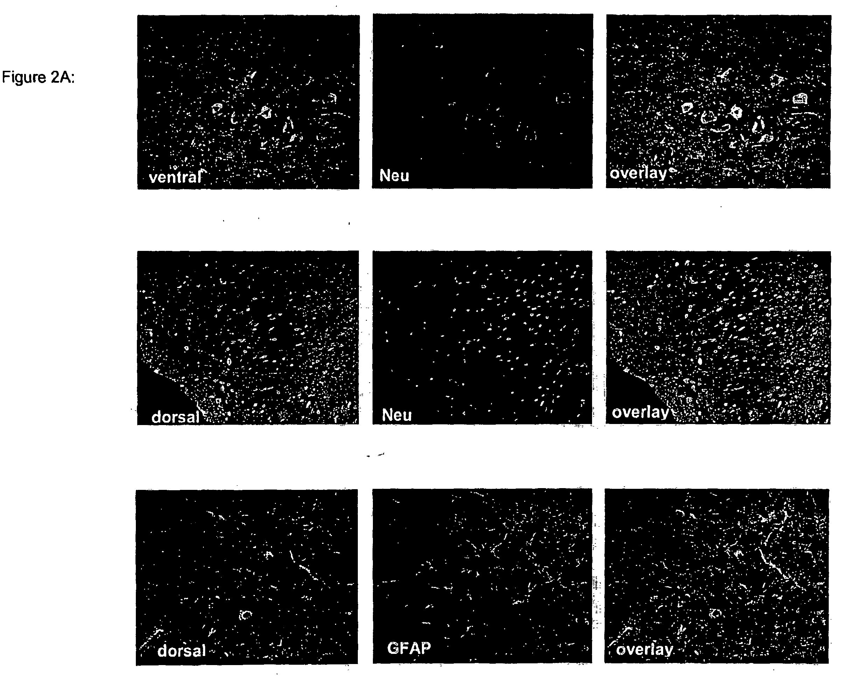

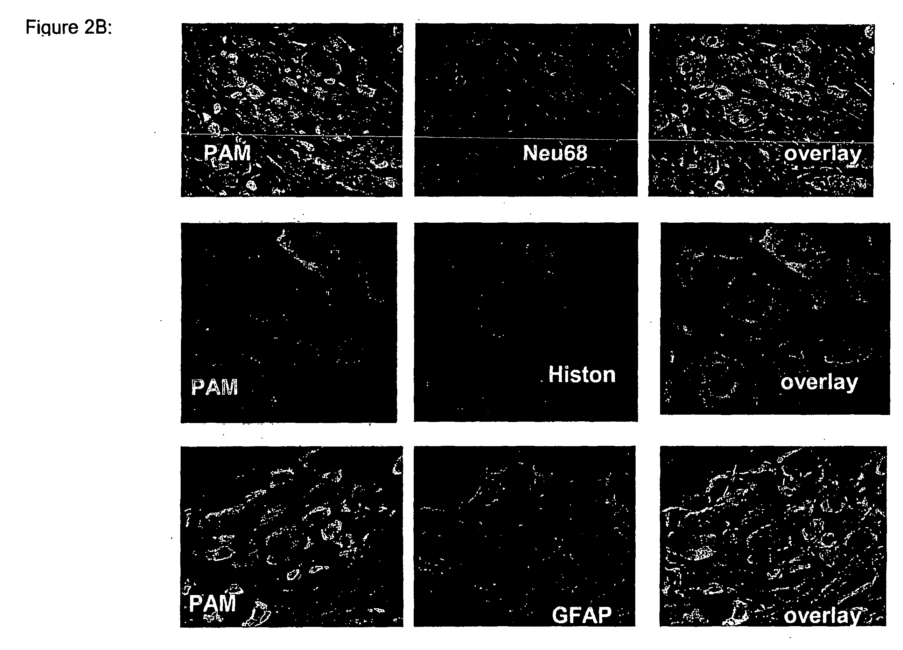

To determine if PAM is expressed in the spinal cord, first *in situ* hybridization was performed. This led to the detection of a clear signal for PAM mRNA throughout the gray matter but not in the white matter of the spinal cord of adult rats (Fig. 1). To define more precisely the cell populations that express PAM in the spinal cord as well as in DRGs, antibodies against PAM using peptides corresponding to the amino acid residues 135-153 and 4601-4614 of human PAM were generated. The immunhistochemical analysis revealed that PAM was co-localized with anti-NeuN but not with anti-GFAP immunoreactivity (Fig. 2a). More specifically, PAM expression was detected predominantly in dorsal horn neurons (Fig. 2a) while non-neuronal cell populations exhibited very little PAM expression. Especially high PAM expression could be detected in DRG neurons (Fig. 2b). Here, PAM immunoreactivity was located in the axons as well as in the cell body of both large- and small-diameter neurons (Fig. 2b). Interestingly, no PAM was detected in the nuclei of the cells as demonstrated by co-staining with anti-Histone antibody (Fig. 2b). As could also be seen in the spinal cord, PAM expression was not detected in GFAP expressing cells.

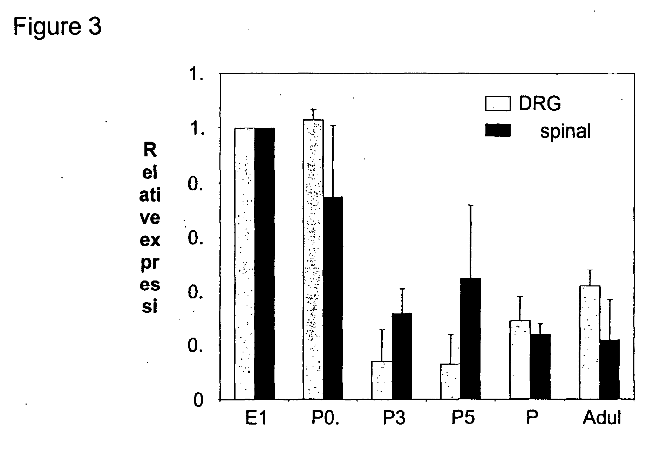

Since PAM expression in the brain is differentially regulated during development in rats and mice (Yang et al. 2002), the inventors investigated if PAM mRNA expression also changes during development in rat spinal cord and DRG. To this end, PAM mRNA expression was determined using quantitative RT-PCR. PAM mRNA was found to be highly expressed in the spinal cord and DRG in late embryonic stages (E16) until shortly after birth (P0.5; Fig. 3). Interestingly, shortly after birth the expression declined to 30-40% of the embryonic expression and then remained constant throughout adulthood (Fig. 3).

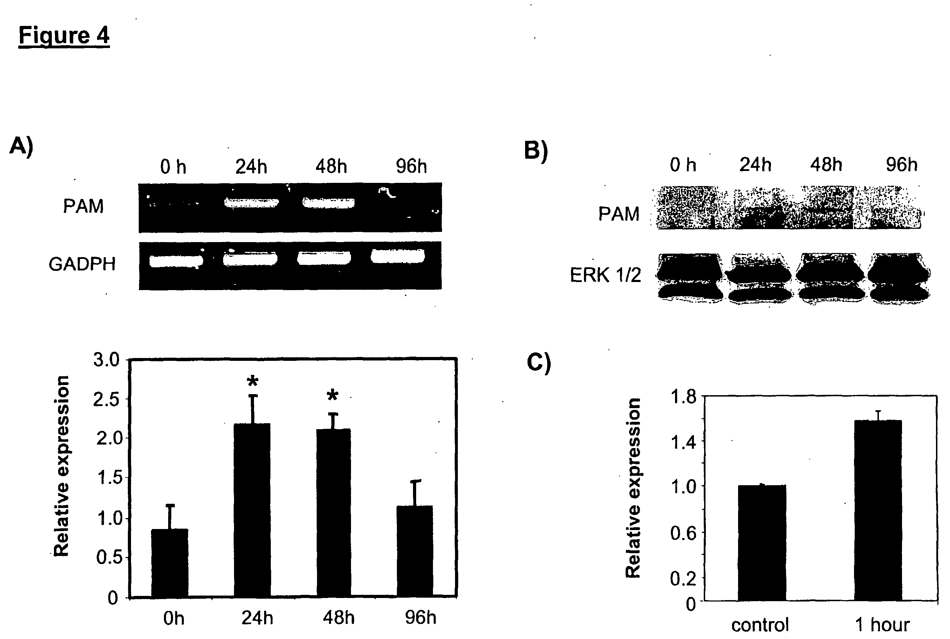

Next, it was examined if PAM expression in the spinal cord is regulated by nociceptive stimuli. Therefore, spinal PAM expression was monitored after zymosan and formalin injection in the hind paws of adult rats. PAM mRNA was up-regulated about two-fold at 24 and 48 hours after zymosan treatment (Fig. 4a). Accordingly, PAM expression was up-regulated at the protein level 24 hours after zymosan injection and stayed elevated for 96 hours (Fig. 4b). 96 hours after zymosan injection, PAM mRNA expression declined. This reduction in PAM mRNA expression was not rejected at the protein level (comp. Fig. 4a and b). Notably, PAM mRNA was also upregulated in the spinal cord of rats 1 hour after formalin injection (Fig. 4c).

Since PAM is known to be a powerful inhibitor of adenylyl cyclases type 1, 5 and 6 (Scholich et al. 2001) it was investigated if PAM is able to inhibit AC activity in spinal cord and DRG lysates. Two major AC isoforms are detected by semi-quantitative RT-PCR in the spinal cord. These AC isoforms are type 5 and 6 (Fig. 5a). Notably, both isoforms are inhibited at nanomolar concentrations of PAM (Scholich et al. 2001). The AC isoform expression pattern in the spinal cord was not significantly altered 24-96 hours after zymosan injection. Interestingly, 1 hour after formalin injection a shift in AC isoform expression was detected (Fig. 5a). The mRNA of AC type 5 is down-regulated and the mRNA of AC type 3 and 9 are up-regulated.

As could be shown by the above experiments, addition of purified PAM to spinal cord lysates resulted in inhibition of Gas-stimulated AC activity in spinal cord preparations. Addition of 30 nM PAM decreased Gαs-stimulated AC activity by 49% in spinal cord lysates of control animals (Fig. 5b). Gαs-stimulated AC activity in spinal cord lysates derived from rats 96 hours after zymosan injection exhibited higher sensitivity to PAM inhibition as compared to untreated animals (70% inhibition at 30 nM; Fig 5b). In contrast, inhibition of AC activity by PAM in spinal cord lysates from animals treated for 1 hour with formalin was inhibited to a lesser extent (25% inhibition at 30 nM; Fig. 5b).

In DRGs the predominantly expressed AC isoforms are AC type 4 and 6 (Fig. 5a). Gαs-stimulated AC activity in DRG lysates was decreased by 32% in presence of 30 nM PAM in contrast to spinal cord lysates (Fig. 5b).

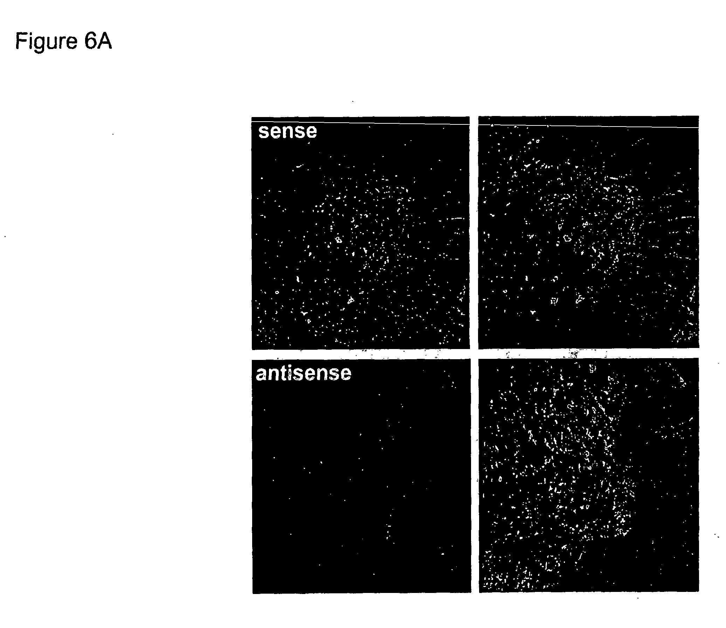

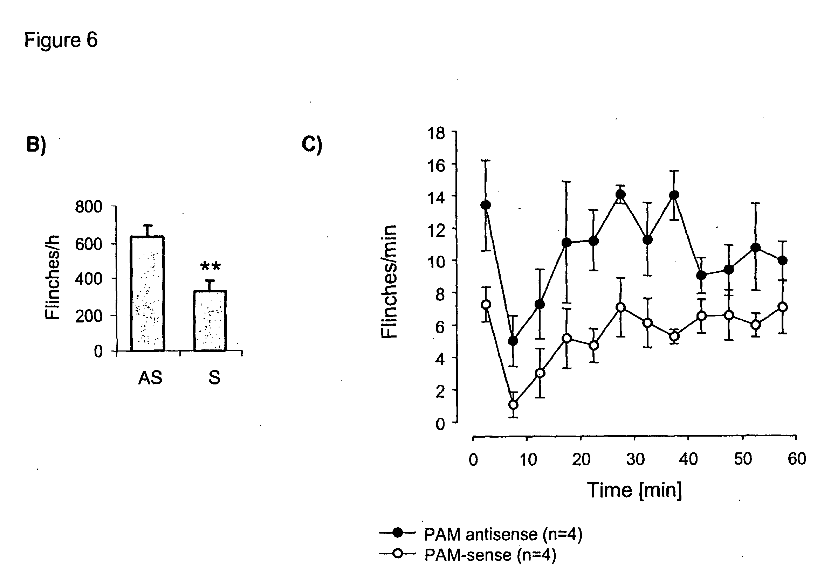

To examine a possible role of PAM in spinal nociceptive transmission, animals were infused with PAM sense and antisense oligonucleotides by lumbar intrathecal catheters before performing a formalin assay. PAM expression was decreased in spinal cord neurons as observed by immunhistochemistry (Fig. 6a). Infusion of PAM antisense oligonucleotides caused a significant increase of the nociceptive response following formalin injection as compared to PAM sense treatment (p=0.007; Fig. 6b and c). The hyperalgesia in PAM antisense-treated rats was accompanied by increased licking and biting behavior. Since PAM is an inhibitor of AC activity (Scholich et al. 2001), basal, Gas- and forskolin-stimulated AC activities were determined in spinal cord lysates of sense and antisense-ODN treated rats. The experiments showed a significant increase (20.7 %) of the basal AC activity in antisense treated rats (Table 1). In contrast, no significant changes were detected in Gαs- and forskolin-stimulated AC activities indicating that the total amount of AC was not altered by the ODN treatment (Table 1).

The above experiments showed that PAM is localized in the cell body and axons of both spinal cord and DRG neurons (Fig. 2a,b). Only very little immunreactivity was detectable in the nucleus suggesting different functions for PAM in neurons and cancer cell lines.

Central sensitization after prolonged nociceptive stimuli is based on neuronal and synaptic changes in the spinal cord (Woolf and Costigan 1999; Woolf and Salter 2000; Ji and Woolf 2001). The finding of the inventors that Pam is expressed in sensory neurons of the spinal cord and DRGs led to the newly formed hypothesis, that PAM could be implicated in synaptic changes during spinal nociceptive processing. The above findings that PAM was up-regulated after nociceptive stimuli (Fig. 4a) supported this hypothesis that PAM may play a role in synaptic changes during spinal nociceptive processing.

Furthermore, the surprising finding of the inventors, that PAM is expressed in sensory nervous of the spinal cord and DRGs led to the question whether PAM is capable of inhibiting AC activity in spinal cord and DRG, as well.

The above experiments showed for the first time that PAM is a potent inhibitor of Gas-stimulated AC activity in spinal cord preparations (Fig. 5b). AC activity was decreased by 50 % after addition of 30 nM PAM. To achieve comparable inhibition using the α-subunit of the inhibitory G-protein, Gαi, 200-800 nM Gαi has to be used (Wittpoth et al. 1999). The inhibitory action of PAM was even stronger in spinal cord preparations of animals treated for 96 hours with zymosan (Fig. 5b) and could be explained by the elevated amounts of endogenous PAM in the spinal cord after zymosan injection (Fig. 4a,b). Inhibition of Gαs-stimulated AC activity in spinal cord preparations from formalin-treated animals (25% inhibition) was less pronounced as compared to control or zymosan-treated animals (50% and 75%, respectively).

Notably, in animals treated with formalin for 1 hour a shift in AC isoform expression was observed (Fig.5a). AC of type 3 and 9 are up-regulated while AC type 5 is down-regulated. To date it is not known if PAM is an inhibitor of AC type 3 and 9. Therefore, these isoforms may not be inhibited by PAM or the tested PAM concentrations were too low to achieve an inhibitory effect. Since PAM is a giant protein of 510 kDa, it is technically not possible to test PAM concentrations greater than 30 nM. Nonetheless, according to the dose response curves shown in Figure 5b, higher PAM concentrations might result in a stronger inhibition of Gas-stimulated AC activity in the tested spinal cord preparations.

Interestingly PAM was a less effective inhibitor of AC enzyme activity in DRG than in spinal cord preparations. The different inhibitory efficiencies of PAM in spinal cord and DRG preparations are most likely due to the observed differences in AC isoform expression. The major AC isoforms that are expressed in the spinal cord are type 5 and 6 that are both strongly inhibited by PAM (Fig. 5a; (Scholich et al. 2001 )). In DRGs AC type 4 and 6 are the dominant AC isoforms (Fig. 5a). Since it is unknown if PAM inhibits AC type 4 either this isoform is not inhibited by PAM or, again, the tested PAM concentrations were too low to achieve the inhibitory effect. However, according to the dose response curve shown in Figure 5b it is seems likely that higher concentrations of PAM would result in a stronger inhibition of Gas-stimulated AC activity in the DRG preparations.

Most surprising, however, were the findings, that PAM activity had an influence on the nociceptive behavior of the test animals: This could be demonstrated for the first time by experiments of the inventors showing a significant increase in basal AC activity (Table 1) and - more important - a significant increase of the nociceptive response following formalin injection as compared to PAM sense treatment (Fig. 6b and c) when endogenous PAM expression in the spinal cord was decreased by infusing animals with PAM antisense oligonucleotides (Fig. 6a).

### 14. determination of the analgesic effect of PAM

The above-listed evidence for the analgesic effect of PAM could for example be supported by the following hypothetic experiment: The analgesic effect of PAM, e.g. in the formalin model of acute pain, could be determined directly by intrathecal application of e.g. a peptide corresponding to amino acid residues 1028 to 1065. This peptide represents the minimal region found to be capable of mediating PAM-adenylyl cyclase interactions as determined by the yeast-two-hybrid system and AC activity assays. The peptide could be applied in a complex with the bioporter lipofection reagent (commercially available at Peqlab, Germany). This approach would allow the peptide to enter the tissue and mimic the actions of physiological PAM towards ACs.

### Literature

Bailey C.H., Bartsch D., Kandel E.R.: Toward a molecular definition of long-term memory storage. Proc. Natl. Acad. Sci. U.S.A. 1996 Nov 26;93(24):13445-52;
Brandon E.P., Idzerda R.L., McKnight G.S.: PKA isoforms, neural pathways, and behaviour: making the connection. Curr. Opin. Neurobiol. 1997 Jun;7(3):397-403;
Bek M. J., Zheng S., Xu J., Yamaguchi I., Asico L. D., Sun X. G. and Jose P. A. (2001) Differential expression of adenylyl cyclases in the rat nephron. *Kidney Int* **60**, 890-899;
Chang Q. and Balice-Gordon R. J. (2000) Highwire, rpm-1, and futsch: balancing synaptic growth and stability. *Neuron* **26**, 287-290;
Chomczynski, P., Sacchi, N.: Single-step method of RNA isolation by acid guanidinium thiocyanate-phenol-chloroform extraction. Anal. Biochem. 162, (1987) 156-159;
Graziano, M.P. FreissmuthM. Gilman A.G.: Purification of recombinant Gs alpha. Meth. Enz. (1991) 195: 192-215;
Graziano M. P., Freissmuth M. and Gilman A. G. (1991) Purification of recombinant Gs alpha. Methods *Enzymol* **195,** 192-202;
Guo Q., Xie J., Dang C.V., Liu E.T., Bishop J.M.: Identification of a large Myc-binding protein that contains RCC1-like repeats. Proc. Natl. Acad. Sci. U.S.A. 1998 Aug 4;95(16):9172-7);
Jin Y. (2002) Synaptogenesis: insights from worm and fly. *Curr Opin Neurobiol* **12,** 71-79.);
Julius and Basbaum " Molecular mechanisms of nociception", Nature, volume 413, 13. September 2001, pp. 203 - 209;
Kassis, S., and Fishman, P. H. (1982) J Biol Chem 257(9), 5312-5318;
Meller S.T., Gebhart G.F. (1997), intraplantar zymosan as a reliable, quantifiable model of thermal and mechanical hyperalgesia in the rat; Eur. J. Pain 1, 43-52;
Nair, B. G., Parikh, B., Milligan, G., and Patel, T. B. (1990) J Biol Chem 265(34), 21317-21322;
Patel T. B., Wittpoth C., Barbier A. J., Yigzaw Y. and Scholich K. (2002) Functional analyses of type V adenylyl cyclase. *Methods Enzymol* **345**, 160-187. Snyder S. H. (1985) Adenosine as a neuromodulator. *Annu Rev Neurosci* **8**, 103-124.
Ruppert C., Goldowitz D., and Wille W. (1986), Proto-oncogene-c-mycis expressed in cerebellar neurons at different developmental stages, Embo J5, 1897-1901;
Schaefer A. M., Hadwiger G. D. and Nonet M. L. (2000) rpm-1, a conserved neuronal gene that regulates targeting and synaptogenesis in C. elegans. *Neuron* 26, 345-356);
Schaible H.G., Vanegas H.: How do we manage chronic pain? Baillieres Best. Pract. Res. Clin. Rheumatol. 2000 Dec;14(4):797-811;
Scherr M., Morgan M.A., Eder M., Gene Silencing Mediated by Small Interfering RNAs in Mammalian Cells, Curr Med Chem. 2003, Feb; 10 (3): 245-256;
Scholich K., Pierre S., Patel T.B.: Protein associated with myc (PAM) is a potent inhibitor of adenylyl cyclase. J. Biol. Chem. 2001, Dec 14;276(50):47583-9;
Scholz and Woolf "Can we conquer pain" , Nature neuroscience supplement, volume 5, November 2002, pp. 1062 - 1067;
Snyder S.H. (1985), Adenosine as a neuromodulator. Annual Reviews of Neuroscience **8**, 103-104;
Trajkovic V, Samardzic T, Stosic-Grujicic S, Ramic Z, Mostarica Stojkovic M. Muramyl dipeptide potentiates cytokine-induced activation of inducible nitric oxide synthase in rat astrocytes. Brain Res. 2000 Nov 10;883(1):157-63;
Wan H. I., DiAntonio A., Fetter R. D., Bergstrom K., Strauss R. and Goodman C. S. (2000) Highwire regulates synaptic growth in Drosophila. *Neuron* 26, 313-329.)
Wittpoth C., Scholich K., Yigzaw Y., Stringfield T.M. and Patel T.B. (1999), Regions on adenylyl cyclase that are necessary for inhibition of activity by beta gamma G(iα) subunits of heterotrimeric G proteins. Proc. Natl. Acad. Sci. USA 96, 7723-7730;
Wood, J.D. "Pathobiology of Visceral Pain: Molecular Mechanisms and Therapeutic implications II. genetic approaches to pain therapy" , American Journal pf Physiological Gastrointestinal Liver Physiology, 2000, volume 278, G507-G512;
Woolf and Mannion "Neuropathic pain: aetiology, symptoms mechanisms, and management", The LANCET, volume 353, June 5, 1999, pp. 1959 -1964;
Woolf J. and Salter M.W. "Neuronal Plasticity: Increasing the Gain in Pain", Science, volume 288, June 9, 2000, pp. 1765-1768;
Xia Z., Storm D.R.: Calmodulin-regulated adenylyl cyclases and neuromodulation. Curr. Opin. Neurobiol. 1997 Jun;7(3):391-6;
Yang H., Scholich K., Poser S., Storm D., Patel T.B., Goldowitz D.: Developmental expression of protein associated with myc (PAM) in the rodent brain. *Brain Res Dev Brain Res* 136, 2002, 35-42;
Zhen M., Huang X., Bamber B. and Jin Y. (2000) Regulation of presynaptic terminal organization by C. elegans RPM-1, a putative guanine nucleotide exchanger with a RING-H2 finger domain. *Neuron* 26, 331-343);

The literature listed above is incorporated herein by reference.

### Standard Literature for Laboratory Methods:

If not indicated otherwise, laboratory methods were or can be performed according to standard methods listed in the below standard literature:
Sambrook et al. (1989) Molecular Cloning: A Laboratory Manual. Second edition. Cold Spring Harbor Laboratory Press, Cold Spring Harbor, NY. 545 pp or Current Protocols in Molecular Biology;
Current Protocols in Molecular Biology; regularly updated, e.g. Volume 2000; John Wiley & Sons, Inc; Editors: Fred M. Ausubel, Roger Brent, Robert Eg. Kingston, David D. Moore, J.G. Seidman, John A. Smith, Kevin Struhl.
Current Protocols in Human Genetics; regularly uptdated, e.g. Volume 2003; John Wiley & Sons, Inc; Editors: Nicholas C. Dracopoli, Honathan L. Haines, Bruce R. Korf, Cynthia C. Morton, Christine E. Seidman, J.G. Seigman, Douglas R. Smith.
Current Protocols in Protein Science; regularly updated, e.g. Volume 2003; John Wiley & Sons, Inc; Editors: John E. Coligan, Ben M. Dunn, Hidde L. Ploegh, David W. Speicher, Paul T. Wingfield.
Molecular Biology of the Cell; third edition; Alberts, B., Bray, D., Lewis, J., Raff, M., Roberts, K., Watson, J.D.; Garland Publishing, Inc. New York & London, 1994;
Gene Targeting: a practical approach (1995), Editor: A.L. Joyner, IRL Press
Genetic Manipulation of Receptor Expression and Function; D. Accili, Wiley-Liss., USA, 2000; ISBN: 0-471-35057-5.
Antisense Therapeutics, S. Agrawal, Humana Press, USA, 1996, ISBN: 0-89603-305-8.

### Abbreviations used:

AC, adenylyl cyclase; Gsα, α subunit of the stimulatory G protein of adenylyl cyclase, Gsα*, constitutively active (Q213L) mutant of Gsα; Giα, α subunit of the inhibitory G protein Gi; Gβγ, βγ subunits of heterotrimeric G proteins; PAM, protein associated with Myc; RCC1, regulator of chromosome condensation; TED, 50 mM Tris-HCl, pH 8.0, 1 mM EDTA, 1 mM DTT; ODN, oligodeoxynucleotide; RT, room temperature;

### Figures:

Figure 1: PAM is highly expressed in spinal cord neurons. In situ hybridization using horizontal sections of spinal cords hybridized with sense or antisense probes against rat PAM as described above.
Figure 2: PAM is expressed in DRG neurons as well as in neuronal cells in rat spinal cord.
   Panel A: Immunhistochemical analysis of rat spinal cord sections. The sections were stained with anti-PAM antibody (green) and anti-NeuN or anti-GFAP (red) to visualize neurons or glia cells, respectively. The overlay of both signals is presented in the right panels. The objects were magnified 20x.
   **Panel B:** Immunhistochemical analysis of rat DRGs sections. The sections were stained with anti-PAM antibody (green) and anti-Neu68, anti-Histon or anti-GFAP (red) to visualize neurons, nuclei or glia cells, respectively. The overlay of both signals is presented in the right panels. The objects were magnified 40x except for the Histon staining, which was magnified 63x.
Figure 3: PAM is differentially expressed in DRGs and spinal cord during different developmental stages. Quantitative RT-PCR (Taqman™) was used to detect PAM in RNA (40 ng) of spinal cord, and DRGs of embryonic rats day 16 (E16), postnatal day 0,5 (P0.5), 3 (P3), 5 (P5), 9 (P9) and adult rats. The mean ± SEM of at least 3 determinations is shown.
Figure 4: PAM is upregulated in the rat spinal cord after zymosan and formalin treatment.
   **Panel A:** RT-PCR analysis with RNA (40 ng) of spinal cords from control animals or animals treated with zymosan after 24h, 48h and 96h. The lower panel shows the mean ± SEM of 7 experiments. Student T test: * p ≤ 0.001.
   **Panel B:** Western blot analysis using a 7% SDS-PAGE gel with rat spinal cord lysates of control animals or animals treated with zymosan after 24h, 48h and 96h (40µg) with anti-PAM antibody and anti-ERK½.
   Panel C: Quantitative RT-PCR analysis with RNA (40 ng) of spinal cords from control animals or animals treated with formalin for 1 hour.
Figure 5: PAM inhibits Gas-stimulated AC activity in spinal cord lysates.
   **Panel A:** RT-PCR was used to determine AC isoform expression in spinal cord and DRG RNA (40 ng).
   **Panel B**: Lysates of spinal cords or DRG (10 µg) were assayed for AC activity in the presence of 80 nM Gαs as described in Material and Methods. The mean ± SEM of at least 3 determinations done in triplicates is shown.
Figure 6: Intrathecal application of antisense ODNs against PAM increases nociceptive behavior.
   **Panel A:** Adult rats were given intrathecal sense and antisense ODN as described. After formalin treatment, the spinal cord was removed and subjected to immunhistological analysis using anti-PAM antibodies (green) or anti NeuN (red).
   **Panel B:** Formalin assay of animals treated with sense or antisense ODNs as described in the material section. The total amount of flinches over 1 hour is shown. The mean ± SE of at least 4 determinations is shown.
   **Panel C**: Formalin assay of animals treated with sense or antisense ODNs as described in the material section. The number of flinches during 1 hour is shown. The mean ± SE of at least 4 determinations is shown.

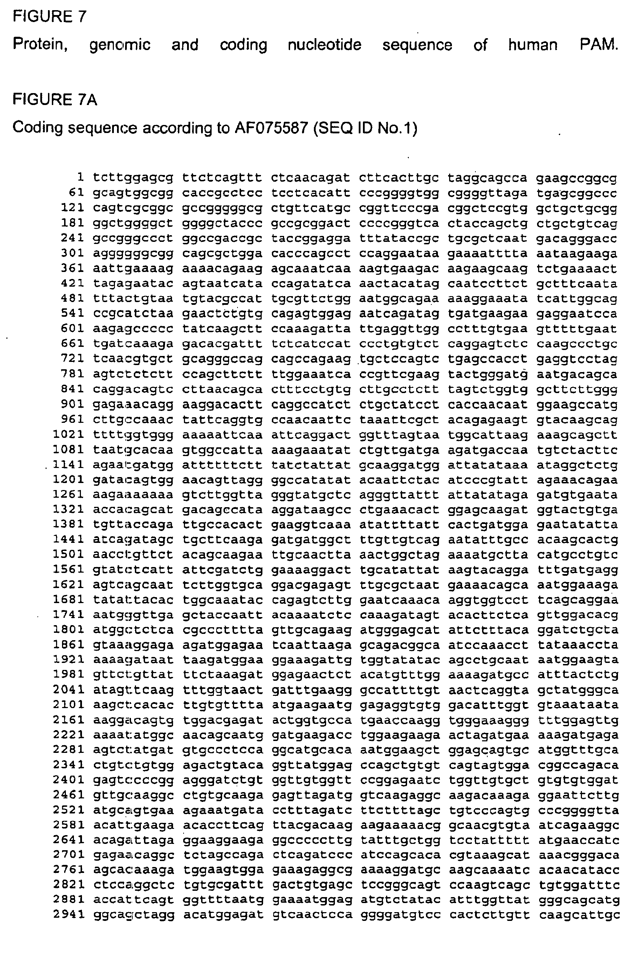

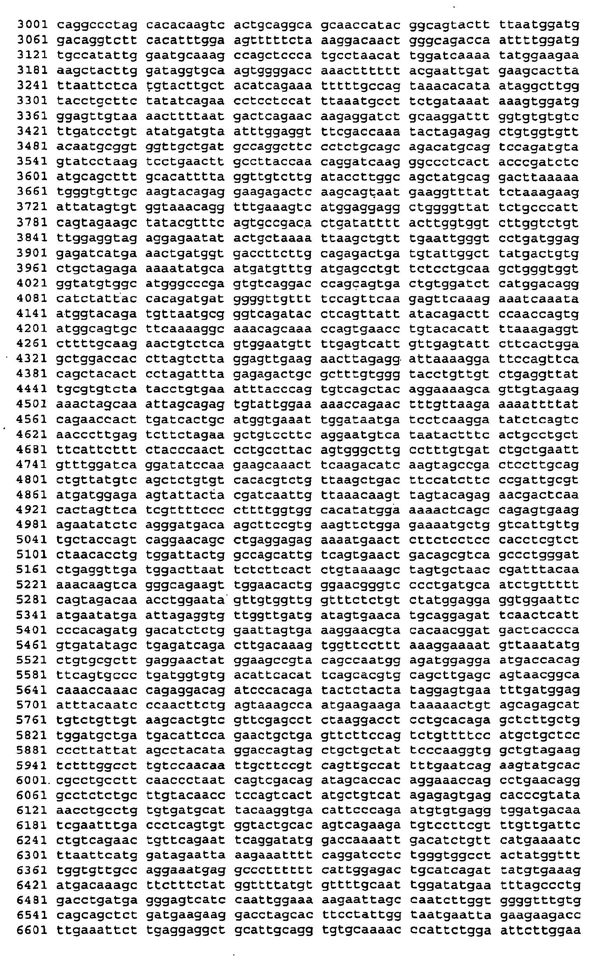

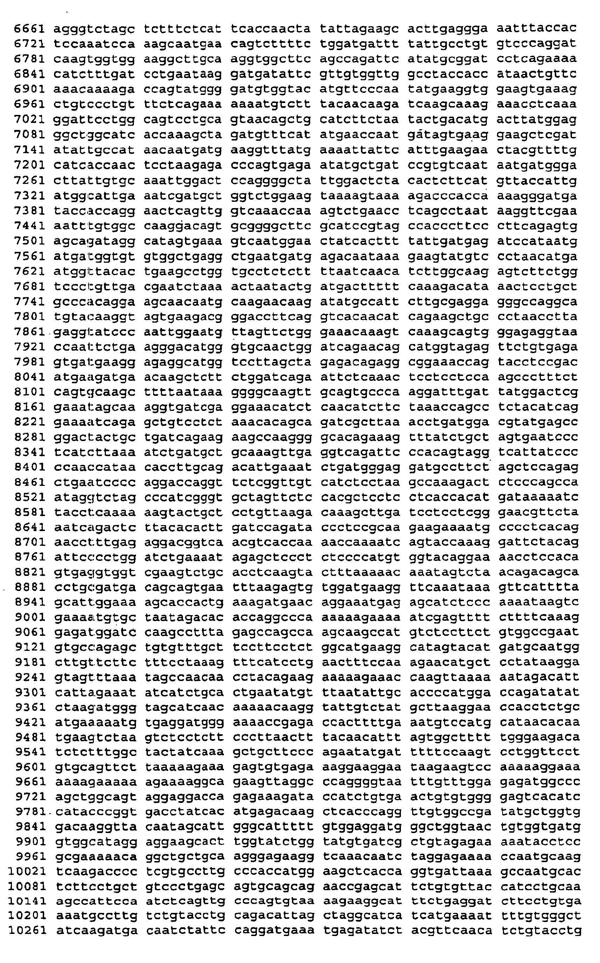

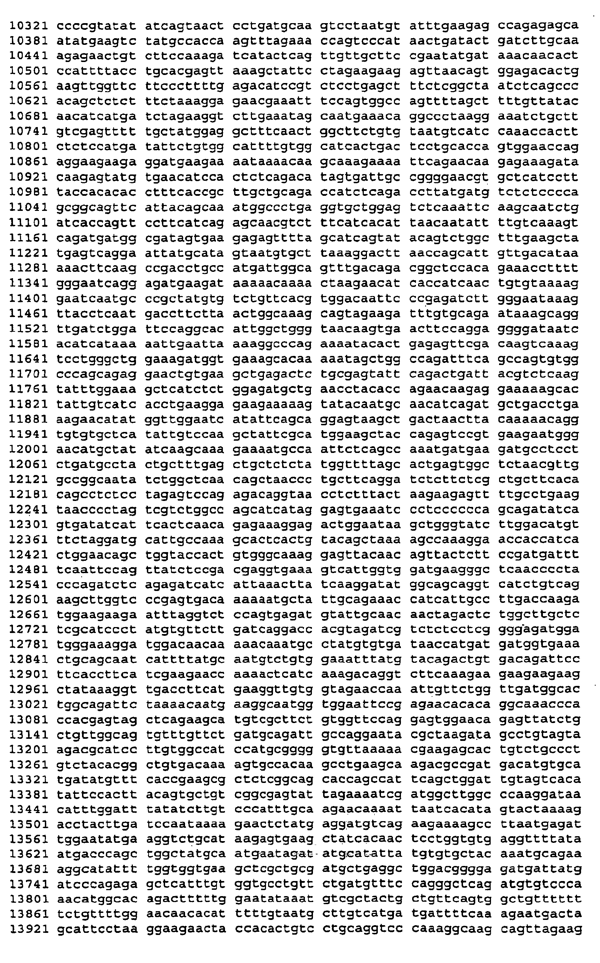

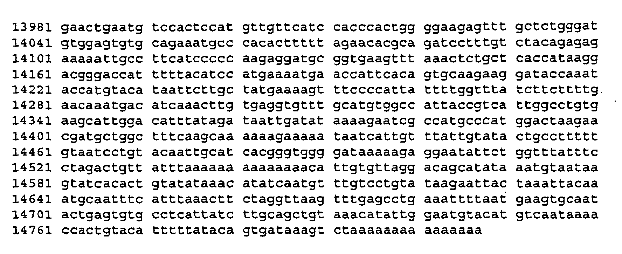

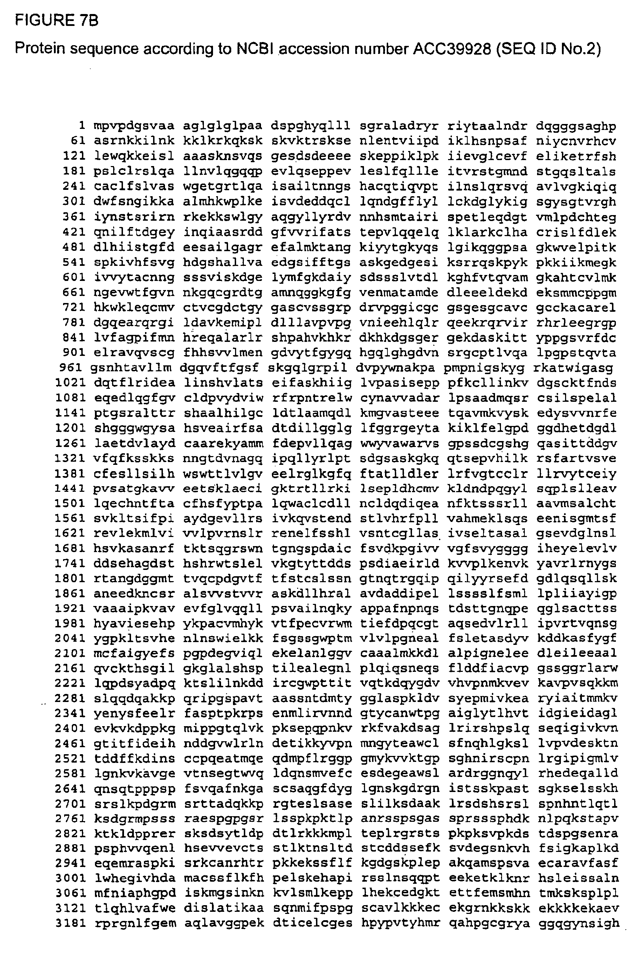

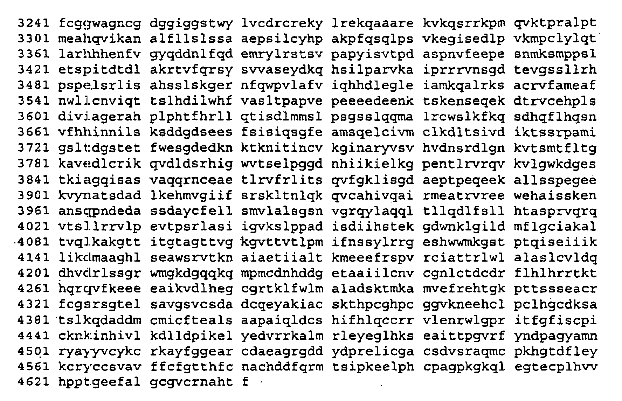

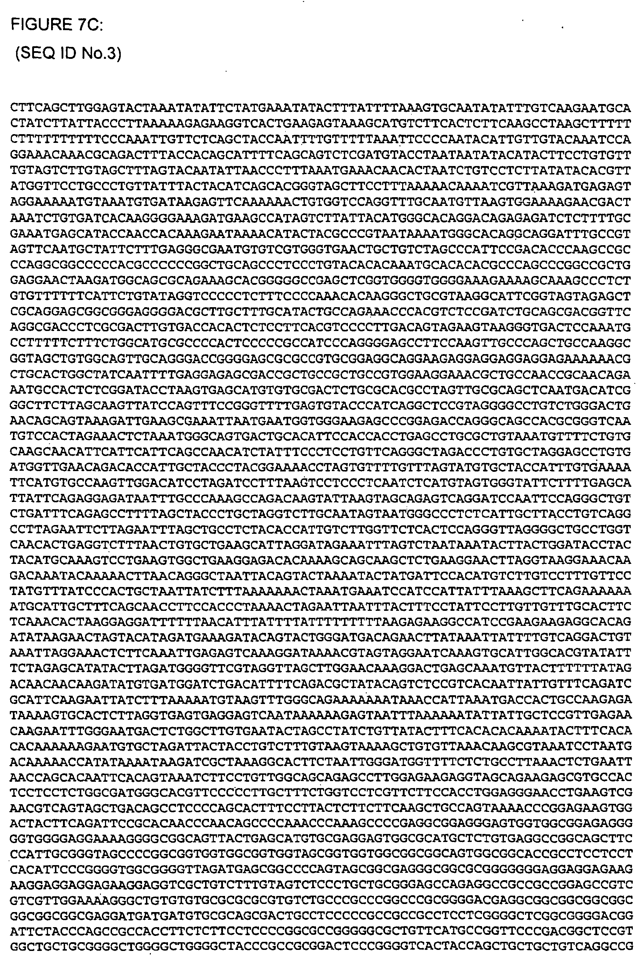

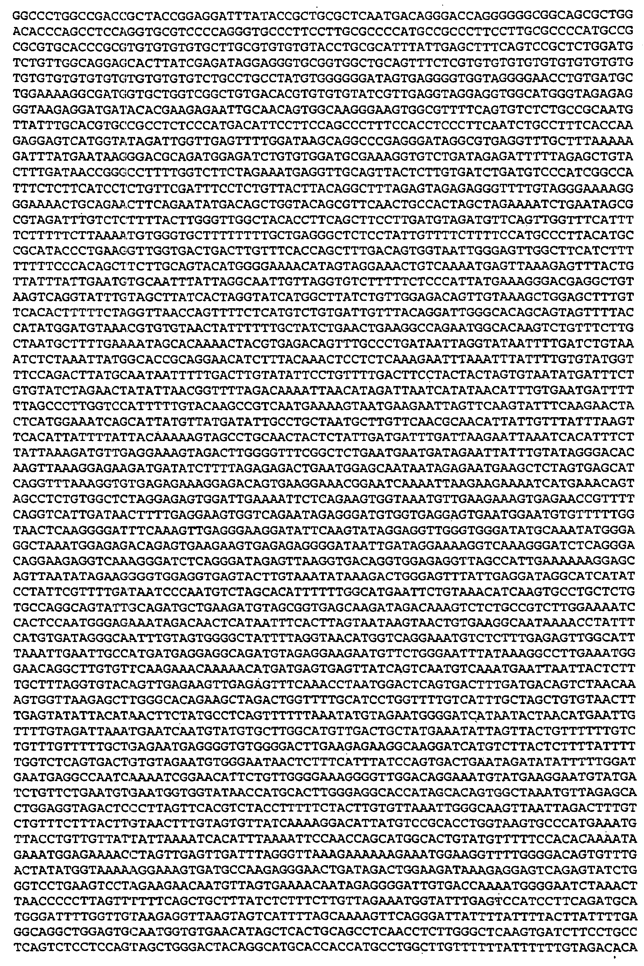

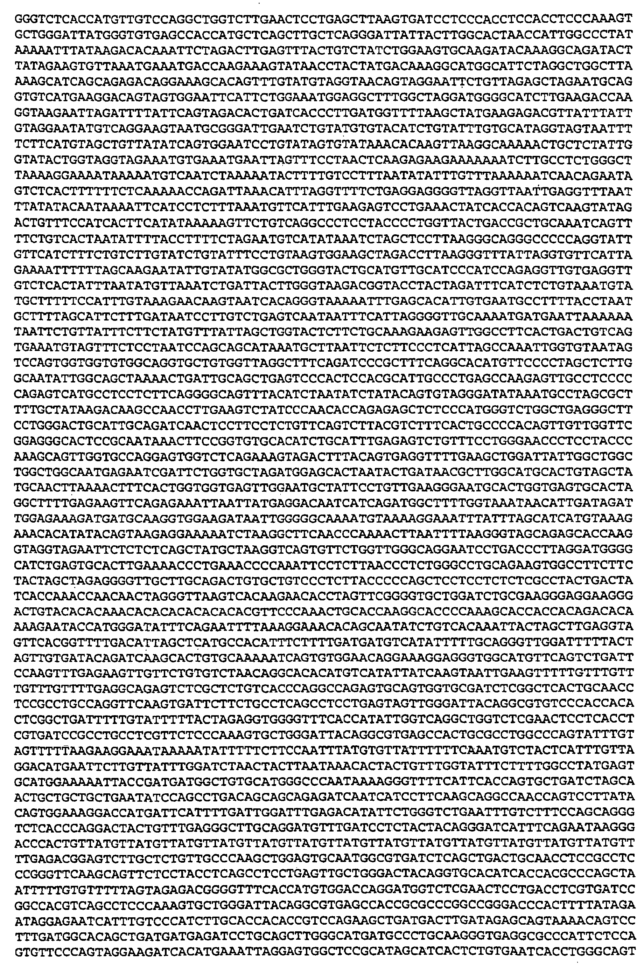

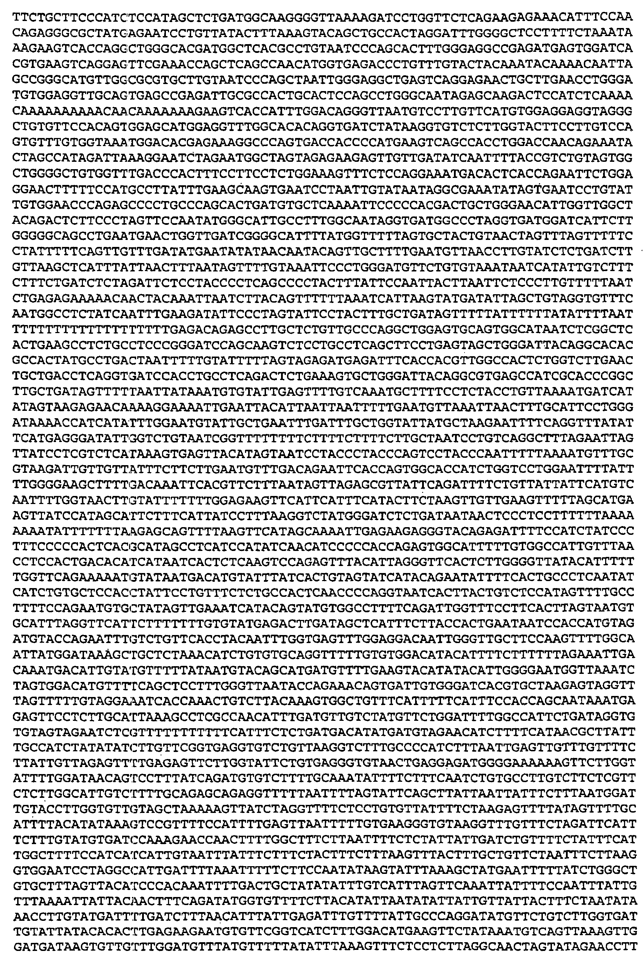

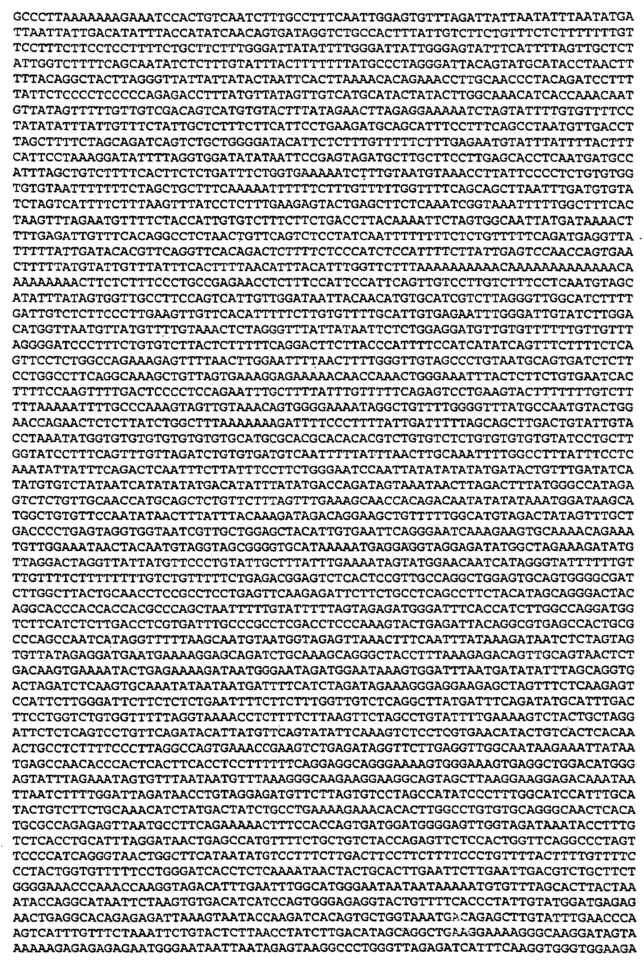

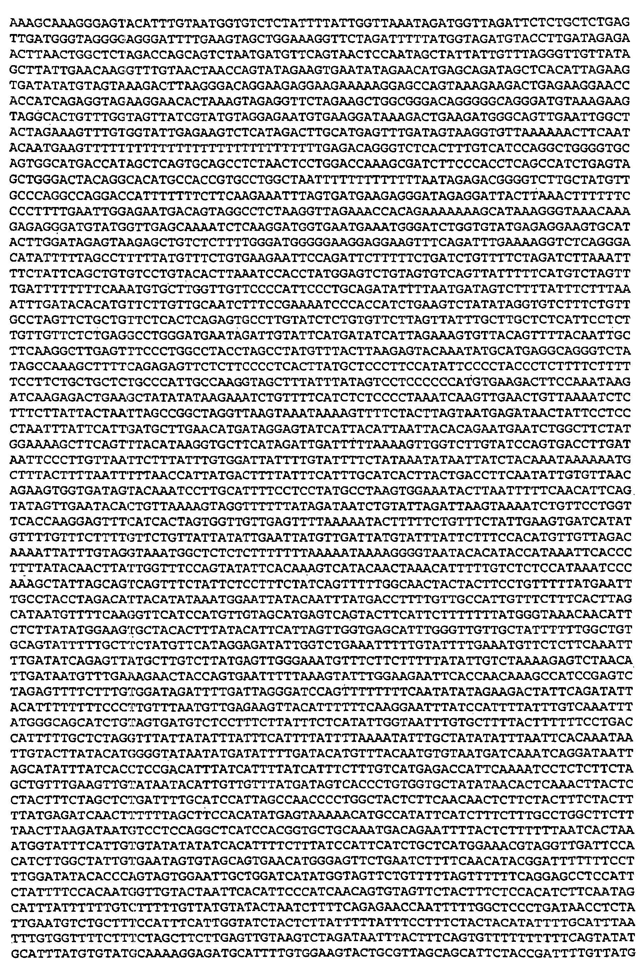

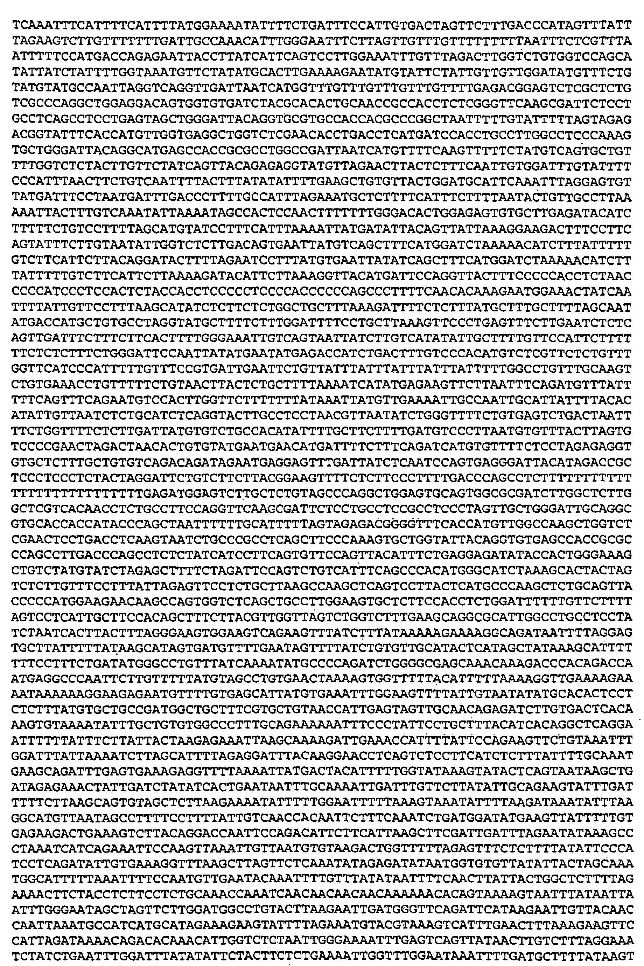

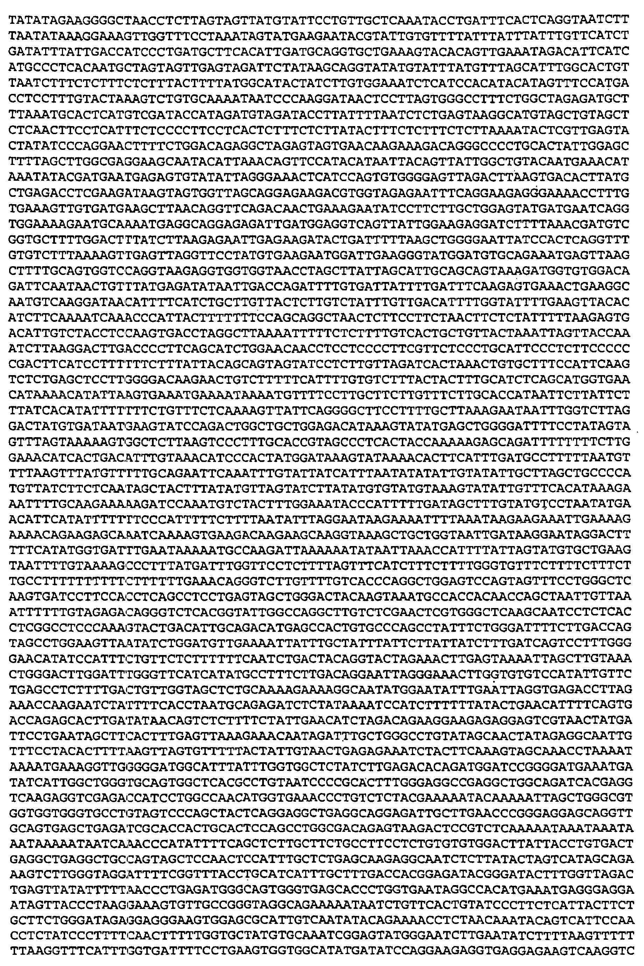

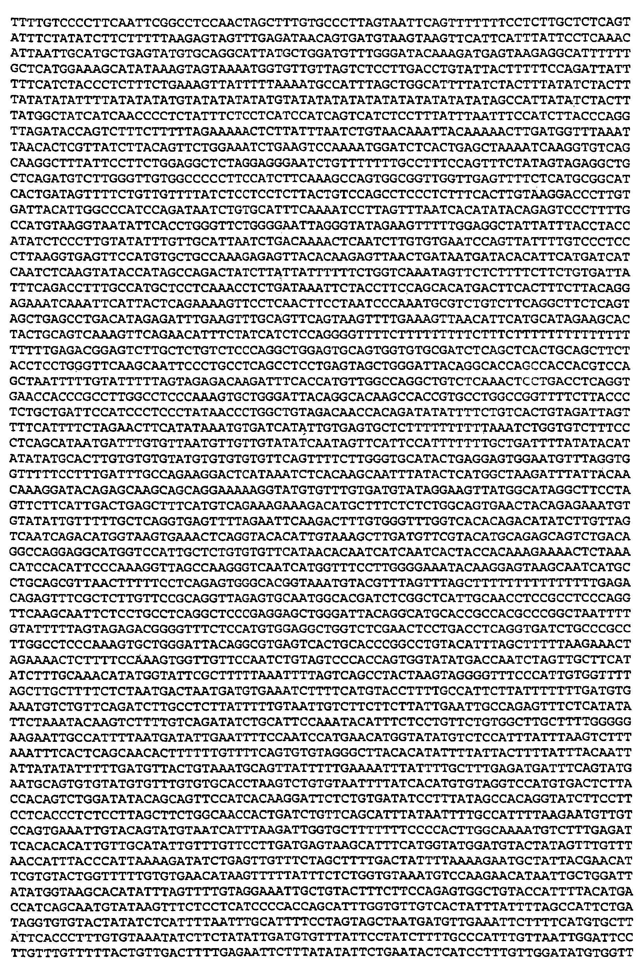

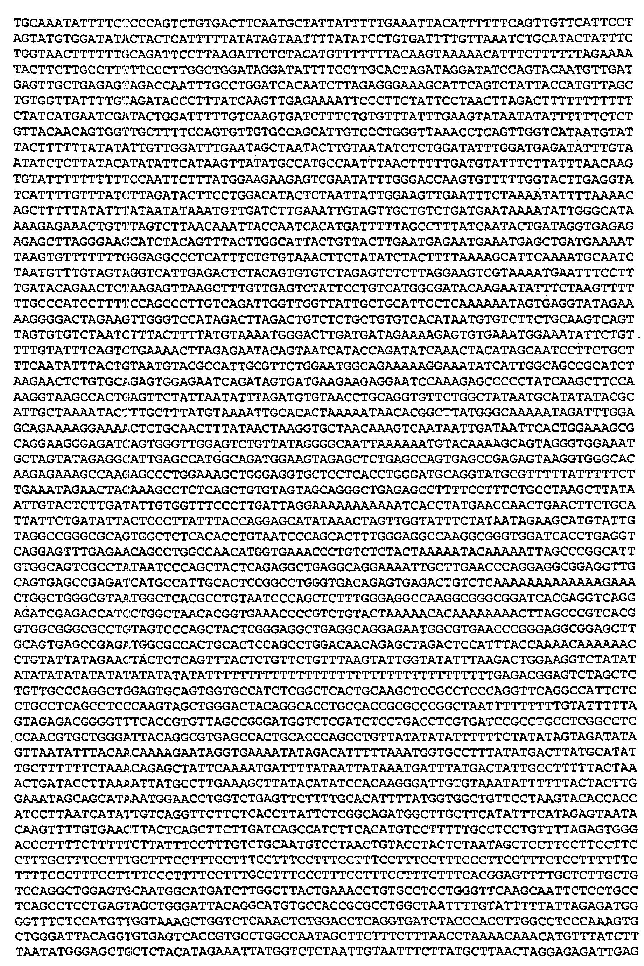

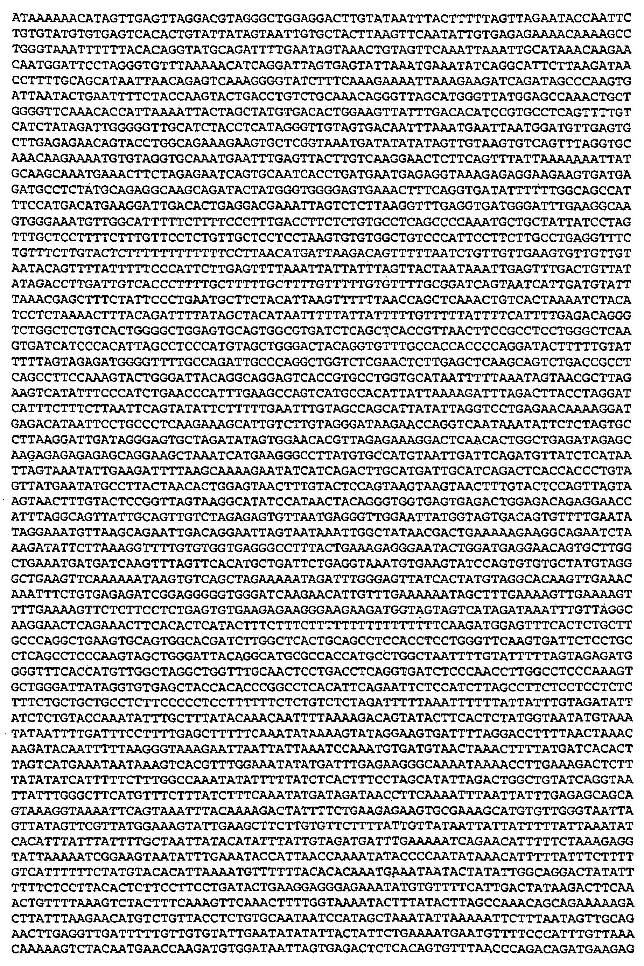

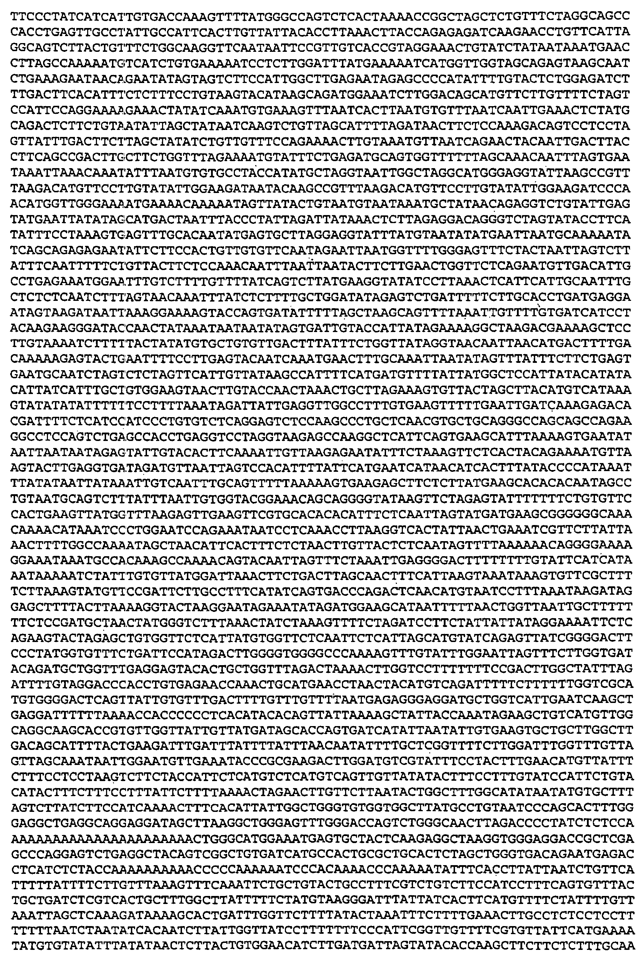

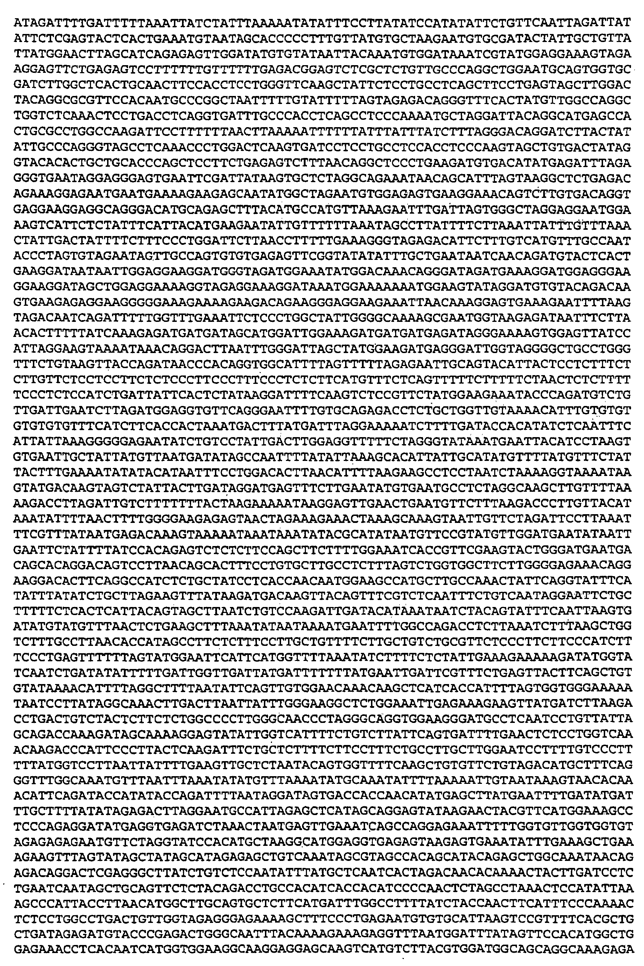

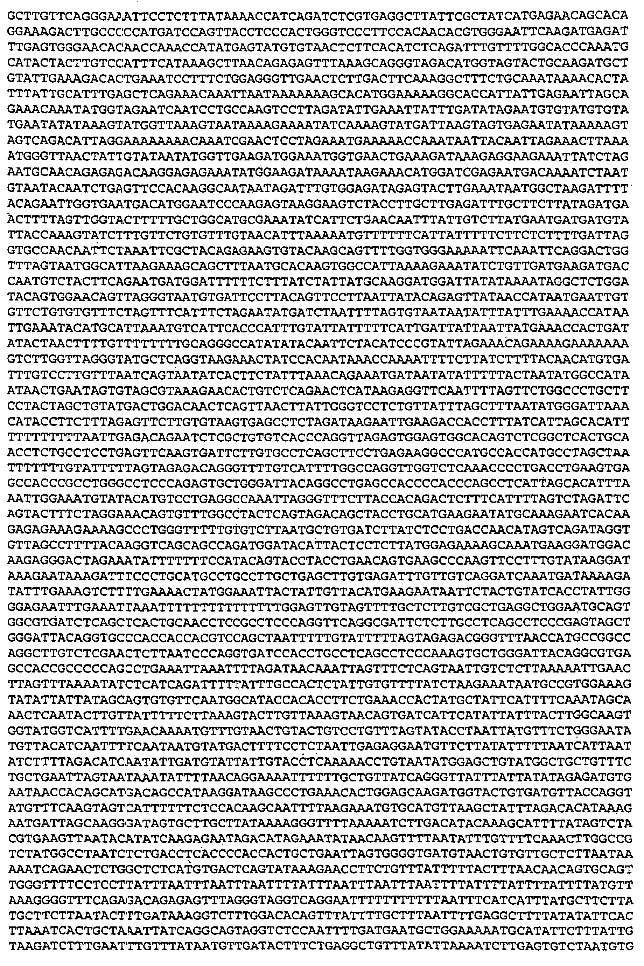

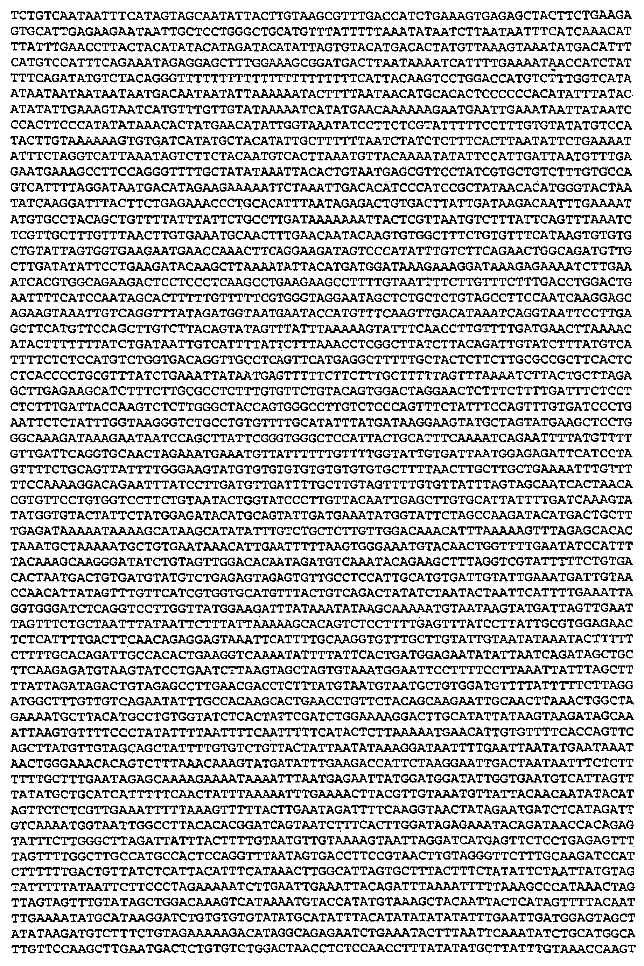

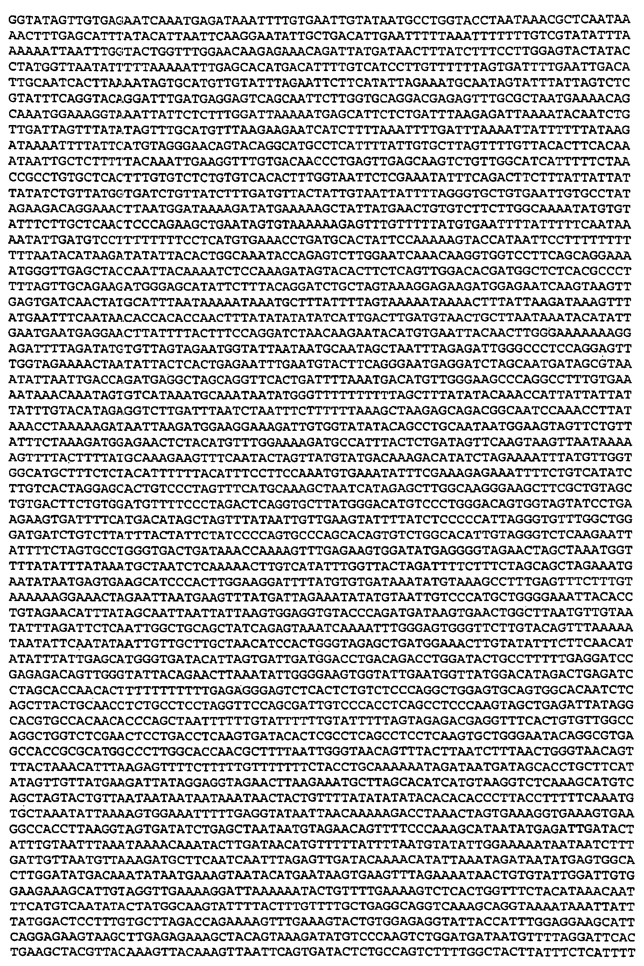

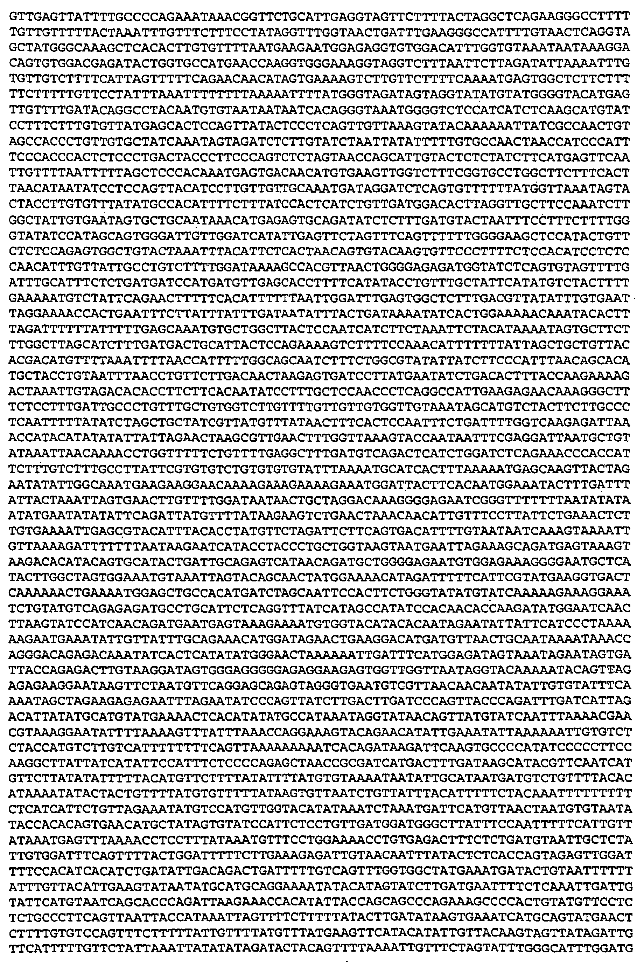

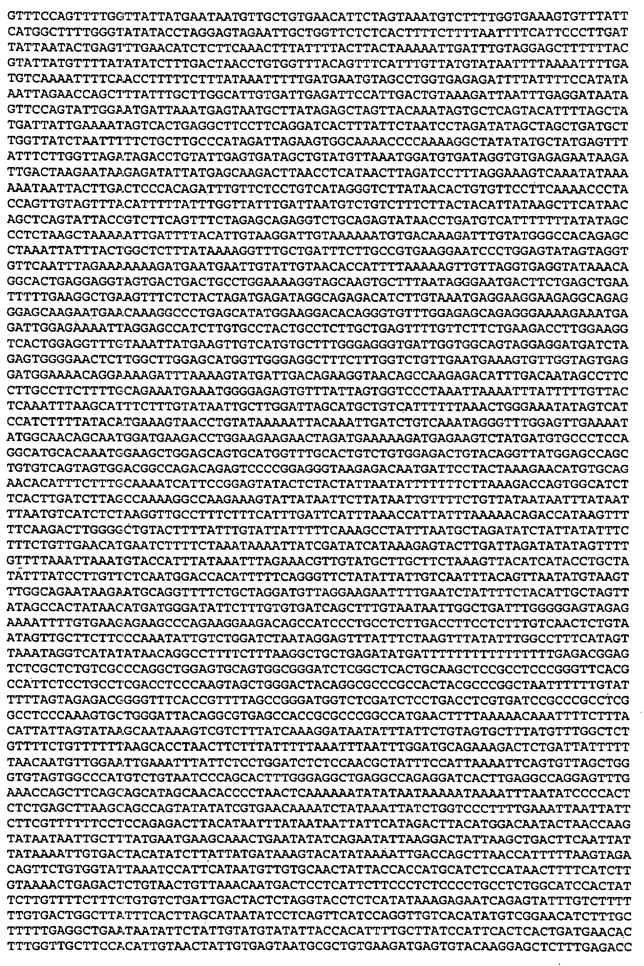

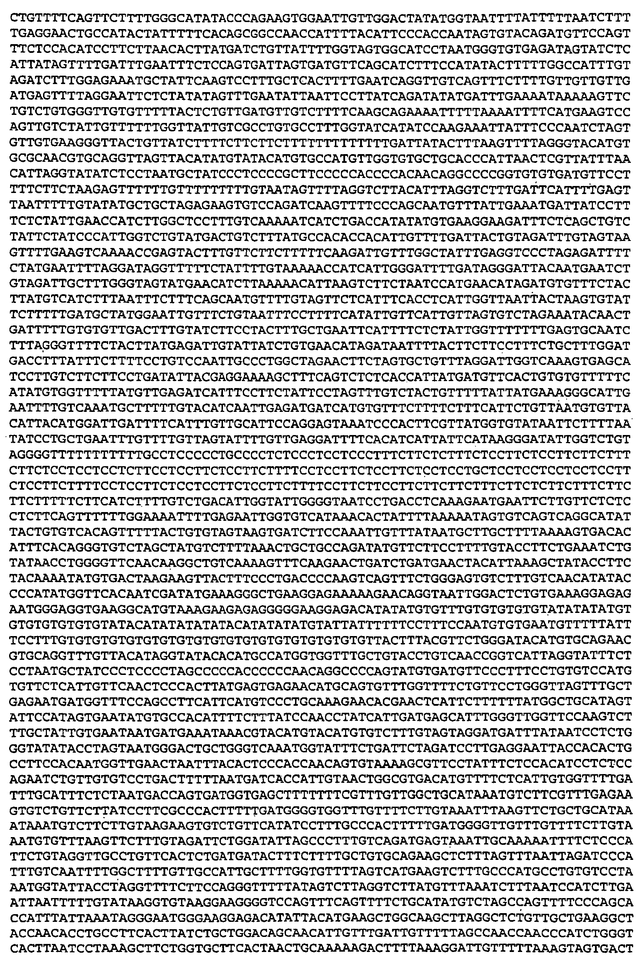

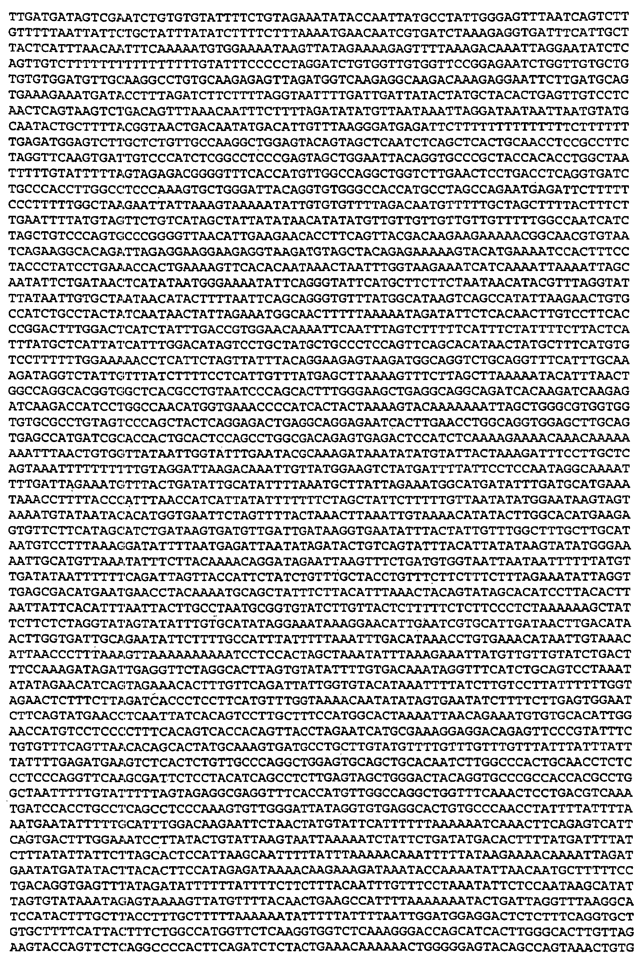

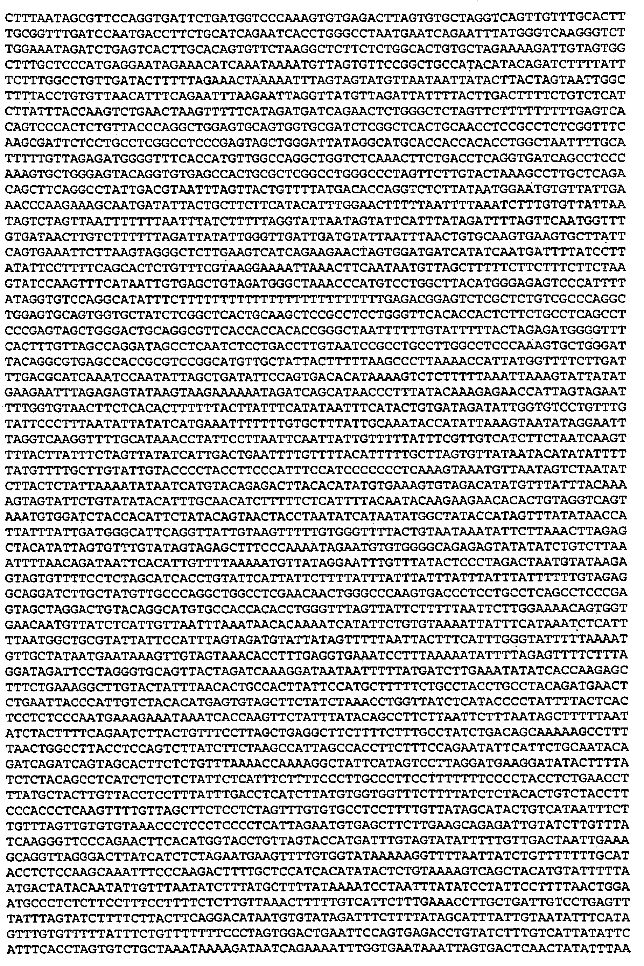

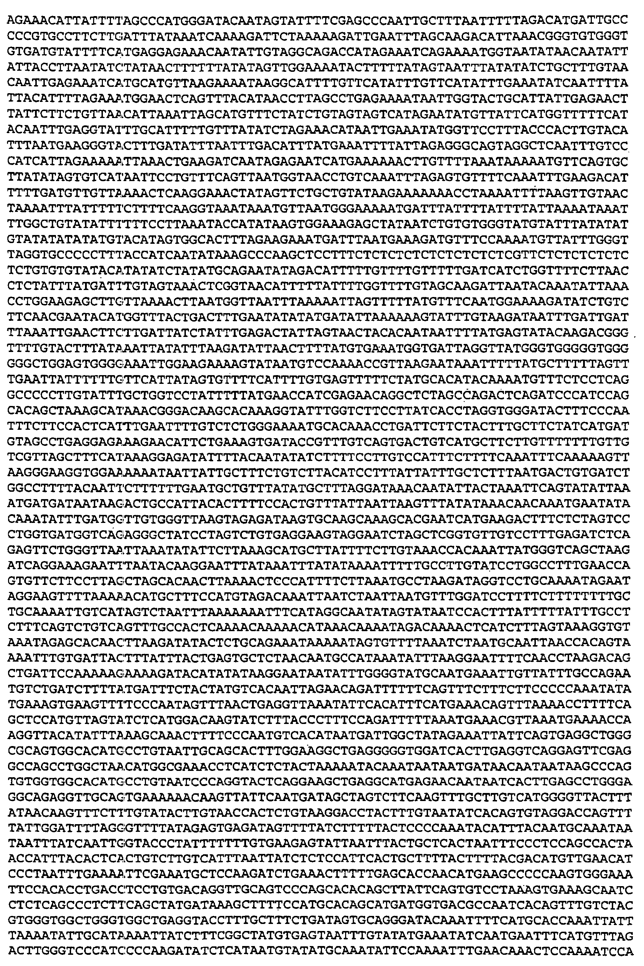

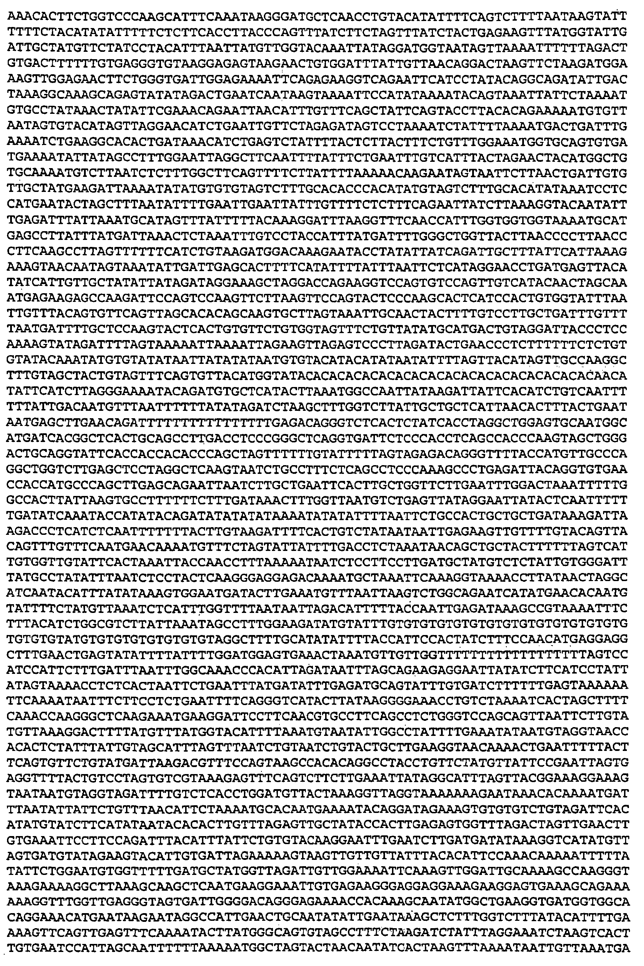

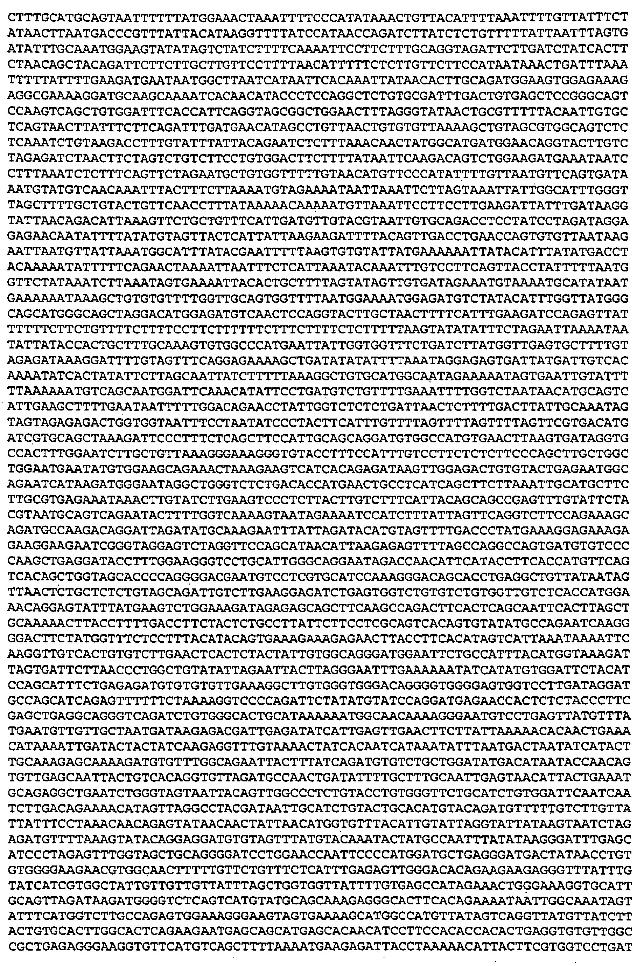

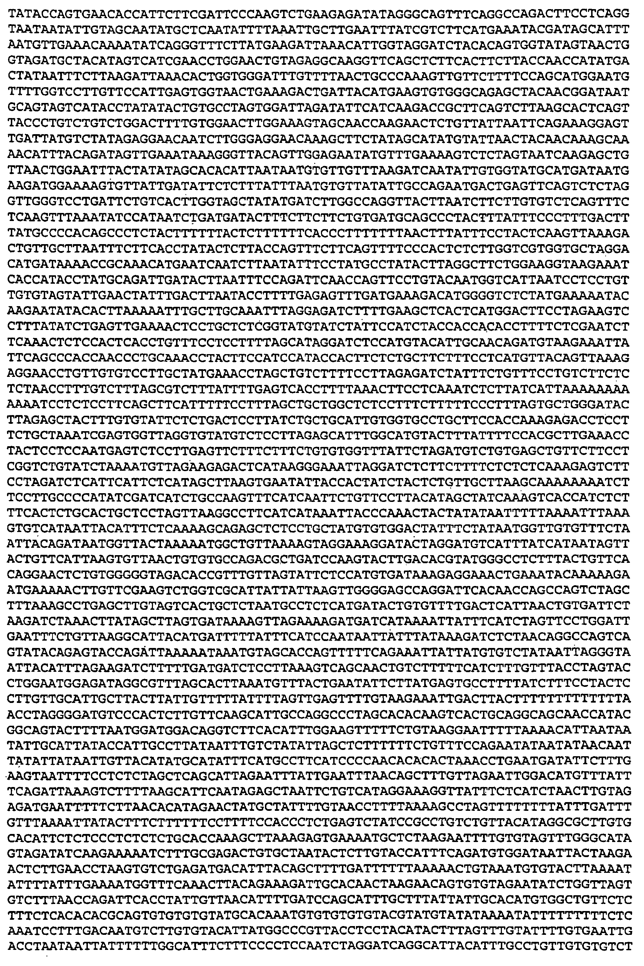

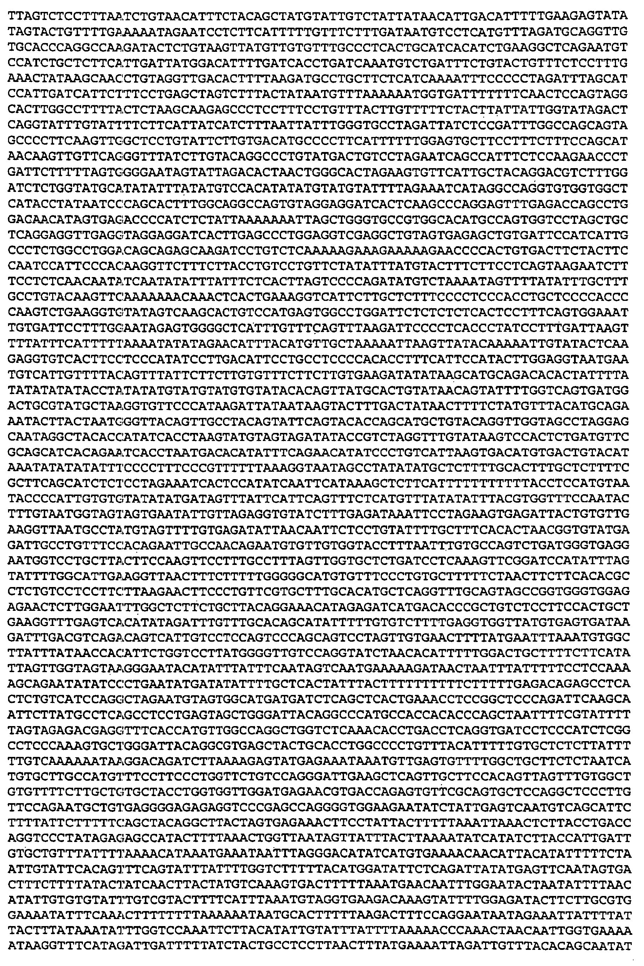

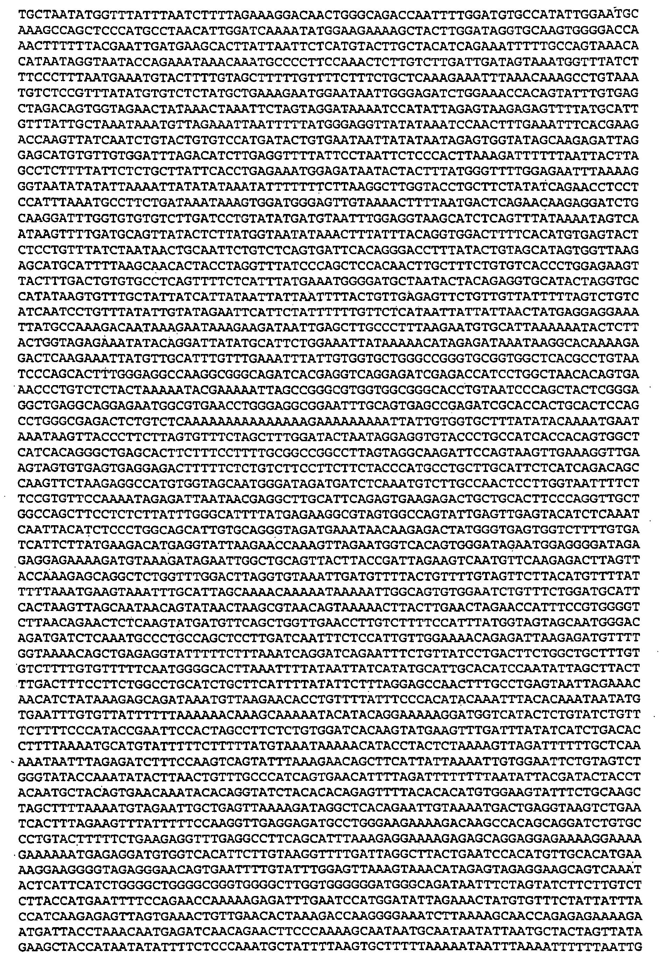

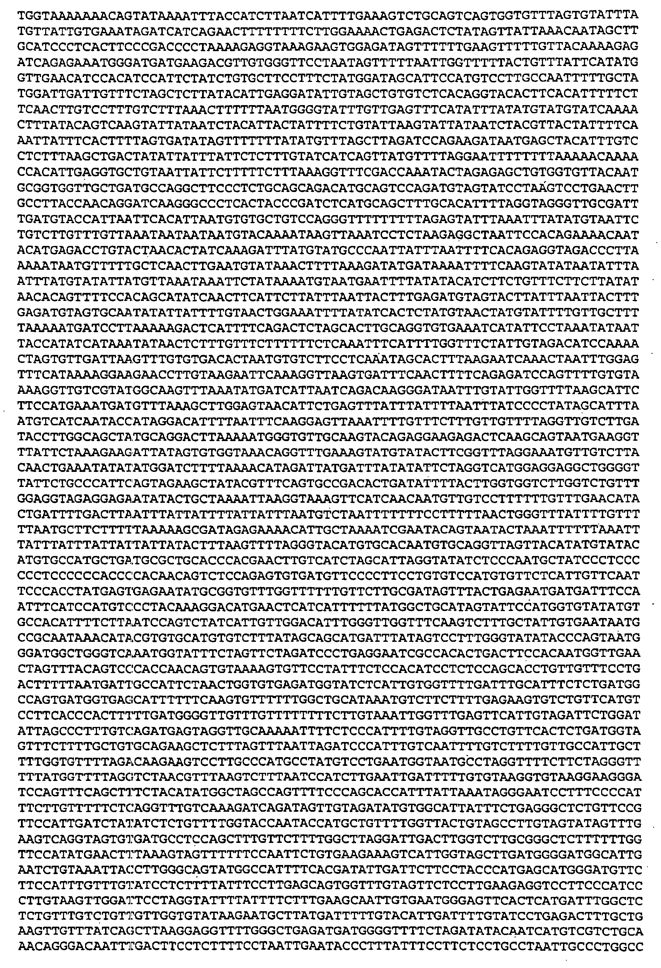

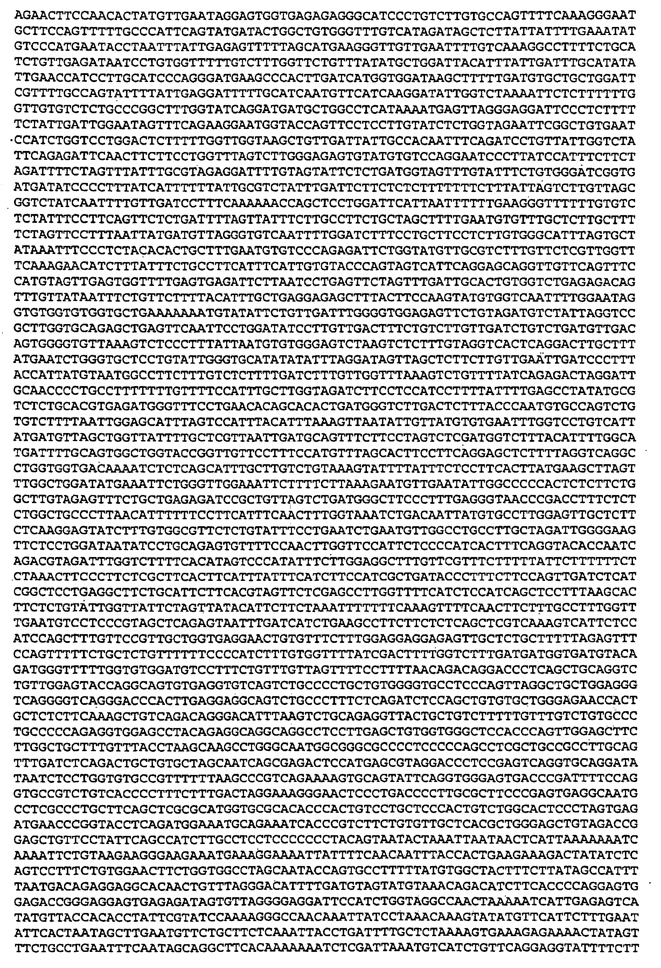

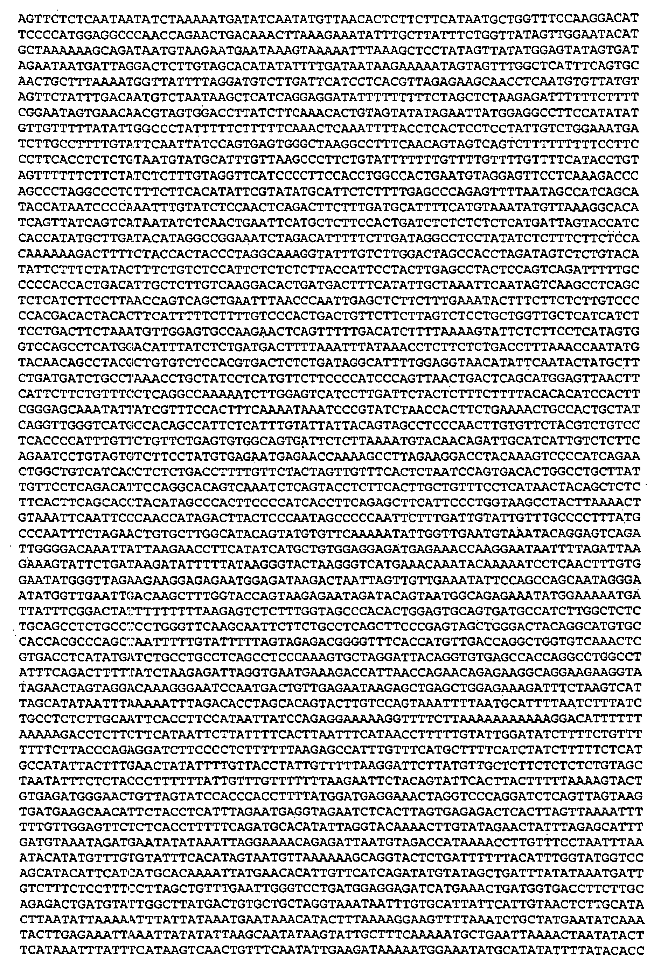

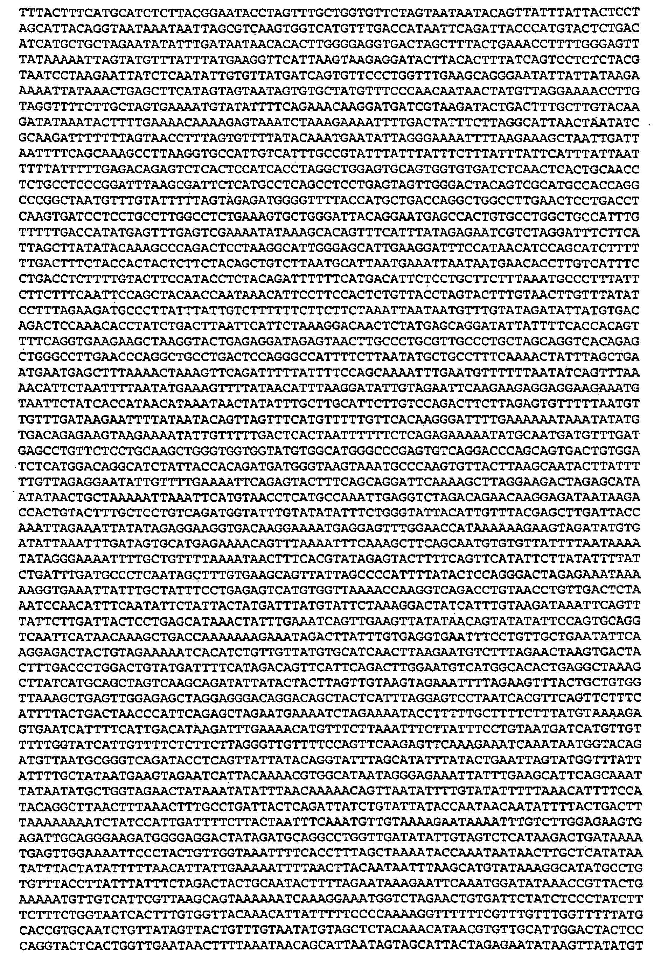

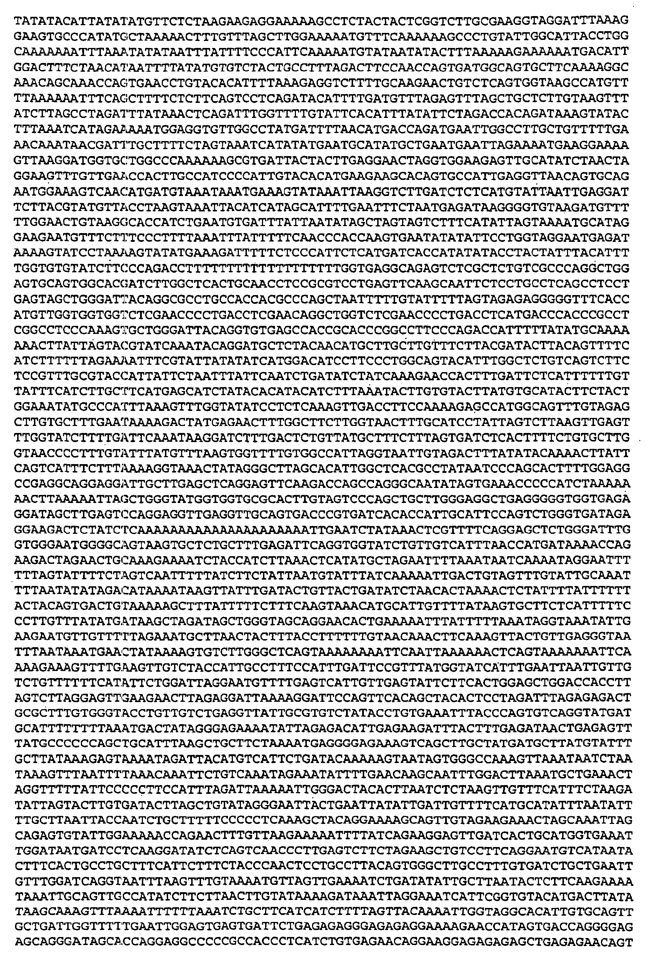

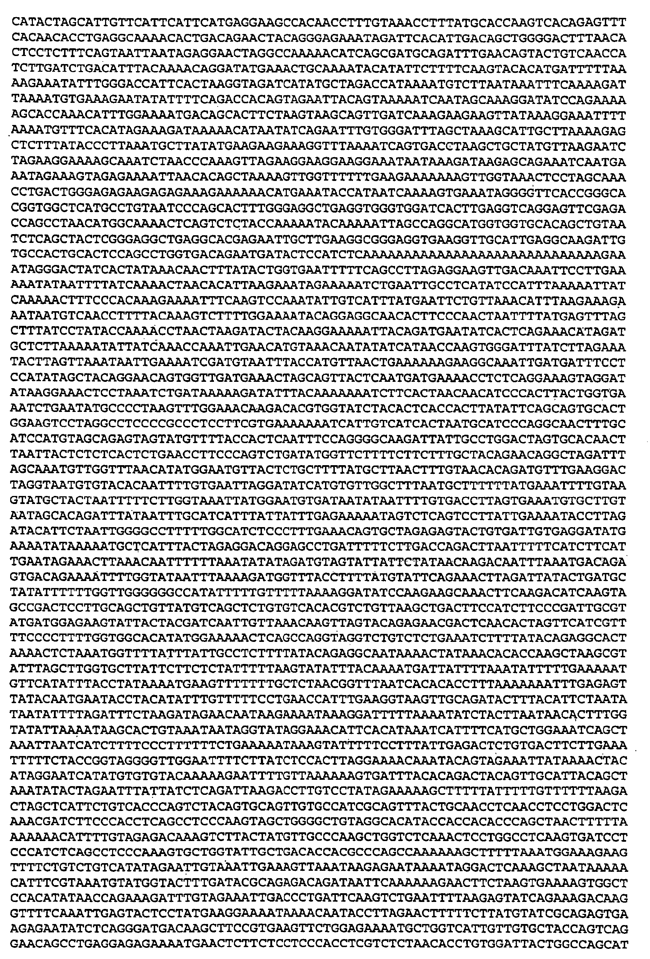

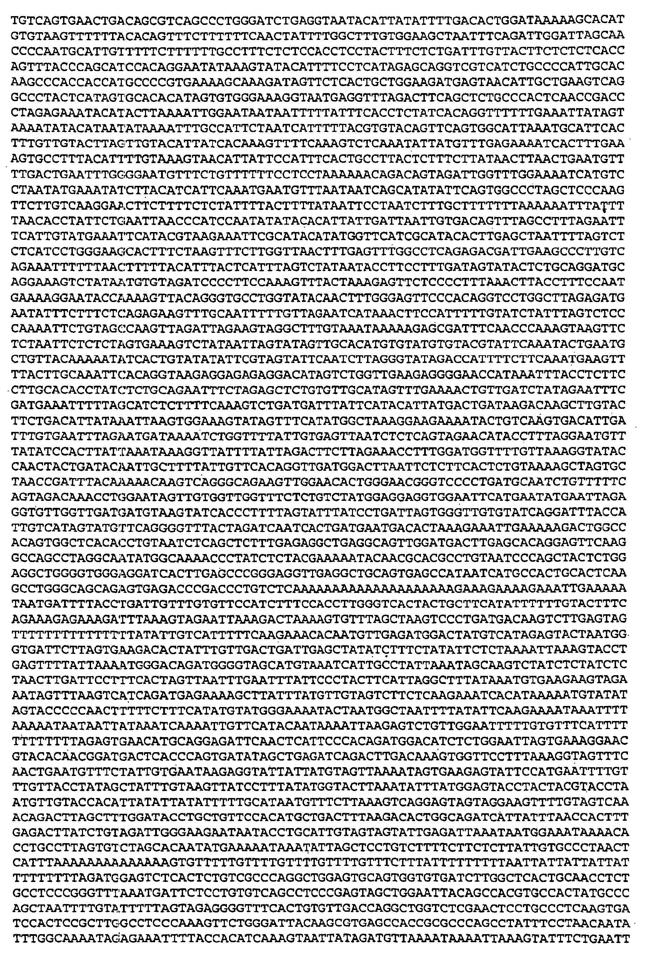

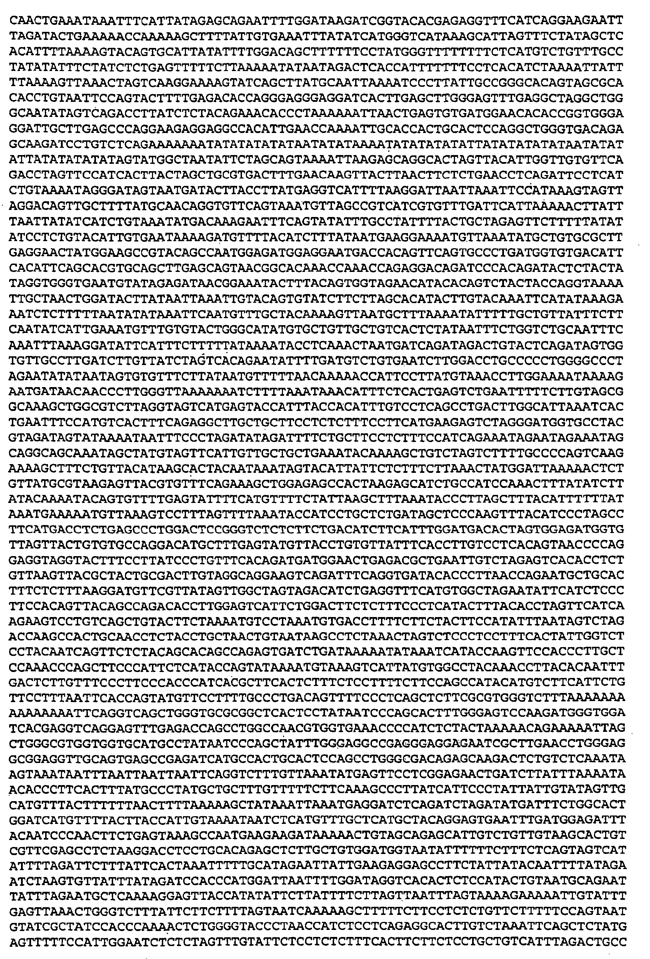

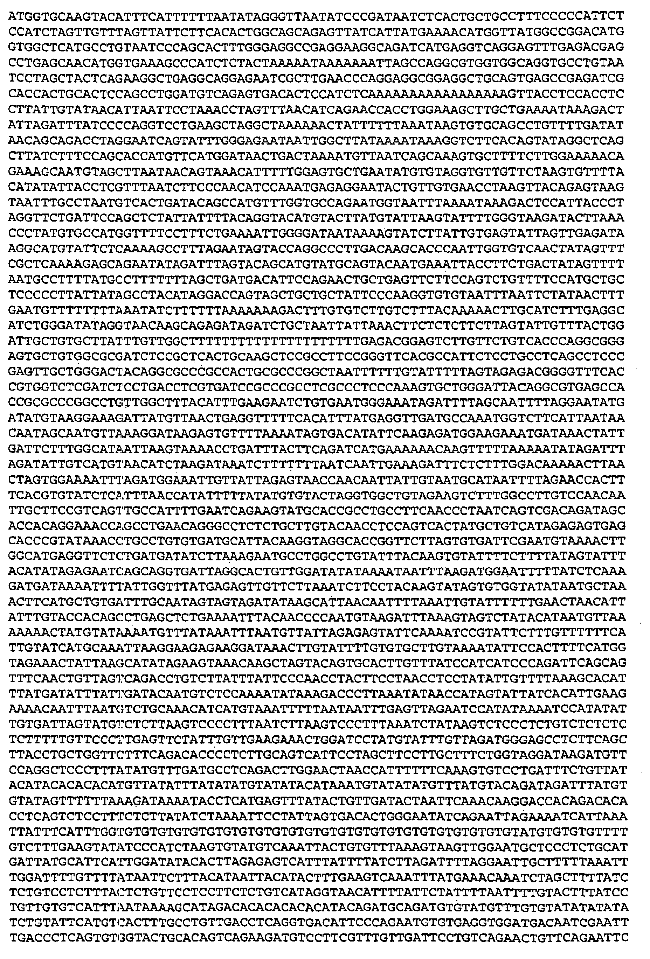

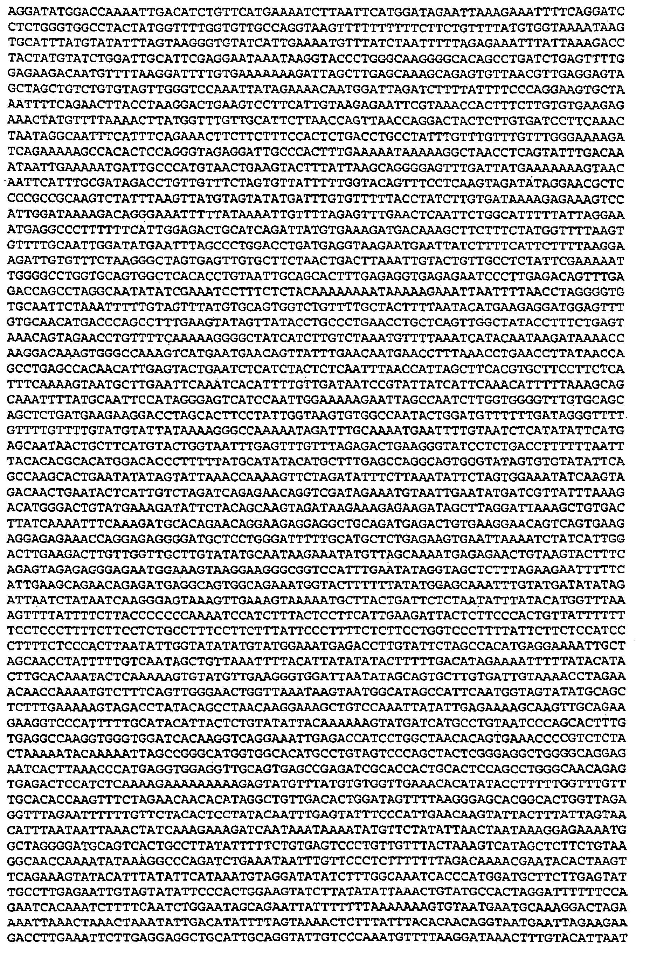

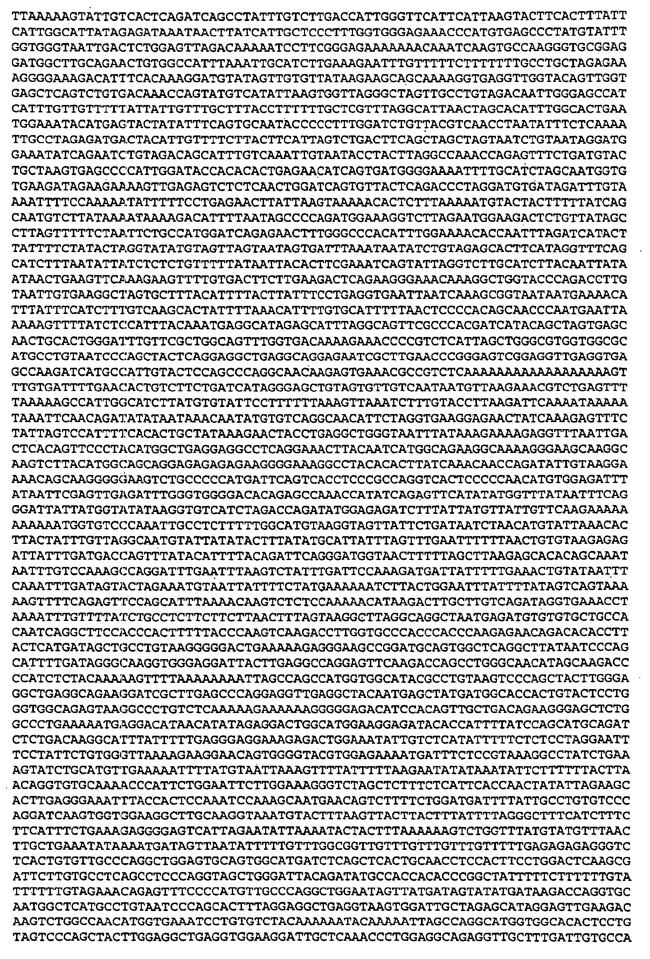

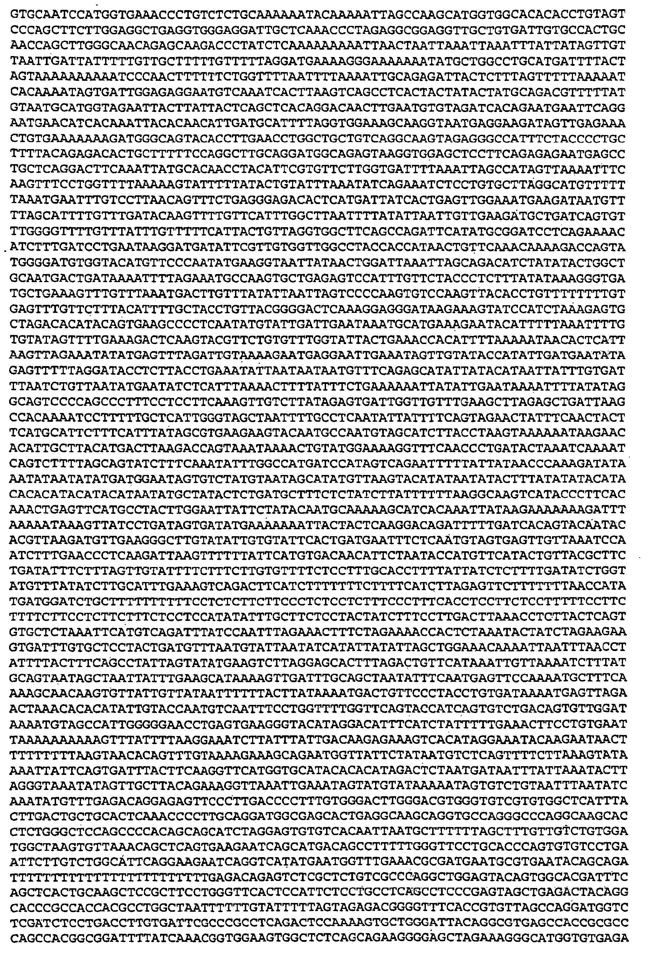

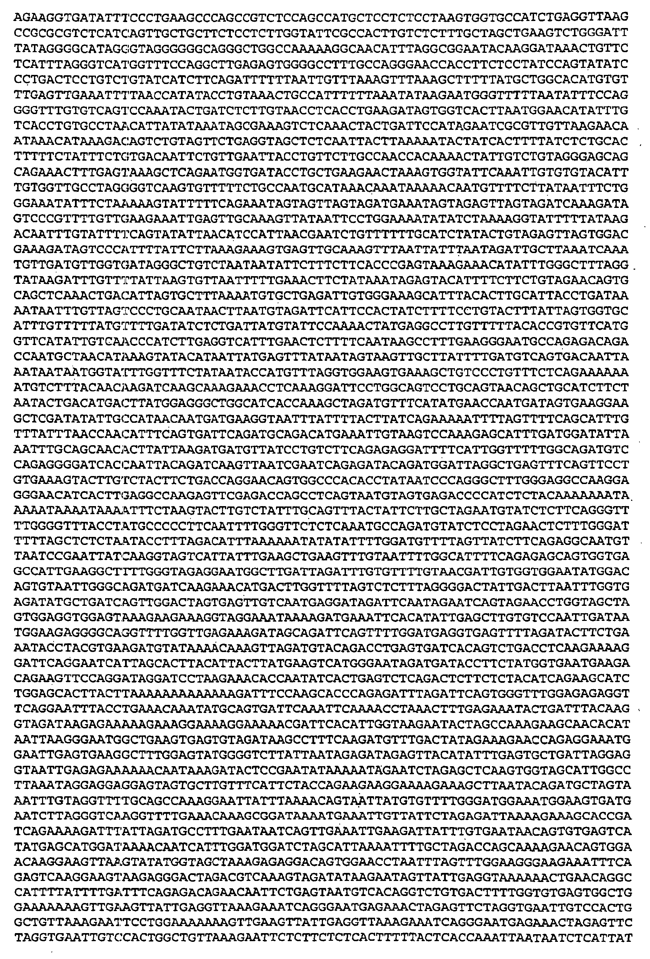

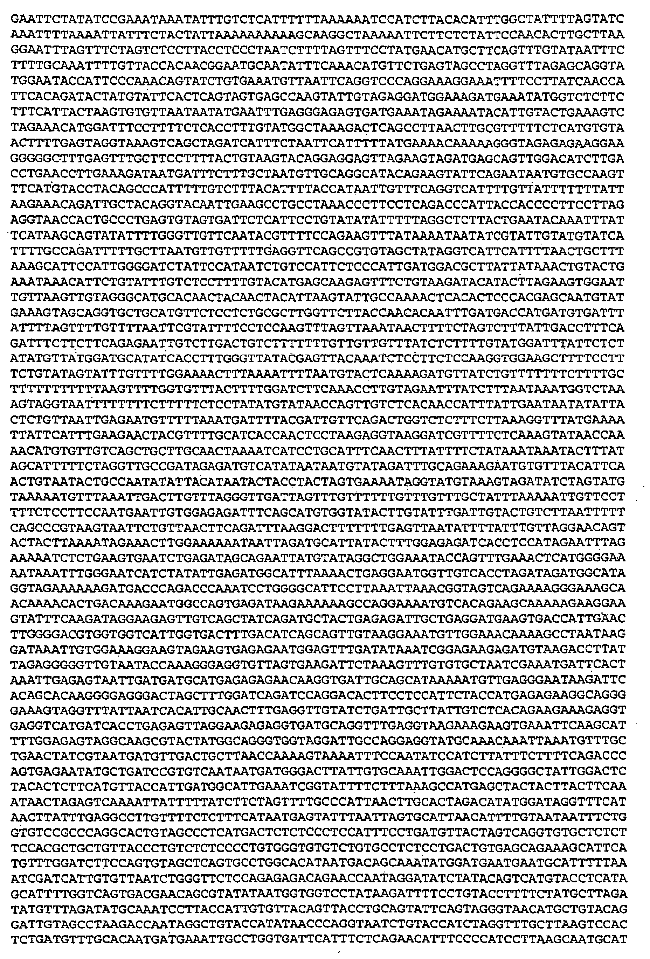

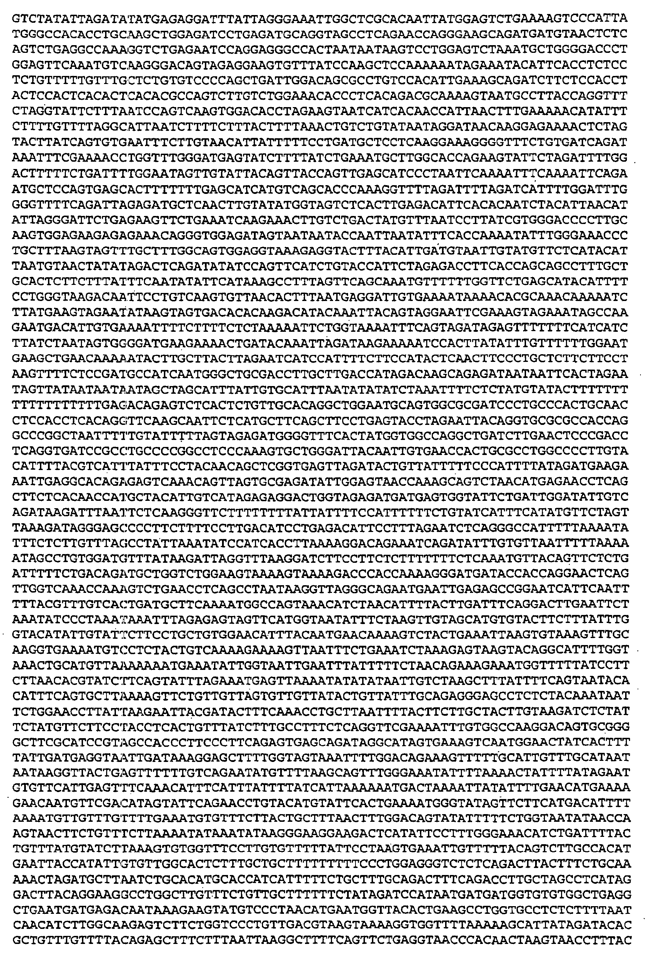

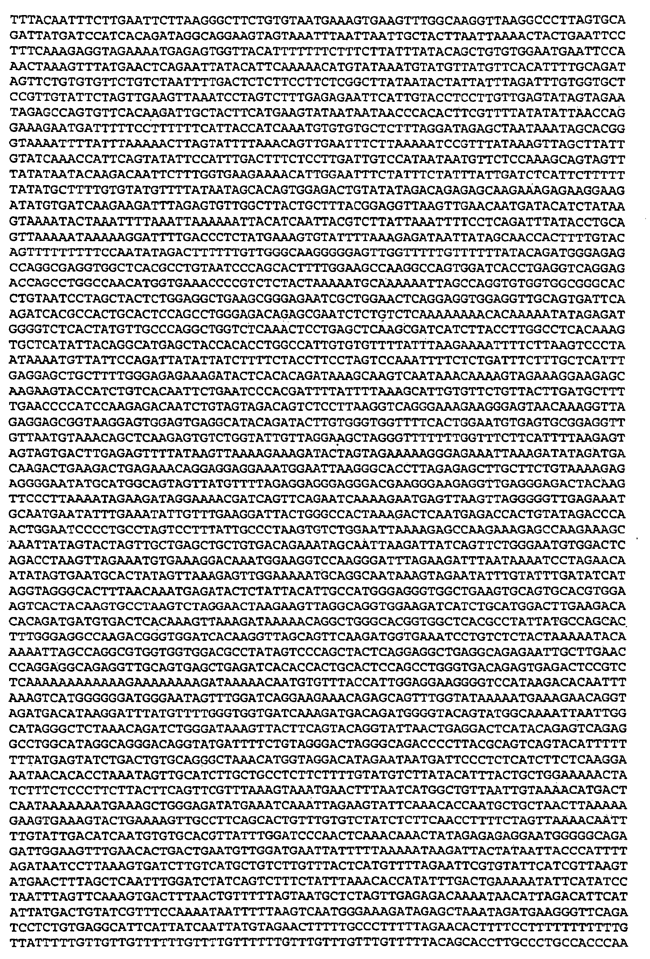

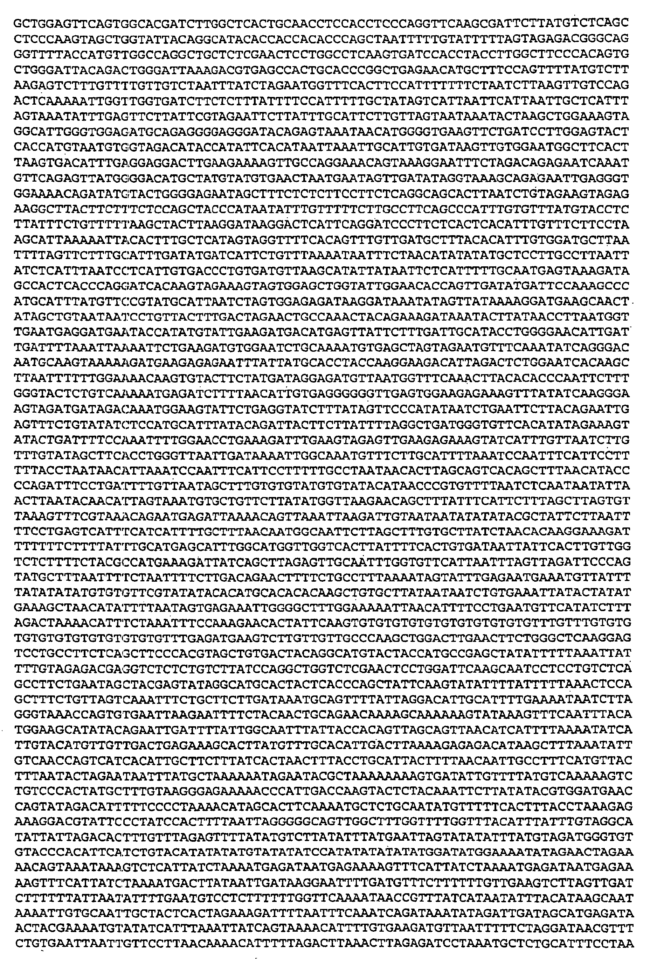

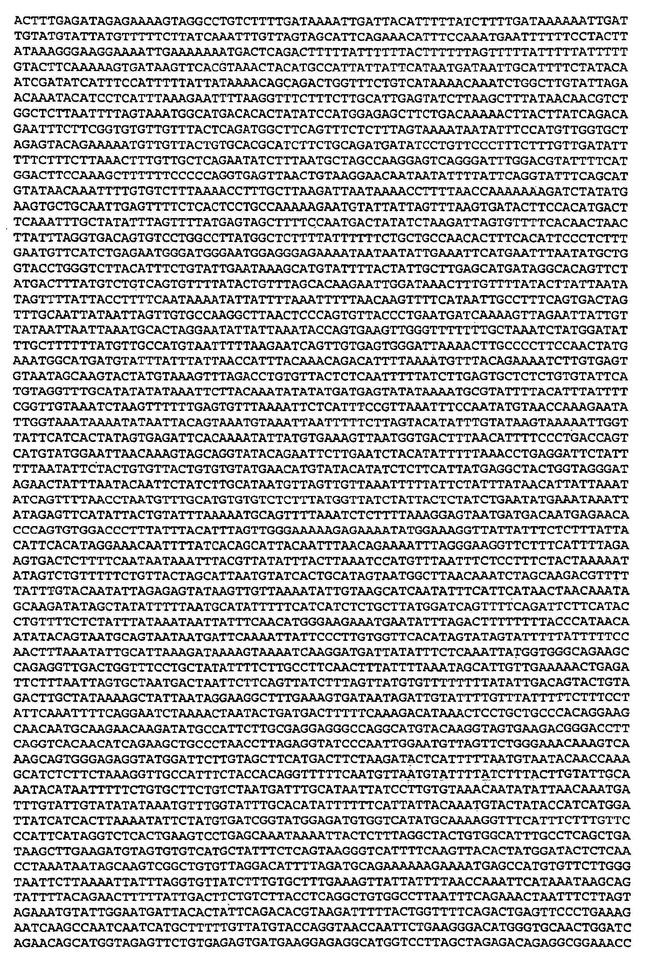

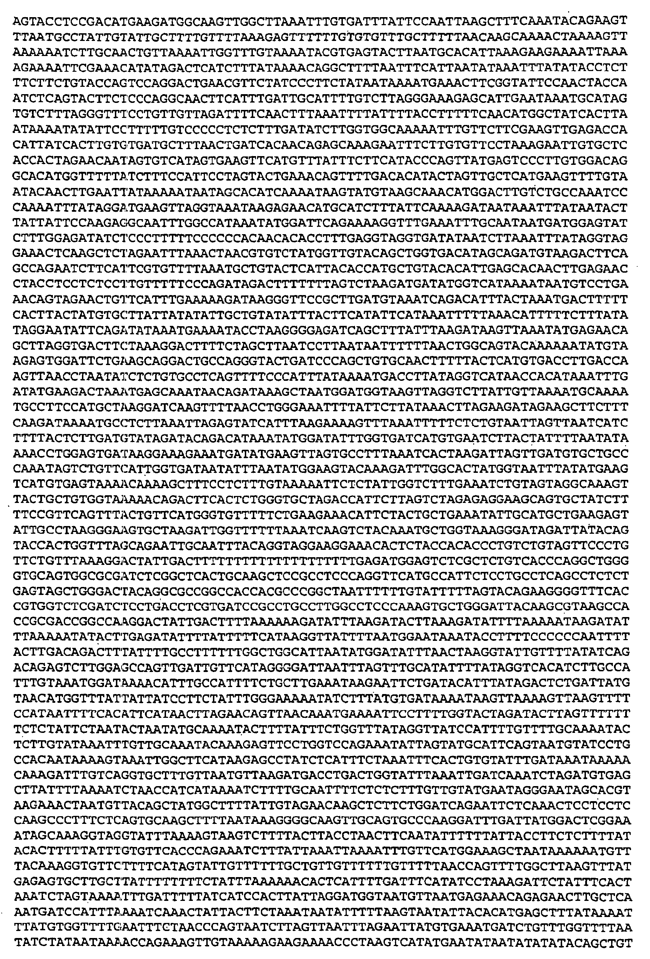

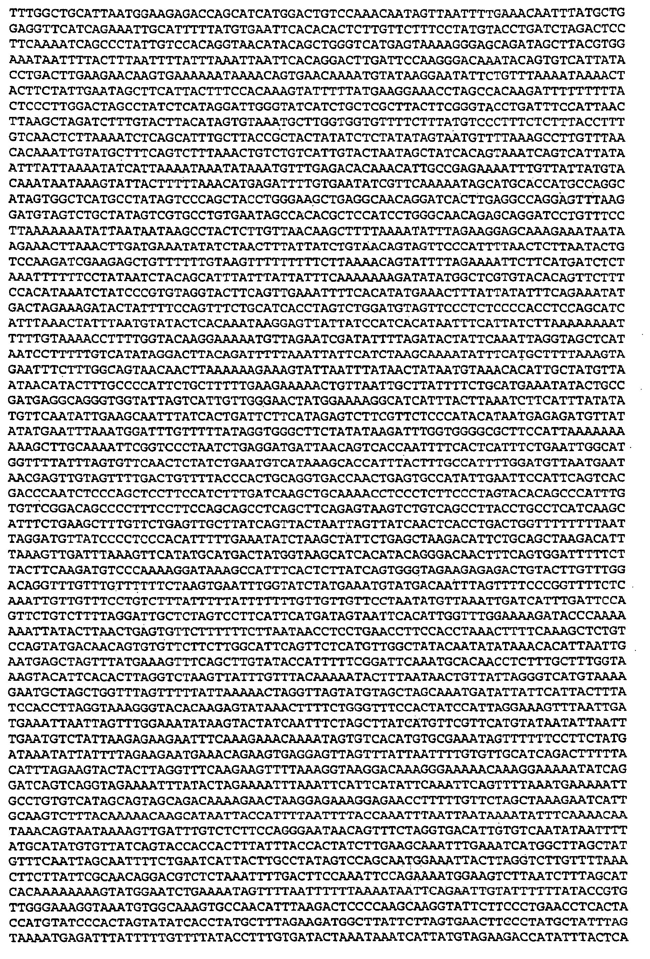

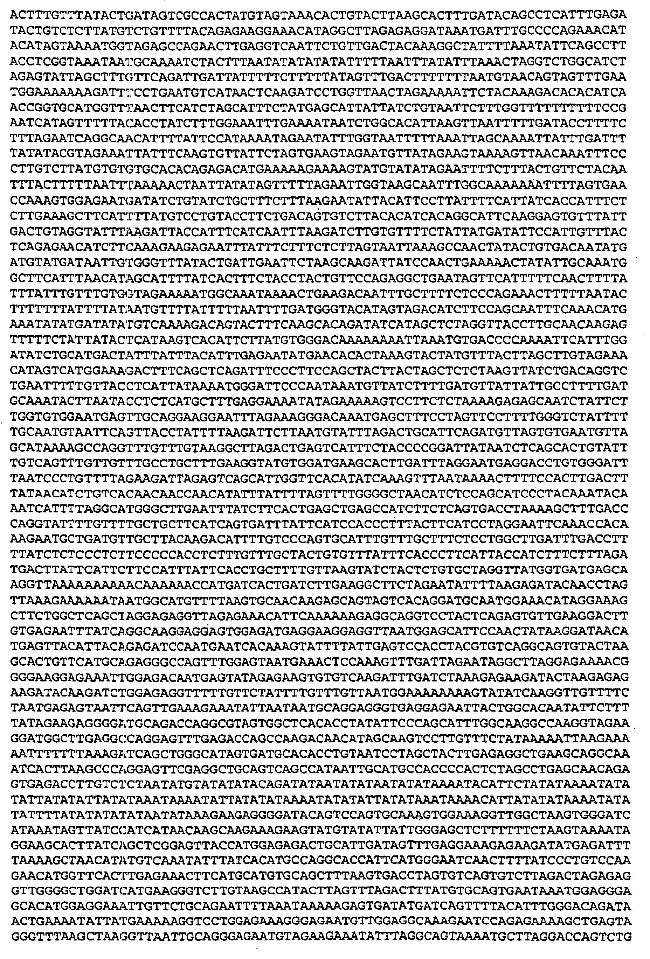

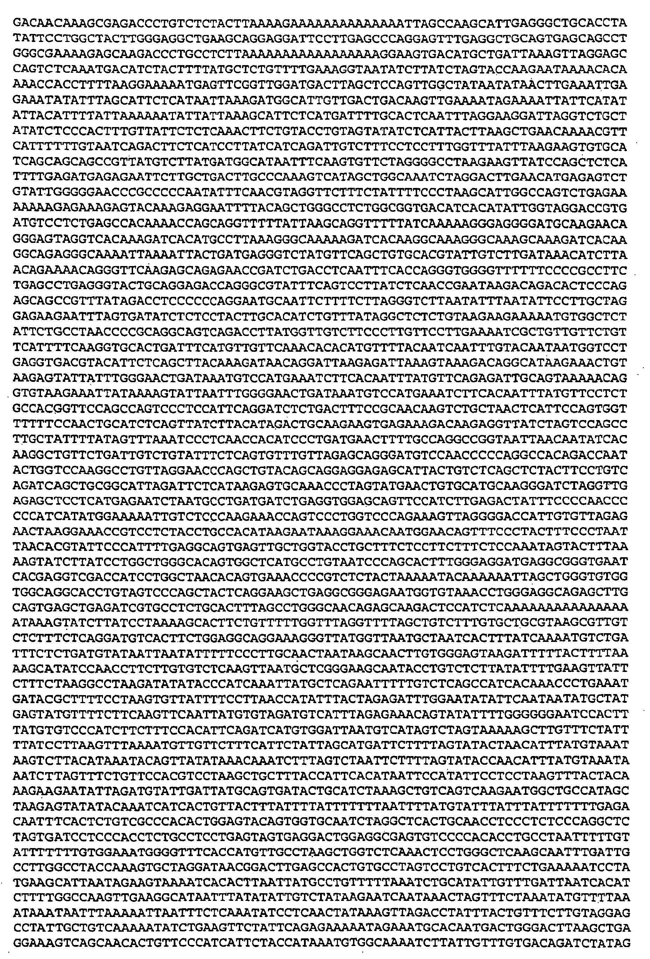

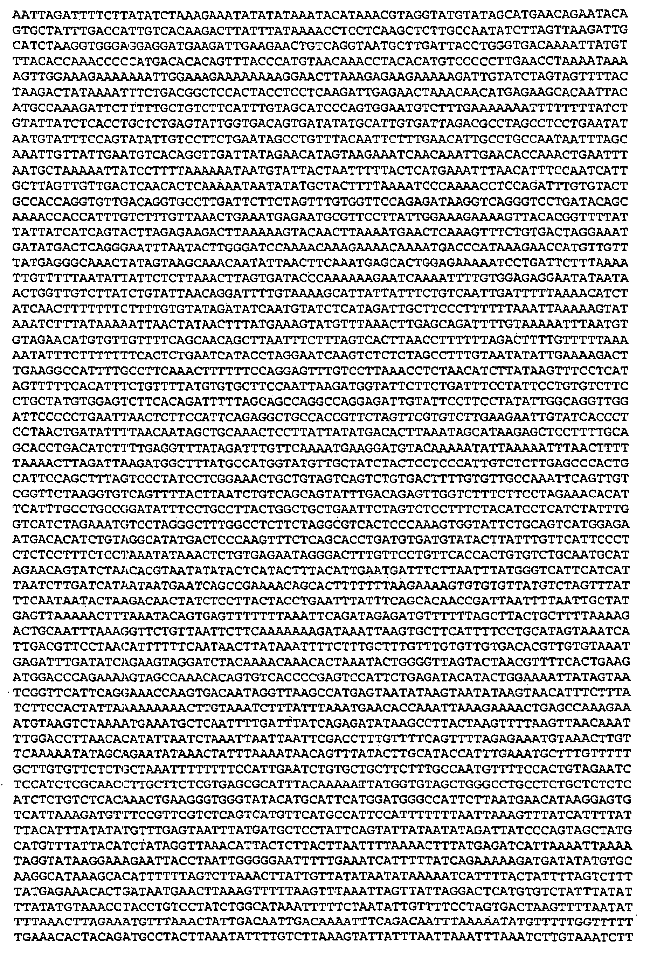

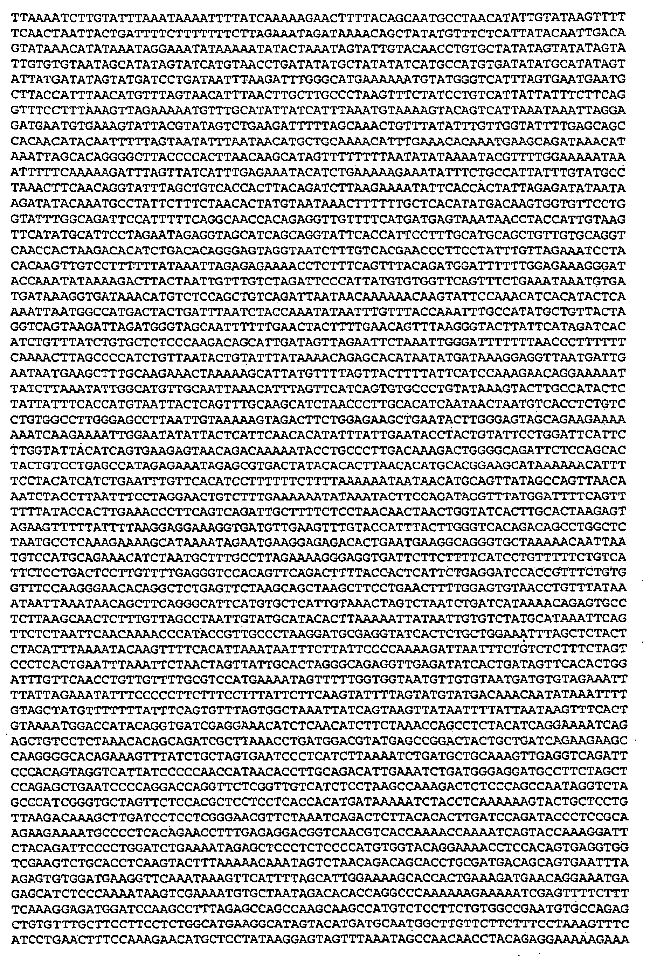

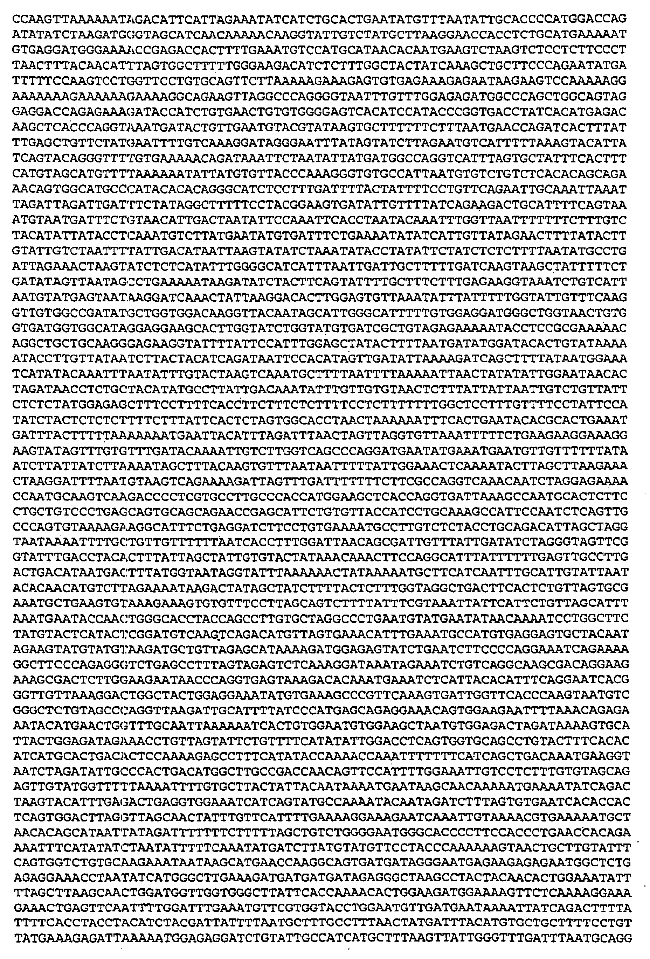

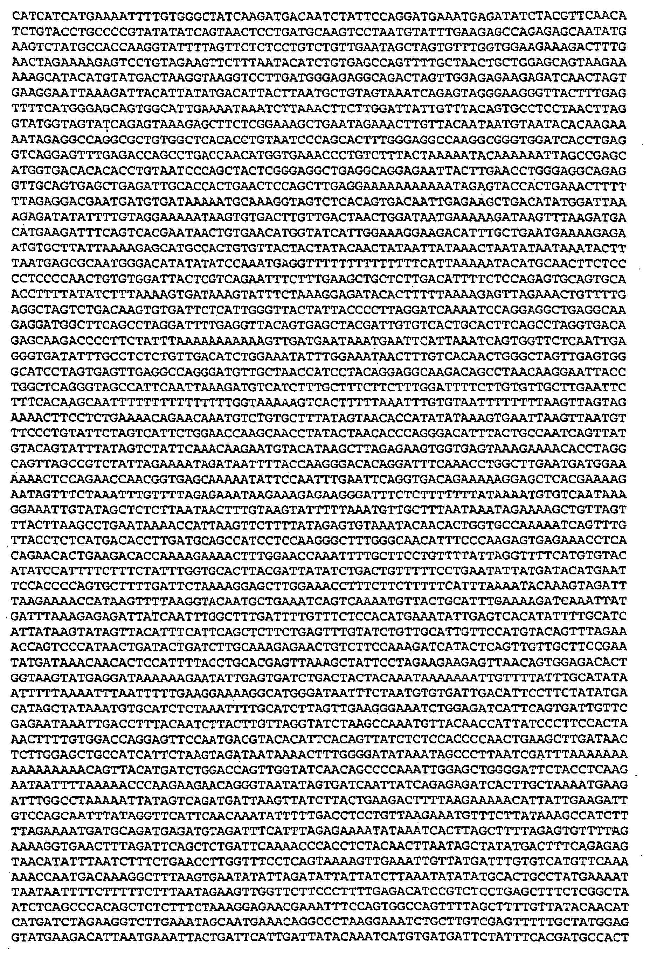

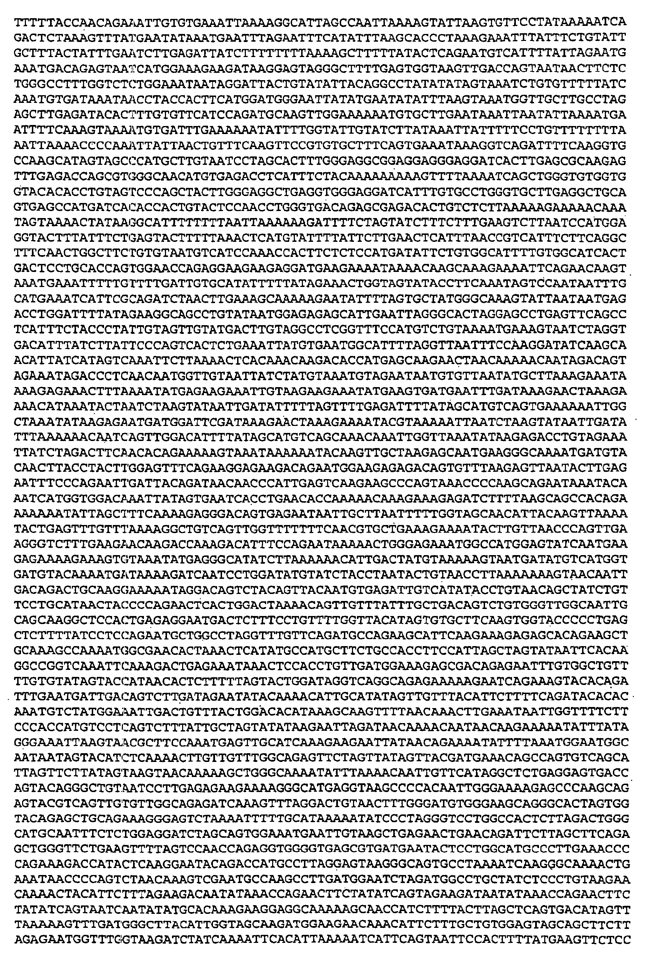

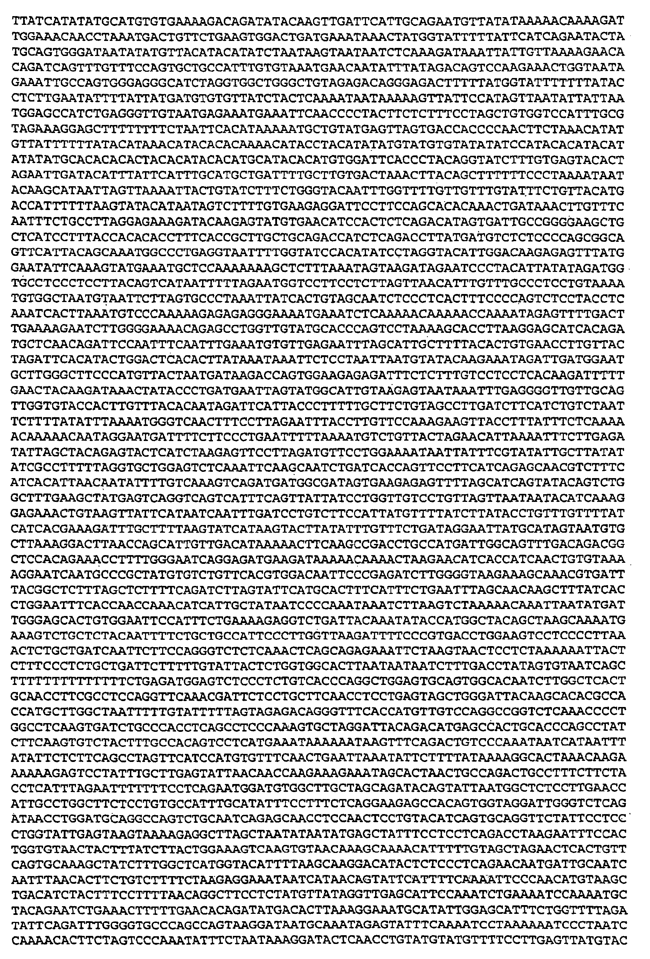

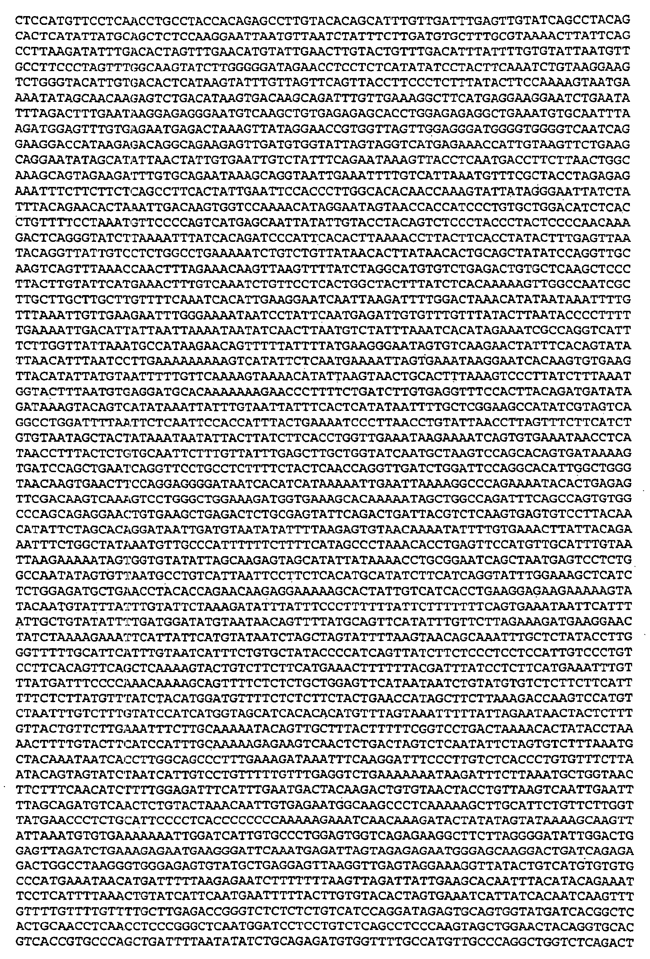

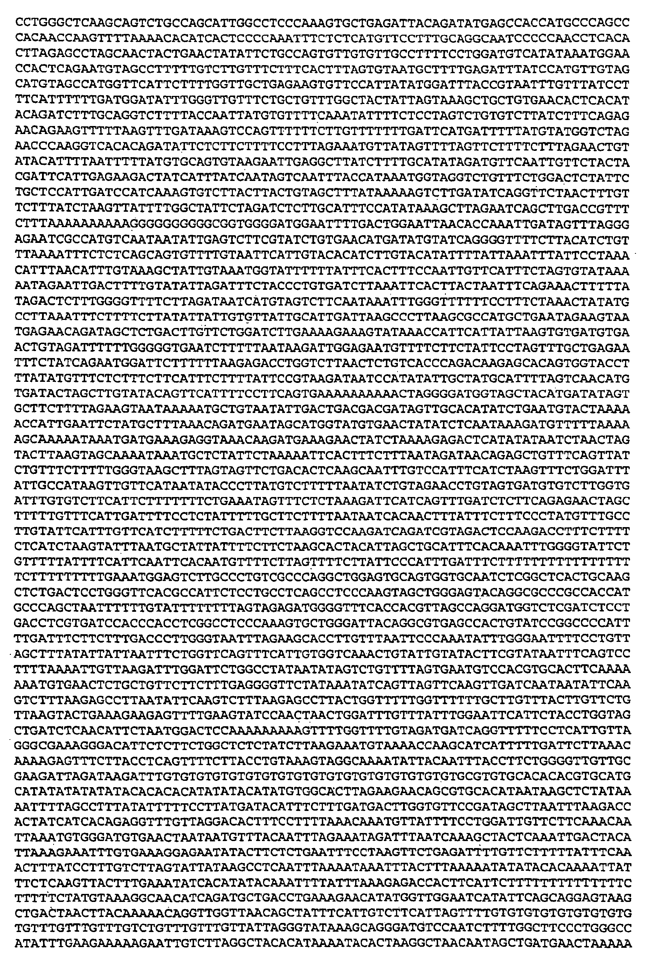

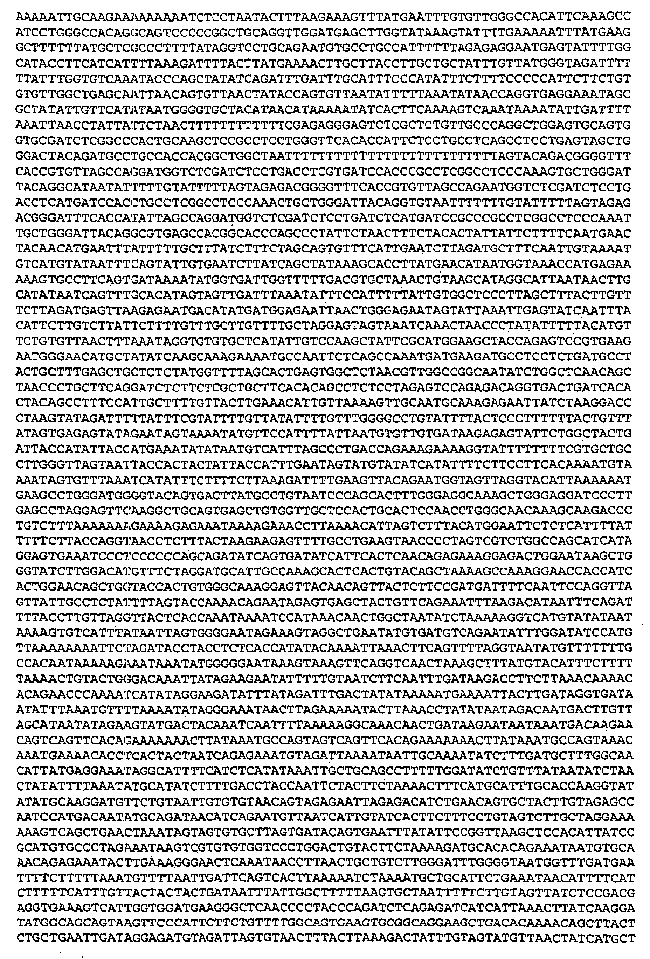

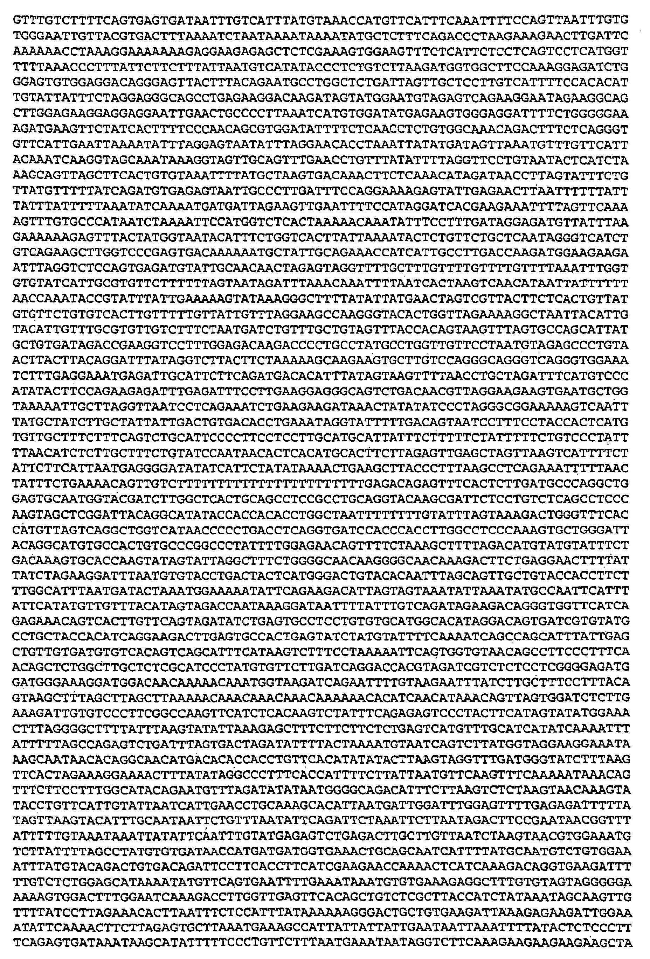

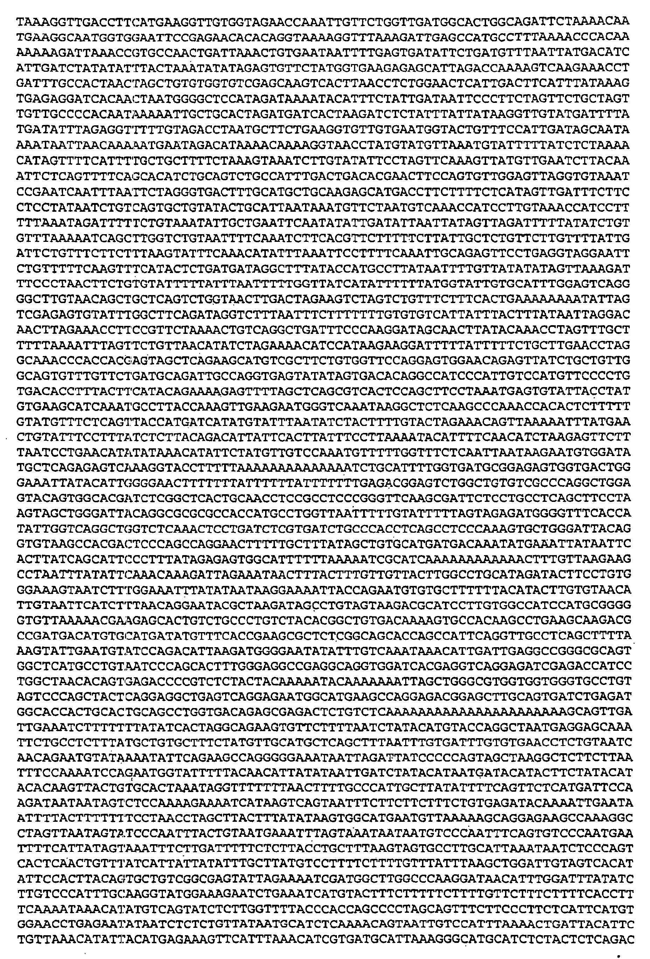

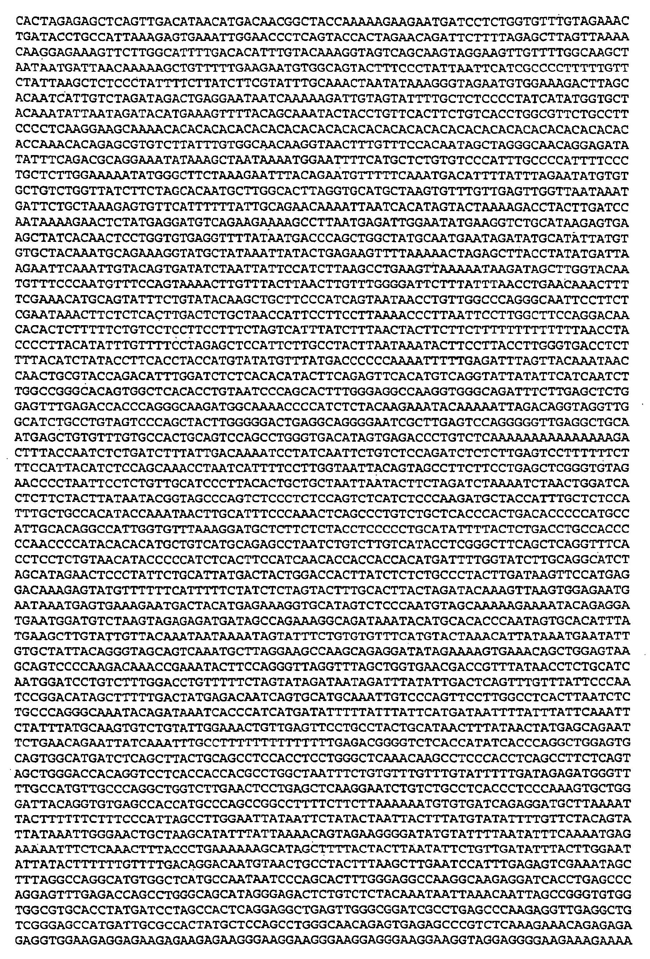

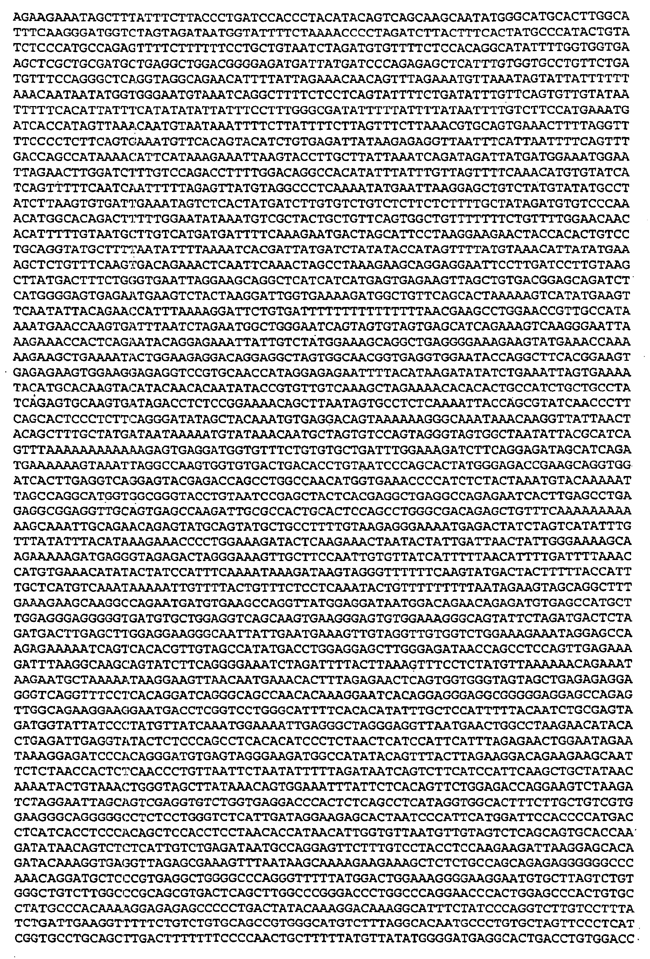

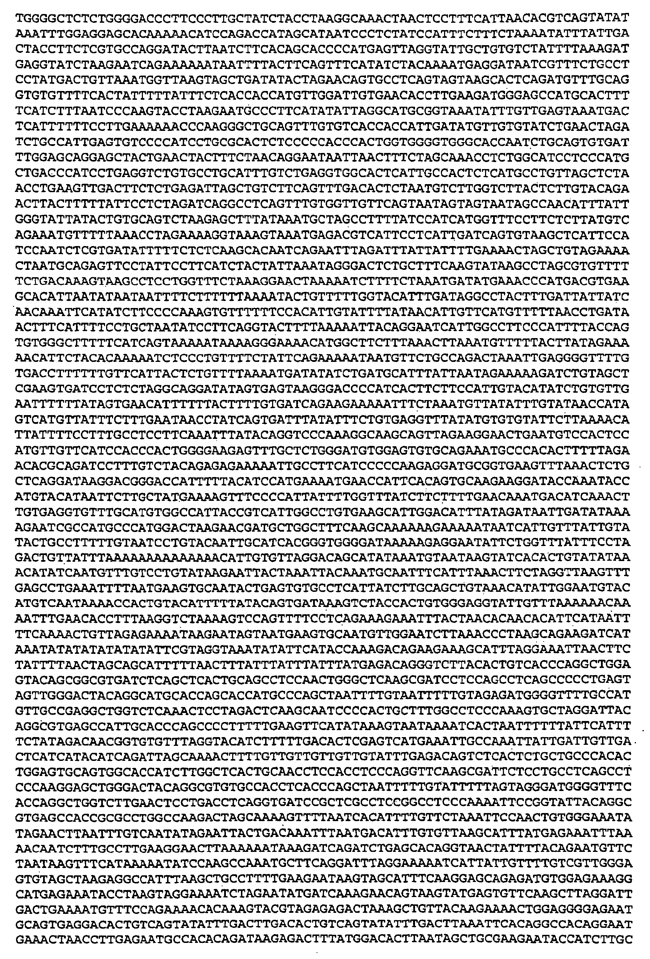

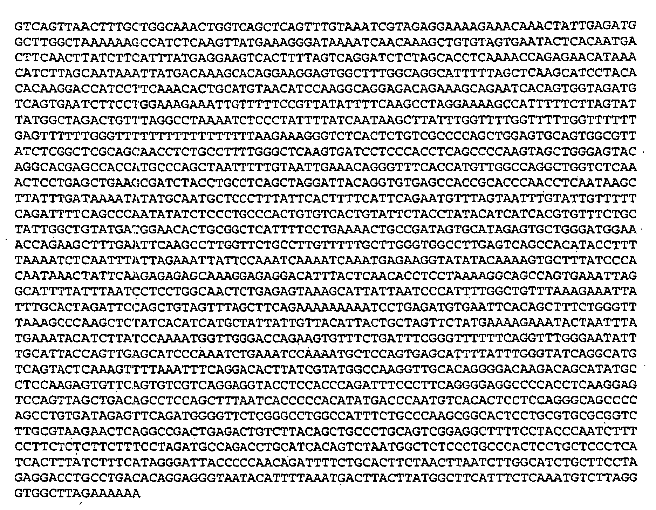

Figure 7: Protein, genomic and coding nucleotide sequence of human PAM according to NCBI accession numbers AAC39928 (protein sequence; SEQ ID No.1). AF075587 (coding sequence; SEQ ID No.2). Human PAM is located on Chromosome 13q22; its genomic sequence is publicly available under NT_024524.11 (Start: position 24679861; Stop: position 24962245; SEQ ID No 3);
   Figure 7C shows the contiguous sequence from position 24679861 to position 24962245.

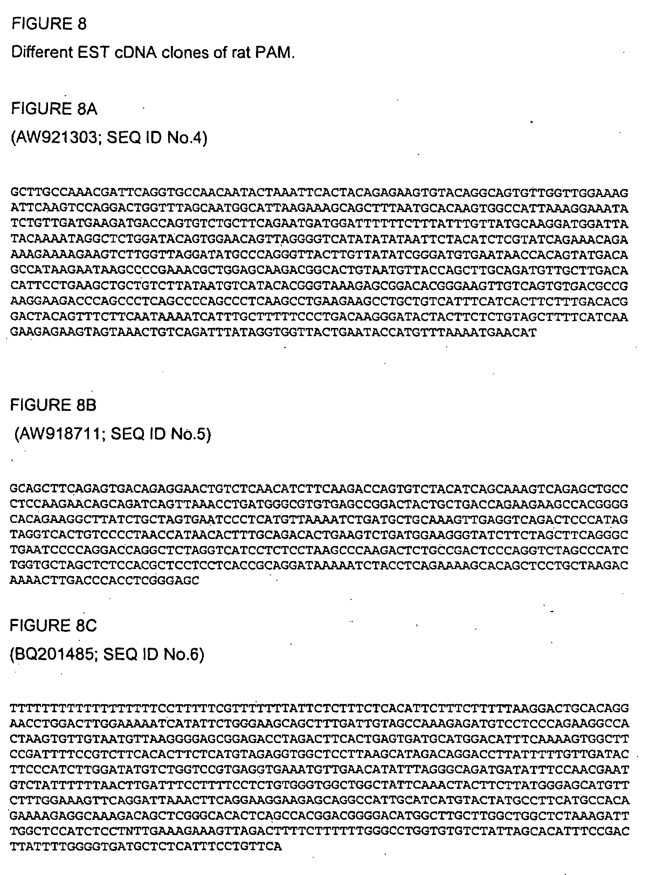

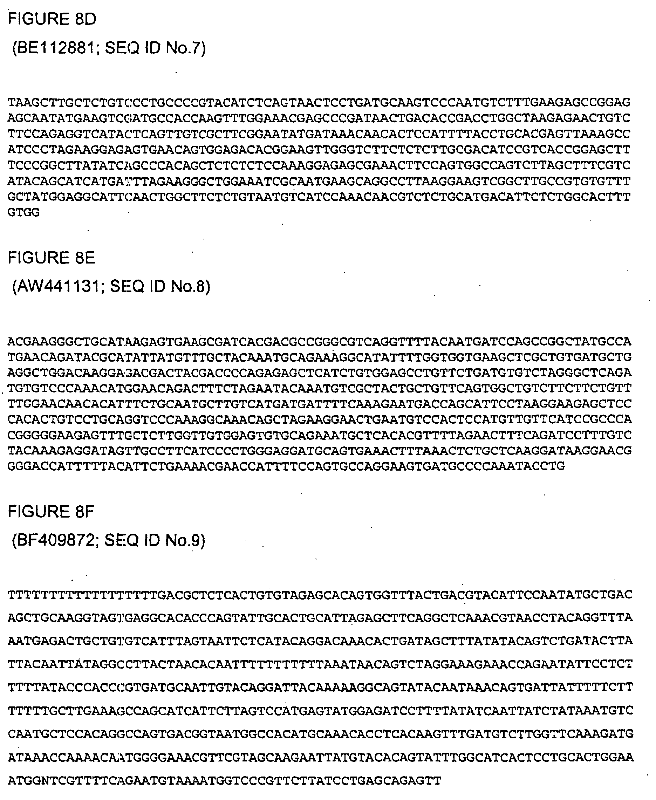

Figure 8: EST-clone coding sequences for rat PAM:
   Figure 8A: AW921303 (corresponds to bp 960-1394 of hs cDNA; SEQ ID No. 4)
   Figure 8B: AW918711 (corresponds to bp 8188-8632 of hs cDNA; SEQ ID No. 5)
   Figure 8C: BQ201485 (corresponds to bp 8966-9633 of hs cDNA; SEQ ID No. 6)
   Figure 8D: BE112881 (corresponds to bp 10311-10830 of hs cDNA; SEQ Id No. 7)
   Figure 8E: AW441131 (corresponds to bp 13569-14152 of hs cDNA; SEQ ID No. 8)
   Figure 8F: BF409872 (corresponds to bp 13569-14807 of hs cDNA). (SEQ ID No. 9)

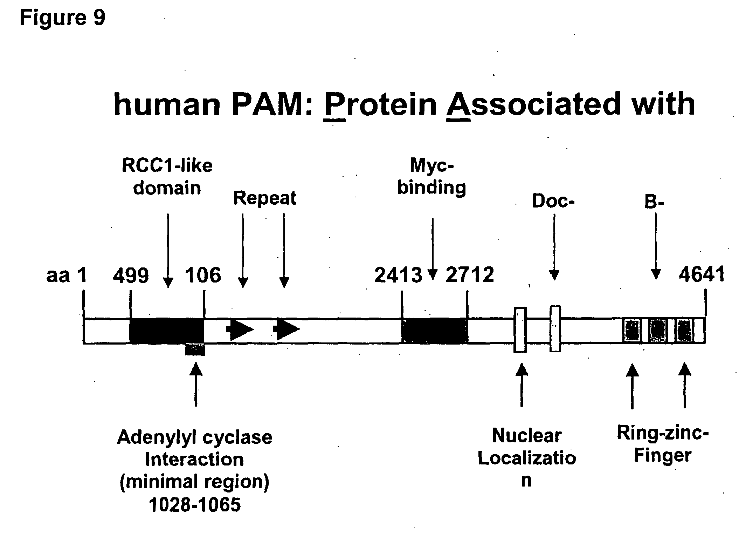

Figure 9: Overview of domain structure of human PAM according to Guo et al., 1988 / Grossberger et al., JBC 1999 and Scholich et al., JBC 2001)

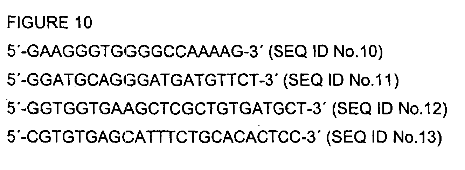

Figure 10: PCR Primers for rat PAM RT PCR.

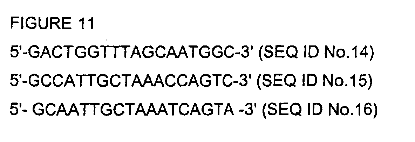

Figure 11: Antisense Oligodesoxynucleotides for inhibiting rat PAM expression and control oligonucleotide.

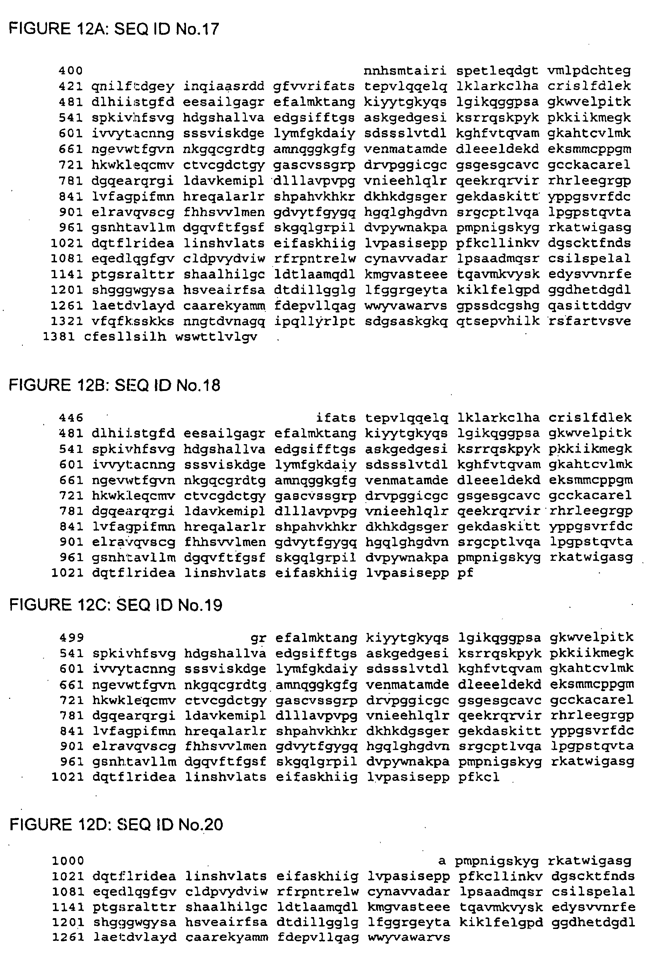

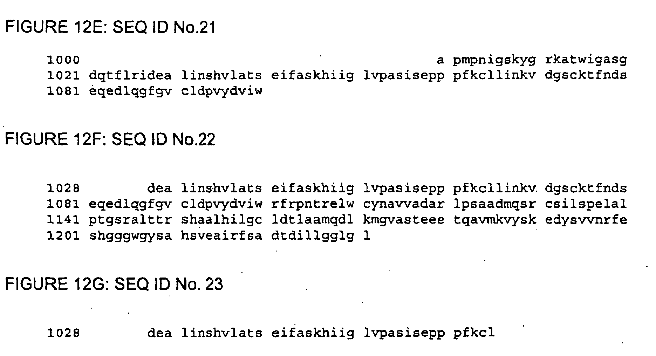

Figure 12: Different PAM hs polypeptides for the use in the context of present invention. The polypeptides are fragments derived from the polypeptide according to SEQ ID No.2.

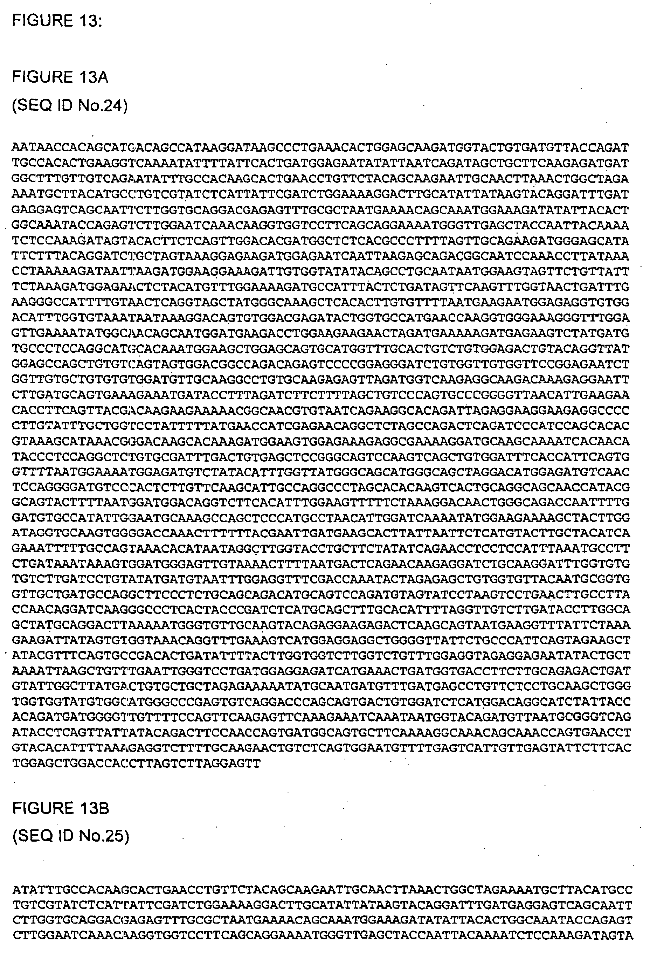

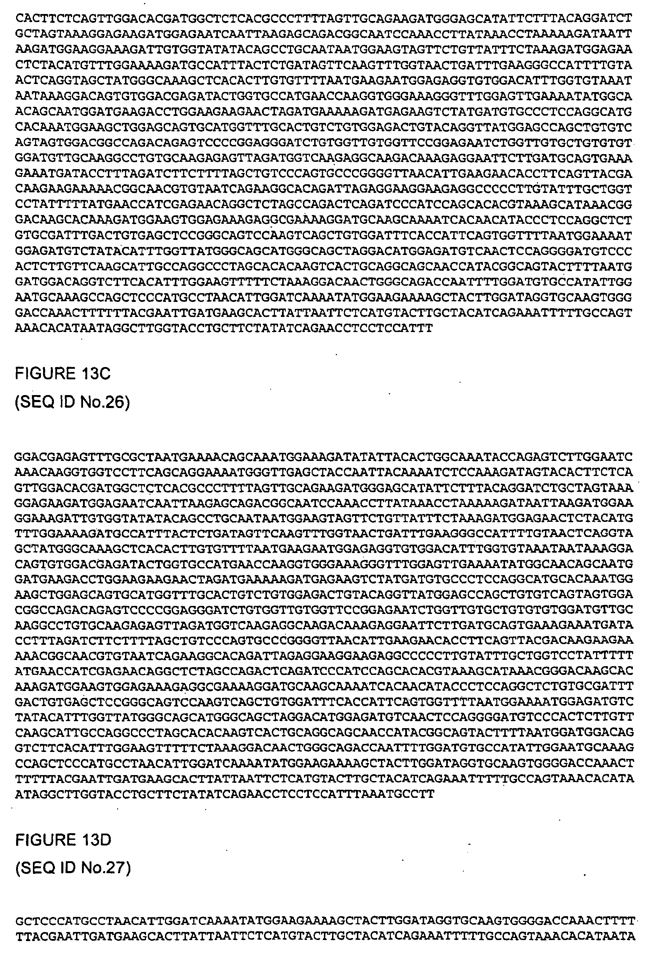

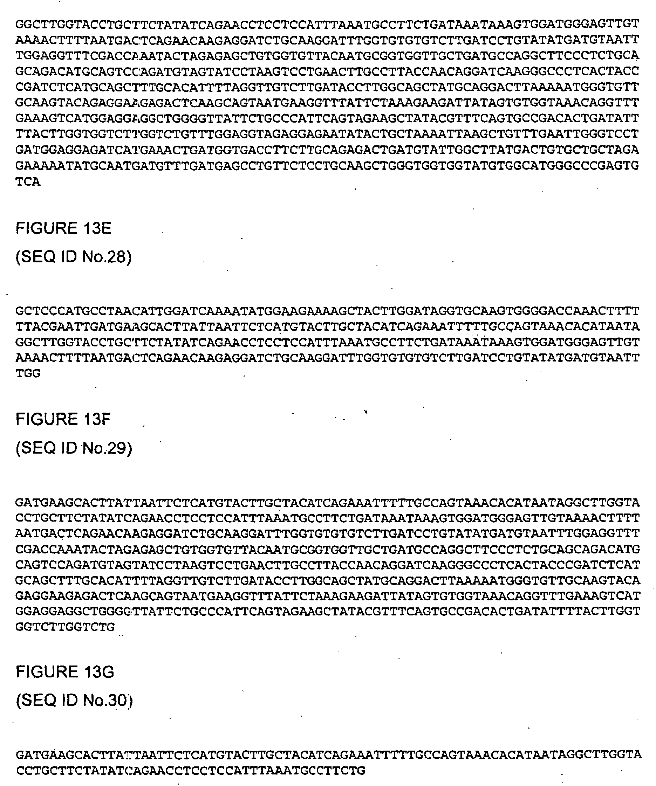

Figure 13: Different PAM hs polynucleotides for the use in the context of present invention.
   Figure 13A: cDNA sequence coding for the protein fragment according to SEQ ID No.17 comprising nucleotide positions 1317 to 4366 of hs Pam cDNA (SEQ ID No. 24);
   Figure 13B: cDNA sequence coding for the protein fragment according to SEQ ID No.18 comprising nucleotide positions 1482 to 3332 of hs Pam cDNA (SEQ ID No. 25);
   Figure 13C: cDNA sequence coding for the protein fragment according to SEQ ID No.19 comprising nucleotide positions 1641 to 3341 of hs Pam cDNA (SEQ ID No. 26);
   Figure 13D: cDNA sequence coding for the protein fragment according to SEQ ID No.20 comprising nucleotide positions 3142 to 4046 of hs Pam cDNA (SEQ ID No. 27);
   Figure 13E: cDNA sequence coding for the protein fragment according to SEQ ID No.21 comprising nucleotide positions 3142 to 3446 of hs Pam cDNA (SEQ ID No. 28);
   Figure 13F: cDNA sequence coding for the protein fragment according to SEQ ID No.22 comprising nucleotide positions 3228 to 3839 of hs Pam cDNA (SEQ ID No. 29);
   Figure 13G: cDNA sequence coding for the protein fragment according to SEQ ID No.23 comprising nucleotide positions 3228 to 3341 of hs Pam cDNA (SEQ ID No. 30);

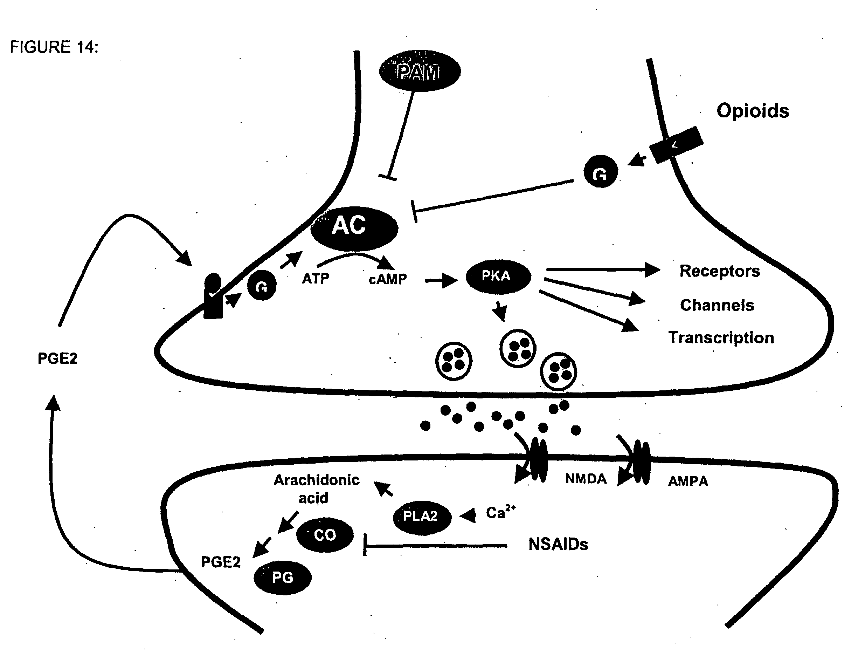

Figure 14: Overview of the postulated PAM signaling pathway in neurons of the spinal cord and the DRGs according to the above findings.

### Table 1

Basal AC activity is elevated in spinal cord lysates of antisense treated rats. Spinal cords lysates (20 µg) were assayed for AC activity in the absence or presence of 80 nM Gαs or 100 µM forskolin as described above. The mean AC activity ± SEM of spinal cord lysates from at least three rats per group, each measured twice in triplicates, is shown (ns = not significant).

**Table**

| Condition | AC activity (pmol/min/mg | |
|---|---|---|
| basal | | |
| sense | 102.7±6.6 | |
| antisense | 1 24.0±6.2 | (p≤0.01) |

| Gαs | | |
|---|---|---|
| sense | 398.8±16.9 | |
| antisense | 432.6±17.2 | (ns) |

| Forskolin | | |
|---|---|---|
| sense | 283.9±25.7 | |
| antisense | 316.2±24.3 | (ns) |

## Claims

1. The use of PAM or functional fragments or derivatives thereof for the preparation of pharmaceutical compounds.

2. The use of PAM or functional fragments or derivatives thereof for the modulation of pain.

3. The use of PAM or functional fragments of derivatives thereof for identifying compounds that modulate pain.

4. A method of lessening pain comprising administering a sufficient amount of PAM or a functional fragment or derivative thereof to an individual.

5. PAM or a functional fragment or derivative thereof for the use as a medicament.

6. PAM or a functional fragment or derivative thereof for the use as a medicament for the prevention or treatment of pain.

7. The use according to claim 1, wherein the pharmaceutical compounds are compounds for the prevention or treatment of pain.

8. The use according to claim 3, wherein the compounds lessen or abolish pain.

9. A method of screening pharmaceuticals useful for modulating and/or preventing pain, comprising the steps
f. Providing two samples
g. contacting one sample containing PAM or a functional fragment or derivative thereof with a compound,
h. determining the PAM activity in the presence of compound,
i. determining the PAM activity in the absence of compound, and
j. comparing the PAM activity according to c) with that according to d).

10. The use according to one of the claims 1,2,3 or 8 or the method according to one of the claims 4 or 9 or PAM according to one of the claims 5 or 6, wherein PAM is human PAM.

11. The use according to one of the claims 1,2,3 or 8 or the method according to one of the claims 4 or 6 or PAM according to one of the claims 5 or 6, wherein PAM is an isolated polypeptide or polynucleotide.

12. The use, method or PAM according to claim 11, wherein PAM is a polypeptide.

13. The use, method or PAM according to claim 11 or 12, wherein PAM is a polypeptide or functional fragment thereof that comprises or consists of the sequence according to SEQ ID No 2 or a fragment thereof or is encoded by a polynucleotide comprising or consisting of the sequence according to SEQ ID No 1 or a fragment thereof.

14. The use, method or PAM according to claim 11, wherein PAM is a polynucleotide.

15. The use, method or PAM according to claim 11, wherein PAM is an polynucleotide comprising or consisting of the sequence or a part of the sequence coding for a functional fragment of PAM according to SEQ ID No 1 or a polynucleotide comprising or consisting of a sequence that is able to hybridize with said polynucleotide under stringent conditions.

16. The use, method or PAM according to one of the above claims, wherein the functional fragments or derivatives thereof are capable of inhibiting adenylyl cyclase activity.

17. The use, method or PAM according to claim 16, wherein the adenylyl cyclase is adenylyl cyclase type I, V or VI.

18. The use, method or PAM according to one of the above claims, wherein the functional fragments comprise or consist of amino acids 400 to 1400, preferably 446 to 1062, 499 to 1065 or 1028 to 1231, more preferably 1000 to 1300 and even more preferably 1000 to 1100 and even more preferably 1028 to 1065 of human PAM, preferably of human PAM according to SEQ ID No.2.

19. The use, method or PAM according to claim 16, wherein the fragments comprise or consist of one of the polypeptides according to SEQ ID No. 17 to 23.

20. The method according to claim 9, wherein a cell expressing, preferably expressing recombinant PAM is used.

21. The method according to claim 9, wherein a modified cell, having a lower PAM activity as compared to its unmodified state, is used.

22. The method according to claim 21, wherein a PAM knock-out cell is used.

23. The method according to one of the claims 9, 20 or 21, wherein the PAM activity is determined directly.

24. The method according to claim 23, wherein the determination of PAM activity concerns its ability to interact with and preferably inactivate adenylate cyclase.

25. The method according to one of the claims 9, 20 or 21, wherein the PAM activity is determined indirectly.

26. A method of identifying a compound that modulates pain comprising
a. Selecting a compound that modulates the activity of PAM as a test compound, and
b. Administering said test compound to a subject to determine whether the pain is modulated.


SEQUENCE LISTING

<110>  Aventis Pharma Deutschland GmbH

<120>  Use of PAM

<130>  DEAV 2003/0048

<140>  03012388.9
<141>  2003-05-30

<160>  30

<170>  PatentIn version 3.2

<210>  1
<211>  14807
<212>  DNA
<213>  Homo sapiens

<400>  1
tcttggagcg ttctcagttt ctcaacagat cttcacttgc taggcagcca gaagccggcg      60

gcagtggcgg caccgcctcc tcctcacatt cccggggtgg cggggttaga tgagcggccc     120

cagtcgcggc gccgggggcg ctgttcatgc cggttcccga cggctccgtg gctgctgcgg     180

ggctggggct ggggctaccc gccgcggact ccccgggtca ctaccagctg ctgctgtcag     240

gccgggccct ggccgaccgc taccggagga tttataccgc tgcgctcaat gacagggacc     300

aggggggcgg cagcgctgga cacccagcct ccaggaataa gaaaatttta aataagaaga     360

aattgaaaag aaaacagaag agcaaatcaa aagtgaagac aagaagcaag tctgaaaact     420

tagagaatac agtaatcata ccagatatca aactacatag caatccttct gctttcaata     480

tttactgtaa tgtacgccat tgcgttctgg aatggcagaa aaaggaaata tcattggcag     540

ccgcatctaa gaactctgtg cagagtggag aatcagatag tgatgaagaa gaggaatcca     600

aagagccccc tatcaagctt ccaaagatta ttgaggttgg cctttgtgaa gtttttgaat     660

tgatcaaaga gacacgattt tctcatccat ccctgtgtct caggagtctc caagccctgc     720

tcaacgtgct gcagggccag cagccagaag tgctccagtc tgagccacct gaggtcctag     780

agtctctctt ccagcttctt ttggaaatca ccgttcgaag tactgggatg aatgacagca     840

caggacagtc cttaacagca ctttcctgtg cttgcctctt tagtctggtg gcttcttggg     900

gagaaacagg aaggacactt caggccatct ctgctatcct caccaacaat ggaagccatg     960

cttgccaaac tattcaggtg ccaacaattc taaattcgct acagagaagt gtacaagcag    1020

ttttggtggg aaaaattcaa attcaggact ggtttagtaa tggcattaag aaagcagctt    1080

taatgcacaa gtggccatta aaagaaatat ctgttgatga agatgaccaa tgtctacttc    1140

agaatgatgg attttttctt tatctattat gcaaggatgg attatataaa ataggctctg    1200

gatacagtgg aacagttagg ggccatatat acaattctac atcccgtatt agaaacagaa    1260

aagaaaaaaa gtcttggtta gggtatgctc agggttattt attatataga gatgtgaata    1320

accacagcat gacagccata aggataagcc ctgaaacact ggagcaagat ggtactgtga    1380

tgttaccaga ttgccacact gaaggtcaaa atattttatt cactgatgga gaatatatta    1440

atcagatagc tgcttcaaga gatgatggct ttgttgtcag aatatttgcc acaagcactg 1500
aacctgttct acagcaagaa ttgcaactta aactggctag aaaatgctta catgcctgtc 1560
gtatctcatt attcgatctg gaaaaggact tgcatattat aagtacagga tttgatgagg 1620
agtcagcaat tcttggtgca ggacgagagt ttgcgctaat gaaaacagca aatggaaaga 1680
tatattacac tggcaaatac cagagtcttg gaatcaaaca aggtggtcct tcagcaggaa 1740
aatgggttga gctaccaatt acaaaatctc caaagatagt acacttctca gttggacacg 1800
atggctctca cgccctttta gttgcagaag atgggagcat attctttaca ggatctgcta 1860
gtaaaggaga agatggagaa tcaattaaga gcagacggca atccaaacct tataaaccta 1920
aaaagataat taagatggaa ggaaagattg tggtatatac agcctgcaat aatggaagta 1980
gttctgttat ttctaaagat ggagaactct acatgtttgg aaaagatgcc atttactctg 2040
atagttcaag tttggtaact gatttgaagg gccattttgt aactcaggta gctatgggca 2100
aagctcacac ttgtgtttta atgaagaatg gagaggtgtg gacatttggt gtaaataata 2160
aaggacagtg tggacgagat actggtgcca tgaaccaagg tgggaaaggg tttggagttg 2220
aaaatatggc aacagcaatg gatgaagacc tggaagaaga actagatgaa aaagatgaga 2280
agtctatgat gtgccctcca ggcatgcaca aatggaagct ggagcagtgc atggtttgca 2340
ctgtctgtgg agactgtaca ggttatggag ccagctgtgt cagtagtgga cggccagaca 2400
gagtccccgg agggatctgt ggttgtggtt ccggagaatc tggttgtgct gtgtgtggat 2460
gttgcaaggc ctgtgcaaga gagttagatg gtcaagaggc aagacaaaga ggaattcttg 2520
atgcagtgaa agaaatgata cctttagatc ttcttttagc tgtcccagtg cccggggtta 2580
acattgaaga acaccttcag ttacgacaag aagaaaaacg gcaacgtgta atcagaaggc 2640
acagattaga ggaaggaaga ggcccccttg tatttgctgg tcctattttt atgaaccatc 2700
gagaacaggc tctagccaga ctcagatccc atccagcaca cgtaaagcat aaacgggaca 2760
agcacaaaga tggaagtgga gaaagaggcg aaaaggatgc aagcaaaatc acaacatacc 2820
ctccaggctc tgtgcgattt gactgtgagc tccgggcagt ccaagtcagc tgtggatttc 2880
accattcagt ggttttaatg gaaaatggag atgtctatac atttggttat gggcagcatg 2940
ggcagctagg acatggagat gtcaactcca ggggatgtcc cactcttgtt caagcattgc 3000
caggccctag cacacaagtc actgcaggca gcaaccatac ggcagtactt ttaatggatg 3060
gacaggtctt cacatttgga agtttttcta aaggacaact gggcagacca attttggatg 3120
tgccatattg gaatgcaaag ccagctccca tgcctaacat tggatcaaaa tatggaagaa 3180
aagctacttg gataggtgca agtggggacc aaactttttt acgaattgat gaagcactta 3240
ttaattctca tgtacttgct acatcagaaa tttttgccag taaacacata ataggcttgg 3300
tacctgcttc tatatcagaa cctcctccat ttaaatgcct tctgataaat aaagtggatg 3360
ggagttgtaa aacttttaat gactcagaac aagaggatct gcaaggattt ggtgtgtgtc 3420
ttgatcctgt atatgatgta atttggaggt ttcgaccaaa tactagagag ctgtggtgtt 3480

acaatgcggt ggttgctgat gccaggcttc cctctgcagc agacatgcag tccagatgta 3540
gtatcctaag tcctgaactt gccttaccaa caggatcaag ggccctcact acccgatctc 3600
atgcagcttt gcacatttta ggttgtcttg ataccttggc agctatgcag gacttaaaaa 3660
tgggtgttgc aagtacagag gaagagactc aagcagtaat gaaggtttat tctaaagaag 3720
attatagtgt ggtaaacagg tttgaaagtc atggaggagg ctggggttat tctgcccatt 3780
cagtagaagc tatacgtttc agtgccgaca ctgatatttt acttggtggt cttggtctgt 3840
ttggaggtag aggagaatat actgctaaaa ttaagctgtt tgaattgggt cctgatggag 3900
gagatcatga aactgatggt gaccttcttg cagagactga tgtattggct tatgactgtg 3960
ctgctagaga aaaatatgca atgatgtttg atgagcctgt tctcctgcaa gctgggtggt 4020
ggtatgtggc atgggcccga gtgtcaggac ccagcagtga ctgtggatct catggacagg 4080
catctattac cacagatgat ggggttgttt tccagttcaa gagttcaaag aaatcaaata 4140
atggtacaga tgttaatgcg ggtcagatac ctcagttatt atacagactt ccaaccagtg 4200
atggcagtgc ttcaaaaggc aaacagcaaa ccagtgaacc tgtacacatt ttaaagaggt 4260
cttttgcaag aactgtctca gtggaatgtt ttgagtcatt gttgagtatt cttcactgga 4320
gctggaccac cttagtctta ggagttgaag aacttagagg attaaaagga ttccagttca 4380
cagctacact cctagattta gagagactgc gctttgtggg tacctgttgt ctgaggttat 4440
tgcgtgtcta tacctgtgaa atttacccag tgtcagctac aggaaaagca gttgtagaag 4500
aaactagcaa attagcagag tgtattggaa aaaccagaac tttgttaaga aaaattttat 4560
cagaaccact tgatcactgc atggtgaaat tggataatga tcctcaagga tatctcagtc 4620
aacccttgag tcttctagaa gctgtccttc aggaatgtca taatactttc actgcctgct 4680
ttcattcttt ctacccaact cctgccttac agtgggcttg cctttgtgat ctgctgaatt 4740
gtttggatca ggatatccaa gaagcaaact tcaagacatc aagtagccga ctccttgcag 4800
ctgttatgtc agctctgtgt cacacgtctg ttaagctgac ttccatcttc ccgattgcgt 4860
atgatggaga agtattacta cgatcaattg ttaaacaagt tagtacagag aacgactcaa 4920
cactagttca tcgttttccc cttttggtgg cacatatgga aaaactcagc cagagtgaag 4980
agaatatctc agggatgaca agcttccgtg aagttctgga gaaaatgctg gtcattgttg 5040
tgctaccagt caggaacagc ctgaggagag aaaatgaact cttctcctcc cacctcgtct 5100
ctaacacctg tggattactg gccagcattg tcagtgaact gacagcgtca gccctgggat 5160
ctgaggttga tggacttaat tctcttcact ctgtaaaagc tagtgctaac cgatttacaa 5220
aaacaagtca gggcagaagt tggaacactg ggaacgggtc ccctgatgca atctgttttt 5280
cagtagacaa acctggaata gttgtggttg gtttctctgt ctatggagga ggtggaattc 5340
atgaatatga attagaggtg ttggttgatg atagtgaaca tgcaggagat tcaactcatt 5400
cccacagatg gacatctctg gaattagtga aaggaacgta cacaacggat gactcaccca 5460
gtgatatagc tgagatcaga cttgacaaag tggttccttt aaaggaaaat gttaaatatg 5520

ctgtgcgctt gaggaactat ggaagccgta cagccaatgg agatggagga atgaccacag 5580
ttcagtgccc tgatggtgtg acattcacat tcagcacgtg cagcttgagc agtaacggca 5640
caaaccaaac cagaggacag atcccacaga tactctacta taggagtgaa tttgatggag 5700
atttacaatc ccaacttctg agtaaagcca atgaagaaga taaaaactgt agcagagcat 5760
tgtctgttgt aagcactgtc gttcgagcct ctaaggacct cctgcacaga gctcttgctg 5820
tggatgctga tgacattcca gaactgctga gttcttccag tctgttttcc atgctgctcc 5880
cccttattat agcctacata ggaccagtag ctgctgctat tcccaaggtg gctgtagaag 5940
tctttggcct tgtccaacaa ttgcttccgt cagttgccat tttgaatcag aagtatgcac 6000
cgcctgcctt caaccctaat cagtcgacag atagcaccac aggaaaccag cctgaacagg 6060
gcctctctgc ttgtacaacc tccagtcact atgctgtcat agagagtgag cacccgtata 6120
aacctgcctg tgtgatgcat tacaaggtga cattcccaga atgtgtgagg tggatgacaa 6180
tcgaatttga ccctcagtgt ggtactgcac agtcagaaga tgtccttcgt ttgttgattc 6240
ctgtcagaac tgttcagaat tcaggatatg gaccaaaatt gacatctgtt catgaaaatc 6300
ttaattcatg gatagaatta aagaaatttt caggatcctc tgggtggcct actatggttt 6360
tggtgttgcc aggaaatgag gccctttttt cattggagac tgcatcagat tatgtgaaag 6420
atgacaaagc ttctttctat ggttttatgt gttttgcaat tggatatgaa tttagccctg 6480
gacctgatga gggagtcatc caattggaaa aagaattagc caatcttggt ggggtttgtg 6540
cagcagctct gatgaagaag gacctagcac ttcctattgg taatgaatta gaagaagacc 6600
ttgaaattct tgaggaggct gcattgcagg tgtgcaaaac ccattctgga attcttggaa 6660
agggtctagc tctttctcat tcaccaacta tattagaagc acttgaggga aatttaccac 6720
tccaaatcca aagcaatgaa cagtcttttc tggatgattt tattgcctgt gtcccaggat 6780
caagtggtgg aaggcttgca aggtggcttc agccagattc atatgcggat cctcagaaaa 6840
catctttgat cctgaataag gatgatattc gttgtggttg gcctaccacc ataactgttc 6900
aaacaaaaga ccagtatggg gatgtggtac atgttcccaa tatgaaggtg gaagtgaaag 6960
ctgtccctgt ttctcagaaa aaaatgtctt tacaacaaga tcaagcaaag aaacctcaaa 7020
ggattcctgg cagtcctgca gtaacagctg catcttctaa tactgacatg acttatggag 7080
ggctggcatc accaaagcta gatgtttcat atgaaccaat gatagtgaag gaagctcgat 7140
atattgccat aacaatgatg aaggtttatg aaaattattc atttgaagaa ctacgttttg 7200
catcaccaac tcctaagaga cccagtgaga atatgctgat ccgtgtcaat aatgatggga 7260
cttattgtgc aaattggact ccaggggcta ttggactcta cactcttcat gttaccattg 7320
atggcattga aatcgatgct ggtctggaag taaaagtaaa agacccacca aaagggatga 7380
taccaccagg aactcagttg gtcaaaccaa agtctgaacc tcagcctaat aaggttcgaa 7440
aatttgtggc caaggacagt gcggggcttc gcatccgtag ccacccttcc cttcagagtg 7500
agcagatagg catagtgaaa gtcaatggaa ctatcacttt tattgatgag atccataatg 7560

atgatggtgt gtggctgagg ctgaatgatg agacaataaa gaagtatgtc cctaacatga 7620
atggttacac tgaagcctgg tgcctctctt ttaatcaaca tcttggcaag agtcttctgg 7680
tccctgttga cgaatctaaa actaatactg atgacttttt caaagacata aactcctgct 7740
gcccacagga agcaacaatg caagaacaag atatgccatt cttgcgagga gggccaggca 7800
tgtacaaggt agtgaagacg ggaccttcag gtcacaacat cagaagctgc cctaacctta 7860
gaggtatccc aattggaatg ttagttctgg gaaacaaagt caaagcagtg ggagaggtaa 7920
ccaattctga agggacatgg gtgcaactgg atcagaacag catggtagag ttctgtgaga 7980
gtgatgaagg agaggcatgg tccttagcta gagacagagg cggaaaccag tacctccgac 8040
atgaagatga acaagctctt ctggatcaga attctcaaac tcctcctcca agccctttct 8100
cagtgcaagc ttttaataaa ggggcaagtt gcagtgccca aggatttgat tatggactcg 8160
gaaatagcaa aggtgatcga ggaaacatct caacatcttc taaaccagcc tctacatcag 8220
gaaaatcaga gctgtcctct aaacacagca gatcgcttaa acctgatgga cgtatgagcc 8280
ggactactgc tgatcagaag aagccaaggg gcacagaaag tttatctgct agtgaatccc 8340
tcatcttaaa atctgatgct gcaaagttga ggtcagattc ccacagtagg tcattatccc 8400
ccaaccataa caccttgcag acattgaaat ctgatgggag gatgccttct agctccagag 8460
ctgaatcccc aggaccaggt tctcggttgt catctcctaa gccaaagact ctcccagcca 8520
ataggtctag cccatcgggt gctagttctc cacgctcctc ctcaccacat gataaaaatc 8580
tacctcaaaa aagtactgct cctgttaaga caaagcttga tcctcctcgg gaacgttcta 8640
aatcagactc ttacacactt gatccagata ccctccgcaa gaagaaaatg cccctcacag 8700
aacctttgag aggacggtca acgtcaccaa aaccaaaatc agtaccaaag gattctacag 8760
attcccctgg atctgaaaat agagctccct ctccccatgt ggtacaggaa aacctccaca 8820
gtgaggtggt cgaagtctgc acctcaagta ctttaaaaac aaatagtcta acagacagca 8880
cctgcgatga cagcagtgaa tttaagagtg tggatgaagg ttcaaataaa gttcatttta 8940
gcattggaaa agcaccactg aaagatgaac aggaaatgag agcatctccc aaaataagtc 9000
gaaaatgtgc taatagacac accaggccca aaaaagaaaa atcgagtttt cttttcaaag 9060
gagatggatc caagccttta gagccagcca agcaagccat gtctccttct gtggccgaat 9120
gtgccagagc tgtgtttgct tccttcctct ggcatgaagg catagtacat gatgcaatgg 9180
cttgttcttc tttcctaaag tttcatcctg aactttccaa agaacatgct cctataagga 9240
gtagtttaaa tagccaacaa cctacagaag aaaaagaaac caagttaaaa aatagacatt 9300
cattagaaat atcatctgca ctgaatatgt ttaatattgc accccatgga ccagatatat 9360
ctaagatggg tagcatcaac aaaaacaagg tattgtctat gcttaaggaa ccacctctgc 9420
atgaaaaatg tgaggatggg aaaaccgaga ccacttttga aatgtccatg cataacacaa 9480
tgaagtctaa gtctcctctt cccttaactt tacaacattt agtggctttt tgggaagaca 9540
tctctttggc tactatcaaa gctgcttccc agaatatgat ttttccaagt cctggttcct 9600

gtgcagttct taaaaagaaa gagtgtgaga aaggaaggaa taagaagtcc aaaaaggaaa 9660
aaaagaaaaa agaaaaggca gaagttaggc ccaggggtaa tttgtttgga gagatggccc 9720
agctggcagt aggaggacca gagaaagata ccatctgtga actgtgtggg gagtcacatc 9780
catacccggt gacctatcac atgagacaag ctcacccagg ttgtggccga tatgctggtg 9840
gacaaggtta caatagcatt gggcattttt gtggaggatg ggctggtaac tgtggtgatg 9900
gtggcatagg aggaagcact tggtatctgg tatgtgatcg ctgtagagaa aaatacctcc 9960
gcgaaaaaca ggctgctgca agggagaagg tcaaacaatc taggagaaaa ccaatgcaag 10020
tcaagacccc tcgtgccttg cccaccatgg aagctcacca ggtgattaaa gccaatgcac 10080
tcttcctgct gtccctgagc agtgcagcag aaccgagcat tctgtgttac catcctgcaa 10140
agccattcca atctcagttg cccagtgtaa aagaaggcat ttctgaggat cttcctgtga 10200
aaatgccttg tctgtacctg cagacattag ctaggcatca tcatgaaaat tttgtgggct 10260
atcaagatga caatctattc caggatgaaa tgagatatct acgttcaaca tctgtacctg 10320
ccccgtatat atcagtaact cctgatgcaa gtcctaatgt atttgaagag ccagagagca 10380
atatgaagtc tatgccacca agtttagaaa ccagtcccat aactgatact gatcttgcaa 10440
agagaactgt cttccaaaga tcatactcag ttgttgcttc cgaatatgat aaacaacact 10500
ccattttacc tgcacgagtt aaagctattc ctagaagaag agttaacagt ggagacactg 10560
aagttggttc ttcccttttg agacatccgt ctcctgagct ttctcggcta atctcagccc 10620
acagctctct ttctaaagga gaacgaaatt tccagtggcc agttttagct tttgttatac 10680
aacatcatga tctagaaggt cttgaaatag caatgaaaca ggccctaagg aaatctgctt 10740
gtcgagtttt tgctatggag gctttcaact ggcttctgtg taatgtcatc caaaccactt 10800
ctctccatga tattctgtgg cattttgtgg catcactgac tcctgcacca gtggaaccag 10860
aggaagaaga ggatgaagaa aataaaacaa gcaaagaaaa ttcagaacaa gagaaagata 10920
caagagtatg tgaacatcca ctctcagaca tagtgattgc cggggaacgt gctcatcctt 10980
taccacacac ctttcaccgc ttgctgcaga ccatctcaga ccttatgatg tctctcccca 11040
gcggcagttc attacagcaa atggccctga ggtgctggag tctcaaattc aagcaatctg 11100
atcaccagtt ccttcatcag agcaacgtct ttcatcacat taacaatatt ttgtcaaagt 11160
cagatgatgg cgatagtgaa gagagtttta gcatcagtat acagtctggc tttgaagcta 11220
tgagtcagga attatgcata gtaatgtgct taaaggactt aaccagcatt gttgacataa 11280
aaacttcaag ccgacctgcc atgattggca gtttgacaga cggctccaca gaaacctttt 11340
gggaatcagg agatgaagat aaaaacaaaa ctaagaacat caccatcaac tgtgtaaaag 11400
gaatcaatgc ccgctatgtg tctgttcacg tggacaattc ccgagatctt gggaataaag 11460
ttacctcaat gaccttctta actggcaaag cagtagaaga tttgtgcaga ataaagcagg 11520
ttgatctgga ttccaggcac attggctggg taacaagtga acttccagga ggggataatc 11580
acatcataaa aattgaatta aaaggcccag aaaatacact gagagttcga caagtcaaag 11640

tcctgggctg gaaagatggt gaaagcacaa aaatagctgg ccagatttca gccagtgtgg 11700
cccagcagag gaactgtgaa gctgagactc tgcgagtatt cagactgatt acgtctcaag 11760
tatttggaaa gctcatctct ggagatgctg aacctacacc agaacaagag gaaaaagcac 11820
tattgtcatc acctgaagga gaagaaaaag tatacaatgc aacatcagat gctgacctga 11880
aagaacatat ggttggaatc atattcagca ggagtaagct gactaactta caaaaacagg 11940
tgtgtgctca tattgtccaa gctattcgca tggaagctac cagagtccgt gaagaatggg 12000
aacatgctat atcaagcaaa gaaaatgcca attctcagcc aaatgatgaa gatgcctcct 12060
ctgatgccta ctgctttgag ctgctctcta tggttttagc actgagtggc tctaacgttg 12120
gccggcaata tctggctcaa cagctaaccc tgcttcagga tctcttctcg ctgcttcaca 12180
cagcctctcc tagagtccag agacaggtaa cctctttact aagaagagtt ttgcctgaag 12240
taacccctag tcgtctggcc agcatcatag gagtgaaatc cctcccccca gcagatatca 12300
gtgatatcat tcactcaaca gagaaaggag actggaataa gctgggtatc ttggacatgt 12360
ttctaggatg cattgccaaa gcactcactg tacagctaaa agccaaagga accaccatca 12420
ctggaacagc tggtaccact gtgggcaaag gagttacaac agttactctt ccgatgattt 12480
tcaattccag ttatctccga cgaggtgaaa gtcattggtg gatgaagggc tcaaccccta 12540
cccagatctc agagatcatc attaaactta tcaaggatat ggcagcaggt catctgtcag 12600
aagcttggtc ccgagtgaca aaaaatgcta ttgcagaaac catcattgcc ttgaccaaga 12660
tggaagaaga atttaggtct ccagtgagat gtattgcaac aactagactc tggcttgctc 12720
tcgcatccct atgtgttctt gatcaggacc acgtagatcg tctctcctcg ggagatggga 12780
tgggaaagga tggacaacaa aaacaaatgc ctatgtgtga taaccatgat gatggtgaaa 12840
ctgcagcaat cattttatgc aatgtctgtg gaaatttatg tacagactgt gacagattcc 12900
ttcaccttca tcgaagaacc aaaactcatc aaagacaggt cttcaaagaa gaagaagaag 12960
ctataaaggt tgaccttcat gaaggttgtg gtagaaccaa attgttctgg ttgatggcac 13020
tggcagattc taaaacaatg aaggcaatgg tggaattccg agaacacaca ggcaaaccca 13080
ccacgagtag ctcagaagca tgtcgcttct gtggttccag gagtggaaca gagttatctg 13140
ctgttggcag tgtttgttct gatgcagatt gccaggaata cgctaagata gcctgtagta 13200
agacgcatcc ttgtggccat ccatgcgggg gtgttaaaaa cgaagagcac tgtctgccct 13260
gtctacacgg ctgtgacaaa agtgccacaa gcctgaagca agacgccgat gacatgtgca 13320
tgatatgttt caccgaagcg ctctcggcag caccagccat tcagctggat tgtagtcaca 13380
tattccactt acagtgctgt cggcgagtat tagaaaatcg atggcttggc ccaaggataa 13440
catttggatt tatatcttgt cccatttgca agaacaaaat taatcacata gtactaaaag 13500
acctacttga tccaataaaa gaactctatg aggatgtcag aagaaaagcc ttaatgagat 13560
tggaatatga aggtctgcat aagagtgaag ctatcacaac tcctggtgtg aggttttata 13620
atgacccagc tggctatgca atgaatagat atgcatatta tgtgtgctac aaatgcagaa 13680

aggcatattt tggtggtgaa gctcgctgcg atgctgaggc tggacgggga gatgattatg 13740
atcccagaga gctcatttgt ggtgcctgtt ctgatgtttc cagggctcag atgtgtccca 13800
aacatggcac agactttttg gaatataaat gtcgctactg ctgttcagtg gctgtttttt 13860
tctgttttgg aacaacacat ttttgtaatg cttgtcatga tgattttcaa agaatgacta 13920
gcattcctaa ggaagaacta ccacactgtc ctgcaggtcc caaaggcaag cagttagaag 13980
gaactgaatg tccactccat gttgttcatc cacccactgg ggaagagttt gctctgggat 14040
gtggagtgtg cagaaatgcc cacacttttt agaacacgca gatcctttgt ctacagagag 14100
aaaaattgcc ttcatccccc aagaggatgc ggtgaagttt aaactctgct caccataagg 14160
acgggaccat ttttacatcc atgaaaatga accattcaca gtgcaagaag gataccaaat 14220
accatgtaca taattcttgc tatgaaaagt ttccccatta ttttggttta tcttcttttg 14280
aacaaatgac atcaaacttg tgaggtgttt gcatgtggcc attaccgtca ttggcctgtg 14340
aagcattgga catttataga taattgatat aaaagaatcg ccatgcccat ggactaagaa 14400
cgatgctggc tttcaagcaa aaaagaaaaa taatcattgt ttattgtata ctgccttttt 14460
gtaatcctgt acaattgcat cacgggtggg gataaaaaga ggaatattct ggtttatttc 14520
ctagactgtt atttaaaaaa aaaaaaaaca ttgtgttagg acagcatata aatgtaataa 14580
gtatcacact gtatataaac atatcaatgt ttgtcctgta taagaattac taaattacaa 14640
atgcaatttc atttaaactt ctaggttaag tttgagcctg aaattttaat gaagtgcaat 14700
actgagtgtg cctcattatc ttgcagctgt aaacatattg gaatgtacat gtcaataaaa 14760
ccactgtaca tttttataca gtgataaagt ctaaaaaaaa aaaaaaa 14807

<210> 2
<211> 4641
<212> PRT
<213> Homo sapiens

<400> 2

Met Pro Val Pro Asp Gly Ser Val Ala Ala Ala Gly Leu Gly Leu Gly
1               5                   10                  15

Leu Pro Ala Ala Asp Ser Pro Gly His Tyr Gln Leu Leu Leu Ser Gly
            20                  25                  30

Arg Ala Leu Ala Asp Arg Tyr Arg Arg Ile Tyr Thr Ala Ala Leu Asn
        35                  40                  45

Asp Arg Asp Gln Gly Gly Gly Ser Ala Gly His Pro Ala Ser Arg Asn
    50                  55                  60

Lys Lys Ile Leu Asn Lys Lys Lys Leu Lys Arg Lys Gln Lys Ser Lys
65                  70                  75                  80

Ser Lys Val Lys Thr Arg Ser Lys Ser Glu Asn Leu Glu Asn Thr Val

                85                  90                  95

Ile Ile Pro Asp Ile Lys Leu His Ser Asn Pro Ser Ala Phe Asn Ile
            100                 105                 110

Tyr Cys Asn Val Arg His Cys Val Leu Glu Trp Gln Lys Lys Glu Ile
        115                 120                 125

Ser Leu Ala Ala Ala Ser Lys Asn Ser Val Gln Ser Gly Glu Ser Asp
    130                 135                 140

Ser Asp Glu Glu Glu Glu Ser Lys Glu Pro Pro Ile Lys Leu Pro Lys
145                 150                 155                 160

Ile Ile Glu Val Gly Leu Cys Glu Val Phe Glu Leu Ile Lys Glu Thr
                165                 170                 175

Arg Phe Ser His Pro Ser Leu Cys Leu Arg Ser Leu Gln Ala Leu Leu
            180                 185                 190

Asn Val Leu Gln Gly Gln Gln Pro Glu Val Leu Gln Ser Glu Pro Pro
        195                 200                 205

Glu Val Leu Glu Ser Leu Phe Gln Leu Leu Leu Glu Ile Thr Val Arg
    210                 215                 220

Ser Thr Gly Met Asn Asp Ser Thr Gly Gln Ser Leu Thr Ala Leu Ser
225                 230                 235                 240

Cys Ala Cys Leu Phe Ser Leu Val Ala Ser Trp Gly Glu Thr Gly Arg
                245                 250                 255

Thr Leu Gln Ala Ile Ser Ala Ile Leu Thr Asn Asn Gly Ser His Ala
            260                 265                 270

Cys Gln Thr Ile Gln Val Pro Thr Ile Leu Asn Ser Leu Gln Arg Ser
        275                 280                 285

Val Gln Ala Val Leu Val Gly Lys Ile Gln Ile Gln Asp Trp Phe Ser
    290                 295                 300

Asn Gly Ile Lys Lys Ala Ala Leu Met His Lys Trp Pro Leu Lys Glu
305                 310                 315                 320

Ile Ser Val Asp Glu Asp Asp Gln Cys Leu Leu Gln Asn Asp Gly Phe
                325                 330                 335

Phe Leu Tyr Leu Leu Cys Lys Asp Gly Leu Tyr Lys Ile Gly Ser Gly
            340                 345                 350

Tyr Ser Gly Thr Val Arg Gly His Ile Tyr Asn Ser Thr Ser Arg Ile

355                 360                 365

Arg Asn Arg Lys Glu Lys Lys Ser Trp Leu Gly Tyr Ala Gln Gly Tyr
    370                 375                 380

Leu Leu Tyr Arg Asp Val Asn Asn His Ser Met Thr Ala Ile Arg Ile
385                 390                 395                 400

Ser Pro Glu Thr Leu Glu Gln Asp Gly Thr Val Met Leu Pro Asp Cys
                405                 410                 415

His Thr Glu Gly Gln Asn Ile Leu Phe Thr Asp Gly Glu Tyr Ile Asn
            420                 425                 430

Gln Ile Ala Ala Ser Arg Asp Asp Gly Phe Val Val Arg Ile Phe Ala
        435                 440                 445

Thr Ser Thr Glu Pro Val Leu Gln Gln Glu Leu Gln Leu Lys Leu Ala
    450                 455                 460

Arg Lys Cys Leu His Ala Cys Arg Ile Ser Leu Phe Asp Leu Glu Lys
465                 470                 475                 480

Asp Leu His Ile Ile Ser Thr Gly Phe Asp Glu Glu Ser Ala Ile Leu
                485                 490                 495

Gly Ala Gly Arg Glu Phe Ala Leu Met Lys Thr Ala Asn Gly Lys Ile
            500                 505                 510

Tyr Tyr Thr Gly Lys Tyr Gln Ser Leu Gly Ile Lys Gln Gly Gly Pro
        515                 520                 525

Ser Ala Gly Lys Trp Val Glu Leu Pro Ile Thr Lys Ser Pro Lys Ile
    530                 535                 540

Val His Phe Ser Val Gly His Asp Gly Ser His Ala Leu Leu Val Ala
545                 550                 555                 560

Glu Asp Gly Ser Ile Phe Phe Thr Gly Ser Ala Ser Lys Gly Glu Asp
                565                 570                 575

Gly Glu Ser Ile Lys Ser Arg Arg Gln Ser Lys Pro Tyr Lys Pro Lys
            580                 585                 590

Lys Ile Ile Lys Met Glu Gly Lys Ile Val Val Tyr Thr Ala Cys Asn
        595                 600                 605

Asn Gly Ser Ser Ser Val Ile Ser Lys Asp Gly Glu Leu Tyr Met Phe
    610                 615                 620

Gly Lys Asp Ala Ile Tyr Ser Asp Ser Ser Ser Leu Val Thr Asp Leu

625                 630                 635                 640

Lys Gly His Phe Val Thr Gln Val Ala Met Gly Lys Ala His Thr Cys
                645                 650                 655

Val Leu Met Lys Asn Gly Glu Val Trp Thr Phe Gly Val Asn Asn Lys
            660                 665                 670

Gly Gln Cys Gly Arg Asp Thr Gly Ala Met Asn Gln Gly Gly Lys Gly
        675                 680                 685

Phe Gly Val Glu Asn Met Ala Thr Ala Met Asp Glu Asp Leu Glu Glu
    690                 695                 700

Glu Leu Asp Glu Lys Asp Glu Lys Ser Met Met Cys Pro Pro Gly Met
705                 710                 715                 720

His Lys Trp Lys Leu Glu Gln Cys Met Val Cys Thr Val Cys Gly Asp
                725                 730                 735

Cys Thr Gly Tyr Gly Ala Ser Cys Val Ser Ser Gly Arg Pro Asp Arg
            740                 745                 750

Val Pro Gly Gly Ile Cys Gly Cys Gly Ser Gly Glu Ser Gly Cys Ala
        755                 760                 765

Val Cys Gly Cys Cys Lys Ala Cys Ala Arg Glu Leu Asp Gly Gln Glu
    770                 775                 780

Ala Arg Gln Arg Gly Ile Leu Asp Ala Val Lys Glu Met Ile Pro Leu
785                 790                 795                 800

Asp Leu Leu Leu Ala Val Pro Val Pro Gly Val Asn Ile Glu Glu His
                805                 810                 815

Leu Gln Leu Arg Gln Glu Glu Lys Arg Gln Arg Val Ile Arg Arg His
            820                 825                 830

Arg Leu Glu Glu Gly Arg Gly Pro Leu Val Phe Ala Gly Pro Ile Phe
        835                 840                 845

Met Asn His Arg Glu Gln Ala Leu Ala Arg Leu Arg Ser His Pro Ala
    850                 855                 860

His Val Lys His Lys Arg Asp Lys His Lys Asp Gly Ser Gly Glu Arg
865                 870                 875                 880

Gly Glu Lys Asp Ala Ser Lys Ile Thr Thr Tyr Pro Pro Gly Ser Val
                885                 890                 895

Arg Phe Asp Cys Glu Leu Arg Ala Val Gln Val Ser Cys Gly Phe His

            900                 905                 910

His Ser Val Val Leu Met Glu Asn Gly Asp Val Tyr Thr Phe Gly Tyr
        915                 920                 925

Gly Gln His Gly Gln Leu Gly His Gly Asp Val Asn Ser Arg Gly Cys
    930                 935                 940

Pro Thr Leu Val Gln Ala Leu Pro Gly Pro Ser Thr Gln Val Thr Ala
945                 950                 955                 960

Gly Ser Asn His Thr Ala Val Leu Leu Met Asp Gly Gln Val Phe Thr
                965                 970                 975

Phe Gly Ser Phe Ser Lys Gly Gln Leu Gly Arg Pro Ile Leu Asp Val
            980                 985                 990

Pro Tyr Trp Asn Ala Lys Pro Ala Pro Met Pro Asn Ile Gly Ser Lys
        995                 1000                1005

Tyr Gly Arg Lys Ala Thr Trp Ile Gly Ala Ser Gly Asp Gln Thr
    1010                1015                1020

Phe Leu Arg Ile Asp Glu Ala Leu Ile Asn Ser His Val Leu Ala
    1025                1030                1035

Thr Ser Glu Ile Phe Ala Ser Lys His Ile Ile Gly Leu Val Pro
    1040                1045                1050

Ala Ser Ile Ser Glu Pro Pro Pro Phe Lys Cys Leu Leu Ile Asn
    1055                1060                1065

Lys Val Asp Gly Ser Cys Lys Thr Phe Asn Asp Ser Glu Gln Glu
    1070                1075                1080

Asp Leu Gln Gly Phe Gly Val Cys Leu Asp Pro Val Tyr Asp Val
    1085                1090                1095

Ile Trp Arg Phe Arg Pro Asn Thr Arg Glu Leu Trp Cys Tyr Asn
    1100                1105                1110

Ala Val Val Ala Asp Ala Arg Leu Pro Ser Ala Ala Asp Met Gln
    1115                1120                1125

Ser Arg Cys Ser Ile Leu Ser Pro Glu Leu Ala Leu Pro Thr Gly
    1130                1135                1140

Ser Arg Ala Leu Thr Thr Arg Ser His Ala Ala Leu His Ile Leu
    1145                1150                1155

Gly Cys Leu Asp Thr Leu Ala Ala Met Gln Asp Leu Lys Met Gly

1160                    1165                    1170

Val Ala  Ser Thr Glu Glu Glu  Thr Gln Ala Val Met  Lys Val Tyr
    1175                    1180                    1185

Ser Lys  Glu Asp Tyr Ser Val  Val Asn Arg Phe Glu  Ser His Gly
    1190                    1195                    1200

Gly Gly  Trp Gly Tyr Ser Ala  His Ser Val Glu Ala  Ile Arg Phe
    1205                    1210                    1215

Ser Ala  Asp Thr Asp Ile Leu  Leu Gly Gly Leu Gly  Leu Phe Gly
    1220                    1225                    1230

Gly Arg  Gly Glu Tyr Thr Ala  Lys Ile Lys Leu Phe  Glu Leu Gly
    1235                    1240                    1245

Pro Asp  Gly Gly Asp His Glu  Thr Asp Gly Asp Leu  Leu Ala Glu
    1250                    1255                    1260

Thr Asp  Val Leu Ala Tyr Asp  Cys Ala Ala Arg Glu  Lys Tyr Ala
    1265                    1270                    1275

Met Met  Phe Asp Glu Pro Val  Leu Leu Gln Ala Gly  Trp Trp Tyr
    1280                    1285                    1290

Val Ala  Trp Ala Arg Val Ser  Gly Pro Ser Ser Asp  Cys Gly Ser
    1295                    1300                    1305

His Gly  Gln Ala Ser Ile Thr  Thr Asp Asp Gly Val  Val Phe Gln
    1310                    1315                    1320

Phe Lys  Ser Ser Lys Lys Ser  Asn Asn Gly Thr Asp  Val Asn Ala
    1325                    1330                    1335

Gly Gln  Ile Pro Gln Leu Leu  Tyr Arg Leu Pro Thr  Ser Asp Gly
    1340                    1345                    1350

Ser Ala  Ser Lys Gly Lys Gln  Gln Thr Ser Glu Pro  Val His Ile
    1355                    1360                    1365

Leu Lys  Arg Ser Phe Ala Arg  Thr Val Ser Val Glu  Cys Phe Glu
    1370                    1375                    1380

Ser Leu  Leu Ser Ile Leu His  Trp Ser Trp Thr Thr  Leu Val Leu
    1385                    1390                    1395

Gly Val  Glu Glu Leu Arg Gly  Leu Lys Gly Phe Gln  Phe Thr Ala
    1400                    1405                    1410

Thr Leu  Leu Asp Leu Glu Arg  Leu Arg Phe Val Gly  Thr Cys Cys

1415                 1420                 1425

Leu Arg Leu Leu Arg Val Tyr Thr Cys Glu Ile Tyr Pro Val Ser
    1430                 1435                 1440

Ala Thr Gly Lys Ala Val Val Glu Glu Thr Ser Lys Leu Ala Glu
    1445                 1450                 1455

Cys Ile Gly Lys Thr Arg Thr Leu Leu Arg Lys Ile Leu Ser Glu
    1460                 1465                 1470

Pro Leu Asp His Cys Met Val Lys Leu Asp Asn Asp Pro Gln Gly
    1475                 1480                 1485

Tyr Leu Ser Gln Pro Leu Ser Leu Leu Glu Ala Val Leu Gln Glu
    1490                 1495                 1500

Cys His Asn Thr Phe Thr Ala Cys Phe His Ser Phe Tyr Pro Thr
    1505                 1510                 1515

Pro Ala Leu Gln Trp Ala Cys Leu Cys Asp Leu Leu Asn Cys Leu
    1520                 1525                 1530

Asp Gln Asp Ile Gln Glu Ala Asn Phe Lys Thr Ser Ser Ser Arg
    1535                 1540                 1545

Leu Leu Ala Ala Val Met Ser Ala Leu Cys His Thr Ser Val Lys
    1550                 1555                 1560

Leu Thr Ser Ile Phe Pro Ile Ala Tyr Asp Gly Glu Val Leu Leu
    1565                 1570                 1575

Arg Ser Ile Val Lys Gln Val Ser Thr Glu Asn Asp Ser Thr Leu
    1580                 1585                 1590

Val His Arg Phe Pro Leu Leu Val Ala His Met Glu Lys Leu Ser
    1595                 1600                 1605

Gln Ser Glu Glu Asn Ile Ser Gly Met Thr Ser Phe Arg Glu Val
    1610                 1615                 1620

Leu Glu Lys Met Leu Val Ile Val Val Leu Pro Val Arg Asn Ser
    1625                 1630                 1635

Leu Arg Arg Glu Asn Glu Leu Phe Ser Ser His Leu Val Ser Asn
    1640                 1645                 1650

Thr Cys Gly Leu Leu Ala Ser Ile Val Ser Glu Leu Thr Ala Ser
    1655                 1660                 1665

Ala Leu Gly Ser Glu Val Asp Gly Leu Asn Ser Leu His Ser Val

1670                    1675                    1680

Lys Ala Ser Ala Asn Arg Phe Thr Lys Thr Ser Gln Gly Arg Ser
    1685                    1690                    1695

Trp Asn Thr Gly Asn Gly Ser Pro Asp Ala Ile Cys Phe Ser Val
    1700                    1705                    1710

Asp Lys Pro Gly Ile Val Val Val Gly Phe Ser Val Tyr Gly Gly
    1715                    1720                    1725

Gly Gly Ile His Glu Tyr Glu Leu Glu Val Leu Val Asp Asp Ser
    1730                    1735                    1740

Glu His Ala Gly Asp Ser Thr His Ser His Arg Trp Thr Ser Leu
    1745                    1750                    1755

Glu Leu Val Lys Gly Thr Tyr Thr Thr Asp Asp Ser Pro Ser Asp
    1760                    1765                    1770

Ile Ala Glu Ile Arg Leu Asp Lys Val Val Pro Leu Lys Glu Asn
    1775                    1780                    1785

Val Lys Tyr Ala Val Arg Leu Arg Asn Tyr Gly Ser Arg Thr Ala
    1790                    1795                    1800

Asn Gly Asp Gly Gly Met Thr Thr Val Gln Cys Pro Asp Gly Val
    1805                    1810                    1815

Thr Phe Thr Phe Ser Thr Cys Ser Leu Ser Ser Asn Gly Thr Asn
    1820                    1825                    1830

Gln Thr Arg Gly Gln Ile Pro Gln Ile Leu Tyr Tyr Arg Ser Glu
    1835                    1840                    1845

Phe Asp Gly Asp Leu Gln Ser Gln Leu Leu Ser Lys Ala Asn Glu
    1850                    1855                    1860

Glu Asp Lys Asn Cys Ser Arg Ala Leu Ser Val Val Ser Thr Val
    1865                    1870                    1875

Val Arg Ala Ser Lys Asp Leu Leu His Arg Ala Leu Ala Val Asp
    1880                    1885                    1890

Ala Asp Asp Ile Pro Glu Leu Leu Ser Ser Ser Ser Leu Phe Ser
    1895                    1900                    1905

Met Leu Leu Pro Leu Ile Ile Ala Tyr Ile Gly Pro Val Ala Ala
    1910                    1915                    1920

Ala Ile Pro Lys Val Ala Val Glu Val Phe Gly Leu Val Gln Gln

1925                1930                1935

Leu Leu Pro Ser Val Ala Ile Leu Asn Gln Lys Tyr Ala Pro Pro
    1940                1945                1950

Ala Phe Asn Pro Asn Gln Ser Thr Asp Ser Thr Thr Gly Asn Gln
    1955                1960                1965

Pro Glu Gln Gly Leu Ser Ala Cys Thr Thr Ser Ser His Tyr Ala
    1970                1975                1980

Val Ile Glu Ser Glu His Pro Tyr Lys Pro Ala Cys Val Met His
    1985                1990                1995

Tyr Lys Val Thr Phe Pro Glu Cys Val Arg Trp Met Thr Ile Glu
    2000                2005                2010

Phe Asp Pro Gln Cys Gly Thr Ala Gln Ser Glu Asp Val Leu Arg
    2015                2020                2025

Leu Leu Ile Pro Val Arg Thr Val Gln Asn Ser Gly Tyr Gly Pro
    2030                2035                2040

Lys Leu Thr Ser Val His Glu Asn Leu Asn Ser Trp Ile Glu Leu
    2045                2050                2055

Lys Lys Phe Ser Gly Ser Ser Gly Trp Pro Thr Met Val Leu Val
    2060                2065                2070

Leu Pro Gly Asn Glu Ala Leu Phe Ser Leu Glu Thr Ala Ser Asp
    2075                2080                2085

Tyr Val Lys Asp Asp Lys Ala Ser Phe Tyr Gly Phe Met Cys Phe
    2090                2095                2100

Ala Ile Gly Tyr Glu Phe Ser Pro Gly Pro Asp Glu Gly Val Ile
    2105                2110                2115

Gln Leu Glu Lys Glu Leu Ala Asn Leu Gly Gly Val Cys Ala Ala
    2120                2125                2130

Ala Leu Met Lys Lys Asp Leu Ala Leu Pro Ile Gly Asn Glu Leu
    2135                2140                2145

Glu Glu Asp Leu Glu Ile Leu Glu Glu Ala Ala Leu Gln Val Cys
    2150                2155                2160

Lys Thr His Ser Gly Ile Leu Gly Lys Gly Leu Ala Leu Ser His
    2165                2170                2175

Ser Pro Thr Ile Leu Glu Ala Leu Glu Gly Asn Leu Pro Leu Gln

2180                    2185                    2190

Ile Gln  Ser Asn Glu Gln Ser  Phe Leu Asp Asp Phe  Ile Ala Cys
    2195                    2200                    2205

Val Pro  Gly Ser Ser Gly Gly  Arg Leu Ala Arg Trp  Leu Gln Pro
    2210                    2215                    2220

Asp Ser  Tyr Ala Asp Pro Gln  Lys Thr Ser Leu Ile  Leu Asn Lys
    2225                    2230                    2235

Asp Asp  Ile Arg Cys Gly Trp  Pro Thr Thr Ile Thr  Val Gln Thr
    2240                    2245                    2250

Lys Asp  Gln Tyr Gly Asp Val  Val His Val Pro Asn  Met Lys Val
    2255                    2260                    2265

Glu Val  Lys Ala Val Pro Val  Ser Gln Lys Lys Met  Ser Leu Gln
    2270                    2275                    2280

Gln Asp  Gln Ala Lys Lys Pro  Gln Arg Ile Pro Gly  Ser Pro Ala
    2285                    2290                    2295

Val Thr  Ala Ala Ser Ser Asn  Thr Asp Met Thr Tyr  Gly Gly Leu
    2300                    2305                    2310

Ala Ser  Pro Lys Leu Asp Val  Ser Tyr Glu Pro Met  Ile Val Lys
    2315                    2320                    2325

Glu Ala  Arg Tyr Ile Ala Ile  Thr Met Met Lys Val  Tyr Glu Asn
    2330                    2335                    2340

Tyr Ser  Phe Glu Glu Leu Arg  Phe Ala Ser Pro Thr  Pro Lys Arg
    2345                    2350                    2355

Pro Ser  Glu Asn Met Leu Ile  Arg Val Asn Asn Asp  Gly Thr Tyr
    2360                    2365                    2370

Cys Ala  Asn Trp Thr Pro Gly  Ala Ile Gly Leu Tyr  Thr Leu His
    2375                    2380                    2385

Val Thr  Ile Asp Gly Ile Glu  Ile Asp Ala Gly Leu  Glu Val Lys
    2390                    2395                    2400

Val Lys  Asp Pro Pro Lys Gly  Met Ile Pro Pro Gly  Thr Gln Leu
    2405                    2410                    2415

Val Lys  Pro Lys Ser Glu Pro  Gln Pro Asn Lys Val  Arg Lys Phe
    2420                    2425                    2430

Val Ala  Lys Asp Ser Ala Gly  Leu Arg Ile Arg Ser  His Pro Ser

2435                2440                2445

Leu Gln Ser Glu Gln Ile Gly Ile Val Lys Val Asn Gly Thr Ile
    2450                2455                2460

Thr Phe Ile Asp Glu Ile His Asn Asp Asp Gly Val Trp Leu Arg
    2465                2470                2475

Leu Asn Asp Glu Thr Ile Lys Lys Tyr Val Pro Asn Met Asn Gly
    2480                2485                2490

Tyr Thr Glu Ala Trp Cys Leu Ser Phe Asn Gln His Leu Gly Lys
    2495                2500                2505

Ser Leu Leu Val Pro Val Asp Glu Ser Lys Thr Asn Thr Asp Asp
    2510                2515                2520

Phe Phe Lys Asp Ile Asn Ser Cys Cys Pro Gln Glu Ala Thr Met
    2525                2530                2535

Gln Glu Gln Asp Met Pro Phe Leu Arg Gly Gly Pro Gly Met Tyr
    2540                2545                2550

Lys Val Val Lys Thr Gly Pro Ser Gly His Asn Ile Arg Ser Cys
    2555                2560                2565

Pro Asn Leu Arg Gly Ile Pro Ile Gly Met Leu Val Leu Gly Asn
    2570                2575                2580

Lys Val Lys Ala Val Gly Glu Val Thr Asn Ser Glu Gly Thr Trp
    2585                2590                2595

Val Gln Leu Asp Gln Asn Ser Met Val Glu Phe Cys Glu Ser Asp
    2600                2605                2610

Glu Gly Glu Ala Trp Ser Leu Ala Arg Asp Arg Gly Gly Asn Gln
    2615                2620                2625

Tyr Leu Arg His Glu Asp Glu Gln Ala Leu Leu Asp Gln Asn Ser
    2630                2635                2640

Gln Thr Pro Pro Pro Ser Pro Phe Ser Val Gln Ala Phe Asn Lys
    2645                2650                2655

Gly Ala Ser Cys Ser Ala Gln Gly Phe Asp Tyr Gly Leu Gly Asn
    2660                2665                2670

Ser Lys Gly Asp Arg Gly Asn Ile Ser Thr Ser Ser Lys Pro Ala
    2675                2680                2685

Ser Thr Ser Gly Lys Ser Glu Leu Ser Ser Lys His Ser Arg Ser

2690                    2695                    2700

Leu Lys Pro Asp Gly Arg Met Ser Arg Thr Thr Ala Asp Gln Lys
    2705                    2710                    2715

Lys Pro Arg Gly Thr Glu Ser Leu Ser Ala Ser Glu Ser Leu Ile
    2720                    2725                    2730

Leu Lys Ser Asp Ala Ala Lys Leu Arg Ser Asp Ser His Ser Arg
    2735                    2740                    2745

Ser Leu Ser Pro Asn His Asn Thr Leu Gln Thr Leu Lys Ser Asp
    2750                    2755                    2760

Gly Arg Met Pro Ser Ser Ser Arg Ala Glu Ser Pro Gly Pro Gly
    2765                    2770                    2775

Ser Arg Leu Ser Ser Pro Lys Pro Lys Thr Leu Pro Ala Asn Arg
    2780                    2785                    2790

Ser Ser Pro Ser Gly Ala Ser Ser Pro Arg Ser Ser Ser Pro His
    2795                    2800                    2805

Asp Lys Asn Leu Pro Gln Lys Ser Thr Ala Pro Val Lys Thr Lys
    2810                    2815                    2820

Leu Asp Pro Pro Arg Glu Arg Ser Lys Ser Asp Ser Tyr Thr Leu
    2825                    2830                    2835

Asp Pro Asp Thr Leu Arg Lys Lys Lys Met Pro Leu Thr Glu Pro
    2840                    2845                    2850

Leu Arg Gly Arg Ser Thr Ser Pro Lys Pro Lys Ser Val Pro Lys
    2855                    2860                    2865

Asp Ser Thr Asp Ser Pro Gly Ser Glu Asn Arg Ala Pro Ser Pro
    2870                    2875                    2880

His Val Val Gln Glu Asn Leu His Ser Glu Val Val Glu Val Cys
    2885                    2890                    2895

Thr Ser Ser Thr Leu Lys Thr Asn Ser Leu Thr Asp Ser Thr Cys
    2900                    2905                    2910

Asp Asp Ser Ser Glu Phe Lys Ser Val Asp Glu Gly Ser Asn Lys
    2915                    2920                    2925

Val His Phe Ser Ile Gly Lys Ala Pro Leu Lys Asp Glu Gln Glu
    2930                    2935                    2940

Met Arg Ala Ser Pro Lys Ile Ser Arg Lys Cys Ala Asn Arg His

2945                2950                2955

Thr Arg Pro Lys Lys Glu Lys Ser Ser Phe Leu Phe Lys Gly Asp
    2960                2965                2970

Gly Ser Lys Pro Leu Glu Pro Ala Lys Gln Ala Met Ser Pro Ser
    2975                2980                2985

Val Ala Glu Cys Ala Arg Ala Val Phe Ala Ser Phe Leu Trp His
    2990                2995                3000

Glu Gly Ile Val His Asp Ala Met Ala Cys Ser Ser Phe Leu Lys
    3005                3010                3015

Phe His Pro Glu Leu Ser Lys Glu His Ala Pro Ile Arg Ser Ser
    3020                3025                3030

Leu Asn Ser Gln Gln Pro Thr Glu Glu Lys Glu Thr Lys Leu Lys
    3035                3040                3045

Asn Arg His Ser Leu Glu Ile Ser Ser Ala Leu Asn Met Phe Asn
    3050                3055                3060

Ile Ala Pro His Gly Pro Asp Ile Ser Lys Met Gly Ser Ile Asn
    3065                3070                3075

Lys Asn Lys Val Leu Ser Met Leu Lys Glu Pro Pro Leu His Glu
    3080                3085                3090

Lys Cys Glu Asp Gly Lys Thr Glu Thr Thr Phe Glu Met Ser Met
    3095                3100                3105

His Asn Thr Met Lys Ser Lys Ser Pro Leu Pro Leu Thr Leu Gln
    3110                3115                3120

His Leu Val Ala Phe Trp Glu Asp Ile Ser Leu Ala Thr Ile Lys
    3125                3130                3135

Ala Ala Ser Gln Asn Met Ile Phe Pro Ser Pro Gly Ser Cys Ala
    3140                3145                3150

Val Leu Lys Lys Lys Glu Cys Glu Lys Gly Arg Asn Lys Lys Ser
    3155                3160                3165

Lys Lys Glu Lys Lys Lys Lys Glu Lys Ala Glu Val Arg Pro Arg
    3170                3175                3180

Gly Asn Leu Phe Gly Glu Met Ala Gln Leu Ala Val Gly Gly Pro
    3185                3190                3195

Glu Lys Asp Thr Ile Cys Glu Leu Cys Gly Glu Ser His Pro Tyr

3200                 3205                 3210

Pro Val Thr Tyr His Met Arg Gln Ala His Pro Gly Cys Gly Arg
    3215                3220                3225

Tyr Ala Gly Gly Gln Gly Tyr Asn Ser Ile Gly His Phe Cys Gly
    3230                3235                3240

Gly Trp Ala Gly Asn Cys Gly Asp Gly Gly Ile Gly Gly Ser Thr
    3245                3250                3255

Trp Tyr Leu Val Cys Asp Arg Cys Arg Glu Lys Tyr Leu Arg Glu
    3260                3265                3270

Lys Gln Ala Ala Ala Arg Glu Lys Val Lys Gln Ser Arg Arg Lys
    3275                3280                3285

Pro Met Gln Val Lys Thr Pro Arg Ala Leu Pro Thr Met Glu Ala
    3290                3295                3300

His Gln Val Ile Lys Ala Asn Ala Leu Phe Leu Leu Ser Leu Ser
    3305                3310                3315

Ser Ala Ala Glu Pro Ser Ile Leu Cys Tyr His Pro Ala Lys Pro
    3320                3325                3330

Phe Gln Ser Gln Leu Pro Ser Val Lys Glu Gly Ile Ser Glu Asp
    3335                3340                3345

Leu Pro Val Lys Met Pro Cys Leu Tyr Leu Gln Thr Leu Ala Arg
    3350                3355                3360

His His His Glu Asn Phe Val Gly Tyr Gln Asp Asp Asn Leu Phe
    3365                3370                3375

Gln Asp Glu Met Arg Tyr Leu Arg Ser Thr Ser Val Pro Ala Pro
    3380                3385                3390

Tyr Ile Ser Val Thr Pro Asp Ala Ser Pro Asn Val Phe Glu Glu
    3395                3400                3405

Pro Glu Ser Asn Met Lys Ser Met Pro Pro Ser Leu Glu Thr Ser
    3410                3415                3420

Pro Ile Thr Asp Thr Asp Leu Ala Lys Arg Thr Val Phe Gln Arg
    3425                3430                3435

Ser Tyr Ser Val Val Ala Ser Glu Tyr Asp Lys Gln His Ser Ile
    3440                3445                3450

Leu Pro Ala Arg Val Lys Ala Ile Pro Arg Arg Arg Val Asn Ser

3455                3460                3465

Gly Asp Thr Glu Val Gly Ser Ser Leu Leu Arg His Pro Ser Pro
    3470                3475                3480

Glu Leu Ser Arg Leu Ile Ser Ala His Ser Ser Leu Ser Lys Gly
    3485                3490                3495

Glu Arg Asn Phe Gln Trp Pro Val Leu Ala Phe Val Ile Gln His
    3500                3505                3510

His Asp Leu Glu Gly Leu Glu Ile Ala Met Lys Gln Ala Leu Arg
    3515                3520                3525

Lys Ser Ala Cys Arg Val Phe Ala Met Glu Ala Phe Asn Trp Leu
    3530                3535                3540

Leu Cys Asn Val Ile Gln Thr Thr Ser Leu His Asp Ile Leu Trp
    3545                3550                3555

His Phe Val Ala Ser Leu Thr Pro Ala Pro Val Glu Pro Glu Glu
    3560                3565                3570

Glu Glu Asp Glu Glu Asn Lys Thr Ser Lys Glu Asn Ser Glu Gln
    3575                3580                3585

Glu Lys Asp Thr Arg Val Cys Glu His Pro Leu Ser Asp Ile Val
    3590                3595                3600

Ile Ala Gly Glu Arg Ala His Pro Leu Pro His Thr Phe His Arg
    3605                3610                3615

Leu Leu Gln Thr Ile Ser Asp Leu Met Met Ser Leu Pro Ser Gly
    3620                3625                3630

Ser Ser Leu Gln Gln Met Ala Leu Arg Cys Trp Ser Leu Lys Phe
    3635                3640                3645

Lys Gln Ser Asp His Gln Phe Leu His Gln Ser Asn Val Phe His
    3650                3655                3660

His Ile Asn Asn Ile Leu Ser Lys Ser Asp Asp Gly Asp Ser Glu
    3665                3670                3675

Glu Ser Phe Ser Ile Ser Ile Gln Ser Gly Phe Glu Ala Met Ser
    3680                3685                3690

Gln Glu Leu Cys Ile Val Met Cys Leu Lys Asp Leu Thr Ser Ile
    3695                3700                3705

Val Asp Ile Lys Thr Ser Ser Arg Pro Ala Met Ile Gly Ser Leu

3710                3715                3720

Thr Asp Gly Ser Thr Glu Thr Phe Trp Glu Ser Gly Asp Glu Asp
    3725                3730                3735

Lys Asn Lys Thr Lys Asn Ile Thr Ile Asn Cys Val Lys Gly Ile
    3740                3745                3750

Asn Ala Arg Tyr Val Ser Val His Val Asp Asn Ser Arg Asp Leu
    3755                3760                3765

Gly Asn Lys Val Thr Ser Met Thr Phe Leu Thr Gly Lys Ala Val
    3770                3775                3780

Glu Asp Leu Cys Arg Ile Lys Gln Val Asp Leu Asp Ser Arg His
    3785                3790                3795

Ile Gly Trp Val Thr Ser Glu Leu Pro Gly Gly Asp Asn His Ile
    3800                3805                3810

Ile Lys Ile Glu Leu Lys Gly Pro Glu Asn Thr Leu Arg Val Arg
    3815                3820                3825

Gln Val Lys Val Leu Gly Trp Lys Asp Gly Glu Ser Thr Lys Ile
    3830                3835                3840

Ala Gly Gln Ile Ser Ala Ser Val Ala Gln Gln Arg Asn Cys Glu
    3845                3850                3855

Ala Glu Thr Leu Arg Val Phe Arg Leu Ile Thr Ser Gln Val Phe
    3860                3865                3870

Gly Lys Leu Ile Ser Gly Asp Ala Glu Pro Thr Pro Glu Gln Glu
    3875                3880                3885

Glu Lys Ala Leu Leu Ser Ser Pro Glu Gly Glu Glu Lys Val Tyr
    3890                3895                3900

Asn Ala Thr Ser Asp Ala Asp Leu Lys Glu His Met Val Gly Ile
    3905                3910                3915

Ile Phe Ser Arg Ser Lys Leu Thr Asn Leu Gln Lys Gln Val Cys
    3920                3925                3930

Ala His Ile Val Gln Ala Ile Arg Met Glu Ala Thr Arg Val Arg
    3935                3940                3945

Glu Glu Trp Glu His Ala Ile Ser Ser Lys Glu Asn Ala Asn Ser
    3950                3955                3960

Gln Pro Asn Asp Glu Asp Ala Ser Ser Asp Ala Tyr Cys Phe Glu

3965                3970                3975

Leu Leu Ser Met Val Leu Ala Leu Ser Gly Ser Asn Val Gly Arg
    3980                3985                3990

Gln Tyr Leu Ala Gln Gln Leu Thr Leu Leu Gln Asp Leu Phe Ser
    3995                4000                4005

Leu Leu His Thr Ala Ser Pro Arg Val Gln Arg Gln Val Thr Ser
    4010                4015                4020

Leu Leu Arg Arg Val Leu Pro Glu Val Thr Pro Ser Arg Leu Ala
    4025                4030                4035

Ser Ile Ile Gly Val Lys Ser Leu Pro Pro Ala Asp Ile Ser Asp
    4040                4045                4050

Ile Ile His Ser Thr Glu Lys Gly Asp Trp Asn Lys Leu Gly Ile
    4055                4060                4065

Leu Asp Met Phe Leu Gly Cys Ile Ala Lys Ala Leu Thr Val Gln
    4070                4075                4080

Leu Lys Ala Lys Gly Thr Thr Ile Thr Gly Thr Ala Gly Thr Thr
    4085                4090                4095

Val Gly Lys Gly Val Thr Thr Val Thr Leu Pro Met Ile Phe Asn
    4100                4105                4110

Ser Ser Tyr Leu Arg Arg Gly Glu Ser His Trp Trp Met Lys Gly
    4115                4120                4125

Ser Thr Pro Thr Gln Ile Ser Glu Ile Ile Ile Lys Leu Ile Lys
    4130                4135                4140

Asp Met Ala Ala Gly His Leu Ser Glu Ala Trp Ser Arg Val Thr
    4145                4150                4155

Lys Asn Ala Ile Ala Glu Thr Ile Ile Ala Leu Thr Lys Met Glu
    4160                4165                4170

Glu Glu Phe Arg Ser Pro Val Arg Cys Ile Ala Thr Thr Arg Leu
    4175                4180                4185

Trp Leu Ala Leu Ala Ser Leu Cys Val Leu Asp Gln Asp His Val
    4190                4195                4200

Asp Arg Leu Ser Ser Gly Arg Trp Met Gly Lys Asp Gly Gln Gln
    4205                4210                4215

Lys Gln Met Pro Met Cys Asp Asn His Asp Asp Gly Glu Thr Ala

4220                    4225                    4230

Ala Ile Ile Leu Cys Asn Val Cys Gly Asn Leu Cys Thr Asp Cys
    4235                    4240                    4245

Asp Arg Phe Leu His Leu His Arg Arg Thr Lys Thr His Gln Arg
    4250                    4255                    4260

Gln Val Phe Lys Glu Glu Glu Glu Ala Ile Lys Val Asp Leu His
    4265                    4270                    4275

Glu Gly Cys Gly Arg Thr Lys Leu Phe Trp Leu Met Ala Leu Ala
    4280                    4285                    4290

Asp Ser Lys Thr Met Lys Ala Met Val Glu Phe Arg Glu His Thr
    4295                    4300                    4305

Gly Lys Pro Thr Thr Ser Ser Ser Glu Ala Cys Arg Phe Cys Gly
    4310                    4315                    4320

Ser Arg Ser Gly Thr Glu Leu Ser Ala Val Gly Ser Val Cys Ser
    4325                    4330                    4335

Asp Ala Asp Cys Gln Glu Tyr Ala Lys Ile Ala Cys Ser Lys Thr
    4340                    4345                    4350

His Pro Cys Gly His Pro Cys Gly Gly Val Lys Asn Glu Glu His
    4355                    4360                    4365

Cys Leu Pro Cys Leu His Gly Cys Asp Lys Ser Ala Thr Ser Leu
    4370                    4375                    4380

Lys Gln Asp Ala Asp Asp Met Cys Met Ile Cys Phe Thr Glu Ala
    4385                    4390                    4395

Leu Ser Ala Ala Pro Ala Ile Gln Leu Asp Cys Ser His Ile Phe
    4400                    4405                    4410

His Leu Gln Cys Cys Arg Arg Val Leu Glu Asn Arg Trp Leu Gly
    4415                    4420                    4425

Pro Arg Ile Thr Phe Gly Phe Ile Ser Cys Pro Ile Cys Lys Asn
    4430                    4435                    4440

Lys Ile Asn His Ile Val Leu Lys Asp Leu Leu Asp Pro Ile Lys
    4445                    4450                    4455

Glu Leu Tyr Glu Asp Val Arg Arg Lys Ala Leu Met Arg Leu Glu
    4460                    4465                    4470

Tyr Glu Gly Leu His Lys Ser Glu Ala Ile Thr Thr Pro Gly Val

4475                4480                4485

Arg Phe Tyr Asn Asp Pro Ala Gly Tyr Ala Met Asn Arg Tyr Ala
    4490                4495                4500

Tyr Tyr Val Cys Tyr Lys Cys Arg Lys Ala Tyr Phe Gly Gly Glu
    4505                4510                4515

Ala Arg Cys Asp Ala Glu Ala Gly Arg Gly Asp Asp Tyr Asp Pro
    4520                4525                4530

Arg Glu Leu Ile Cys Gly Ala Cys Ser Asp Val Ser Arg Ala Gln
    4535                4540                4545

Met Cys Pro Lys His Gly Thr Asp Phe Leu Glu Tyr Lys Cys Arg
    4550                4555                4560

Tyr Cys Cys Ser Val Ala Val Phe Phe Cys Phe Gly Thr Thr His
    4565                4570                4575

Phe Cys Asn Ala Cys His Asp Asp Phe Gln Arg Met Thr Ser Ile
    4580                4585                4590

Pro Lys Glu Glu Leu Pro His Cys Pro Ala Gly Pro Lys Gly Lys
    4595                4600                4605

Gln Leu Glu Gly Thr Glu Cys Pro Leu His Val Val His Pro Pro
    4610                4615                4620

Thr Gly Glu Glu Phe Ala Leu Gly Cys Gly Val Cys Arg Asn Ala
    4625                4630                4635

His Thr Phe
    4640

<210>  3
<211>  290040
<212>  DNA
<213>  Homo sapiens

<400>  3
cttcagcttg gagtactaaa tatattctat gaaatatact ttattttaaa gtgcaatata      60

tttgtcaaga atgcactatc ttattaccct taaaaagaga aggtcactga agagtaaagc     120

atgtcttcac tcttcaagcc taagcttttt cttttttttt tcccaaattg ttctcagcta     180

ccaattttgt ttttaaattc cccaatacat tgttgtacaa atccaggaaa caaacgcaga     240

ctttaccaca gcattttcag cagtctcgat gtacctaata atatacatac ttcctgtgtt     300

tgtagtcttg tagctttagt acaatattaa ccctttaaat gaaacaacac taatctgtcc     360

tcttatatac acgttatggt tcctgccctg ttatttacta catcagcacg ggtagcttcc     420

tttaaaaaca aaatcgttaa agatgagagt aggaaaaatg taaatgtgat aagagttcaa     480

aaaactgtgg tccaggtttg caatgttaag tggaaaagaa cgactaaatc tgtgatcaca 540
aggggaaaga tgaagccata gtcttattac atgggcacag gacagagaga tctcttttgc 600
gaaatgagca taccaaccac aaagaataaa acatactacg cccgtaataa aatgggcaca 660
ggcaggattt gccgtagttc aatgctattc tttgagggcg aatgtgtcgt gggtgaactg 720
ctgtctagcc cattccgaca cccaagccgc ccaggcggcc cccacgcccc ccggctgcag 780
ccctccctgt acacacaaat gcacacacgc ccagcccggc cgctggagga actaagatgg 840
cagcgcagaa agcacggggg ccgagctcgg tggggtgggg aaagaaaagc aaagccctct 900
gtgttttttc attctgtata ggtccccctc tttccccaaa cacaagggct gcgtaaggca 960
ttcggtagta gagctcgcag gagcggcggg aggggacgct tgctttgcat actgccagaa 1020
acccacgtct ccgatctgca gcgacggttc aggcgaccct cgcgacttgt gaccacactc 1080
tccttcacgt ccccttgaca gtagaagtaa gggtgactcc aaatgccttt ttctttctgg 1140
catgcgcccc actcccccgc catcccaggg gagccttcca agttgcccag ctgccaaggc 1200
ggtagctgtg gcagttgcag ggaccgggga gcgcgccgtg cggaggcagg aagaggagga 1260
ggaggagaaa aaacgctgca ctggctatca attttgagga gagcgaccgc tgccgctgcc 1320
gtggaaggaa acgctgccaa ccgcaacaga aatgccactc tcggatacct aagtgagcat 1380
gtgtgcgact ctgcgcacgc ctagttgcgc agctcaatga catcgggctt cttagcaagt 1440
tatccagttt ccgggttttg agtgtaccca tcaggctccg taggggcctg tctgggactg 1500
aacagcagta aagattgaag cgaaattaat gaatggtggg aagagcccgg agaccagggc 1560
agccacgcgg gtcaatgtcc actagaaact ctaaatgggc agtgactgca cattccacca 1620
cctgagcctg cgctgtaaat gttttctgtg caagcaacat tcattcattc agccaacatc 1680
tatttccctc ctgttcaggg ctagaccctg tgctaggagc ctgtgatggt tgaacagaca 1740
ccattgctac cctacggaaa acctagtgtt ttgtttagta tgtgctacca tttgtgaaaa 1800
ttcatgtgcc aagttggaca tcctagatcc tttaagtcct ccctcaatct catgtagtgg 1860
gtattctttt gagcattatt cagaggagat aatttgccca aagccagaca agtattaagt 1920
agcagagtca ggatccaatt ccagggctgt ctgatttcag agccttttag ctaccctgct 1980
aggtcttgca atagtaatgg gccctctcat tgcttacctg tcaggcctta gaattcttag 2040
aatttagctg cctctacacc attgtcttgg ttctcactcc agggttaggg gctgcctggt 2100
caacactgag gtctttaact gtgctgaagc attaggatag aaatttagtc taataaatac 2160
ttactggata cctactacat gcaaagtcct gaagtggctg aaggagacac aaaagcagca 2220
agctctgaag gaacttaggt aaggaaacaa gacaaataca aaaacttaac agggctaatt 2280
acagtactaa aatactatga ttccacatgt cttgtccttt gttcctatgt ttatcccact 2340
gctaattatc tttaaaaaaa ctaaatgaaa tccatccatt atttaaagct tcagaaaaaa 2400
atgcattgct ttcagcaacc ttccacccta aaactagaat taatttactt tcctattcct 2460
tgttgtttgc acttctcaaa cactaaggag gattttttaa catttatttt attttttttt 2520

aagagaaggc catccgaaga agaggcacag atataagaac tagtacatag atgaaagata 2580
cagtactggg atgacagaac ttataaatta ttttgtcagg actgtaaatt aggaaactct 2640
tcaaattgag agtcaaagga taaaacgtag taggaatcaa agtgcattgg cacgtatatt 2700
tctagagcat atacttagat ggggttcgta ggttagcttg gaacaaagga ctgagcaaat 2760
gttacttttt tatagacaac aacaagatat gtgatggatc tgacattttc agacgctata 2820
cagtctccgt cacaattatt gtttcagatc gcattcaaga attatcttta aaaatgtaag 2880
tttgggcaga aaaaaataaa ccattaaatg accactgcca agagataaaa gtgcactctt 2940
aggtgagtga ggagtcaata aaaaagagta atttaaaaaa tattattgct ccgttgagaa 3000
caagaatttg ggaatgactc tggcttgtga atactagcct atctgttata ctttcacaca 3060
caaaatactt tcacacacaa aaaagaatgt gctagattac tacctgtctt tgtaagtaaa 3120
agctgtgtta aacaagcgta aatcctaatg acaaaaacca tataaaataa gatcgctaaa 3180
ggcacttcta attgggatgg ttttctctgc cttaaactct gaattaacca gcacaattca 3240
cagtaaatct tcctgttggc agcagagcct tggagaagag gtagcagaag agcgtgccac 3300
tcctcctctg gcgatgggca cgttccccct tgctttctgg tcctcgttct tccacctgga 3360
gggaacctga agtcgaacgt cagtagctga cagcctcccc agcactttcc ttactcttct 3420
tcaagctgcc agtaaaaccc ggagaagtgg actacttcag attccgcaca acccaacagc 3480
cccaaaccca aagccccgag gcggagggag tggtggcgga gagggggtgg ggaggaaaag 3540
gggcggcagt tactgagcat gtgcgaggag tggcgcatgc tctgtgaggc cggcagcttc 3600
ccattgcggg tagccccggc ggtggtggcg gtggtagcgg tggtggcggc ggcagtggcg 3660
gcaccgcctc ctcctcacat tcccggggtg gcggggttag atgagcggcc ccagtagcgg 3720
cgagggcggc gcggggggga ggaggagaag aaggaggagg agaaggaggt cgctgtcttt 3780
gtagtctccc tgctgcggga gccagaggcc gccgccggag ccgtcgtcgt tggaaaaggg 3840
ctgtgtgtgc gcgcgcgtgt ctgcccgccc ggcccgcggg gacgaggcgg cggcggcggc 3900
ggcggcggcg aggatgatga tgtgcgcagc gactgcctcc cccgccgccg cctcctcggg 3960
gctcggcggg gacggattct acccagccgc caccttctct tcctccccgg cgccgggggc 4020
gctgttcatg ccggttcccg acggctccgt ggctgctgcg gggctggggc tggggctacc 4080
cgccgcggac tcccggggtc actaccagct gctgctgtca ggccgggccc tggccgaccg 4140
ctaccggagg atttataccg ctgcgctcaa tgacagggac caggggggcg gcagcgctgg 4200
acacccagcc tccaggtgcg tccccagggt gcccttcctt gcgccccatg ccgcccttcc 4260
ttgcgcccca tgccgcgcgt gcacccgcgt gtgtgtgtgc ttgcgtgtgt gtacctgcgc 4320
atttattgag ctttcagtcc gctctggatg tctgttggca ggagcactta tcgagatagg 4380
agggtgcggt ggctgcagtt tctcgtgtgt gtgtgtgtgt gtgtgtgtgt gtgtgtgtgt 4440
gtgtgtgtct gcctgcctat gtggggggat agtgaggggt ggtaggggaa cctgtgatgc 4500
tggaaaaggc gatggtgctg gtcggctgtg acacgtgtgt gtatcgttga ggtaggaggt 4560

ggcatgggta gagagggtaa gaggatgata cacgaagaga attgcaacag tggcaaggga 4620
agtggcgttt tcagtgtctc tgccgcaatg ttatttgcac gtgccgcctc tcccatgaca 4680
ttccttccag ccctttccac ctcccttcaa tctgcctttc accaagagga gtcatggtat 4740
agattggttg agttttggat aagcaggccc gagggatagg cgtgaggttt gctttaaaaa 4800
gatttatgaa taagggacgc agatggagat ctgtgtggat gcgaaaggtg tctgatagag 4860
atttttagag ctgtactttg ataaccgggc cttttggtct tctagaaatg aggttgcagt 4920
tactcttgtg atctgatgtc ccatcggcca tttctcttca tcctctgttc gatttcctct 4980
gttacttaca ggctttagag tagagagggt tttgtaggga aaaggggaaa actgcagaac 5040
ttcagaatat gacagctggt acagcgttca actgccacta gctagaaaat ctgaatagcg 5100
cgtagatttg tctcttttac ttgggttggc tacaccttca gcttccttga tgtagatgtt 5160
cagttggttt catttctttt ttcttaaaat gtgggtgctt ttttttgctg agggctctcc 5220
tattgttttc ttttccatgc ccttacatgc cgcataccct gaaggttggt gactgacttg 5280
tttcaccagc tttgacagtg gtaattggga gttggcttca tctttttttt cccacagctt 5340
cttgcagtac atggggaaaa catagtagga aactgtcaaa atgagttaaa gagtttactg 5400
ttatttattg aatgtgcaat ttattaggca attgttaggt gtctttttct cccattatga 5460
aagggacgag gctgtaagtc aggtatttgt agcttatcac taggtatcat ggcttatctg 5520
ttggagacag ttgtaaagct ggagctttgt tcacactttt tctaggttaa ccagttttct 5580
catgtctgtg attgtttaca ggattgggca cagcagtagt tttaccatat ggatgtaaac 5640
gtgtgtaact attttttgct atctgaactg aaggccagaa tggcacaagt ctgtttcttg 5700
ctaatgcttt tgaaaatagc acaaaactac gtgagacagt ttgccctgat aattaggtat 5760
aattttgatc tgtaaatctc taaattatgg caccgcagga acatctttac aaactcctct 5820
caaagaattt aaatttattt tgtgtatggt ttccagactt atgcaataat ttttgacttg 5880
tatattcctg ttttgacttc ctactactag tgtaatatga tttctgtgta tctagaacta 5940
tattaacggt tttagacaaa attaacatag attaatcata taacatttgt gaatgatttt 6000
ttagcccttg gtccattttt gtacaagccg tcaatgaaaa gtaatgaaga attagttcaa 6060
gtatttcaag aactactcat ggaaatcagc attatgttat gatattgcct gctaatgctt 6120
gttcaacgca acattattgt ttatttaagt tcacattatt ttattacaaa aagtagcctg 6180
caactactct attgatgatt tgattaagaa ttaaatcaca tttcttatta aagatgttga 6240
ggaaagtaga cttggggttt cggctctgaa tgaatgatag aattatttgt atagggacac 6300
aagttaaagg agaagatgat atcttttaga gagactgaat ggagcaataa tagagaatga 6360
agctctagtg agcatcaggt ttaaaggtgt gagagaaagg agacagtgaa ggaaacggaa 6420
tcaaaattaa gaagaaaatc atgaaacagt agcctctgtg gctctaggag agtggattga 6480
aaattctcag aagtggtaaa tgttgaagaa agtgagaacc gttttcaggt cattgataac 6540
ttttgaggaa gtggtcagaa tagagggatg tggtgaggag tgaatggaat gtgtttttgg 6600

taactcaagg ggatttcaaa gttgagggaa ggatattcaa gtataggagg ttgggtggga 6660
tatgcaaata tgggaggcta aatggagaga cagagtgaag aagtgagaga ggggataatt 6720
gataggaaaa ggtcaaaggg atctcaggga caggaagagg tcaaagggat ctcagggata 6780
gagttaaggt gacaggtgga gaggttagcc attgaaaaaa ggagcagtta atatagaagg 6840
ggtggaggtg agtacttgta aatataaaga ctgggagttt attgaggata ggcatcatat 6900
cctattcgtt ttgataatcc caatgtctag cacatttttt ggcatgaatt ctgtaaacat 6960
caagtgcctg ctctgtgcca ggcagtattg cagatgctga agatgtagcg gtgagcaaga 7020
tagacaaagt ctctgccgtc ttggaaaatc cactccaatg ggagaaatag acaactcata 7080
atttcactta gtaataagta actgtgaagg caataaaacc tatttcatgt gatagggcaa 7140
tttgtagtgg ggctatttta ggtaacatgg tcaggaaatg tctctttgag agttggcatt 7200
taaattgaat tgccatgatg aggaggcaga tgtagaggaa gaatgttctg ggaatttata 7260
aaggccttga aatgggaaca ggcttgtgtt caagaaacaa aaacatgatg agtgagttat 7320
cagtcaatgt caaatgaatt aattactctt tgctttaggt gtacagttga gaagttgaga 7380
gtttcaaacc taatggactc agtgactttg atgacagtct aacaaagtgg ttaagagctt 7440
gggcacagaa gctagactgg ttttgcatcc tggttttgtc atttgctagc tgtgtaactt 7500
tgagtatatt acataacttc tatgcctcag tttttaaat atgtagaatg gggatcataa 7560
tactaacatg aattgttttg tagattaaat gaatcaatgt atgtgcttgg catgttgact 7620
gctatgaaat attagttact gttttttgtc tgtttgtttt tgctgagaat gaggggtgtg 7680
gggacttgaa gagaaggcaa ggatcatgtc ttactctttt atttttggtc tcagtgactg 7740
tgtagaatgt gggaataact ctttcattta tccagtgact gaatagatat atttttggat 7800
gaatgaggcc aatcaaaatc ggaacattct gttggggaaa ggggttggac aggaaatgta 7860
tgaaggaatg tatgatctgt tctgaatgtg aatggtggta taaccatgca cttgggaggc 7920
accatagcac agtggctaaa tgttagagca ctggaggtag actcccttag ttcacgtcta 7980
cctttttcta cttgtgttaa attgggcaag ttaattagac tttgtctgtt tctttacttg 8040
taactttgta gtgttatcaa aaggacatta tgtccgcacc tggtaagtgc ccatgaaatg 8100
ttacctgttg ttattattaa aatcacattt aaaattccaa ccagcatggc actgtatgtt 8160
tttccacaca aaatagaaat ggagaaaacc tagttgagtt gatttagggt taaagaaaaa 8220
agaaatggaa ggttttgggg acagtgtttg actatatggt aaaaaggaaa gtgatgccaa 8280
gagggaactg atagactgga agataaagag gagtcagagt atctgggtcc tgaagtccta 8340
gaagaacaat gttagtgaaa acaatagagg ggattgtgac caaaatgggg aatctaaact 8400
taaccccctt agttttttca gctgctttat ctctttcttg ttagaaatgg tatttgagtc 8460
catccttcag atgcatggga ttttggttgt aagaggttaa gtagtcattt tagcaaaagt 8520
tcagggatta ttttatttta cttattttga ggcaggctgg agtgcaatgg tgtgaacata 8580
gctcactgca gcctcaacct cttgggctca agtgatcttc ctgcctcagt ctcctccagt 8640

agctgggact acaggcatgc accaccatgc ctggcttgtt ttttattttt tgtagacaca 8700
gggtctcacc atgttgtcca ggctggtctt gaactcctga gcttaagtga tcctcccacc 8760
tccacctccc aaagtgctgg gattatgggt gtgagccacc atgctcagct tgctcaggga 8820
ttattacttg gcactaacca ttggccctat aaaaatttat aagacacaaa ttctagactt 8880
gagtttactg tctatctgga agtgcaagat acaaaggcag atacttatag aagtgttaaa 8940
tgaaatgacc aagaaagtat aacctactat gacaaaggca tggcattcta ggctggctta 9000
aaagcatcag cagagacagg aaagcacagt ttgtatgtag gtaacagtag gaattctgtt 9060
agagctagaa tgcaggtgtc atgaaggaca gtagtggaat tcattctgga aatggaggct 9120
ttggctagga tggggcatct tgaagaccaa ggtaagaatt agattttatt cagtagacac 9180
tgatcaccct tgatggtttt aagctatgaa gagacgttat ttattgtagg aatatgtcag 9240
gaagtaatgc gggattgaat ctgtatgtgt acatctgtat ttgtgcatag gtagtaattt 9300
tcttcatgta gctgttatat cagtggaatc ctgtatagtg tataaacaca agttaaggca 9360
aaaactgctc tattggtata ctggtaggta gaaatgtgaa atgaattagt ttcctaactc 9420
aagagaagaa aaaaatcttg cctctgggct taaaaggaaa ataaaaatgt caatctaaaa 9480
atacttttgt cctttaatat atttgtttaa aaaatcaaca gaatagtctc acttttttct 9540
caaaaaccag attaaacatt taggttttct gaggaggggt taggttaatt gaggtttaat 9600
ttatatacaa taaaattcat cctctttaaa tgttcatttg aagagtcctg aaactatcac 9660
cacagtcaag tatagactgt ttccatcact tcatataaaa agttctgtca ggccctccta 9720
cccctggtta ctgaccgctg caaatcagtt ttctgtcact aatattttac cttttctaga 9780
atgtcatata aatctagctc cttaagggca gggcccccag gtattgttca tctttctgtc 9840
ttgtatctgt atttcctgta agtggaagct agaccttaag ggtttattag gtgttcatta 9900
gaaaattttt tagcaagaat attgtatatg gcgctgggta ctgcatgttg catcccatcc 9960
agaggttgtg aggttgtctc actatttaat atgttaaatc tgattacttg ggtaagacgg 10020
tacctactag atttcatctc tgtaaatgta tgctttttcc atttgtaaag aacaagtaat 10080
cacagggtaa aaatttgagc acattgtgaa tgccttttac ctaatgcttt tagcattctt 10140
tgataatcct tgtctgagtc aataatttca ttaggggttg caaaatgatg aattaaaaaa 10200
taattctgtt atttcttcta tgtttattag ctggtactct tctgcaaaga agagttggcc 10260
ttcactgact gtcagtgaaa tgtagtttct cctaatccag cagcataaat gcttaattct 10320
cttccctcat tagccaaatt ggtgtaatag tccagtggtg gtgtggcagg tgctgtggtt 10380
aggctttcag atcccgcttt caggcacatg ttcccctagc tcttggcaat attggcagct 10440
aaaactgatt gcagctgagt cccactccac gcattgccct gagccaagag ttgcctcccc 10500
cagagtcatg cctcctcttc aggggcagtt tacatctaat atctatacag tgtagggata 10560
taaatgccta gcgcttttgc tataagacaa gccaaccttg aagtctatcc caacaccaga 10620
gagctctccc atgggtctgg ctgagggctt cctgggactg cattgcagat caactccttc 10680

ctctgttcag tcttacgtct ttcactgccc cacagttgtt ggttcggagg gcactccgca 10740
ataaacttcc ggtgtgcaca tctgcatttg agagtctgtt tcctgggaac cctcctaccc 10800
aaagcagttg gtgccaggag tggtctcaga aagtagactt tacagtgagg ttttgaagct 10860
ggattattgg ctggctggct ggcaatgaga atcgattctg gtgctagatg gagcactaat 10920
actgataacg cttggcatgc actgtagcta tgcaacttaa aactttcact ggtggtgagt 10980
tggaatgcta ttcctgttga agggaatgca ctggtgagtg cactaggctt ttgagaagtt 11040
cagagaaatt aattatgagg acaatcatca gatggctttt ggtaaataac attgatagat 11100
tggagaaaga tgatgcaagg tggaagataa ttgggggcaa aatgtaaaag gaaatttatt 11160
tagcatcatg taaagaaaca catatacagt aagaggaaaa atctaaggct tcaacccaaa 11220
acttaatttt aagggtagca gagcaccaag gtaggtagaa ttctctctca gctatgctaa 11280
ggtcagtgtt ctggttgggc aggaatcctg acccttagga tggggcatct gagtgcactt 11340
gaaaaccctg aaaccccaaa ttcctcttaa ccctctgggc ctgcagaagt ggccttcttc 11400
tactagctag aggggttgct tgcagactgt gctgtccctc ttacccccag ctcctcctct 11460
ctcgcctact gactatcacc aaaccaacaa ctagggttaa gtcacaagaa cacctagttc 11520
ggggtgctgg atctgcgaag ggaggaaggg actgtacaca caaacacaca cacacacacg 11580
ttcccaaact gcaccaaggc accccaaagc accaccacag acacaaaaga ataccatggg 11640
atatttcaga attttaaagg aaacacagca atatctgtca caaattacta gcttgaggta 11700
gttcacggtt ttgacattag ctcatgccac atttcttttg atgatgtcat atttttgcag 11760
ggttggattt ttactagttg tgatacagat caagcactgt gcaaaaatca gtgtggaaca 11820
ggaaaggagg gtggcatgtt cagtctgatt ccaagtttga gaagttgttc tgtgtctaac 11880
aggcacacat gtcatattat caagtaattg aagtttttgt ttgtttgttt gttttgaggc 11940
agagtctcgc tctgtcaccc aggccagagt gcagtggtgc gatctcggct cactgcaacc 12000
tccgcctgcc aggttcaagt gattcttctg cctcagcctc ctgagtagtt gggattacag 12060
gcgtgtccca ccacactcgg ctgatttttg tatttttact agaggtgggg tttcaccata 12120
ttggtcaggc tggtctcgaa ctcctcacct cgtgatccgc ctgcctcgtt ctcccaaagt 12180
gctgggatta caggcgtgag ccactgcgcc tggcccagta tttgtagttt ttaagaagga 12240
aataaaaata tttttcttcc aatttatgtg ttattttttc aaatgtctac tcatttgtta 12300
ggacatgaat tcttgttatt tggatctaac tacttaataa acactactgt ttggtatttc 12360
ttttggccta tgagtgcatg gaaaaattac cgatgatggc tgtgcatggg cccaataaaa 12420
gggttttcat tcaccagtgc tgatctagca actgctgctg ctgaatatcc agcctgacag 12480
cagcagagat caatcatcct tcaagcaggc caaccagtcc ttatacagtg gaaaggacca 12540
tgattcattt tgattggatt tgagacatat tctgggtctg aatttgtctt tccagcaggg 12600
tctcacccag gactactgtt tgagggcttg caggatgttt gatcctctac tacagggatc 12660
atttcagaat aagggaccca ctgttatgtt atgttatgtt atgttatgtt atgttatgtt 12720

atgttatgtt atgttatgtt atgttatgtt ttgagacgga gtcttgctct gttgcccaag 12780
ctggagtgca atggcgtgat ctcagctgac tgcaacctcc gcctcccggg ttcaagcagt 12840
tctcctacct cagcctcctg agttgctggg actacaggtg cacatcacca cgcccagcta 12900
atttttgtgt ttttagtaga gacggggttt caccatgtgg accaggatgg tctcgaactc 12960
ctgacctcgt gatccggcca cgtcagcctc ccaaagtgct gggattacag gcgtgagcca 13020
ccgcgcccgg ccgggaccca cttttataga ataggagaat catttgtccc atcttgcacc 13080
acaccgtcca gaagctgatg acttgataga gcagtaaaac agtcctttga tggcacagct 13140
gatgatgaga tcctgcagct tgggcatgat gccctgcaag ggtgaggcgc ccattctcca 13200
gtgttcccag taggaagatc acatgaaatt aggagtggct ccgcatagca tcactctgtg 13260
aatcacctgg gcagtttctg cttcccatct ccatagctct gatggcaagg ggttaaaaga 13320
tcctggttct cagaagagaa acatttccaa cagagggcgc tatgagaatc ctgttatact 13380
ttaaagtaca gctgccacta ggatttgggg ctccttttct aaataaagaa gtcaccaggc 13440
tgggcacgat ggctcacgcc tgtaatccca gcactttggg aggccgagat gagtggatca 13500
cgtgaagtca ggagttcgaa accagctcag ccaacatggt gagaccctgt ttgtactaca 13560
aatacaaaac aattagccgg gcatgttggc gcgtgcttgt aatcccagct aattgggagg 13620
ctgagtcagg agaactgctt gaacctggga tgtggaggtt gcagtgagcc gagattgcgc 13680
cactgcactc cagcctgggc aatagagcaa gactccatct caaaacaaaa aaaaaacaac 13740
aaaaaaagaa gtcaccattt ggacagggtt aatgtccttg ttcatgtgga ggaggtaggg 13800
ctgtgttcca cagtggagca tggaggtttg gcacacaggt gatctataag gtgtctcttg 13860
gtacttcctt gtccagtgtt tgtggtaaat ggacacgaga aaggcccagt gaccacccca 13920
tgaagtcagc cacctggacc aacagaaata ctagccatag attaaaggaa tctagaatgg 13980
ctagtagaga agagttgttg atatcaattt taccgtctgt agtggctggg gctgtggttt 14040
gacccacttt ccttcctctg gaaagtttct ccaggaaatg acactcacca gaattctgga 14100
ggaacttttt ccatgcctta tttgaagcaa gtgaatccta attgtataat aggcgaaata 14160
tagtgaatcc tgtattgtgg aacccagagc ccctgcccag cactgatgtg ctcaaaattc 14220
ccccacgact gctgggaaca ttggttggct acagactctt ccctagttcc aatatgggca 14280
ttgcctttgg caataggtga tggccctagg tgatggatca ttcttggggg cagcctgaat 14340
gaactggttg atcggggcat tttatggttt ttagtgctac tgtaactagt ttagtttttc 14400
ctatttttca gttgtttgat atgaatatat aacaatacag ttgcttttga atgttaacct 14460
tgtatctctg atcttgttaa gctcatttat taactttaat agttttgtaa attccctggg 14520
atgttctgtg taaataatca tattgtcttt ctttctgatc tctagattct cctacccctc 14580
agcccctact ttattccaat tacttaattc tcccttgttt ttaatctgag agaaaaacaa 14640
ctacaaatta atcttacagt tttttaaatc attaagtatg atattagctg taggtgtttc 14700
aatggcctct atcaatttga agatattccc tagtattcct actttgctga tagtttttat 14760

tttttatatt ttaatttttt tttttttttt ttttgagaca gagccttgct ctgttgccca 14820
ggctggagtg cagtggcata atctcggctc actgaagcct ctgcctcccg ggatccagca 14880
agtctcctgc ctcagcttcc tgagtagctg ggattacagg cacacgccac tatgcctgac 14940
taatttttgt atttttagta gagatgagat ttcaccacgt tggccactct ggtcttgaac 15000
tgctgacctc aggtgatcca cctgcctcag actctgaaag tgctgggatt acaggcgtga 15060
gccatcgcac ccggcttgct gatagttttt aattataaat gtgtattgag ttttgtcaaa 15120
tgcttttcct ctacctgtta aaatgatcat atagtaagag aacaaaagga aaattgaatt 15180
acattaatta atttttgaat gttaaattaa ctttgcattc ctgggataaa accatcatat 15240
ttggaatgta ttgctgaatt tgatttgctg gtattatgct aagaattttc aggtttatat 15300
tcatgaggga tattggtctg taatcggttt tttttctttt cttttcttgc taatcctgtc 15360
aggctttaga attagttatc ctcgtctcat aaagtgagtt acatagtaat cctaccctac 15420
ccagtcctac ccaattttta aaatgtttgc gtaagattgt tgttatttct tcttgaatgt 15480
ttgacagaat tcaccagtgg caccatctgg tcctggaatt ttattttggg gaagcttttg 15540
acaaattcac gttctttaat agttagagcg ttattcagat tttctgttat tattcatgtc 15600
aattttggta acttgtattt ttttggagaa gttcattcat ttcatacttc taagttgttg 15660
aagtttttag catgaagtta tccatagcat tctttcatta tcctttaagg tctatgggat 15720
ctctgataat aactccctcc ttttttaaaa aaaatatttt tttaagagca gttttaagtt 15780
catagcaaaa ttgagaagag ggtacagaga ttttccatct atccctttcc cccactcacg 15840
catagcctca tccatatcaa catcccccac cagagtggca tttttgtggc cattgtttaa 15900
cctccactga cacatcataa tcactctcaa gtccagagtt tacattaggg ttcactcttg 15960
gggttataca ttttttggtt cagaaaaatg tataatgaca tgtatttatc actgtagtat 16020
catacagaat attttcactg ccctcaatat catctgtgct ccacctattc ctgtttctct 16080
gccactcaac cccaggtaat cacttactgt ctccatagtt ttgccttttc cagaatgtgc 16140
tatagttgaa atcatacagt atgtggcctt ttcagattgg tttccttcac ttagtaatgt 16200
gcatttaggt tcattctttt tttgtgtatg agacttgata gctcatttct taccactgaa 16260
taatccacca tgtagatgta ccagaatttg tctgttcacc tacaatttgg tgagtttgga 16320
ggacaattgg gttgcttcca agttttggca attatggata aagctgctct aaacatctgt 16380
gtgcaggttt ttgtgtggac atacattttc ttttttagaa attgacaaat gacattgtat 16440
gtttttataa tgtacagcat gatgttttga agtacatata cattggggaa tggttaaatc 16500
tagtggacat gttttcagct cctttgggtt aataccagaa acagtgattg tgggatcacg 16560
tgctaagagt aggtttagtt tttgtaggaa atcaccaaac tgtcttacaa agtggctgtt 16620
tcatttttca tttccaccag caataaatga gagttcctct tgcattaaag cctcgccaac 16680
atttgatgtt gtctatgttc tggattttgg ccattctgat aggtgtgtag tagaatctcg 16740
tttttttttt tcatttctct gatgacatat gatgtagaac atcttttcat aacgcttatt 16800

tgccatctat atatcttgtt cggtgaggtg tctgttaagg tctttgcccc atctttaatt 16860
gagttgtttg ttttcttatt gttagagttt tgagagttct tggtattctg tgagggtgta 16920
actgaggaga tgggggaaaa agttcttggt attttggata acagtccttt atcagatgtg 16980
tcttttgcaa atattttctt tcaatctgtg ccttgtcttc tcgttctctt ggcattgtct 17040
tttgcagagc agaggttttt aattttagta ttcagcttat taattatttc tttaatggat 17100
tgtaccttgg tgttgtagct aaaaagttat ctaggttttc tcctgtgtta ttttctaaga 17160
gttttatagt tttgcatttt acatataaag tccgttttcc attttgagtt aatttttgtg 17220
aagggtgtaa ggtttgtttc tagattcatt tctttgtatg tgatccaaag aaccaacttt 17280
tggctttctt aattttctct attattgatc tgttttctat ttcattggct tttccatcat 17340
cattgtaatt tatttctttc tactttcttt aagtttactt tgctgttcta atttcttaag 17400
gtggaatcct aggccattga ttttaaattt ttcttccaat ataagtattt aaagctatga 17460
atttttatct gggctgtgct ttagttacat cccacaaatt ttgactgcta tatatttgtc 17520
atttagttca aattattttc caatttattg tttaaaatta ttacaacttt cagatatggt 17580
gttttcttac atattaatat attattgtta ttactttcta atataaacct tgtatgattt 17640
tgatctttaa catttattga gatttgtttt attgcccagg atatgttctg tcttggtgat 17700
tgtattatac acacttgaga agaatgtgtt cggtcatctt tggacatgaa gttctataaa 17760
tgtcagttaa agttggatga taagtgttgt ttggatgttt atgtttttat atttaaagtt 17820
tctcctctta ggcaactagt atagaacctt gcccttaaaa aaagaaatcc actgtcaatc 17880
tttgcctttc aattggagtg tttagattat taatatttaa tatgattaat tattgacata 17940
tttaccatat caacagtgat aggtctgcca ctttattgtc ttctgtttct ctttttttgt 18000
tcctttcttc ctcctttctt gcttctttgg gattatattt tgggattatt gggagtattt 18060
cattttagtt gctctattgg tcttttcagc aatatctctt tgtatttact ttttttatgc 18120
cctagggatt acagtatgca tacctaactt tttacaggct acttagggtt attattatac 18180
taattcactt aaaacacaga aaccttgcaa ccctacagat ccttttattc tcccctcccc 18240
cagagacctt tatgttatag ttgtcatgca tactatactt ggcaaacatc accaaacaat 18300
gttatagttt ttgttgtcga cagtcatgtg tactttatag aacttagagg aaaaatctag 18360
tattttgtgt tttcctatat atttattgtt tctattgctc tttcttcatt cctgaagatg 18420
cagcatttcc tttcagccta atgttgacct tagcttttct agcagatcag tctgctgggg 18480
atacattctc tttgtttttc tttgagaatg tatttatttt actttcattc ctaaaggata 18540
ttttaggtgg atatataatt ccgagtagat gcttgcttcc ttgagcacct caatgatgcc 18600
atttagctgt cttttcactt ctctgatttc tggtgaaaaa tctttgtaat gtaaacctta 18660
ttcccctctg tgtggtgtgt aatttttttc tagctgcttt caaaaatttt ttctttgttt 18720
ttggttttca gcagcttaat ttgatgtgta tctagtcatt ttctttaagt ttatcctctt 18780
tgaagagtac tgagcttctc aaatcggtaa atttttggct ttcactaagt ttagaatgtt 18840

ttctaccatt gtgtctttct tctgacctta caaaattcta gtggcaatta tgataaaact 18900
tttgagattg tttcacaggc ctctaactgt tcagtctcct atcaattttt tttctctgtt 18960
tttcagatga ggttattttt attgatacac gttcaggttc acagactctt ttctcccatc 19020
tccattttct tattgagtcc aaccagtgaa ctttttatgt attgtttatt tcacttttaa 19080
catttacatt tggttctta aaaaaaaac aaaaaaaaa aaacaaaaa aaacttctct 19140
ttccctgccg agaacctctt tccattccat tcagttgtcc ttgtctttcc tcaatgtagc 19200
atatttatag tggttgcctt ccagtcattg ttggataatt acaacatgtg catcgtctta 19260
gggttggcat cttttgattg tctcttccct tgaagttgtt cacatttttc ttgtgttttg 19320
cattgtgaga atttgggatt gtatcttgga catggttaat gttatgtttt gtaaactcta 19380
gggtttatta taattctctg gaggatgttg tgttttttgt tgtttagggg atccctttct 19440
gtgtcttact ctttttcagg acttcttacc cattttccat catatcagtt tcttttctca 19500
gttcctctgg ccagaaagag ttttaacttg gaattttaac ttttgggttg tagccctgta 19560
atgcagtgat ctcttcctgg ccttcaggca aagctgttag tgaaaggaga aaaacaacca 19620
aactgggaaa tttactcttc tgtgaatcac ttttccaagt tttgactccc ctccagaatt 19680
tgcttttatt tgtttttcag agtcctgaag tacttttttt gtctttttaa aaattttgcc 19740
caaagtagtt gtaaacagtg gggaaaatag gctgttttgg ggtttatgcc aatgtactgg 19800
aaccagaact ctcttatctg gctttaaaaa aagattttcc cttttattga tttttagcag 19860
cttgactgta ttgtacctaa atatggtgtg tgtgtgtgtg tgcatgcgca cgcacacacg 19920
tctgtgtctc tgtgtgtgtg tatcctgctt ggtatccttt cagtttgtta gatctgtgtg 19980
atgtcaattt ttatttaact tgcaaatttt ggcctttatt tcctcaaata ttatttcaga 20040
ctcaatttct tatttccttc tgggaatcca attatatata tatgatactg tttgatatca 20100
tatgtgtcta taatcatata tatgacatat ttatatgacc agatagtaaa taacttagac 20160
tttatgggcc atagagtctc tgttgcaacc atgcagctct gttctttagt ttgaaagcaa 20220
ccacagacaa tatatataaa tggataagca tggctgtgtt ccaatataac tttatttaca 20280
aagatagaca ggaagctgtt tttggcatgt agactatagt ttgctgaccc ctgagtaggt 20340
ggtaatcgtt gctggagcta cattgtgaat tcagggaatc aaagaagtgc aaaacagaaa 20400
tgttggaaat aactacaatg taggtagcgg ggtgcataaa aatgaggagg taggagatat 20460
ggctagaaag atatgttagg actaggttat tatgttccct gtattgcttt atttgaaaat 20520
agtatggaac aatcataggg tattttttgt ttgttttctt ttttttgtct gtttttctga 20580
gacggagtct cactccgttg ccaggctgga gtgcagtggg gcgatcttgg cttactgcaa 20640
cctccgcctc ctgagttcaa gagattcttc tgcctcagcc ttctacatag cagggactac 20700
aggcacccac caccacgccc agctaatttt tgtattttta gtagagatgg gatttcacca 20760
tcttggccag gatggtcttc atctcttgac ctcgtgattt gcccgcctcg acctcccaaa 20820
gtactgagat tacaggcgtg agccactgcg cccagccaat cataggtttt taagcaatgt 20880

aatggtagag ttaaactttc aatttataaa gataatctct agtagtgtta tagaggatga 20940
atgaaaagga gcagatctgc aaagcagggc tacctttaaa gagacagttg cagtaactct 21000
gacaagtgaa aatactgaga aaagataatg ggaatagatg gaataaagtg gatttaatga 21060
tatatttagc aggtgactag atctcaagtg caaatataat aatgattttc atctagatag 21120
aaagggagga agagctagtt tctcaagagt ccattcttgg gattcttctc tctgaatttt 21180
cttctttggt tgtctcaggc ttatgatttc agatatgcat ttgacttcct ggtctgtggt 21240
ttttaggtaa aacctctttt cttaagttct agcctgtatt ttgaaaagtc tactgctagg 21300
attctctcag tcctgttcag atacattatg ttcagtatat tcaaagtctc ctcgtgaaca 21360
tactgtcact cacaaactgc ctcttttccc ttaggccagt gaaaccgaag tctgagatag 21420
gttcttgagg ttggcaataa gaaattataa tgagccaaca cccactcact tcacctcctt 21480
ttttcaggag gcagggaaaa gtgggaaagt gaggctggac atgggagtat ttagaaatag 21540
tgtttaataa tgtttaaagg gcaagaagga aggcagtagc ttaaggaagg agacaaataa 21600
ttaatctttt ggattagata acctgtagga gatgttctta gtgtcctagc catatccctt 21660
tggcatccat ttgcatactg tcttctgcaa acatctatga ctatctgcct gaaaagaaac 21720
acacttggcc tgtgtgcagg gcaactcaca tgcgccagag agttaatgcc ttcagaaaaa 21780
ctttccacca gtgatggatg gggagttggt agataaatac ctttgtctca cctgcattta 21840
ggataactga gccatgtttt ctgctgtcta ccagagttct ccactggttc aggccctagt 21900
tccccatcag ggtaactggc ttcataatat gtcctttctt gacttccttc ttttccctgt 21960
tttacttttg ttttccctac tggtgttttt cctgggatca cctctcaaaa taactactgc 22020
acttgaattc ttgaattgac gtctgcttct ggggaaaccc aaaccaaggt agacatttga 22080
atttggcatg ggaataataa taaaaatgtg tttagcactt actaaatacc aggcataatt 22140
ctaagtgtga catcatccag tgggagaggt actgttttca ccctattgta tggatgagag 22200
aactgaggca cagagagatt aaagtaatac caagatcaca gtgctggtaa atgacagagc 22260
ttgtatttga acccaagtca tttgtttcta aattctgtac tcttaaccta tcttgacata 22320
gcaggctgaa ggaaaagggc aaggatagta aaaaagagag agagaatggg aataattaat 22380
agagtaaggc cctgggttag agatcatttc aaggtgggtg gaagaaaagc aaagggagta 22440
catttgtaat ggtgtctcta ttttattggt taaatagatg gttagattct ctgctctgag 22500
ttgatgggta ggggagggat tttgaagtag ctggaaaggt tctagatttt tatggtagat 22560
gtaccttgat agagaactta actggctcta gaccagcagt ctaatgatgt tcagtaactc 22620
caatagctat tattgtttag ggttgttata gcttattgaa caaggtttgt aactaaccag 22680
tatagaagtg aatatagaac atgagcagat agctcacatt agaagtgata tatgtagtaa 22740
agacttaagg gacaggaaga ggaagaaaaa ggagccagta aagaagactg agaaggaacc 22800
accatcagag gtagaaggaa cactaaagta gaggttctag aagctggcgg gacagggggc 22860
agggatgtaa agaagtaggc actgtttggt agttatcgta tgtaggagaa tgtgaaggat 22920

aaagactgaa gatgggcagt tgaattggct actagaaagt ttgtggtatt gagaagtctc 22980
atagacttgc atgagtttga tagtaaggtg ttaaaaaact tcaatacaat gaagtttttt 23040
tttttttttt tttttttttt tttgagacag ggtctcactt tgtcatccag gctggggtgc 23100
agtggcatga ccatagctca gtgcagcctc taactcctgg accaaagcga tcttcccacc 23160
tcagccatct gagtagctgg gactacaggc acatgccacc gtgcctggct aatttttttt 23220
tttttaatag agacggggtc ttgctatgtt gcccaggcca ggaccatttt tttcttcaag 23280
aaatttagtg atgaagaggg atagaggatt acttaaactt ttttcccctt ttgaattgga 23340
gaatgacagt aggcctctaa ggttagaaac cacagaaaaa aagcataaag ggtaaacaaa 23400
gagagggatg tatggttgag caaaatctca aggatggtga atgaaatggg atctggtgta 23460
tgagaggaag tgcatacttg gatagagtaa gagctgtctc ttttgggatg ggggaaggag 23520
gaagtttcag atttgaaaag gtctcaggga catattttta gcctttttat gtttctgtga 23580
agaattccag attctttttc tgatctgttt tctagatctt aaattttcta ttcagctgtg 23640
tcctgtacac ttaaatccac ctatggagtc tgtagtgtca gttatttttc atgtctagtt 23700
tgattttttt tcaaatgtgc ttggttgttc cccattccct gcagatattt taatgatagt 23760
cttttatttc tttaaatttg atacacatgt tcttgttgca atctttccga aaatcccacc 23820
atctgaagtc tatataggtg tctttctgtt gcctagttct gctgttctca ctcagagtgc 23880
cttgtatctc tgtgttctta gttatttgct tgctctcatt cctcttgttg ttctctgagg 23940
cctgggatga atagattgta ttcatgatat cattagaaag tgttacagtt ttacaattgc 24000
ttcaaggctt gagtttccct ggcctaccta gcctatgttt acttaagagt acaaatatgc 24060
atgaggcagg gtctatagcc aaagcttttc agagagttct cttcccctca cttatgctcc 24120
cttccatatt cccctaccct cttttctttt tccttctgct gctctgccca ttgccaaggt 24180
agctttattt atagtcctcc ccccatgtga agacttccaa ataagatcaa gagactgaag 24240
ctatatataa gaaatctgtt ttcatctctc ccctaaatca agttgaactg ttaaaatctc 24300
tttcttatta ctaattagcc ggctaggtta agtaaataaa agttttctac ttagtaatga 24360
gataactatt cctccctaat ttattcattg atgcttgaac atgataggag tatcattaca 24420
ttaattacac agaatgaatc tggcttctat ggaaaagctt cagtttacat aaggtgcttc 24480
atagattgat ttttaaaagt tggtcttgta tccagtgacc ttgataattc ccttgttaat 24540
tctttatttg tggattattt tgtattttct ataaatataa ttatctacaa ataaaaaatg 24600
ctttactttt aatttttaac cattatgact tttatttcat ttgcatcact tactgacctt 24660
caatattgtg ttaacagaag tggtgatagt acaaatcctt gcattttcct cctatgccta 24720
agtggaaata cttaattttt caacattcag tatagttgaa tacactgtta aaagtaggtt 24780
ttttatagat aatctgtatt agattaagta aaatctgttc ctggttcacc aaggagtttc 24840
atcactagtg gttgttgagt tttaaaaata ctttttctgt ttctattgaa gtgatcatat 24900
gttttgtttc ttttgttctg ttattatatt gaattatgtt gattatgtat ttattctttc 24960

cacatgttgt tagacaaaat tatttgtagg taaatggctc tctctttttt taaaaataaa 25020
aggggtaata cacataccat aaattcaccc ttttatacaa cttattggtt tccagtatat 25080
tcacaaagtc atacaactaa acatttttgt ctctccataa atcccaaagc tattagcagt 25140
cagtttctat tctcctttct atcagttttt ggcaactact acttcctgtt tttatgaatt 25200
tgcctaccta gacattacat ataaatggaa ttatacaatt tatgaccttt tgttgccatt 25260
gtttctttca cttagcataa tgttttcaag gttcatccat gttgtagcat gagtcagtac 25320
ttcattcttt ttttatgggt aaacaacatt ctcttatatg gaagtgctac actttataca 25380
ttcattagtt ggtgagcatt tgggttgttg ctattttttg gctgtgcagt atttttgctt 25440
ctatgttcat aggagatatt ggtctgaaat ttttgtattt tgaaatgttc tcttcaaatt 25500
ttgatatcag agttatgctt gtcttatgag ttgggaaatg tttcttcttt ttatattgtc 25560
taaaagagtc taacattgat aatgtttgaa agaactacca gtgaattttt aaaagtattg 25620
gaagaattca ccaacaaagc catccgagtc tagagttttc tttgtggata gattttgatt 25680
agggatccag ttttttttca atatatagaa gactattcag atattacatt ttttttccct 25740
tgtttaatgt tgagaagtta cattttttca aggaatttat ccattttatt tgtcaaattt 25800
atgggcagca tctgtagtga tgtctccttt cttatttctc atattggtaa tttgtgcttt 25860
tacttttttc ctgaccattt ttgctctagg tttattatat ttatttcatt ttattttaaa 25920
atatttgcta tatatttaat tcacaaataa ttgtacttat acatggggta taatatgata 25980
ttttgataca tgtttacaat gtgtaatgat caaatcagga taattagcat atttatcacc 26040
tccgacattt atcattttat catttctttg tcatgagacc attcaaaatc ctctcttcta 26100
gctgtttgaa gttgtataat acattgttgt ttatgatagt caccctgtgg tgctatataa 26160
cactcaaact tactcctact ttctagctct gattttgcat ccattagcca acccctggct 26220
actcttcaac aactcttcta ctttctactt ttatgagatc aactttttta gcttccacat 26280
atgagtaaaa acatgccata ttcatctttc tttgcctggc ttctttaact taagataatg 26340
tcctccaggc tcatccacgg tgctgcaaat gacagaattt tactcttttt taatcactaa 26400
atggtatttc attgtgtata tatatcacat tttctttatc cattcatctg ctcatggaaa 26460
cgtaggttga ttccacatct tggctattgt gaatagtgta gcagtgaaca tgggagttct 26520
gaatcttttc aacatacgga tttttttcct ttggatatac acccagtagt ggaattgctg 26580
gatcatatgg tagttctgtt tttagttttt tcaggagcct ccattctatt ttccacaatg 26640
gttgtactaa ttcacattcc catcaacagt gtagttctac tttctccaca tcttcaatag 26700
catttatttt ttgtcttttt gttatgtata ctaatctttt cagagaacca atttttggct 26760
ccctgataac ctctattgaa tgtctgcttt ccatttcatt ggtatctact cttattttta 26820
tttcctttct actacatatt ttgcatttaa tttgtggttt tctttctagc ttcttgagtt 26880
gtaagtctag ataatttact ttcagtgttt tttttttcag tatatgcatt tatgtgtatg 26940
caaaaggaga tgcattttgt ggaagtactg cgttagcagc attctaccga ttttgttatg 27000

tcaaatttca ttttcatttt atggaaaata ttttctgatt tccattgtga ctagttcttt 27060
gacccatagt ttatttagaa gtcttgtttt tttgattgcc aaacatttgg gaatttctta 27120
gttgtttgtt tttttttaat ttctcgttta atttttccat gaccagagaa ttaccttatc 27180
attcagtcct tggaaatttg tttagacttg gtctgtggtc cagcatatta tctattttgg 27240
taaatgttct atatgcactt gaaaagaata tgtattctat tgttgttgga tatgtttctg 27300
tatgtatgcc aattaggtca ggttgattaa tcatggtttg tttgtttgtt tgttttgaga 27360
cggagtctcg ctctgtcgcc caggctggag gacagtggtg tgatctacgc acactgcaac 27420
cgccacctct cgggttcaag cgattctcct gcctcagcct cctgagtagc tgggattaca 27480
ggtgcgtgcc accacgcccg gctaattttt gtatttttag tagagacggt atttcaccat 27540
gttggtgagg ctggtctcga acacctgacc tcatgatcca cctgccttgg cctcccaaag 27600
tgctgggatt acaggcatga gccaccgcgc ctggccgatt aatcatgttt tcaagttttt 27660
ctatgtcagt gctgttttgg tctctacttg ttctatcagt tacagagagg tatgttagaa 27720
cttactcttt caattgtgga tttgtatttt cccatttaac ttctgtcaat tttactttat 27780
atattttgaa gctgtgttac tggatgcatt caaatttagg agtgttatga tttcctaatg 27840
atttgaccct tttgccattt agaaatgctc ttttcatttc ttttaatact gttgccttaa 27900
aaattacttt gtcaaatatt aaaatagcca ctccaacttt ttttgggaca ctggagagtg 27960
tgcttgagat acatcttttt ctgtcctttt agcatgtatc ctttcattta aaattatgat 28020
attacagtta ttaaaggaag actttccttc agtatttctt gtaatattgg tctcttgaca 28080
gtgaattatg tcagctttca tggatctaaa aacatcttta tttttgtctt cattcttaca 28140
ggatactttt agaatccttt atgtgaatta tatcagcttt catggatcta aaaacatctt 28200
tatttttgtc ttcattctta aaagatacat tcttaaaggt tacatgattc caggttactt 28260
tcccccacct ctaaccccca tccctccact ctaccacctc cccctcccca ccccccagcc 28320
cttttcaaca caaagaatgg aaactatcaa ttttattgtt cctttaagca tatctcttct 28380
ctggctgctt taaagatttt ctctttatgc tttgctttta gcaatatgac catgctgtgc 28440
ctaggtatgc ttttctttgg attttcctgc ttaaagttcc ctgagtttct tgaatctctc 28500
agttgatttc tttcttcact tttgggaaat tgtcagtaat tatcttgtca tatattgctt 28560
ttgttccatt cttttttctc tctttctggg attccaatta tatgaatatg agaccatctg 28620
actttgtccc acatgtctcg ttctctgttt ggttcatccc attttttgttt ccgtgattga 28680
attctgttat ttatttattt atttattttt ggcctgtttg caagtctgtg aaacctgttt 28740
ttctgtaact tactctgctt ttaaaatcat atgagaagtt cttaatttca gatgtttatt 28800
tttcagtttc agaatgtcca cttggttctt tttttataaa ttatgttgaa aattgccaat 28860
tgcattattt tacacatatt gttaatctct gcatctcagg tacttgcctc ctaacgttaa 28920
tatctgggtt ttctgtgagt ctgactaatt ttctggtttt ctcttgatta tgtgtctgcc 28980
acatattttg cttcttttga tgtcccttaa tgtgtttact tagtgtcccc gaactagact 29040

aacactgtgt atgaatgaac atgattttct ttcagatcat gtgttttctc ctagagaggt 29100
gtgctctttg ctgtgtcaga cagatagaat gaggagtttg attatctcaa tccagtgagg 29160
gattacatag accgctccct ccctctacta ggattctgtc ttcttacgga agttttctct 29220
tcccttttga cccagcctct tttttttttt tttttttttt ttttttgaga tggagtcttg 29280
ctctgtagcc caggctggag tgcagtggcg cgatcttggc tcttggctcg tcacaacctc 29340
tgccttccag gttcaagcga ttctcctgcc tccgcctccc tagttgctgg gattgcaggc 29400
gtgcaccacc atacccagct aattttttgc atttttagta gagacggggt ttcaccatgt 29460
tggccaagct ggtctcgaac tcctgacctc aagtaatctg cccgcctcag cttcccaaag 29520
tgctggtatt acaggtgtga gccaccgcgc ccagccttga cccagcctct ctatcatcct 29580
tcagtgttcc agttacattt ctgaggagat ataccactgg gaaagctgtc tatgtatcta 29640
gagcttttct agattccagt ctgtcatttc agcccacatg ggcatctaaa gcactactag 29700
tctcttgttt cctttattag agttcctctg cttaagccaa gctcagtcct tactcatgcc 29760
caagctctgc agttaccccc atggaagaac aagccagtgg tctcagctgc cttggaagtg 29820
ctcttccacc tctggatttt ttgttctttt agtcctcatt gcttccacag ctttcttacg 29880
ttggttagtc tggtctttga agcaggcgca ttggcctgcc tcctatctaa tcacttactt 29940
tagggaagtg gaagtcagaa gtttatcttt ataaaaagaa aaggcagata attttaggag 30000
tgcttatttt tataagcata gtgatgtttt gaatagtttt atctgtgttg catactcata 30060
gctataaagc atttttttcc tttctgatat gggcctgttt atcaaaatat gccccagatc 30120
tggggcgagc aaacaaagac ccacagacca atgaggccca attcttgttt ttatgtagcc 30180
tgtgaactaa aagtggtttt tacattttta aaaggttgaa aagaaaataa aaaaggaaga 30240
gaatgttttg tgagcattat gtgaaatttg gaagttttat tgtaatatat gcacactcct 30300
ctctttatgt gctgccgatg gctgctttcg tgctgtaacc attgagtagt tgcaacagag 30360
atcttgtgac tcacaaagtg taaaatattt gctgtgtggc cctttgcaga aaaaatttcc 30420
ctattcctgc tttacatcac aggctcagga atttttattt tcttattact aagagaaatt 30480
aagcaaaaga ttgaaaccat tttattccag aagttctgta aatttggatt tattaaaatc 30540
ttagcatttt agaggattta caaggaacct cagtctcctt catctcttta ttttgcaaat 30600
gaagcagatt tgagtgaaag aggttttaaa attatgacta catttttggt ataaagtata 30660
ctcagtaata agctgataga gaaactattg atctatatca ctgaataatt tgcaaaattg 30720
atttgttctt atattgcaga agtatttgat ttttcttaag cagtgtagct cttaagaaaa 30780
tatttttgga atttttaaag taaatatttt aagataaata tttaaggcat gttaatagcc 30840
ttttcctttt attgtcaacc acaattcttt caaatctgat ggatatgaag ttatttttgt 30900
gagaagactg aaagtcttac aggaccaatt ccagacattc ttcattaagc ttcgattgat 30960
ttagaatata aagccctaaa tcatcagaaa ttccaagtta aattgttaat gtgtaagact 31020
ggtttttaga gtttctcttt tatattccca tcctcagata ttgtgaaagg tttaagctta 31080

gttctcaaat atagagatat aatggtgtgt tatattacta gcaaatggca tttttaaatt 31140
ttccaatgtt gaatacaaat tttgtttata taattttcaa cttattactg gctcttttag 31200
aaaacttcta cctcttcctc tgcaaaccaa atcaacaaca acaacaaaaa acacagtaaa 31260
agtaatttat aattaatttg ggaatagcta gttcttggat ggcctgtact taagaattga 31320
tgggttcaga ttcataagaa ttgttacaac caattaaatg ccatcatgca tagaaagaag 31380
tattttagaa atgtacgtaa agtcatttga actttaaaga agttccatta gataaaacag 31440
acacaaacat tggtctctaa ttgggaaaat ttgagtcagt tataacttgt ctttaggaaa 31500
tctatctgaa tttggattta tatattctac ttctctgaaa attggtttgg aataaatttt 31560
gatgctttta taagttatat agaaggggct aacctcttag tagttatgta ttcctgttgc 31620
tcaaatacct gatttcactc aggtaatctt taatataaag gaaagttggt ttcctaaata 31680
gtatgaagaa tacgtattgt gttttattta tttatttgtt catctgatat ttattgacca 31740
tccctgatgc ttcacattga tgcaggtgct gaaagtacac agttgaaata gacattcatc 31800
atgccctcac aatgctagta gttgagtaga ttctataagc aggtatatgt atttatgttt 31860
agcatttggc actgttaatc tttctctttc tctttacttt tatggcatac tatcttgtgg 31920
aaatctcatc cacatacata gtttccatga cctcctttgt actaaagtct gtgcaaaata 31980
atcccaagga taactcctta gtgggccttt ctggctagag atgctttaaa tgcactcatg 32040
tcgataccat agatgtagat accttatttt aatctctgag taaggcatgt agctgtagct 32100
ctcaacttcc tcatttctcc ccttcctcac tctttctctt atactttctc tttctcttaa 32160
aatactcgtt gagtactata tcccaggaac ttttctggac agaggctaga gtagtgaaca 32220
agaaagacag ggcccctgca ctattggagc ttttagcttg gcgaggaagc aatacattaa 32280
acagttccat acataattac agttattggc tgtacaatga aacataaata tacgatgaat 32340
gagagtgtat attagggaaa ctcatccagt gtggggagtt agacttaagt gacacttatg 32400
ctgagacctc gaagataagt agtggttagc aggagaagac gtggtagaga atttcaggaa 32460
gagggaaaac ctttgtgaaa gttgtgatga agcttaacag gttcagacaa ctgaaagaat 32520
atccttcttg ctggagtatg atgaatcagg tggaaaagaa tgcaaaatga ggcaggagag 32580
attgatggag gtcagttatt ggaagaggat cttttaaacg atgtcggtgc ttttggactt 32640
tatcttaaga gaattgagaa gatactgatt tttaagctgg ggaattatcc actcaggttt 32700
gtgtctttaa aagttgagtt aggttcctat gtgaagaatg gattgaaggg tatggatgtg 32760
cagaaatgag ttaagctttt gcagtggtcc aggtaagagg tggtggtaac ctagcttatt 32820
agcattgcag cagtaaagat ggtgtggaca gattcaataa ctgtttatga gatataattg 32880
accagatttt gtgattattt tgatttcaag agtgaaactg aaggcaatgt caaggataac 32940
attttcatct gcttgttact cttgtctatt tgttgacatt ttggtatttt gaagttacac 33000
atcttcaaaa tcaaacccat tacttttttt ccagcaggct aactcttcct tctaacttct 33060
ctatttttaa gagtgacatt gtctacctcc aagtgaccta ggcttaaaat ttttctcttt 33120

tgtcactgct gttactaaat tagttaccaa atcttaagga cttgacccct tcagcatctg 33180
gaacaacctc ctccccttcg ttctccctgc attccctctt ccccccgact tcatcctttt 33240
ttctttatta cagcagtagt atcctcttgt tagatcacta aactgtgctt tccattcaag 33300
tctctgagct ccttggggac aagaactgtc tttttcattt tgtgtcttta ctactttgca 33360
tctcagcatg gtgaacataa aacatattaa gtgaaatgaa aataaaatgt tttccttgct 33420
tcttgtttct tgcaccataa ttcttattct ttatcacata tttttttctg tttctcaaaa 33480
gttattcagg ggcttccttt tgcttaaaga ataatttggt cttaggacta tgtgataatg 33540
aagtatccag actggctgct ggagacataa agtatatgag ctggggattt tcctatagta 33600
gtttagtaaa aagtggctct taagtccctt tgcaccgtag ccctcactac caaaaagagc 33660
agattttttt tcttggaaac atcactgaca tttgtaaaca tcccactatg gataaagtat 33720
aaaacacttc atttgatgcc tttttaatgt tttaagttta tgtttttgca gaattcaaat 33780
ttgtattatc atttaatata tattgtatat tgcttagctg ccccatgtta tcttctcaat 33840
agctacttta tatgttagta tcttatatgt gtatgtaaag tatattgttt cacataaaga 33900
aattttgcaa gaaaaagatc caaatgtcta ctttggaaat acccattttt gatagctttg 33960
tatgtcctaa tatgaacatt catatttttt tcccattttt cttttaatat ttaggaataa 34020
gaaaatttta aataagaaga aattgaaaag aaaacagaag agcaaatcaa aagtgaagac 34080
aagaagcaag gtaaagctgc tggtaattga taaggaatag gactttttca tatggtgatt 34140
tgaataaaaa tgccaagatt aaaaaatata attaaaccat tttattagta tgtgctgaag 34200
taattttgta aaagcccttt atgatttggt tcctctttta gtttcatctt tcttttgggt 34260
gtttcttttc tttcttgcct tttttttttc ttttttgaaa cagggtcttg ttttgtcacc 34320
caggctggag tccagtagtt tcctgggctc aagtgatcct tccacctcag cctcctgagt 34380
agctgggact acaagtaaat gccaccacaa ccagctaatt gttaaatttt ttgtagagac 34440
agggtctcac ggtattggcc aggcttgtct cgaactcgtg ggctcaagca atcctctcac 34500
ctcggcctcc caaagtactg acattgcaga catgagccac tgtgcccagc ctatttctgg 34560
gattttcttg accagtagcc tggaagttaa tatctggatg ttgaaaatta tttgctattt 34620
attcttatta tctttgatca gtcctttggg gaacatatcc atttctgttc tcttttttca 34680
atctgactac aggtactaga aacttgagta aaattagctt gtaaactggg acttggattt 34740
gggttcatca tatgcctttc ttgacaggaa ttagggaaac ttggtgtgtc catattgttc 34800
tgagcctctt ttgactgttg gtagctctgc aaaagaaaag gcaatatgga atatttgaat 34860
taggtgagac cttagaaacc aagaatctat tttcacctaa tgcagagatc tctataaaat 34920
ccatcttttt tatactgaac attttcagtg accagagcac ttgatataac agtctctttt 34980
ctattgaaca tctagacaga aggaagagag gagtcgtaac tatgattcct gaatagcttc 35040
actttgagtt aaagaaacaa tagatttgct gggcctgtat agcaactata gaggcaattg 35100
tttcctacac ttttaagtta gtgtttttac tattgtaact gagagaaatc tacttcaaag 35160

tagcaaacct aaaataaaat gaaaggttgg gggatggcat ttatttggtg gctctatctt 35220
gagacacaga tggatccggg gatgaaatga tatcattggc tgggtgcagt ggctcacgcc 35280
tgtaatcccc gcactttggg aggccgaggc tggcagatca cgaggtcaag aggtcgagac 35340
catcctggcc aacatggtga aaccctgtct ctacgaaaaa tacaaaaatt agctgggcgt 35400
ggtggtgggt gcctgtagtc ccagctactc aggaggctga ggcaggagat tgcttgaacc 35460
cgggaggagc aggttgcagt gagctgagat cgcaccactg cactccagcc tggcgacaga 35520
gtaagactcc gtctcaaaaa taaataaata aataaaaata atcaaaccca tattttcagc 35580
tcttgcttct gccttcctct gtgtgtggac ttattacctg tgactgaggc tgaggctgcc 35640
agtagctcca actccatttg ctctgagcaa gaggcaatct cttatactag tcatagcaga 35700
aagtcttggg taggattttc ggtttacctg catcatttgc tttgaccacg gagatacggg 35760
atactttggt tagactgagt tatattttta accctgagat gggcagtggg tgagcaccct 35820
ggtgaatagg ccacatgaaa tgagggagga atagttaccc taaggaaagt gttgccgggt 35880
aggcagaaaa ataatctgtt cactgtatcc cttctcatta cttctgcttc tgggatagag 35940
gagggaagtg gagcgcattg tcaatataca gaaaacctct aacaaataca gtcattccaa 36000
cctctatccc ttttcaactt tttggtgcta tgtgcaaatc ggagtatggg aatcttgaat 36060
atcttttaag tttttttaag gtttcatttg gtgattttcc tgaagtggtg gcatatgata 36120
tccaggaaga ggtgaggaga agtcaaggtc ttttgtcccc ttcaattcgg cctccaacta 36180
gctttgtgcc cttagtaatt cagttttttt cctcttgctc tcagtatttc tatatcttct 36240
ttttaagagt agtttgagat aacagtgatg taagtaagtt cattcattta ttcctcaaac 36300
attaattgca tgctgagtat gtgcaggcat tatgctggat gtttgggata caaagatgag 36360
taagaggcat tttttgctca tggaaagcat ataaagtagt aaaatggtgt tgttagtctc 36420
cttgacctgt attacttttt ccagattatt tttcatctac cctctttctg aaagttattt 36480
ttaaaatgcc atttagctgg cattttatct actttatatc tactttatat atattttata 36540
tatatgtata tatatatgta tatatatata tatatatata tatagccatt atatctactt 36600
tatggctatc atcaacccct ctatttctcc tcatccatca gtcatctcct ttatttaatt 36660
tccatcttac ccaggttaga taccagtctt tctttttaga aaaactctta tttaatctgt 36720
aacaaattac aaaaacttga tggtttaaat taacactcgt tatcttacag ttctggaaat 36780
ctgaagtcca aaatggatct cactgagcta aaatcaaggt gtcagcaagg ctttattcct 36840
tctggaggct ctaggaggga atctgttttt ttgcctttcc agtttctata gtagaggctg 36900
ctcagatgtc ttgggttgtg gcccccttcc atcttcaaag ccagtggcgg ttggttgagt 36960
tttctcatgc ggcatcactg atagttttct gttgttttat ctcctcctct tactgtccag 37020
cctccctctt tcacttgtaa ggacccttgt gattacattg gcccatccag ataatctgtg 37080
catttcaaaa tccttagttt aatcacatat acagagtccc ttttgccatg taaggtaata 37140
ttcacctggg ttctggggaa ttagggtata gaagtttttg gaggctatta tttacctacc 37200

atatctccct tgtatatttg ttgcattaat ctgacaaaac tcaatcttgt gtgaatccag 37260
ttattttgtc cctcccttaa ggtgagttcc atgtgctgcc aaagagagtt acacaagagt 37320
taactgataa tgatacacat tcatgatcat caatctcaag tataccatag ccagactatc 37380
ttattatttt ttctggtcaa atagttctct tttcttctgt gattatttca gacctttgcc 37440
atgctcctca aacctctgat aaattctacc ttccagcaca tgacttcact ttcttacagg 37500
agaaatcaaa ttcattactc agaaaagttc ctcaacttcc taatcccaaa tgcgtctgtc 37560
ttcaggcttc tcagtagctg agcctgacat agagatttga agtttgcagt tcagtaagtt 37620
ttgaaagtta acattcatgc atagaagcac tactgcagtc aaagttcaga acatttctat 37680
catctccagg ggttttcttt ttttttcttt cttttttttt tttttttttt gagacggagt 37740
cttgctctgt ctcccaggct ggagtgcagt ggtgtgcgat ctcagctcac tgcagcttct 37800
acctcctggg ttcaagcaat tccctgcctc agcctcctga gtagctggga ttacaggcac 37860
cagccaccac gtccagctaa tttttgtatt tttagtagag acaagatttc accatgttgg 37920
ccaggctgtc tcaaactcct gacctcaggt gaaccacccg ccttggcctc ccaaagtgct 37980
gggattacag gcacaagcca ccgtgcctgg ccggttttct taccctctgc tgattccatc 38040
cctccctata accctggctg tagacaacca cagatatatt ttctgtcact gtagattagt 38100
tttcattttc tagaacttca tataaatgtg atcatattgt gagtgctctt ttttttttaa 38160
atctggtgtc tttccctcag cataatgatt tgtgttaatg ttgttgtata tcaatagttc 38220
attccatttt tttgctgatt ttatatacat atatatgcac ttgtgtgtgt atgtgtgtgt 38280
gttcagtttt cttgggtgca tactgaggag tggaatgttt aggtggtttt tcctttgatt 38340
tgccagaagg actcataaat ctcacaagca atttatactc atggctaaga tttattacaa 38400
caaaggatac agagcaagca gcaggaaaaa ggtatgtgtt tgtgatgtat aggaagttat 38460
ggcataggct tcctagttct tcattgactg agctttcatg tcagaaagaa agacatgctt 38520
tctctctggc agtgaactac agagaaatgt gtatattgtt tttgctcagg tgagttttag 38580
aattcaagac tttgtgggtt tggtcacaca gacatatctt gttagtcaat cagacatggt 38640
aagtgaaact caggtacaca ttgtaaagct tgatgttcgt acatgcagag cagtctgaca 38700
ggccaggagg catggtccat tgctctgtgt gttcataaca caatcatcaa tcactaccac 38760
aaagaaaact ctaaacatcc acattcccaa aggttagcca agggtcaatc atggtttcct 38820
tggggaaata caaggagtaa gcaatcatgc ctgcagcgtt aactttttcc tcagagtggg 38880
cacggtaaat gtacgtttag tttagctttt tttttttttt tgagacagag tttcgctctt 38940
gttccgcagg ttagagtgca atggcacgat ctcggctcat tgcaacctcc gcctcccagg 39000
ttcaagcaat tctcctgcct caggctcccg aggagctggg attacaggca tgcaccgcca 39060
cgcccggcta atttgtattt tttagtagag acggggtttc tccatgtgga ggctggtctc 39120
gaactcctga cctcaggtga tctgcccgcc ttggcctccc aaagtgctgg gattacaggc 39180
gtgagtcact gcacccggcc tgtacattta gctttttaag aaactagaaa actcttttcc 39240

aaagtggttg ttccaatctg tagtcccacc agtggtatat gaccaatcta gttgcttcat 39300
atctttgcaa acatatggta ttcgcttttt aaattttagt cagcctacta agtaggggtt 39360
tcccattgtg gttttagctt gcttttctct aatgactaat gatgtgaaat cttttcatgt 39420
accttttgcc attcttattt ttttgatgtg aaatgtctgt tcagatcttg cctcttattt 39480
ttgtaattgt cttcttctta ttgaattgcc agagtttctc atatattcta aatacaagtc 39540
ttttgtcaga tatctgcatt ccaaatacat ttctcctgtt ctgtggcttg cttttggggg 39600
aagaattgcc atttaatgga tattgaattt tccaatccat gaacatggta tatgtctcca 39660
tttatttaag tctttaaatt tcactcagca acactttttt gttttcagtg tgtagggctt 39720
acacatattt tattactttt atttacaatt attatatatt tttgatgtta ctgtaaatgc 39780
agttattttt gaaaatttat tttgctttga gatgatttca gtatgaatgc agtgtgtatg 39840
tgtttgtgtg cacctaagtc tgtgtaattt tatcacatgt gtaggtccat gtgactctta 39900
ccacagtctg gatatacagc agttccatca caaggattct ctgtgatatc ctttatagcc 39960
acaggtatct tccttcctca ccctctcctt agcttctggc aaccactgat ctgttcagca 40020
tttataattt tgccatttta agaatgttgt ccagtgaaat tgtacagtat gtaatcattt 40080
aagattggtg cttttttccc ccacttggca aaatgtcttt gagattcaca cacattgttg 40140
catattgttt gttccttgat gagtaagcat ttcatggtat ggatgtacta tagtttgttt 40200
aaccatttac ccattaaaag atatctgagt tgtttctagc ttttgactat tttaaaagaa 40260
tgctattacg aacattcgtg tactggtttt tgtgtgaaca taagttttta tttctctggt 40320
gtaaatgtcc aagaacataa ttgctggatt atatggtaag cacatattta gttttgtagg 40380
aaattgctgt actttcttcc agagtggctg taccatttta catgaccatc agcaatgtat 40440
aagtttctcc tcatccccac cagcatttgg tgttgtcact atttatttta gccattctga 40500
taggtgtgta ctatatctca ttttaatttg cattttccta gtagctaatg atgttgaaat 40560
tcttttcatg tgcttattca ccctttgtgt aaatatcttc tatattgatg tgtttattcc 40620
tatcttttgc ccatttgtta attggattcc ttgtttgttt ttactgttga cttttgagaa 40680
ttctttatat attctgaata ctcatccttt gttggatatg tggtttgcaa atattttctc 40740
ccagtctgtg acttcaatgc tattattttt gaaattacat tttttcagtt gttcattcct 40800
agtatgtgga tatactactc atttttatat agtaatttta tatcctgtga ttttgttaaa 40860
tctgcatact atttctggta acttttttgc agattcctta agattctcta catgtttttt 40920
tacaagtaaa aacatttctt ttttagaaaa tacttcttgc ctttttccct tggctggata 40980
ggatattttc cttgcactag ataggatatc cagtacaatg ttgatgagtt gctgagagta 41040
gaccaatttg cctggatcac aatcttagag ggaaagcatt cagtctatta ccatgttagc 41100
tgtggttatt ttgtagatac cctttatcaa gttgagaaaa ttcccttcta ttcctaactt 41160
agactttttt tttttctatc atgaatcgat actggatttt tgtcaagtga tctttctgtg 41220
tttatttgaa gtataatata ttttttctct gttacaacag tggttgcttt tccagtgttg 41280

tgccagcatt gtccctgggt taaacctcag ttggtcataa tgtattactt ttttatatat 41340
tgttggattt gaatagctaa tacttgtaat atctctggat atttggatga gatatttgta 41400
atatctctta tacatatatt cataagttat atgccatgcc aatttaactt tttgatgtat 41460
ttcttattta acaagtgtat tttttttttc caattcttta tggaagaaga gtcgaatatt 41520
tgggaccaag tgtttttggt acttgaggta tcattttgtt tatcttagat acttcctgga 41580
catactctaa ttattggaag ttgaatttct aaaatatttt aaaacagctt tttatattta 41640
taatataaat gttgatcttg aaattgtagt tgctgtctga tgaataaaat attgggcata 41700
aaagagaaac tgtttagtct taacaaatta ccaatcacat gatttttagc ctttatcaat 41760
actgataggt gagagagagc ttagggaagc atctacagtt tacttggcat tactgttact 41820
tgaatgagaa tgaaatgagc tgatgaaaat taagtgtttt tttgggaggc cctcatttct 41880
gtgtaaactt ctatatctac ttttaaaagc attcaaaatg caatctaatg tttgtagtag 41940
gtcattgaga ctctacagtg tgtctagagt ctcttaggaa gtcgtaaaat gaatttcctt 42000
tgatacagaa ctctaagagt taagctttgt tgagtctatt cctgtcatgg cgatacaaga 42060
atatttctaa gttttttgcc catccttttc cagcccttgt cagattggtt ggttattgct 42120
gcattgctca aaaaatagtg aggtatagaa aaggggacta gaagttgggt ccatagactt 42180
agactgtctc tgctgtgtca cataatgtgt cttctgcaag tcagttagtg tgtctaatct 42240
ttacttttat gtaaaatggg acttgatgat agaaaagagt gtgaaatgga aatattctgt 42300
tttgtatttc agtctgaaaa cttagagaat acagtaatca taccagatat caaactacat 42360
agcaatcctt ctgctttcaa tatttactgt aatgtacgcc attgcgttct ggaatggcag 42420
aaaaaggaaa tatcattggc agccgcatct aagaactctg tgcagagtgg agaatcagat 42480
agtgatgaag aagaggaatc caaagagccc cctatcaagc ttccaaaggt aagccactga 42540
gttctattaa tatttagatg tgtaacctgc aggtgttctg gctataatgc atatatacgc 42600
attgctaaaa tactttgctt tatgtaaaat tgcacactaa aaataacacg gcttatgggc 42660
aaaaatagat ttggagcaga aaaggaaaac tctgcaactt tataactaag gtgctaacaa 42720
agtcaataat tgataattca ctggaaagcg caggaaggga gatcagtggg ttggagtctg 42780
ttataggggc aattaaaaaa tgtacaaaag cagtagggtg gaaatgctag tatagaggca 42840
ttgagccatg gcagatggaa gtagagctct gagccagtga gccgagagta aggtgggcac 42900
aagagaaagc caagagccct ggaaagctgg gaggtgctcc tcacctggga tgcaggtatg 42960
cgtttttatt tttcttgaaa tagaactaca aagcctctca gctgtgtagt agcagggctg 43020
agagcctttt cctttctgcc taagcttata attgtactct tgatattgtg gtttcccttg 43080
attaggaaaa aaaaaaatca cctatgaacc aactgaactt ctgcattatt ctgatattac 43140
tcccttattt accaggagca tataaactag ttggtatttc tataatagaa gcatgtattg 43200
taggccgggc gcagtggctc tcacacctgt aatcccagca ctttgggagg ccaaggcggg 43260
tggatcacct gaggtcagga gtttgagaac agcctggcca acatggtgaa accctgtctc 43320

tactaaaaat acaaaaatta gcccggcatt gtggcagtcg cctataatcc cagctactca 43380
gaggctgagg caggaaaatt gcttgaaccc aggaggcgga ggttgcagtg agccgagatc 43440
atgccattgc actccggcct gggtgacaga gtgagactgt ctcaaaaaaa aaaaaagaaa 43500
ctggctgggc gtaatggctc acgcctgtaa tcccagctct ttgggaggcc aaggcgggcg 43560
gatcacgagg tcaggagatc gagaccatcc tggctaacac ggtgaaaccc cgtctgtact 43620
aaaaacacaa aaaaacttta gcccgtcacg gtggcgggcg cctgtagtcc cagctactcg 43680
ggaggctgag gcaggagaat ggcgtgaacc cgggaggcgg agcttgcagt gagccgagat 43740
ggcgccactg cactccagcc tggacaacag agctagactc catttaccaa aacaaaaaac 43800
ctgtattata gaactactct cagtttactc tgttctgttt aagtattggt atatttaaga 43860
ctggaaggtc tatatatata tatatatata tatatatatt tttttttttt tttttttttt 43920
tttttttttt tttgagacgg agtctagctc tgttgcccag gctggagtgc agtggtgcca 43980
tctcggctca ctgcaagctc cgcctcccag gttcaggcca ttctcctgcc tcagcctccc 44040
aagtagctgg gactacaggc acctgccacc gcgcccggct aatttttttt tgtattttta 44100
gtagagacgg ggtttcaccg tgttagccgg gatggtctcg atctcctgac ctcgtgatcc 44160
gcctgcctcg gcctcccaac gtgctgggat tacaggcgtg agccactgca cccagcctgt 44220
tatatatatt ttttctatat agtagatata gttaatattt acaacaaaag aataggtgaa 44280
aatatagaca tttttaaatg gtgcctttat atgacttatg catattgctt ttttctaaac 44340
agagctattc aaaatgattt tataattata aatgatttat gactattgcc tttttactaa 44400
actgatacct taaaattatg ccttgaaagc ttatacatat ccacaaggga ttgtgtaaat 44460
attttttact acttggaaat agcagcataa atggaacctg gtctgagttc ttttgcacat 44520
tttatggtgg ctgttcctaa gtacaccacc atccttaatc atattgtcag gttcttctca 44580
ccttattctc ggcagatggc ttgcttcata tttcatagag taatacaagt tttgtgaact 44640
tactcagctt cttgatcagc catcttcaca tgtccttttt gcctcctgtt ttagagtggg 44700
acccttttct ttttcttatt tcctttgtct gcaatgtcct aactgtacct actctaatag 44760
ctccttcctt ccttcctttg ctttcctttg ctttcctttc ctttcctttc ctttcctttc 44820
ctttcctttc ccttcctttc tccttttttc ttttcccttt ccttttccct tttcctttgc 44880
ctttcccttt cctttccttt ctttcacgga gttttgctct tgctgtccag gctggagtgc 44940
aatggcatga tcttggctta ctgaaacctg tgcctcctgg gttcaagcaa ttctcctgcc 45000
tcagcctcct gagtagctgg gattacaggc atgtgccacc gcgcctggct aattttgtat 45060
ttttattaga gatggggttt ctccatgttg gtaaagctgg tctcaaactc tggacctcag 45120
gtgatctacc caccttggcc tcccaaagtg ctgggattac aggtgtgagt caccgtgcct 45180
ggccaatagc ttctttcttt aacctaaaac aaacatgttt atctttaata tgggagctgc 45240
tctacataga aattatggtc tctaattgta atttcttatg cttaactagg agagattgag 45300
ataaaaaaca tagttgagtt aggacgtagg gctggaggac ttgtataatt tactttttag 45360

ttagaatacc aattctgtgt atgtgtgagt cacactgtat tatagtaatt gtgctactta 45420
agttcaatat tgtgagagaa aacaaaagcc tgggtaaatt ttttacacag gtatgcagat 45480
tttgaatagt aaactgtagt tcaaattaaa ttgcataaac aagaacaatg gattcctagg 45540
gtgtttaaaa acatcaggat tagtgagtat taaatgaaat atcaggcatt cttaagataa 45600
ccttttgcag cataattaac agagtcaaag gggtatcttt caaagaaaat taaagaagat 45660
cagatagccc aagtgattaa tactgaattt tctaccaagt actgacctgt ctgcaaacag 45720
ggttagcatg ggttatggag ccaaactgct ggggttcaaa caccattaaa attactagct 45780
atgtgacact ggaagttatt tgacacatcc gtgcctcagt tttgtcatct atagattggg 45840
ggttgcatct acctcatagg gttgtagtga caatttaaat gaattaatgg atgttgagtg 45900
cttgagagaa cagtacctgg cagaaagaag tgctcggtaa atgatatata tagttgtaag 45960
tgtcagttta ggtgcaaaca agaaaatgtg taggtgcaaa tgaatttgag ttacttgtca 46020
aggaactctt cagtttatta aaaaaattat gcaagcaaat gaaacttcta gagaatcagt 46080
gcaatcacct gatgaatgag aggtaaagag aggaagaagt gatgagatgc ctctatgcag 46140
aggcaagcag atactatggg tggggagtga aactttcagg tgatattttt tggcagccat 46200
ttccatgaca tgaaggattg acactgagga cgaaattagt ctcttaaggt ttgaggtgat 46260
gggatttgaa ggcaagtggg aaatgttggc atttttcttt tccctttgac cttctctgtg 46320
cctcagcccc aaatgctgct attatcctag tttgctcctt ttctttgttc ctctgttgct 46380
cctcctaagt gtgtggctgt cccattcctt cttgcctgag gtttctgttt cttgtactct 46440
tttttttttt tccttaacat gattaagaca gtttttaatc tgttgttgaa gtgttgttgt 46500
aatacagttt tatttttccc attcttgagt tttaaattat tatttagtta ctaataaatt 46560
gagtttgact gttatataga ccttgattgt cacccttttg ctttttgctt ttgtttttgt 46620
gttttgcgga tcagtaatca ttgatgtatt taaacgagct ttctattccc tgaatgcttc 46680
tacattaagt tttttaacca gctcaaactg tcactaaaat ctacatcctc taaaacttta 46740
cagattttat agctacataa tttttattat ttttgttttt attttcattt tgagacaggg 46800
tctggctctg tcactggggc tggagtgcag tggcgtgatc tcagctcacc gttaacttcc 46860
gcctcctggg ctcaagtgat catcccacat tagcctccca tgtagctggg actacaggtg 46920
tttgccacca ccccaggata ctttttgtat ttttagtaga gatggggttt tgccagattg 46980
cccaggctgg tctcgaactc ttgagctcaa gcagtctgac cgcctcagcc ttccaaagta 47040
ctgggattac aggcaggagt caccgtgcct ggtgcataat ttttaaatag taacgcttag 47100
aagtcatatt tcccatctga acccatttga agccagtcat gccacattat taaaagattt 47160
agacttacct aggatcattt ctttcttaat tcagtatatt ctttttgaat ttgtagccag 47220
cattatatta ggtcctgaga acaaaaggat gagacataat tcctgccctc aagaaagcat 47280
tgtcttgtag ggataagaac caggtcaata aatattctct agtgccttaa ggattgatag 47340
ggagtgctag atatagtgga acacgttaga gaaaggactc aacactggct gagatagagc 47400

aagagagaga gagcaggaag ctaaatcatg aagggcctta tgtgccatgt aattgattca 47460
gatgttatct cataattagt aaatattgaa gattttaagc aaaagaatat catcagactt 47520
gcatgattgc atcagactca ccaccctgta gttatgaata tgccttacta acactggagt 47580
aactttgtac tccagtaagt aagtaacttt gtactccagt tagtaagtaa ctttgtactc 47640
cggttagtaa ggcatatcca taactacagg gtggtgagtg agactggaga cagaggaacc 47700
atttaggcag ttattgcagt tgtctagaga gtgttaatga gggttggaat tatggtagtg 47760
acagtgtttt gaatatagga aatgttaagc agaattgaca ggaattagta ataaattggc 47820
tataacgact gaaaaagaag gcagaatcta aagatattct taaaggtttt gtgtggtgag 47880
ggcctttact gaaagaggga atactggatg aggaacagtg cttggctgaa atgatgatca 47940
agtttagttc acatgctgat tctgaggtaa atgtgaagta tccagtgtgt gctatgtagg 48000
gctgaagttc aaaaaataag tgtcagctag aaaaatagat ttgggagtta tcactatgta 48060
ggcacaagtt gaaacaaatt tctgtgagag atcggagggg gtgggatcaa gaacattgtt 48120
tgaaaaaata gctttgaaaa gttgaaaagt tttgaaaagt tctcttcctc tgagtgtgaa 48180
gagaagggaa gaagatggta gtagtcatag ataaatttgt taggcaagga actcagaaac 48240
ttcacactca tactttcttt cttttttttt tttttttcaa gatggagttt cactctgctt 48300
gcccaggctg aagtgcagtg gcacgatctt ggctcactgc agcctccacc tcctgggttc 48360
aagtgattct cctgcctcag cctcccaagt agctgggatt acaggcatgc gccaccatgc 48420
ctggctaatt ttgtattttt agtagagatg gggtttcacc atgttggcta ggctggtttg 48480
caactcctga cctcaggtga tctcccaacc ttggcctccc aaagtgctgg gattataggt 48540
gtgagctacc acacccggcc tcacattcag aattctccat cttagccttc tcctcctctc 48600
tttctgctgc tgcctcttcc ccctcctttt ttctctgtct ctagattttt aaatttttta 48660
ttatttgtag atattatctc tgtaccaaat atttgcttta tacaaacaat tttaaaagac 48720
agtatacttc actctatggt aatatgtaaa tataattttg atttcctttt gagctttttc 48780
aaatataaaa gtataggaag tgattttagg accttttaac taaacaagat acaattttta 48840
agggtaaaga attaattatt aaatccaaat gtgatgtaac taaactttta tgatcacact 48900
tagtcatgaa ataataaagt cacgtttgga aatatatgat ttgagaaggg caaaataaaa 48960
ccttgaaaga ctctttatat atcatttttc tttggccaaa tatattttta tctcactttc 49020
ctagcatatt agactggctg tatcaggtaa ttatttgggc ttcatgtttc tttatctttc 49080
aaatatgata gataaccttc aaaatttaat tatttgagag cagcagtaaa ggtaaaattc 49140
agtaaattta caaaagacta ttttctgaag agaagtgcga aagcatgtgt tgggtaatta 49200
gttatagttc gttatggaaa gtattgaagc ttcttgtgtt cttttattgt tataattatt 49260
atttttatta aatatcacat ttatttattt tgctaattat acatatttat tgtagatgat 49320
ttgaaaaatc agaacatttt tctaaagagg tattaaaaat cggaagtaat atttgaaata 49380
ccattaacca aaatataccc caatataaac atttttattt cttttgtcat tttttctatg 49440

tacacattaa aatgtttttt acacacaaat gaaataatac tatattggca ggactatatt 49500
ttttctcctt acactcttcc ttcctgatac tgaaggaggg agaaatatgt gttttcattg 49560
actataagac ttcaaactgt tttaaagtct actttcaaag ttcaaacttt tggtaaaata 49620
ctttatactt agccaaacag cagaaaaaga cttatttaag aacatgtctg ttacctctgt 49680
gcaataatcc atagctaaat attaaaaatt ctttaatagt tgcagaactt gaggttgatt 49740
tttgttgtgt attgaatata tattactatt ctgaaaatga atgttttccc atttgttaaa 49800
caaaaagtct acaatgaacc aagatgtgga taattagtga gactctcaca gtgtttaacc 49860
cagacagatg aagagttccc tatcatcatt gtgaccaaag ttttatgggc cagtctcact 49920
aaaaccggct agctctgttt ctaggcagcc cacctgagtt gcctattgcc attcacttgt 49980
tattacacct taaacttacc agagagatca agaacctgtt cattaggcag tcttactgtt 50040
tctggcaagg ttcaataatt ccgttgtcac cgtaggaaac tgtatctata ataaatgaac 50100
cttagccaaa aatgtcatct gtgaaaaatc ctcttggatt tatgaaaaat catggttggt 50160
agcagagtaa gcaatctgaa agaataacag aatatagtag tcttccattg gcttgagaat 50220
agagccccat attttgtact ctggagatct ttgacttcac atttctcttt cctgtaagta 50280
cataagcaga tggaaatctt ggacagcatg ttcttgtttt ctagtccatt ccaggaaaag 50340
aaactatatc aaatgtgaaa gtttaatcac ttaatgtgtt taatcaattg aaactctatg 50400
cagactcttc tgtaatatta gctataatca agtctgttag cattttagat aacttctcca 50460
aagacagtcc tcctagttat ttgacttctt agctatatct gttgtttcca gaaaacttgt 50520
aaatgttaat cagaactaca attgacttac cttcagccga cttgcttctg gtttagaaaa 50580
tgtatttctg agatgcagtg gttttttagc aaacaattta gtgaataaat taaacaaata 50640
tttaatgtgt gcctaccata tgctaggtaa ttggctaggc atgggaggta ttaagccgtt 50700
taagacatgt tccttgtata ttggaagata atacaagccg tttaagacat gttccttgta 50760
tattggaaga tcccaacatg gttgggaaaa tgaaaacaaa aatagttata ctgtaatgta 50820
ataaatgcta taacagaggt ctgtattgag tatgaattat atagcatgac taatttaccc 50880
tattagatta taaactctta gaggacaggg tctagtatac cttcatattt cctaaagtga 50940
gtttgcacaa tatgagtgct taggaggtat ttatgtaata tatgaattaa tgcaaaaata 51000
tcagcagaga gaatattctt ccactgttgt gttcaataga attaatggtt ttgggagttt 51060
ctactaatta gtcttatttc aatttttctg ttacttctcc aaacaattta attaatactt 51120
cttgaactgg ttctcagaat gttgacattg cctgagaaat ggaatttgtc ttttgtttta 51180
tcagtcttat gaaggtatat ccttaaactc attcattgca atttgctctc tcaatcttta 51240
gtaacaaatt tatctctttt gctggatata gagtctgatt tttcttgcac ctgatgagga 51300
atagtaagat aattaaagga aaagtaccag tgatattttt agctaagcag ttttaaattg 51360
ttttgtgatc atcctacaag aagggatacc aactataaat aataatatag tgattgtacc 51420
attatagaaa aggctaagac gaaaagctcc ttgtaaaatc tttttactat atgtgctgtg 51480

ttgactttat ttctggttat aggtaacaat taacatgact tttgacaaaa agagtactga 51540
attttccttg agtacaatca aatgaacttt gcaaattaat atagtttatt tcttctgagt 51600
gaatgcaatc tagtctctag ttcattgtta taagccattt tcatgatgtt ttattatggc 51660
tccattatac atatacatta tcatttgctg tggaagtaac ttgtaccaac taaactgctt 51720
agaaagtgtt actagcttac atgtcataaa gtatatatat tttttccttt taaatagatt 51780
attgaggttg gcctttgtga agtttttgaa ttgatcaaag agacacgatt ttctcatcca 51840
tccctgtgtc tcaggagtct ccaagccctg ctcaacgtgc tgcagggcca gcagccagaa 51900
ggcctccagt ctgagccacc tgaggtccta ggtaagagcc aaggctcatt cagtgaagca 51960
tttaaaagtg aatataatta ataatagagt attgtacact tcaaaattgt taagagaata 52020
tttctaaagt tctcactaca gaaaatgtta agtacttgag gtgatagatg ttaattagtc 52080
cacattttat tcatgaatca taacatcact ttatacccca taaatttata taattataaa 52140
ttgtcaattt gcagttttta aaaagtgaag agcttctctt atgaagcaca cacaatagcc 52200
tgtaatgcag tctttattta attgtggtac ggaaacagca ggggtataag ttctagagta 52260
tttttttctg tgttccactg aagttatggt ttaagagttg aagttcgtgc acacacattt 52320
ctcaattagt atgatgaagc ggggggcaaa caaaacataa atccctggaa tccagaaata 52380
atcctcaaac cttaaggtca ctattaactg aaatcgttct tattaaactt ttggccaaaa 52440
tagctaacat tcactttctc taacttgtta ctctcaatag ttttaaaaaa caggggaaaa 52500
ggaaataaat gccacaaagc caaaacagta caattagttt ctaaattgag gggacttttt 52560
tttgtattca tcataaataa aaatctattt gtgttatgga ttaaacttct gacttagcaa 52620
ctttcattaa gtaaataaag tgttcgcttt tcttaaagta tgttccgatt cttgcctttc 52680
atatcagtga cccagactca acatgtaatc ctttaaataa gataggagct tttacttaaa 52740
aggtactaag gaatagaaat atagatggaa gcataatttt taactggtta attgcttttt 52800
ttctccgatg ctaactatgg gtctttaaac tatctaaagt tttctagatc cttctattat 52860
tataggaaaa ttctcagaag tactagagct gtggttctca ttatgtggtt ctcaattctc 52920
attagcatgt atcagagtta tcggggactt ccctatggtg tttctgattc catagacttg 52980
gggtggggcc caaaagtttg tatttggaat tagtttcttg gtgatacaga tgctggtttg 53040
aggagtacac tgctggttta gactaaaact tggtcctttt tttccgactt ggctatttag 53100
attttgtagg acccacctgt gagaaccaaa ctgcatgaac ctaactacat gtcagatttt 53160
tcttttttgg tcgcatgtgg ggactcagtt attgtgtttg acttttgttt gttttaatga 53220
gagggaggat gctggtcatt gaatcaagct gaggattttt taaaaccacc cccctcacat 53280
acacagttat taaaagctat taccaaatag aagctgtcat gttggcaggc aagcaccgtg 53340
ttggttattg ttatgatagc accagtgatc atattaatat tgtgaagtgc tgcttggctt 53400
gacagcattt tactgaagat ttgatttatt ttatttaaca atattttgct cggttttctt 53460
ggatttggtt tgttagttag caaataattg gaatgttgaa atacccgcga agacttggat 53520

gtcgtatttc ctactttgaa catgttattt ctttcctcct aagtcttcta ccattctcat 53580
gtctcatgtc agttgttata tactttcctt tgtatccatt ctgtacatac tttctttcct 53640
ttattctttt aaaactagaa cttgttctta atactggctt tggcatataa tatgtgcttt 53700
agtcttatct tccatcaaaa ctttcacatt attggctggg tgtggtggct tatgcctgta 53760
atcccagcac tttgggaggc tgaggcagga ggatagctta aggctgggag tttgggacca 53820
gtctgggcaa cttagacccc tatctctcca aaaaaaaaaa aaaaaaaaaa aactgggcat 53880
ggaaatgagt gctactcaag aggctaaggt gggaggaccg ctcgagccca ggagtctgag 53940
gctacagtcg gctgtgatca tgccactgcg ctgcactcta gctgggtgac agaatgagac 54000
ctcatctcta ccaaaaaaaa aacccccaaa aaatcccaca aaacccaaaa atatttcacc 54060
ttattaatct gttcattttt attttcttgt ttaaagtttc aaattctgct gtactgcctt 54120
tcgtctgtct tccatccttt cagtgtttac tgctgatctc gtcactgctt tggcttattt 54180
ttctatgtaa gggatttatt atcacttcat gttttctatt ttgttaaatt agctcaaaga 54240
taaaagcact gatttggttc ttttatacta aatttctttt gaaacttgcc tctcctcctt 54300
tttttaatct aatatcacaa tcttattggt tatccttttt ttcccattcg gttgttttcg 54360
tgttattcat gaaaatatgt gtatatttat ataactctta ctgtggaaca tcttgatgat 54420
tagtatacac caagcttctt ctctttgcaa atagattttg atttttaaat tatctattta 54480
aaaatatatt tccttatatc catatattct gttcaattag attatattct cgagtactca 54540
ctgaaatgta atagcacccc ctttgttatg tgctaagaat gtgcgatact attgctgtta 54600
ttatggaact tagcatcaga gagttggata tgtgtataat tacaaatgtg gataaatcgt 54660
atggaggaaa gtagaaggag ttctgagagt ccttttttgt tttttgagac ggagtctcgc 54720
tctgttgccc aggctggaat gcagtggtgc gatcttggct cactgcaact tccacctcct 54780
gggttcaagc tattctcctg cctcagcttc ctgagtagct tggactacag gcgcgttcca 54840
caatgcccgg ctaatttttg tatttttagt agagacaggg tttcactatg ttggccaggc 54900
tggtctcaaa ctcctgacct caggtgattt gcccacctca gcctcccaaa atgctaggat 54960
tacaggcatg agccactgcg cctggccaag attccttttt taacttaaaa attttttatt 55020
tatttatctt tagggacagg atcttactat attgcccagg gtagcctcaa accctggact 55080
caagtgatcc tcctgcctcc acctcccaag tagctgtgac tataggtaca cactgctgca 55140
cccagctcct tctgagagtc tttaacaggc tccctgaaga tgtgacatat gagatttaga 55200
gggtgaatag gagggagtga attcgattat aagtgctcta ggcagaaata acagcattta 55260
gtaaggctct gagacagaaa ggagaatgaa tgaaaagaag agcaatatgg ctagaatgtg 55320
gagagtgaag gaaacagtct tgtgacaggt gaggaaggag gcagggacat gcagagcttt 55380
acatgccatg ttaaagaatt tgattagtgg gctaggagga atggaaagtc attctctatt 55440
tcattacatg aagaatattg ttttttaaat agccttattt tcttaaatta tttgtttaaa 55500
ctattgacta ttttctttcc ctggattctt aacctttttg aaagggtaga gacattcttt 55560

gtcatgtttg ccaataccct agtgtagaat agttgccagt gtgtgagagt tcggtatata 55620
tttgctgaat aatcaacaga tgtactcact gaaggataat aattggagga aggatgggta 55680
gatggaaata tggacaaaca gggatagatg aaaggatgga gggaaggaag gatagctgga 55740
ggaaaaggta gaggaaagga taaatggaaa aaaatggaag tataggatgt gtacagacaa 55800
gtgaagagag gaagggggaa agaaaagaag acagaaggga ggaagaaatt aacaaaggag 55860
tgaaagaatt ttaagtagac aatcagattt ttggtttgaa attctccctg gctattgggg 55920
caaaagcgaa tggtaagaga taatttctta acacttttta tcaaagagat gatgatagca 55980
tggattggaa agatgatgat gagataggga aaagtggagt tatccattag gaagtaaaat 56040
aaacaggact taatttggga ttagctatgg aagatgaggg attggtaggg gctgcctggg 56100
tttctgtaag ttaccagata acccacaggt ggcattttag tttttagaga attgcagtac 56160
attactcctc tttctcttgt tctcctcctt ctctcccttc cctttccctc tcttcatgtt 56220
tctcagtttt tctttttcta actctctttt tccctctcca tctgattatt cactctataa 56280
ggattttcaa gtctccgttc tatggaagaa atacccagat gtctgttgat tgaatcttag 56340
atggaggtgt tcagggaatt ttgtgcagag acctctgctg gttgtaaaac atttgtgtgt 56400
gtgtgtgttt catcttcacc actaaatgac tttatgattt aggaaaaatc ttttgatacc 56460
acatatctca atttcattat taaaggggga gaatatctgt cctattgact tggaggtttt 56520
tctagggtat aaatgaatta catcctaagt gtgaattgct attatgttaa tgatatagcc 56580
aattttatat taaagcacat tattgcatat gttttatgtt tctattactt tgaaaatata 56640
tacataattt cctggacact taacatttta agaagcctcc taatctaaaa ggtaaaataa 56700
gtatgacaag tagtctatta cttgatagga tgagtttctt gaatatgtga atgcctctag 56760
gcaagcttgt tttaaaagac cttagattgt ctttttttac taagaaaaat aaggagttga 56820
actgaatgtt ctttaagacc cttgttacat aaatatttta acttttgggg aagagagtaa 56880
ctagaaagaa actaaagcaa agtaattgtt ctagattcct taaatttcgt ttataatgag 56940
acaaagtaaa aataaataaa tatacgcata taatgttccg tatgttggat gaatataatt 57000
gaattctatt ttatccacag agtctctctt ccagcttctt ttggaaatca ccgttcgaag 57060
tactgggatg aatgacagca caggacagtc cttaacagca ctttcctgtg cttgcctctt 57120
tagtctggtg gcttcttggg gagaaacagg aaggacactt caggccatct ctgctatcct 57180
caccaacaat ggaagccatg cttgccaaac tattcaggta tttcatattt atatctgctt 57240
agaagtttat aagatgacaa gttacagttt cgtctcaatt tctgtcaata ggaattctgc 57300
tttttctcac tcattacagt agcttaatct gtccaagatt gatacataaa taatctacag 57360
tatttcaatt aagtgatatg tatgtttaac tctgaagctt taaatataat aaaatgaatt 57420
ttggccagac ctcttaaatc tttaagctgg tctttgcctt aacaccatag ccttctcttt 57480
ccttgctgtt ttcttgctgt ctgcgttctc ccttcttccc atctttccct gagtttttta 57540
gtatggaatt cattcatggt tttaaatatc ttttctctat tgaaagaaaa agatatggta 57600

tcaatctgat atatttttga ttggttgatt atgatttttt tatgaattga ttcgtttctg 57660
agttacttca gctgtgtata aaacatttta ggcttttaat attcagttgt ggaacaaaca 57720
agctcatcac cattttagtg gtgggaaaaa taatccttat aggcaaactt gacttaatta 57780
tttgggaagg ctctggaaat tgagaaagaa gttatgatct taagacctga ctgtctactc 57840
ttctctggcc ccttgggcaa ccctagggca ggtggaaggg atgcctcaat cctgttatta 57900
gcagaccaaa gatagcaaaa ggagtatatt ggtcattttc tgtcttattc agtgattttg 57960
aactctcctg gtcaaacaag acccattccc ttactcaaga tttctgctct tttcttcctt 58020
tctgccttgc ttggaatcct tttgtccctt tttatggtcc ttaattattt tgaagttgct 58080
ctaatacagt ggttttcaag ctgtgttctg tagacatgct ttcagggttt ggcaaatgtt 58140
taatttaaat atatgtttaa aatatgcaaa tattttaaaa attgtaataa agtaacacaa 58200
acattcagat accatatacc agattttaat aggatagtga ccaccaacat atgagcttat 58260
gaattttgat atgatttgct tttatataga gacttaggaa tgccattaga gctcatagca 58320
ggagtataag aactacgttc atggaaagcc tcccagagga tatgaggtga gatctaaact 58380
aatgagttga aatcagccag gagaaatttt tggtgttggt ggtgtagaga gagaatgttc 58440
taggtatcca catgctaagg catggaggtg agagtaagag tgaaatattt gaaagctgaa 58500
agaagtttag tatagctata gcatagagag ctgtcaaata gcgtagccac agcatacaga 58560
gctggcaaat aacagagaca ggactcgagg gcttatctgt ctccaatatt tatgctcaat 58620
cactagacaa cacaaaacta cttgatcctc tgaatcaata gctgcagttc tctacagacc 58680
tgccacatca ccacatcccc aactctagcc taaactccat attaaagccc attaccttaa 58740
catggcttgc agtgctcttc atgatttggc cttttatcta ccaacttcat ttcccaaaac 58800
tctcctggcc tgactgttgg tagagggaga aaagctttcc ctgagaatgt gtgcattaag 58860
tccgttttca cgctgctgat agagatgtac ccgagactgg gcaatttaca aaagaaagag 58920
gtttaatgga tttatagttc cacatggctg gagaaacctc acaatcatgg tggaaggcaa 58980
ggaggagcaa gtcatgtctt acgtggatgg cagcaggcaa agagagcttg ttcagggaaa 59040
ttcctcttta taaaaccatc agatctcgtg aggcttattc gctatcatga gaacagcaca 59100
ggaaagactt gcccccatga tccagttacc tcccactggg tcccttccac aacacgtggg 59160
aattcaagat gagatttgag tgggaacaca accaaaccat atgagtatgt gtaactcttc 59220
acatctcaga tttgttttgg cacccaaatg catactactt gtccatttca taaagcttaa 59280
cagagagttt aaagcagggt agacatggta gtactgcaag atgctgtatt gaaagacact 59340
gaaatccttt ctggagggtt gaactcttga cttcaaaggc tttctgcaaa taaaacacta 59400
tttattgcat ttgagctcag aaacaaatta ataaaaaaag cacatggaaa aaggcaccat 59460
tattgagaat tagcagaaac aaatatggta gaatcaatcc tgccaagtcc ttagatattg 59520
aaattatttg atatagaatg tgtatgtgta tgaatatata aagtatggtt aaagtaataa 59580
aagaaaatat caaaagtatg attaagtagt gagaatataa aaagtagtca gacattagga 59640

aaaaaaacaa atcgaactcc tagaaatgaa aaaccaaata attacaatta gaaacttaaa 59700
atgggttaac tattgtataa tatggttgaa gatggaaatg gtgaactgaa agataaagag 59760
gaagaaatta tctagaatgc aacagagaga caaggagaga aatatggaag ataaaataag 59820
aaacatggat cgagaatgac aaaatctaat gtaatacaat ctgagttcca caaggcaata 59880
atagatttgt ggagatagag tacttgaaat aatggctaag attttacaga attggtgaat 59940
gacatggaat cccaagagta aggaagtcta ccttgcttga gatttgcttc ttatagatga 60000
acttttagtt ggtacttttt gctggcatgc gaaatatcat tctgaacaat ttattgtctt 60060
atgaatgatg atgtattacc aaagtatctt tgttctgtgt ttgtaacatt taaaaatgtt 60120
ttttcattat ttttcttctc ttttgattag gtgccaacaa ttctaaattc gctacagaga 60180
agtgtacaag cagttttggt gggaaaaatt caaattcagg actggtttag taatggcatt 60240
aagaaagcag ctttaatgca caagtggcca ttaaaagaaa tatctgttga tgaagatgac 60300
caatgtctac ttcagaatga tggatttttt ctttatctat tatgcaagga tggattatat 60360
aaaataggct ctggatacag tggaacagtt agggtaatgt gattccttac agttccttaa 60420
ttatacagag ttataaccat aatgaattgt gttctgtgtg tttctagttt catttctaga 60480
atatgatcta attttagtgt aataatattt atttgaaaac cataattgaa atacatgcat 60540
taaatgtcat tcacccattt gtattatttt tcattgatta ttaattatga aaccactgat 60600
atactaactt ttgttttttt tgcagggcca tatatacaat tctacatccc gtattagaaa 60660
cagaaaagaa aaaaagtctt ggttagggta tgctcaggta agaaactatc cacaataaac 60720
caaaattttc ttatctttta caacatgtga tttgtccttg tttaatcagt aatatcactt 60780
ctatttaaac agaaatgata atatattttt actaatatgg ccataataac tgaatagtgt 60840
agcgtaaaga acactgtctc agaactcata agaggttcaa ttttagttct ggccctgctt 60900
cctactagct gtatgactgg acaactcagt taacttattg ggtcctctgt tatttagctt 60960
taatatggga ttaaacatac cttctttaga gttcttgtgt aagtgagcct ctagataaga 61020
attgaagacc acctttatca ttagcacatt ttttttttta attgagacag aatctcgctg 61080
tgtcacccag gttagagtgg agtggcacag tctcggctca ctgcaacctc tgcctcctga 61140
gttcaagtga ttcttgtgcc tcagcttcct gagaaggccc atgccaccat gcctagctaa 61200
tttttttgta tttttagtag agacagggtt ttgtcatttt ggccaggttg gtctcaaacc 61260
cctgacctga agtgagccac ccgcctgggc ctcccagagt gctgggatta caggcctgag 61320
ccaccccacc cagcctcatt agcacattta aattggaaat gtatacatgt cctgaggcca 61380
aattagggtt tcttaccaca gactctttca ttttagtcta gattcagtac tttctaggaa 61440
acagtgtttg gcctactcag tagacagcta cctgcatgaa gaatatgcaa agaatcacaa 61500
gagagaaaga aaagccctgg gtttttgtgt cttaatgctg tgatcttatc tcctgaccaa 61560
catagtcaga taggtgttag ccttttacaa ggtcagcagc cagatggata cattactcct 61620
cttatggaga aaagcaaatg aaggatggac aagagggact agaaatattt ttttccatac 61680

agtacctacc tgaacagtga agcccaagtt cctttgtata aggataaaga ataaagattt 61740
ccctgcatgc ctgccttgct gagcttgtga gatttgttgt caggatcaaa tgataaaaga 61800
tatttgaaag tcttttgaaa actatggaaa ttactattgt tacatgaaga ataattctac 61860
tgtatcacct attggggaga atttgaaatt aaattttttt ttttttttgg agttgtagtt 61920
ttgctcttgt cgctgaggct ggaatgcagt ggcgtgatct.cagctcactg caacctccgc 61980
ctcccaggtt caggcgattc tcttgcctca gcctcccgag tagctgggat tacaggtgcc 62040
caccaccacg tccagctaat tttgtatttt ttagtagaga cgggtttaac catgccggcc 62100
aggcttgtct cgaactctta atcccaggtg atccacctgc ctcagcctcc caaagtgctg 62160
ggattacagg cgtgagccac cgcccccagc ctgaaattaa attttagata acaaattagt 62220
ttctcagtaa ttgtctctta aaaattgaac ttagtttaaa atatctcatc agatttttat 62280
ttgccactct attgtgtttt atctaagaaa taatgccgtg gaaagtatat tattatagca 62340
gtgtgttcaa tggcatacca caccttctga aaccactatg ctattcattt tcaaatagca 62400
aactcaatac ttgttatttt tcttaaagta cttgttaaag taacagtgat cattcatatt 62460
atttacttgg caagtggtat ggtcattttg aacaaaatgt ttgtaactgt actgtcctgt 62520
ttagtatacc taattatgtt tctgggaata tgttacatca attttcaata atgtatgact 62580
tttcctctaa ttgagaggaa tgttcttata tttttaatca ttaatatctt ttagacatca 62640
atattgatgt attattgtac ctcaaaaacc tgtaatatgg agctgtatgg ctgctgtttc 62700
tgctgaatta gtaataaata ttttaacagg aaaatttttt gctgttatca gggttattta 62760
ttatatagag atgtgaataa ccacagcatg acagccataa ggataagccc tgaaacactg 62820
gagcaagatg gtactgtgat gttaccaggt atgtttcaag tagtcatttt ttctccacaa 62880
gcaattttaa gaaatgtgca tgttaagcta tttagacaca taaagaatga ttagcaaggg 62940
atagtgcttg cttataaaag ggttttaaaa atcttgacat acaaagcatt ttatagtcta 63000
cgtgaagtta atacatatca agagaataga catagaaata taacaagttt taatatttgt 63060
tttcaaactt ggccgtctat ggcctaatct ctgacctcac cccaccactg ctgaattagt 63120
ggggtgatgt aactgtgttg ctcttaataa aaatcagaac tctggctctc atgtgactca 63180
gtataaagaa ccttctgttt atttttactt taacaacagt gcagttgggt tttcctcctt 63240
atttaattta atttaatttt atttaattta atttaatttt attttatttt attttatgtt 63300
aaaggggttt cagagacaga gagtttaggg taggtcagga attttttttt ttaatttcat 63360
catttatgct tcttatgctt cttaatactt tgataaaggt ctttggacac agtttatttt 63420
gctttaattt tgaggctttt atatattcac ttaaatcact gctaaattat caggcagtag 63480
gtctccaatt ttgatgaatg ctggaaaaat gcatattctt tattgtaaga tctttgaatt 63540
tgtttataat gttgatactt tctgaggctg tttatattaa aatcttgagt gtctaatgtg 63600
tctgtcaata atttcatagt agcaatatta cttgtaagcg tttgaccatc tgaaagtgag 63660
agctacttct gaagagtgca ttgagaagaa taattgctcc tgggctgcat gtttattttt 63720

aaatataatc ttaataattt catcaaacat ttatttgaac cttactacat atacatagat 63780
acatattagt gtacatgaca ctatgttaaa gtaaatatga catttcatgt ccatttcaga 63840
aatagaggag ctttggaaag cggatgactt aataaaatca ttttgaaaat aaccatctat 63900
tttcagatat gtctacaggg tttttttttt tttttttttt ttcattacaa gtcctggacc 63960
atgtctttgg tcataataat aataataata atgacaataa tattaaaaaa tacttttaat 64020
aacatgcaca ctccccccac atatttatac atatattgaa agtaatcatg tttgttgtat 64080
aaaaatcata tgaacaaaaa agaatgaatt gaaataatta taatcccact tcccatatat 64140
aaacactatg aacatattgg taaatatcct tctcgtattt ttcctttgtg tatatgtcca 64200
tacttgtaaa aaagtgtgat catatgctac atattgcttt tttaatctat ctctttcact 64260
taatattctg aaaatatttc taggtcatta aatagtcttc tacaatgtca cttaaatgtt 64320
acaaaatata ttccattgat taatgtttga gaatgaaagc cttccagggt tttgctatat 64380
aaattacact gtaatgagcg ttcctatcgt gctgtctttg tgccagtcat tttaggataa 64440
tgacatagaa gaaaaattct aaattgacac atcccatccg ctataacaca tgggtactaa 64500
tatcaaggat ttacttctga gaaaccctgc acatttaata gagactgtga cttattgata 64560
agacaatttg aaaatatgtg cctacagctg ttttatttat tctgccttga taaaaaaatt 64620
actcgttaat gtctttattc agtttaaatc tcgttgcttt gtttaacttg tgaaatgcaa 64680
ctttgaacaa tacaagtgtg gctttctgtg tttcataagt gtgtgctgta ttagtggtga 64740
agaatgaacc aaacttcagg aagatagtcc catatttgtc ttcagaactg gcagatgttg 64800
cttgatatat tcctgaagat acaagcttaa aatattacat gatggataaa gaaaggataa 64860
agagaaaatc ttgaaatcac gtggcagaag actcctccct caagcctgaa gaagcctttt 64920
gtaattttct tgtttctttg acctggactg aattttcatc caatagcact ttttgttttt 64980
cgtgggtagg aatagctctg ctctgtagcc ttccaatcaa ggagcagaag taaattgtca 65040
ggtttataga tggtaatgaa taccatgttt caagttgaca taaatcaggt aattccttga 65100
gcttcatgtt ccagcttgtc ttacagtata gtttatttaa aaagtatttc aaccttgttt 65160
tgatgaactt aaaacatact ttttttatct gataattgtc attttattct ttaaacctcg 65220
gcttatctta cagattgtat ctttatgtca ttttctctcc atgtctggtg acaggttgcc 65280
tcagttcatg aggctttttg ctactcttct tgcgccgctt cactcctcac ccctgcgttt 65340
atctgaaatt ataatgagtt tttcttcttt gctttttagt ttaaaatctt actgcttaga 65400
gcttgagaag catctttctt gcgcctcttt gtgttctgta cagtggacta ggaactcttt 65460
cttttgattt ctcctctctt tgattaccaa gtctcttggg ctaccagtgg gccttgtctc 65520
ccagtttcta tttccagttt gtgatccctg aattctctat ttggtaaggg tctgcctgtg 65580
ttttgcatat ttatgataag gaagtatgct agtatgaagc tcctgggcaa agataaagaa 65640
taatccagct tattcgggtg ggctccatta ctgcatttca aaatcagaat tttatgtttt 65700
gttgattcag gtgcaactag aaatgaaatg ttattttttg ttttggtatt gtgattaatg 65760

gagagattca tcctagtttt ctgcagttat tttgggaagt atgtgtgtgt gtgtgtgtgt 65820
gcttttaact tgcttgctga aaatttgttt ttccaaaagg acagaattta tccttgatgt 65880
tgattttgct tgtagttttg tgttatttag tagcaatcac taacacgtgt tcctgtggtc 65940
cttctgtaat actggtatcc cttgttacaa ttgagcttgt gcattatttt gatcaaagta 66000
tatggtgtac tattctatgg agatacatgc agtattgatg aaatatggta ttctagccaa 66060
gatacatgac tgctttgaga taaaaataaa agcataagca tatatttgtc tgctcttgtt 66120
ggacaaacat ttaaaaagtt tagagcacac taaatgctaa aaatgctgtg aataaacatt 66180
gaatttttaa gtgggaaatg tacaactggt tttgaatatc cattttacaa agcaagggat 66240
atctgtagtt ggacacaata gatgtcaaat acagaagctt taggtcgtat ttttctgtga 66300
cactaatgac tgtgatgtat gtctgagagt agagtgttgc ctccattgca tgtgattgta 66360
ttgaaatgat tgtaaccaac attatagttt gttcatcgtg gtgcatgttt actgtcagac 66420
tatatctaat actaattcat tttgaaatta ggtgggatct caggtccttg gttatggaag 66480
atttataaat ataagcaaaa atgtaataag tatgattagt tgaattagtt tctgctaatt 66540
tataattctt tattaaaaag cacagtctcc ttttgagttt atccttattg cgtggagaac 66600
tctcattttg acttcaacag aggagtaaat tcattttgca aggtgtttgc ttgtattgta 66660
atataaatac tttttctttt gcacagattg ccacactgaa ggtcaaaata ttttattcac 66720
tgatggagaa tatattaatc agatagctgc ttcaagagat gtaagtatcc tgaatcttaa 66780
gtagctagtg taaatggaat tccttttcct taaattattt agcttttatt agatagactg 66840
tagagccttg aacgacctct ttatgtaatg taatgctgtg gatgttttat ttttcttagg 66900
atggctttgt tgtcagaata tttgccacaa gcactgaacc tgttctacag caagaattgc 66960
aacttaaact ggctagaaaa tgcttacatg cctgtggtat ctcactattc gatctggaaa 67020
aggacttgca tattataagt aagatagcaa attaagtgtt ttccctatat tttaattttc 67080
aatttttcat actcttaaaa atgaacattg tgttttcacc agttcagctt atgttgtagc 67140
agctattttg tgtctgttac tattaatata aaggataatt ttgaattaat atgaataaat 67200
aactgggaaa cacagtcttt aaacaaagta tgatatttga agaccattct aaggaattga 67260
ctaataattt ctcttttttg ctttgaatag agcaaaagaa aataaaattt aatgagaatt 67320
atggatggat attggtgaat gtcattagtt tatatgctgc atcatttttc aactatttaa 67380
aaatttgaaa acttacgttg taaatgttat tacaacaata tacatagttc tctcgttgaa 67440
atttttaaag tttttacttg aatagatttt caaggtaact atagaatgat ctcatagatt 67500
gtcaaaatgg taattggcct tacacacgga tcagtaatct ttcacttgga tagagaaata 67560
cagataacca cagagtattt cttgggctta gattatttac ttttgtaatg ttgtaaaagt 67620
aattaggatc atgagttctc ctgagagttt tagttttggc ttgccatgcc actccaggtt 67680
taatagtgac cttccgtaac ttgtagggtt ctttgcaaga tccatctttt ttgactgtta 67740
tctcattaca tttcataaac ttggcattag tgctttactt tctatattct aattatgtag 67800

tatttttata attcttccct agaaaaatct tgaattgaaa ttacagattt aaaattttta 67860
aagcccataa actagttagt agtttgtata gctggacaaa gtcataaaat gtaccatatg 67920
taaagctaca attactcata gttttacaat ttgaaaatat gcataaggat ctgtgtgtgt 67980
atatgcatat ttacatatat atatatttga attgatggag tagctatata agatgtcttt 68040
ctgtagaaaa agacataggc agagaatctg aaatacttta attcaaatat ctgcatggca 68100
ttgttccaag cttgaatgac tctgtgtctg gactaacctc tccaaccttt atatatgctt 68160
atttgtaaac caagtggtat agttgtgaga atcaaatgag ataaattttg tgaattgtat 68220
aatgcctggt acctaataaa cgctcaataa aactttgagc atttatacat taattcaagg 68280
aatattgctg acattgaatt tttaaatttt tttgtcgtat atttaaaaaa ttaatttggt 68340
actggtttgg aacaagagaa acagattatg ataactttat ctttccttgg agtactatac 68400
ctatggttaa tatttttaaa aatttgagca catgacattt tgtcatcctt gttttttagt 68460
gattttgaat tgacattgca atcacttaaa atagtgcatg ttgtatttag aattcttcat 68520
attagaaatg caatagtatt tattagtctc gtatttcagg tacaggattt gatgaggagt 68580
cagcaattct tggtgcagga cgagagtttg cgctaatgaa aacagcaaat ggaaaggtaa 68640
attattctct ttggattaaa aatgagcatt ctctgattta agagattaaa atacaatctg 68700
ttgattagtt tatatagttt gcatgtttaa gaagaatcat cttttaaatt ttgatttaaa 68760
attatttttt ataagataaa attttattca tgtagggaac agtacaggca tgcctcattt 68820
tattgtgctt agttttgtta cacttcacaa ataattgctc tttttacaaa ttgaaggttt 68880
gtgacaaccc tgagttgagc aagtctgttg gcatcatttt tctaaccgcc tgtgctcact 68940
ttgtgtctct gtgtcacact ttggtaattc tcgaaatatt tcagacttct ttattattat 69000
tatatctgtt atggtgatct gttatctttg atgttactat tgtaattatt ttagggtgct 69060
gtgaattgtg cctatagaag acaggaaact taatggataa aagatatgaa aaagctatta 69120
tgaactgtgt cttcttggca aaatatgtgt atttcttgct caactcccag aagctgaata 69180
gtgtaaaaaa gagtttgttt ttatgtgaat tttatttttc aataaaatat tgatgtcctt 69240
ttttttcctc atgtgaaacc tgatgcacta ttccaaaaag taccataatt cctttttttt 69300
tttaatacat aagatatatt acactggcaa ataccagagt cttggaatca aacaaggtgg 69360
tccttcagca ggaaaatggg ttgagctacc aattacaaaa tctccaaaga tagtacactt 69420
ctcagttgga cacgatggct ctcacgccct tttagttgca gaagatggga gcatattctt 69480
tacaggatct gctagtaaag gagaagatgg agaatcaagt aagttgagtg atcaactatg 69540
catttaataa aaataaatgc tttattttag taaaaataaa actttattaa gataaagttt 69600
atgaatttca ataacaccac accaacttta tatatatatc attgacttga tgtaactgct 69660
taataaatac atattgaatg aatgaggaac ttattttact ttccaggatc taacaagaat 69720
acatgtgaat tacaacttgg gaaaaaaagg agattttaga tatgtgttag tagaatggta 69780
ttaataatgc aatagctaat ttagagattg ggccctccag gagtttggta gaaaactaat 69840

attactcact gagaatttga atgtacttca gggaatgagg atctagcaat gatagcgtaa 69900
atattaattg accagatgag gctagcaggt tcactgattt taaatgacat gttgggaagc 69960
ccaggccttt gtgaaaataa acaaatagtg tcataaatgc aaataatatg ggtttttttt 70020
ttagctttat atacaaacca ttattattat tatttgtaca tagaggtctt gatttaatct 70080
aatttctttt ttaaagctaa gagcagacgg caatccaaac cttataaacc taaaaagata 70140
attaagatgg aaggaaagat tgtggtatat acagcctgca ataatggaag tagttctgtt 70200
atttctaaag atggagaact ctacatgttt ggaaaagatg ccatttactc tgatagttca 70260
agtaagttaa taaaaagttt tacttttatg caaagaagtt tcaatactag ttatgtatga 70320
caaagacata tctagaaaat ttatgttggt ggcatgcttt ctctacattt tttacatttc 70380
cttccaaatg tgaaatattt cgaaagagaa attttctgtc atatcttgtc actaggagca 70440
ctgtccctag tttcatgcaa agctaatcat agagcttggc aagggaagct tcgctgtagc 70500
tgtgacttct gtggatgttt tccctagact caggtgctta tgggacatgt ccctgggaca 70560
gtggtagtat cctgaagaag tgattttcat gacatagcta gtttataatt gttgaagtat 70620
tttatctccc ccattagggt gtttggctgg gatgatctgt cttatttact attctatccc 70680
cagtgcccag cacagtgtct ggcacattgt agggtctcaa gaattatttt ctagtgcctg 70740
ggtgactgat aaaccaaaag tttgagaagt ggatatgagg ggtagaacta gctaaatggt 70800
tttatattta taaatgctaa tctcaaaaac ttgtcatatt tggttactag attttctttc 70860
tagcagctag aaatgaatat aatgagtgaa gcatcccact tggaaggatt ttatgtgtga 70920
taaatatgta aagcctttga gtttctttgt aaaaaaggaa actagaatta atgaagttta 70980
tgattagaaa tatatgtaat tgtcccatgc tggggaaatt acacctgtag aacatttata 71040
gcaattaatt attaagtgga ggtgtaccca gatgataagt gaactggctt aatgttgtaa 71100
tatttagatt ctcaattggc tgcagctatc agagtaaatc aaaatttggg agtgggttct 71160
tgtacagttt aaaaataata ttcaatataa ttgttgcttg ctaacatcca ctgggtagag 71220
ctgatggaaa cttgtatatt tcttcaacat atatttattg agcatgggtg atacattagt 71280
gattgatgga cctgacagac ctggatactg cctttttgag gatccgagag acagttgggt 71340
attacagaac ttaaatattg gggaagtggt attgaatggt tatggacata gactgagatc 71400
ctagcaccaa cacttttttt ttttgagagg gagtctcact ctgtctccca ggctggagtg 71460
cagtggcaca atctcagctt actgcaacct ctgcctccta ggttccagcg attgtcccac 71520
ctcagcctcc caagtagctg agattatagg cacgtgccac aacacccagc taattttttg 71580
tattttttgt atttttagta gagacgaggt ttcactgtgt tggccaggct ggtctcgaac 71640
tcctgacctc aagtgataca ctcgcctcag cctcctcaag tgctgggaat acaggcgtga 71700
gccaccgcgc atggcccttg gcaccaacgc ttttaattgg gtaacagttt acttaatctt 71760
taactgggta acagtttact aaacatttaa gagttttctt tttgtttttt tctacctgca 71820
aaaaatagat aatgatagca cctgcttcat atagttgtta tgaagattat aggaggtaga 71880

acttaagaaa tgcttagcac atcatgtaag gtctcaaagc atgtcagcta gtactgttaa 71940
taataataat aaataactac tgttttatat atatacacac acccttacct ttttcaaatg 72000
tgctaaatat taaaagtgga aatttttgag gtataattaa caaaaagacc taaactagtg 72060
aaaggtgaaa gtgaaggcca ccttaaggta gtgatatctg agctaataat gtagaacagt 72120
tttcccaaag cataatatga gattgatact atttgtaatt taaataaaac aaatacttga 72180
taacatgttt ttattttaat gtatattgga aaaataataa tctttgattg ttaatgttaa 72240
agatgcttca atcaatttag agttgataca aaacatatta aatagataat atgagtggca 72300
cttggatatg acaaatataa tgaaagtaat acatgaataa gtgaagttta gaaaataact 72360
gtgtattgga ttgtggaaga aagcattgta ggttgaaaag gattaaaaaa tactgttttg 72420
aaaagtctca ctggtttcta cataaacaat ttcatgtcaa tatactatgg caagtatttt 72480
actttgtttt gctgaggcag gtcaaagcag gtaaaataaa ttatttatgg actcctttgt 72540
gcttagacca gaaaagtttg aaagtactgt ggagaggtat taccatttgg aggaagcatt 72600
caggagaagt aagcttgaga gaaagctaca gtaaagatat gtcccaagtc tggatgataa 72660
tgttttagga ttcactgaag ctacgttaca aagttacaaa gttaattcag tgatactctg 72720
ccagtctttt ggctacttat ttctcatttt gttgagttat tttgccccag aaataaacgg 72780
ttctgcattg aggtagttct tttactaggc tcagaagggc ctttttgttg tttttactaa 72840
atttgtttct ttcctatagg tttggtaact gatttgaagg gccattttgt aactcaggta 72900
gctatgggca aagctcacac ttgtgtttta atgaagaatg gagaggtgtg gacatttggt 72960
gtaaataata aaggacagtg tggacgagat actggtgcca tgaaccaagg tgggaaaggt 73020
aggtctttaa ttcttagata ttaaaatttg tgttgtcttt tcattagttt ttcagaacaa 73080
catagtgaaa agtcttgttc ttttcaaaat gagtggctct tctttttctt tttgttccta 73140
tttaaatttt ttttaaaaat tttatgggta gatagtaggt atatgtatgg ggtacatgag 73200
ttgttttgat acaggcctac aatgtgtaat aataatcaca gggtaaatgg ggtctccatc 73260
atctcaagca tgtatccttt ctttgtgtta tgagcactcc agttatactc cctcagttgt 73320
taaagtatac aaaaaattat cgccaactgt agccaccctg ttgtgctatc aaatagtaga 73380
tctcttgtat ctaattatat ttttgtgcca actaaccatc ccatttccca cccactctcc 73440
ctgactaccc ttcccagtct ctagtaacca gcattgtact ctctatcttc atgagttcaa 73500
ttgttttaat ttttagctcc cacaaatgag tgacaacatg tgaagttggt ctttcggtgc 73560
ctggcttctt tcacttaaca taatatcctc cagttacatc cttgttgttg caaatgatag 73620
gatctcagtg ttttttatgg ttaaatagta ctaccttgtg tttatatgcc acattttctt 73680
tatccactca tctgttgatg gacacttagg ttgcttccaa atcttggcta ttgtgaatag 73740
tgctgcaata aacatgagag tgcagatatc tctttgatgt actaatttcc tttcttttgg 73800
gtatatccat agcagtggga ttgttggatc atattgagtt ctagtttcag ttttttgggg 73860
aagctccata ctgttctctc cagagtggct gtactaaatt tacattctca ctaacagtgt 73920

acaagtgttc ccttttctcc acatcctctc caacatttgt tattgcctgt cttttggata 73980
aaagccacgt taactgggga gagatggtat ctcagtgtag ttttgatttg catttctctg 74040
atgatccatg atgttgagca ccttttcata tacctgtttg ctattcatat gtctactttt 74100
gaaaaatgtc tattcagaac tttttcacat tttttaattg gatttgagtg gctctttgac 74160
gttatatttg tgaattagga aaaccactga atttcttatt tatttgataa tatttactga 74220
taaaatatca ctggaaaaac aaatacactt tagatttttt atttttgagc aaatgtgctg 74280
gcttactcca atcatcttct aaattctaca taaaatagtg cttctttggc ttagcatctt 74340
tgatgactgc attactccag aaaagtcttt tccaaacatt tttttattag ctgctgttac 74400
acgacatgtt ttaaatttta accatttttg gcagcaatct ttctggcgta tattatcttc 74460
ccatttaaca gcacatgcta cctgtaattt aacctgttct tgacaactaa gagtgatcct 74520
tatgaatatc tgacacttta ccaagaaaag actaaattgt agacacacct tcttcacaat 74580
atcctttgct ccaaccctca ggccattgaa gagaacaaag ggctttctcc tttgattgcc 74640
ctgtttgctg tggtcttgtt ttgttgttgt ggttgtaaat agcatgtcta cttcttgccc 74700
tcaattttta tatctagctg ctatcgttat gtttataact ttcactccaa tttctgattt 74760
tggtcaagag attaaaccat acatatatat tattagaact aagcgttgaa ctttggttaa 74820
agtaccaata atttcgagga ttaatgctgt ataaattaac aaaacctggt ttttctgttt 74880
tgaggctttg atgtcagact catctggatc tcagaaaccc accattcttt gtctttgcct 74940
tattcgtgtg tctgtgtgtg tatttaaaat gcatcacttt aaaaatgagc aagttactag 75000
aatatattgg caaatgaaga aggaacaaaa gaaagaaaag aaatggatta cttcacaatg 75060
gaaatacttt gatttattac taaattagtg aacttgtttt ggataataac tgctaggaca 75120
aaggggagaa tcgggttttt ttaatatata atatgaatat atattcagat tatgttttat 75180
aagaagtctg aactaaacaa cattgtttcc ttattctgaa actcttgtga aaattgagcg 75240
tacatttaca cctatgttct agattcttca gtgacatttt gtaataatca aagtaaaatt 75300
gttaaaagat ttttttaata agaatcatac ctaccctgct ggtaagtaat gaattagaaa 75360
gcagatgagt aaagtaagac acatacagtg catactgatt gcagagtcat aacagatgct 75420
ggggagaatg tggagaaagg ggaatgctca tacttggcta gtggaaatgt aaattagtac 75480
agcaactatg gaaaacatag atttttcatt cgtatgaagg tgactcaaaa aactgaaaat 75540
ggagctgcca catgatctag caattccact tctgggtata tgtatcaaaa agaaaggaaa 75600
tctgtatgtc agagagatgc ctgcattctc aggtttatca tagccatatc cacaacacca 75660
agatatggaa tcaacttaag tatccatcaa cagatgaatg agtaaagaaa atgtggtaca 75720
tacacaatag aatattattc atccctaaaa aagaatgaaa tattgttatt tgcagaaaca 75780
tggatagaac tgaaggacat gatgttaact gcaataaaat aaaccaggga cagagacaaa 75840
tatcactcat atatgggaac taaaaaattg atttcatgga gatagtaaat agaatagtga 75900
ttaccagaga cttgtaagga tagtgggagg gggagaggaa gagtggttgg ttaataggta 75960

caaaaataca gttagagaga aggaataagt tctaatgttc aggagcagag tagggtgaat 76020
gtcgttaaca acaatatatt gtgtatttca aaatagctag aagagagaat ttagaatatc 76080
ccagttatct tgacttgatc ccagttaccc agatttgatc attagacatt atatgcatgt 76140
atgaaaactc acatatatgc cataaatagg tataacagtt atgtatcaat ttaaaacgaa 76200
cgtaaaggaa tattttaaaa gtttatttaa accaggaaag tacagaacat attgaaatat 76260
taaaaaattg tgtctctacc atgtcttgtc attttttttc agttaaaaaa aaatcacaga 76320
taagattcaa gtgccccata tcccccttcc aaggcttatt atcatattcc atttctcccc 76380
agagctaacc gcgatcatga ctttgataag catacgttca atcatgttct tatatatttt 76440
tacatgttct tttatatttt atgtgtaaaa taatattgca taatgatgtc tgttttacac 76500
ataaaatata ctactgtttt atgtgttttt ataagtgtta atctgttatt tacatttttc 76560
tacaaatttt tttttctcat cattctgtta gaaatatgtc catgttggta catataaatc 76620
taaatgattc atgttaacta atgtgtaata taccacacag tgaacatgct atagtgtatc 76680
cattctcctg ttgatggatg ggcttatttc caatttttca ttgttataaa tgagtttaaa 76740
acctccttta taaatgtttc ctggaaaacc tgtgagactt tctctgatgt aattgctcta 76800
ttgtggattt cagttttact ggatttttct tgaaagagat tgtaacaatt tatactctca 76860
ccagtagagt tggattttcc acatcacatc tgatattgac agactgattt ttgtcagttt 76920
ggtggctatg aaatgatact gtaatttttt atttgttaca ttgaagtata atatgcatgc 76980
aggaaaatat acatagtatc ttgatgaatt ttctcaaatt gattgtattc atgtaatcag 77040
cacccagatt aagaaaccac atattaccag cagcccagaa agccccactg tatgttcctc 77100
tctgcccttc agttaattac cataaattag ttttcttttt atacttgata taagtgaaat 77160
catgcagtat gaactctttt gtgtccagtt tctttttatt gttttatgtt tatgaagttc 77220
atacatattg ttacaagtag ttatagattg ttcatttttg ttctattaaa ttatatatag 77280
atactacagt ttttaaattg tttctagtat ttgggcattt ggatggtttc cagttttggt 77340
tattatgaat aatgttgctg tgaacattct agtaaatgtc ttttggtgaa agtgtttatt 77400
catggctttt gggtatatac ctaggagtag aattgctggt tctctcactt ttcttttaat 77460
tttcattccc ttgattatta atactgagtt tgaacatctc ttcaaacttt attttactta 77520
ctaaaaattg atttgtagga gcttttttac gtattatgtt ttatatatct ttgactaacc 77580
tgtggtttac agtttcattt gttatgtata attttaaaat tttgatgtca aaattttcaa 77640
cctttttctt tataaatttt tgatgaatgt agcctggtga gagattttat tttccatata 77700
aattagaacc agctttattt gcttggcatt gtgattgaga ttccattgac tgtaaagatt 77760
aatttgagga taatagttcc agtattggaa tgattaaatg agtaatgctt atagagctag 77820
ttacaaatag tgctcagtac attttagcta tgattattga aaatagtcac tgaggcttcc 77880
ttcaggatca ctttattcta atcctagata tagctagctg atgcttggtt atctaatttt 77940
tctgcttgcc catagattag aagtggcaaa accccaaaag gctatatatg ctatgagttt 78000

atttcttggt tagatagacc tgtattgagt gatagctgta tgttaaatgg atgtgatagg 78060
tgtgagagaa taagattgac taagaataag agatattatg agcaagactt aacctcataa 78120
cttagatcct ttaggaaagt caaatataaa aaataattac ttgactccca cagatttgtt 78180
ctcctgtcat agggtcttat aacactgtgt tccttcaaaa ccctaccagt tgtagtttac 78240
atttttattt ggttatttga ttaatgtctg tctttcttac tacattataa gcttcataac 78300
agctcagtat taccgtcttc agtttctaga gcagaggtct gcagagtata acctgatgtc 78360
atttttttat atagccctct aagctaaaaa ttgattttac attgtaagga ttgtaaaaaa 78420
tgtgacaaag atttgtatgg gccacagagc ctaaattatt tactggctct ttataaaagg 78480
tttgctgatt tcttgccgtg aaggaatccc tggagtatag taggtgttca atttagaaaa 78540
aaagatgaat gaattgtatt gtaacaccat tttaaaaagt tgttaggtga ggtataaaca 78600
ggcactgagg aggtagtgac tgactgcctg gaaaaggtag caagtgcttt aatagggaat 78660
gacttctgag ctgaattttt gaaggctgaa gtttctctac tagatgagat aggcagagac 78720
atcttgtaaa tgaggaagga agaggcagag ggagcaagaa tgaacaaagg ccctgagcat 78780
atggaaggac acagggtgtt tggagagcag agggaaaaga aatgagattg gagaaaatta 78840
ggagccatct tgtgcctact gcctcttgct gagttttgtt cttctgaaga ccttggaagg 78900
tcactggagg tttgtaaatt atgaagttgt catgtgcttt gggagggtga ttggtggcag 78960
taggaggatg atctagagtg gggaactctt ggcttggagc atggttggga ggctttcttt 79020
ggtctgttga atgaaagtgt tggtagtgag gatggaaaac aggaaaagat ttaaaagtat 79080
gattgacaga aggtaacagc caagagacat ttgacaatag ccttccttgc cttcttttgc 79140
agaaatgaaa tggggagagt gtttattagt ggtccctaaa ttaaaattta tttttgttac 79200
tcaaatttaa gcatttcttt gtataattgc ttggattagc atgctgtcat tttttaaact 79260
gggaaatata gtcatccatc ttttatacat gaaagtaacc tgtataaaaa ttacaaattg 79320
atctgtcaaa tagggtttgg agttgaaaat atggcaacag caatggatga agacctggaa 79380
gaagaactag atgaaaaaga tgagaagtct atgatgtgcc ctccaggcat gcacaaatgg 79440
aagctggagc agtgcatggt ttgcactgtc tgtggagact gtacaggtta tggagccagc 79500
tgtgtcagta gtggacggcc agacagagtc cccggagggt aagagacaat gattcctact 79560
aaagaacatg tgcagaacac atttctttgc aaaatcattc cggagtatac tctactatta 79620
atattttttt cttaaagacc agtggcatct tcacttgatc ttagccaaaa ggccaagaaa 79680
gtattataat tcttataatt gttttctgtt ataataattt ataatttaat gtcatctcta 79740
aggttgcctt tctttcattt gattcattta aaccattatt taaaaacaga ccataagttt 79800
ttcaagactt ggggctgtac ttttatttgt attatttttc aaagcctatt taatgctaga 79860
tatctattat atttctttct gttgaacatg aatcttttct aaataaaatt atcgatatca 79920
taaagagtac ttgattagat atatagtttt gttttaaatt aaatgtacca tttataaatt 79980
tagaaacgtt gtatgcttgc ttctaaagtt acatcatacc tgctatattt atccttgttc 80040

tcaatggacc acatttttca gggttctata ttattgtcaa tttacagtta atatgtaagt 80100
ttggcagaat aagaatgcag gttttctgct aggatgttag gaagaatttt gaatctattt 80160
tctacattgc tagttatagc cactataaca tgatgggata ttctttgtgt gatcagcttt 80220
gtaataattg gctgatttgg gggagtagag aaaattttgt gaagagaagc ccagaaggaa 80280
gacagccatc cctgcctctt gaccttcctc tttgtcaact ctgtaatagt tgcttcttcc 80340
caaatattgt ctggatctaa taggagttta tttctaagtt tatatttggc ctttcatagt 80400
taaataggtc atatataaca ggccttttct ttaaggctgc tgagatatga tttttttttt 80460
ttttttgaga cggagtctcg ctctgtcgcc caggctggag tgcagtggcg ggatctcggc 80520
tcactgcaag ctccgcctcc cgggttcacg ccattctcct gcctcgacct cccaagtagc 80580
tgggactaca ggcgcccgcc actacgcccg gctaattttt tgtattttta gtagagacgg 80640
ggtttcaccg ttttagccgg gatggtctcg atctcctgac ctcgtgatcc gcccgcctcg 80700
gcctcccaaa gtgctgggat tacaggcgtg agccaccgcg cccggccatg aacttttaaa 80760
aacaaatttt ctttacatta ttagtataag caataaagtc gtctttatca aaggataata 80820
tttattctgt agtgctttat gtttggctct gttttctgtt ttttaagcac ctaacttctt 80880
tatttttaaa tttaatttgg atgcagaaag actctgatta ttttttaaca atgttggaat 80940
tgaaatttat tctcctggat ctctccaacg ctatttccat taaaattcag tgttagctgg 81000
gtgtagtggc ccatgtctgt aatcccagca ctttgggagg ctgaggccag aggatcactt 81060
gaggccagga gtttgaaacc agcttcagca gcatagcaac acccctaact caaaaaatat 81120
ataataaaaa taaaatttaa tatccccact ctctgagctt aagcagccag tatatatcgt 81180
gaacaaaatc tataaattat ctggtccctt ttgaaattaa ttattcttcg ttttttcctc 81240
cagagactta cataatttat aataattatt catagactta catggacaat actaaccaag 81300
tataataatt gctttatgaa tgaagcaaac tgaatatatc agaatattaa ggactattaa 81360
gctgacttca attattataa aattgtgact acatatctta ttatgataaa gtacatataa 81420
aattgaccag cttaaccatt tttaagtaga cagttctgtg gtattaaatc cattcataat 81480
gttgtgcaac tattaccacc atgcatctcc ataacttttc atcttgtaaa actgagactc 81540
tgtaactgtt aaacaatgac tcctcattct tccctctccc ctgcctctgg catccactat 81600
tcttgttttc tttctgtgtc tgattgacta ctctaggtac ctctcatata aagagaatca 81660
gagtatttgt cttttttgtg actggcttat ttcacttagc ataatatcct cagttcatcc 81720
aggttgtcac atatgtcgga acatctttgc tttttgaggc tgaataatat tctattgtat 81780
gtatattacc acattttgct tatccattca ctcactgatg aacactttgg ttgcttccac 81840
attgtaacta ttgtgagtaa tgcgctgtga agatgagtgt acaaggagct ctttgagacc 81900
ctgttttcag ttcttttggg catataccca gaagtggaat tgttggacta tatggtaatt 81960
ttatttttaa tcttttgagg aactgccata ctatttttca cagcggccaa ccattttaca 82020
ttcccaccaa tagtgtacag atgttccagt ttctccacat ccttcttaac acttatgatc 82080

tgttattttg gtagtggcat cctaatgggt gtgagatagt atctcattat agttttgatt 82140
tgaatttctc cagtgattag tgatgttcag catctttcca tatacttttt ggccatttgt 82200
agatctttgg agaaatgcta ttcaagtcct ttgctcactt ttgaatcagg ttgtcagttt 82260
cttttgttgt tgttgatgag ttttaggaat tctctatata gtttgaatat taattcctta 82320
tcagatatat gatttgaaaa taaaaagttc tgtctgtggg ttgtgttttt actctgttga 82380
tgttgtcttt tcaagcagaa aatttttaaa attttcatga agtccagttg tctattgttt 82440
tttggttatt gtcgcctgtg cctttggtat catatccaag aaattatttc ccaatctagt 82500
gttgtgaagg gttactgtta tcttttcttc ttcttttttt ttttttgatt atactttaag 82560
ttttagggta catgtgcgca acgtgcaggt tagttacata tgtatacatg tgccatgttg 82620
gtgtgctgca cccattaact cgttatttaa cattaggtat atctcctaat gctatccctc 82680
cccgcttccc ccaccccaca acaggccccg gtgtgtgatg ttcctttcct tctaagagtt 82740
ttttgttttt ttttgtaata gttttaggtc ttacatttag gtctttgatt cattttgagt 82800
taatttttgt atatgctgct agagaagtgt ccagatcaag ttttcccagc aatgtttatt 82860
gaaatgatta tcctttctct attgaaccat cttggctcct ttgtcaaaaa tcatctgacc 82920
atatatgtga aggaagattt ctcagctgtc tattctatcc cattggtctg tatgactgtc 82980
tttatgccac accacattgt tttgattact gtagatttgt agtaagtttt gaagtcaaaa 83040
ccgagtactt tgttcttctt tttcaagatt gtttggctat ttgaggtccc tagagatttt 83100
ctatgaattt taggataggt tttctatttt tgtaaaaacc atcattggga ttttgatagg 83160
gattacaatg aatctgtaga ttgctttggg tagtatgaac atcttaaaaa cattaagtct 83220
tctaatccat gaacatagat gtgtttctac ttatgtcatc tttaatttct ttcagcaatg 83280
ttttgtagtt ctcatttcac ctcattggtt aattactaag tgtattcttt ttgatgctat 83340
ggaattgttt ctgtaatttc cttttcatat tgttcattgt tagtgtctag aaatacaact 83400
gatttttgtg tgttgacttt gtatcttcct actttgctga attcattttc tctattggtt 83460
tttttgagtg caatctttag ggttttctac ttatgagatt gtattatctg tgaacataga 83520
taattttact tcttcctttc tgctttggat gacctttatt tcttttcctg tccaattgcc 83580
ctggctagaa cttctagtgc tgtttaggat tggtcaaagt gagcatcctt gtcttcttcc 83640
tgatattacg aggaaaagct ttcagtctct caccattatg atgttcactg tgtgtttttc 83700
atatgtggtt tttatgttga gatcatttcc ttctattcct agtttgtcta ctgtttttat 83760
tatgaaaggg cattgaattt tgtcaaatgc tttttgtaca tcaattgaga tgatcatgtg 83820
tttcttttct ttcattctgt taatgtgtta cattacatgg attgattttc atttgttgca 83880
ttccaggagt aaatcccact tcgttatggt gtataattct tttaatatcc tgctgaattt 83940
gttttgttag tattttgttg aggattttca catcattatt cataagggat attggtctgt 84000
aggggttttt ttttttgcct ccccctgccc ctctccctcc tccctttctt ctctttctcc 84060
ttctccttct tctttcttct cctcctcctc ttcctccttc tccttctttt cctccttctc 84120

cttctcctcc tgctcctcct cctcctcctt ctccttcttt tcctccttct cctccttctc 84180
cttcttttcc ttcttccttc ttcttctttc ttctcttctt tcttcttctt tttcttcatc 84240
ttttgtctga cattggtatt ggggtaatcc tgacctcaaa gaatgaattc ttgttctctc 84300
ctcttcagtt ttttggaaaa ttttgagaat tggtgtcata aacactattt taaaaatagt 84360
gtcagtcagg catattactg tgtcacagtt tttactgtgt agtaagtgat cttccaaatt 84420
gtttataatg cttgctttta aaagtgacac atttcacagg gtgtctagct atgtctttta 84480
aactgctgcc agatatgttc ttccttttgt accttctgaa atctgtataa cctggggttc 84540
aacaaggctg tcaaaagttt caagaactga tctgatgaac tacattaaag ctataccttc 84600
tacaaaatat gtgactaaga agttactttc cctgacccca agtcagtttc tgggagtgtc 84660
tttgtcaaca tataccccat atggttcaca atcgatatga aagggctgaa ggagaaaaag 84720
aacaggtaat tggactctgt gaaaggagag aatgggaggt gaaggcatgt aaagaagaga 84780
gggggaagga gacatatatg tgtttgtgtg tgtgtatata tatgtgtgtg tgtgtgtata 84840
catatatata tacatatata tgtattattt tttcctttcc aatgtgtgaa tgtttttatt 84900
tcctttgtgt gtgtgtgtgt gtgtgtgtgt gtgtgtgtgt gtgttacttt acgttctggg 84960
atacatgtgc agaacgtgca ggtttgttac ataggtatac acatgccatg gtggtttgct 85020
gtacctgtca accggtcatt aggtatttct cctaatgcta tccctcccct agcccccacc 85080
ccccaacagg ccccagtatg tgatgttccc tttcctgtgt ccatgtgttc tcattgttca 85140
actcccactt atgagtgaga acatgcagtg tttggttttc tgttcctggg ttagtttgct 85200
gagaatgatg gtttccagcc ttcattcatg tccctgcaaa gaacacgaac tcattctttt 85260
ttatggctgc atagtattcc atagtgaata tgtgccacat tttctttatc caacctatca 85320
ttgatgagca tttgggttgg ttccaagtct ttgctattgt gaataatgat gaaataaacg 85380
tacatgtaca tgtgtctttg tagtaggatg atttataatc ctctgggtat atacctagta 85440
atgggactgc tgggtcaaat ggtatttctg attctagatc cttgaggaat taccacactg 85500
ccttccacaa tggttgaact aatttacact cccaccaaca gtgtaaaagc gttcctattt 85560
ctccacatcc tctccagaat ctgttgtgtc ctgacttttt aatgatcacc attgtaactg 85620
gcgtgacatg ttttctcatt gtggttttga tttgcatttc tctaatgacc agtgatggtg 85680
agcttttttt cgtttgttgg ctgcataaat gtcttcgttt gagaagtgtc tgttcttatc 85740
cttcgcccac tttttgatgg ggtggtttgt tttcttgtaa atttaagttc tgctgcataa 85800
ataaatgtct tcttgtaaga agtgtctgtt catatccttt gcccactttt tgatggggtt 85860
gtttgttttc ttgtaaatgt gtttaagttc tttgtagatt ctggatatta gccctttgtc 85920
agatgagtaa attgcaaaaa ttttctccca ttctgtaggt tgcctgttca ctctgatgat 85980
actttctttt gctgtgcaga agctctttag tttaattaga tcccatttgt caattttggc 86040
ttttgttgcc attgcttttg gtgttttagt catgaagtct ttgcccatgc ctgtgtccta 86100
aatggtatta cctaggtttt cttccagggt ttttatagtc ttaggtctta tgtttaaatc 86160

tttaatccat cttgaattaa tttttgtata aggtgtaagg aaggggtcca gtttcagttt 86220
tctgcatatg tctagccagt tttcccagca ccatttatta aatagggaat gggaaggaga 86280
catattacat gaagctggca agcttaggct ctgttgctga aggctaccaa cacctgcctt 86340
cacttatctg ctggacagca acattgtttg attgttttta gccaaccaac ccatctgggt 86400
cacttaatcc taaagcttct ggtgcttcac taactgcaaa aagactttta aaggattgtt 86460
tttaaagtag tgactttgat gatagtcgaa tctgtgtgta ttttctgtag aaatatacca 86520
attatgccta ttgggagttt aatcagtctt gtttttaatt attctgctat ttatatcttt 86580
tctttaaaat gaacaatcgt gatctaaaga ggtgatttca ttgcttactc atttaacaat 86640
ttcaaaaatg tggaaaataa gttatagaaa agagttttaa agacaaatta ggaatatctc 86700
agttgtcttt tttttttttt tttgtatttc cccctaggat ctgtggttgt ggttccggag 86760
aatctggttg tgctgtgtgt ggatgttgca aggcctgtgc aagagagtta gatggtcaag 86820
aggcaagaca aagaggaatt cttgatgcag tgaaagaaat gataccttta gatcttcttt 86880
taggtaattt tgattgatta tactatgcta cactgagttg tcctcaactc agtaagtctg 86940
acagtttaaa caatttcttt tagatatatg ttaataaatt aggataataa ttaatgtatg 87000
caatactgct tttacggtaa ctgacaatat gacattgttt aagggatgag attctttttt 87060
ttttttttct ttttttgaga tggagtcttg ctctgttgcc aaggctggag tacagtagct 87120
caatctcagc tcactgcaac ctccgccttc taggttcaag tgattgtccc atctcggcct 87180
cccgagtagc tggaattaca ggtgcccgct accacacctg gctaattttt gtatttttag 87240
tagagacggg gtttcaccat gttggccagg ctggtcttga actcctgacc tcaggtgatc 87300
tgcccacctt ggcctcccaa agtgctggga ttacaggtgt gggccaccat gcctagccag 87360
aatgagattc tttttccctt tttggctaag aattattaaa gtaaaaatat tgtgtgtttt 87420
agacaatgtt tttgctagct tttactttct tgaattttat gtagttctgt catagctatt 87480
atataacata tatgttgttg ttgttgttgt ttttggccaa tcatctagct gtcccagtgc 87540
ccggggttaa cattgaagaa caccttcagt tacgacaaga agaaaaacgg caacgtgtaa 87600
tcagaaggca cagattagag gaaggaagag gtaagatgta gctacagaga aaaagtacat 87660
gaaaatccac tttcctaccc tatcctgaaa ccactgaaaa gttcacacaa taaactaatt 87720
tggtaagaaa tcatcaaaat taaaattagc aatattctga taactcatat aatgggaaaa 87780
tattcagggt attcatgctt cttctaataa catacgttta ggtatttata attgtgctaa 87840
taacatactt ttaattcagc agggtgttta tggcataagt cagccatatt aagaactgtg 87900
ccatctgcct actatcaata actattagaa atggcaactt tttaaaaata gatattctca 87960
caacttgtcc ttcacccgga ctttggactc atctatttga ccgtggaaca aaattcaatt 88020
tagtcttttt catttctatt ttcttactca tttatgctca ttatcatttg gacatagtcc 88080
tgctatgctg ccctccagtt cagcacataa ctatgctttc atgtgtcctt ttttggaaaa 88140
acctcattct agttatttac aggaagagta agatggcagg tctgcaggtt tcatttgcaa 88200

agataggtct attgtttatc ttttcctcat tgtttatgag cttaaaagtt tcttagctta 88260
aaaatacatt taactggcca ggcacggtgg ctcacgcctg taatcccagc actttgggaa 88320
gctgaggcag gcagatcaca agatcaagag atcaagacca tcctggccaa catggtgaaa 88380
ccccatcact actaaaagta caaaaaaatt agctgggcgt ggtggtgtgc gcctgtagtc 88440
ccagctactc aggagactga ggcaggagaa tcacttgaac ctggcaggtg gagcttgcag 88500
tgagccatga tcgcaccact gcactccagc ctggcgacag agtgagactc catctcaaaa 88560
gaaaacaaac aaaaaaaatt taactgtggt tataattggt atttgaatac gcaaagataa 88620
atatatgtat tactaaagat ttccttgctc agtaaatttt ttttttgtag gattaagaca 88680
aattgttatg gaagtctatg attttattcc tccaataggc aaaattttga ttagaaatgt 88740
ttactgatat tgcatatttt aaatgcttat tagaaatggc atgatatttg atgcatgaaa 88800
taaacctttt acccatttaa ccatcattat atttttttct agctattctt tttgttaata 88860
tatggaataa gtagtaaaat gtataataca catggtgaat tctagtttta ctaaacttaa 88920
attgtaaaac atatacttgg cacatgaaga gtgttcttca tagcatctga taagtgatgt 88980
tgattgataa ggtgaatatt tactattgtt tggctttgct tgcataatgt cctttaaagg 89040
atattttaat gagattaata tagatactgt cagtatttac attatataag tatatgggaa 89100
aattgcatgt taaatatttc ttacaaaaca ggatagaatt aagtttctga tgtggtaatt 89160
aataattttt tatgttgata taattttttc agattagtta ccattctatc tgtttgctac 89220
ctgtttcttc tttctttaga aatattaggt tgagcgacat gaatgaacct acaaaatgca 89280
gctatttctt acatttaaac tacagtatag cacatcctta cacttaatta ttcacattta 89340
attacttgcc taatgcggtg tatcttgtta ctctttttct cttccctcta aaaaagctat 89400
tcttctctag gtatagtata tttgtgcata taggaaataa aggaacattg aatcgtgcat 89460
tgataacttg acataacttg gtgattgcag aatattcttt tgccatttat ttttaaattt 89520
gacataaacc tgtgaaacat aattgtaaac attaaccctt taaagttaaa aaaaaaatcc 89580
tccactagct aaatatttaa agaaattatg ttgttgtatc tgactttcca aagatagatt 89640
gaggttctag gcacttagtg tatattttgt gacaaatagg tttcatctgc agtcctaaat 89700
atatagaaca tcagtagaaa cactttgttc agattattgg tgtacataaa ttttatcttg 89760
tccttatttt ttggtagaac tctttcttag atcaccctcc ttcatgtttg gtaaaacaat 89820
atatagtgaa tatcttttct tgagtggaat cttcagtatg aacctcaatt atcacagtcc 89880
ttgctttcca tggcactaaa attaacagaa atgtgtgcac attggaacca tgtcctcccc 89940
tttcacagtc accacagtta cctagaatca tgcgaaagga ggacagagtt cccgtatttc 90000
tgtgtttcag ttaacacagc actatgcaaa gtgatgcctg cttgtatgtt ttgtttgttt 90060
gtttatttat ttatttattt tgagatgaag tctcactctg ttgcccaggc tggagtgcag 90120
ctgcacaatc ttggcccact gcaacctctc cctcccaggt tcaagcgatt ctcctacatc 90180
agcctcttga gtagctggga ctacaggtgc ccgccaccac gcctggctaa tttttgtatt 90240

tttagtagag gcgaggtttc accatgttgg ccaggctggt ttcaaactcc tgacgtcaaa 90300
tgatccacct gcctcagcct cccaaagtgt tgggattata ggtgtgaggc actgtgccca 90360
acctatttta ttttaaatga atatttttgc atttggacaa gaattctaac tatgtattca 90420
ttttttaaaa aatcaaactt cagagtcatt cagtgacttt ggaaatcctt atactgtatt 90480
aagtaattaa aaatctattc tgatatgaca cttttatgat tttatcttta tattattctt 90540
agcactccat taagcaattt ttatttaaaa acaaattttt ataagaaaac aaaattagat 90600
gaatatgata tacttacact tccatagaga taaaacaaga aagataaata ccaaaatatt 90660
aacaatgctt tttcctgaca ggtgagttta tagatatttt ttattttctt ctttacaatt 90720
tgtttcctaa atattctcca ataagcatat tagtgtataa atagagtaaa agttatgttt 90780
tacaactgaa gccattttaa aaaaatactg attaggttta aggcatccat actttgctta 90840
cctttgcttt ttaaaaaata tttttatttt aattggatgg aggactctct ttcaggtgct 90900
gtgcttttca ttactttctg gccatggttc tcaaggtggt ctcaaaggga ccagcatcac 90960
ttgggcactt gttagaagta ccagttctca ggccccactt cagatctcta ctgaaacaaa 91020
aaactggggg agtacagcca gtaaactgtg ctttaatagc gttccaggtg attctgatgg 91080
tcccaaagtg tgagacttag tgtgctaggt cagttgtttg cactttgcgg tttgatccaa 91140
tgaccttctg catcagaatc acctgggcct aatgaatcag aatttatggg tcaagggtct 91200
tggaaataga tctgagtcac ttgcacagtg ttctaaggct cttctctggc actgtgctag 91260
aaaagattgt agtggctttg ctcccatgag gaatagaaac atcaaataaa atgttagtgt 91320
tccggctgcc atacatacag atcttttatt tctttggcct gttgatactt tttagaaact 91380
aaaaatttag tagtatgtta ataattatac ttactagtaa ttggctttta cctgtgttaa 91440
catttcagaa tttaagaatt aggttatgtt agattatttt acttgacttt tctgtctcat 91500
cttatttacc aagtctgaac taagtttttc atagatgatc agaactctgg gctctagttc 91560
tttttttttg agtcacagtc ccactctgtt acccaggctg gagtgcagtg gtgcgatctc 91620
ggctcactgc aacctccgcc tctcggtttc aagcgattct cctgcctcgg cctcccgagt 91680
agctgggatt ataggcatgc accaccacac ctggctaatt ttgcattttt gttagagatg 91740
gggtttcacc atgttggcca ggctggtctc aaacttctga cctcaggtga tcagcctccc 91800
aaagtgctgg gagtacaggt gtgagccact gcgctcggcc tgggccctag ttcttgtact 91860
aaagccttgc tcagacagct tcaggcctat tgacgtaatt tagttactgt tttatgacac 91920
caggtctctt ataatggaat gtgttattga aacccaagaa agcaatgata ttactgcttc 91980
ttcatacatt tggaactttt taattttaaa tctttgtgtt attaatagtc tagttaattt 92040
ttttaattta tctttttagg tattaatagt attcatttat agattttagt tcaatggttt 92100
gtgataactt gtctttttta gattatattg ggttgattga tgtattaatt taactgtgca 92160
agtgaagtgc ttattcagtg aaattcttaa gtagggctct tgaagtcatc agaagaacta 92220
gtggatgatc atatcaatga ttttatcctt atattccttt tcagcactct gtttcgtaag 92280

gaaaattaaa cttcaataat gttagctttt tcttctttct tctaagtatc caagtttcat 92340
aattgtgagc tgtagatggg ctaaacccat gtcctggctt acatggagaa gtcccatttt 92400
ataggtgtcc aggcatattt cttttttttt tttttttttt tttttgagaa cggagtctcg 92460
ctctgtcgcc caggctggag tgcagtggtg ctatctcggc tcactgcaag ctccgcctcc 92520
tgggttcaca ccactcttct gcctcagcct cccgagtagc tgggactgca ggcgttcacc 92580
accacaccgg gctaattttt tgtattttta ctagagatgg ggtttcactt tgttagccag 92640
gatagcctca atctcctgac cttgtaatcc gcctgccttg gcctcccaaa gtgctgggat 92700
tacaggcgtg agccaccgcg tccggcatgt tgctattact ttttaagccc ttaaaaccat 92760
tatggttttc ttgatttgac gcatcaaatc caatattagc tgatattcca gtgacacata 92820
aaagtctctt tttaaattaa agtattatat gaagaattta gagagtataa gtaagaaaaa 92880
atagatcagc ataacccttt atacaaagag aaccattagt agaattttgg tgtaacttct 92940
cacacttttt tacttatttc atataatttc atactgtgat agatattggt gtcctgtttg 93000
tattcccttt aatattatat catgaaattt ttttgtgctt tattgcaaat accatattaa 93060
agtaatatag gaatttaggt caaggttttg cataaaccta ttccttaatt caattattgt 93120
ttttatttcg ttgtcatctt ctaatcaagt tttacttatt tctagttata tcattgactg 93180
aattttgttt tacatttttg cttagtgtta taatacatat atttttatgt tttgcttgta 93240
ttgtacccct accttcccat ttccatcccc ccctcaaagt aaatgttaat agtctaatat 93300
cttactctat taaaatataa tcatgtacag agacttacac atatgtgaaa gtgtagacat 93360
atgtttattt acaaaagtag tattctgtat atacatttgc aacatctttt tctcatttta 93420
caatacaaga agaacacact gtaggtcagt aaatgtggat ctaccacatt ctatacagta 93480
actacctaat atcataatat ggctatacca tagtttatat aaccattatt tattgatggg 93540
cattcaggtt attgtaagtt tttgtgggtt ttactgtaat aaatattctt aaacttagag 93600
ctacatatta gtgtttgtat agtagagctt tcccaaaata gaatgtgtgg ggcagagagt 93660
atatatctgt cttaaatttt aacagataat tcacattgtt ttaaaaatgt tataggaatt 93720
tgtttatact ccctagacta atgtataaga gtagtgtttt cctctagcat cacctgtatt 93780
cattattctt ttatttattt atttatttat ttattttttg tagaggcagg atcttgctat 93840
gttgcccagg ctggcctcga acaactgggc ccaagtgacc ctcctgcctc agcctcccga 93900
gtagctagga ctgtacaggc atgtgccacc acacctgggt ttagttattc tttttaattc 93960
ttggaaaaca gtggtgaaca atgttatctc attgttaatt taaataacac aaaatcatat 94020
tctgtgtaaa attatttcat aaatctcatt ttaatggctg cgtattattc catttagtag 94080
atgtattata gtttttaatt actttcattt gggtattttt aaaatgttgc tataatgaat 94140
aaagttgtag taaacacctt tgaggtgaaa tcctttaaaa atattttaga gttttcttta 94200
ggatagattc ctagggtgca gttactagat caaaggataa taatttttat gatcttgaaa 94260
tatatcacca agagctttct gaaaggcttg tactatttaa cactgccact tattccatgc 94320

tttttctgcc tacctgccta cagatgaact ctgaattacc cattgtctac acatgagtgt 94380
agcttctatc taaacctggt tatctcatac ccctatttta ctcactcctc tcccaatgaa 94440
agaaataaat caccaagttc tatttataca gccttcttaa ttctttaata gctttttaat 94500
atctactttt cagaatctta ctgtttcctt agctgaggct tcttttcttt gcctatctga 94560
cagcaaaaag ccttttaact ggccttacct ccagtcttat cttctaagcc attagccacc 94620
ttctttccag aatattcatt ctgcaataca gatcagatca gtagcacttc tctgtttaaa 94680
accaaaaggc tattcatagt ccttaggatg aaggatatac ttttatctct acagcctcat 94740
ctctctctat tctcatttct tttcccttgc ccttcctttt tttcccctac ctctgaacct 94800
ttatgctact tgttacctcc tttatttgac ctcatcttat gtggtggttt cttttatctc 94860
tacactgtct accttcccac cctcaagttt tgttagcttc tcctctagtt tgtgtgcctc 94920
cttttgttat agcatactgt cataatttct tgtttagttg tgtgtaaacc ctccctcccc 94980
tcattagaat gtgagcttct tgaagcagag attgtatctt gtttatcaag ggttcccaga 95040
acttcacatg gtacctgtta gtaccatgat ttgtagtata tttttgttga ctaattgaaa 95100
gcaggttagg gacttatcat ctctagaatg aagttttgtg gtataaaaag gttttaatta 95160
tctgtttttt tgcatacctc tccaagcaaa tttcccaaga cttttgctcc atcacatata 95220
ctctgtaaaa gtcagctaca tgtattttta atgactatac aatattgttt aatatcttta 95280
tgcttttata aaatcctaat ttatatccta ttccttttaa ctggaatgcc ctctcttcct 95340
ttccttttct cttgttaaac tttttgtcat tctttgaaac cttgctgatt gtcctgagtt 95400
tatttagtat cttttcttac ttcaggacat aatgtgtata gatttctttt atagcattta 95460
ttgtaatatt tcatagtttg tgtttttatt tctgtttttt tccctagtgg actgaattcc 95520
agtgagacct gtatctttgt cattatattc atttcaccta gtgtctgcta aataaaagat 95580
aatcagaaaa tttggtgaat aaattagtga ctcaactata tttaaagaaa cattatttta 95640
gcccatggga tacaatagta ttttcgagcc caattgcttt aatttttaga catgattgcc 95700
cccgtgcctt cttgatttat aaatcaaaag attctaaaaa gattgaattt agcaagacat 95760
taaacgggtg tgggtgtgat gtattttcat gaggagaaac aatattgtag gcagaccata 95820
gaaatcagaa aatggtaata taacaatatt attaccttaa tatctataac ttttttatat 95880
agttggaaaa tactttttat agtaatttat atatctgctt tgtaacaatt gagaaatcat 95940
gcatgttaag aaaataaggc attttgttca tatttgttca tatttgaaat atcaatttta 96000
ttacatttta gaaatggaac tcagtttaca taaccttagc ctgagaaaat aattggtact 96060
gcattattga gaacttattc ttctgttaac attaaattag catgtttcta tctgtagtag 96120
tcatagaata tgttattcat ggtttttcat acaatttgag gtatttgcat ttttgtttat 96180
atctagaaac ataattgaaa tatggttcct ttacccactt gtacatttaa tgaagggtac 96240
tttgatattt aatttgacat ttatgaaatt ttattagagg gcagtaggct caatttgtcc 96300
catcattaga aaaattaaac tgaagatcaa tagagaatca tgaaaaaact tgttttaaat 96360

aaaaatgttc agtgcttata tagtgtcata attcctgttt cagttaatgg taacctgtca 96420
aatttagagt gttttcaaat ttgaagacat ttttgatgtt gttaaaactc aaggaaacta 96480
tagttctgct gtataagaaa aaaacctaaa attttaagtt gtaactaaaa tttatttttc 96540
ttttcaaggt aaataaatgt taatgggaaa aatgatttat tttattttat taaaataaat 96600
ttggctgtat attttttcct taaataccat ataagtggaa agagctataa tctgtgtggg 96660
tatgtattta tatatgtata tatatatgta catagtggca ctttagaaga aatgatttaa 96720
tgaaagatgt ttccaaaatg ttatttgggt taggtgcccc ctttaccatc aatataaagc 96780
ccaagctcct ttctctctct ctctctctct cgttctctct ctctctctgt gtgtatacat 96840
atatctatat gcagaatata gacatttttg ttttgttttt gatcatctgg ttttcttaac 96900
ctctatttat gatttgtagt aaactcggta acatttttat tttggttttg tagcaagatt 96960
aatacaaata ttaaacctgg aagagcttgt taaaacttaa tggttaattt aaaaattagt 97020
ttttatgttt caatggaaaa gatatctgtc ttcaacgaat acatggttta ctgactttga 97080
atatatatga tattaaaaaa gtatttgtaa gataatttga ttgatttaaa ttgaacttct 97140
tgattatcta tttgagacta ttagtaacta cacaataatt ttatgagtat acaagacggg 97200
ttttgtactt tataaattat atttaagata ttaactttta tgtgaaatgg tgattaggtt 97260
atgggtgggg gtggggggct ggagtgggga aattggaaga aaagtataat gtccaaaacc 97320
gttaagaata aatttttatg ctttttagtt tgaattattt tttgttcatt atagtgtttt 97380
cattttgtga gtttttctat gcacatacaa aatgtttctc ctcaggcccc cttgtatttg 97440
ctggtcctat ttttatgaac catcgagaac aggctctagc cagactcaga tcccatccag 97500
cacagctaaa gcataaacgg gacaagcaca aaggtatttg gtcttcctta tcacctaggt 97560
gggatacttt cccaatttct tccactcatt tgaattttgt ctctgggaaa atgcacaaac 97620
ctgattcttc tactttgctt ctatcatgat gtagcctgag gagaaagaac attctgaaag 97680
tgataccgtt tgtcagtgac tgtcatgctt cttgtttttt tgttgtcgtt agctttcata 97740
aaggagatat tttacaatat atcttttcct tgtccatttc ttttcaaatt tcaaaaagtt 97800
aagggaaggt ggaaaaaata attattgctt tctgtcttac atcctttatt atttgctctt 97860
taatgactgt gatctggcct tttacaattc ttttttgaat gctgtttata tgctttagga 97920
taaacaatat tactaaattc agtatattaa atgatgataa taagactgcc attacacttt 97980
tccactgttt attaattaag tttatataaa caacaaatga atatacaaat atttgatggt 98040
tgtgggttaa gtagagataa gtgcaagcaa agcacgaatc atgaagactt tctctagtcc 98100
ctggtgatgg tcagagggct atcctagtct gtgaggaagt aggaatctag ctcggtgttg 98160
tcctttgaga tctcagagtt ctgggttaat taaatatatt cttaaagcat gcttattttc 98220
ttgtaaacca caaattatgg gtcagctaag atcaggaaag aatttaatac aaggaattta 98280
taaatttata taaaattttt gccttgtatc ctggcctttg aaccagtgtt cttccttagc 98340
tagcacaact taaaactccc attttcttaa atgcctaaga taggtcctgc aaaatagaat 98400

aggaagtttt aaaaacatgc tttccatgta gacaaattaa tctaattaat gtttggatcc 98460
ttttcttttt tttgctgcaa aattgtcata gtctaattta aaaaaatttc ataggcaata 98520
tagtataatc cactttattt ttatttgcct ctttcagtct gtcagtttgc cactcaaaac 98580
aaaaacataa acaaaataga caaaactcat ctttagtaaa ggtgtaaata gagcacaact 98640
taagatatac tctgcagaaa taaaaatagt gttttaaatc taatgcaatt aaccacagta 98700
aaatttgtga ttactttatt tactgagtgc tctaacaatg ccataaatat ttaaggaatt 98760
ttcaacctaa gacagctgat tccaaaaaga aaagatacat atataaggaa taatatttgg 98820
ggtatgcaat gaaattgtta tttgccagaa tgtctgatct tttatgattt ctactatgtc 98880
acaattagaa cagatttttt cagtttcttt cttcccccaa atatatgaaa gtgaagtttt 98940
cccaatagtt taactgaggt taaatattca catttcatga aacagtttaa aaccttttca 99000
gctccatgtt agtatctcat ggacaagtat ctttaccctt tccagatttt taaatgaaac 99060
gttaaatgaa aaccaaggtt acatatttaa agcaaacttt tcccaatgtc acataatgat 99120
tggctataga aattattcag tgaggctggg cgcagtggca catgcctgta attgcagcac 99180
tttggaaggc tgagggggtg gatcacttga ggtcaggagt tcgaggccag cctggctaac 99240
atggcgaaac ctcatctcta ctaaaaatac aaataataat gataacaata ataagcccag 99300
tgtggtggca catgcctgta atcccaggta ctcaggaagc tgaggcatga gaacaataat 99360
cacttgagcc tgggaggcag aggttgcagt gaaaaaacaa gttattcaat gatagctagt 99420
cttcaagttt gcttgtcatg gggttacttt ataacaagtt tctttgtata cttgtaacca 99480
ctctgtaagg acctactttg taatatcaca gtgtaggacc agttttattg gattttaggg 99540
ttttatagag tgagatagtt ttatcttttt actccccaaa tacatttaca atgcaaataa 99600
taatttatca attggtaccc tatttttttg tgaagagtat taatttactg ctcactaatt 99660
tccctccagc cactaaccat ttacactcac tgtcttgtca tttaattatc tctccattca 99720
ctgcttttac ttttacgaca tgttgaacat ccctaatttg aaaattcgaa atgctccaag 99780
atctgaaact ttttgagcac caacatgaag cccccaagtg ggaaattcca cacctgacct 99840
cctgtgacag gttgcagtcc cagcacacag cttattcagt gtcctaaagt gaaagcaatc 99900
ctctcagccc tcttcagcta tgataaagct tttccatgca cagcatgatg gtgacgccaa 99960
tcacagtttg tctacgtggg tggctgggtg gctgaggtac ctttgctttc tgatagtgca 100020
gggatacaaa ttttcatgca ccaaattatt taaaatattg cataaaatta tctttcggct 100080
atgtgagtaa tttgtatatg aaatatcaat gaatttcatg tttagacttg ggtcccatcc 100140
ccaagatatc tcataatgta tatgcaaata ttccaaaatt tgaacaaact ccaaaatcca 100200
aaacacttct ggtcccaagc atttcaaata agggatgctc aacctgtaca tattttcagt 100260
cttttaataa gtattttttc tacatatatt tttctcttca ccttacccag tttatcttct 100320
agtttatcta ctgagaagtt tatggtattg attgctatgt tctatcctac atttaattat 100380
gttggtacaa attataggat ggtaatagtt aaaatttttt agactgtgac ttttttgtga 100440

gggtgtaagg agagtaagaa ctgtggattt attgttaaca ggactaagtt ctaagatgga 100500
aagttggaga acttctgggt gattggagaa aattcagaga aggtcagaat tcatcctata 100560
caggcagata ttgactaaag gcaaagcaga gtatatagac tgaatcaata agtaaaattc 100620
catataaaat acagtaaatt attctaaaat gtgcctataa actatattcg aaacagaatt 100680
aacatttgtt tcagctattc agtaccttac acagaaaaat gtgttaatag tgtacatagt 100740
taggaacatc tgaattgttc tagagatagt cctaaaatct attttaaaat gactgatttg 100800
aaaatctgaa ggcacactga taaacatctg agtctatttt actcttactt tctgtttgga 100860
aatggtgcag tgtgatgaaa atattatagc ctttggaatt aggcttcaat tttatttctg 100920
aatttgtcat ttactagaac tacatggctg tgcaaaatgt cttaatctct ttggcttcag 100980
ttttcttatt ttaaaaacaa gaatagtaat tcttaactga ttgtgttgct atgaagatta 101040
aaatatatgt gtgtagtctt tgcacaccca catatgtagt ctttgcacat ataaatcctc 101100
catgaatact agctttaata ttttgaattg aattatttgt tttctctttc agaattatct 101160
taaaggtaca atatttgaga tttattaaat gcatagttta tttttacaaa ggatttaagg 101220
tttcaaccat ttggtggtgg taaaatgcat gagccttatt tatgattaaa ctctaaattt 101280
gtcctaccat tttatgattt gggctggtta cttaacccct taacccttca agccttagtt 101340
ttttcatctg taagatggac aaagaatacc tatattatca gattgcttta ttcattaaag 101400
aaagtaacaa tagtaaatat tgattgagca cttttcatat tttatttaat tctcatagga 101460
acctgatgag ttacatatca ttgttgctat attatagata ggaaagctag gaccagaagg 101520
tccagtgtcc agttgtcata caactagcaa atgagaagag ccaagattcc agtccaagtt 101580
cttaagttcc agtactccca agcactcatc cactgtggta tttaattgtt tacagtgttc 101640
agttagcaca cagcaagtgc ttagtaaatt gcaactactt ttgtccttgc tgatttgttt 101700
taatgatttt gctccaagta ctcactgtgt tctgtggtag tttctgttat atgcatgact 101760
gtaggattac cctccaaaag tatagatttt agtaaaaatt aaaattagaa gttagagtcc 101820
cttagatact gaaccctctt ttttctctgt gtatacaaat atgtgtatat aattatatat 101880
aatgtgtaca tacatataat attttagtta catagttgcc aaggctttgt agctactgta 101940
gtttcagtgt tacatggtat acacacacac acacacacac acacacacac acacacaaca 102000
tattcatctt agggaaaata cagatgtgct catacttaaa tggccaatta taagattatt 102060
cacatctgtc aattttttat tgacaatgtt taatttttta tatagatcta agctttggtc 102120
ttattgctgc tcattaacac tttactgaat aatgagcttg aacagatttt tttttttttt 102180
ttgagacagg gtctcactct atcacctagg ctggagtgca atggcatgat cacggctcac 102240
tgcagccttg acctcccggg ctcaggtgat tctcccacct cagccaccca agtagctggg 102300
actgcaggta ttcaccacca cacccagcta gttttttgta tttttagtag agacagggtt 102360
ttaccatgtt gcccaggctg gtcttgagct cctaggctca agtaatctgc ctttctcagc 102420
ctcccaaagc cctgagatta caggtgtgaa ccaccatgcc cagcttgagc agaattaatc 102480

ttgctgaatt cacttgctgg ttcttgaatt tggactaaat ttttggccac ttattaagtg 102540
ccttttttct ttgataaact ttggttaatg tctgagttat aggaattata ctcaattttt 102600
tgatatcaaa taccatatac agatatatat ataaaatata tattttaatt ctgccactgc 102660
tgctgataaa gattaagacc ctcatctcaa tttttttact tgtaagattt tcactgtcta 102720
taataattga gaagttgttt tgtacagtta cagtttgttt caatgaacaa aatgtttcta 102780
gtattatttt gacctctaaa taacagctgc tactttttta gtcattgtgg ttgtattcac 102840
taaattacca acctttaaaa ataatctcct tccttgatgc tatgtctcta ttgtgggatt 102900
tatgcctata tttaatctcc tactcaaggg aggagacaaa atgctaaatt caaaggtaaa 102960
accttataac taggcatcaa tacatttata taaagtggaa tgatacttga aatgtttaat 103020
taagtctggc agaatcatat gaacacaatg tattttctat gttaaatctc atttggtttt 103080
aataattaga catttttacc aattgagata aagccgtaaa atttctttac atctggcgtc 103140
ttattaaata gcctttggaa gatatgtatt tgtgtgtgtg tgtgtgtgtg tgtgtgtgtg 103200
tgtgtgtatg tgtgtgtgtg tgtgtaggct tttgcatata ttttaccatt ccactatctt 103260
tccaacatga ggaggctttg aactgagtat attttatttt ggatggagtg aaactaaatg 103320
ttgttggttt tttttttttt tttttagtcc atccattctt tgatttaatt tggcaaaccc 103380
acattagata atttagcaga agaggaatta tatcttcatc ctattatagt aaaacctctc 103440
actaattctg aatttatgat atttgagatg cagtatttgt gatctttttt gagtaaaaaa 103500
ttcaaaataa tttcttcctc tgaattttca gggtcatact tataagggga aacctgtcta 103560
aaatcactag cttttcaaac caagggctca agaaatgaag gattccttca acgtgccttc 103620
agcctctggg tccagcagtt aattcttgta tgttaaagga cttttatgtt tatggtacat 103680
tttaaatgta atattggcct attttgaaat ataatgtagg taaccacact ctatttattg 103740
tagcatttag tttaatctgt aatctgtact gcttgaaggt aacaaaactg aatttttact 103800
tcagtgttct gtatgattaa gacgtttcca gtaagccaca caggcctacc tgttctatgt 103860
tattccgaat tagtgaggtt ttactgtcct agtgtcgtaa agagtttcag tcttcttgaa 103920
attataggca tttagttacg gaaaggaaag taataatgta ggtagatttt gtctcacctg 103980
gatgttacta aaggttaggt aaaaaaagaa taaacacaaa atgatttaat attattctgt 104040
ttaacattct aaaatgcaca atgaaaatac aggatagaaa gtgtgtgtct gtagattcac 104100
atatgtatct tcatataata cacacttgtt tagagttgct ataccacttg agagtggttt 104160
agactagttg aacttgtgaa attccttcca gatttacatt tattctgtgt acaaggaatt 104220
tgaatcttga tgatataaag gtcatatgtt agtgatgtat agaagtacat tgtgattaga 104280
aaaagtaagt tgttgttatt tacacattcc aaacaaaaat ttttatattc tggaatgtgg 104340
tttttgatgc tatggttaga ttgttggaaa attcaaagtt ggattgcaaa agccaagggt 104400
aaagaaaagg cttaaagcaa gctcaatgaa ggaaattgtg agaagggagg aggaaagaag 104460
gagtgaaagc agaaaaaagg tttggttgag ggtagtgatt ggggacaggg agaaaaccac 104520

aaagcaatat ggctgaaggt gatggtggca caggaaacat gaataagaat aggccattga 104580
actgcaatat attgaataaa gctctttggt ctttatacat tttgaaaagt tcagttgagt 104640
ttcaaaatac ttatgggcag tgtagccttt ctaagatcta tttaggaaat ctaagtcact 104700
tgtgaatcca ttagcaattt tttaaaaatg gctagtacta acaatatcac taagtttaaa 104760
ataattgtta aatgactttg catgcagtaa ttttttatgg aaactaaatt ttcccatata 104820
aactgttaca ttttaaattt tgttatttct ataacttaat gacccgttta ttacataagg 104880
ttttatccat aaccagatct tatctctgtt tttattaatt tagtgatatt tgcaaatgga 104940
agtatatagt ctatcttttc aaaattcctt ctttgcaggt agattcttga tctatcactt 105000
ctaacagcta cagattcttc ttgcttgttc cttttaacat ttttctcttg ttcttccata 105060
ataaactgat ttaaattttt attttgaaga tgaataatgg cttaatcata attcacaaat 105120
tataacactt gcagatggaa gtggagaaag aggcgaaaag gatgcaagca aaatcacaac 105180
ataccctcca ggctctgtgc gatttgactg tgagctccgg gcagtccaag tcagctgtgg 105240
atttcaccat tcaggtagcg gctggaactt tagggtataa ctgcgttttt acaattgtgc 105300
tcagtaactt atttcttcag atttgatgaa catagcctgt taactgtgtg ttaaaagctg 105360
tagcgtggca gtctctcaaa tctgtaagac ctttgtattt attacagaat ctctttaaac 105420
aactatggca tgatggaaca ggtacttgtc tagagatcta acttctagtc tgtcttcctg 105480
tggacttctt ttataattca agacagtctg gaagatgaaa taatccttta aatctctttc 105540
agttctagaa tgctgtggtt tttgtaacat gttcccatat tttgttaatg ttcagtgata 105600
aatgtatgtc aacaaattta ctttcttaaa atgtagaaaa taattaaatt cttagtaaat 105660
tattggcatt tgggttagct tttgctgtac tgttcaacct ttataaaaac aaaaatgtta 105720
aattccttcc ttgaagatta tttgataagg tattaacaga cattaaagtt ctgctgtttc 105780
attgatgttg tacgtaattg tgcagacctc ctatcctaga taggagagaa caatatttta 105840
tatgtagtta ctcattatta agaagatttt acagttgacc tgaaccagtg tgttaataag 105900
aattaatgtt attaaatggc atttatacga atttttaagt gtgtattatg aaaaaattat 105960
acatttatat gacctacaaa aatatttttc agaactaaaa ttaatttctc attaaataca 106020
aatttgtcct tcagttacct atttttaatg gttctataaa tcttaaatag tgaaaattac 106080
actgctttta gtatagttgt gatagaaatg taaaatgcat ataatgaaaa aataaagctg 106140
tgtgttttgg ttgcagtggt tttaatggaa aatggagatg tctatacatt tggttatggg 106200
cagcatgggc agctaggaca tggagatgtc aactccaggt acttgctaac ttttcatttg 106260
aagatccaga gttatttttt cttctgtttt cttttccttc ttttttcttt cttttctctt 106320
tttaagtata tatttctaga attaaaataa tattatacca ctgctttgca aagtgtggcc 106380
catgaattat tggtggtttc tgatcttatg gttgagtgct tttgtagaga taaaggattt 106440
tgtagtttca ggagaaaagc tgatatatat tttaaatagg agagtgatta tgattgtcac 106500
aaaatatcac tatattctta gcaattatct ttttaaaggc tgtgcatggc aatagaaaaa 106560

tagtgaattg tatttttaaa aaatgtcagc aatggattca aacatattcc tgatgtctgt 106620
tttgaaattt tggtctaata acatgcagtc attgaagctt ttgaataatt tttggacaga 106680
acctattggt ctctctgatt aactcttttg acttattgca aatagtagta gagagactgg 106740
tggtaatttc ctaatatccc tacttcattt gttttagttt tagttttagt tcgtgacatg 106800
atcgtgcagc taaagattcc ctttctcagc ttccattgca gcaggatgtg gccatgtgaa 106860
cttaagtgat aggtgccact ttggaatctt gctgttaaag ggaaagggtg tacctttcca 106920
tttgtccttc tctcttccca gcttgctggc tggaatgaat atgtggaagc agaaactaaa 106980
gaagtcatca cagagataag ttggagactg tgtactgaga atggcagaat cataagatgg 107040
gaataggctg ggtctctgac accatgaact gcctcatcag cttcttaaat tgcatgcttc 107100
ttgcgtgaga aataaacttg tatcttgaag tccctcttac ttgtctttca ttacagcagc 107160
cgagtttgta ttctacgtaa tgcagtcaga atacttttgg tcaaaagtaa tagaaaatcc 107220
atctttatta gttcaggtct tccagaaagc agatgccaag acaggattag atatgcaaag 107280
aatttattag atacatgtag ttttgaccct atgaaaggag aaagagaagg aagaatcggg 107340
taggagtcta ggttccagca taacattaag agagttttag ccaggccagt gatgtgtccc 107400
caagctgagg atacctttgg aagggtcctg cattgggcag gaatagacca acattcatac 107460
cttcaccatg ttcagtcaca gctggtagca ccccagggga cgaatgtcct cgtgcatcca 107520
aagggacagc acctgaggct gttataatag ttaactctgc tctctgtagc agattgtctt 107580
gaaggagatc tgagtggtct gtgtctgtgg ttgtctcacc atggaaacag gagtatttat 107640
gaagtctgga aagatagaga gcagcttcaa gccagacttc actcagcaat tcacttagct 107700
gcaaaaactt accttttgac cttctactct gccttattct tcctcgcagt cacagtgtat 107760
atgccagaat caaggggact tctatggttt ctcctttaca tacagtgaaa gaaagagaac 107820
ttaccttcac atagtcatta aataaaattc aaggttgtca ctgtgtcttg aactcactct 107880
actattgtgg cagggatgga attctgccat ttacatggta aagattagtg attcttaacc 107940
ctggctgtat attagaatta cttagggaat ttgaaaaaat atcatatgtg gattctacat 108000
ccagcatttc tgagagatgt gtgtgttgaa aggcttgtgg gtgggacagg ggtggggagt 108060
ggtccttgat aggatgccag catcagagtt ttttctaaaa ggtccccaga ttctatatgt 108120
atccaggatg agaaccactc tctacccttc gagctgaggc agggtcagat ctgtgggcac 108180
tgcataaaaa atggcaacaa aagggaatgt cctgagttat gtttatgaat gttgttgcta 108240
atgataagag acgattgaga tatcattgag ttgaacttct tattaaaaac acaactgaaa 108300
cataaaattg atactactat caagaggttt gtaaaactat cacaatcata aatatttaat 108360
gactaatatc atacttgcaa agagcaaaag atgtgtttgg cagaattact ttatcagatg 108420
tgtctgctgg atatgacata ataccaacag tgttgagcaa ttactgtcac aggtgttaga 108480
tgccaactga tattttgctt tgcaattgag taacattact gaaatgcaga ggctgaatct 108540
gggtagtaat tacagttggc cctctgtacc tgtgggttct gcatctgtgg attcaatcaa 108600

tcttgacaga aaacatagtt aggcctacga taattgcatc tgtactgcac atgtacagat 108660
gtttttgtct tgttattatt tcctaaacaa cagagtataa caactattaa catggtgttt 108720
acattgtatt aggtattata agtaatctag agatgtttta aagtatacag gaggatgtgt 108780
agtttatgta caaatactat gccaatttat ataagggatt tgagcatccc tagagtttgg 108840
tagctgcagg ggatcctgga accaattccc catggatgct gagggatgac tataacctgt 108900
gtggggaaga acgtggcaac tttttgttct gtttctcatt tgagagttgg gacacagaag 108960
aagagggttt atttgtatca tcgtggctat tgttgttgtt atttagctgg tggttatttt 109020
gtgagccata gaaactggga aaggtgcatt gcagttagat aagatggggt ctcagtcatg 109080
tatgcagcaa agagggcact tcacagaaaa taattggcaa atagtatttc atggtcttgc 109140
cagagtggaa agggaagtag tgaaaagcat ggccatgtta tagtcaggtt atgttatctt 109200
actgtgcact tggcactcag aagaatgagc agcatgagca caacatcctt ccacaccaca 109260
ctgaggtgtg ttggccgctg agagggaagg tgttcatgtc agcttttaaa atgaagagat 109320
tacctaaaaa cattacttcg tggtcctgat tataccagtg aacaccattc ttcgattccc 109380
aagtctgaag agatataggg cagtttcagg ccagacttcc tcaggtaata atattgtagc 109440
aatatgctca atattttaaa ttgcttgaat ttatcgtctt catgaaatac gatagcattt 109500
aatgttgaaa caaaatatca gggtttctta tgaagattaa acattggtag gatctacaca 109560
gtggtatagt aactggtaga tgctacatag tcatcgaacc tggaactgta gaggcaaggt 109620
tcagctcttc acttcttacc aaccatatga ctataatttc ttaagattaa acactggtgg 109680
gatttgtttt aactgcccaa agttgttctt ttccagcatg gaatgttttg gtccttgttc 109740
cattgagtgg taactgaaag actgattaca tgaagtgtgg gcagagctac aacggataat 109800
gcagtagtca tacctatata ctgtgcctag tggattagat attcatcaag accgcttcag 109860
tcttaagcac tcagttaccc tgtctgtctg gacttttgtg gaacttggaa agtagcaacc 109920
aagaactctg ttattaattc agaaaggagt tgattatgtc tatagaggaa caatcttggg 109980
aggaacaaag cttctatagc atatgtatta actacaacaa agcaaaacat ttacagatag 110040
ttgaaataaa gggttacagt tggagaatat gtttgaaaag tctctagtaa tcaagagctg 110100
ttaactggaa tttactatat agcacacatt aataatgtgt tgtttaagat caatattgtg 110160
gtatgcatga taatgaagat ggaaaagtgt tattgatatt ctctttattt aatgtgttat 110220
attgccagaa tgactgagtt cagtctctag gttgggtcct gattctgtca cttggtagct 110280
atatgatctt ggccaggtta cttaatcttc ttgtgtctca gtttctcaag tttaaatatc 110340
cataatctga tgatactttc ttcttctgtg atgcagccct actttatttc cctttgactt 110400
tatgccccac agccctctac ttttttactc ttttttcacc ctttttttaa ctttatttcc 110460
tactcaagtt aaagactgtt gcttaatttc ttcacctata ctcttaccag tttcttcagt 110520
tttcccactc tcttggtcgt ggtgctagga catgataaaa ccgcaaacat gaatcaatct 110580
taatatttcc tatgcctata cttaggcttc tggaaggtaa gaaatcacca tacctatgca 110640

gattgatact taatttccag attcaaccag ttcctgtaca atggtcatta atcctcctgt 110700
tgtgtagtat tgaactattt gacttaatac cttttgagag tttgatgaaa gacatggggt 110760
ctctatgaaa aatacaagaa tatacactta aaaatttgct tgcaaattta ggagatcttt 110820
tgaagctcac tcatggactt cctagaagtc ctttatatct gagttgaaaa ctcctgctct 110880
cggtatgtat ctattccatc taccaccaca ccttttctcg aatcttcaaa ctctccactc 110940
acctgtttcc tccttttagc ataggatctc catgtacatt gcaacagatg taagaaatta 111000
ttcagcccac caaccctgca aacctacttc catccatacc acttctctgc ttctttcctc 111060
atgttacagt taaagaggaa cctgttgtgt ccttgctatg aaacctagct gtcttttcct 111120
tagagatcta tttctgtttc ctgtcttctc tctaaccttt gtctttagcg tctttatttt 111180
gagtcacctt ttaaacttcc tcaaatctct tatcattaaa aaaaaaaaat cctctccttc 111240
agcttcattt ttcctttagc tgctggctct cctttctttt tccctttagt gctgggatac 111300
ttagagctac tttgtgtatt ctctgactcc ttatctgctg cattgtggtg cctgcttcca 111360
ccaaagagac ctccttctgc taaatcgagt ggttaggtgt atgtctcctt agagcatttg 111420
gcatgtactt tattttccac gcttgaaacc tactcctcca atgagtctcc ttgagttctt 111480
tctttctgtg tggtttattc tagatgtctg tgagctgttc ttcctcggtc tgtatctaaa 111540
atgttagaag agactcataa gggaaattag gatctcttct tttctctctc aaagagtctt 111600
cctagatctc attcattctc atagcttaag tgaatattac cactatctac tctgttgctt 111660
aagcaaaaaa aatcttcctt gccccatatc gatcatctgc caagtttcat caattctgtt 111720
ccttacatag ctatcaaagt caccatctct ttcactctgc actgctccta gttaaggcct 111780
tcatcataaa ttacccaaac tactatataa tttttaaaat ttaaagtgtc ataattacat 111840
ttctcaaaag cagagctctc ctgctatgtg tggactattt ctataatggt tgtgtttcta 111900
attacagata atggttacta aaaatggctg ttaaaagtag gaaaggatac taggatgtca 111960
tttatcataa tagttactgt tcattaagtg ttaactgtgt gccagacgct gatccaagta 112020
cttgacacgt atgggcctct ttactgttca caggaactct gtgggggtag acaccgtttg 112080
ttagtattct ccatgtgata aagaggaaac tgaaatacaa aaagaatgaa aaacttgttc 112140
gaagtctggt cgcattatta ttaagttggg gagccaggat tcacaaccag ccagtctagc 112200
tttaaagcct gagcttgtag tcactgctct aatgcctctc atgatactgt gttttgactc 112260
attaactgtg attctaagat ctaaacttat agcttagtga taaaagttag aaaagatgat 112320
cataaaatta tttcatctag ttcctggatt gaatttctgt taaggcatta catgattttt 112380
atttcatcca ataattattt ataaagatct ctaacaggcc agtcagtata cagagtacca 112440
gattaaaaat aaatgtagca ccagtttttc agaaattatt atgtgtctat aattagggta 112500
attacattta gaagatcttt ttgatgatct ccttaaagtc agcaactgtc tttttcatct 112560
ttgtttacct agtacctgga atggagatag gcgtttagca cttaaatgtt tactgaatat 112620
tcttatgagt gccttttatc tttcctactc cttgttgcat tgcttactta ttgtttttat 112680

tttagttgag ttttgtaaga aattgactta cttttttttt ttttaaccta ggggatgtcc 112740
cactcttgtt caagcattgc caggccctag cacacaagtc actgcaggca gcaaccatac 112800
ggcagtactt ttaatggatg gacaggtctt cacatttgga agtttttctg taaggaattt 112860
ttaaaacatt aataatattg cattatacca ttgccttata atttgtctat attagctctt 112920
ttttctgttt ccagaatata atataacaat tatattataa ttgttacata tgcatatttc 112980
atgccttcat ccccaacaca cactaaacct gaatgatatt ctttgaagta attttcctct 113040
ctagctcagc attagaattt attgaattta acagctttgt tagaattgga catgtttatt 113100
tcagattaaa gtcttttaag cattcaatag agctaattct gtcataggaa aggttatttc 113160
tcatctaact tgtagagatg aatttttctt aacacataga actatgctat tttgtaacct 113220
tttaaaagcc tagttttttt tatttgattt gtttaaaatt atactttctt ttttcctttt 113280
ccaccctctg agtctatccg cctgtctgtt acataggcgc ttgtgcacat tctctccctc 113340
tctctgcacc aaagcttaaa gagtgaaaat gctctaagaa ttttgtgtag tttgggcata 113400
gtagatatca agaaaaatct ttgcgagact gtgctaatac tcttgtacca tttcagatgt 113460
ggataattac taagaactct tgaacctaag tgtctgagat gacatttaca gcttttgatt 113520
ttttaaaaac tgtaaatgtg tacttaaaat attttatttg aaaatggttt caaacttaca 113580
gaaagattgc acaactaaga acagtgtgta gaatatctgg ttagtgtctt taaccagatt 113640
cacctattgt taacattttg atccagcatt tgctttatta ttgcacatgt ggctgttctc 113700
tttctcacac acgcagtgtg tgtgtatgca caaatgtgtg tgtgtacgta tgtatataaa 113760
atattttttt ttctcaaatc ctttgacaat gtcttgtgta cattatggcc cgttacctcc 113820
tacatacttt agtttgtatt ttgtgaattg acctaataat tattttttgg catttctttc 113880
ccctccaatc taggatcagg cattacattt gcctgttgtg tgtctttagt ctcctttaat 113940
ctgtaacatt tctacagcta tgtattgtct attataacat tgacattttt gaagagtata 114000
tagtactgtt ttgaaaaata gaatcctctt catttttgtt tctttgataa tgtcctcatg 114060
tttagatgca ggttgtgcac ccaggccaag atactctgta agttatgttg tgtttgccct 114120
cactgcatca catctgaagg ctcagaatgt ccatctgctc ttcattgatt atggacattt 114180
tgatcacctg atcaaatgtc tgatttctgt actgtttctc ctttgaaact ataagcaacc 114240
tgtaggttga cacttttaag atgcctgctt ctcatcaaaa tttcccccta gatttagcat 114300
ccattgatca ttctttcctg agctagtctt tactataatg ttttaaaaat ggtgattttt 114360
ttcaactcca gtaggcactt ggccttttac tctaagcaag agccctcctt tcctgtttac 114420
ttgtttttct acttattatt ggtatagact caggtatttg tattttcttc attatcatct 114480
ttaattattt gggtgcctag attatctccg atttggccag cagtagcccc ttcaagttgg 114540
ctcctgtatt cttgtgacat gccccttcat tttttggagt gcttcctttc tttccagcat 114600
aacaagttgt tcagggttta tcttgtacag gccctgtatg actgtcctag aatcagccat 114660
ttctccaaga accctgattc tttttagtgg ggaatagtat tagacactaa ctgggcacta 114720

gaagtgttca ttgctacagg acgtctttgg atctctggta tgcatatatt tatatgtcca 114780
catatatgta tgtattttag aaatcatagg ccaggtgtgg tggctcatac ctataatccc 114840
agcactttgg caggccagtg taggaggatc actcaagccc aggagtttga gaccagcctg 114900
gacaacatag tgagacccca tctctattaa aaaaattagc tgggtgccgt ggcacatgcc 114960
agtggtccta gctgctcagg aggttgaggt aggaggatca cttgagccct ggaggtcgag 115020
gctgtagtga gagctgtgat tccatcattg ccctctggcc tggacagcag agcaagatcc 115080
tgtctcaaaa agaaagaaaa agaaccccac tgtgacttct acttccaatc cattcccaca 115140
aggttctttc ttacctgtcc tgttctatat ttatgtactt tcttcctcag taagaatctt 115200
tcctctcaac aatatcaata tatttatttc tcacttagtc cccagatatg tctaaaatag 115260
ttttatattt gctttgcctg tacaagttca aaaaaacaaa ctcactgaaa ggtcattctt 115320
gctctttccc ctcccacctg ctccccaccc caagtctgaa ggtgtatagt caagcactgt 115380
ccatgagtgg cctggattct ctctctcact cctttcagtg gaaattgtga ttcctttgga 115440
atagagtggg gctcatttgt ttcagtttaa gattcccctc accctatcct ttgattaagt 115500
tttatttcat ttttaaaata tatagaacat ttacatgttg ctaaaaatta agttatacaa 115560
aaattgtata ctcaagaggt gtcacttcct cccatatcct tgacattcct gcctccccac 115620
acctttcatt ccatacttgg aggtaatgaa tgtcattgtt ttacagttta ttcttcttgt 115680
gtttcttctt gtgaagatat ataagcatgc agacacacta ttttatatat atatacctat 115740
atatgtatgt atgtgtatac acagttatgc actgtataac agtattttgg tcagtgatgg 115800
actgcgtatg ctaaggtgtt cccataagat tataataagt actttgacta taacttttct 115860
atgtttacat gcagaaatac ttactaatgg gttacagttg cctacagtat tcagtacacc 115920
agcatgctgt acaggttggt agcctaggag caataggcta caccatatca cctaagtatg 115980
tagtagatat accgtctagg tttgtataag tccactctga tgttcgcagc atcacagaat 116040
cacctaatga cacatatttc agaacatatc cctgtcatta agtgacatgt gactgtacat 116100
aaatatatat atttcccctt tcccgttttt taaaggtaat agcctatata tgctcttttg 116160
cactttgctc ttttcgcttc agcatctctc ctagaaatca ctccatatca attcataaag 116220
ctcttcattt ttttttttac ctccatgtaa taccccattg tgtgtatata tgatagttta 116280
ttcattcagt ttctcatgtt tatatattta cgtggtttcc aatactttgt aatggtagta 116340
gtgaatattg ttagaggtgt atctttgaga taaattccta gaagtgagat tactgtgttg 116400
aaggttaatg cctatgtagt tttgtgagat attaacaatt ctcctgtatt ttgctttcac 116460
actaacggtg tatgagattg cctgtttcca cagaattgcc aacagaatgt gttgtggtac 116520
ctttaatttg tgccagtctg atgggtgagg aatggtcctg cttacttcca agttcctttg 116580
cctttagttg gtgctctgat cctcaaagtt cggatccata tttagtattt tggcattgaa 116640
ggttaacttt ctttttgggg gcatgtgttt ccctgtgctt tttctaactt cttcacacgc 116700
ctctgtcctc cttcttaaga acttccctgt tcgtgctttg cacatgctca ggtttgcagt 116760

agccggtggg tggagagaac tcttggaatt tggctcttct gcttacagga aacatagaga 116820
tcatgacacc cgctgtctcc ttccactgct gaaggtttga gtcacatata gatttgtttg 116880
cacagcatat tttgtgtctt tttgaggtgg ttatgtgagt gataagattt gacgtcagac 116940
agtcattgtc ctccagtccc agcagtccta gttgtgaact tttatgaatt taaatgtggc 117000
ttatttataa ccacattctg gtccttatgg ggttgtccag gtatctaaca catttttgga 117060
ctgcttttct tcatattagt tggtagtaag ggaatacata tttatttcaa tagtcaatga 117120
aaaagataac taatttattt ttcctccaaa agcagaatat atccctgaat atgatatatt 117180
ttgctcacta tttacttttt tttttctttt tgagacagag cctcactctg tcatccaggc 117240
tagaatgtag tggcatgatg atctcagctc actgaaacct ccggctccca gattcaagca 117300
attcttatgc ctcagcctcc tgagtagctg ggattacagg cccatgccac cacacccagc 117360
taattttcgt atttttagta gagacgaggt ttcaccatgt tggccaggct ggtctcaaac 117420
acctgacctc aggtgatcct cccatctcgg cctcccaaag tgctgggatt acaggcgtga 117480
gctactgcac ctggcccctg tttacatttt tgtgctctct tatttttgtc aaaaaataag 117540
gacagatctt aaaagagtat gagaaataaa tgttgagtgt tttggctgct tctctaatca 117600
tgtgcttgcc atgtttcctt ccctggttct gtccagggat tgaagctcag ttgcttccac 117660
agttagtttg tggctgtgtt ttcttgctgt gctacctggt ggttggatga gaacgtgacc 117720
agagtgttcg cagtgctcca ggctcccttg ttccagaatg ctgtgagggg agagaggtcc 117780
cgagccaggg gtggaagaat atctattgag tcaatgtcag cattctttta ttctttttca 117840
gctacaggct tactagtgag aaacttccta ttacttttta aattaaactc ttacctgacc 117900
aggtccctat agagagccat acttttaaac tggttaatag ttatttactt aaaatatcat 117960
atcttaccat tgattgtgct gtttatttta aaacataaat gaaataattt agggacatat 118020
catgtgaaaa caacattaca tatttttcta attgtattca cagtttcagt atttattttg 118080
gtctttttac atggatattc tcagattata tgagttcaat agtgactttc ttttatacta 118140
tcaacttact atgtcaaagt gactttttaa atgaacaatt tggaatacta atattttaac 118200
atattgtgtg tatttgtcgt acttttcatt taaatgtagg tgaagacaaa gtattttgga 118260
gatacttctt gcgtggaaaa tatttcaaac ttttttttaa aaaataatgc actttttaag 118320
actttccagg aataatagaa attattttat tactttataa atatttggtc caaattctta 118380
catattgtat ttattttaaa aacccaaact aacaattggt gaaaaataag gtttcataga 118440
ttgattttta tctactgcct ccttaacttt atgaaaatta gattgtttac acagcaatat 118500
tgctaatatg gtttatttaa tcttttagaa aggacaactg ggcagaccaa ttttggatgt 118560
gccatattgg aatgcaaagc cagctcccat gcctaacatt ggatcaaaat atggaagaaa 118620
agctacttgg ataggtgcaa gtggggacca aactttttta cgaattgatg aagcacttat 118680
taattctcat gtacttgcta catcagaaat ttttgccagt aaacacataa taggtaatac 118740
cagaaataaa caaatgcccc ttccaaactc ttgtcttgat tgatagtaaa tggtttatct 118800

ttccctttaa tgaaatgtac ttttgtagct ttttgttttc tttctgctca aagaaattta 118860
aacaaagcct gtaaatgtct ccgtttatat gtgtctctat gctgaaagaa tggaataatt 118920
gggagatctg gaaaccacag tatttgtgag ctagacagtg gtagaactat aaactaaatt 118980
ctagtaggat aaaatccata ttagagtaag agagttttat gcattgttta ttgctaaata 119040
aatgttagaa attaattttt atgggaggtt atataaatcc aactttgaaa tttcacgaag 119100
accaagttat caatctgtac tgtgtccatg atactgtgaa taattatata atagagtggt 119160
atagcaagag attaggagca tgtgttgtgg atttagacat cttgaggttt tattcctaat 119220
tctcccactt aaagattttt taattactta gcctctttta ttctctgctt attcacctga 119280
gaaatggaga taatactact ttatgggttt tggagaattt aaaagggtaa tatatattaa 119340
aattatatat aaatattttt ttcttaaggc ttggtacctg cttctatatc agaacctcct 119400
ccatttaaat gccttctgat aaataaagtg gatgggagtt gtaaaacttt taatgactca 119460
gaacaagagg atctgcaagg atttggtgtg tgtcttgatc ctgtatatga tgtaatttgg 119520
aggtaagcat ctcagtttat aaaatagtca ataagttttg atgcagttat actcttatgg 119580
taatataaac tttatttaca ggtggacttt tcacatgtga gtactctcct gtttatctaa 119640
taactgcaat tctgtctcag tgattcacag ggacctttat actgtagcat agtggttaag 119700
agcatgcatt ttaagcaaca ctacctaggt ttatcccagc tccacaactt gctttctgtg 119760
tcaccctgga gaagttactt tgactgtgtg cctcagtttt ctcatttatg aaatggggat 119820
gctaatacta cagaggtgca tactaggtgc catataagtg tttgctatta tcattataat 119880
tattaatttt actgttgaga gttctgttgt tatttttagt ctgtcatcaa tcctgtttat 119940
attgtataga attcattcta ttttttgttc tcataattat tattaactat gaggaggaaa 120000
ttatgccaaa gacaataaag aataaagaag ataattgagc ttgcccttta agaatgtgca 120060
ttaaaaaata ctcttactgg tagagaaata tacaggatta tatgcattct ggaaattata 120120
aaaacataga gataaataag gcacaaaaga gactcaagaa attatgttgc atttgtttga 120180
aatttattgt ggtgctgggc cgggtgcggt ggctcacgcc tgtaatccca gcactttggg 120240
aggccaaggc gggcagatca cgaggtcagg agatcgagac catcctggct aacacagtga 120300
aaccctgtct ctactaaaaa tacgaaaaat tagccgggcg tggtggcggg cacctgtaat 120360
cccagctact cgggaggctg aggcaggaga atggcgtgaa cctgggaggc ggaatttgca 120420
gtgagccgag atcgcaccac tgcactccag cctgggcgag actctgtctc aaaaaaaaaa 120480
aaaaagaaaa aaaaattatt gtggtgcttt atatacaaaa tgaataaata agttaccctt 120540
cttagtgttt ctagctttgg atactaatag gaggtgtacc ctgccatcac cacagtggct 120600
catcacaggg ctgagcactt ctttcctttt gcggccggcc ttagtaggca agattccagt 120660
aagttgaaag gttgaagtag tgtgagtgag gagacttttt ctctgtcttc cttcttctac 120720
ccatgcctgc ttgcattctc atcagacagc caagttctaa gaggccatgt ggtagcaatg 120780
ggatagatga tctcaaatgt cttgccaact ccttggtaat tttcttccgt gttccaaaat 120840

agagattaat aacgaggctt gcattcagag tgaagagact gctgcacttc ccaggttgct 120900
ggccagcttc ctctcttatt tgggcatttt atgagaaggc gtagtggcca gtattgagtt 120960
gagtacatct caaatcaatt acatctccct ggcagcattg tgcagggtag atgaaataac 121020
aagagactat gggtgagtgg tcttttgtga tcattcttat gaagacatga ggtattaaga 121080
accaaagtta gaatggtcac agtgggatag aatggagggg atagagagga gaaaagatgt 121140
aaagatagaa ttggctgcag ttacttaccg attagaagtc aatgttcaag agacttagtt 121200
accaaagagc aggctctggt ttggacttag gtgtaaattg atgttttact gttttgtagt 121260
tcttacatgt tttattttta aatgaagtaa atttgcatta gcaaaacaaa aataaaaatt 121320
ggcagtgtgg aatctgtttc tggatgcatt cactaagtta gcaataacag tataactaag 121380
cgtaacagta aaaacttact tgaactagaa ccatttccgt ggggtcttaa cagaactctc 121440
aagtatgatg ttcagctggt tgaaccttgt cttttccatt tatggtagta gcaatgggac 121500
agatgatctc aaatgccctg ccagctcctt gatcaatttc tccattgttg gaaaacagag 121560
attaagagat gttttggtaa aacagctgag aggtattttt ctttaaatca ggatcagaat 121620
ttctgttatc ctgacttctg gctgctttgt gtcttttgtg tttttcaatg gggcacttaa 121680
attttataat tatcatatgc attgcacatc caatattagc ttactttgac tttccttctg 121740
gcctgcatct gcttcatttt atattcttta ggagccaact ttgcctgagt aattagaaac 121800
aacatctata aagagcagat aaatgttaag aacacctgtt ttatttccca catacaaatt 121860
tacacaaata atatgtgaat ttgtgttatt ttttaaaaaa caaagcaaaa atacatacag 121920
gaaaaaggat ggtcatactc tgtatctgtt tcttttccca taccgaattc cactagcctt 121980
ctctgtggat cacaagtatg aagtttgatt tatatcatct gacacctttt aaaatgcatg 122040
tatttttctt tttatgtaaa taaaaacata cctactctaa aagttagatt ttttgctcaa 122100
aaataattta gagatctttc caagtcagta tttaaagaac agcttcatta ttaaaattgt 122160
ggaattctgt agtctgggta taccaaatat acttaactgt ttgcccatca gtgaacattt 122220
tagatttttt ttaatattac gatactacct acaatgctac agtgaacaaa tacacaggta 122280
tctacacaca gagttttaca cacatgtgga agtatttctg caagctagct tttaaaatgt 122340
agaattgctg agttaaaaga taggctcaca gaattgtaaa atgactgagg taagtctgaa 122400
tcactttaga agtttatttt tccaaggttg aggagatgcc tgggaagaaa agacaagcca 122460
cagcaggatc tgtgccctgt actttttctg aagaggtttg aggccttcag catttaaaga 122520
ggaaaagaga gcaggaggag aaaaggaaaa gaaaaaatga gaggatgtgg tcacattctt 122580
gtaaggtttt gattaggctt actgaatcca catgttgcac atgaaaagga aggggtagag 122640
ggaacagtga attttgtatt tggagttaaa gtaaacatag agtagaggaa gcagtcaaat 122700
actcattcat ctggggctgg ggcgggtggg gcttggtggg gggatgggca gataatttct 122760
agtatcttct tgtctcttac catgaatttt ccagaaccaa aaagagattt gaatccatgg 122820
atattagaaa ctatgtgttt ctattattta ccatcaagag agttagtgaa actgttgaac 122880

actaaagacc aaggggaaat cttaaaagca accagagaga aaagaatgat tacctaaaca 122940
atgagatcaa cagaacttcc caaaagcaat aatgcaataa tattaatgct actagttata 123000
gaagctacca taatatattt tctcccaaat gctattttaa gtgcttttta aaaataattt 123060
aaaatttttt aattgtggta aaaaaacagt ataaaattta ccatcttaat cattttgaaa 123120
gtctgcagtc agtggtgttt agtgtattta tgttattgtg aaatagatca tcagaacttt 123180
tttttcttgg aaaactgaga ctctatagtt attaaacaat agcttgcatc cctcacttcc 123240
cgacccctaa aagaggtaaa gaagtggaga tagttttttg aagtttttgt tacaaaagag 123300
atcagagaaa tgggatgatg aagacgttgt gggttcctaa tagtttttaa ttggttttta 123360
ctgtttattc atatggttga acatccacat ccattctatc tgtgcttcct ttctatggat 123420
agcattccat gtccttgcca atttttgcta tggattgatt gtttctagct cttatacatt 123480
gaggatattg tagctgtgtc tcacaggtac acttcacatt tttcttcaac ttgtcctttg 123540
tctttaaact tttttaatgg ggtatttgtt gagtttcata tttatatgta tgtatcaaaa 123600
ctttatacag tcaagtatta taatctacat tactattttc tgtattaagt attataatct 123660
acgttactat tttcaaatta tttcactttt agtgatatag tttttttata tgtttagctt 123720
agatccagaa gataatgagc tacatttgtc ctctttaagc tgactatatt atttattctc 123780
tttgtatcat cagttatgtt ttaggaattt tttttaaaaa caaaaccaca ttgaggtgct 123840
gtaattattc tttttcttta aaggtttcga ccaaatacta gagagctgtg gtgttacaat 123900
gcggtggttg ctgatgccag gcttccctct gcagcagaca tgcagtccag atgtagtatc 123960
ctaagtcctg aacttgcctt accaacagga tcaagggccc tcactacccg atctcatgca 124020
gctttgcaca ttttaggtag ggttgcgatt tgatgtacca ttaattcaca ttaatgtgtg 124080
ctgtccaggg ttttttttta gagtatttaa atttatatgt aattctgtct tgtttgttaa 124140
ataataataa tgtacaaaat aagttaaatc ctctaagagg ctaattccac agaaaacaat 124200
acatgagacc tgtactaaca ctatcaaaga tttatgtatg cccaattatt taattttcac 124260
agaggtagac ccttaaaaat aatgtttttg ctcaacttga atgtataaac ttttaaagat 124320
atgataaaat tttcaagtat ataatattta atttatgtat attatgttaa ataaattcta 124380
taaaatgtaa tgaattttat atacatcttc tgtttcttct tatataacac agttttccac 124440
agcatatcaa cttcattctt atttaattac tttgagatgt agtacttatt taattacttt 124500
gagatgtagt gcaatatatt attttgtaac tggaaatttt atatcactct atgtaactat 124560
gtattttgtt gcttttaaaa atgatcctta aaaagactca ttttcagact ctagcacttg 124620
caggtgtgaa atcatattcc taaatataat taccatatca taaatataac tctttgtttc 124680
ttttttctca aatttcattt tggtttctat tgtagacatc caaaactagt gttgattaag 124740
tttgtgtgac actaatgtgt cttcctcaaa tagcacttta agaatcaaac taatttggag 124800
tttcataaaa ggaagaacct tgtaagaatt caaaggttaa gtgatttcaa cttttcagag 124860
atccagtttt gtgtaaaagg ttgtcgtatg gcaagtttaa atatgatcat taatcagaca 124920

agggataatt tgtattggtt ttaagcattc ttccatgaaa tgatgtttaa agcttggagt 124980
aacattctga gtttatttat tttaatttat cccctatagc atttaatgtc atcaatacca 125040
taggacattt taatttcaag gagttaaatt ttgtttcttt gttgttttag gttgtcttga 125100
taccttggca gctatgcagg acttaaaaat gggtgttgca agtacagagg aagagactca 125160
agcagtaatg aaggtttatt ctaaagaaga ttatagtgtg gtaaacaggt ttgaaagtat 125220
gtatacttcg gtttaggaaa tgttgtctta caactgaaat atatatggat cttttaaaac 125280
atagattatg atttatatat tctaggtcat ggaggaggct ggggttattc tgcccattca 125340
gtagaagcta tacgtttcag tgccgacact gatattttac ttggtggtct tggtctgttt 125400
ggaggtagag gagaatatac tgctaaaatt aaggtaaagt tcatcaacaa tgttgtcctt 125460
ttttgtttga acatactgat tttgacttaa tttattattt tattatttaa tgtctaattt 125520
ttttcctttt taactgggtt tattttgttt ttaatgcttc tttttaaaaa gcgatagaga 125580
aaacattgct aaaatcgaat acagtaatac taaatttttt aaatttattt atttattatt 125640
attatacttt aagttttagg gtacatgtgc acaatgtgca ggttagttac atatgtatac 125700
atgtgccatg ctgatgcgct gcacccacga acttgtcatc tagcattagg tatatctccc 125760
aatgctatcc ctcccccctc cccccacccc acaacagtct ccagagtgtg atgttcccct 125820
tcctgtgtcc atgtgttctc attgttcaat tcccacctat gagtgagaat atgcggtgtt 125880
tggttttttg ttcttgcgat agtttactga gaatgatgat ttccaatttc atccatgtcc 125940
ctacaaagga catgaactca tcatttttta tggctgcata gtattccatg gtgtatatgt 126000
gccacatttt cttaatccag tctatcattg ttggacattt gggttggttt caagtctttg 126060
ctattgtgaa taatgccgca ataaacatac gtgtgcatgt gtctttatag cagcatgatt 126120
tatagtcctt tgggtatata cccagtaatg ggatggctgg gtcaaatggt atttctagtt 126180
ctagatccct gaggaatcgc cacactgact tccacaatgg ttgaactagt ttacagtccc 126240
accaacagtg taaaagtgtt cctatttctc cacatcctct ccagcacctg ttgtttcctg 126300
actttttaat gattgccatt ctaactggtg tgagatggta tctcattgtg gttttgattt 126360
gcatttctct gatggccagt gatggtgagc attttttcaa gtgttttttg gctgcataaa 126420
tgtcttcttt tgagaagtgt ctgttcatgt ccttcaccca ctttttgatg gggttgtttg 126480
ttttttttct tgtaaattgg tttgagttca ttgtagattc tggatattag ccctttgtca 126540
gatgagtagg ttgcaaaaat tttctcccat tttgtaggtt gcctgttcac tctgatggta 126600
gtttcttttg ctgtgcagaa gctctttagt ttaattagat cccatttgtc aattttgtct 126660
tttgttgcca ttgcttttgg tgttttagac aagaagtcct tgcccatgcc tatgtcctga 126720
atggtaatgc ctaggttttc ttctagggtt tttatggttt taggtctaac gtttaagtct 126780
ttaatccatc ttgaattgat ttttgtgtaa ggtgtaagga agggatccag tttcagcttt 126840
ctacatatgg ctagccagtt ttcccagcac catttattaa atagggaatc ctttccccat 126900
ttcttgtttt tctcaggttt gtcaaagatc agatagttgt agatatgtgg cattatttct 126960

gagggctctg ttccgttcca ttgatctata tctctgtttt ggtaccaata ccatgctgtt 127020
ttggttactg tagccttgta gtatagtttg aagtcaggta gtgtgatgcc tccagctttg 127080
ttcttttggc ttaggattga cttggtcttg cgggctcttt tttggttcca tatgaacttt 127140
aaagtagttt tttccaattc tgtgaagaaa gtcattggta gcttgatggg gatggcattg 127200
aatctgtaaa ttaccttggg cagtatggcc attttcacga tattgattct tcctacccat 127260
gagcatggga tgttcttcca tttgtttgta tcctctttta tttccttgag cagtggtttg 127320
tagttctcct tgaagaggtc cttcccatcc cttgtaagtt ggattcctag gtattttatt 127380
ttctttgaag caattgtgaa tgggagttca ctcatgattt ggctctctgt ttgtctgttg 127440
ttggtgtata agaatgctta tgatttttgt acattgattt tgtatcctga gactttgctg 127500
aagttgttta tcagcttaag gaggttttgg gctgagatga tggggttttc tagatataca 127560
atcatgtcgt ctgcaaacag ggacaatttg acttcctctt ttcctaattg aatacccttt 127620
atttccttct cctgcctaat tgccctggcc agaacttcca acactatgtt gaataggagt 127680
ggtgagagag ggcatccctg tcttgtgcca gttttcaaag ggaatgcttc cagtttttgc 127740
ccattcagta tgatactggc tgtgggtttg tcatagatag ctcttattat tttgaaatat 127800
gtcccatgaa tacctaattt attgagagtt tttagcatga agggttgttg aattttgtca 127860
aaggcctttt ctgcatctgt tgagataatc ctgtggtttt tgtctttggt tctgtttata 127920
tgctggatta catttattga tttgcatata ttgaaccatc cttgcatccc agggatgaag 127980
cccacttgat catggtggat aagctttttg atgtgctgct ggattcgttt tgccagtatt 128040
ttattgagga tttttgcatc aatgttcatc aaggatattg gtctaaaatt ctcttttttg 128100
gttgtgtctc tgcccggctt tggtatcagg atgatgctgg cctcataaaa tgagttaggg 128160
aggattccct ctttttctat tgattggaat agtttcagaa ggaatggtac cagttcctcc 128220
ttgtatctct ggtagaattc ggctgtgaat ccatctggtc ctggactctt tttggttggt 128280
aagctgttga ttattgccac aatttcagat cctgttattg gtctattcag agattcaact 128340
tcttcctggt ttagtcttgg gagagtgtat gtgtccagga atcccttatc catttcttct 128400
agattttcta gtttatttgc gtagaggatt ttgtagtatt ctctgatggt agtttgtatt 128460
tctgtgggat cggtgatgat atccccttta tcatttttta ttgcgtctat ttgattcttc 128520
tctctttttt tctttattag tcttgttagc ggtctatcaa ttttgttgat cctttcaaaa 128580
aaccagctcc tggattcatt aattttttga agggtttttt gtgtctctat ttccttcagt 128640
tctctgattt tagttatttc ttgccttctg ctagcttttg aatgtgtttg ctcttgcttt 128700
tctagttcct ttaattatga tgttagggtg tcaattttgg atctttcctg cttcctcttg 128760
tgggcattta gtgctataaa tttccctcta cacactgctt tgaatgtgtc ccagagattc 128820
tggtatgttg cgtctttgtt ctcgttggtt tcaaagaaca tctttatttc tgccttcatt 128880
tcattgtgta cccagtagtc attcaggagc aggttgttca gtttccatgt agttgagtgg 128940
ttttgagtga gattcttaat cctgagttct agtttgattg cactgtggtc tgagagacag 129000

tttgttataa tttctgttct tttacatttg ctgaggagag ctttacttcc aagtatgtgg 129060
tcaattttgg aataggtgtg gtgtggtgct gaaaaaaatg tatattctgt tgatttgggg 129120
tggagagttc tgtagatgtc tattaggtcc gcttggtgca gagctgagtt caattcctgg 129180
atatccttgt tgactttctg tcttgttgat ctgtctgatg ttgacagtgg ggtgttaaag 129240
tctcccttta ttaatgtgtg ggagtctaag tctctttgta ggtcactcag gacttgcttt 129300
atgaatctgg gtgctcctgt attgggtgca tatatattta ggatagttag ctcttcttgt 129360
tgaattgatc cctttaccat tatgtaatgg ccttctttgt ctcttttgat ctttgttggt 129420
ttaaagtctg ttttatcaga gactaggatt gcaacccctg cctttttttg ttttccattt 129480
gcttggtaga tcttcctcca tcctttattt ttgagcctat atgcgtctct gcacgtgaga 129540
tgggtttcct gaacacagca cactgatggg tcttgactct ttacccaatg tgccagtctg 129600
tgtcttttaa ttggagcatt tagtccattt acatttaaag ttaatattgt tatgtgtgaa 129660
tttggtcctg tcattatgat gttagctggt tattttgctc gttaattgat gcagtttctt 129720
cctagtctcg atggtcttta cattttggca tgattttgca gtggctggta ccggttgttc 129780
ctttccatgt ttagcacttc cttcaggagc tcttttaggt caggcctggt ggtgacaaaa 129840
tctctcagca tttgcttgtc tgtaaagtat tttatttctc cttcacttat gaagcttagt 129900
ttggctggat atgaaattct gggttggaaa ttcttttctt aaagaatgtt gaatattggc 129960
ccccactctc ttctggcttg tagagtttct gctgagagat ccgctgttag tctgatgggc 130020
ttccctttga gggtaacccg acctttctct ctggctgccc ttaacatttt ttccttcatt 130080
tcaactttgg taaatctgac aattatgtgc cttggagttg ctcttctcaa ggagtatctt 130140
tgtggcgttc tctgtatttc ctgaatctga atgttggcct gccttgctag attggggaag 130200
ttctcctgga taatatcctg cagagtgttt tccaacttgg ttccattctc cccatcactt 130260
tcaggtacac caatcagacg tagatttggt cttttcacat agtcccatat ttcttggagg 130320
ctttgttcgt ttctttttat tcttttttct ctaaacttcc cttctcgctt cacttcattt 130380
atttcatctt ccatcgctga taccctttct tccagttgat ctcatcggct cctgaggctt 130440
ctgcattctt cacgtagttc tcgagccttg gttttcatct ccatcagctc ctttaagcac 130500
ttctctgtat tggttattct agttatacat tcttctaaat ttttttcaaa gttttcaact 130560
tctttgcctt tggtttgaat gtcctcccgt agctcagagt aatttgatca tctgaagcct 130620
tcttctctca gctcgtcaaa gtcattctcc atccagcttt gttccgttgc tggtgaggaa 130680
ctgtgtttct ttggaggagg agagttgctc tgctttttag agtttccagt ttttctgctc 130740
tgttttttcc ccatctttgt ggttttatcg acttttggtc tttgatgatg gtgatgtaca 130800
gatgggtttt tggtgtggat gtcctttctg tttgttagtt ttccttttaa cagacaggac 130860
cctcagctgc aggtctgttg gagtaccagg cagtgtgagg tgtcagtctg cccctgctgt 130920
ggggtgcctc ccagttaggc tgctggaggg tcaggggtca gggacccact tgaggaggca 130980
gtctgccctt tctcagatct ccagctgtgt gctgggagaa ccactgctct cttcaaagct 131040

gtcagacagg gacatttaag tctgcagagg ttactgctgt ctttttgttt gtctgtgccc 131100
tgcccccaga ggtggagcct acagaggcag gcaggcctcc ttgagctgtg gtgggctcca 131160
cccagttgga gcttcttggc tgctttgttt acctaagcaa gcctgggcaa tggcgggcgc 131220
ccctccccca gcctcgctgc cgccttgcag tttgatctca gactgctgtg ctagcaatca 131280
gcgagactcc atgagcgtag gaccctccga gtcaggtgca ggatataatc tcctggtgtg 131340
ccgtttttta agcccgtcag aaaagtgcag tattcaggtg ggagtgaccc gattttccag 131400
gtgccgtctg tcaccccttt ctttgactag gaaagggaac tccctgaccc cttgcgcttc 131460
ccgagtgagg caatgcctcg ccctgcttca gctcgcgcat ggtgcgcaca cccactgtcc 131520
tgctcccact gtctggcact ccctagtgag atgaacccgg tacctcagat ggaaatgcag 131580
aaatcacccg tcttctgtgt tgctcacgct gggagctgta gaccggagct gttcctattc 131640
agccatcttg cctcctcccc ccctacagta atactaaatt aataactcat taaaaaaatc 131700
aaaattctgt aagaagggaa gaaatgaaag gaaaattatt ttcaacaatt taccactgaa 131760
gaaagactat atctcagtcc tttctgtgga acttctggtg gcctagcaat accagtgcct 131820
ttttatgtgg ctactttctt atagccattt taatgacaga ggaggcacaa ctgtttaggg 131880
acattttgat gtagtatgta aacagacatc ttcaccccag gagtggagac cgggaggagt 131940
gagagatagt gttaggggag gattccatct ggtaggccaa ctaaaaatca ttgagagtca 132000
tatgttacca cacctattcg tatccaaaag ggccaacaaa ttatcctaaa caaagtatat 132060
gttcattctt tgaatattca ctaatagctt gaatgttctg cttctcaaat tacctgattt 132120
tgctctaaaa gtgaaagaga aaactatagt ttctgcctga atttcaatag caggcttcac 132180
aaaaaaatct cgattaaatg tcatctgttc aggaggtatt ttcttagttc tctcaataat 132240
atctaaaaat gatatcaata tgttaacact cttcttcata atgctggttt ccaaggacat 132300
tccccatgga ggcccaacca gaactgacaa acttaaagaa atatttgctt atttctggtt 132360
atagttggaa tacatgctaa aaaagcagat aatgtaagaa tgaataaagt aaaaatttaa 132420
agctcctata gttatatgga gtatagtgat agaataatga ttaggactct tgtagcacat 132480
atattttgat aataagaaaa atagtagttt ggctcatttc agtgcaactg ctttaaaatg 132540
gttattttag gatgtcttga ttcatcctca cgttagagaa gcaacctcaa tgtgttatgt 132600
agttctattt gacaatgtct aataagctca tcaggaggat attttttttt ctagctctaa 132660
gagatttttt cttttcggaa tagtgaacaa cgtagtggac cttatcttca aacactgtag 132720
tatatagaat tatggaggcc ttccatatat gttgttttta tattggccct attttctttt 132780
ttcaaactca aattttacct cactcctcct attgtctgga aatgatcttg ccttttgtat 132840
tcaattatcc agtgagtggg ctaaggcctt tcaacagtag tcagtctttt tttttccttc 132900
ccttcacctc tctgtaatgt atgcatttgt taagcccttc tgtatttttt tgttttgttt 132960
tgttttcata cctgtagttt tttcttctat ctctttgtag gttcatcccc ttccacctgg 133020
ccactgaatg taggagttcc tcaaagaccc agccctaggc cctctttctt cacatattcg 133080

tatatgcatt ctcttttgag cccagagttt taatagccat cagcatacca taatccccaa 133140
atttgtatct ccaactcaga cttctttgat gcattttcat gtaaatatgt taaaggcaca 133200
tcagttatca gtcataatat ctcaactgaa ttcatgctct tccactgatc tctctctctc 133260
atgattagta ccatccacca tatgcttgat acataggccg gaaatctaga catttttctt 133320
gataggcctc ctatatctct ttcttctcca caaaaaagac ttttctacca ctaccctagg 133380
caaaggtatt tgtcttggac tagccaccta gatagtctct gtacatattc tttctatact 133440
ttctgtctcc attctctctc ttaccattcc tacttgagcc tactccagtc agatttttgc 133500
ccccaccact gacattgctc ttgtcaagga cactgatgac tttcatattg ctaaattcaa 133560
tagtcaagcc tcagctctca tcttccttaa ccagtcagct gaatttaacc caattgagct 133620
cttctttgaa atactttctt ctcttgtccc ccacgacact acacttcatt tttcttttgt 133680
cccactgact gttcttctta gtctcctgct ggttgctcat catcttcctg acttctaaat 133740
gttggagtgc caagaactca gtttttgaca tcttttaaaa gtattctctt cctcatagtg 133800
gtccagcctc atggacattt atctctgatg acttttaaat ttataaacct cttctctgac 133860
ctttaaacca atatgtacaa cagcctacgc tgtgtctcca cgtgactctc tgataggcat 133920
tttggaggta acatattcaa tactatgctt ctgatgatct gcctaaacct gctatcctca 133980
tgttcttccc catcccagtt aactgactca gcatggagtt aacttcattc ttctgtttcc 134040
tcaggccaaa aatcttggag tcatccttga ttctactctt tcttttacac acatccactt 134100
cgggagcaaa tattatcgtt tccactttca aaataaatcc cgtatctaac cacttctgaa 134160
aactgccact gctatcaggt tgggtcatgc cacagccatt ctcatttgta ttattacagt 134220
agcctcccaa cttgtgttct acgtctgtcc tcaccccatt tgttctgttc tgagtgtggc 134280
agtgattctc ttaaaatgta caacagattg catcattgtc tcttcagaat cctgtagtgt 134340
cttcctatgt gagaatgaga accaaaagcc ttagaaggac ctacaaagtc cccatcagaa 134400
ctggctgtca tcacctctct gaccttttgt tctactagtt gtttcactct aatccagtga 134460
cactggcctg cttattgttc ctcagacatt ccaggcacag tcaaatctca gtacctcttc 134520
acttgctgtt tcctcataac tacagctctc ttcacttcag cacctacata gcccacttcc 134580
ccatcacctt cagagcttca ttccctggta agcctactta aaactgtaaa ttcaattccc 134640
aaccatagac ttactcccaa tagcccccaa ttctttgatt gtattgtttg cccctttatg 134700
cccaatttct agaactgtgc ttggcataca gtatgtgttc aaaaatattg gttgaatgta 134760
aatacaggag tcagattggg gacaaattat taagaacctt catatcatgc tgtggaggag 134820
atgagaaacc aaggaataat tttagattaa gaaagtattc tgataagata tttttataag 134880
ggtactaagg gtcatgaaac aaatacaaaa atcctcaact ttgtggaata tgggttagaa 134940
gaaggagaga atggagataa gactaattag ttgttgaaat attccagcca gcaataggga 135000
atatggttga attgacaagc tttggtacca gtaagagaat agatacagta atggcagaga 135060
aatatggaaa aatgattatt tcggactatt tttttttaag agtctctttg gtagcccaca 135120

ctggagtgca gtgatgccat cttggctctc tgcagcctct gcctcctggg ttcaagcaat 135180
tcttctgcct cagcttcccg agtagctggg actacaggca tgtgccacca cgcccagcta 135240
atttttgtat ttttagtaga gacggggttt caccatgttg accaggctgg tgtcaaactc 135300
gtgacctcat atgatctgcc tgcctcagcc tcccaaagtg ctaggattac aggtgtgagc 135360
caccaggcct ggcctatttc agacttttta tctaagagat taggtgaatg aaagaccatt 135420
aaccagaaca gagaaggcag gaagaaggta tagaactagt aggacaaagg gaatccaatg 135480
actgttgaga ataagagctg agctggagaa agatttctaa gtcattagca tataatttaa 135540
aaatttagac acctagcaca gtacttgtcc agtaaatttt aatgcatttt aatctttatc 135600
tgcctctctt gcaattcacc ttccataatt atccagagga aaaaggtttt cttaaaaaaa 135660
aaaaggacat tttttaaaaa gacctcttct tcataattct tattttcact taatttcata 135720
acctttttgt attggatatc ttttctgttt ttttcttacc cagaggatct tcccctcttt 135780
tttaagagcc atttgtttca tgcttttcat ctatcttttt ctcatgccat attactttga 135840
actatatttt gttacctatt gtttttaagg attcttatgt tgctcttctc tctctgtagc 135900
taatatttct ctaccctttt ttattgtttg tttttttaag aattctacag tattcactta 135960
ctttttaaaa gtactgtgag atgggaactg ttagtatcca cccacctttt atggatgagg 136020
aaactaggtc ccaggatctc agttagtaag tgatgaagca acattctacc tcatttagaa 136080
tgaggtagaa tctcacttag tgagagactc acttagttaa aattttttgt tggagttctc 136140
tcaccttttt cagatgcaca tattaggtac aaaacttgta tagaactatt tagagcattt 136200
gatgtaaata gatgaatata taaattagga aaacagagat taatgtagac cataaaacct 136260
tgtttcctaa tttaaataca tatgtttgtg tatttcacat agtaatgtta aaaaagcagg 136320
tactctgatt ttttacattt ggtatggtcc agcatacatt catcatgcac aaaattatga 136380
acacattgtt catcagatat gtatagctga tttatataaa tgattgtctt tctcctttcc 136440
ttagctgttt gaattgggtc ctgatggagg agatcatgaa actgatggtg accttcttgc 136500
agagactgat gtattggctt atgactgtgc tgctaggtaa ataatttgtg cattattcat 136560
tgtaactctt gcatacttaa tattaaaaat ttattataaa tgaataaaca tactttaaaa 136620
ggaagtttta aatctgctat gaatatcaaa tacttgagaa attaaattat atattaagca 136680
atataagtat tgctttcaaa aatgctgaat taaaactaat atacttcata aatttatttc 136740
ataagtcaac tgtttcaata ttgaagataa aaatggaaat atgcatatat tttatacacc 136800
tttactttca tgcatctctt acggaatacc tagtttgctg gtgttctagt aataatacag 136860
ttatttatta ctcctagcat tacaggtaat aaataattag cgtcaagtgg tcatgtttga 136920
ccataattca gattacccat gtactctgac atcatgctgc tagaatatat ttgataataa 136980
cacacttggg gaggtgacta gctttactga aaccttttgg gagtttataa aaattagtat 137040
gtttatttat gaaggttcat taagtaagag gatacttaca ctttatcagt cctctctacg 137100
taatcctaag aattatctca atattgtgtt atgatcagtg ttccctggtt tgaagcaggg 137160

aatattatta taagaaaaat tataaactga gcttcatagt agtaatagtg tgctatgttt 137220
cccaacaata actatgttag gaaaaccttg taggttttct tgctagtgaa aatgtatatt 137280
ttcagaaaca aggatgatcg taagatactg actttgcttg tacaagatat aaatactttt 137340
gaaaacaaaa gagtaaatct aaagaaaatt ttgactattt cttaggcatt aactaatatc 137400
gcaagatttt tttagtaacc tttagtgttt tatacaaatg aatattaggg aaaattttaa 137460
gaaagctaat tgattaattt tcagcaaagc cttaaggtgc cattgtcatt tgccgtattt 137520
atttatttct ttatttattc atttattaat ttttattttt gagacagagt ctcactccat 137580
cacctaggct ggagtgcagt ggtgtgatct caactcactg caacctctgc ctcccggatt 137640
taagcgattc tcatgcctca gcctcctgag tagttgggac tacagtcgca tgccaccagg 137700
cccggctaat gtttgtattt ttagtagaga tggggtttta ccatgctgac caggctggcc 137760
ttgaactcct gacctcaagt gatcctcctg ccttggcctc tgaaagtgct gggattacag 137820
gaatgagcca ctgtgcctgg ctgccatttg tttttgacca tatgagtttg agtcgaaaat 137880
ataaagcaca gtttcattta tagagaatcg tctaggattt cttcattagc ttatatacaa 137940
agcccagact cctaaggcat tgggagcatt gaaggatttc cataacatcc agcatctttt 138000
ttgactttct accactactc ttctacagct gtcttaatgc attaatgaaa ttaataatga 138060
acaccttgtc atttcctgac ctcttttgta cttccatacc tctacagatt ttttcatgac 138120
attctcctgc ttctttaaat gccctttatt cttctttcaa ttccagctac aaccaataaa 138180
cattccttcc actctgttac ctagtacttt gtaacttgtt tatatccttt agaagatgcc 138240
cttatttatt gtcttttttc ttcttctaaa ttaataatgt ttgtatagat attatgtgac 138300
agactccaaa cacctatctg acttaattca ttctaaagga caactctatg agcaggatat 138360
tattttcacc acagttttca ggtgaagaag ctaaggtact gagaggatag agtaacttgc 138420
cctgcgttgc cctgctagca ggtcacagag ctgggccttg aacccaggct gcctgactcc 138480
agggccattt tcttaatatg ctgcctttca aaactattta gctgaatgaa tgagctttaa 138540
aactaaagtt cagattttta ttttccagca aaatttgaat gttttttaat atcagtttaa 138600
aacattctaa ttttaatatg aaagttttat aacatttaag gatattgtag aattcaagaa 138660
gaggaggaag aaatgtaatt ctatcaccat aacataaata actatatttg cttgcattct 138720
tgtccagact tcttagagtg tttttaatgt tgtttgataa gaattttata atacagttag 138780
tttcatgttt ttgttcacaa gggattttga aaaaataaat atatgtgaca gagaagtaag 138840
aaaatattgt ttttgactca ctaatttttt ctcagagaaa aatatgcaat gatgtttgat 138900
gagcctgttc tcctgcaagc tgggtggtgg tatgtggcat gggcccgagt gtcaggaccc 138960
agcagtgact gtggatctca tggacaggca tctattacca cagatgatgg gtaagtaaat 139020
gcccaagtgt tacttaagca atacttattt ttgttagagg aatattgttt tgaaaattca 139080
gagtactttc agcaggattc aaaagcttag gaagactaga gcataatata actgctaaaa 139140
attaaattca tgtaacctca tgccaaattg aggtctagac agaacaagga gataataaga 139200

ccactgtact ttgctcctgt cagatggtat ttgtatatat ttctgggtat tacattgttt 139260
acgagcttga ttaccaaatt agaaattata tagaggaagg tgacaaggaa aatgaggagt 139320
ttggaaccat aaaaaagaag tagatatgtg atattaaatt tgatagtgca tgagaaaaca 139380
gtttaaaatt tcaaagcttc agcaatgtgt gttattttaa taaaatatag ggaaaatttt 139440
gctgttttaa aataactttc acgtatagag tacttttcag ttcatattct tatattttat 139500
ctgatttgat gccctcaata gctttgtgaa gcagttatta gccccatttt atactccagg 139560
gactagagaa ataaaaaggt gaaattattt gctatttcct gagagtcatg tggttaaaac 139620
caaggtcaga cctgtaacct gttgactcta aatccaacat ttcaatattc tattactatg 139680
atttatgtat tctaaaggac tatcatttgt aagataaatt cagtttattc ttgattactc 139740
ctgagcataa actatttgaa atcagttgaa gttatataac agtatatatt ccagtgcagg 139800
tcaattcata acaaagctga ccaaaaaaag aaatagactt atttgtgagg tgaatttcct 139860
gttgctgaat attcaaggag actactgtag aaaaatcaca tctgttgtta tgtgcatcaa 139920
cttaagaatg tctttagaac taagtgacta ctttgaccct ggactgtatg attttcatag 139980
acagttcatt cagacttgga atgtcatggc acactgaggc taaagcttat catgcagcta 140040
gtcaagcaga tattatacta cttagttgta agtagaaatt ttagaagttt actgctgtgg 140100
ttaaagctga gttggagagc taggagggac aggacagcta ctcatttagg agtcctaatc 140160
acgttcagtt ctttcatttt actgactaac ccattcagag ctagaatgaa aatctagaaa 140220
ataccttttt gcttttcttt atgtaaaaga gtgaatcatt ttcattgaca taagatttga 140280
aaacatgttt cttaaatttc ttatttcctg taatgatcat gttgtttttg gtatcattgt 140340
tttctcttct tagggttgtt ttccagttca agagttcaaa gaaatcaaat aatggtacag 140400
atgttaatgc gggtcagata cctcagttat tatacaggta tttagcatat ttatactgaa 140460
ttagtatggt ttattatttt gctataatga agtagaatca ttacaaaacg tggcataata 140520
gggagaaatt atttgaagca ttcagcaaat tataatatgc tggtagaact ataaatatat 140580
ttaacaaaaa cagttaatat tttgtatatt tttaaacatt ttccatacag gcttaacttt 140640
aaactttgcc tgattactca gattatctgt attataccaa taacaatatt ttactgactt 140700
taaaaaaaat ctatccattg attttcttac taatttcaaa tgttgtaaaa gaataaaatt 140760
tgtcttggag aagtgagatt gcagggaaga tggggaggac tatagatgca ggcctggttg 140820
atatattgta gtctcataag actgataaaa tgagttggaa aattccctac tgttggtaaa 140880
ttttcacctt tagctaaaat accaaataat aacttgctca tataatattt actatatttt 140940
taacattatt gaaaaatttt aacttacaat aatttaagca tgtataaagg catatgcctg 141000
tgtttacctt atttatttct agactactgc aatactttta gaataaagaa ttcaaatgga 141060
tataaaccgt tactgaaaaa tgttgtcatt cgttaagcag taaaaaatca aaggaaatgg 141120
tctagaactg tgattctatc tccctatctt tctttctggt aatcactttg tggttacaaa 141180
cattattttt ccccaaaagg ttttttcgtt tgtttggttt ttatgcaccg tgcaatctgt 141240

tatagttact gtttgtaata tgtagctcta caaacataac gtgttgcatt ggactactcc 141300
caggtactca ctggttgaat aacttttaaa taacagcatt aatagtagca ttactagaga 141360
atataagtta tatgttatat acattatata tgttctctaa gaagaggaaa aagcctctac 141420
tactcggtct tgcgaaggta ggatttaaag gaagtgccca tatgctaaaa actttgttta 141480
gcttggaaaa atgtttcaaa aaagccctgt attggcatta cctggcaaaa aaatttaaat 141540
atataattta ttttcccatt caaaaatgta taatatactt taaaaagaaa aaatgacatt 141600
ggactttcta acataatttt atatgtgtct actgccttta gacttccaac cagtgatggc 141660
agtgcttcaa aaggcaaaca gcaaaccagt gaacctgtac acattttaaa gaggtctttt 141720
gcaagaactg tctcagtggt aagccatgtt ttaaaaaatt tcagcttttc tcttcagtcc 141780
tcagatacat tttgatgttt agagtttagc tgctcttgta agtttatctt agcctagatt 141840
tataaactca gatttggttt tgtattcaca tttatattct agaccacaga taaagtatac 141900
tttaaatcat agaaaaatgg aggtgttggc ctatgatttt aacatgacca gatgaattgg 141960
ccttgctgtt tttgaaacaa ataacgattt gcttttctag taaatcatat atgaatgcat 142020
atgctgaatg aattagaaaa tgaaggaaaa gttaaggatg gtgctggccc aaaaaagcgt 142080
gattactact tgaggaacta ggtggaagag ttgcatatct aactaggaag tttgttgaac 142140
cacttgccat ccccattgta cacatgaaga agcacagtgc cattgaggtt aacagtgcag 142200
aatggaaagt caacatgatg taaataaatg aaagtataaa ttaaggtctt gatctctcat 142260
gtattaattg aggattctta cgtatgttac ctaagtaaat tacatcatag cattttgaat 142320
ttctaatgag ataaggggtg taagatgttt ttggaactgt aaggcaccat ctgaatgtga 142380
tttattaata tagctagtag tctttcatat tagtaaaatg cataggaaga atgtttcttt 142440
cccttttaaa tttatttttc aacccaccaa gtgaatatat attcctggta ggaatgagat 142500
aaaagtatcc taaaagtata tgaaagattt ttctcccatt ctcatgatca ccatatatac 142560
ctactattta cattttggtg tgtatcttcc cagacctttt tttttttttt tttttggtga 142620
ggcagagtct cgctctgtcg cccaggctgg agtgcagtgg cacgatcttg gctcactgca 142680
acctccgcgt cctgagttca agcaattctc ctgcctcagc ctcctgagta gctgggatta 142740
caggcgcctg ccaccacgcc cagctaattt ttgtattttt agtagagagg gggtttcacc 142800
atgttggtgg tggtctcgaa cccctgacct cgaacaggct ggtctcgaac ccctgacctc 142860
atgacccacc cgcctcggcc tcccaaagtg ctgggattac aggtgtgagc caccgcaccc 142920
ggccttccca gaccattttt atatgcaaaa aaacttatta gtacgtatca aatacaggat 142980
gctctacaac atgcttgctt gtttcttacg atacttacag ttttcatctt ttttagaaaa 143040
tttcgtatta tatatcatgg acatccttcc ctggcagtac atttggctct gtcagtcttc 143100
tccgtttgcg taccattatt ctaatttatt caatctgata tctatcaaag aaccactttg 143160
attctcattt tttgttattt catcttgctt catgagcatc tatacacata catctttaaa 143220
tacttgtgta cttatgtgca tacttctact ggaaatatgc ccatttaaag tttggtatat 143280

cctctcaaag ttgaccttcc aaaagagcca tggcagtttg tagagcttgt gctttgaata 143340
aaagactatg agaactttgg cttcttggta actttgcatc ctattagtct taagttgagt 143400
ttggtatctt ttgattcaaa taaggatctt tgactctgtt atgctttctt tagtgatctc 143460
acttttctgt gcttggtaac ccctttgtat ttatgtttaa gtggttttgt ggccattagg 143520
taattgtaga ctttatatac aaaacttatt cagtcatttc tttaaaaggt aaactatagg 143580
gcttagcaca ttggctcacg cctataatcc cagcactttt ggaggccgag gcaggaggat 143640
tgcttgagct caggagttca agaccagcca gggcaatata gtgaaacccc catctaaaaa 143700
aacttaaaaa ttagctgggt atggtggtgc gcacttgtag tcccagctgc ttgggaggct 143760
gagggggtgg tgagaggata gcttgagtcc aggaggttga ggttgcagtg acccgtgatc 143820
acaccattgc attccagtct gggtgataga ggaagactct atctcaaaaa aaaaaaaaaa 143880
aaaaaattga atctataaac tcgttttcag gagctctggg atttggtggg aatggggcag 143940
taagtgctct gctttgagat tcaggtggta tctgttgtca tttaaccatg ataaaaccag 144000
aagactagaa ctgcaaagaa aatctaccat cttaaactca tatgctagaa ttttaaataa 144060
tcaaaatagg aattttttag tattttctag tcaattttta tcttctatta atgtatttat 144120
caaaaattga ctgtagtttg tattgcaaat tttaatatat agacataaaa taagttattt 144180
gatactgtta ctgatatcta acactaaaac tctattttat tttttactac agtgactgta 144240
aaaagcttta tttttctttc aagtaaacat gcattgtttt ataagtgctt ctcatttttc 144300
ccttgtttat atgataagct agatagctgg gtagcaggaa cactgaaaaa tttattttta 144360
aataggtaaa tattgaagaa tgttgttttt agaaatgctt aactacttta ccttttttgt 144420
aacaaacttc aaagttactg ttgagggtaa tttaataaat gaactataaa agtgtcttgg 144480
gctcagtaaa aaaaattcaa ttaaaaaact cagtaaaaaa attcaaaaga aagttttgaa 144540
gttgtctacc attgcctttc catttgattc cgtttatggt atcatttgaa ttaattgttg 144600
tctgtttttt catattctgg attaggaatg ttttgagtca ttgttgagta ttcttcactg 144660
gagctggacc accttagtct taggagttga agaacttaga ggattaaaag gattccagtt 144720
cacagctaca ctcctagatt tagagagact gcgctttgtg ggtacctgtt gtctgaggtt 144780
attgcgtgtc tatacctgtg aaatttaccc agtgtcaggt atgatgcatt ttttttaaat 144840
gactataggg agaaaatatt agagacattg agaagattta ctttgagata actgagagtt 144900
tatgcccccc agctgcattt aagctgcttc taaaatgagg ggagaaagtc agcttgctat 144960
gatgcttatg tatttgctta taaagagtaa aatagattac atgtcattct gatacaaaaa 145020
gtaatagtgg gccaaagtta aataatctaa taaagtttaa ttttaaacaa attctgtcaa 145080
atagaaatat tttgaacaag caatttggac ttaaatgctg aaactaggtt tttattcccc 145140
cttccattta gattaaaaat tgggactaca cttaatctct aagttgtttc atttctaaga 145200
tattagtact tgtgatactt agctgtatag ggaattactg aattatattg attgttttca 145260
tgcatattta atattttgct taattaccaa tctgcttttt ccccctcaaa gctacaggaa 145320

aagcagttgt agaagaaact agcaaattag cagagtgtat tggaaaaacc agaactttgt 145380
taagaaaaat tttatcagaa ggagttgatc actgcatggt gaaattggat aatgatcctc 145440
aaggatatct cagtcaaccc ttgagtcttc tagaagctgt ccttcaggaa tgtcataata 145500
ctttcactgc ctgctttcat tctttctacc caactcctgc cttacagtgg gcttgccttt 145560
gtgatctgct gaattgtttg gatcaggtaa tttaagtttg taaaatgtta gttgaaaatc 145620
tgatatattg cttaatactc ttcaagaaaa taaattgcag ttgccatatc ttcttaactt 145680
gtataaaaga taaattagga aatcattcgg tgtacatgac ttatataagc aaagtttaaa 145740
attttttaaa tctgcttcat catcttttag ttacaaaatt ggtaggcaca ttgtgcagtt 145800
gctgattggt ttttgaattg gagtgagtga ttctgagaga gggagagagg aaaagaacca 145860
tagtgaccag gggagagcag ggatagcacc aggaggcccc cgccaccctc atctgtgaga 145920
acaggaagga gagagagctg agagaacagt catactagca ttgttcattc attcatgagg 145980
aagccacaac ctttgtaaac ctttatgcac caagtcacag agtttcacaa cacctgaggc 146040
aaaacactga cagaactaca gggagaaata gattcacatt gacagctggg gactttaaca 146100
ctcctctttc agtaattaat agaggaacta ggccaaaaac atcagcgatg cagatttgaa 146160
cagtactgtc aaccatcttg atctgacatt tacaaaacag gatatgaaac tgcaaaatac 146220
atattctttt caagtacaca tgatttttaa aagaaatatt tgggaccatt cactaaggta 146280
gatcatatgc tagaccataa aatgtcttaa taaatttcaa aagattaaaa tgtgaaagaa 146340
tatattttca gaccacagta gaattacagt aaaaatcaat agcaaaggat atccagaaaa 146400
agcaccaaac atttggaaaa tgacagcact tctaagtaag cagttgatca aagaagaagt 146460
tataaaggaa attttaaaat gtttcacata gaaagataaa aacataatat cagaatttgt 146520
gggatttagc taaagcattg cttaaaagag ctctttatac ccttaaatgc ttatatgaag 146580
aagaaaggtt taaaatcagt gacctaagct gctatgttaa gaatctagaa ggaaaagcaa 146640
atctaaccca aagttagaag gaaggaagga aataataaag ataagagcag aaatcaatga 146700
aatagaaagt agagaaaatt aacacagcta aaagttggtt ttttgaagaa aaaaagttgg 146760
taaactccta gcaaacctga ctgggagaga agagagaaag aaaaaacatg aaataccata 146820
atcaaaagtg aaataggggt tcaccgggca cggtggctca tgcctgtaat cccagcactt 146880
tgggaggctg aggtgggtgg atcacttgag gtcaggagtt cgagaccagc ctaacatggc 146940
aaaactcagt ctctaccaaa aatacaaaaa ttagccaggc atggtggtgc acagctgtaa 147000
tctcagctac tcgggaggct gaggcacgag aattgcttga aggcgggagg tgaaggttgc 147060
attgaggcaa gattgtgcca ctgcactcca gcctggtgac agaatgatac tccatctcaa 147120
aaaaaaaaaa aaaaaaaaaa aaaaaaagaa atagggacta tcactataaa caactttata 147180
ctggtgaatt tttcagcctt agaggaagtt gacaaattcc ttgaaaaata taattttatc 147240
aaaactaaca cattaagaaa tagaaaaatc tgaattgcct catatccatt taaaaattat 147300
caaaaacttt cccacaaaga aaatttcaag tccaaatatt gtcatttatg aattctgtta 147360

aacatttaag aaagaaataa tgtcaacctt ttacaaagtc ttttggaaaa tacaggaggc 147420
aacacttccc aactaatttt atgagtttag ctttatccta taccaaaacc taactaagat 147480
actacaagga aaaattacag atgaatatca ctcagaaaca tagatgctct taaaaatatt 147540
atcaaaccaa attgaacatg taaacaatat atcataacca agtgggattt atcttagaaa 147600
tacttagtta aataattgaa aatcgatgta atttaccatg ttaactgaaa aaagaaggca 147660
aattgatgat ttcctccata tagctacagg aacagtggtt gatgaaacta gcagttactc 147720
aatgatgaaa acctctcagg aaagtaggat ataaggaaac tcctaaatct gataaaaaga 147780
tatttacaaa aaaatcttca ctaacaacat cccacttact ggtgaaatct gaatatgccc 147840
ctaagtttgg aaacaagaca cgtggtatct acactcacca cttatattca gcagtgcact 147900
ggaagtccta ggcctccccg ccctccttcg tgaaaaaaat cattgtcatc actaatgcat 147960
cccaggcaac tttgcatcca tgtagcagag tagtatgttt taccactcaa tttccagggg 148020
caagattatt gcctggacta gtgcacaact taattactct ctcactctga accttcccag 148080
tctgatatgg ttcttttctt ctttgctaca gaacaggcta gatttagcaa atgttggttt 148140
aacatatgga atgttactct gcttttatgc ttaactttgt aacacagatg tttgaaggac 148200
taggtaatgt gtacacaatt ttgtgaatta ggatatcatg tgttggcttt aatgcttttt 148260
tatgaaattt tgtaagtatg ctactaattt ttcttggtaa attatggaat gtgataatat 148320
aattttgtga ccttagtgaa atgtgcttgt aatagcacag atttataatt tgcatcattt 148380
attatttgag aaaaatagtc tcagtcctta ttgaaaatac cttagataca ttctaattgg 148440
ggcctttttg gcatctccct ttgaaacagt gctagagagt actgtgattg tgaggatatg 148500
aaaatataaa aatgctcatt tactagagga caggagcctg atttttcttg accagactta 148560
atttttcatc ttcattgaat agaaacttaa acaatttttt aaatatatag atgtagtatt 148620
attctataac aagacaattt aaatgacaga gtgacagaaa attttggtat aatttaaaag 148680
atggtttacc ttttatgtat tcagaaactt agattatact gatgctatat tttttggttg 148740
gggggccata tttttgtttt taaaaggata tccaagaagc aaacttcaag acatcaagta 148800
gccgactcct tgcagctgtt atgtcagctc tgtgtcacac gtctgttaag ctgacttcca 148860
tcttcccgat tgcgtatgat ggagaagtat tactacgatc aattgttaaa caagttagta 148920
cagagaacga ctcaacacta gttcatcgtt ttcccctttt ggtggcacat atggaaaaac 148980
tcagccaggt aggtctgtct ctgaaatctt ttatacagag gcactaaaac tctaaatggt 149040
tttatttatt gcctctttta tacagaggca ataaaactat aaacacacca agctaagcgt 149100
atttagcttg gtgcttattc ttctctattt ttaagtatat ttacaaaatg attattttaa 149160
atatttttga aaaatgttca tatttaccta taaaatgaag tttttttgct ctaacggttt 149220
aatcacacac ctttaaaaaa atttgagagt tatacaatga atacctacat atttgttttt 149280
cctgaaccat ttgaaggtaa gttgcagata ctttacattc taatataata ttttagattt 149340
ctaagataga acaataagaa aataaaggat ttttaaaata tctacttaat aacactttgg 149400

tatattaaaa taagcactgt aaataatagg tataggaaac attcacataa atcattttca 149460
tgctggaaat cagctaaatt aatcatcttt tccctttttt ctgaaaaata aagtattttt 149520
cctttattga gactctgtga cttcttgaaa tttttctacc ggtaggggtt ggaattttct 149580
tatctccact taggaaaaca aatacagtag aaattataaa actacatagg aatcatatgt 149640
gtgtacaaaa agaattttgt taaaaaagtg atttacacag actacagttg cattacagct 149700
aaatatacta gaatttatta tctcagatta agaccttgtc ctatagaaaa agctttttat 149760
ttttgttttt taagactagc tcattctgtc acccagtcta cagtgcagtt gtgccatcgc 149820
agtttactgc aacctcaacc tcctggactc aaacgatctt cccacctcag cctcccaagt 149880
agctggggct gtaggcacat accaccacac ccagctaact ttttaaaaaa acattttgta 149940
gagacaaagt cttactatgt tgcccaagct ggtctcaaac tcctggcctc aagtgatcct 150000
cccatctcag cctcccaaag tgctggtatt gctgacacca cgcccagcca aaaaagcttt 150060
ttaaatggaa agaagttttc tgtctgtcat atagaattgt aaattgaaag ttaaataaga 150120
gaataaaata ggactcaaag ctaataaaaa catttcgtaa atgtatggta ctttgatacg 150180
cagagacaga taattcaaaa aagaacttct aagtgaaaag tggctccaca tataaccaga 150240
aagatttgta gaaattgacc ctgattcaag tctgaatttt aagagtatca gaaagacaag 150300
gttttcaaat tgagtactcc tatgaaggaa aataaaacaa taccttagaa ctttttctta 150360
tgtatcgcag agtgaagaga atatctcagg gatgacaagc ttccgtgaag ttctggagaa 150420
aatgctggtc attgttgtgc taccagtcag gaacagcctg aggagagaaa atgaactctt 150480
ctcctcccac ctcgtctcta acacctgtgg attactggcc agcattgtca gtgaactgac 150540
agcgtcagcc ctgggatctg aggtaataca ttatattttg acactggata aaaagcacat 150600
gtgtaagttt tttacacagt ttcttttttc aactattttg gctttgtgga agctaatttc 150660
agattggatt agcaacccca atgcattgtt tttctttttt gcctttctct ccacctccta 150720
ctttctctga tttgttactt ctctctcacc agtttaccca gcatccacag gaatataaag 150780
tatacatttt cctcatagag caggtcgtca tctgccccat tgcacaagcc caccaccatg 150840
ccccgtgaaa agcaaagata gttctcactg ctggaagatg agtaacattg ctgaagtcag 150900
gccctactca tagtgcacac atagtgtggg aaaggtaatg aggtttagac ttcagctctg 150960
cccactcaac cgaccctaga gaaatacata cttaaaattg gaataataat ttttatttca 151020
cctctatcac aggttttttg aaattatagt aaaatataca taatataaaa tttgccattc 151080
taatcatttt tacgtgtaca gttcagtggc attaaatgca ttcactttgt tgtacttagt 151140
tgtacattat cacaaagttt tcaaagtctc aaatattatg tttgagaaaa tcactttgaa 151200
agtgccttta cattttgtaa agtaacatta ttccatttca ctgccttact ctttcttata 151260
acttaactga atgttttgac tgaatttggg gaatgtttct gttttttcct cctaaaaaac 151320
agacagtaga ttggtttgga aaatcatgtc ctaatatgaa atatcttaca tcattcaaat 151380
gaatgtttaa taatcagcat atattcagtg gccctagctc ccaagttctt gtcaaggaac 151440

ttcttttctc tattttactt ttataattcc taatctttgc tttttttaaa aaatttattt 151500
taacacctat tctgaattaa cccatccaat atatacacat tattgattaa ttgtgacagt 151560
ttagccttta gaatttcatt gtatgaaatt catacgtaag aaattcgcat acatatggtt 151620
catcgcatac acttgagcta attttagtct ctcatcctgg gaagcacttt ctaagtttct 151680
tggttaactt tgagtttggc ctcagagacg attgaagccc ttgtcagaaa ttttttaact 151740
ttttacattt actcatttag tctataatac cttcctttga tagtatactc tgcaggatgc 151800
aggaaagtct ataatgtgta gatccccttc caaagtttac taaagagttc tcccctttaa 151860
acttaccttt ccaatgaaaa ggaataccaa aagttacagg gtgcctggta tacaactttg 151920
ggagttccca caggtcctgg cttagagatg aatatttctt tctcagagaa gtttgcaatt 151980
tttgttagaa tcataaactt ccatttttgt atctatttag tctcccaaaa ttctgtagcc 152040
aagttagatt agaagtaggc tttgtaaata aaaagagcga tttcaaccca aagtaagttc 152100
tctaattctc tctagtgaaa gtctataatt agtatagttg cacatgtgta tgtgtacgta 152160
ttcaaatact gaatgctgtt acaaaaatat cactgtatat attcgtagta ttcaatctta 152220
gggtatagac cattttcttc aaatgaagtt ttacttgcaa attcacaggt aagaggagag 152280
aggacatagt ctggttgaag aggggaacca taaatttacc tcttccttgc acacctatct 152340
ctgcagaatt tctagagctc tgtgttgcat agtttgaaaa ctgttgatct atagaatttc 152400
gatgaaattt ttagcatctc ttttcaaagt ctgatgattt attcatacat tatgactgat 152460
aagacaagct tgtacttctg acattataaa ttaagtggaa agtatagttt catatggcta 152520
aaggaagaaa atactgtcaa gtgacattga tttgtgaatt tagaatgata aaatctggtt 152580
ttattgtgag ttaatctctc agtagaacat acctttagga atgtttatat ccacttatta 152640
aataaaggtt attttattag acttcttaga aacctttgga tggttttgtt aaaggtatac 152700
caactactga tacaattgct tttattgttc acaggttgat ggacttaatt ctcttcactc 152760
tgtaaaagct agtgctaacc gatttacaaa aacaagtcag ggcagaagtt ggaacactgg 152820
gaacgggtcc cctgatgcaa tctgtttttc agtagacaaa cctggaatag ttgtggttgg 152880
tttctctgtc tatggaggag gtggaattca tgaatatgaa ttagaggtgt tggttgatga 152940
tgtaagtatc acccttttag tatttatcct gattagtggg ttgtgtatca ggatttacca 153000
ttgtcatagt atgttcaggg gtttactaga tcaatcactg atgaatgaca ctaaagaaat 153060
tgaaaaagac tggccacagt ggctcacacc tgtaatctca gctctttgag aggctgaggc 153120
agttggatga cttgagcaca ggagttcaag gccagcctag gcaatatggc aaaaccctat 153180
ctctacgaaa aatacaacgc acgcctgtaa tcccagctac tctggaggct ggggtgggag 153240
gatcacttga gcccgggagg ttgaggctgc agtgagccat aatcatgcca ctgcactcaa 153300
gcctgggcag cagagtgaga cccgaccctg tctcaaaaaa aaaaaaaaaa aaaagaaaga 153360
aaagaaattg aaaaataatg attttacctg attgtttgtg ttccatcttt ccaccttggg 153420
tcactactgc ttcatatttt ttgtactttc agaaagagaa agatttaaag tagaattaaa 153480

gactaaaagt gtttagctaa gtccctgatg acaagtcttg agtagttttt tttttttttt 153540
atattgtcat ttttcaagaa acacaatgtt gagatggact atgtcataga gtactaatgg 153600
gtgattctta gtgaagacac tatttgttga ctgattgagc tatatctttc tatattctct 153660
aaaattaaag tacctgagtt ttattaaaat gggacagatg gggtagcatg taaatcattg 153720
cctattaaat agcaagtcta tctctatctc taacttgatt cctttcacta gttaatttga 153780
atttattccc tacttcatta ggctttataa atgtgaagaa gtagaaatag tttaagtcat 153840
cagatgagaa aagcttattt atgttgtagt cttctcaaga aatcacataa aaatgtatat 153900
agtaccccca actttttctt tcatatgtat gggaaaatac taatggctaa ttttatattc 153960
aagaaaataa attttaaaaa taataattat aaatcaaaat tgttcataca ataaaattaa 154020
gagtctgttg gaatttttgt gtttcatttt ttttttttag agtgaacatg caggagattc 154080
aactcattcc cacagatgga catctctgga attagtgaaa ggaacgtaca caacggatga 154140
ctcacccagt gatatagctg agatcagact tgacaaagtg gttcctttaa aggtagtttc 154200
aactgaatgt ttctattgtg aataagaggt attattatgt agttaaaata gtgaagagta 154260
ttccatgaat tttgtttgtt acctatagct atttgtaagt tatcctttat atggtactta 154320
aatatttatg gagtacctac tacgtaccta atgttgtacc acattatatt atatttttgc 154380
ataatgtttc ttaaagtcag gagtagtagg aagttttgta gtcaaacaga cttagctttg 154440
gatacctgct gttccacatg ctgactttaa gacactggca gatcattatt taaccacttt 154500
gagacttatc tgtagattgg gaagaataat acctgcattg tagtagtatt gagattaaat 154560
aatggaaata aaacacctgc cttagtgtct agcacaatat gaaaaataaa tattagctcc 154620
tgtcttttct tctcttattg tgccctaact catttaaaaa aaaaaaaaag tgtttttgtt 154680
ttgttttgtt ttgtttcttt attttttttt aattattatt attatttttt tttagatgga 154740
gtctcactct gtcgcccagg ctggagtgca gtggtgtgat cttggctcac tgcaacctct 154800
gcctcccggg tttaaatgat tctcctgtgt cagcctcccg agtagctgga attacagcca 154860
cgtgccacta tgcccagcta attttgtatt tttagtagag gggtttcact gtgttgacca 154920
ggctggtctc gaactcctgc cctcaagtga tccactccgc ttggcctccc aaagttctgg 154980
gattacaagc gtgagccacc gcgcccagcc tatttcctaa caatatttgg caaaatagag 155040
aaattttacc acatcaaagt aattatagat gttaaaataa aattaaagta tttctgaatt 155100
caactgaaat aaatttcatt atagagcaga attttggata agatcggtac acgagaggtt 155160
tcatcaggaa gaatttagat actgaaaaac caaaaagctt ttattgtgaa atttatatca 155220
tgggtcataa agcattagtt tctatagctc acattttaaa agtacagtgc attatatttt 155280
ggacagcttt tttcctatgg gttttttttc tcatgtctgt ttgcctatat atttctatct 155340
ctgagttttt cttaaaaata taatagactc accatttttt tcctcacatc taaaattatt 155400
ttaaaagtta aactagtcaa ggaaaagtat cagcttatgc aattaaaatc ccttattgcc 155460
gggcacagta gcgcacacct gtaattccag tacttttgag acaccaggga gggaggatca 155520

cttgagcttg ggagtttgag gctaggctgg gcaatatagt cagaccttat ctctacagaa 155580
acaccctaaa aaattaactg agtgtgatgg aacacaccgg tgggaggatt gcttgagccc 155640
aggaagagga ggccacattg aaccaaaatt gcaccactgc actccaggct gggtgacaga 155700
gcaagatcct gtctcagaaa aaaatatata tatataatat ataaaatata tatatattat 155760
atatatataa tatatattat atatatatag tatggctaat attctagcag taaaattaag 155820
agcaggcact agttacattg gttgtgttca gacctagttc catcacttac tagctgcgtg 155880
actttgaaca agttacttaa cttctctgaa cctcagattc ctcatctgta aaatagggat 155940
agtaatgata cttaccttat gaggtcattt taaggattaa ttaaattcca taaagtagtt 156000
aggacagttg cttttatgca acaggtgttc agtaaatgtt agccgtcatc gtgtttgatt 156060
cattaaaaac ttatttaatt atatcatctg taaatatgac aaagaatttc agtatatttg 156120
cctattttac tgctagagtt ctttttatat atcctctgta cattgtgaat aaaagatgtt 156180
ttacatcttt ataatgaagg aaaatgttaa atatgctgtg cgcttgagga actatggaag 156240
ccgtacagcc aatggagatg gaggaatgac cacagttcag tgccctgatg gtgtgacatt 156300
cacattcagc acgtgcagct tgagcagtaa cggcacaaac caaaccagag gacagatccc 156360
acagatactc tactataggt gggtgaatgt atagagataa cggaaatact ttacagtggt 156420
agaacataca cagtctacta ccaggtaaaa ttgctaactg gatacttata attaaattgt 156480
acagtgtatc ttcttagcac atacttgtac aaattcatat aaagaaatct ctttttaata 156540
tataaattca atgtttgcta caaaagttaa tgctttaaaa tatttttgct gttatttctt 156600
caatatcatt gaaatgtttg tgtactgggc atatgtgctg ttgctgtcac tctataattt 156660
ctggtctgca atttcaaatt taaaggatat tcatttcttt ttataaaata cctcaaacta 156720
atgatcagat agactgtact cagatagtgg tgttgccttg atcttgttat ctagtcacag 156780
aatattttga tgtctgtgaa tcttggacct gccccctggg gccctagaat atataatagt 156840
gtgtttctta taatgttttt aacaaaaacc attccttatg taaaccttgg aaaataaaag 156900
aatgataaca acccttgggt taaaaaaatc ttttaaataa acatttctca ctgagtctga 156960
atttttcttg tagcggcaaa gctggcgtct taggtagtca tgagtaccat ttaccacatt 157020
tgtcctcagc ctgacttggc attaaatcac tgaatttcca tgtcactttc agaggcttgc 157080
tgcttcctct ctttccttca tgaagagtct agggatggtg cctacgtaga tagtataaaa 157140
taatttccct agatatagat tttctgcttc ctctttccat cagaaataga atagaaatag 157200
caggcagcaa atagctatgt agttcattgt tgctgctgaa atacaaaagc tgtctagtct 157260
tttgccccag tcaagaaaag ctttctgtta cataagcact acaataaata gtacattatt 157320
ctctttctta aactatggat taaaaactct gttatgcgta agagttacgt gtttcagaaa 157380
gctggagagc cactaagagc atctgccatc caaactttat atcttataca aaatacagtg 157440
ttttgagtat tttcatgttt tctattaagc tttaaatacc cttagcttta cattttttat 157500
aaatgaaaaa tgttaaagtc ctttagtttt aaataccatc ctgctctgat agctcccaag 157560

tttacatccc tagccttcat gacctctgag ccctggactc cgggtctctc ttctgacatc 157620
ttcatttgga tgacactagt ggagatggtg ttagttactg tgtgccagga catgctttga 157680
gtatgttacc tgtgttattt caccttgtcc tcacagtaac cccaggaggt aggtactttc 157740
cttatccctg tttcacagat gatggaactg agacgctgaa ttgtctagag tcacacctct 157800
gttaagttac gctactgcga cttgtaggca ggaagtcaga tttcaggtga tacaccctta 157860
accagaatgc tgcactttct ctttaaggat gttcgttata gttggctagt agacatctga 157920
ggtttcatgt ggctagaata ttcatctccc ttccacagtt acagccagac accttggagt 157980
cattctggac ttctctttcc ctcatacttt acacctagtt catcaagaag tcctgtcagc 158040
tgtacttcta aaatgtccta aatgtgacct tttcttctac ttccatattt aatagtctag 158100
accaagccac tgcaacctct acctgctaac tgtaataagc ctctaaacta gtctccctcc 158160
tttcactatt ggtctcctac aatcagttct ctacagcaca gccagagtga tctgataaaa 158220
atataaatca taccaagttc cacccttgct ccaaacccag cttcccattc tcataccagt 158280
ataaaatgta aagtcattat gtggcctaca aaccttacac aatttgactc ttgtttccct 158340
tcccacccat cacgcttcac tctttctcct tttcttccag ccatacatgt cttcattctg 158400
ttcctttaat tcaccagtat gttccttttg ccctgacagt tttccctcag ctcttcgcgt 158460
gggtctttaa aaaaaaaaaa aaattcaggt cagctgggtg cgcggctcac tcctataatc 158520
ccagcacttt gggagtccaa gatgggtgga tcacgaggtc aggagtttga gaccagcctg 158580
gccaacgtgg tgaaacccca tctctactaa aaacagaaaa attagctggg cgtggtggtg 158640
catgcctata atcccagcta tttgggaggc cgagggagga gaatcgcttg aacctgggag 158700
gcggaggttg cagtgagccg agatcatgcc actgcactcc agcctgggcg acagagcaag 158760
actctgtctc aaataagtaa ataatttaat taattaattc aggtctttgt taaatatgag 158820
ttcctcggag aactgatctt atttaaaata acacccttca ctttatgccc tatgctgctt 158880
tgtttttctt caaagccctt atcattccct attattgtat agttgcatgt ttactttttt 158940
aacttttaaa aagctataaa ttaaatgagg atctcagatc tagatatgat ttctggcact 159000
ggatcatgtt ttacttacca ttgtaaaata atctcatgtt tgctcatgct acaggagtga 159060
atttgatgga gatttacaat cccaacttct gagtaaagcc aatgaagaag ataaaaactg 159120
tagcagagca ttgtctgttg taagcactgt cgttcgagcc tctaaggacc tcctgcacag 159180
agctcttgct gtggatggta atattttttc tttctcagta gtcatatttt agattcttta 159240
ttcactaaat ttttgcatag aattattgaa gaggagcctt ctattataca attttataga 159300
atctaagtgt tatttataga tccacccatg gattaatttt ggataggtca cactctccat 159360
actgtaatgc agaattattt agaatgctca aaaggagtta ccatatattc ttattttctt 159420
agttaattta gtaaaagaaa aattgtattt gagttaaact gggtctttat tcttctttta 159480
gtaatcaaaa agctttttct tcctctctgt tctttttcca gtaatgtatc gctatccacc 159540
caaaactctg gggtacccta accatctcct cagaggcact tgtctaaatt cagctctatg 159600

agtttttcca ttggaatctc tctagtttgt attctcctct ctttcacttc ttctcctgct 159660
gtcatttaga ctgccatggt gcaagtacat ttcatttttt aatatagggt taatatcccg 159720
ataatctcac tgctgccttt cccccattct ccatctagtt gtttagttat tcttcacact 159780
ggcagcagag ttatcattat gaaaacatgg ttatggccgg acatggtggc tcatgcctgt 159840
aatcccagca ctttgggagg ccgaggaagg cagatcatga ggtcaggagt ttgagacgag 159900
cctgagcaac atggtgaaag cccatctcta ctaaaaataa aaaaattagc caggcgtggt 159960
ggcaggtgcc tgtaatccta gctactcaga aggctgaggc aggagaatcg cttgaaccca 160020
ggaggcggag gctgcagtga gccgagatcg caccactgca ctccagcctg gatgtcagag 160080
tgacactcca tctcaaaaaa aaaaaaaaaa agttacctcc acctccttat tgtataacat 160140
taattcctaa acctagttta acatcagaac cacctggaaa gcttgctgaa aataaagact 160200
attagattta tccccaggtc ctgaagctag gctaaaaaac tattttttaa ataagtgtgc 160260
agcctgtttt gatataacag cagacctagg aatcagtatt tgggagaata attggcttat 160320
aaaataaagg tcttcacagt ataggctcag cttatctttc cagcaccatg ttcatggata 160380
actgactaaa atgttaatca gcaaagtgct tttcttggaa aaacagaaag caatgtagct 160440
taataacagt aaacattttt ggagtgctga atatgtgtag gtgttgttct aagtgtttta 160500
catatattac ctcgtttaat cttcccaaca tccaaatgag aggaatactg ttgtgaacct 160560
aagttacaga gtaagtaatt tgcctaatgt cactgataca gccatgtttg gtgccagaat 160620
ggtaatttaa aataaagact ccattaccct aggttctgat tccagctcta ttattttaca 160680
ggtacatgta cttatgtatt aagtattttg ggtaagatac ttaaacccta tgtgccatgg 160740
ttttcctttc tgaaaattgg ggataataaa agtatcttat tgtgagtatt agttgagata 160800
aggcatgtat tctcaaaagc ctttagaata gtaccaggcc cttgacaagc acccaattgg 160860
tgtcaactat agtttcgctc aaaagagcag aatatagatt tagtacagca tgtatgcagt 160920
acaatgaaat taccttctga ctatagtttt aatgcctttt atgccttttt ttagctgatg 160980
acattccaga actgctgagt tcttccagtc tgttttccat gctgctcccc cttattatag 161040
cctacatagg accagtagct gctgctattc ccaaggtgtg taatttaatt ctataacttt 161100
gaatgttttt tttaaatatc ttttttaaaa aaagactttg tgtcttgtct ttacaaaaac 161160
ttgcatcttt gaggcatctg ggatataggt aacaagcaga gatagatctg ctaattatta 161220
aacttctctc ttcttagtat tgtttactgg attgctgtgc ttatttgttg gctttttttt 161280
tttttttttt tttgagacgg agtcttgttc tgtcacccag gcgggagtgc tgtggcgcga 161340
tctccgctca ctgcaagctc cgccttccgg gttcacgcca ttctcctgcc tcagcctccc 161400
gagttgctgg gactacaggc gcccgccact gcgcccggct aattttttgt atttttagta 161460
gagacggggt ttcaccgtgg tctcgatctc ctgacctcgt gatccgcccg cctcgccctc 161520
ccaaagtgct gggattacag gcgtgagcca ccgcgcccgg cctgttggct ttacatttga 161580
agaatctgtg aatgggaaat agattttagc aattttagga atatgatatg taaggaaaga 161640

ttatgttaac tgaggttttt cacatttatg aggttgatgc caaatggtct tcattaataa 161700
caatagcaat gttaaaggat aagagtgttt taaaatagtg acatattcaa gagatggaag 161760
aaatgataaa ctattgattc tttggcataa ttaagtaaaa cctgatttac ttcagatcat 161820
gaaaaaacaa gtttttaaaa atatagattt agatattgtc atgtaacatc taagataaat 161880
ctttttttaa tcaattgaaa gatttctctt tggacaaaaa cttaactagt ggaaaattta 161940
gatggaaatt gttattagag taaccaacaa ttattgtaat gcataatttt agaaccactt 162000
tcacgtgtat ctcatttaac catattttta tatgtgtact aggtggctgt agaagtcttt 162060
ggccttgtcc aacaattgct tccgtcagtt gccattttga atcagaagta tgcaccgcct 162120
gccttcaacc ctaatcagtc gacagatagc accacaggaa accagcctga acagggcctc 162180
tctgcttgta caacctccag tcactatgct gtcatagaga gtgagcaccc gtataaacct 162240
gcctgtgtga tgcattacaa ggtaggcacc ggttcttagt gtgattcgaa tgtaaaactt 162300
ggcatgaggt tctctgatga tatcttaaag aatgcctggc ctgtatttac aagtgtattt 162360
tcttttatag tatttacata tagagaatca gcaggtgatt aggcactgtt ggatatataa 162420
aataatttaa gatggaattt ttatctcaaa gatgataaaa ttttattggt ttatgagagt 162480
tgttcttaaa tcttcctaca agtatagtgt ggtatataat gctaaacttc atgctgtgat 162540
ttgcaatagt agtagatata agcattaaca attttaaatt gtattttttg aactaacatt 162600
atttgtacca cagcctgagc tctgaaaatt tacaacccca atgtaagatt taaagtagtc 162660
tatacataat gttaaaaaaa ctatgtataa aatgtttata aatttaatgt tattagagag 162720
tattcaaaat ccgtattctt tgttttttca ttgtatcatg caaattaagg aagagaagga 162780
taaacttgta ttttgtgtgc ttgtaaaata ttccactttt catggtagaa actattaagc 162840
atatagaagt aaacaagcta gtacagtgca cttgtttatc catcatccca gattcagcag 162900
tttcaactgt tagtcagacc tgtcttattt attcccaacc tacttcctaa cctcctatat 162960
tgttttaaag cacatttatg atatttattg atacaatgtc tccaaaatat aaagaccctt 163020
aaatataacc atagtattat cacattgaag aaaacaattt aatgtctgca aacatcatgt 163080
aaatttttaa taatttgagt tagaatccat ataaaatcca tatattgtga ttagtatgtc 163140
tcttaagtcc cctttaatct taagtccctt taaatctata agtctccctc tgtctctctc 163200
tctttttgtt cccttgagtt ctatttgttg aagaaactgg atcctatgta tttgttagat 163260
gggagcctct tcagcttacc tgctggttct ttcagacacc cctcttgcag tcattcctag 163320
cttccttgct ttctggtagg ataagatgtt ccaggctccc tttatatgtt tgatgcctca 163380
gacttggaac taaccatttt ttcaaagtgt cctgatttct gttatacata cacacacatg 163440
ttatatttat atatgtatat acataaatgt atatatgttt atgtacagat agatttatgt 163500
gtatagtttt ttaaagataa aatacctcat gagtttatac tgttgatact aattcaaaca 163560
aggaccacag acacacctca gtctcctttc tcttatatct aaaattccta ttagtgacac 163620
tgggaatatc agaattagaa aatcattaaa ttatttcatt tggtgtgtgt gtgtgtgtgt 163680

gtgtgtgtgt gtgtgtgtgt gtgtgtgtgt gtatgtgtgt gttttgtctt tgaagtatat 163740
cccatctaag tgtatgtcaa attactgtgt ttaaagtaag ttggaatgct ccctctgcat 163800
gattatgcat tcattggata tacacttaga gagtcattta ttttatctta gattttagga 163860
attgcttttt aaatttggat tttgttttat aattctttac ataattacat actttgaagt 163920
caaatttatg aaacaaatct agcttttatc tctgtcctct ttactctgtt cctccttctc 163980
tgtcataggt aacattttat tctattttaa ttttgtactt tatcctgttg tgtcatttaa 164040
taaaagcata gacacacaca cacatacaga tgcagatgtg tatgtttgtg tatatatata 164100
tctgtattca tgtcactttg cctgttgacc tcaggtgaca ttcccagaat gtgtgaggtg 164160
gatgacaatc gaatttgacc ctcagtgtgg tactgcacag tcagaagatg tccttcgttt 164220
gttgattcct gtcagaactg ttcagaattc aggatatgga ccaaaattga catctgttca 164280
tgaaaatctt aattcatgga tagaattaaa gaaattttca ggatcctctg ggtggcctac 164340
tatggttttg gtgttgccag gtaagttttt tttttcttct gttttatgtg gtaaaataag 164400
tgcatttatg tatatttagt aagggtgtat cattgaaaat gtttatctaa tttttagaga 164460
aatttattaa agacctacta tgtatctgga ttgcattcga ggaataaata aggtaccctg 164520
ggcaaggggc acagcctgat ctgagttttg gagaagacaa tgttttaagg attttgtgaa 164580
aaaaagatta gcttgagcaa agcagagtgt taacgttgag gagtagctag ctgtctgtgt 164640
agttgggtcc aaattataga aaacaatgga ttagatcttt tattttccca ggaagtgcta 164700
aattttcaga acttacctaa ggactgaagt ccttcattgt aagagaattc gtaaaccact 164760
ttcttgtgtg aagagaaact atgttttaaa acttatggtt tgttgcattc ttaaccagtt 164820
aaccaggact actcttgtga tccttcaaac taataggcaa tttcatttca gaaacttctt 164880
ctttccactc tgacctgcct atttgtttgt ttgtttggga aaagatcaga aaaagccaca 164940
ctccagggta gaggattgcc cactttgaaa aataaaaagg ctaacctcag tatttgacaa 165000
ataattgaaa aatgattgcc catgtaactg aagtacttta ttaagcaggg gagtttgatt 165060
atgaaaaaaa gtaacaattc atttgcgata gacctgttgt ttctagtgtt atttttggta 165120
cagtttcctc aagtagatat aggaacgctc cccgccgcaa gtctatttaa gttatgtagt 165180
atatgatttg tgtttttacc tatcttgtga taaaagagaa agtccattgg ataaaagaca 165240
gggaaatttt tataaaattg ttttagagtt tgaactcaat tctggcattt ttattaggaa 165300
atgaggccct tttttcattg gagactgcat cagattatgt gaaagatgac aaagcttctt 165360
tctatggttt taagtgtttt gcaattggat atgaatttag ccctggacct gatgaggtaa 165420
gaatgaatta tcttttcatt cttttaagga agattgtgtt tctaagggct agtgagttgt 165480
gcttctaact gacttaaatt gtactgttgc ctctattcga aaaattgggg cctggtgcag 165540
tggctcacac ctgtaattgc agcactttga gaggtgagag aatcccttga gacagtttga 165600
gaccagccta ggcaatatat cgaaatcctt tctctacaaa aaaaataaaa agaaattaat 165660
tttaacctag gggtgtgcaa ttctaaattt ttgtagttta tgtgcagtgg tctgttttgc 165720

tacttttaat acatgaagag gatggagttt gtgcaacatg acccagcctt tgaagtatag 165780
ttatacctgc cctgaacctg ctcagttggc tatacctttc tgagtaaaca gtagaacctg 165840
ttttcaaaaa ggggctatca tcttgtctaa atgttttaaa tcatacaata agataaaacc 165900
aaggacaaag tgggccaaag tcatgaatga acagttattt gaacaatgaa cctttaaacc 165960
tgaaccttat aaccagcctg agccacaaca ttgagtactg aatctcatct actctcaatt 166020
taaccattag cttcacgtgc ttccttctca tttcaaaagt aatgcttgaa ttcaaatcac 166080
attttgttga taatccgtat tatcattcaa acatttttaa agcagcaaat tttatgcaat 166140
tccataggga gtcatccaat tggaaaaaga attagccaat cttggtgggg tttgtgcagc 166200
agctctgatg aagaaggacc tagcacttcc tattggtaag tgtggccaat actggatgtt 166260
ttttgatagg gttttgtttt gttttgtatg tattataaaa gggccaaaaa tagatttgca 166320
aaatgaattt tgtaatctca tatattcatg agcaataact gcttcatgta ctggtaattt 166380
gagtttgttt agagactgaa gggtatcctc tgaccttttt taatttacac acgcacatgg 166440
acaccctttt tatgcatata catgctttga gccaggcagt gggtatagtg tgtatattca 166500
gccaagcact gaatatatag tattaaacca aaagttctag atatttctta aatattctag 166560
tggaaatatc aagtagacaa ctgaatactc attgtctaga tcagagaaca ggtcgataga 166620
aatgtaattg aatatgatcg ttatttaaag acatgggact gtatgaaaga tattctacag 166680
caagtagata agaaagagaa gatagcttag gattaaagct gtgacttatc aaaatttcaa 166740
agatgcacag aacaggaaga ggaggctgca gatgagactg tgaaggaaca gtcagtgaag 166800
aggagagaaa ccaggagagg ggatgctcct gggatttttg catgctctga gaagtgaatt 166860
aaaatctatc attggacttg aagacttgtt ggttgcttgt atatgcaata agaaatatgt 166920
tagcaaaatg agagaactgt aagtactttc agagtagaga gggagaatgg aaagtaagga 166980
agggcggtcc atttgaatat aggtagctct ttagaagaat ttttcattga agcagaacag 167040
agatgaggca gtggcagaaa tggtactttt ttatatggag caaatttgta tgatatatag 167100
attaatctat aatcaaggga gtaaagttga aagtaaaaat gcttactgat tctctaatat 167160
ttatacatgg tttaaagttt tattttctta cccccccaaa atccatcttt actccttcat 167220
tgaagattac tcttcccact gttatttttt tcctcccttt tcttcctctg cctttccttc 167280
tttattccct tttctcttcc tggtcccttt tattcttctc catccctttt ctcccactta 167340
atattggtat atatgtatgg aaatgagacc ttgtattcta gccacatgag gaaaattgct 167400
agcaacctat ttttgtcaat agctgttaaa ttttacatta tatatacttt ttgacataga 167460
aaatttttat acatacttgc acaaatactc aaaaagtgta tgttgaaggg tggattaata 167520
tagcagtgct tgtgattgta aaacctagaa acaaccaaaa tgtctttcag ttgggaactg 167580
gttaaataag taatggcata gccattcaat ggtagtatat gcagctcttt gaaaaagtag 167640
acctatacag cctaacaagg aaagctgtcc aaattatatt gagaaaagca agttgcagaa 167700
gaaggtccca tttttgcata cattactctg tatattacaa aaaagtatga tcatgcctgt 167760

aatcccagca ctttgtgagg ccaaggtggg tggatcacaa ggtcaggaaa ttgagaccat 167820
cctggctaac acagtgaaac cccgtctcta ctaaaaatac aaaaattagc cgggcatggt 167880
ggcacatgcc tgtagtccca gctactcggg aggctggggc aggagaatca cttaaaccca 167940
tgaggtggag gttgcagtga gccgagatcg caccactgca ctccagcctg ggcaacagag 168000
tgagactcca tctcaaaaga aaaaaaaaga gtatgtttat gtgtggttga aacacatata 168060
cctttttggt ttgtttgcac accaagtttc tagaacaaca cataggctgt tgacactgga 168120
tagttttaag ggagcacggc actggttaga ggtttagaat tttttgttct acactcctat 168180
acaatttgag tatttcccat tgaacaagta ttactttatt agtaacattt aataattaaa 168240
ctatcaaaga aagatcaata aataaaatat gttctatatt aactaataaa ggagaaaatg 168300
gctaggggat gcagtcactg ccttatattt ttctgtgagt ccctgttgtt tactaaagtc 168360
atagctcttc tgtaaggcaa ccaaaatata aaggcccaga tctgaaataa tttgttccct 168420
ctttttttag acaaaacgaa tacactaagt tcagaaagta tacatttata ttcataaatg 168480
taggatatat ctttggcaaa tcacccatgg atgcttcttg agtattgcct tgagaattgt 168540
agtatattcc cactggaagt atcttatata ttaaactgta tgccactagg attttttcca 168600
gaatcacaaa tcttttcaat ctggaatagc agaattattt ttttaaaaaa agtgtaatga 168660
atgcaaagga ctagaaaatt aaactaaact aaatattgac atattttagt aaaactcttt 168720
atttacacaa caggtaatga attagaagaa gaccttgaaa ttcttgagga ggctgcattg 168780
caggtattgt cccaaatgtt ttaaggataa actttgtaca ttaatttaaa aagtattgtc 168840
actcagatca gcctatttgt cttgaccatt gggttcattc attaagtact tcactttatt 168900
cattggcatt atagagataa ataacttatc attgctccct ttggtgggag aaacccatgt 168960
gagccctatg tatttggtgg gtaattgact ctggagttag acaaaaatcc ttcgggagaa 169020
aaaaacaaat caagtgccaa gggtgcggag gatggcttgc agaactgtgg ccatttaaat 169080
tgcatcttga aagaatttgt tttctttttt ttgcctgcta gagaaagggg aaagacattt 169140
cacaaaggat gtatagttgt gttataagaa gcagcaaaag gtgaggttgg tacagttggt 169200
gagctcagtc tgtgacaaac cagtatgtca tattaagtgg ttagggctag ttgcctgtag 169260
acaattggga gccatcattt gttgttttta ttattgtttg ctttaccttt tttgctcgtt 169320
taggcattaa ctagcacatt tggcactgaa tggaaataca tgagtactat atttcagtgc 169380
aataccccct ttggatctgt tacgtcaacc taatatttct caaaattgcc tagagatgac 169440
tacattgttt tcttacttca ttagtctgac ttcagctagc tagtaatctg taataggatg 169500
gaaatatcag aatctgtaga cagcatttgt caaattgtaa tacctactta ggccaaacca 169560
gagtttctga tgtactgcta agtgagcccc attggatacc acacactgag aacatcagtg 169620
atggggaaaa ttttgcatct agcaatggtg tgaagataga agaaaagttg agagtctctc 169680
aactggatca gtgttactca gaccctagga tgtgatagat ttgtaaaatt ttccaaaaat 169740
atttttcctg agaacttatt aagtaaaaac actctttaaa aatgtactac tttttatcag 169800

caatgtctta taaaataaaa gacattttaa tagccccaga tggaaaggtc ttagaatgga 169860
agactctgtt atagccttag tttttctaat tctgccatgg atcagagaac tttgggccca 169920
catttggaaa acaccaattt agatcatact tattttctat actaggtata tgtagttagt 169980
aatagtgatt taaataatat ctgtagagca cttcataggt ttcagcatct ttaatattat 170040
ctctctgttt ttataattac acttcgaaat cagtattagg tcttgcatct tacaattata 170100
ataactgaag ttcaaagaag ttttgtgact tcttgaagac tcagaaggga aacaaaggct 170160
ggtacccaga ccttgtaatt gtgaaggcta gtgctttaca ttttacttat ttcctgaggt 170220
gaattaatca aagcggtaat aatgaaaaca tttatttcat ctttgtcaag cactatttta 170280
aacattttgt gcatttttaa ctccccacag caacccaatg aattaaaaag ttttatctcc 170340
atttacaaat gaggcataga gcatttaggc agttcgccca cgatcataca gctagtgagc 170400
aactgcactg ggatttgttc gctggcagtt tggtgacaaa agaaaccccg tctcattagc 170460
tgggcgtggt ggcgcatgcc tgtaatccca gctactcagg aggctgaggc aggagaatcg 170520
cttgaacccg ggagtcggag gttgaggtga gccaagatca tgccattgta ctccagccca 170580
ggcaacaaga gtgaaacgcc gtctcaaaaa aaaaaaaaaa aaagtttgtg attttgaaca 170640
ctgtcttctg atcataggga gctgtagtgt tgtcaataat gttaagaaac gtctgagttt 170700
taaaaagcca ttggcatctt atgtgtattc cttttttaaa gttaaatctt tgtaccttaa 170760
gattcaaaat aaaaataaat tcaacagata tataataaac aatatgtgtc aggcaacatt 170820
ctaggtgaag gagaactatc aaagagtttc tattagtcca ttttcacact gctataaaga 170880
actacctgag gctgggtaat ttataaagaa aagaggttta attgactcac agttccctac 170940
atggctgagg aggcctcagg aaacttacaa tcatggcaga aggcaaaagg gaagcaaggc 171000
aagtcttaca tggcagcagg agagagagaa ggggaaaggc ctacacactt atcaaacaac 171060
cagatattgt aaggaaaaca gcaaggggga agtctgcccc catgattcag tcacctcccg 171120
ccaggtcact cccccaacat gtggagattt ataattcgag ttgagatttg ggtggggaca 171180
cagagccaaa ccatatcaga gttcatatat ggtttataat ttcagggatt attatggtat 171240
ataaggtgtc atctagacca gatatggaga gatctttatt atgttattgt tcaagaaaaa 171300
aaaaaatggt gtcccaaatt gcctcttttt ggcatgtaag gtagttattc tgataatcta 171360
acatgtatta aacacttact atttgttagg caatgtatta tatactttat atgcattatt 171420
tagtttgaat tttttaactg tgtaagagag attatttgat gaccagttta tacattttac 171480
agattcaggg atggtaactt tttagcttaa gagcacacag caaataattt gtccaaagcc 171540
aggatttgaa tttaagtcta tttgattcca aagatgatta tttttgaaac tgtataattt 171600
caaatttgat agtactagaa atgtaattat tttctatgaa aaaatcttac tggaatttat 171660
tttatagtca gtaaaaagtt ttcagagttc cagcatttaa aacaagtctc tccaaaaaca 171720
taagacttgc ttgtcagata ggtgaaacct aaaatttgtt ttatctgcct cttcttctta 171780
actttagtaa ggcttaggca ggctaatgag atgtgtgtgc tgccacaatc aggcttccac 171840

ccacttttta cccaagtcaa gaccttggtg cccacccacc caagagaaca gacacacctt 171900
actcatgata gctgcctgta agggggactg aaaaagaggg aagccggatg cagtggctca 171960
ggcttataat cccagcattt tgatagggca aggtgggagg attacttgag gccaggagtt 172020
caagaccagc ctgggcaaca tagcaagacc ccatctctac aaaaagtttt aaaaaaaatt 172080
agccagccat ggtggcatac gcctgtaagt cccagctact tgggaggctg aggcagaagg 172140
atcgcttgag cccaggaggt tgaggctaca atgagctatg atggcaccac tgtactcctg 172200
ggtggcagag taaggccctg tctcaaaaag aaaaaagggg gagacatcca cagttgctga 172260
cagaagggag ctctggccct gaaaaatgag gacataacat atagaggact ggcatggaag 172320
gagatacacc attttatcca gcatgcagat ctctgacaag gcatttattt ttgagggagg 172380
aaagagactg gaaatattgt ctcatatttt tctctcctag gaatttccta ttctgtgggt 172440
taaaagaagg aacagtgggg tacgtggaga aaatgatttc tccgtaaagg cctatctgaa 172500
agtatctgca tgttgaaaaa ttttatgtaa ttaaagtttt atttttaaga atatataaat 172560
attctttttt acttaacagg tgtgcaaaac ccattctgga attcttggaa agggtctagc 172620
tctttctcat tcaccaacta tattagaagc acttgaggga aatttaccac tccaaatcca 172680
aagcaatgaa cagtcttttc tggatgattt tattgcctgt gtcccaggat caagtggtgg 172740
aaggcttgca aggtaaatgt actttaagtt acttacttta ttttagggct ttcatctttc 172800
ttcatttctg aaagagggga gtcattagaa tattaaaata ctactttaaa aaagtctggt 172860
ttatgtatgt ttaacttgct gaaatataaa atgatagtta atatttttgt ttggcggttg 172920
tttgtttgtt tgtttttgag agagagggtc tcactgtgtt gcccaggctg gagtgcagtg 172980
gcatgatctc agctcactgc aacctccact tcctggactc aagcgattct tgtgcctcag 173040
cctcccaggt agctgggatt acagatatgc caccacaccc ggctattttt cttttttgta 173100
ttttttgtag aaacagagtt tccccatgtt gcccaggctg gaatagttat gatagtatat 173160
gataagacca ggtgcaatgg ctcatgcctg taatcccagc actttaggag gctgaggtaa 173220
gtggattgct agagcatagg agttgaagac aagtctggcc aacatggtga aatcctgtgt 173280
ctacaaaaaa tacaaaaatt agccaggcat ggtggcacac tcctgtagtc ccagctactt 173340
ggaggctgag gtggaaggat tgctcaaacc ctggaggcag aggttgcttt gattgtgcca 173400
gtgcaatcca tggtgaaacc ctgtctctgc aaaaaataca aaaattagcc aagcatggtg 173460
gcacacacct gtagtcccag cttcttggag gctgaggtgg gaggattgct caaaccctag 173520
aggcggaggt tgctgtgatt gtgccactgc aaccagcttg ggcaacagag caagacccta 173580
tctcaaaaaa aaattaacta attaaattaa atttattata gttgttaatt gattattttt 173640
gttgcttttt gtttttagga tgaaaaggga aaaaaatatg ctggcctgca tgattttact 173700
agtaaaaaaa aaatcccaac ttttttctgg ttttaatttt aaaattgcag agattactct 173760
ttagttttta aaaatcacaa aatagtgatt ggagaggaat gtcaaatcac ttaagtcagc 173820
ctcactacta tactatgcag acgtttttat gtaatgcatg gtagaattac ttattactca 173880

gctcacagga caacttgaat gtgtagatca cagaatgaat tcaggaatga acatcacaaa 173940
ttacacaaca ttgatgcatt ttaggtggaa agcaaggtaa tgaggaagat agttgagaaa 174000
ctgtgaaaaa aagatgggca gtacaccttg aacctggctg ctgtcaggca agtagagggc 174060
catttctacc cctgctttta cagagacact gctttttcca ggcttgcagg atggcagagt 174120
aaggtggagc tccttcagag agaatgagcc tgctcaggac ttcaaattat gcacaaccta 174180
cattcgtgtt cttggtgatt ttaaattagc catagttaaa atttcaagtt tcctggtttt 174240
aaaaagtatt tttatactgt atttaaatat cagaaatctc ctgtgcttag gcatgttttt 174300
taaatgaatt tgtccttaac agtttctgag ggagacactc atgattatca ctgagttgga 174360
aatgaagata atgttttagc attttgtttg atacaagttt tgttcatttg gcttaatttt 174420
atattaattg ttgaagatgc tgatcagtgt ttggggtttt gtttatttgt ttttcattac 174480
tgttaggtgg cttcagccag attcatatgc ggatcctcag aaaacatctt tgatcctgaa 174540
taaggatgat attcgttgtg gttggcctac caccataact gttcaaacaa aagaccagta 174600
tggggatgtg gtacatgttc ccaatatgaa ggtaattata actggattaa attagcagac 174660
atctatatac tggctgcaat gactgataaa attttagaaa tgccaagtgc tgagagtcca 174720
tttgttctac cctctttata taaagggtga tgctgaaagt ttgtttaaat gacttgttta 174780
tattaattag tccccaagtg tccaagttac acctgttttt tttgtgagtt tgttctttac 174840
attttgctac ctgttacggg gactcaaagg agggataaga aagtatccat ctaaagagtg 174900
ctagacacat acagtgaagc ccctcaatat gtattgattg aataaatgca tgaaagaata 174960
catttttaaa ttttgtgtat agttttgaaa gactcaagta cgttctgtgt ttggtattac 175020
tgaaaccaca ttttaaaaat aacactcatt aagttagaaa tatatgagtt tagattgtaa 175080
aagaatgagg aattgaaata gttgtatacc atattgatga atatagagtt tttaggatac 175140
ctcttacctg aaatattaat aataatgttt cagagcatat tatacataat tatttgtgat 175200
ttaatctgtt aatatgaata tctcatttaa aacttttatt tctgaaaaaa ttatattgaa 175260
taaaatttta tataggcagt ccccagccct ttcctccttc aaagttgtct tatagagtga 175320
ttggttgttt gaagcttaga gctgattaag ccacaaaatc ctttttgctc attgggtagc 175380
taattttgcc tcaatattat tttcagtaga actatttcaa ctacttcatg cattctttca 175440
tttatagcgt gaagaagtac aatgccaatg tagcatctta cctaagtaaa aaataagaac 175500
acattgctta catgacttaa gaccagtaaa taaaactgta tggaaaaggt ttcaaccctg 175560
atactaaatc aaaatcagtc ttttagcagt atctttcaaa tatttggcca tgatccatag 175620
tcagaatttt tattataacc caaagatata aatataatat atgatggaat agtgtctatg 175680
taatagcata tgttaagtac atataatata ctttatatat acatacacac atacatacat 175740
aatatgctat actctgatgc tttctctatc ttatttttta aggcaagtca tacccttcac 175800
aaactgagtt catgcctact tggaattatt ctatacaatg caaaaagcat cacaaattat 175860
aagaaaaaaa gatttaaaaa taaagttatc ctgatagtga tatgaaaaaa attactactc 175920

aaggacagat ttttgatcac agtacaatac acgttaagat gttgaagggc ttgtatattg 175980
tgtattcact gatgaatttc tcaatgtagt gagttgttaa atccaatctt tgaaccctca 176040
agattaagtt ttttattcat gtgacaacat tctaatacca tgttcatact gttacgcttc 176100
tgatatttct ttagttgtat tttctttctt gtgttttctc ctttgcacct tttattatct 176160
cttttgatat ctggtatgtt tatatcttgc atttgaaagt cagacttcat ctttttttct 176220
tttcatctta gagttctttt tttaaccata tgatggatct gctttttttt ttcctctctt 176280
cttccctctc ctctttccct ttcacctcct tctccttttt ccttcttttc ttcctcttct 176340
ttctcctcca tatatttgct tctcctacta tctttccttg acttaaacct cttactcagt 176400
gtgctctaaa ttcatgtcag atttatccaa tttagaaact ttctagaaaa ccactctaaa 176460
tactatctag aagaagtgat ttgtgctcct actgatgttt aatgtattaa tatcatatta 176520
tattagctgg aaacaaaatt aatttaacct attttacttt cagcctatta gtatatgaag 176580
tcttaggagc actttagact gttcataaat tgttaaaatc tttatgcagt aatagctaat 176640
tatttgaagc ataaaagttg atttgcagct aatatttcaa tgagttccaa aatgctttca 176700
aaagcaacaa gtgttattgt tataattttt tacttataaa atgactgttc cctacctgtg 176760
ataaaatgag ttagaactaa acacacatat tgtaccaatg tcaatttcct ggttttggtt 176820
cagtaccatc agtgtctgac agtgttggat aaaatgtagc cattggggga acctgagtga 176880
agggtacata ggacatttca tctatttttg aaacttcctg tgaattaaaa aaaaaagttt 176940
attttaagga aatcttattt attgacaaga gaaagtcaca taggaaatac aagaataact 177000
ttttttttaa gtaacacagt ttgtaaaaga aagcagaatg gttattctat aatgtctcag 177060
ttttcttaaa gtataaaatt attcagtgat ttacttcaag gttcatggtg catacacaca 177120
tagactctaa tgataattta ttaaatactt agggtaaata tagttgctta cagaaaggtt 177180
aaattgaaat agtatgtata aaaatagtgt ctgtaattta atatcaaata tgtttgagac 177240
aggagagttc ccttgacccc tttgtgggac ttgggacgtg ggtgtcgtgt ggctcattta 177300
cttgactgct gcactcaaac cccttgcagg atggcgagca ctgaggcaag caggtgccag 177360
ggcccaggca agcacctctg ggctccagcc ccacagcagc atctaggagt gtgtcacaat 177420
taatgctttt ttagctttgt tgtctgtgga tggctaagtg ttaaacagct cagtgaagaa 177480
tcagcatgac agcctttttg ggttcctgca cccagtgtgt cctgaattct tgtctggcat 177540
tcaggaagaa tcaggtcata tgaatggttt gaaacgcgat gaatgcgtga atacagcaga 177600
tttttttttt tttttttttt ttttgagaca gagtctcgct ctgtcgccca ggctggagta 177660
cagtggcacg atttcagctc actgcaagct ccgcttcctg ggttcactcc attctcctgc 177720
ctcagcctcc cgagtagctg agactacagg cacccgccac cacgcctggc taattttttg 177780
tatttttagt agagacgggg tttcaccgtg ttagccagga tggtctcgat ctcctgacct 177840
tgtgattcgc ccgcctcaga ctccaaaagt gctgggatta caggcgtgag ccaccgcgcc 177900
cagccacggc ggattttatc aaacggtgga agtggctctc agcagaaggg gagctagaaa 177960

gggcatggtg tgagaagaag gtgatatttc cctgaagccc agccgtctcc agccatgctc 178020
ctctcctaag tggtgccatc tgaggttaag ccgcgcgtct catcagttgc tgcttctcct 178080
cttggtattc gccacttgtc tctttgctag ctgaagtctg ggatttatag gggcataggg 178140
taggggggca gggctggcca aaaaggcaac atttaggcgg aatacaagga taaactgttc 178200
tcatttaggg tcatggtttc caggcttgag agtggggcct ttgccaggga accaccttct 178260
cctatccagt atatccctga ctcctgtctg tatcatcttc agatttttta attgtttaaa 178320
gtttaaagct ttttatgctg gcacatgtgt ttgagttgaa attttaacca tatacctgta 178380
aactgccatt ttttaaatat aagaatgggt ttttaatatt tccaggggtt tgtgtcagtc 178440
caaatactga tctcttgtaa cctcacctga agatagtggt cacttaatgg aacatatttg 178500
tcacctgtgc ctaacattat ataaatagcg aaagtctcaa actactgatt ccatagaatc 178560
gcgttgttaa gaacaataaa cataaagaca gtctgtagtt ctgaggtagc tctcaattac 178620
ttaaaaatac tatcactttt atctctgcac tttttctatt tctgtgacaa ttctgttgaa 178680
ttacctgttc ttgccaacca caaaactatt gtctgtaggg agcagcagaa actttgagta 178740
aagctcagaa tggtgatacc tgctgaagaa ctaaagtggt attcaaattg tgtgtacatt 178800
tgtggttgcc taggggtcaa gtgtttttct gccaatgcat aaacaaataa aaacaatgtt 178860
ttcttataat ttctgggaaa tatttctaaa aagtattttt cagaaatagt agttagtaga 178920
tgaaatagta gagttagtag atcaaagata gtcccgtttt gttgaagaaa ttgagttgca 178980
aagttataat tcctggaaaa tatatctaaa aggtattttt ataagacaat ttgtattttc 179040
agtatattaa catccattaa cgaatctgtt ttttgcatct atactgtaga gttagtggac 179100
gaaagatagt cccattttat tcttaaagaa agtgagttgc aaagtttaat tatttaatag 179160
attgcttaaa tcaaatgttg atgttggtga tagggctgtc taataatatt ctttcttcac 179220
ccgagtaaag aaacatattt gggctttagg tataagattt gttttattaa gtgttaattt 179280
ttgaaacttc tataaataga gtacattttc ttctgtagaa cagtgcagct caaactgaca 179340
ttagtgcttt aaaatgtgct gagattgtgg gaaagcattt acacttgcat tacctgataa 179400
aataatttgt tagtccctgc aataacttaa tgtagattca ttccactatc ttttcctgta 179460
ctttattagt ggtgcatttg tttttatgtt ttgatatctc tgattatgta ttccaaaact 179520
atgaggcctt gtttttacac cgtgttcatg gttcatattg tcaacccatc ttgaggtcat 179580
ttgaactctt ttcaataagc ctttgaaggg aatgccagag acagaccaat gctaacataa 179640
agtatacata attatgagtt tataatagta agttgcttat tttgatgtca gtgacaatta 179700
aataataatg gtatttggtt tctataatac catgtttagg tggaagtgaa agctgtccct 179760
gtttctcaga aaaaaatgtc tttacaacaa gatcaagcaa agaaacctca aaggattcct 179820
ggcagtcctg cagtaacagc tgcatcttct aatactgaca tgacttatgg agggctggca 179880
tcaccaaagc tagatgtttc atatgaacca atgatagtga aggaagctcg atatattgcc 179940
ataacaatga tgaaggtaat ttattttact tatcagaaaa attttagttt tcagcatttg 180000

tttatttaac caacatttca gtgattcaga tgcagacatg aaattgtaag tccaaagagc 180060
atttgatgga tattaaattt gcagcaacac ttattaagat gatgttatcc tgtcttcaga 180120
gaggattttc attggttttt ggcagatgtc cagaggggat caccaattac agatcaagtt 180180
aatcgaatca gagatacaga tggattaggc tgagtttcag ttcctgtgaa agtacttgtc 180240
tacttctgac caggaacagt ggcccacacc tataatccca gggctttggg aggccaagga 180300
gggaacatca cttgaggcca agagttcgag accagcctca gtaatgtagt gagaccccat 180360
ctctacaaaa aaataaaaat aaaataaaat ttctaagtac ttgtctattt gcagtttact 180420
attcttgcta gaatgtatct cttcagggtt ttggggttta cctatgcccc cttcaatttt 180480
gggttctctc aaatgccaga tgtatctcct agaactcttt gggattttta gctctctaat 180540
acctttagac atttaaaaaa tatatatttt ggatgtttta gttatcttca gaggcaatgt 180600
taatccgaat tatcaaggta gtcattattt gaagctgaag tttgtaattt tggcattttc 180660
agagagcagt ggtgagccat tgaaggcttt tgggtagagg aatggcttga ttagatttgt 180720
gttttgtaac gattgtggtg gaatatggac agtgtaattg ggcagatgat caagaaacat 180780
gacttggttt tagtctcttt aggggactat tgacttaatt tggtgagata tgctgatcag 180840
ttggactagt gagttgtcaa tgaggataga ttcaatagaa tcagtagaac ctggtagcta 180900
gtggaggtgg agtaaagaag aaaggtagga aataaaagat gaaattcaca tattgagctt 180960
gtgtccaatt gataatggaa gaggggcagg ttttggttga gaaagatagc agattcagtt 181020
ttggatgagg tgagttttag atacttctga aatacctacg tgaagatgta taaaacaaag 181080
ttagatgtac agacctgagt gatcacagtc tgacctcaag aaaaggattc aggaatcatt 181140
agcacttaca ttacttatga agtcatggga atagatgata ccttctatgg tgaatgaaga 181200
cagaagttcc aggataggat cctaagaaac accaatatca ctgagtctca gactcttctc 181260
tacatcagaa gcatctggag cacttactta aaaaaaaaaa aagatttcca agcacccaga 181320
gatttagatt cagtgggttt ggagagaggt tcaggaattt acctgaaaca aatatgcagt 181380
gattcaaatt caaaacctaa actttgagaa atactgattt acaaggtaga taagagaaaa 181440
agaaaggaaa aggaaaaacg attcacattg gtaagaatac tagccaaaga agcaacacat 181500
aattaaggga atggctgaag tgagtgtaga taagcctttc aagatgtttg actatagaaa 181560
gaaccagagg aaatggaatt gagtgaaggc tttggagtat ggggtcttat taatagagat 181620
agagttacat atttgagtgc tgattaggag gtaattgaga gaaaaaacaa taaagatact 181680
ccgaatataa aaatagaatc tagagctcaa gtggtagcat tggccttaaa taggaggagg 181740
agtagtgctt gtttcattct accagaagaa ggaaaagaaa gcttaataca gatgctagta 181800
aatttgtagg ttttgcagcc aaaggaatta tttaaaacag taattatgtg ttttgggatg 181860
gaaatggaag tgatgaatct tagggtcaag gttttgaaac aaagcggata aaatgaaatt 181920
gttattctag agattaaaag aaagcaccga tcagaaaaga tttattagat gcctttgaat 181980
aatcagttga aattgaagat tatttgtgaa taacagtgtg agtcatatga gcatggataa 182040

aacaatcatt tggatggatc tagcattaaa attttgctag accagcaaaa gaacagtgga 182100
acaaggaagt taagtatatg gtagctaaag agaggacagt ggaacctaat ttagtttgga 182160
agggaagaaa tttcagagtc aaggaagtaa gagggactag acgtcaaagt agatataaga 182220
atagttattg aggtaaaaaa ctgaacaggc cattttattt tgatttcaga gacagaacaa 182280
ttctgagtaa tgtcacaggt ctgtgacttt tggtgtgagt ggctggaaaa aaagttgaag 182340
ttattgaggt taaagaaatc agggaatgag aaactagagt tctaggtgaa ttgtccactg 182400
gctgttaaag aattcctgga aaaaaagttg aagttattga ggttaaagaa atcagggaat 182460
gagaaactag agttctaggt gaattgtcca ctggctgtta aagaattctc ttctctcact 182520
ttttactcac caaattaata atctcattat gaattctata tccgaaataa atatttgtct 182580
cattttttaa aaaatccatc ttacacattt ggctatttta gtatcaaatt ttaaaattat 182640
ttctactatt aaaaaaaaaa gcaaggctaa aaattcttct ctattccaac acttgcttaa 182700
ggaatttagt ttctagtctc cttacctccc taatctttta gtttcctatg aacatgcttc 182760
agtttgtata atttcttttg caaattttgt taccacaacg gaatgcaata tttcaaacat 182820
gttctgagta gcctaggttt agagcaggta tggaatacca ttcccaaaca gtatctgtga 182880
aatgttaatt caggtcccag gaaaggaaat tttccttatc aaccattcac agatactatg 182940
tattcactca gtagtgagcc aagtattgta gaggatggaa agatgaaata tggtctcttc 183000
tttcattact aagtgtgtta ataatatgaa tttgagggag agtgatgaaa tagaaaatac 183060
attgtactga aagtctagaa acatggattt ccttttctca cctttgtatg gctaaagact 183120
cagccttaac ttgcgttttt ctcatgtgta acttttgagt aggtaaagtc agctagatca 183180
tttctaattc atttttatga aaacaaaaag ggtagagaga aggaaggggg ctttgagttt 183240
gcttcctttt actgtaagta caggaggagt tagaagtaga tgagcagttg gacatcttga 183300
cctgaacctt gaaagataat gatttctttg ctaatgttgc aggcatacag aagtattcag 183360
aataatgtgc caagtttcat gtacctacag cccatttttg tctttacatt ttaccataat 183420
tgtttcaggt cattttgtta tttttttatt aagaaacaga ttgctacagg tacaattgaa 183480
gcctgcctaa acccttcctc agacccatta ccaccccttc cttagaggta accactgccc 183540
tgagtgtagt gattctcatt cctgtatata tttttaggct cttactgaat acaaatttat 183600
tcataagcag tatattttgg gttgttcaat acgttttcca gaagtttata aaataatatc 183660
gtattgtatg tatcattttg ccagattttt gcttaatgtt gtttttgagg ttcagccgtg 183720
tagctatagg tcattcattt taactgcttt aaagcattcc attggggatc tattccataa 183780
tctgtccatt ctcccattga tggacgctta ttataaactg tactgaaata aacattctgt 183840
atttgtctcc ttttgtacat gagcaagagt ttctgtaaga tacatactta gaagtggaat 183900
tgttaagttg tagggcatgc acaactacaa ctacattaag tattgccaaa actcacactc 183960
ccacgagcaa tgtatgaaag tagcaggtgc tgcatgttct cctctgcgct tggttcttac 184020
caacacaatt tgatgaccat gatgtgattt attttagttt tgttttaatt cgtattttcc 184080

tccaagttta gttaaataac ttttctagtc tttattgacc tttcagattt cttcttcaga 184140
gaattgtctt gactgtcttt ttttgttgtt gtttatctct tttgtatgga tttattctct 184200
atatgttatg gatgcatatc acctttgggt tatacgagtt acaaatctcc ttctccaagg 184260
tggaagcttt tcctttctgt atagtatttg ttttggaaaa ctttaaaatt ttaatgtact 184320
caaaagatgt tatctgtttt tttcttttgc tttttttttt taagttttgg tgtttacttt 184380
tggatcttca aaccttgtag aatttatctt taataaatgg tctaaagtag gtaatttttt 184440
ttctttttct cctatatgta taaccagttg tctcacaacc atttattgaa taatatatta 184500
ctctgttaat tgagaatgtt tttaaatgat tttacgattg ttcagactgg tctctttctt 184560
aaaggtttat gaaaattatt catttgaaga actacgtttt gcatcaccaa ctcctaagag 184620
gtaaggatcg ttttctcaaa gtataaccaa aacatgtgtt gtcagctgct tgcaactaaa 184680
atcatcctgc atttcaactt tattttctat aaataaatac tttatagcat ttttctaggt 184740
tgccgataga gatgtcatat aataatgtat agatttgcag aaagaatgtg tttacattca 184800
actgtaatac tgccaatata ttacataata ctacctacta gtgaaaatag gtatgtaaag 184860
tagatatcta gtatgtaaaa atgtttaaat tgacttgttt agggttgatt agtttgtttt 184920
ttgtttgttt gctatttaaa aattgttcct tttctccttc caatgaattg tggagagatt 184980
tcagcatgtg gtatacttgt atttgattgt actgtcttaa tttttcagcc cgtaagtaat 185040
tctgttaact tcagatttaa ggactttttt tgagttaata ttttatttgt taggaacagt 185100
actacttaaa atagaaactt ggaaaaaata attagatgca ttatactttg gagagatcac 185160
ctccatagaa tttagaaaaa tctctgaagt gaatctgaga tagcagaatt atgtataggc 185220
tggaaatacc agtttgaaac tcatggggaa aataaatttg ggaatcatct atattgagat 185280
ggcatttaaa actgaggaat ggttgtcacc tagatagatg gcataggtag aaaaaagatg 185340
acccagaccc aaatcctggg gcattcctta aattaaacgg tagtcagaaa agggaaagca 185400
acaaaacact gacaaagaat ggccagtgag ataagaaaaa agccaggaaa atgtcacaga 185460
agcaaaaaga aggaagtatt tcaagatagg aagagttgtc agctatcaga tgctactgag 185520
agattgctga ggatgaagtg accattgaac ttggggacgt ggtggtcatt ggtgactttg 185580
acatcagcag ttgtaaggaa atgttggaaa caaaagccta ataaggataa attgtggaaa 185640
ggaagtagaa gtgagagaat ggagtttgat ataaatcgga gaagagatgt aagaccttat 185700
tagagggggt tgtaatacca aagggaggtg ttagtgaaga ttctaaagtt tgtgtgctaa 185760
tcgaaatgat tcactaaatt gagagtaatt gatgatgcat gagagagaac aaggtgattg 185820
cagcataaaa atgttgaggg aataagattc acagcacaag gggagggact agctttggat 185880
cagatccagg acacttcctc cattctacca tgagagaagg caggggaaag taggtttatt 185940
aatcacattg caactttgag gttgtatctg attgcttatt gtctcacaga agaaagaggt 186000
gaggtcatga tcacctgaga gttaggaaga gaggtgatgc aggtttgagg taagaaagaa 186060
gtgaaattca agcattttgg agagtaggca agcgtactat ggcagggtgg taggattgcc 186120

aggaggtatg caaacaaatt aaatgtttgc tgaactatcg taatgatgtt gactgcttaa 186180
ccaaaagtaa aatttccaat atccatctta tttcttttca gacccagtga gaatatgctg 186240
atccgtgtca ataatgatgg gacttattgt gcaaattgga ctccaggggc tattggactc 186300
tacactcttc atgttaccat tgatggcatt gaaatcggta ttttctttaa agccatgagc 186360
tactacttac ttcaaataac tagagtcaaa attattttta tcttctagtt ttgcccatta 186420
acttgcacta gacatatgga taggtttcat aacttatttg aggccttgtt ttctctttca 186480
taatgagtat ttaattagtg cattaacatt ttgtaataat ttctggtgtc cgcccaggca 186540
ctgtagccct catgactctc tccctccatt tcctgatgtt actagtcagg tgtgctctct 186600
tccacgctgc tgttaccctg tctctcccct gtgggtgtgt ctgtgcctct cctgactgtg 186660
agcagaaagc attcatgttt ggatcttcca gtgtagctca gtgcctggca cataatgaca 186720
gcaaatatgg atgaatgaat gcatttttaa atcgatcatt gtgttaatct gggttctcca 186780
gagagacaga accaatagga tatctataca gtcatgtacc tcatagcatt ttggtcagtg 186840
acgaacagcg tatataatgg tggtcctata agattttcct gtaccttttc tatgcttaga 186900
tatgtttaga tatgcaaatc cttaccattg tgttacagtt acctgcagta ttcagtaggg 186960
taacatgctg tacaggattg tagcctaaga ccaataggct gtaccatata acccaggtaa 187020
tctgtaccat ctaggtttgc ttaagtccac tctgatgttt gcacaatgat gaaattgcct 187080
ggtgattcat ttctcagaac atttccccat ccttaagcaa tgcatgtcta tattagatat 187140
atgagaggat ttattaggga aattggctcg cacaattatg gagtctgaaa agtcccatta 187200
tgggccacac ctgcaagctg gagatcctga gatgcaggta gcctcagaac cagggaagca 187260
gatgatgtaa ctctcagtct gaggccaaag gtctgagaat ccaggagggc cactaataat 187320
aagtcctgga gtctaaatgc tggggaccct ggagttcaaa tgtcaaggga cagtagagga 187380
agtgtttatc caagctccaa aaaatagaaa tacattcacc tctcctctgt ttttgtttgc 187440
tctgtgtccc cagctgattg gacagcgcct gtccacattg aaagcagatc ttctccacct 187500
actccactca cactcacacg ccagtcttgt ctggaaacac cctcacagac gcaaaagtaa 187560
tgccttacca ggtttctagg tattctttaa tccagtcaag tggacaccta gaagtaatca 187620
tcacaaccat taactttgaa aaacatattt cttttgtttt aggcattaat cttttcttta 187680
cttttaaact gtctgtataa taggataaca aggagaaaac tctagtactt atcagtgtga 187740
atttcttgta acattatttt tcctgatgct cctcaaggaa aggggtttct gtgatcagat 187800
aaatttcgaa aacctggttt gggatgagta tcttttatct gaaatgcttg gcaccagaag 187860
tattctagat tttggacttt ttctgatttt ggaatagttg tattacagtt accagttgag 187920
catccctaat tcaaaatttc aaaattcaga atgctccagt gagcactttt tttgagcatc 187980
atgtcagcac ccaaaggttt tagattttag atcattttgg atttggggtt ttcagattag 188040
agatgctcaa cttgtatatg gtagtctcac ttgagacatt cacacaatct acattaacat 188100
attagggatt ctgagaagtt ctgaaatcaa gaaacttgtc tgactatgtt taatccttat 188160

cgtgggaccc cttgcaagtg gagaagagag aaacagggtg gagatagtaa taataccaat 188220
taatatttca ccaaaatatt tgggaaaccc tgctttaagt agtttgcttt ggcagtggag 188280
gtaaagaggt actttacatt gatgtaattg tatgttctca tacattaatg taactatata 188340
gactcagata tatccagttc atctgtacca ttctagagac cttcaccagc agcctttgct 188400
gcactcttct ttatttcaat atattcataa agcctttagt tcagcaaatg ttttttggtt 188460
ctgagcatac attttcctgg gtaagacaat tcctgtcaag tgttaacact ttaatgagga 188520
ttgtgaaaat aaaacacgca aacaaaaatc ttatgaagta gaatataagt agtgacacac 188580
aagacataca aattacagta ggaattcgaa agtagaaata gccaagaatg acattgtgaa 188640
aattttcttt tctctaaaaa ttctggtaaa atttcagtag atagagtttt tttcatcatc 188700
ttatctaata gtggggatga agaaaactga tacaaattag ataagaaaaa tccacttata 188760
tttgtttttt ggaatgaagc tgaacaaaaa tacttgctta cttagaatca tccattttct 188820
tccatactca acttccctgc tcttcttcct aagttttctc cgatgccatc aatgggctgc 188880
gaccttgctt gaccatagac aagcagagat aataattcac tagaatagtt ataataataa 188940
tagctagcat ttattgtgca tttaatatat atctaaattt tctctatgta tacttttttt 189000
tttttttttt tgagacagag tctcactctg ttgcacaggc tggaatgcag tggcgcgatc 189060
cctgcccact gcaacctcca cctcacaggt tcaagcaatt ctcatgcttc agcttcctga 189120
gtacctagaa ttacaggtgc gcgccaccag gcccggctaa tttttgtatt tttagtagag 189180
atggggtttc actatggtgg ccaggctgat cttgaactcc cgacctcagg tgatccgcct 189240
gccccggcct cccaaagtgc tgggattaca attgtgaacc actgcgcctg gccccttgta 189300
cattttacgt catttatttc ctacaacagc tcggtgagtt agatactgtt atttttccca 189360
ttttatagat gaagaaattg aggcacagag agtcaaacag ttagtgcgag atattggagt 189420
aaccaaagca gtctaacatg agaacctcag cttctcacaa ccatgctaca ttgtcataga 189480
gaggactggt agagatgatg agtggtattc tgattggata ttgtcagata agatttaatt 189540
ctcaagggtt ctttttttta ttattttcca ttttttctgt atcatttcat atgttctagt 189600
taaagatagg gagccccttc ttttccttga catcctgaga cattccttta gaatctcagg 189660
gccattttta aaatatttct cttgtttagc ctattaaata tccatcacct taaaaggaca 189720
gaaatcagat atttgtgtta atttttaaaa atagcctgtg gatgtttata agattaggtt 189780
taaggatctt ccttctcttt ttttctcaaa tgttacagtt ctctgatttt tctgacagat 189840
gctggtctgg aagtaaaagt aaaagaccca ccaaaaggga tgataccacc aggaactcag 189900
ttggtcaaac caaagtctga acctcagcct aataaggtta gggcagaatg aattgagagc 189960
cggaatcatt caatttttac gtttgtcact gatgcttcaa aatggccagt aaacatctaa 190020
cattttactt gatttcagga cttgaattct aaatatccct aaataaattt agagagtagt 190080
tcatggtaat atttctaagt tgtagcatgt gtacttcttt atttggtaca tattgtattc 190140
ttcctgctgt ggaacattta caatgaacaa aagtctactg aaattaagtg taaagtttgc 190200

aaggtgaaaa tgtcctctac tgtcaaaaga aaagttaatt tctgaaatct aaagagtaag 190260
tacaggcatt ttggtaaact gcatgttaaa aaaatgaaat attggtaatt gaatttattt 190320
ttctaacaga aagaaatggt ttttatcctt cttaacacgt atcttcagta tttagaaatg 190380
agttaaaata tatataattg tctaagcttt attttcagta atacacattt cagtgcttaa 190440
aagttctgtt gttagtgttg ttatactgtt atttgcagag ggagcctctc tacaaataat 190500
tctggaacct tattaagaat tacgatactt tcaaacctgc ttaattttac ttcttgctac 190560
ttgtaagatc tctattctat gttcttccta cctcactgtt tatctttgcc tttctcaggt 190620
tcgaaaattt gtggccaagg acagtgcggg gcttcgcatc cgtagccacc cttcccttca 190680
gagtgagcag ataggcatag tgaaagtcaa tggaactatc acttttattg atgaggtaat 190740
tgataaagga gcttttggta gtaaattttg gacagaaagt ttttgcattg tttgcataat 190800
aataaggtta ctgagttttt tgtcagaata tgttttaagc agtttgggaa atattttaaa 190860
actattttat agaatgtgtt cattgagttt caaacatttc atttatttta tcattaaaaa 190920
atgactaaaa ttatattttg aacatgaaaa gaacaatgtt cgacatagta ttcagaacct 190980
gtacatgtat tcactgaaaa tgggtatagt tcttcatgac attttaaaat gttgtttgtt 191040
ttgaaatgtg tttcttactg ctttaacttt ggacagtata tttttctggt aatataacca 191100
agtaacttct gtttcttaaa atataaatat aagggaagga agactcatat tcctttggga 191160
aacatctgat tttactgttt atgtatctta aagtgtggtt tccttgtgtt tttattccta 191220
agtgaaattg tttttacagt cttgccacat gaattaccat attgtgttgg cactctttgc 191280
tgcttttttt ttccctggag ggtctctcag acttactttc tgcaaaaact agatgcttaa 191340
tctgcacatg caccatcatt tttctgcttt gcagactttc agaccttgct agcctcatag 191400
gacttacagg aaggcctggc ttgtttctgt tgcttttttc tatagatcca taatgatgat 191460
ggtgtgtggc tgaggctgaa tgatgagaca ataaagaagt atgtccctaa catgaatggt 191520
tacactgaag cctggtgcct ctcttttaat caacatcttg gcaagagtct tctggtccct 191580
gttgacgtaa gtaaaaggtg gttttaaaaa gcattataga tacacgctgt ttgttttaca 191640
gagctttctt taattaaggc ttttcagttc tgaggtaacc cacaactaag taacctttac 191700
tttacaattt cttgaattct taagggcttc tgtgtaatga aagtgaagtt tggcaaggtt 191760
aaggccctta gtgcagatta tgatccatca cagataggca ggaagtagta aatttaatta 191820
attgctactt aattaaaact actgaattcc tttcaaagag gtagaaaatg agagtggtta 191880
catttttttc tttcttattt atacagctgt gtggaatgaa ttccaaacta aagtttatga 191940
actcagaatt atacattcaa aaacatgtat aaatgtatgt tatgttcaca ttttgcagat 192000
agttctgtgt gttctgtcta attttgactc tcttccttct cggcttataa tactattatt 192060
tagatttgtg gtgctccgtt gtattctagt tgaagttaaa tcctagtctt tgagagaatt 192120
cattgtacct ccttgttgag tatagtagaa tagagccagt gttcacaaga ttgctacttc 192180
atgaagtata ataataaccc acacttcgtt ttatatatta accaggaaag aatgattttt 192240

ccttttttca ttaccatcaa atgtgtgtgc tctttaggat agagctaata aatagcacgg 192300
gtaaaatttt atttaaaaac ttagtatttt aaacagttga atttcttaaa aatccgttta 192360
taaagttagc ttattgtatc aaaccattca gtatattcca tttgactttc tccttgattg 192420
tccataataa tgttctccaa agcagtagtt tatataatac aagacaattc tttggtgaag 192480
aaaacattgg aatttctatt tctatttatt gatctcattc ttttttatat gcttttgtgt 192540
atgttttata atagcacagt ggagactgta tatagacaga gagcaagaaa gagaaggaag 192600
atatgtgatc aagaagattt agagtgttgg cttactgctt tacggaggtt aagttgaaca 192660
atgatacatc tataagtaaa atactaaatt ttaaattaaa aaattacatc aattacgtct 192720
tattaaattt tcctcagatt tatacctgca gttaaaaata aaaaggattt tgaccctcta 192780
tgaaagtgta ttttaaagag ataattatag caaccacttt tgtacagttt ttttttccaa 192840
tatagacttt tttgttgggc aagggggagt tggtttttgt tttttataca gatgggagag 192900
ccaggcgagg tggctcacgc ctgtaatccc agcacttttg gaagccaagg ccagtggatc 192960
acctgaggtc aggagaccag cctggccaac atggtgaaac cccgtctcta ctaaaaatgc 193020
aaaaaattag ccaggtgtgg tggcgggcac ctgtaatcct agctactctg gaggctgaag 193080
cgggagaatc gctggaactc aggaggtgga ggttgcagtg attcaagatc acgccactgc 193140
actccagcct gggagacaga gcgaatctct gtctcaaaaa aaacacaaaa atatagagat 193200
ggggtctcac tatgttgccc aggctggtct caaactcctg agctcaagcg atcatcttac 193260
cttggcctca caaagtgctc atattacagg catgagctac cacacctggc cattgtgtgt 193320
tttatttaag aaaattttct taagtcccta ataaaatgtt attccagatt atattatctt 193380
ttctaccttc ctagtccaaa ttttctctga tttctttgct catttgagga gctgcttttg 193440
ggagagaaag atactcacac agataaagca agtcaataaa caaaagtaga aaggaagagc 193500
aagaagtacc atctgtcaca attctgaatc ccacgatttt attttaaagc attgtgttct 193560
gttacttgat gcttttgaac cccatccaag agacaatctg tagtagacag tctccttaag 193620
gtcagggaaa gaagggagta acaaaggtta gaggagcggt aaggagtgga gtgaggcata 193680
cagatacttg tgggtggttt tcactggaat gtgagtgcgg aggttgttaa tgtaaacagc 193740
tcaagagtgt ctggtattgt taggaagcta gggttttttt ggtttcttca ttttaagagt 193800
agtagtgact tgagagtttt ataagttaaa agaaagatac tagtagaaaa agggagaaat 193860
taaagatata gatgacaaga ctgaagactg agaaacagga ggaggaaatg gaattaaggg 193920
caccttagag agcttgcttc tgtaaaagag aggggaatat gcatggcagt agttatgttt 193980
tagaggaggg agggacgaag ggaagaggtt gagggagact acaagttccc ttaaaataga 194040
agataggaaa acgatcagtt cagaatcaaa agaatgagtt aagttagggg gttgagaaat 194100
gcaatgaata tttgaaatat tgtttgaagg attactgggc cactaaagac tcaatgagac 194160
cactgtatag acccaactgg aatcccctgc ctagtccttt attgccctaa gtgtctggaa 194220
ttaaaagagc caagaaagag ccaagaaagc aaattatagt actagttgct gagctgctgt 194280

gacagaaata gcaattaaga ttatcagttc tgggaatgtg gactcagacc taagttagaa 194340
atgtgaaagg acaaatggaa ggtccaaggg atttagaaga tttaataaaa tcctagaaca 194400
atatagtgaa tgcactatag ttaaagagtt ggaaaaatgc aggcaataaa gtagaatatt 194460
tgtatttgat atcataggta gggcacttta acaaatgaga tactctatta cattgccatg 194520
ggagggtggc tgaagtgcag tgcacgtgga agtcactaca agtgcctaag tctaggaact 194580
aagaagttag gcaggtggaa gatcatctgc atggacttga agacacacag atgatgtgac 194640
tcacaaagtt aaagataaaa acaggctggg cacggtggct cacgcctatt atgccagcac 194700
tttgggaggc caagacgggt ggatcacaag gttagcagtt caagatggtg aaatcctgtc 194760
tctactaaaa atacaaaaat tagccaggcg tggtggtgga cgcctatagt cccagctact 194820
caggaggctg aggcagagaa ttgcttgaac ccaggaggca gaggttgcag tgagctgaga 194880
tcacaccact gcactccagc ctgggtgaca gagtgagact ccgtctcaaa aaaaaaaaag 194940
aaaaaaaaga taaaaacaat gtgtttacca ttggaggaag gggtccataa gacacaattt 195000
aaagtcatgg ggggatggga atagtttgga tcaggaagaa acagagcagt ttggtataaa 195060
aatgaaagaa caggtagatg acataaggat ttatgttttg ggtggtgatc aaagatgaca 195120
gatggggtac agtatggcaa aattaattgg catagggctc taaacagatc tgggataaag 195180
ttacttcagt acaggtatta actgaggact catacagagt cagaggcctg gcataggcag 195240
ggacaggtat gattttctgt agggactagg gcagacccct tacgcagtca gtacattttt 195300
tttatgagta tctgactgtg cagggctaaa catggtagga catagaataa tgattccctc 195360
tcatcttctc aaggaaataa cacacctaaa tagttgcatc ttgctgcctc ttcttttgta 195420
tgtcttatac atttactgct ggaaaaacta tctttctccc ttcttacttc agttcgttta 195480
aagtaaatga actttaatca tggctgttaa ttgtaaaaca tgactcaata aaaaaatgaa 195540
agctgggaga tatgaaatca aattagaagt attcaaacac caatgctgct aacttaaaaa 195600
gaagtgaaag tactgaaaag ttgccttcag cactgtttgt gtctatctct tcaacctttt 195660
ctagttaaaa caattttgta ttgacatcaa tgtgtgcacg ttatttggat cccaactcaa 195720
acaaactata gagagaggaa tgggggcaga gattggaagt ttgaacactg actgaatgtt 195780
ggatgaatta tttttaaaaa taagattact ataattaccc attttagata atccttaaag 195840
tgatcttgtc atgctgtctt gtttactcat gttttagaat tcgtgtattc atcgttaagt 195900
atgaacttta gctcaatttg gatctatcag tctttctatt taaacaccat atttgactga 195960
aaaatattca tatcctaatt tagttcaaag tgactttaac tgtttttagt aatgctctag 196020
ttgagagaca aaataacatt agacattcat attatgactg tatcgtttcc aaaataattt 196080
ttaagtcaat gggaaagata gagctaaata gatgaagggt tcagatcctc tgtgaggcat 196140
tcattatcaa ttatgtagaa ctttttgccc tttttagaac actttccttt tttttttttg 196200
ttatttttgt tgttgttttt tgttttgttt tttgtttgtt tgtttgtttt tacagcacct 196260
tgccctgcca cccaagctgg agttcagtgg cacgatcttg gctcactgca acctccacct 196320

cccaggttca agcgattctt atgtctcagc ctcccaagta gctggtatta caggcataca 196380
ccaccacacc cagctaattt ttgtattttt agtagagacg ggcagggttt taccatgttg 196440
gccaggctgc tctcgaactc ctggcctcaa gtgatccacc taccttggct tcccacagtg 196500
ctgggattac agactgggat taaagacgtg agccactgca cccggctgag aacatgcttt 196560
ccagttttat gtcttaagag tctttgtttt gttgtctaat ttatctagaa tggtttcact 196620
tccatttttt tctaatctta agttgtccag actcaaaaat tggttggtga tcttctcttt 196680
attttccatt tttgctatag tcattaattc attaattgct catttagtaa atatttgagt 196740
tcttattcgt agaattctta tttgcattct tgttagtaat aaatactaag ctggaaagta 196800
ggcattgggt ggagatgcag aggggaggga tacagagtaa ataacatggg gtgaagttct 196860
gatccttgga gtactcacca tgtaatgtgg tagacatacc atattcacat aattaaattg 196920
cattgtgata agttgtggaa tggcttcact taagtgacat ttgaggagga cttgaagaaa 196980
agttgccagg aaacagtaaa ggaatttcta gacagagaat caaatgttca gagttatggg 197040
gacatgctat gtatgtgaac taatgaatag ttgatatagg taaagcagag aattgagggt 197100
ggaaaacaga tatgtactgg ggagaatagc tttctctctt ccttctcagg cagcacttaa 197160
tctgtagaag tagagaaggc ttacttcttt ctccagctac ccataatatt tgtttttctt 197220
gccttcagcc catttgtgtt tatgtacctc ttatttctgt ttttaagcta cttaaggata 197280
aggactcatt caggatccct tctcactcac atttgtttct tcctaagcat taaaaattac 197340
actttgctca tagtaggttt tcacagtttg ttgatgcttt acacatttgt ggatgcttaa 197400
ttttagttct ttgcatttga tatgatcatt ctgtttaaaa taatttctaa catatatatg 197460
ctccttgcct taattatctc atttaatcct cattgtgacc ctgtgatgtt aagcatatta 197520
taattctcat ttttgcaatg agtaaagata gccactcacc caggatcaca agtagaaagt 197580
agtggagctg gtattggaac accagttgat atgattccaa agcccatgca tttatgttcc 197640
gtatgcatta atctagtgga gagataagga taaatatagt tataaaagga tgaagcaact 197700
atagctgtaa taatcctgtt actttgacta gaactgccaa actacagaaa gataaatact 197760
tataacctta atggttgaat gaggatgaat accatatgta ttgaagatga catgagttat 197820
tctttgattg catacctggg gaacattgat tgattttaaa ttaaaattct gaagatgtgg 197880
aatctgcaaa atgtgagcta gtagaatgtt tcaaatatca gggacaatgc aagtaaaaag 197940
atgaagagag aatttattat gcacctacca aggaagacat tagactctgg aatcacaagc 198000
ttaatttttt ggaaaacaag tgtacttcta tgataggaga tgttaatggt ttcaaactta 198060
cacacccaat tctttgggta ctctgtcaaa aatgagatct tttaacattg tgaggggggt 198120
tgagtggaag agaaagttta tatcaaggga agtagatgat agacaaatgg aagtattctg 198180
aggtatcttt atagttccca tataatctga attcttacag aattgagttt ctgtatatct 198240
ccatgcattt atacagatta cttcttattt taggctgatg ggtgttcaca tatagaaagt 198300
atactgattt tccaaatttt ggaacctgaa agatttgaag tagagttgaa gagaaagtat 198360

catttgttaa tcttgtttgt atagcttcac ctgggttaat tgataaaatt ggcaaatgtt 198420
tcttgcattt taaatccaat ttcattcctt tttacctaat aacattaaat ccaatttcat 198480
tcctttttgc ctaataacac ttagcagtca cagctttaac ataccccaga tttcctgatt 198540
ttgttaatag ctttgtgtgt atgtgtatac ataacccgtg ttttaatctc aataatatta 198600
acttaataca acattagtaa atgtgctgtt cttatatggt taagaacagc tttatttcat 198660
tctttagctt agtgttaaag tttcgtaaac agaatgagat taaaacagtt aaattaagat 198720
tgtaataata tatatacgct attcttaatt ttcctgagtc atttcatcat tttgctttaa 198780
caatggcaat tcttagcttt gtgcttatct aacacaagga aagatttttt tcttttattt 198840
gcatgagcat ttggcatggt tggtcactta ttttcactgt gataattatt cacttgttgg 198900
tctcttttct acgccatgaa agattatcag cttagagttg caatttggtg ttcattaatt 198960
tagttagatt cccagtatgc tttaattttc taattttctt gacagaactt ttctgccttt 199020
aaaatagtat ttgagaatga aatgttattt tatatatatg tgtgttcgta tatacacatg 199080
cacacacaag ctgtgcttat aataatctgt gaaattatac tatatgaaag ctaacatatt 199140
ttaatagtga gaaattgggg ctttggaaaa attaacattt tcctgaatgt tcatatcttt 199200
agactaaaac atttctaaat ttccaaagaa cactattcaa gtgtgtgtgt gtgtgtgtgt 199260
gtgtttgttt gtgtgtgtgt gtgtgtgtgt gtgtttgaga tgaagtcttg ttgttgccca 199320
agctggactt gaacttctgg gctcaaggag tcctgccttc tcagcttccc acgtagctgt 199380
gactacaggc atgtactacc atgccgagct atatttttaa attattttgt agagacgagg 199440
tctctctgtc ttatccaggc tggtctcgaa ctcctggatt caagcaatcc tcctgtctca 199500
gccttctgaa tagctacgag tataggcatg cactactcac ccagctattc aagtatattt 199560
tatttttaaa ctccagcttt ctgttagtca aatttctgct tcttgataaa tgcagtttta 199620
ttaggacatt gcattttgaa aataatctta gggtaaacca gtgtgaatta agaattttct 199680
acaactgcag aacaaaagca aaaaagtata aagtttcaat ttacatggaa gcatatacag 199740
aattgatttt attggcaatt tattaccaca gttagcagtt aacatcattt taaaatatca 199800
ttgtacatgt tgttgactga gaaagcactt atgtttgcac attgacttaa aagagagaca 199860
taagctttaa atattgtcaa ccagtcatca cattgcttct ttatcactaa ctttacctgc 199920
attactttta acaattgcct ttcatgttac tttaatacta gaataattta tgctaaaaaa 199980
tagaatacgc taaaaaaaag tgatattgtt ttatgtcaaa aagtctgtcc cactatgctt 200040
tgtaagggag aaaaacccat tgaccaagta ctctacaaat tcttatatac gtggatgaac 200100
cagtatagac atttttcccc taaaacatag cacttcaaaa tgctctgcaa tatgtttttc 200160
actttaccta aagagaaagg acgtattccc tatccacttt taattagggg gcagttggct 200220
ttggttttgg tttacattta tttgtaggca tattattaga cactttgttt agagttttat 200280
atgtcttata tttatgaatt agtatatatt tatgtagatg ggtgtgtacc cacattcatc 200340
tgtacatata tatgtatata tccatatata tatatggata tggaaatatt agaactagaa 200400

aacagtaaat aaagtctcat tatctaaaat gagataatga gaaaagtttc attatctaaa 200460
atgagataat gagaaaagtt tcattatcta aaatgactta taattgataa ggaattttga 200520
tgtttctttt ttgttgaagt cttagttgat ctttttattt aatattttga atgtcctctt 200580
ttttggttca aaataaccgt ttatcataat atttacataa gcaataaaat tgtgcaattg 200640
ctactcacta gaaagatttt aatttcaaat cagataaata tagattgata gcatgagata 200700
actacgaaaa tgtatatcat ttaaattatc agtaaaacat tttgtgaaga tgttaatttt 200760
tctaggataa cgtttctgtg aattaattgt tccttaacaa aacattttta gacttaaact 200820
tagagatcct aaatgctctg catttcctaa actttgagat agagaaaagt aggcctgtct 200880
tttgataaaa ttgattacat ttttatcttt tgataaaaaa ttgattgtat gtattatgtt 200940
tttcttatca aatttgttag tagcattcag aaacatttcc aaatgaattt tttcctactt 201000
ataaagggaa ggaaaattga aaaaaatgac tcagactttt tattttttac ttttttagtt 201060
tttattttta tttttgtact tcaaaaagtg ataagttcac gtaaactaca tgccattatt 201120
attcataatg ataattgcat tttctataca atcgatatca tttccatttt tattataaaa 201180
cagcagactg gtttctgtca taaaacaaat ctggcttgta ttagaacaaa tacatcctca 201240
tttaaagaat tttaaggttt ctttcttgca ttgagtatct taagctttat aacaacgtct 201300
ggctcttaat tttagtaaat ggcatgacac actatatcca tggagagctt ctgacaaaaa 201360
cttacttatc agacagaatt tcttcggtgt gttgtttact cagatggctt cagtttctct 201420
ttagtaaaat aatatttcca tgttggtgct agagtacaga aaaatgttgt tactgtgcac 201480
gcatcttctg cagatgatat cctgttccct ttctttgttg atatttttct ttcttaaact 201540
ttgttgctca gaatatcttt aatgctagcc aaggagtcag ggatttggac gtattttcat 201600
ggacttccaa agcttttttc ccccaggtga gttaactgta aggaacaata atattttatt 201660
caggtatttc agcatgtata acaaattttg tgtctttaaa acctttgctt aagattaata 201720
aaacctttta accaaaaaaa gatctatatg aagtgctgca attgagtttt ctcactcctg 201780
ccaaaaagaa tgtattatta gtttaagtga tacttccaca tgacttcaaa tttgctatat 201840
ttagttttat gagtagcttt tccaatgact atatctaaga ttagtgtttt cacaactaac 201900
ttatttaggt gacagtgtcc tggccttatg gctcttttat tttttctgct gccaacactt 201960
tcacattccc tctttgaatg ttcatctgag aatgggatgg gaatggaggg agaaaataat 202020
aatattgaaa ttcatgaatt taatatgctg gtacctgggt cttacatttc tgtattgaat 202080
aaagcatgta ttttactatt gcttgagcat gataggcaca gttctatgac tttatgtctg 202140
tcagtgtttt atactgttta gcacaagaat tggataaact ttgttttata cttattaata 202200
tagttttatt accttttcaa taaaatatta ttttaaattt ttaacaagtt ttcataattg 202260
cctttcagtg actagtttgc aattataatt agttgtgcca aggcttaact cccagtgtta 202320
ccctgaatga tcaaaagtta gaattattgt tataattaat taaatgcact aggaatatta 202380
ttaaatacca gtgaagttgg gtttttttgc taaatctatg gatatttgct tttttatgtt 202440

gccatgtaat ttttaagaat cagttgtgag tgggattaaa acttgcccct tccaactatg 202500
aaatggcatg atgtatttat ttattaacca tttacaaaca gacattttaa aatgtttaca 202560
gaaaatcttg tgagtgtaat agcaagtact atgtaaagtt tagacctgtg ttactctcaa 202620
tttttatctt gagtgctctc tgtgtattca tgtaggtttg catatatata aattcttaca 202680
aatatatatg atgagtatat aaaatgcgta ttttacattt attttcggtt gtaaatctaa 202740
gttttttgag tgtttaaaat tctcatttcc gttaaatttc caatatgtaa ccaaagaata 202800
ttggtaaata aaatataatt acagtaaatg taaattaatt tttcttagta catatttgta 202860
taagtaaaaa ttggttattc atcactatag tgagattcac aaaatattat gtgaaagtta 202920
atggtgactt taacattttc cctgaccagt catgtatgga attaacaaag tagcaggtat 202980
acagaattct tgaatctaca tatttttaaa cctgaggatt ctatttttaa tattctactg 203040
tgttactgtg tgtatgaaca tgtatacata tctcttcatt atgaggctac tggtagggat 203100
agaactattt aatacaattc tatcttgcat aatgttagtt gttaaatttt tattctattt 203160
ataacattat taaatatcag ttttaaccta atgtttgcat gtgtgtctct ttatggttat 203220
ctattactct atctgaatat gaaataaatt atagagttca tattactgta tttaaaaatg 203280
cagttttaaa tctcttttaa aggagtaatg atgacaatga gaacacccag tgtggaccct 203340
ttatttacat ttagttggga aaaagagaaa atatggaaag gttattattt ctctttatta 203400
cattcacata ggaaacaatt ttatcacagc attacaattt aacagaaaat ttagggaagg 203460
ttctttcatt ttagaagtga ctcttttcaa taataaattt acgttatatt tacttaaatc 203520
catgtttaat ttctcctttc tactaaaaat atagtctgtt tttctgttac tagcattaat 203580
gtatcactgc atagtaatgg cttaacaaat ctagcaagac gtttttattt gtacaatatt 203640
agagagtata agttgttaaa atattgtaag catcaatatt tcattcataa ctaacaaata 203700
gcaagatata gctatatttt taatgcatat ttttcatcat ctctgcttat ggatcagttt 203760
tcagattctt catacctgtt ttctctattt ataaataatt atttcaacat gggaagaaat 203820
gaatatttag actttttttt acccataaca atatacagta atgcagtaat aatgattcaa 203880
aattattccc ttgtggttca catagtatag tatttttatt tttccaactt taaatattgc 203940
attaaagata aaagtaaaat caaggatgat tatatttctc aaattatggt gggcagaagc 204000
cagaggttga ctggtttcct gctatatttt cttgccttca actttatttt aaatagcatt 204060
gttgaaaaac tgagattctt taattagtgc taatgactaa ttcttcagtt atctttagtt 204120
atgtgttttt tttatattga cagtactgta gacttgctat aaaagctatt aataggaagg 204180
ctttgaaagt gataatagat tgtattttgt ttatttttct ttcctattca aattttcagg 204240
aatctaaaac taatactgat gactttttca aagacataaa ctcctgctgc ccacaggaag 204300
caacaatgca agaacaagat atgccattct tgcgaggagg gccaggcatg tacaaggtag 204360
tgaagacggg accttcaggt cacaacatca gaagctgccc taaccttaga ggtatcccaa 204420
ttggaatgtt agttctggga aacaaagtca aagcagtggg agaggtatgg attcttgtag 204480

cttcatgact tctaagatac tcatttttaa tgtaatacaa ccaaagcatc tcttctaaag 204540
gttgccattt ctaccacagg tttttcaatg ttaatgtatt ttatctttac ttgtattgca 204600
aatacataat ttttctgtgc ttctgtctaa tgatttgcat aattatcctt gtgtaaacaa 204660
tatattaaca aatgatttgt attgtatata taaatgtttg gtatttgcac atattttttc 204720
attattacaa atgtactata ccatcatgga ttatcatcac ttaaaatatt ctatgtgatc 204780
ggtatggaga tgtggtcata tgcaaaaggt ttcatttctt tgttcccatt cataggtctc 204840
actgaagtcc tgagcaaata aaattactct ttaggctact gtggcatttg cctcagctga 204900
taagcttgaa gatgtagtgt gtcatgctat ttctcagtaa gggtcatttt caagttacac 204960
tatggatact ctcaacctaa ataatagcaa gtcggctgtg ttaggacatt ttagatgcag 205020
aaaaaagaaa atgagccatg tgttcttggg taattcttaa aattatttag gtgttatctt 205080
tgtgctttga aagttattat tttaaccaaa ttcataaata agcagtattt tacagaactt 205140
tttattgact tctgtcttac ctcaggctgt ggccttaatt tcagaaacta atttcttagt 205200
agaaatgtat tggaatgatt acactattca gacacgtaag atttttactg gttttcagac 205260
tgagttccct gaaagaatca agccaatcaa tcatgctttt tgttatgtac caggtaacca 205320
attctgaagg gacatgggtg caactggatc agaacagcat ggtagagttc tgtgagagtg 205380
atgaaggaga ggcatggtcc ttagctagag acagaggcgg aaaccagtac ctccgacatg 205440
aagatggcaa gttggcttaa atttgtgatt tattccaatt aagctttcaa atacagaagt 205500
ttaatgccta ttgtattgct tttgttttaa agagtttttt gtgtgtttgc tttttaacaa 205560
gcaaaactaa aagttaaaaa atcttgcaac tgttaaaatt ggtttgtaaa atacgtgagt 205620
acttaatgca cattaaagaa gaaaattaaa agaaaattcg aaacatatag actcatcttt 205680
ataaaacagg cttttaattt cattaatata aatttatata cctctttctt ctgtaccagt 205740
ccaggactga acgttctatc ccttctataa taaaatgaaa cttcggtatt ccaactacca 205800
atctcagtac ttctcccagg caacttcatt tgattgcatt ttgtcttagg gaaagagcat 205860
tgaataaatg catagtgtct ttagggtttc ctgttgttag attttcaact ttaaatttta 205920
ttttaccttt ttcaacatgg ctatcactta ataaaatata ttcctttttg tccccctctc 205980
tttgatatct tggtggcaaa aatttgttct tcgaagttga gaccacatta tcacttgtgt 206040
gatgctttaa ctgatcacaa cagagcaaag aatttcttgt gttcctaaag aattgtgctc 206100
accactagaa caatagtgtc atagtgaagt tcatgtttat ttcttcatac ccagttatga 206160
gtcccttgtg gacaggcaca tggtttttat ctttccattc ctagtactga aacagttttg 206220
acacatacta gttgctcatg aagttttgta atacaacttg aattataaaa ataatagcac 206280
atcaaaataa gtatgtaagc aaacatggac ttgtctgcca aatcccaaaa tttataggat 206340
gaagttaggt aaataagaga acatgcatct ttattcaaaa gataataaat ttataatact 206400
tattattcca agaggcaatt tggccataaa tatggattca gaaaaggttt gaaatttgca 206460
ataatgatgg agtatctttg gagatatctc cctttttccc cccacaacac acctttgagg 206520

taggtgatat aatcttaaat ttataggtag gaaactcaag ctctagaatt taaactaacg 206580
tgtctatggt tgtacagctg gtgacatagc agatgtaaga cttcagccag aatcttcatt 206640
cgtgttttaa atgctgtact cattacacca tgctgtacac attgagcaca acttgagaac 206700
ctacctcctc tccttgtttt tcccagatag acttttttta gtctaagatg atatggtcat 206760
aaaataatgt cctgaaacag tagaactgtt catttgaaaa agataagggt tccgcttgat 206820
gtaaatcaga catttactaa atgacttttt cacttactat gtgcttatta tatattgctg 206880
tatatttact tcatattcat aaatttttaa acatttttct ttatatagga atattcagat 206940
ataaatgaaa atacctaagg ggagatcagc tttatttaag ataagttaaa tatgagaaca 207000
gcttaggtga cttctaaagg acttttctag cttaatcctt aataattttt taactggcag 207060
tacaaaaaat atgtaagagt ggattctgaa gcaggactgc cagggtactg atcccagctg 207120
tgcaactttt tactcatgtg accttgacca agttaaccta atatctctgt gcctcagttt 207180
tcccatttat aaaatgacct tataggtcat aaccacataa atttgatatg aagactaaat 207240
gagcaaataa cagataaagc taatggatgg taagttaggt cttattgtta aaatgcaaaa 207300
tgccttccat gctaaggatc aagttttaac ctgggaaatt ttattcttat aaacttagaa 207360
gatagaagct tctttcaaga taaaatgcct cttaaattag agtatcattt aagaaaagtt 207420
taaatttttc tctgtaatta gttaatcatc ttttactctt gatgtataga tacagacata 207480
aatatggata tttggtgatc atgtgaatct tactatttta atataaaacc tggagtgata 207540
aggaaagaaa tgatatgaag ttagtgcctt taaatcacta agattagttg atgtgctgcc 207600
caaatagtct gttcattggt gataatattt aatatggaag tacaaagatt tggcactatg 207660
gtaatttata tgaagtcatg tgagtaaaac aaaagctttc ctctttgtaa aaattctcta 207720
ttggtctttg aaatctgtag taggcaaagt tactgctgtg gtaaaaacag acttcactct 207780
gggtgctaga ccattcttag tctagagagg aagcagtgct atcttttccg ttcagtttac 207840
tgttcatggg tgtttttctg aagaaacatt ctactgctga aatattgcat gctgaagagt 207900
attgcctaag ggaagtgcta agattggttt tttaaatcaa gtctacaaat gctggtaaag 207960
ggatagatta tacagtacca ctggtttagc agaattgcaa tttacaggta ggaaggaaac 208020
actctaccac accctgtctg tagttccctg ttctgtttaa aggactattg actttttttt 208080
tttttttttt ttgagatgga gtctcgctct gtcacccagg ctggggtgca gtggcgcgat 208140
ctcggctcac tgcaagctcc gcctcccagg ttcatgccat tctcctgcct cagcctctct 208200
gagtagctgg gactacaggc gccggccacc acgcccggct aattttttgt atttttagta 208260
cagaaggggt ttcaccgtgg tctcgatctc ctgacctcgt gatccgcctg ccttggcctc 208320
ccaaagtgct gggattacaa gcgtaagcca ccgcgaccgg ccaaggacta ttgactttta 208380
aaaaagatat ttaagatact taaagatatt ttaaaaataa gatatttaaa aatatacttg 208440
agatatttta tttttcataa ggttatttta atggaataaa taccttttcc ccccaatttt 208500
acttgacaga ctttattttg ccttttttgg ctggcattaa tatggatatt taactaaggt 208560

attgttttat atcagacaga gtcttggagc cagttgattg ttcatagggg attaatttag 208620
tttgcatatt ttataggtca catcttgcca tttgtaaatg gataaaacat ttgccatttt 208680
ctgcttgaaa taagaattct gatacattta tagactctga ttatgtaaca tggtttatta 208740
ttatccttct atttgggaaa aatatcttta tgtgataaaa taagttaaaa gttaagtttt 208800
ccataatttt cacattcata acttagaaca gttaacaaat gaaaattcct tttggtacta 208860
gatacttagt ttttttctct attctaatac taatatgcaa aatactttta tttctggttt 208920
ataggttatc cattttgttt tgcaaaatac tcttgtataa atttgttgca aatacaaaga 208980
gttcctggtc cagaaatatt agtatgcatt cagtaatgta tcctgccaca ataaaagtaa 209040
attggcttca taagagccta tctcatttct aaatttcact gtgtatttga taaataaaaa 209100
caaagatttg tcaggtgctt tgttaatgtt aagatgacct gactggtatt taaattgatc 209160
aaatctagat gtgagcttat tttaaaatct aaccatcata aaatcttttg caattttctc 209220
tctttgttgt atgaataggg aatagcacgt aagaaactaa tgttacagct atggctttta 209280
ttgtagaaca agctcttctg gatcagaatt ctcaaactcc tcctccaagc cctttctcag 209340
tgcaagcttt taataaaggg gcaagttgca gtgcccaagg atttgattat ggactcggaa 209400
atagcaaagg taggtattta aaagtaagtc ttttacttac ctaacttcaa tattttttat 209460
taccttctct tttatacact ttttatttgt gttcacccag aaatctttat taaattaaaa 209520
tttgttcatg gaaagctaat aaaaaatgtt tacaaaggtg ttcttttcat agtattgttt 209580
tttgctgttg ttttttgttt ttaaccagtt ttggcttaag tttatgagag tgcttgctta 209640
ttttttttct atttaaaaaa cactcatttt gatttcatat cctaaagatt ctatttcact 209700
aaatctagta aaatttgatt tttatcatcc acttattagg atggtaatgt taatgagaaa 209760
cagagaactt gctcaaatga tccatttaaa atcaaactat tacttctaaa taatattttt 209820
aagtaatatt acacatgagc tttataaaat ttatgtggtt ttgaatttct aacccagtaa 209880
tcttagttaa tttagaatta tgtgaaatga tctgtttggt tttaatatct ataataaaac 209940
cagaaagttg taaaaagaag aaaaccctaa gtcatatgaa tataatatat atacagctgt 210000
tttggctgca ttaatggaag agaccagcat catggactgt ccaaacaata gttaattttg 210060
aaacaattta tgctggaggt tcatcagaaa ttgcattttt atgtgaattc acacactctt 210120
gttctttcct atgtacctga tctagactcc ttcaaaatca gccctattgt ccacaggtaa 210180
catacagctg ggtcatgagt aaaagggagc agatagctta cgtggaaata attttacttt 210240
aattttattt aaattaattc acaggacttg attccaaggg acaaatacag tgtcattata 210300
cctgacttga agaacaagtg aaaaaataaa acagtgaaca aaatgtataa ggaatattct 210360
gtttaaaata aaactacttc tattgaatag cttcattact ttccacaaag tatttttatg 210420
aaggaaacct agccacaaga ttttttttta ctcccttgga ctagcctatc tcataggatt 210480
gggtatcatc tgctcgctta cttcgggtac ctgatttcca ttaacttaag ctagatcttt 210540
gtacttacat agtgtaaatg cttggtggtg ttttctttat gtccctttct ctttaccttt 210600

gtcaactctt aaaatctcag catttgctta ccgctactat atctctatat agtaatgttt 210660
taaagccttg tttaacacaa attgtatgct ttcagtcttt aaactgtctg tcattgtact 210720
aatagctatc acagtaaatc agtcattata atttattaaa atatcattaa aataaatata 210780
aatgtttgag acacaaacat tgccgagaaa atttgttatt atgtacaaat aataaagtat 210840
tactttttaa acatgagatt ttgtgaatat cgttcaaaaa tagcatgcac catgccaggc 210900
atagtggctc atgcctatag tcccagctac ctgggaagct gaggcaacag gatcacttga 210960
ggccaggagt ttaaggatgt agtctgctat agtcgtgcct gtgaatagcc acacgctcca 211020
tcctgggcaa cagagcagga tcctgtttcc ttaaaaaaat attaataata agcctactct 211080
tgttaacaag cttttaaaat atttagaagg agcaaagaaa taataagaaa cttaaacttg 211140
atgaaatata tctaacttta ttatctgtaa cagtagttcc cattttaact cttaatactg 211200
tccaagatcg aagagctgtt ttttgtaagt ttttttttct taaaacagta ttttagaaaa 211260
ttcttcatga tctctaaatt ttttcctata atctacagca tttatttatt atttcaaaaa 211320
aagatatatg gctcgtgtac acagttcttt ccacataaat ctatcccgtg taggtacttc 211380
agttgaaatt ttcacatatg aaactttatt atatttcaga aatatgacta gaaagatact 211440
attttccagt ttctgcatca cctagtctgg atgtagttcc ctctccccac ctccagcatc 211500
atttaaacta tttaatgtat actcacaaat aaggagttat tatccatcac ataatttcat 211560
tatcttaaaa aaaatttttg taaaaccttt tggtacaagg aaaaatgtta gaatcgatat 211620
tttagatact attcaaatta ggtagctcat aatccttttt gtcatatagg acttacagat 211680
ttttaaatta ttcatctaag caaaatattt catgctttta aagtagaatt tctttggcag 211740
taacaactta aaaaagaaag tattaattta taactataat gtaaacacat tgctatgtta 211800
ataacatact ttgccccatt ctgctttttg aagaaaaact gttaattgct tattttctgc 211860
atgaaatata ctgccgatga ggcagggtgg tattagtcat tgttgggaac tatggaaaag 211920
gcatcattta cttaaatctt catttatata tgttcaatat tgaagcaatt tatcactgat 211980
tcttcataga gtcttcgttc tcccatacat aatgagagat gttatatatg aatttaaatg 212040
gatttgtttt tataggtggg cttctatata agatttggtg gggcgcttcc attaaaaaaa 212100
aaagcttgca aaattcggtc cctaatctga ggatgattaa cagtcaccaa ttttcactca 212160
tttctgaatt ggcatggttt tatttagtgt tcaactctat ctgaatgtca taaagcacca 212220
tttactttgc cattttggat gttaatgaat aacgagttgt agttttgact gttttaccca 212280
ctgcaggtga ccaactgagt gccatattga attccattca gtcacgaccc aatctcccag 212340
ctccttccat ctttgatcaa gctgcaaaac ctccctcttc cctagtacac agcccatttg 212400
tgttcggaca gcccctttcc ttccagcagc ctcagcttca gagtaagtct gtcagcctta 212460
cctgcctcat caagcatttc tgaagctttg ttctgagttg cttatcagtt actaattagt 212520
tatcaactca cctgactggt tttttttaat taggatgtta tcccctccca catttttgaa 212580
atatctaagc tattctgagc taagacattc tgcagctaag acatttaaag ttgatttaaa 212640

gttcatatgc atgactatgg taagcatcac atacagggac aactttcagt ggatttttct 212700
tacttcaaga tgtcccaaaa ggataaagcc atttcactct tatcagtggg tagaagagag 212760
actgtacttg tttggacagg tttgtttgtt ttttctaagt gaatttggta tctatgaaat 212820
gtatgacaat ttagttttcc cggttttctc aaattgttgt ttcctgtctt tatttttatt 212880
tttttgttgt tgttcctaat atgttaaatt gatcatttga ttccagttct gtcttttagg 212940
attgctctag tccttcattc atgatagtaa ttcacattgg tttggaaaag atacccaaaa 213000
aaattatact taactgagtg ttcttttttc ttaataacct cctgaacctt ccacctaaac 213060
ttttcaaagc tctgtccagt atgacaacag tgtgttcttc ttggcattca gttctcatgt 213120
tggctataca atatataaac acattaattg aatgagctag tttatgaaag tttcagcttg 213180
tataccattt ttcggattca aatgcacaac ctctttgctt tggtaaagta cattcacact 213240
taggtctaag ttatttgttt acaaaaatac tttaataact gttattaggg tcatgtaaaa 213300
gaatgctagc tggtttagtt tttattaaaa actaggttag tatgtagcta gcaaatgata 213360
ttattcatta ctttatccac cttaggtaaa gggtacacaa gagtataaac ttttctgggt 213420
ttccactatc cattaggaaa gtttaattga tgaaattaat tagtttggaa atataagtac 213480
tatcaatttc tagcttatca tgttcgttca tgtataatat taatttgaat gtctattaag 213540
agaagaattt caaagaaaca aaatagtgtc acatgtgcga aatagttttt tccttctatg 213600
ataaatatta ttttagaaga atgaaacaga agtgaggagt tagtttatta attttgtgtt 213660
gcatcagact ttttacattt agaagtacta cttaggtttc aagaagtttt aaaggtaagg 213720
acaaagggaa aaacaaagga aaaatatcag gatcagtcag gtagaaaatt tatactagaa 213780
aatttaaatt cattcatatt caaattcagt tttaaatgaa aaattgcctg tgtcatagca 213840
gtagcagaca aaagaactaa ggagaaagga gaaccttttt gttctagcta aagaatcatt 213900
gcaagtcttt acaaaaacaa gcataattac cattttaatt ttaccaaatt taattaataa 213960
aatatttcaa aacaataaac agtaataaaa gttgatttgt ctcttccagg gaataacagt 214020
ttctaggtga cattgtgtca atataatttt atgcatatgt gttatcagta ccaccacttt 214080
atttaccact atcttgaagc aaatttgaaa tcatggctta gctatgtttc aattagcaat 214140
tttctgaatc attacttgcc tatagtccag caatggaaat tacttaggtc ttgttttaaa 214200
cttcttattc gcaacaggac gtctctaaat tttgacttcc aaattccaga aaatggaagt 214260
cttaatcttt agcatcacaa aaaaaagtat ggaatctgaa aatagtttta attttttaaa 214320
ataattcaga attgtatttt ttataccgtg ttgggaaagg taaatgtggc aaagtgccaa 214380
catttaagac tccccaagca aggtattctt ccctgaacct cactaccatg tatcccacta 214440
gtatatcacc tatgctttag aagatggctt attcttagtg aacttcccta tgctatttag 214500
taaaatgaga tttatttttg ttttatacct ttgtgatact aaataaatca ttatgtagaa 214560
gaccatattt actcaacttt gtttatactg atagtcgcca ctatgtagta aacactgtac 214620
ttaagcactt tgatacagcc tcatttgaga tactgtctct tatgtctgtt ttacagagaa 214680

ggaaacatag gcttagagag gataaatgat ttgccccaga aacatacata gtaaaatggt 214740
agagccagaa cttgaggtca attctgttga ctacaaaggc tattttaaat attcagcctt 214800
acctcggtaa ataatgcaaa atctacttta atatatatat atttttaatt tatatttaaa 214860
ctaggtctgg catctagagt attagctttg ttcagattga ttatttttct ttttatagtt 214920
tgactttttt taatgtaaca gtagtttgaa tggaaaaaaa gatttcctga atgtcataac 214980
tcaagatcct ggttaactag aaaaattcta caaagacaca catcaaccgg tgcatggttt 215040
aacttcatct agcatttcta tgagcattat tatctgtaat tctttggttt tttttttccg 215100
aatcatagtt tttacaccta tctttggaaa tttgaaaata atctggcaca ttaagttaat 215160
ttttgatacc ttttctttag aatcaggcaa cattttattc cataaaatag aatatttggt 215220
aatttttaaa ttagcaaaat tatttgattt tatatacgta gaaattattt caagtgttat 215280
tctagtgaag tagaatgtta tagaagtaaa agttaacaaa tttcccttgt cttatgtgtg 215340
tgcacacaga gacatgaaaa agaaaagtat gtatatagaa ttttctttac tgttctacaa 215400
tttacttttt aatttaaaaa ctaattatat agtttttaga attggtaagc aatttggcaa 215460
aaaaatttta gtgaaccaaa gtggagaatg atatctgtat ctgctttctt taagaatatt 215520
acattcctta ttttcattat caccatttct cttgaaagct tcattttatg tcctgtacct 215580
tctgacagtg tcttacacat cacaggcatt caaggagtgt ttattgactg taggtattta 215640
agattaccat ttcatcaatt taagatcttg tgttttctat tatgatattc cattgtttac 215700
tcagagaaca tcttcaaaga agagaattta tttctttctc ttagtaatta aagccaacta 215760
tactgtgaca atatgatgta tgataattgt gggtttatac tgattgaatt ctaagcaaga 215820
ttatccaact gaaaaactat attgcaaatg gcttcattta acatagcatt ttatcacttt 215880
ctacctactg ttccagaggc tgaatagttc atttttcaac ttttatttat ttgtttgtgg 215940
tagaaaaatg gcaaataaaa ctgaagacaa tttgcttttc tcccagaaac tttttaatac 216000
tttttttatt ttataatgtt ttatttttaa ttttgatggg tacatagtag acatcttcca 216060
gcaatttcaa acatgaaata tatgatatat gtcaaaagac agtactttca agcacagata 216120
tcatagctct aggttacctt gcaacaagag tttttctatt atactcataa gtcacattct 216180
tatgtgggac aaaaaaaatt aaatgtgacc ccaaaattca tttggatatc tgcatgacta 216240
tttatttaca tttgagaata tgaacacact aaagtactat gtttacttag cttgtagaaa 216300
catagtcatg gaaagacttt cagctcagat ttcccttcca gctacttact agctctctaa 216360
gttatctgac aggtctgaat ttttgttacc tcattataaa atgggattcc caataaatgt 216420
tatcttttga tgttattatt gccttttgat gcaaatactt aatacctctc atgctttgag 216480
gaaaatatag aaaaagtcct tctctaaaag agagcaatct attcttggtg tggaatgagt 216540
tgcaggaagg aatttagaaa gggacaaatg agctttccta gttccttttg ggtctatttt 216600
tgcaatgtaa ttcagttacc tattttaaga ttcttaatgt atttagactg cattcagatg 216660
ttagtgtgaa tgttagcata aaagccaggt ttgtttgtaa ggcttagact gagtcatttc 216720

taccccggat tataatctca gcactgtatt tgtcagtttg ttgtttgcct gctttgaagg 216780
tatgtggatg aagcacttga tttaggaatg aggacctgtg ggatttaatc cctgttttag 216840
aagattagag tcagcattgg ttcacatatc aaagtttaat aaaacttttc cacttgactt 216900
tataacatct gtcacaacaa ccaacatatt tattttagtt ttggggctaa catctccagc 216960
atccctacaa atacaaatca ttttaggcat gggcttgaat ttatcttcac tgagctgagc 217020
catcttctca gtgacctaaa agctttgacc caggtatttt gttttgctgc ttcatcagtg 217080
atttattcat ccacccttta cttcatccta ggaattcaaa ccacaaagaa tgctgatgtt 217140
gcttacaaga cattttgtcc cagtgcattt gtttgctttc tcctggcttg atttgacctt 217200
ttatctctcc ctcttccccc acctctttgt ttgctactgt gtttatttca cccttcatta 217260
ccatctttct ttagatgact tattcattct tccatttatt cacctgcttt tgttaagtat 217320
ctactctgtg ctaggttatg gtgatgagca aggttaaaaa aaaaacaaaa aaccatgatc 217380
actgatcttg aaggcttcta gaatatttta agagatacaa cctagttaaa gaaaaaataa 217440
tggcatgttt taagtgcaac aagagcagta gtcacaggat gcaatggaaa cataggaaag 217500
cttctggctc agctaggaga ggttagagaa acattcaaaa aagaggcagg tcctactcag 217560
agtgttgaag gacttgtgag aatttatcag gcaaggagga gtggagatga ggaaggaggt 217620
taatggagca ttccaactat aaggataaca tgagttacat tacagagatc caatgaatca 217680
caaagtattt tattgagtcc acctacgtgt caggcagtgt actaagcact gttcatgcag 217740
agggccagtt tggagtaatg aaactccaaa gtttgattag aataggctta ggagaaaacg 217800
gggaaggaga aattggagac aatgagtata gagaagtgtg tcaagatttg atctaaagag 217860
aagatactaa gagagaagat acaagatctg gagaggtttt tgttctattt tgtttgttaa 217920
tggaaaaaaa agtatatcaa ggttgttttc taatgagagt aattcagttg aaagaaatat 217980
taataatgca ggagggtgag gagaattact ggcacaatat tcttttatag aagaggggat 218040
gcagaccagg cgtagtggct cacacctata ttcccagcat ttggcaaggc caaggtagaa 218100
ggatggcttg aggccaggag tttgagacca gccaagacaa catagcaagt ccttgtttct 218160
ataaaaatta agaaaaattt ttttaaagat cagctgggca tagtgatgca cacctgtaat 218220
cctagctact tgagaggctg aagcaggcaa atcacttaag cccaggagtt cgaggctgca 218280
gtcagccata attgcatgcc accccactct agcctgagca acagagtgag accttgtctc 218340
taatatgtat atatacagat ataatatata atatataaaa tacattctat ataaaatata 218400
tattatatat tatataaata aaatattata tataaaatat atattatata aataaaacat 218460
tatatataaa atatatattt tatatatata taatataaag aagaggggat acagtccagt 218520
gcaaagtgga aaggttggct aagtgggatc ataaatagtt atccatcata acaagcaaga 218580
aagaagtatg tatattattg ggagctcttt tttctaagta aaataggaag cacttatcag 218640
ctcggagtta ccatggagag actgcattga tagtttgagg aaagagaaga tatgagattt 218700
taaaagctaa catatgtcaa atatttatca catgccaggc accattcatg ggaatcaact 218760

tttatccctg tccaagaaca tggttcactt gagaaacttc atgcatgtgc agctttaagt 218820
gacctagtgt cagtgtctta gactagagag gttggggctg gatcatgaag ggtcttgtaa 218880
gccatactta gtttagactt tatgtgcagt gaataaatgg agggagcaca tggaggaaat 218940
tgttctgcag aattttaaat aaaaagagtg atatgatcag ttttacattt gggacagata 219000
actgaaaata ttatgaaaaa ggtcctggag aaagggagaa tgttggaggc aaagaatcca 219060
gagaaaagct gagtagggtt taagctaagg ttaattgcag ggagaatgta gaagaaatat 219120
ttaggcagta aaatgcttag gaccagtctg gacaacaaag cgagaccctg tctctactta 219180
aaagaaaaaa aaaaaaaatt agccaagcat tgagggctgc acctatattc ctggctactt 219240
gggaggctga agcaggagga ttccttgagc ccaggagttt gaggctgcag tgagcagcct 219300
gggcgaaaag agcaagaccc tgcctcttaa aaaaaaaaaa aaaaaggaag tgacatgctg 219360
attaaagtta ggagccagtc tcaaatgaca tctactttta tgctctgttt tgaaaggtaa 219420
tatcttatct agtaccaaga ataaaacaca aaaccacctt ttaaggaaaa atgagttcgg 219480
ttggatgact tagctccagt tggctataat ataacttgaa attgagaaat atatttagca 219540
ttctcataat taaagatggc attgttgact gacaagttga aaatagaaaa ttattcatat 219600
attacatttt attaaaaaat attattaaag cattctcatg attttgcact caatttagga 219660
aggattaggt ctgctatatc tcccactttg ttattctctc aaacttctgt acctgtagta 219720
tatctcatta cttaagctga acaaaacgtt cattttttgt aatcagactt ctcatcctta 219780
tcatcagatt gtctttcctc ctttggttta tttaagaagt gtgcatcagc agcagccgtt 219840
atgtcttatg atggcataat ttcaagtgtt ctaggggcct aagaagttat ccagctctca 219900
ttttgagatg agagaattct tgctgacttg cccaaagtca tagctggcaa atctaggact 219960
tgaacatgag agtctgtatt gggggaaccc gcccccaata tttcaacgta ggttctttct 220020
attttcccta agcattggcc agtctgagaa aaaaagagaa agagtacaaa gaggaatttt 220080
acagctgggc ctctggcggt gacatcacat attggtagga ccgtgatgtc ctctgagcca 220140
caaaaccagc aggtttttat taagcaggtt tttatcaaaa agggagggga tgcaagaaca 220200
gggagtaggt cacaaagatc acatgcctta aagggcaaaa agatcacaag gcaaagggca 220260
aagcaaagat cacaaggcag agggcaaaat taaaattact gatgagggtc tatgttcagc 220320
tgtgcacgta ttgtcttgat aaacatctta acagaaaaca gggttcaaga gcagagaacc 220380
gatctgacct caatttcacc agggtggggt tttttccccg ccttctgagc ctgagggtac 220440
tgcaggagac cagggcgtat ttcagtcctt atctcaaccg aataagacag acactcccag 220500
agcagccgtt tatagacctc cccccaggaa tgcaattctt ttcttagggt cttaatattt 220560
aatattcctt gctaggagaa gaatttagtg atatctctcc tacttgcaca tctgtttata 220620
ggctctctgt aagaagaaaa atgtggctct attctgccta accccgcagg cagtcagacc 220680
ttatggttgt cttcccttgt tccttgaaaa tcgctgttgt tctgttcatt ttcaaggtgc 220740
actgatttca tgttgttcaa acacacatgt tttacaatca atttgtacaa taatggtcct 220800

gaggtgacgt acattctcag cttacaaaga taacaggatt aagagattaa agtaaagaca 220860
ggcataagaa actgtaagag tattatttgg gaactgataa atgtccatga aatcttcaca 220920
atttatgttc agagattgca gtaaaaacag gtgtaagaaa ttataaaagt attaatttgg 220980
ggaactgata aatgtccatg aaatcttcac aatttatgtt cctctgccac ggttccagcc 221040
agtccctcca ttcaggatct ctgactttcc gcaacaagtc tgctaactca ttccagtggt 221100
tttttccaac tgcatctcag ttatcttaca tagactgcaa gaagtgagaa agacaagagg 221160
ttatctagtc cagccttgct attttatagt ttaaatccct caaccacatc cctgatgaac 221220
ttttgccagg ccggtaatta acaatatcac aaggctgttc tgattgtctg tatttctcag 221280
tgtttgttag agcagggatg tccaaccccc aggccacaga ccaatactgg tccaaggcct 221340
gttaggaacc cagctgtaca gcaggaggag agcattactg tctcagctct acttcctgtc 221400
agatcagctg cggcattaga ttctcataag agtgcaaacc ctagtatgaa ctgtgcatgc 221460
aagggatcta ggttgagagc tcctcatgag aatctaatgc ctgatgatct gaggtggagc 221520
agttccatct tgagactatt tccccaaccc cccatcatat ggaaaaattg tctcccaaga 221580
aaccagtccc tggtcccaga aagttagggg accattgtgt tagagaacta aggaaaccgt 221640
cctctacctg ccacataaga ataaaggaaa caatggaaca gtttccctac tttccctaat 221700
taacacgtat tcccattttg aggcagtgag ttgctggtac ctgctttctc cttctttctc 221760
caaatagtac tttaaaagta tcttatcctg gctgggcaca gtggctcatg cctgtaatcc 221820
cagcactttg ggaggatgag gcgggtgaat cacgaggtcg accatcctgg ctaacacagt 221880
gaaaccccgt ctctactaaa aatacaaaaa attagctggg tgtggtggca ggcacctgta 221940
gtcccagcta ctcaggaagc tgaggcggga gaatggtgta aacctgggag gcagagcttg 222000
cagtgagctg agatcgtgcc tctgcacttt agcctgggca acagagcaag actccatctc 222060
aaaaaaaaaa aaaaaataaa gtatcttatc ctaaaagcac ttctgttttt ggtttaggtt 222120
ttagctgtct ttgtgctgcg taagcgttgt ctctttctca ggatgtcact tctggaggca 222180
ggaaagggtt atggttaatg ctaatcactt tatcaaaatg tctgatttct ctgatgtata 222240
attaatattt ttcccttgca actaataagc aacttgtggg agtaagattt ttacttttaa 222300
aagcatatcc aaccttcttg tgtctcaagt taatgctcgg gaagcaatac ctgtctctta 222360
tattttgaag ttattctttc taaggcctaa gatatatacc catcaaatta tgctcagaat 222420
ttttgtctca gccatcacaa accctgaaat gatacgcttt tcctaagtgt tattttcctt 222480
aaccatattt actagagatt tggaatatat tcaataatat gctatgagta tgttttcttc 222540
aagttcaatt atgtgtagat gtcatttaga gaaacagtat attttggggg gaatccactt 222600
tatgtgtccc atcttctttc cacattcaga tcatgtggat taatgtcata gtctagtaaa 222660
aagcttgttt ctattttatc cttaagttta aaatgttgtt ctttcattct attagcatga 222720
ttcttttagt atactaacat ttatgtaaat aagtcttaca taaatacagt tatataaaca 222780
aatctttagt ctaattcttt tagtatacca acatttatgt aaataaatct tagtttctgt 222840

tccacgtcct aagctgcttt accattcaca taattccata ttcctcctaa gtttactaca 222900
aagaagaata ttagatgtat tgattatgca gtgatactgc atctaaagct gtcagtcaag 222960
aatggctgcc atagctaaga gtatatacaa atcatcactg ttactttatt ttattttttt 223020
aattttatgt atttatttat ttttttgaga caatttcact ctgtcgccca cactggagta 223080
cagtggtgca atctaggctc actgcaacct ccctctccca ggctctagtg atcctcccac 223140
ctctgcctcc tgagtagtga ggactggagg cgagtgtccc cacacctgcc taatttttgt 223200
atttttttgt ggaaatgggg tttcaccatg ttgcctaagc tggtctcaaa ctcctgggct 223260
caagcaattt gattgccttg gcctaccaaa gtgctaggat aacggacttg agccactgtg 223320
cctagtcctg tcactttctg aaaaatccta tgaagcatta atagaagtaa aatcacactt 223380
aattatgcct gtttttaaat ctgcatattg tttgattaat cacatctttt ggccaagttg 223440
aaggcataat ttatatattg tctataagaa tcaataaact agtttctaaa tatgttttaa 223500
ataaataatt taaaaattaa tttctcaaat atcctcaact ataaagttag acctatttac 223560
tgtttcttgt aggagcctat tgctgtcaaa aatatctgaa gttctattca gagaaaaata 223620
gaaatgcaca atgactggga cttaagctga ggaaagtcag caacactgtt cccatcattc 223680
taccataaat gtggcaaaat cttattgttt gtgacagatc tatagaatta gatttcttta 223740
tatctaaaga aatatatata aatacataaa cgtaggtatg tatagcatga acagaataca 223800
gtgctatttg accattgtca caagacttat ttataaaacc tcctcaagct cttgccaata 223860
tcttagttaa gattgcatct aaggtgggag gaggatgaag attgaagaac tgtcaggtaa 223920
tgcttgatta cctgggtgac aaaattatgt ttacaccaaa cccccatgac acacagttta 223980
cccatgtaac aaacctacac atgtccccct tgaacctaaa ataaaagttg gaaagaaaaa 224040
aattggaaag aaaaaaaagg aacttaaaga gaagaaaaag attgtatcta gtagttttac 224100
taagactata aaatttctga cggctccact acctcctcaa gattgagaac taaacaacat 224160
gagaagcaca attacatgcc aaagattctt tttgctgtct tcatttgtag catcccagtg 224220
gaatgtcttt gaaaaaaatt ttttttatct gtattatctc acctgctctg agtattggtg 224280
acagtgatat atgcattgtg attagacgcc tagcctcctg aatataatgt atttccagta 224340
tattgtcctt ctgaatagcc tgtttacaat tctttgaaca ttgcctgcca ataatttagc 224400
aaattgttat tgaatgtcac agcttgatta tagaacatag taagaaatca acaaattgaa 224460
caccaaactg aatttaatgc taaaaattat ccttttaaaa aataatgtat tactaatttt 224520
tactcatgaa atttaacatt tccaatcatt gcttagttgt tgactcaaca ctcaaaaata 224580
atatatgcta cttttaaaat cccaaaacct ccagatttgt gtactgccac caggtgttga 224640
caggtgcctt gattcttcta gtttgtggtt ccagagataa ggtcagggtc ctgatacagc 224700
aaaaccacca tttgtctttg ttaaactgaa atgagaatgc gttccttatt ggaaagaaaa 224760
gttacacggt tttattatta tcatcagtac ttagagaaga cttaaaaagt acaacttaaa 224820
atgaactcaa agtttctgtg actaggaaat gatatgactc agggaattta atacttggga 224880

tccaaaacaa agaaaacaaa atgacccata aagaaccatg ttgtttatga gggcaaacta 224940
tagtaagcaa acaatattaa cttcaaatga gcactggaga aaaatcctga ttctttaaaa 225000
ttgtttttaa tattattctc ttaaacttag tgatacccaa aaaagaatca aaattttgtg 225060
gagaggaata taataactgg ttgtcttatc tgtattaaca ggattttgta aaagcattat 225120
tatttctgtc aattgatttt taaaacatct atcaactttt tttcttttgt gtatagatat 225180
caatgtatct catagattgc ttcccttttt taaattaaaa agtataaatc tttataaaaa 225240
ttaactataa ctttatgaaa gtatgtttaa acttgagcag attttgtaaa aatttaatgt 225300
gtagaacatg tgttgttttc agcaacagct taatttcttt agtcacttaa ccttttttag 225360
acttttgttt ttaaaaatat ttcttttttt cactctgaat catacctagg aatcaagtct 225420
ctctagcctt tgtaatatat tgaaaagact tgaaggccat tttgccttca aacttttttc 225480
caggagtttg tccttaaacc tctaacatct tataagtttc ctcatagttt ttcacatttc 225540
tgttttatgt gtgcttccaa ttaagatggt attcttctga tttcctattc ctgtgtcttc 225600
ctgctatgtg gagtcttcac agatttttag cagccaggcc aggagattgt attccttcct 225660
atattggcag gttggattcc ccctgaatta actcttccat tcagaggctg ccaccgttct 225720
agttcgtgtc ttgaagaatt gtatcaccct cctaactgat attttaacaa tagctgcaaa 225780
ctccttatta tatgacactt aaatagcata agagctcctt ttgcagcacc tgacatcttt 225840
tgaggtttat agatttgttc aaaatgaagg atgtacaaaa atattaaaaa tttaactttt 225900
taaaacttag attaagatgg ctttatgcca tggtatgttg ctatctactc ctcccattgt 225960
ctcttgagcc cactgcattc cagctttagt ccctatcctc ggaaactgct gtagtcagtc 226020
tgtgactttt gtgttgccaa attcagttgt cggttctaag gtgtcagttt tacttaatct 226080
gtcagcagta tttgacagag ttggtctttc ttcctagaaa cacattcatt tgcctgccgg 226140
atatttcctg ccttactggc tgctgaattc tagtctcctt tctacatcct catctatttg 226200
gtcatctaga aatgtcctag ggctttggcc tcttctaggc gtcactccca aagtggtatt 226260
ctgcagtcat ggagaatgac acatctgtag gcatatgact cccaagtttc tcagcacctg 226320
atgtgatgta tacttatttg ttcattccct ctctcctttc tcctaaatat aaactctgtg 226380
agaataggga ctttgttcct gttcaccact gtgtctgcaa tgcatagaac agtatctaac 226440
acgtaatata tactcatact ttacattgaa tgatttctta atttatgggt cattcatcat 226500
taatcttgat cataataatg aatcagccga aaacagcact ttttttaaga aaagtgtgtg 226560
ttatgtctag tttatttcaa taatactaag acaactatct ccttactacc tgaatttatt 226620
tcagcacaac cgattaattt taattgctat gagttaaaaa ctttaaatac agtgagtttt 226680
tttaaattca gatagagatg ttttttagct tactgctttt aaaagactgc aatttaaagg 226740
ttctgttaat tcttcaaaaa aagataaatt aagtgcttca ttttcctgca tagtaaatca 226800
ttgacgttcc taacattttt tcaataactt ataaattttc tttgctttgt ttgtgttgtg 226860
acacgttgtg taaatgagat ttgatatcag aagtaggatc tacaaaacaa acactaaata 226920

ctggggttag tactaacgtt ttcactgaag atggacccag aaaagtagcc aaacacagtg 226980
tcaccccgag tccattctga gatacatact ggaaaattat agtaatcggt tcattcagga 227040
aaccaagtga caataggtta agccatgagt aatataagta atataagtaa catttcttta 227100
tcttccacta ttaaaaaaaa acttgtaaat ctttatttaa atgaacacca aattaaagaa 227160
aactgagcca aagaaatgta agtctaaaat gaaatgctca attttgattt atcagagata 227220
taagccttac taagttttaa gttaacaaat ttggacctta acacatatta atctaaatta 227280
attaattcga cctttgtttt cagttttaga gaaatgtaaa cttgttcaaa aatatagcag 227340
aatataaact atttaaaata acagtttata cttgcatacc atttgaaatg ctttgttttt 227400
gcttgtgttc tctgctaaat tttttttcca ttgaatctgt gctgcttctt tgccaatgtt 227460
ttccactgta gaatctccat ctcgcaacct tgcttctcgt gagcgcattt acaaaaatta 227520
tggtgtagct gggcctgcct ctgctctctc atctctgtct cacaaactga agggtgggta 227580
tacatgcatt catggatggg ccattcttaa tgaacataag gagtgtcatt aaagatgttt 227640
ccgttcgtct cagtcatgtt catgccattc cattttttta attaaagttt atcattttat 227700
ttacatttat atatgtttga gtaatttatg atgctcctat tcagtattat aatatagatt 227760
atcccagtag ctatgcatgt ttattacatc tataggttaa acattactct tacttaattt 227820
taaaacttta tgagatcatt aaaattaaaa taggtataag gaaagaatta cctaattggg 227880
ggaatttttg aaatcatttt tatcagaaaa agatgatata tgtgcaaggc ataaagcaca 227940
ttttttagtc ttaaacttat tgttatataa tataaaaatc attttactat tttagtcttt 228000
tatgagaaac actgataatg aacttaaagt ttttaagttt aaattagtta ttaggactca 228060
tgtgtctatt tatatttata tgtaaaccta cctgtcctat ctggcataaa tttttctaat 228120
attgttttcc tagtgactaa gttttaatat tttaaactta gaaatgttta aactattgac 228180
aattgacaaa atttcagaca atttaaaaat atgtttttgg ttttttgaaa cactacagat 228240
gcctacttaa atattttgtc ttaaagtatt atttaattaa atttaaatct tgtaaatctt 228300
ttaaaatctt gtatttaaat aaaattttat caaaaagaac ttttacagca atgcctaaca 228360
tattgtataa gtttttcaac taattactga ttttcttttt ttcttagaaa tagataaaac 228420
agctatatgt ttctcattat acaattgaca gtataaacat ataaatagga aatataaaaa 228480
tatactaaat agtattgtac aacctgtgct atatagtata tagtattgtg tgtaatagca 228540
tatagtatca tgtaacctga tatatgctat atatcatgcc atgtgatata tgcatatagt 228600
attatgatat agtatgatcc tgataattta agatttgggc atgaaaaaat gtatgggtca 228660
tttagtgaat gaatgcttac catttaacat gtttagtaac atttaacttg cttgccctaa 228720
gtttctatcc tgtcattatt atttcttcag gtttccttta aagttagaaa aatgtttgca 228780
tattatcatt taaatgtaaa agtacagtca ttaaataaat taggagatga atgtgaaagt 228840
attacgtata gtctgaagat ttttagcaaa ctgtttatat ttgttggtat tttgagcagc 228900
cacaacatac aatttttagt aatatttaat aacatgctgc aaaacatttg aaacacaaat 228960

gaagcagata aacataaatt agcacagggg cttaccccac ttaacaagca tagttttttt 229020
taatatataa aatacgtttt ggaaaaataa atttttcaaa aagatttagt tatcatttga 229080
gaaatacatc tgaaaaagaa atatttctgc cattatttgt atgcctaaac ttcaacaggt 229140
atttagctgt caccacttac agatcttaag aaaatattca ccactattag agatataata 229200
agatatacaa atgcctattc tttctaacac tatgtaataa acttttttgc tcacatatga 229260
caagtggtgt tcctggtatt tggcagattc catttttcag gcaaccacag aggttgtttt 229320
catgatgagt aaataaccta ccattgtaag ttcatatgca ttcctagaat agaggtagca 229380
tcagcaggta ttcaccattc ctttgcatgc agctgttgtg caggtcaacc actaagacac 229440
atctgacaca gggagtaggt aatctttgtc acgaaccctt cctatttgtt agaaatccta 229500
cacaagttgt ccttttttat aaattagaga gaaaacctct ttcagtttac agatggattt 229560
tttggagaaa gggataccaa atataaaaga cttactaatt gtttgtctag attcccatta 229620
tgtgtggttc agtttctgaa ataaatgtga tgataaaggt gataaacatg tctccagctg 229680
tcagattaat aacaaaaaac aagtattcca aacatcacat actcaaaatt aatggccatg 229740
actactgatt taatctacca aatataattt gtttaccaaa tttgccatat gctgttacta 229800
ggtcagtaag attagatggg tagcaatttt ttgaactact tttgaacagt ttaagggtac 229860
ttattcatag atcacatctg tttatctgtg ctctcccaag acagcattga tagttagaat 229920
tctaaattgg gattttttta accctttttt caaaacttag ccccatctgt taatactgta 229980
tttataaaac agagcacata atatgataaa ggaggttaat gattgaataa tgaagctttg 230040
caagaaacta aaaagcatta tgttttagtt acttttattc atccaaagaa caggaaaaat 230100
tatcttaaat attggcatgt tgcaattaaa catttagttc atcagtgtgc cctgtataaa 230160
gtacttgcca tactctatta tttcaccatg taattactca gtttgcaagc atctaaccct 230220
tgcacatcaa taactaatgt cacctctgtc ctgtggcctt gggagcctta attgtaaaaa 230280
gtagacttct ggagaagctg aatacttggg agtagcagaa gaaaaaaatc aagaaaattg 230340
gaatatatta ctcattcaac acatatttat tgaataccta ctgtattcct ggattcattc 230400
ttggtattac atcagtgaag agtaacagac aaaaatacct gcccttgaca aagactgggg 230460
cagattctcc agcactactg tcctgagcca tagagaaata gagcgtgact atacacactt 230520
aacacatgca cggaagcata aaaaacattt tcctacatca tctgaatttg ttcacatcct 230580
tttttctttt aaaaaataat aacatgcagt tatagccagt taacaaatct accttaattt 230640
cctaggaact gtctttgaaa aaatataaat acttccagat aggtttatgg attttcagtt 230700
ttttatacca cttgaaaccc ttcagtcaga ttgcttttct cctaacaact aactggtatc 230760
acttgcacta agagtagaag tttttatttt aaggaggaaa ggtgatgttg aagtttgtac 230820
catttacttg ggtcacagac agcctggctc taatgcctca aagaaaagca taaaatagaa 230880
tgaaggagag acactgaatg aaggcagggt gctaaaaaca attaatgtcc atgcagaaac 230940
atctaatgct ttgccttaga aaagggaggt gattcttctt ttcatcctgt ttttctgtca 231000

ttctcctgac tccttgtttt gagggtccac agttcagact tttaccactc attctgagga 231060
tccaccgttt ctgtggtttc caagggaaca caggctctga gttctaagca gctaagcttc 231120
ctgaactttt ggagtgtaac ctgtttataa ataattaaat aacagcttca gggcattcat 231180
gtgctcattg taaactagtc taatctgatc ataaaacaga gtgcctctta agcaactctt 231240
tgttagccta attgtatgca tacacttaaa aattataatt gtgtctatgc ataaattcag 231300
ttctctaatt caacaaaacc cataccgttg ccctaaggat gcgaggtatc actctgctgg 231360
aaatttagct ctactctaca tttaaaatac aagttttcac attaaataat ttcttattcc 231420
ccaaaagatt aatttctgtc tctttctagt ccctcactga atttaaattc taactagtta 231480
ttgcactagg gcagaggttg agatatcact gatagttcac actggatttg ttcaacctgt 231540
tgttttgcgt ccatgaaaat agtttttggt ggtaatgttg tgtaatgatg tgtagaaatt 231600
ttattagaaa tatttccccc ttctttcctt tattcttcaa gtattttagt atgtatgaca 231660
aacaatataa attttgtagc tatgtttttt tatttcagtg tttagtggct aaattatcag 231720
taagttataa ttttattaat aagtttcact gtaaaatgga ccatacaggt gatcgaggaa 231780
acatctcaac atcttctaaa ccagcctcta catcaggaaa atcagagctg tcctctaaac 231840
acagcagatc gcttaaacct gatggacgta tgagccggac tactgctgat cagaagaagc 231900
caaggggcac agaaagttta tctgctagtg aatccctcat cttaaaatct gatgctgcaa 231960
agttgaggtc agattcccac agtaggtcat tatcccccaa ccataacacc ttgcagacat 232020
tgaaatctga tgggaggatg ccttctagct ccagagctga atccccagga ccaggttctc 232080
ggttgtcatc tcctaagcca aagactctcc cagccaatag gtctagccca tcgggtgcta 232140
gttctccacg ctcctcctca ccacatgata aaaatctacc tcaaaaaagt actgctcctg 232200
ttaagacaaa gcttgatcct cctcgggaac gttctaaatc agactcttac acacttgatc 232260
cagataccct ccgcaagaag aaaatgcccc tcacagaacc tttgagagga cggtcaacgt 232320
caccaaaacc aaaatcagta ccaaaggatt ctacagattc ccctggatct gaaaatagag 232380
ctccctctcc ccatgtggta caggaaaacc tccacagtga ggtggtcgaa gtctgcacct 232440
caagtacttt aaaaacaaat agtctaacag acagcacctg cgatgacagc agtgaattta 232500
agagtgtgga tgaaggttca aataaagttc attttagcat tggaaaagca ccactgaaag 232560
atgaacagga aatgagagca tctcccaaaa taagtcgaaa atgtgctaat agacacacca 232620
ggcccaaaaa agaaaaatcg agtttctttt tcaaaggaga tggatccaag cctttagagc 232680
cagccaagca agccatgtct ccttctgtgg ccgaatgtgc cagagctgtg tttgcttcct 232740
tcctctggca tgaaggcata gtacatgatg caatggcttg ttcttctttc ctaaagtttc 232800
atcctgaact ttccaaagaa catgctccta taaggagtag tttaaatagc caacaaccta 232860
cagaggaaaa agaaaccaag ttaaaaaata gacattcatt agaaatatca tctgcactga 232920
atatgtttaa tattgcaccc catggaccag atatatctaa gatgggtagc atcaacaaaa 232980
acaaggtatt gtctatgctt aaggaaccac ctctgcatga aaaatgtgag gatgggaaaa 233040

ccgagaccac ttttgaaatg tccatgcata acacaatgaa gtctaagtct cctcttccct 233100
taactttaca acatttagtg gctttttggg aagacatctc tttggctact atcaaagctg 233160
cttcccagaa tatgattttt ccaagtcctg gttcctgtgc agttcttaaa aagaaagagt 233220
gtgagaaaga gaataagaag tccaaaaagg aaaaaaagaa aaaagaaaag gcagaagtta 233280
ggcccagggg taatttgttt ggagagatgg cccagctggc agtaggagga ccagagaaag 233340
ataccatctg tgaactgtgt ggggagtcac atccataccc ggtgacctat cacatgagac 233400
aagctcaccc aggtaaatga tactgttgaa tgtacgtata agtgcttttt tctttaatga 233460
accagatcac tttatttgag ctgttctatg aattttgtca aaggataggg aatttatagt 233520
atcttagaat gtcattttta aagtacatta tcagtacagg gttttgtgaa aaacagataa 233580
attctaatat tatgatggcc aggtcattta gtgctatttc actttcatgt agcatgtttt 233640
aaaaaatatt atgtgttacc caaagggtgt gccattaatg tgtctgtctc acacagcaga 233700
aacagtggca tgcccataca cacagggcat ctcctttgat tttactattt tcctgttcag 233760
aattgcaaat taaattagat tagattgatt tctataggct ttttcctacg gaagtgatat 233820
tgttttatca gaagactgca ttttcagtaa atgtaatgat ttctgtaaca ttgactaata 233880
ttccaaattc acctaataca aatttggtta atttttttct ttgtctacat attatacctc 233940
aaatgtctta tgaatatgtg atttctgaaa atatatcatt gttatagaac ttttatactt 234000
gtattgtcta attttattga cataattaag tatatctaaa tatacctata ttctatctct 234060
cttttaatat gcctgattag aaactaagta tctctcatat ttggggcatc atttaattga 234120
ttgctttttg atcaagtaag ctatttttct gatatagtta atagcctgaa aaataagata 234180
tctacttcag tattttgctt tctttgagaa ggtaaatctg tcattaatgt atgagtaata 234240
aggatcaaac tattaaggac acttggagtg ttaaatattt atttttggta ttgtttcaag 234300
gttgtggccg atatgctggt ggacaaggtt acaatagcat tgggcatttt tgtggaggat 234360
gggctggtaa ctgtggtgat ggtggcatag gaggaagcac ttggtatctg gtatgtgatc 234420
gctgtagaga aaaatacctc cgcgaaaaac aggctgctgc aagggagaag gtattttatt 234480
ccatttggag ctatactttt aatgatatgg atacactgta taaaaatacc ttgttataat 234540
cttactacat cagataattc cacatagttg atattaaaag atcagctttt ataatggaaa 234600
tcatatacaa atttaatatt tgtactaagt caaatgcttt taattttaaa aattaactat 234660
atattggaat aacactagat aacctctgct acatatgcct tattgacaaa tatttgttgt 234720
gtaactcttt attattaatt gtctgttatt ctctctatgg agagctttcc ttttcacctt 234780
ctttctcttt tcctcttttt ttggctcctt tgttttccta ttccatatct actctctctt 234840
ttctttattc actctagtgg cacctaacta aaaaatttca ctgaatacac gcactgaaat 234900
gatttacttt ttaaaaaaat gaattacatt tagatttaac tagttaggtg ttaaattttt 234960
ctgaagaagg aaaggaagta tagtttgtgt ttgatacaaa attgtcttgg tcagcccagg 235020
atgaatatga aatgaatgtt gttttttata atcttattat cttaaaatag ctttacaagt 235080

gtttaataat ttttattgga aactcaaaat acttagctta agaaactaag gattttaatg 235140
taagtcagaa aagattagtt tgattttttt cttcgccagg tcaaacaatc taggagaaaa 235200
ccaatgcaag tcaagacccc tcgtgccttg cccaccatgg aagctcacca ggtgattaaa 235260
gccaatgcac tcttcctgct gtccctgagc agtgcagcag aaccgagcat tctgtgttac 235320
catcctgcaa agccattcca atctcagttg cccagtgtaa aagaaggcat ttctgaggat 235380
cttcctgtga aaatgccttg tctctacctg cagacattag ctaggtaata aaattttgct 235440
gttgtttttt aatcaccttt ggattaacag cgattgttta ttgatatcta gggtagttcg 235500
gtatttgacc tacactttat tagctattgt gtactataaa caaacttcca ggcatttatt 235560
ttttgagttg ccttgactga cataatgact ttatggtaat aggtatttaa aaaactataa 235620
aaatgcttca tcaatttgca ttgtattaat acacaacatg tcttagaaaa taagactata 235680
gctatctttt actctttggt aggctgactt cactctgtta gtgcgaaatg ctgaagtgta 235740
aagaaagtgt gtttccttag cagtctttta tttcgtaaat tattcattct gttagcattt 235800
aaatgaatac caactgggca cctaccagcc ttgtgctagg ccctgaatgt atgaatataa 235860
caaaatcctg gcttctatgt actcatactc ggatgtcaag tcagacatgt tagtgaaaca 235920
tttgaaatgc catgtgagga gtgctacaat agaagtatgt atgtaagatg ctgttagagc 235980
ataaaagatg gagagtatct gaatcttccc caggaaatca gaaaaggctt cccagagggt 236040
ctgagccttt agtagagtct caaaggataa atagaaatct gtcaggcaag cgacaggaag 236100
aaagcgactc ttggaagaat aacccaggtg agtaaagaca caaatgaaat ctcattacac 236160
atttcaggaa tcacgggttg ttaaaggact ggctactgga ggaaatatgt gaaagcccgt 236220
tcaaagtgat tggttcaccc aagtaatgtc gggctctgta gcccaggtta agattgcatt 236280
ttatcccatg agcagaggaa acagtggaag aattttaaac agagaaatac atgaactggt 236340
ttgcaattaa aaaatcactg tggaatgtgg aagctaatgt ggagactaga taaaagtgca 236400
ttactggaga tagaaacctg ttagtattct gttttcatat attggacctc agtggtgcag 236460
cctgtacttt cacacatcat gcactgacac tccaaaagag cctttcatat accaaaacca 236520
aatttttttc atcagctgac aaatgaaggt aatctagata ttgcccactg acatggcttg 236580
ccgaccaaca gttccatttt ggaaattgtc ctctttgtgt agcagagttg tatggttttt 236640
aaaattttgt gcttactatt acaataaaat gaataagcaa caaaaatgaa aatatcagac 236700
taagtacatt tgagactgag gtggaaatca tcagtatgcc aaaatacaat agatctttag 236760
tgtgaatcac accactcagt ggacttaggt tagcaactat ttgttcattt tgaaaaggaa 236820
agaatcaaat tgtaaaacgt gaaaaatgct aacacagcat aattatagat tttttctttt 236880
ttagctgtct ggggaatggg caccccttcc accctgaacc acagaaaatt tcatatatct 236940
aatatttttc aaatatgatc ttatgtatgt tcctacccaa aaaagtaact gcttgtattt 237000
cagtggtctg tgcaagaaat aataagcatg aaccaaggca gtgatgatag ggaatgagaa 237060
gagagaatgg ctctgagagg aaacctaata tcatgggctt gaaagatgat gatgatagag 237120

ggctaagcct actacaacac tggaaatatt ttagcttaag caactggatg gttggtgggc 237180
ttattcacca aaacactgga agatggaaaa gttctcaaaa ggaaagaaac tgagttcaat 237240
tttggatttg aaatgttcgt ggtacctgga atgttgatga ataaaattat cagactttta 237300
ttttcaccta cctacatcta cgattatttt aatgctttgc ctttaactat gatttacatg 237360
tgctgctttt cctgttatga aagagattaa aaatggagag gatctgtatt gccatcatgc 237420
tttaagttat tgggtttgat ttaatgcagg catcatcatg aaaattttgt gggctatcaa 237480
gatgacaatc tattccagga tgaaatgaga tatctacgtt caacatctgt acctgccccg 237540
tatatatcag taactcctga tgcaagtcct aatgtatttg aagagccaga gagcaatatg 237600
aagtctatgc caccaaggta ttttagttct ctcctgtctg ttgaatagct agtgtttggt 237660
ggaagaaaga ctttgaacta gaaaagagtc ctgtagaagt tctttaatac atctgtgagc 237720
cagttttgct aactgctgga gcagtaagaa aaagcataca tgtatgacta aggtaaggtc 237780
cttgatggga gaggcagact agttggagag aagagatcaa ctagtgaagg aattaaagat 237840
tacattatat gacattactt aatgctgtag taaatcagag tagggaaggg ttactttgag 237900
ttttcatggg agcagtggca ttgaaaataa atcttaaact tcttggatta ttgtttacag 237960
tgcctcctaa cttaggtatg gtagtatcag agtaaagagc ttctcggaaa gctgaataga 238020
aacttgttac aataatgtaa tacacaagaa aatagaggcc aggcgctgtg gctcacacct 238080
gtaatcccag cactttggga ggccaaggcg ggtggatcac ctgaggtcag gagtttgaga 238140
ccagcctgac caacatggtg aaaccctgtc tttactaaaa atacaaaaaa ttagccgagc 238200
atggtgacac acacctgtaa tcccagctac tcgggaggct gaggcaggag aattacttga 238260
acctgggagg cagaggttgc agtgagctga gattgcacca ctgaactcca gcttgaggaa 238320
aaaaaaaaat agagtaccac tgaaactttt ttagaggacg aatgatgtga taaaaatgca 238380
aaggtagtct cacagtgaca attgagaagc tgacatatgg attaaagaga tatattttgt 238440
aggaaaaata agtgtgactt gttgactaac tggataatga aaaagataag tttaagatga 238500
catgaagatt tcagtcacga ataactgtga acatggtatc attggaaagg aagacatttg 238560
ctgaatgaaa agagaatgtg cttattaaaa gagcatgcca ctgtgttact actatacaac 238620
tataattata aactaatata ataaatactt taatgagcgc aatgggacat atatatccaa 238680
atgaggtttt tttttttttt cattaaaaat acatgcaact tctcccctcc ccaactgtgt 238740
ggattactcg tcagaatttc tttgaagctg ctcttgacat tttctccaga gtgcagtgca 238800
accttttata tctttaaaag tgataaagta tttctaaagg agatacactt tttaaaagag 238860
ttagaaactg ttttgaggct agtctgacaa gtgtgattct cattgggtta ctattacccc 238920
ttaggatcaa aatccaggag gctgaggcaa gaggatggct tcagcctagg attttgaggt 238980
tacagtgagc tacgattgtg tcactgcact tcagcctagg tgacagagca agaccccttc 239040
tatttaaaaa aaaaaagttg atgaataaat gaattcatta aatcagtggt tctcaattga 239100
gggtgatatt tgcctctctg ttgacatctg gaaatatttg gaaataactt tgtcacaact 239160

gggctagttg agtgggcatc ctagtgagtt gaggccaggg atgttgctaa ccatcctaca 239220
ggaggcaaga cagcctaaca aggaattacc tggctcaggg tagccattca attaaagatg 239280
tcatctttgc tttcttcttt ggattttctt gtgttgcttg aattctttca caagcaattt 239340
tttttttttt ttggtaaaaa gtcacttttt aaatttgtgt aatttttttt aagttagtag 239400
aaaacttcct ctgaaaacag aacaaatgtc tgtgctttat agtaacacca tatataaagt 239460
gaattaagtt aatgtttccc tgtattctag tcattctgga accaagcaac ctatactaac 239520
acccagggac atttactgcc aatcagttat gtacagtatt tatagtctat tcaaacaaga 239580
atgtacataa gcttagagaa gtggtgagta aagaaaacac ctaggcagtt agccgtctat 239640
tagaaaatag ataattttac caagggacac aggatttcaa acctggcttg aatgatggaa 239700
aaaactccag aaccaacggt gagcaaaaat attccaattt gaattcaggt gacagaaaaa 239760
ggagctcacg aaaagaatag tttctaaatt tgttttagag aaataagaaa gagaagggat 239820
ttctcttttt ttataaaatg tgtcaataaa ggaaattgta tagctctctt aataactttg 239880
taagtatttt taaatgttgc tttaataaat agaaaagctg ttagtttact taagcctgaa 239940
taaaaccatt aagttctttt atagagtgta aatacaacac tggtgccaaa aatcagtttg 240000
ttacctctca tgacaccttg atgcagccat cctccaaggg ctttgggcaa catttcccaa 240060
gagtgagaaa cctcacagaa cactgaagac accaaaagaa aactttggaa ccaaattttg 240120
cttcctgttt tattaggttt tcatgtgtac atatccattt tctttctatt tggtgcactt 240180
acgattatat ctgactgttt ttcctgaata ttatgataca tgaattccac cccagtgctt 240240
ttgattctaa aaggagcttg gaaacctttc ttctttttca tttaaaatac aaagtagatt 240300
taagaaaacc ataagtttta aggtacaatg ctgaaatcag tcaaaatgtt actgcatttg 240360
aaaagatcaa attatgattt aaagagagat tatcaatttg gctttgattt tgtttctcca 240420
catgaaatat tgagtcacat attttgcatc attataagta tagttacatt tcattcagct 240480
cttctgagtt tgtatctgtt gcattgttcc atgtacagtt tagaaaccag tcccataact 240540
gatactgatc ttgcaaagag aactgtcttc caaagatcat actcagttgt tgcttccgaa 240600
tatgataaac aacactccat tttacctgca cgagttaaag ctattcctag aagaagagtt 240660
aacagtggag acactggtaa gtatgaggat aaaaaagaat attgagtgat ctgactacta 240720
caaataaaaa aattgtttta tttgcatata atttttaaaa tttaattttt gaaggaaaag 240780
gcatgggata atttctaatg tgtgattgac attccttcta tatgacatag ctataaatgt 240840
gcatctctaa attttgcatc ttagttgaag ggaaatctgg agatcattca gtgattgttc 240900
gagaataaat tgacctttac aatcttactt gttaggtatc taagccaaat gttacaacca 240960
ttatcccttc cactaaactt ttgtggacca ggagttccaa tgacgtacac attcacagtt 241020
atctctccac cccaactgaa gcttgataac tcttggagct gccatcattc taagtagata 241080
ataaaacttt ggggatataa atagccctta atcgatttaa aaaaaaaaaa aaaacagtta 241140
catgatctgg accagttggt atcaacagcc ccaaattgga gctggggatt ctacctcaag 241200

aataatttta aaaacccaag aagaacaggg taatatagtg atcaattatc agagagatca 241260
cttgctaaaa tgaagatttg gcctaaaaat tatagtcaga tgattaagtt atcttactga 241320
agacttttaa gaaaaacatt attgaagatt gtccagcaat ttataggttc attcaacaaa 241380
tatttttgac ctcctgttaa gaaatgtttc ttataaagcc atcttttaga aaatgatgca 241440
gatgagatgt agatttcatt tagagaaaat ataaatcact tagcttttag agtgttttag 241500
aaaaggtgaa ctttagattc agctctgatt caaaacccac ctctacaact taatagctat 241560
atgactttca gagagtaaca tatttaatct ttctgaacct tggtttcctc agtaaaagtt 241620
gaaattgtta tgatttgtgt catgttcaaa aaaccaatga caaaggcttt aagtgaatat 241680
attagatatt attatcttaa atatatatgc actgcctatg aaaattaata attttctttt 241740
tctttaatag aagttggttc ttcccttttg agacatccgt ctcctgagct ttctcggcta 241800
atctcagccc acagctctct ttctaaagga gaacgaaatt tccagtggcc agttttagct 241860
tttgttatac aacatcatga tctagaaggt cttgaaatag caatgaaaca ggccctaagg 241920
aaatctgctt gtcgagtttt tgctatggag gtatgaagac attaatgaaa ttactgattc 241980
attgattata caaatcatgt gatgattcta tttcacgatg ccactttttt accaacagaa 242040
attgtgtgaa attaaaaggc attagccaat taaaagtatt aagtgttcct ataaaaatca 242100
gactctaaag tttatgaata taaatgaatt tagaatttca tatttaagca ccctaaagaa 242160
atttatttct gtattgcttt actatttgaa tcttgagatt atcttttttt taaaagcttt 242220
ttatactcag aatgtcattt tattagaatg aaatgacaga gtaatcatgg aaagaagata 242280
aggagtaggg cttttgagtg gtaagttgac cagtaataac ttctctgggc ctttggtctc 242340
tggaaataat aggattactg tatattacag gcctatatat agtaaatctg tgtttttatc 242400
aaatgtgata aataacctac cacttcatgg atgggaatta tatgaatata tttaagtaaa 242460
tggttgcttg cctagagctt gagatacact ttgtgttcat ccagatgcaa gttggaaaaa 242520
atgtgcttga ataaattaat attaaaatga attttcaaag taaaatgtga tttgaaaaaa 242580
tattttggta ttgtatctta taaattattt ttcctgtttt ttttaaatta aaaccccaaa 242640
ttattaactg tttcaagttc cgtgtgcttt cagtgaaata aaggtcagat tttcaaggtg 242700
ccaagcatag tagcccatgc ttgtaatcct agcactttgg gaggcggagg agggaggatc 242760
acttgagcgc aagagtttga gaccagcgtg ggcaacatgt gagacctcat ttctacaaaa 242820
aaaaagtttt aaaatcagct gggtgtggtg gtacacacct gtagtcccag ctacttggga 242880
ggctgaggtg ggaggatcat ttgtgcctgg gtgcttgagg ctgcagtgag ccatgatcac 242940
accactgtac tccaacctgg gtgacagagc gagacactgt ctcttaaaaa gaaaaacaaa 243000
tagtaaaact ataaggcatt ttttttaatt aaaaaagatt ttctagtatc tttctttgaa 243060
gtcttaatcc atggaggtac tttatttctg agtacttttt aaactcatgt attttattct 243120
tgaactcatt taaccgtcat ttcttcaggc tttcaactgg cttctgtgta atgtcatcca 243180
aaccacttct ctccatgata ttctgtggca ttttgtggca tcactgactc ctgcaccagt 243240

ggaaccagag gaagaagagg atgaagaaaa taaaacaagc aaagaaaatt cagaacaagt 243300
aaatgaaatt tttgttttga ttgtgcatat ttttatagaa actggtagta taccttcaaa 243360
tagtccaata atttgcatga aatcattcgc agatctaact tgaaagcaaa aagaatattt 243420
tagtgctatg ggcaaagtat taataatgag acctggattt tatagaaggc agcctgtata 243480
atggagagag cattgaatta gggcactagg agcctgagtt cagcctcatt tctaccctat 243540
tgtagttgta tgacttgtag gcctcggttt ccatgtctgt aaaatgaaag taatctaggt 243600
gacatttatc ttattcccag tcactctgaa attatgtgaa tggcatttta ggttaatttc 243660
caaggatatc aagcaacatt atcatagtca aattcttaaa actcacaaac aagacaccat 243720
gagcaagaac taacaaaaac aatagacagt agaaatagac cctcaacaat ggttgtaatt 243780
atctatgtaa atgtagaata atgtgttaat atgcttaaag aaataaaaga gaaactttaa 243840
aatatgagaa gaaattgtaa gaagaaatat gaagtgatga atttgataaa gaactaaaga 243900
aaacataaat actaatctaa gtataattga tatttttagt tttgagattt tatagcatgt 243960
cagtgaaaaa attggctaaa tataagagaa tgatggattc gataaagaac taaagaaaat 244020
acgtaaaaat taatctaagt ataattgata tttaaaaaac aatcagttgg acattttata 244080
gcatgtcagc aaacaaattg gttaaatata agagacctgt agaaattatc tagacttcaa 244140
cacagaaaaa gtaaataaaa aatacaagtt gctaagagca atgaagggca aaatgatgta 244200
caacttacct acttggagtt tcagaaggag aagacagaat ggaagagaga cagtgtttaa 244260
gagttaatac ttgagaattt cccagaattg attacagata acaacccatt gagtcaagaa 244320
gcccagtaaa ccccaagcag aataaataca aatcatggtg gacaaattat agtgaatcac 244380
ctgaacacca aaaacaaaga aagagatctt ttaagcagcc acagaaaaaa atattagctt 244440
tcaaaagagg gacagtgaga ataattgctt aatttttggt agcaacatta caagttaaaa 244500
tactgagttt gtttaaaagg ctgtcagttg gtttttttca acgtgctgaa agaaaatact 244560
tgttaaccca gttgaagggt ctttgaagaa caagaccaaa gacatttcca gaataaaaac 244620
tgggagaaat ggccatggag tatcaatgaa gagaaaagaa agtgtaaata tgagggcata 244680
tcttaaaaaa cattgactat gtaaaaagta atgatatgtc atggtgatgt acaaaatgat 244740
aaaagatcaa tcctggatat gtatctacct aatactgtaa ccttaaaaaa agtaacaatt 244800
gacagactgc aaggaaaaat aggacagtct acagttacaa tgtgagattg tcatatacct 244860
gtaacagcta tctgttcctg cataactacc ccagaactca ctggactaaa acagttgttt 244920
atttgctgac agtctgtggg ttggcaattg cagcaaggct ccactgagag gaatgactct 244980
ttcctgtttt ggttacatag tgtgcttcaa gtggtacccc ctgagctctt ttatcctcca 245040
gaatgctggc ctaggtttgt tcagatgcca gaagcattca agaaagagag cacagaagct 245100
gcaaagccaa aatggcgaac actaaactca tatgccatgc ttctgccacc ttccattagc 245160
tagtataatt cacaaggccg gtcaaattca aagactgaga aataaactcc acctgttgat 245220
ggaaagagcg acagagaatt tgtggctgtt ttgtgtatag taccataaca ctctttttag 245280

tactggatag gtcaggcaga gaaaaagaat cagaaagtac acagatttga atgattgaca 245340
gtcttgatag aatatacaaa acattgcata tagttgttta cattcttttc agatacacac 245400
aaatgtctat ggaaattgac tgtttactgg acacataaag caagttttaa caaacttgaa 245460
ataattggtt ttcttcccac catgtcctca gtctttattg ctagtatata agaattagat 245520
aacaaaacaa taacaagaaa aatatttata gggaaattaa gtaacgcttc caaatgagtt 245580
gcatcaaaga agaattataa cagaaaatat tttaaatgga atggcaataa tagtacatct 245640
caaaacttgt tgtttggcag agttctagtt atagttacga tgaaacagcc agtgtcagca 245700
ttagttctta tagtaagtaa caaaaagctg ggcaaaatat ttaaaacaat tgttcatagg 245760
ctctgaggag tgaccagtac agggctgtaa tccttgagag aagaaaaggg catgaggtaa 245820
gccccacaat tgggaaaaga gcccaagcag agtacgtcag ttgtgttggc agagatcaaa 245880
gtttaggact gtaactttgg gatgtgggaa gcagggcact agtggtacag agctgcagaa 245940
agggagtcta aaatttttgc ataaaaatat ccctagggtc ctggccactc ttagactggg 246000
catgcaattt ctctggagga tctagcagtg gaaatgaatt gtaagctgag aactgaacag 246060
attcttagct tcagagctgg gttctgaagt tttagtccaa ccagaggtgg ggtgagcgtg 246120
atgaatactc ctggcatgcc cttgaaaccc cagaaagacc atactcaagg aatacagacc 246180
atgccttagg agtaagggca gtgcctaaaa tcaagggcaa aactgaaata accccagtct 246240
aacaaagtcg aatgccaagc cttgatggaa tctagatggc ctgctatctc cctgtaagaa 246300
caaaactaca ttctttagaa gacaatataa accagaactt ctatatcagt agaagataat 246360
ataaaccaga acttctatat cagtaatcaa tatatgcaca aagaaggagg caaaaagcaa 246420
ccatctttta cttagctcag tgacatagtt taaaaagttt gatgggctta cattggtagc 246480
aagatggaag aacaaacatt ctttgctgtg gagtagcagc ttcttagaga atggtttggt 246540
aagatctatc aaaattcaca ttaaaaatca ttcagtaatt ccacttttat gaagttctcc 246600
ttatcatata tgcatgtgtg aaaagacaga tatacaagtt gattcattgc agaatgttat 246660
ataaaaacaa aagattggaa acaacctaaa tgactgttct gaagtggact gatgaaataa 246720
actatggtat ttttattcat cagaatacta tgcagtggga taatatatgt tacatacata 246780
tctaataagt aataatctca aagataaatt attgttaaaa gaacacagat cagtttgttt 246840
ccagtgctgc catttgtgta aatgaacaat atttatagac agtccaagaa actggtaata 246900
gaaattgcca gtgggagggc atctaggtgg ctgggctgta gagacaggga gactttttat 246960
ggtatttttt tatacctctt gaatatttta ttatgatgtg tgttatctac tcaaaataat 247020
aaaaagttat tccatagtta atattattaa tggagccatc tgagggttgt aatgagaaat 247080
gaaattcaac ccctacttct ctttcctagc tgtggtccat ttgcgtagaa aggagctttt 247140
ttttctaatt cacataaaaa tgctgtatga gttagtgacc accccaactt ctaaacatat 247200
gttatttttt atacataaac atacacacaa aacataccta catatatgta tgtgtatata 247260
tccatacaca tacatatata tgcacacaca ctacacatac acatgcatac acatgtggat 247320

tcaccctaca ggtatctttg tgagtacact agaattgata catttattca tttgcatgct 247380
gattttgctt gtgactaaac ttacagcttt tttccctaaa ataatacaag cataattagt 247440
taaaattact gtatctttct gggtacaatt tggttttgtt gtttgtattt ctgttacatg 247500
accatttttt aagtatacat aatagtcttt tgtgaagagg attccttcca gcacacaaac 247560
tgataaactt gtttcaattt ctgccttagg agaaagatac aagagtatgt gaacatccac 247620
tctcagacat agtgattgcc ggggaagctg ctcatccttt accacacacc tttcaccgct 247680
tgctgcagac catctcagac cttatgatgt ctctccccag cggcagttca ttacagcaaa 247740
tggccctgag gtaattttgg tatccacata tcctaggtac attggacaag agagtttatg 247800
gaatattcaa agtatgaaat gctccaaaaa aagctcttta aatagtaaga tagaatccct 247860
acattatata gatggtgcct ccctccttac agtcataatt tttagaatgg tccttcctct 247920
tagttaacat ttgtttgccc tcctgtaaaa tgtggctaat gtaattctta gtgccctaaa 247980
ttatcactgt agcaatctcc ctcactttcc ccagtctcct acctcaaatc acttaaatgt 248040
cccaaaaaga gagagggaaa atgaaatctc aaaaacaaaa accaaaatag agttttgact 248100
tgaaaagaat cttggggaaa acagagcctg gttgtatgca cccagtccta aaagcacctt 248160
aaggagcatc acagatgctc aacagattcc aatttcaatt tgaaatgtgt tgagaattta 248220
gcattgcttt tacactgtga accttgttac tagattcaca tactggactc acacttataa 248280
ataaattctc ctaattaatg tatacaagaa atagattgat ggaatgcttg ggcttcccat 248340
gttactaatg ataagaccag tggaagagag atttctcttt gtcctcctca caagattttt 248400
gaactacaag ataaactata ccctgatgaa ttagtatggc attgtaagag taataaattt 248460
gaggggttgt tgcagttggt gtaccacttg tttacacaat agattcatta ccctttttgc 248520
ttctgtagcc ttgatcttca tctgtctaat tcttttatat ttaaaatggg tcaactttcc 248580
ttagaattta ccttgttcca aagaagttac ctttatttct caaaaacaaa aacaatagga 248640
atgattttct tccctgaatt tttaaaatgt ctgttactag aacattaaaa tttcttgaga 248700
tattagctac agagtactca tctaagagtt ccttagatgt tcctggaaaa taattatttc 248760
gtatattgct tatatatcgc ctttttaggt gctggagtct caaattcaag caatctgatc 248820
accagttcct tcatcagagc aacgtctttc atcacattaa caatattttg tcaaagtcag 248880
atgatggcga tagtgaagag agttttagca tcagtataca gtctggcttt gaagctatga 248940
gtcaggtcag tcatttcagt tattatcctg gttgtcctgt tagttaataa tacatcaaag 249000
gagaaactgt aagttattca taatcaattt gatcctgtct tccattatgt tttatcttat 249060
acctgtttgt tttatcatca cgaaagattt gcttttaagt atcataagta cttatatttg 249120
tttctgatag gaattatgca tagtaatgtg cttaaaggac ttaaccagca ttgttgacat 249180
aaaaacttca agccgacctg ccatgattgg cagtttgaca gacggctcca cagaaacctt 249240
ttgggaatca ggagatgaag ataaaaacaa aactaagaac atcaccatca actgtgtaaa 249300
aggaatcaat gcccgctatg tgtctgttca cgtggacaat tcccgagatc ttggggtaag 249360

aaagcaaacg tgatttacgg ctctttagct cttttcagat cttagtattc atgcactttc 249420
atttctgaat ttagcaacaa gctttatcac ctggaatttc accaaccaaa catcattgct 249480
ataatcccca aataaatctt aagtctaaaa acaaattaat atgattggga gcactgtgga 249540
attccatttc tgaaaagagg tctgattaca aatataccat ggctacagct aagcaaaatg 249600
aaagtctgct ctacaatttt ctgctgccat tcccttggtt aagattttcc cgtgacctgg 249660
aagtcctccc cttaaactct gctgatcaat tcttccaggg tctctcaaac tcagcagaga 249720
aattctaagt aactcctcta aaaaattact ctttccctct gctgattctt tttgtattac 249780
tctggtggca cttaataata atctttgacc tatagtgtaa tcagcttttt tttttttttc 249840
tgagatggag tctccctctg tcacccaggc tggagtgcag tggcacaatc ttggctcact 249900
gcaaccttcg cctccaggtt caaacgattc tcctgcttca acctcctgag tagctgggat 249960
tacaagcaca cgccaccatg cttggctaat tttgtatttt ttagtagaga cagggtttca 250020
ccatgttgtc caggccggtc tcaaacccct ggcctcaagt gatctgccca cctcagcctc 250080
ccaaagtgct aggattacag acatgagcca ctgcacccag cctatcttca agtgtctact 250140
ttgccacagt cctcatgaaa taaaaaataa gtttcagact gtcccaaata atcataattt 250200
atattctctt cagcctagtt catccatgtg tttcaactga attaaatatt cttttataaa 250260
aggcactaaa caagaaaaaa gagtcctatt tgcttgagta ttaacaacca agaaagaaat 250320
agcactaact gccagactgc ctttcttcta cctcatttag aatttttttc ctcagaatgg 250380
atgtggcttg ctagcagata cagtattaat ggctctcctt gaaccattgc ctggcttctc 250440
ctgtgccatt tgcatatttc ctttctcagg aagagccaca gtggtaggat tgggtctcag 250500
ataacctgga tgcaggccag tctgcaatca gagcaacctc caactcctgt acatcagtgc 250560
aggttctatt cctccctggt attgagtaag taaaagaggc ttagctaata taatatgagc 250620
tatttcctcc tcagacctaa gaatttccac tggtgtaact actttatctt actggaaagt 250680
caagtgtaac aaagcaaaac atttttgtag ctagaactca ctgttcagtg caaagctatc 250740
tttggctcat ggtacatttt aagcaaggac atactctccc tcagaacaat gattgcaatc 250800
aatttaacac ttctgtcttt tctaagagga aataatcata acagtattca ttttcaaaat 250860
tcccaacatg taagctgaca tctactttcc ttttaacagg cttcctctat gttataggtt 250920
gagcattcca aatctgaaaa tccaaaatgc tacagaatct gaaacttttt gaacacagat 250980
atgacactta aaggaaatgc atattggagc atttctggtt ttagatattc agatttgggg 251040
tgcccagcca gtaaggataa tgcaaataga gtatttcaaa atcctaaaaa atccctaatc 251100
caaaacactt ctagtcccaa atatttctaa taaaggatac tcaacctgta tgtatgtttt 251160
ccttgagtta tgtacctcca tgttcctcaa cctgcctacc acagagcctt gtacacagca 251220
tttgttgatt tgagttgtat cagcctacag cactcatatt atgcagctct ccaaggaatt 251280
aatgttaatc tatttcttga tgtgctttgc gtaaaactta ttcagcctta agatatttga 251340
cactagtttg aacatgtatt gaacttgtac tgtttgacat ttattttgtg tattaatgtt 251400

gccttcccta gtttggcaag tatcttgggg gatagaacct cctctcatat atcctacttc 251460
aaatctgtaa ggaagtctgg gtacattgtg acactcataa gtatttgtta gttcagttac 251520
cttccctctt tatacttcca aaagtaatga aaatatagca acaagagtct gacataagtg 251580
acaagcagat ttgttgaaag gcttcatgag gaaggaatct gaatatttag actttgaata 251640
aggagaggga atgtcaagct gtgagagagc acctggagag aggctgaaat gtgcaattta 251700
agatggagtt tgtgagaatg agactaaagt tataggaacc gtggttagtt ggagggatgg 251760
ggtggggtca atcaggaagg accataagag acaggcagaa gagttgatgt ggtattagta 251820
ggtcatgaga aaccattgta agttctgaag caggaatata gcatattaac tattgtgaat 251880
tgtctatttc agaataaagt tacctcaatg accttcttaa ctggcaaagc agtagaagat 251940
ttgtgcagaa taaagcaggt aattgaaatt ttgtcattaa atgtttcgct acctagagag 252000
aaatttcttc ttctcagcct tcactattga attccaccct tggcacacaa ccaaagtatt 252060
atagggaatt atctatttac agaacactaa attgacaagt ggtccaaaac ataggaatag 252120
taaccaccat ccctgtgctg gacatctcac ctgttttcct aaatgttccc cagtcatgag 252180
caattatatt gtacctacag tctccctacc ctactcccca acaaagactc agggtatctt 252240
aaaatttatc acagatccca ttcacactta aaaccttact tcacctatac tttgagttaa 252300
tacaggttat tgtcctctgg cctgaaaaat ctgtctgtta taacacttat aacactgcag 252360
ctatatccag gttgcaagtc agtttaaacc aactttagaa acaagttaag ttttatctag 252420
gcatgtgtct gagactgtgc tcaagctccc ttacttgtat tcatgaaact ttgtcaaatc 252480
tgttcctcac tggctacttt atctcacaaa aagttggcca atcgcttgct tgcttgcttg 252540
ttttcaaatc acattgaagg aatcaattaa gattttggac taaacatata ataaattttg 252600
tttaaattgt tgaagaattt gggaaaataa tcctattcaa tgagattgtg tttgtttata 252660
cttaataccc ctttttgaaa attgacatta ttaattaaaa taatatcaac ttaatgtcta 252720
tttaaatcac atagaaatcg ccaggtcatt tcttggttat taaatgccat aagaacagtt 252780
tttattttat gaagggaata gtgtcaagaa ctatttcaca gtatattaac atttaatcct 252840
tgaaaaaaaa agtcatattc tcaatgaaaa ttagtgaaat aaggaatcac aagtgtgaag 252900
ttacatatta tgtaattttt gttcaaaagt aaaacatatt aagtaactgc actttaaagt 252960
cccttatctt taaatggtac tttaatgtga ggatgcacaa aaaaagaacc cttttctgat 253020
cttgtgaggt ttccacttac agatgatata gataaagtac agtcatataa attatttgta 253080
attatttcac tcatataatt ttgctcggaa gccatatcgt agtcaggcct ggattttaat 253140
tctcaattcc accatttact gaaaatccct taacctgtat taaccttagt ttcttcatct 253200
gtgtaatagc tactataaat aatattactt atcttcacct ggttgaaata agaaaatcag 253260
tgtgaaataa cctcataacc tttactctgt gcaattcttt gttatttgag cttgctggta 253320
tcaatgctaa gtccagcaca gtgataaaag tgatccagct gaatcaggtt cctgcctctt 253380
ttctactcaa ccaggttgat ctggattcca ggcacattgg ctgggtaaca agtgaacttc 253440

caggagggga taatcacatc ataaaaattg aattaaaagg cccagaaaat acactgagag 253500
ttcgacaagt caaagtcctg ggctggaaag atggtgaaag cacaaaaata gctggccaga 253560
tttcagccag tgtggcccag cagaggaact gtgaagctga gactctgcga gtattcagac 253620
tgattacgtc tcaagtgagt gtccttacaa catattctag cacaggataa ttgatgtaat 253680
atattttaag agtgtaacaa aatattttgt gaaacttatt acagaaattt ctggctataa 253740
atgttgccca tttttctttt tcatagccct aaacacctga gttccatgtt gcatttgtaa 253800
ttaagaaaaa tagtggtgta tattagcaag agtagcatat tataaaacct gcggaatcag 253860
ctaatgagtc ctctggccaa tatagtgtta atgcctgtca ttaattcctt ctcacatgca 253920
tatcttcatc aggtatttgg aaagctcatc tctggagatg ctgaacctac accagaacaa 253980
gaggaaaaag cactattgtc atcacctgaa ggagaagaaa aagtatacaa tgtatttatt 254040
tgtattctaa agatatttat ttcccttttt tattcttttt ttcagtgaaa taattcattt 254100
attgctgtat attttgatgg atatgtaata acagttttat gcagttcata tttgttctta 254160
gaaagatgaa ggaactatct aaaagaaatt cattattcat gtataatcta gctagtattt 254220
taagtaacag caaatttgct ctataccttg ggtttttgca ttcatttgta atcatttctg 254280
tgctataccc catcagttat cttctccctc ctccattgtc cctgtccttc acagttcagc 254340
tcaaaagtac tgtcttcttc atgaaacttt tttacgattt atcctcttca tgaaatttgt 254400
ttatgatttc cccaaacaaa agcagttttc tctctgctgg agttcataat aatctgtatg 254460
tgtctcttct tcatttttct cttatgttta tctacatgga tgttttctct cttctactga 254520
accatagctt cttaaagacc aagtccatgt ctaatttgtc tttgtatcca tcatggtagc 254580
atcacacaca tgtttagtaa atttttatta gaataactac tctttgttac tgttcttgaa 254640
atttcttgca aaaatacagt tgctttactt tttcggtcct gactaaaaca ctatacctaa 254700
aacttttgta cttcatccat ttgcaaaaag agaagtcaac tctgactagt ctcaatattc 254760
tagtgtcttt aaatgctaca aataatcacc ttggcagccc tttgaaagat aaatttcaag 254820
gatttccctt gtctcaccct gtgtttctta atacagtagt atctaatcat tgtcctgttt 254880
ttgtttgagg tctgaaaaaa ataagatttc ttaaatgctg gtaacttctt tcaacatctt 254940
ttggagattt catttgaatg actacaagac tgtgtaacta cctgttaagt caattgaatt 255000
ttagcagatg tcaactctgt actaaacaat tgtgagaatg gcaagccctc aaaaagcttg 255060
cattctgttc ttggttatga accctctgca ttcccctcac cccccccaaa aagaaatcaa 255120
caaagatact atatagtata aaagcaagtt attaaatgtg tgaaaaaaat tggatcattg 255180
tgccctggag tggtcagaga aggcttctta ggggatattg gactggagtt agatctgaaa 255240
gagaatgaag ggattcaaat gagattagta gagagaatgg gagcaaggac tgatcagaga 255300
gactggccta agggtgggag agtgtatgct gaggagttaa ggttgagtag gaaaggttat 255360
actgtcatgt gtgtgcccat gaaataacat gatttttaag agaatctttt tttaagttag 255420
attattgaag cacaatttac atacagaaat tcctcatttt aaactgtatc attcaatgaa 255480

tttttacttg tgtacactag tgaaatcatt atcacaatca agtttgtttt gttttgtttt 255540
gcttgagacc gggtctctct ctgtcatcca ggatagagtg cagtggtatg atcacggctc 255600
actgcaacct caacctcccg ggctcaatgg atcctcctgt ctcagcctcc caagtagctg 255660
gaactacagg tgcacgtcac cgtgcccagc tgattttaat atatctgcag agatgtggtt 255720
ttgccatgtt gcccaggctg gtctcagact cctgggctca agcagtctgc cagcattggc 255780
ctcccaaagt gctgagatta cagatatgag ccaccatgcc cagcccacaa ccaagtttta 255840
aaacacatca ctccccaaat ttctctcatg ttcctttgca ggcaatcccc caacctcaca 255900
cttagagcct agcaactact gaactatatt ctgccagtgt tgtgttgcct tttcctggat 255960
gtcatataaa tggaaccact cagaatgtag cctttttgtc ttgtttcttt cactttagtg 256020
taatgctttt gagatttatc catgttgtag catgtagcca tggttcattc ttttggttgc 256080
tgagaagtgt tccattatat ggatttaccg taatttgttt atcctttcat tttttgatgg 256140
atatttgggt tgtttctgct gtttggctac tattagtaaa gctgctgtga acactcacat 256200
acagatcttt gcaggtcttt taccaattat gtgttttcaa atatttctcc ctagtctgtg 256260
tcttatcttt cagagaacag aagtttttaa gtttgataaa gtccagtttt ttcttgtttt 256320
tttgattcat gatttttatg tatggtctag aacccaaggt cacacagata ttctcttctt 256380
ttcctttaga aatgttatag ttttagttct tttctttaga actgtataca ttttaatttt 256440
tatgtgcagt gtaagaattg aggcttatct tttgcatata gatgttcaat tgttctacta 256500
cgattcattg agaagactat catttatcaa tagtcaattt accataaatg gtaggtctgt 256560
ttctggactc tattctgctc cattgatcca tcaaagtgtc ttacttactg tagctttata 256620
aaaagtcttg atatcaggtt ctaactttgt tctttatcta agttattttg gctattctag 256680
atctcttgca tttccatata aagcttagaa tcagcttgac cgtttcttta aaaaaaaaag 256740
gggggggggc ggtggggatg gaattttgac tggaattaac accaaattga tagtttaggg 256800
agaatcgcca tgtcaataat attgagtctt cgtatctgtg aacatgatat gtatcagggg 256860
ttttcttaca tctgtttaaa atttctctca gcagtgtttt gtaattcatt gtacacatct 256920
tgtacatatt ttattaaatt tattcctaaa catttaacat ttgtaaagct attgtaaatg 256980
gtatttttta tttcactttc caattgttca tttctagtgt ataaaaatag aattgacttt 257040
tgtatattag atttctaccc tgtgatctta aattcactta ctaatttcag aaacttttta 257100
tagactcttt ggggtttctt tagataatca tgtagtcttc aataaatttg ggtttttttcc 257160
tttctaaact atatgcctta aatttctttt cttatattat tgtgttattg cattgattaa 257220
gcccttaagc gccatgctga atagaagtaa tgagaacaga tagctctgac ttgttctgga 257280
tcttgaaaag aaagtataaa ccattcatta ttaagtgtga tgtgaactgt agattttttg 257340
ggggtgaatc tttttaataa gattggagaa tgttttcttc tattcctagt ttgctgagaa 257400
tttctatcag aatggattct tttttaagag acctggtctt aactctgtca cccagacaag 257460
agcacagtgg tacctttata tgtttctctt tcttcatttc ttttattccg taagataatc 257520

catatattgc tatgcatttt agtcaacatg tgatactagc ttgtatacag ttcattttcc 257580
ttcagtgaaa aaaaaaacta ggggatggta gctacatgat atagtgcttc ttttagaagt 257640
aataaaaatg ctgtaatatt gactgacgac gatagttgca catatctgaa tgtactaaaa 257700
accattgaat tctatgcttt aaacagatga atagcatggt atgtgaacta tatctcaata 257760
aagatgtttt taaaaagcaa aaataaatga tgaaagaggt aaacaagatg aaagaactat 257820
ctaaaagaga ctcatatata atctaactag tacttaagta gcaaaataaa tgctctattc 257880
taaaaattca ctttctttaa tagataacag agctgtttca gttatctgtt tctttttggg 257940
taagctttag tagttctgac actcaagcaa tttgtccatt tcatctaagt ttctggattt 258000
attgccataa gttgttcata atataccctt atgtcttttt aatatctgta gaacctgtag 258060
tgatgtgtct tggtgatttg tgtcttcatt ctttttttct gaaatagttt ctctaaagat 258120
tcatcagttt gatctcttca gagaactagc tttttgtttc attgattttc ctctattttt 258180
gcttctttta ataatcacaa ctttatttct ttccctatgt ttgccttgta ttcatttgtt 258240
catctttttc tgacttctta aggtccaaga tcagatcgta gactccaaga cctttctttt 258300
ctcatctaag tatttaatgc tattattttc ttctaagcac tacattagct gcatttcaca 258360
aatttggggt attctgtttt tattttcatt caattcacaa tgttttctta gttttcttat 258420
tcccatttga tttctttttt tttttttttt tctttttttt tgaaatggag tcttgccctg 258480
tcgcccaggc tggagtgcag tggtgcaatc tcggctcact gcaagctctg actcctgggt 258540
tcacgccatt ctcctgcctc agcctcccaa gtagctggga gtacaggcgc ccgccaccat 258600
gcccagctaa ttttttgtat tttttttagt agagatgggg tttcaccacg ttagccagga 258660
tggtctcgat ctcctgacct cgtgatccac ccacctcggc ctcccaaagt gctgggatta 258720
caggcgtgag ccactgtatc cggccccatt ttgatttctt ctttgaccct tgggtaattt 258780
agaagcacct tgtttaattc ccaaatattt gggaattttc ctgttagctt tatattatta 258840
atttctggtt cagtttcatt gtggtcaaac tgtattgtat acttcgtata atttcagtcc 258900
ttttaaaatt gttaagattt ggattctggc ctataatata gtctgtttta gtgaatgtcc 258960
acgtgcactt caaaaaaatg tgaactctgc tgttcttctt tgaggggttc tataaatatc 259020
agttagttca agttgatcaa taatattcaa gtctttaaga gccttaatat tcaagtcttt 259080
aagagcctta ctggtttttg gttttttgct tgtttacttg ttctgttaag tactgaaaga 259140
agagttttga agtatccaac taactggatt tgtttatttg gaattcattc tacctggtag 259200
ctgatctcaa cattctaatg gactccaaaa aaaaagtttt ggttttgtag atgatcaggt 259260
ttttcctcat tgttagggcg aaagggacat tctcttctgg ctctctatct taagaaatgt 259320
aaaaccaagc atcatttttg attcttaaac aaaagagttt cttacctcag ttttcttacc 259380
tgtaaagtag gcaaaatatt acaatttacc ttctggggtt gttgcgaaga ttagataaga 259440
tttgtgtgtg tgtgtgtgtg tgtgtgtgtg tgtgtgtgtg cgtgtgcaca cacgtgcatg 259500
catatatata tatacacaca catatataca tatgtggcac ttagaagaac agcgtgcaca 259560

taataagctc tataaaattt tagcctttat atttttcctt atgatacatt tctttgatga 259620
cttggtgttc cgatagctta atttaagacc actatcatca cagaggtttg ttaggacact 259680
ttccttttaa acaaatgtta ttttcctgga ttgttcttca aacaattaaa tgtgggatgt 259740
gaactaataa tgtttacaat ttagaaatag atttaatcaa agctactcaa attgactaca 259800
ttaaagaaat ttgtgaaagg agaatatact tctctgaatt tcctaagttc tgagattttg 259860
ttctttttat ttcaaacttt atcctttgtc ttagtattat aagcctcaat ttaaaataaa 259920
tttactttaa aaatatatac acaaaattat ttctcaagtt actttgaaat atcacatata 259980
caaattttat ttaaagagac cacttcattc tttttttttt ttttcttttt ctatgtaaag 260040
gcaacatcag atgctgacct gaaagaacat atggttggaa tcatattcag caggagtaag 260100
ctgactaact tacaaaaaca ggttggttaa cagctatttc attgtcttca ttagttttgt 260160
gtgtgtgtgt gtgtgtgttt gtttgtttgt ctgtttgttt gttattaggg tataaagcag 260220
ggatgtccaa tcttttggct tccctgggcc atatttgaag aaaaagaatt gtcttaggct 260280
acacataaaa tacactaagg ctaacaatag ctgatgaact aaaaaaaaaa ttgcaagaaa 260340
aaaaaatctc ctaatacttt aagaaagttt atgaatttgt gttgggccac attcaaagcc 260400
atcctgggcc acaggcagtc ccccggctgc aggttggatg agcttggtat aaagtatttt 260460
gaaaaattta tgaaggcttt tttatgctcg cccttttata ggtcctgcag aatgtgcctg 260520
ccatttttta gagaggaatg agtattttgg cataccttca tcattttaaa gattttactt 260580
atgaaaactt gcttaccttg ctgctatttg ttatgggtag attttttatt tggtgtcaaa 260640
tacccagcta tatcagattt gatttgcatt tcccatattt cttttccccc attcttctgt 260700
gtgttggctg agcaattaac agtgttaact ataccagtgt taatattttt aaatataacc 260760
aggtgaggaa atagcgctat attgttcata taatggggtg ctacataaca taaaaatatc 260820
acttcaaaag tcaaataaaa tattgatttt aaattaacct attattctaa cttttttttt 260880
tttcgagagg gagtctcgct ctgttgccca ggctggagtg cagtggtgcg atctcggccc 260940
actgcaagct ccgcctcctg ggttcacacc attctcctgc ctcagcctcc tgagtagctg 261000
ggactacaga tgcctgccac cacggctggc taattttttt tttttttttt ttttttttta 261060
gtacagacgg ggtttcaccg tgttagccag gatggtctcg atctcctgac ctcgtgatcc 261120
acccgcctcg gcctcccaaa gtgctgggat tacaggcata atatttttgt atttttagta 261180
gagacggggt ttcaccgtgt tagccagaat ggtctcgatc tcctgacctc atgatccacc 261240
tgcctcggcc tcccaaactg ctgggattac aggtgtaatt tttttgtatt tttagtagag 261300
acgggatttc accatattag ccaggatggt ctcgatctcc tgatctcatg atccgcccgc 261360
ctcggcctcc caaattgctg ggattacagg cgtgagccac ggcacccagc cctattctaa 261420
ctttctacac tattattctt ttcaatgaac tacaacatga atttattttt gctttatctt 261480
tctagcagtg tttcattgaa tcttagatgc tttcaattgt aaaatgtcat gtataatttc 261540
agtattgtga atcttatcag ctataaagca ccttatgaac ataatggtaa accatgagaa 261600

aaagtgcctt cagtgataaa atatggtgat tggtttttga cgtgctaaac tgtaagcata 261660
ggcattaata acttgcatat aatcagtttg cacatagtag ttgatttaaa tatttccatt 261720
tttattgtgg ctcccttagc tttacttgtt tcttagatga gttaagagaa tgacatatga 261780
tggagaatta actgggagaa tagtattaaa ttgagtatca atttacattc ttgtcttatt 261840
cttttgtttg cttgttttgc taggagtagt aaatcaaact aaccctatat ttttacatgt 261900
tctgtgttaa ctttaaatag gtgtgtgctc atattgtcca agctattcgc atggaagcta 261960
ccagagtccg tgaagaatgg gaacatgcta tatcaagcaa agaaaatgcc aattctcagc 262020
caaatgatga agatgcctcc tctgatgcct actgctttga gctgctctct atggttttag 262080
cactgagtgg ctctaacgtt ggccggcaat atctggctca acagctaacc ctgcttcagg 262140
atctcttctc gctgcttcac acagcctctc ctagagtcca gagacaggtg actgatcaca 262200
ctacagcctt tccattgctt ttgttacttg aaacattgtt aaaagttgca atgcaaagag 262260
aattatctaa ggaccctaag tatagatttt tatttcgtat tttgttatat tttgtttggg 262320
gcctgtattt tactcccttt tttactgttt atagtgagag tatagaatag taaaatatgt 262380
tccattttat taatgtgttg tgataagaga gtattctggc tactgattac catattacca 262440
tgaaatatat aatgtcattt agccctgacc agaaagaaaa ggtatttttt ttcgtgctgc 262500
cttgggttag taattaccac tactattacc atttgaatag tatgtatatc atattttctt 262560
ccttcacaaa atgtaaaata gtgtttaaat catatttctt ttcttaaaga ttttgaagtt 262620
acagaatggt agttaggtac attaaaaaat gaagcctggg atggggtaca gtgacttatg 262680
cctgtaatcc cagcactttg ggaggcaaag ctgggaggat cccttgagcc taggagttca 262740
aggctgcagt gagctgtggt tgctccactg cactccaacc tgggcaacaa agcaagaccc 262800
tgtctttaaa aaaagaaaag agaaataaaa gaaaccttaa aacattagtc tttacatgga 262860
attctctcat tttatttttc ttaccaggta acctctttac taagaagagt tttgcctgaa 262920
gtaaccccta gtcgtctggc cagcatcata ggagtgaaat ccctcccccc agcagatatc 262980
agtgatatca ttcactcaac agagaaagga gactggaata agctgggtat cttggacatg 263040
tttctaggat gcattgccaa agcactcact gtacagctaa aagccaaagg aaccaccatc 263100
actggaacag ctggtaccac tgtgggcaaa ggagttacaa cagttactct tccgatgatt 263160
ttcaattcca ggttagttat tgcctctatt ttagtaccaa aacagaatag agtgagctac 263220
tgttcagaaa tttaagacat aatttcagat tttaccttgt taggttactc accaaataaa 263280
atccataaac aactggctaa tatctaaaaa ggtcatgtat ataataaaag tgtcatttat 263340
aattagtggg gaatagaaag taggctgaat atgtgatgtc agaatatttg gatatccatg 263400
ttaaaaaaaa ttctagatac ctacctctca ccatatacaa aattaaactt cagttttagg 263460
taatatgttt ttttgccaca ataaaaagaa ataaatatgg gggaataaag taaagttcag 263520
gtcaactaaa gctttatgta catttctttt taaaactgta ctgggacaaa ttatagaaga 263580
atatttttgt aatcttcaat ttgataagac cttcttaaac aaaacacaga acccaaaatc 263640

atataggaag atatttatag atttgactat ataaaaatga aaattacttg ataggtgata 263700
atatttaaat gttttaaaat atagggaaat aacttagaaa aatacttaaa cctatataat 263760
agacaatgac ttgttagcat aatatagaag tatgactaca aatcaatttt aaaaaggcaa 263820
acaactgata agaataataa atgacaagaa cagtcagttc acagaaaaaa acttataaat 263880
gccagtagtc agttcacaga aaaaaactta taaatgccag taaacaaatg aaaacacctc 263940
actactaatc agagaaatgt agattaaaat aattgcaaaa tatctttgat gctttggcaa 264000
cattatgagg aaataggcat tttcatctca tataaattgc tgcagccttt ttggatatct 264060
gtttataata tctaactata ttttaaatat gcatatcttt tgacctacca attctacttc 264120
taaaactttc atgcatttgc accaaggtat atatgcaagg atgttctgta attgtgtgta 264180
acagtagaga attagagaca tctgaacagt gctacttgta gagccaatcc atgacaatat 264240
gcagataaca tcagaatgtt aatcattgta tcacttcttt cctgtagtct tgctaggaaa 264300
aaagtcagct gaactaaata gtagtgtgct tagtgataca gtgaatttat attccggtta 264360
agctccacat tatccgcatg tgccctagaa ataagtcgtg tgtggtccct ggactgtact 264420
tctaaaagat gcacacagaa ataatgtgca aacagagaaa tacttgaaag ggaactcaaa 264480
taaccttaac tgctgtcttg ggatttgggg taatggtttg atgaattttc tttttaaatg 264540
ttttaattga ttcagtcact taaaaatcta aaatgctgca ttctgaaata acattttcat 264600
ctttttcatt tgttactact actgataatt tattggcttt ttaagtgcta atttttcttg 264660
tagttatctc cgacgaggtg aaagtcattg gtggatgaag ggctcaaccc ctacccagat 264720
ctcagagatc atcattaaac ttatcaagga tatggcagca gtaagttccc attcttctgt 264780
tttggcagtg aagtgcggca ggaagctgac acaaaacagc ttactctgct gaattgatag 264840
gagatgtaga ttagtgtaac tttacttaaa gactatttgt agtatgttaa ctatcatgct 264900
gtttgtcttt tcagtgagtg ataatttgtc atttatgtaa accatgttca tttcaaattt 264960
tccagttaat ttgtgtggga attgttacgt gactttaaaa tctaataaaa taaaatatgc 265020
tctttcagac cctaagaaag aacttgattc aaaaaaccta aaggaaaaaa agaggaagag 265080
agctctcgaa agtggaagtt tctcattctc ctcagtcctc atggttttta aaccctttat 265140
tcttctttat taatgtcata taccctctgt cttaagatgg tggcttccaa aggagatctg 265200
ggagtgtgga ggacagggag ttactttaca gaatgcctgg ctctgattag ttgctccttg 265260
tcattttcca cacattgtat tatttctagg agggcagcct gagaaggaca agatagtatg 265320
gaatgtagag tcagaaggaa tagaaggcag cttggagaag gaggaggaat tgaactgccc 265380
cttaaatcat gtggatatga gaagtgggag gattttctgg gggaaagatg aagttctatc 265440
acttttccca acagcgtgga tattttctca acctctgtgg caaacagact ttctcagggt 265500
gttcattgaa ttaaaatatt taggagtaat atttaggaac acctaaatta tatgatagtt 265560
aaatgtttgt tcattacaaa tcaaggtagc aaataaaggt agttgcagtt tgaacctgtt 265620
tatattttag gttcctgtaa tactcatcta aagcagttag cttcactgtg taaattttat 265680

gctaagtgac aaacttctca aacatagata accttagtat ttctgttatg tttttatcag 265740
atgtgagagt aattgccctt gatttccagg aaaagagtat tgagaactta attttttatt 265800
tatttatttt taaatatcaa aatgatgatt agaagttgaa ttttccatag gatcacgaag 265860
aaattttagt tcaaaagttt gtgcccataa tctaaaattc catggtctca ctaaaaacaa 265920
atatttcctt tgataggaga tgttatttaa gaaaaaagag tttactatgg taatacattt 265980
ctggtcactt attaaaatac tctgttctgc tcaatagggt catctgtcag aagcttggtc 266040
ccgagtgaca aaaaatgcta ttgcagaaac catcattgcc ttgaccaaga tggaagaaga 266100
atttaggtct ccagtgagat gtattgcaac aactagagta ggttttgctt tgttttgttt 266160
tgttttaaat ttggtgtgta tcattgcgtg ttctttttta gtaatagatt taaacaaatt 266220
ttaatcacta agtcaacata attatttttt aaccaaatac cgtatttatt gaaaaagtat 266280
aaagggcttt tatattatga actagtcgtt acttctcact gttatgtgtt ctgtgtcact 266340
tgtttttgtt attgtttagg aagccaaggg tacactggtt agaaaaggct aattacattg 266400
tacattgttt gcgtgttgtc tttctaatga tctgtttgct gtagtttacc acagtaagtt 266460
tagtgccagc attatgctgt gatagaccga aggtcctttg gagacaagac ccctgcctat 266520
gcctggttgt tcctaatgta gagccctgta acttacttac aggatttata ggtcttactt 266580
ctaaaaagca agaagtgctt gtccagggca gggtcagggt ggaaatcttt gaggaaatga 266640
gattgcattc ttcagatgac acatttatag taagttttaa cctgctagat ttcatgtccc 266700
atatacttcc agaagagatt tgagatttcc ttgaaggagg gcagtctgac aacgttagga 266760
agaagtgaat gctggtaaaa attgcttagg ttaatcctca gaaatctgaa gaagataaac 266820
tatatatccc tagggcggaa aaagtcaatt tatgctatct tgctattatt gactgtgaca 266880
cctgaaatag gtatttttga cagtaatcct ttcctaccac tcatgtgttg ctttctttca 266940
gtctgcattc cccttcctcc ttgcatgcat tatttctttt tctatttttc tgtccctatt 267000
ttaacatctc ttgctttctg tatccaataa cactcacatg cacttcttag agttgagcta 267060
gttaagtcat tttctattct tcattaatga ggggatatat cattctatat aaaactgaag 267120
cttacccttt aagcctcaga aatttttaac tatttctgaa aacagttgtc tttttttttt 267180
tttttttttt tttgagacag agtttcactc ttgatgccca ggctggagtg caatggtacg 267240
atcttggctc actgcagcct ccgcctgcag gtacaagcga ttctcctgtc tcagcctccc 267300
aagtagctcg gattacaggc atataccacc acacctggct aatttttttt gtatttagta 267360
aagactgggt ttcaccatgt tagtcaggct ggtcataacc ccctgacctc aggtgatcca 267420
cccaccttgg cctcccaaag tgctgggatt acaggcatgt gccactgtgc ccggccctat 267480
tttggagaac agttttctaa agcttttaga catgtatgta tttctgacaa agtgcaccaa 267540
gtatagtatt aggctttctg ggcaacaagg ggcaacaaaa gacttctgag gaacttttat 267600
tatctagaag gatttaatgt gtacctgact actcatggga ctgtacacaa tttagcagtt 267660
gctgtaccac cttctttggc atttaatgat actaaatgga aaaatattca gaagacatta 267720

gtagtaaata ttaaatatgc caattcattt attcatatgt tgtttacata gtagaccaat 267780
aaaggataat tttatttgtc agatagaaga cagggtggtt catcagagaa acagtcactt 267840
gttcagtaga tatctgagtg cctcctgtgt gcatggcaca taggacagtg atcgtgtatg 267900
cctgctacca catcaggaag acttgagtgc cactgagtat ctatgtattt tcaaaatcag 267960
ccagcattta ttgagctgtt gtgatgtgtc acagtcagca tttcataagt ctttcctaaa 268020
aattcagtgg tgtaacagcc ttccctttca acagctctgg cttgctctcg catccctatg 268080
tgttcttgat caggaccacg tagatcgtct ctcctcgggg agatggatgg gaaaggatgg 268140
acaacaaaaa caaatggtaa gatcagaatt ttgtaagaat ttatcttgct ttcctttaca 268200
gtaagcttta gcttagctta aaaacaaaca aacaaacaaa aaacacatca acataaacag 268260
ttagtggatc tcttgaaaga ttgtgtccct tcggccaagt tcatctcaca agtctatttc 268320
agagagtccc tacttcatag tatatggaaa ctttaggggc ttttatttaa gtatattaaa 268380
gagctttctt cttctctgag tcatgtttgc atcatatcaa aatttatttt tagccagagt 268440
ctgatttagt gactagatat tttactaaaa tgtaatcagt cttatggtag gaaggaaata 268500
aagcaataac acaggcaaca tgacacacca cctgttcaca tatatactta agtaggtttg 268560
atgggtatct ttaagttcac tagaaaggaa aactttatat aggccctttc accattttct 268620
tattaatgtt caagtttcaa aaataaacag tttcttcctt tggcatacag aatgtttaga 268680
tatataatgg ggcagacatt tcttaagtct ctaagtaaca aagtatacct gttcattgta 268740
ttaatcattg aacctgcaaa gcacattaat gattggattt ggagttttga gagattttta 268800
tagttaagta catttgcaat aattctgttt aatattcaga ttctaaattc ttaatagact 268860
tccgaataac ggtttatttt tgtaaataaa ttatattcaa tttgtatgag agtctgagac 268920
ttgcttgtta atctaagtaa cgtggaaatg tcttatttta gcctatgtgt gataaccatg 268980
atgatggtga aactgcagca atcattttat gcaatgtctg tggaaattta tgtacagact 269040
gtgacagatt ccttcacctt catcgaagaa ccaaaactca tcaaagacag gtgaagattt 269100
ttgtctctgg agcataaaat atgttcagtg aattttgaaa taaatgtgtg aaagaggctt 269160
tgtgtagtag ggggaaaaag tggactttgg aatcaaagac cttggttgag ttcacagctg 269220
tctcgcttac catctataaa tagcaagttg ttttatcctt agaaacactt aatttctcca 269280
tttataaaaa agggactgct gtgaagatta aagagaagat tggaaatatt caaaacttct 269340
tagagtgctt aaatgaaagc cattattatt attgaataat taaattttat actctccctt 269400
tcagagtgat aaataagcat atttttccct gttctttaat gaaataatag gtcttcaaag 269460
aagaagaaga agctataaag gttgaccttc atgaaggttg tggtagaacc aaattgttct 269520
ggttgatggc actggcagat tctaaaacaa tgaaggcaat ggtggaattc cgagaacaca 269580
caggtaaaag gtttaaagat tgagccatgc ctttaaaacc cacaaaaaaa gattaaaccg 269640
tgccaactga ttaaactgtg aataattttg agtgatattc tgatgtttaa ttatgacatc 269700
attgatctat atatttacta aatatataga gtgttctatg gtgaagagag cattagacca 269760

aaagtcaaga aacctgattt gccactaact agctgtgtgg tgtcgagcaa gtcacttaac 269820
ctctggaact cattgacttc atttataaag tgagaggatc acaactaatg gggctccata 269880
gataaaatac atttctattg ataattccct tctagttctg ctagttgttg ccccacaata 269940
aaaattgctg cactagatga tcactaagat ctctatttat tataaggttg tatgatttta 270000
tgatatttag aggtttttgt agacctaatg cttctgaagg tgttgtgaat ggtactgttt 270060
ccattgatag caataaaata attaacaaaa atgaatagac ataaaacaaa aggtaaccta 270120
tgtatgttaa atgtattttt atctctaaaa catagttttc attttgctgc ttttctaaag 270180
taaatcttgt atattcctag ttcaaagtta tgttgaatct tacaaattct cagttttcag 270240
cacatctgca gtctgccatt tgactgacac gaacttccag tgttggagtt aggtgtaaat 270300
ccgaatcaat ttaattctag ggtgactttg catgctgcaa gagcatgacc ttcttttctc 270360
atagttgatt tcttcctcct ataatctgtc agtgctgtat actgcattaa taaatgttct 270420
aatgtcaaac catccttgta aaccatcctt tttaaataga tttttctgta aatattgctg 270480
aattcaatat attgatatta attatagtta gatttttata tctgtgttta aaaatcagct 270540
tggtctgtaa ttttcaaatc ttcacgttct ttttcttatt gctctgttct tgttttattg 270600
attctgtttc ttctttaagt atttcaaaca tatttaaatt ccttttcaaa ttgcagagtt 270660
cctgaggtag gaattctgtt tttcaagttt catactctga tgataggctt tataccatgc 270720
cttataattt tgttatatat agttaaagat ttccctaact tctgtgtatt tttatttaat 270780
ttttggttat catatttttt atggtattgt gcatttggag tcaggggctt gtaacagctg 270840
ctcagtctgg taacttgact agaagtctag tctgtttctt tcactgaaaa aaaatattag 270900
tcgagagtgt atttggcttc agataggtct ttaatttctt tttttgtgtg tcattattta 270960
ctttataatt aggacaactt agaaaccttc cgttctaaaa ctgtcaggct gatttcccaa 271020
ggatagcaac ttatacaaac ctagtttgct ttttaaaatt tagttctgtt aacatatcta 271080
gaaaacatcc ataagaagga tttttatttt tctgcttgaa cctaggcaaa cccaccacga 271140
gtagctcaga agcatgtcgc ttctgtggtt ccaggagtgg aacagagtta tctgctgttg 271200
gcagtgtttg ttctgatgca gattgccagg tgagtatata gtgacacagg ccatcccatt 271260
gtccatgttc ccctgtgaca cctttacttc atacagaaaa gagttttagc tcagcgtcac 271320
tccagcttcc taaatgagtg tattacctat gtgaagcatc aaatgcctta ccaaagttga 271380
agaatgggtc aaataaggct ctcaagccca aaccacactc tttttgtatg tttctcagtt 271440
accatgatca tatgtattta atatctactt ttgtactaga aacagttaaa aatttatgaa 271500
ctgtatttcc tttatctctt acagacatta ttcacttatt tccttaaaat acattttcaa 271560
catctaagag ttctttaatc ctgaacatat ataaacatat tctatgttgt ccaaatgttt 271620
ttggtttctc aattaataag aatgtggata tgctcagaga gtcaaaggta cctttttaaa 271680
aaaaaaaaaa atctgcattt tggtgatgcg gagagtggtg actgggaaat tatacattgg 271740
ggaacttttt ttatttttta tttttttgag acggagtctg gctgtgtcgc ccaggctgga 271800

gtacagtggc acgatctcgg ctcactgcaa cctccgcctc ccgggttcaa gcgattctcc 271860
tgcctcagct tcctaagtag ctgggattac aggcgcgcgc caccatgcct ggttaatttt 271920
tgtattttta gtagagatgg ggtttcacca tattggtcag gctggtctca aactcctgat 271980
ctcgtgatct gcccacctca gcctcccaaa gtgctgggat tacaggtgta agccacgact 272040
cccagccagg aactttttgc tttatagctg tgcatgatga caaatatgaa attataattc 272100
acttatcagc attcccttta tagagagtgg cattttttaa aaatcgcatc aaaaaaaaaa 272160
aactttgtta agaagcctaa tttatattca aacaaagatt agaaataact ttactttgtt 272220
gttacttggc ctgcatagat acttcctgtg ggaaagtaat ctttggaaat ttatataata 272280
aggaaaatta ccagaatgtg tgctttttta catacttgtg taacattgta attcatcttt 272340
aacaggaata cgctaagata gcctgtagta agacgcatcc ttgtggccat ccatgcgggg 272400
gtgttaaaaa cgaagagcac tgtctgccct gtctacacgg ctgtgacaaa agtgccacaa 272460
gcctgaagca agacgccgat gacatgtgca tgatatgttt caccgaagcg ctctcggcag 272520
caccagccat tcaggttgcc tcagctttta aagtattgaa tgtatccaga cattaagatg 272580
gggaatatat ttgtcaaata aacattgatt gaggccgggc gcagtggctc atgcctgtaa 272640
tcccagcact ttgggaggcc gaggcaggtg gatcacgagg tcaggagatc gagaccatcc 272700
tggctaacac agtgagaccc cgtctctact acaaaaatac aaaaaaatta gctgggcgtg 272760
gtggtgggtg cctgtagtcc cagctactca ggaggctgag tcaggagaat ggcatgaagc 272820
caggagacgg agcttgcagt gatctgagat ggcaccactg cactgcagcc tggtgacaga 272880
gcgagactct gtctcaaaaa aaaaaaaaaa aaaaaaagca gttgattgaa atcttttttt 272940
atatcactag gcagaagtgt tcttttaatc tatacatgta ccaggctaat gaggagcaaa 273000
ttctgcctct ttatgctgtg ctttctatgt tgcatgctca gctttaattt gtgatttgtg 273060
tgaacctctg taatcaacag aatgtataaa atattcagaa gccaggggga aataattaga 273120
ttatccccca gtagctaagg ctcttcttaa tttccaaaat ccagaatggt atttttacaa 273180
cattatataa ttgatctata cataatgata catacttcta tacatacaca agttactgtg 273240
cactaaatag gtttttttaa cttttgccca ttgcttatat tttcagttct catgattcca 273300
agataataat agtctccaaa agaaaatcat aagtcagtaa tttcttcttc tttctgtgag 273360
atacaaaatt gaataatttt actttttttc ctaacctagc ttactttata taagtggcat 273420
gaatgttaaa aagcaggaga agccaaaggc ctagttaata gtatcccaat ttactgtaat 273480
gaaatttagt aaataataat gtcccaattt cagtgtccca atgaattttc attatagtaa 273540
atttcttgat ttttctctta cctgctttaa gtagtgcctt gcattaaata atctcccagt 273600
cactcaactg tttatcatta ttatatttgc ttatgtcctt ttcttttgtt tatttaagct 273660
ggattgtagt cacatattcc acttacagtg ctgtcggcga gtattagaaa atcgatggct 273720
tggcccaagg ataacatttg gatttatatc ttgtcccatt tgcaaggtat ggaaagaatc 273780
tgaaatcatg tactttcttt ttcttttgtt ctttcttttc acctttcaaa ataaacatat 273840

gtcagtatct cttggtttta cccaccagcc cctagcagtt tcttcccttc tcattcatgt 273900
ggaacctgag aatataatct ctctgttata atgcatctca aaacagtaat tgtccattta 273960
aaactgatta cattctgtta aacatattac atgagaaagt tcatttaaac atcgtgatgc 274020
attaaagggc atgcatctct actctcagac cactagagag ctcagttgac ataacatgac 274080
aacggctacc aaaaagaaga atgatcctct ggtgtttgta gaaactgata cctgccatta 274140
aagagtgaaa ttggaaccct cagtaccact agaacagatt cttttagagc ttagttaaaa 274200
caaggagaaa gttcttggca ttttgacaca tttgtacaaa ggtagtcagc aagtaggaag 274260
ttgttttggc aagctaataa tgattaacaa aaagctgttt ttgaagaatg tggcagtact 274320
ttccctatta attcatcgcc cctttttgtt ctattaagct ctccctattt tcttatcttc 274380
gtatttgcaa actaatataa agggtagaat gtggaaagac ttagcacaat cattgtctag 274440
atagactgag gaataatcaa aaagattgta gtattttgct ctcccctatc atatggtgct 274500
acaaatatta atagatacat gaaagtttta cagcaaatac tacctgttca cttctgtcac 274560
ctggcgttct gccttcccct caaggaagca aaacacacac acacacacac acacacacac 274620
acacacacac acacacacac acacacacac accaaacaca gagcgtgtct tatttgtggc 274680
aacaaggtaa ctttgtttcc acaatagcta gggcaacagg agatatattt cagacgcagg 274740
aaatataaag ctaataaaat ggaattttca tgctctgtgt cccatttgcc ccattttccc 274800
tgctcttgga aaaatatggg cttctaaaga atttacagaa tgtttttcaa atgacatttt 274860
atttagaata tgtgtgctgt ctggttatct tctagcacaa tgcttggcac ttaggtgcat 274920
gctaagtgtt tgttgagttg gttaataaat gattctgcta aagagtgttc attttttatt 274980
gcagaacaaa attaatcaca tagtactaaa agacctactt gatccaataa aagaactcta 275040
tgaggatgtc agaagaaaag ccttaatgag attggaatat gaaggtctgc ataagagtga 275100
agctatcaca actcctggtg tgaggtttta taatgaccca gctggctatg caatgaatag 275160
atatgcatat tatgtgtgct acaaatgcag aaaggtatgc tataaattat actgagaagt 275220
tttaaaaact agagcttacc tatatgatta agaattcaaa ttgtacagtg atatctaatt 275280
attccatctt aagcctgaag ttaaaaataa gatagcttgg tacaatgttt cccaatgttt 275340
ccagtaaaac ttgtttactt aacttgtttg gggattcttt atttaacctg aacaaacttt 275400
tcgaaacatg cagtatttct gtatacaagc tgcttcccat cagtaataac ctgttggccc 275460
agggcaattc cttctcgaat aaacttctct cacttgactc tgctaaccat tccttcctta 275520
aaacccttaa ttccttggct tccaggacaa cacactcttt ttctgtcctc cttcctttct 275580
agtcatttat ctttaactac ttcttctttt ttttttttta acctacccct tacatatttg 275640
ttttcctaga gctccattct tgcctactta ataaatactt ccttaccttg ggtgacctct 275700
tttacatcta taccttcacc taccatgtat atgtttatga ccccccaaaa tttttgagat 275760
ttagttacaa ataaccaact gcgtaccaga catttggatc tctcacacat acttcagagt 275820
tcacatgtca ggtattatat tcatcaatct tggccgggca cagtggctca cacctgtaat 275880

cccagcactt tgggaggcca aggtgggcag atttcttgag ctctggagtt tgagaccacc 275940
cagggcaaga tggcaaaacc ccatctctac aagaaataca aaaattagac aggtaggttg 276000
gcatctgcct gtagtcccag ctacttgggg gactgaggca ggggaatcgc ttgagtccag 276060
ggggttgagg ctgcaatgag ctgtgtttgt gccactgcag tccagcctgg gtgacatagt 276120
gagaccctgt ctcaaaaaaa aaaaaaaaga ctttaccaat ctctgatctt tattgacaaa 276180
atcctatcaa ttctgtctcc agatctctct tgagtccttt tttctttcca ttacatctcc 276240
agcaaaccta atcattttcc ttggtaatta cagtagcctt cttcctgagc tcgggtgtag 276300
aacccctaat tcctctgttg catcccttac actgctgcta attaatactt ctagatctaa 276360
aatctaactg gatcactctt ctacttataa tacggtagcc cagtctccct ctccagtctc 276420
atctcccaag atgctaccat ttgctctcca tttgctgcca cataccaaat aacttgcatt 276480
tcccaaactc agccctgtct gctcacccac tgacaccccc atgccattgc acaggccatt 276540
ggtgtttaaa ggatgctctt ctctacctcc ccctgcatat tttactctga cctgccaccc 276600
ccaaccccat acacacatgc tgtcatgcag agcctaatct gtcttgtcat acctcgggct 276660
tcagctcagg tttcacctcc tctgtaacat acccccatct cacttccatc aacaccacca 276720
ccacatgatt ttggtatctt gcaggcatct agcatagaac tccctattct gcattatgac 276780
tactggacca cttatctctc tgccctactt gataagttcc atgaggacaa agagtatgtt 276840
ttttcatttt tctatctcta gtactttgca cttactagat acaaagttaa gtggagaatg 276900
aataaatgag tgaaagaatg actacatgag aaaggtgcat agtctcccaa tgtagcaaaa 276960
agaaaataca gaggatgaat ggatgtctaa gtagagagat gatagccaga aaggcagata 277020
aatacatgca cacccaatag tgcacattta tgaagcttgt attgttacaa ataataaaat 277080
agtatttctg tgtgtttcat gtactaaaca ttataaatga atattgtgct attacagggt 277140
agcagtcaaa tgcttaggaa gccaagcaga ggatatagaa aagtgaaaca gctggagtaa 277200
gcagtcccca agacaaaccg aaatacttcc agggttaggt ttagctggtg aacgaccgtt 277260
tataacctct gcatcaatgg atcctgtctt tggacctgtt tttctagtat agataataga 277320
tttatattga ctcagtttgt ttattcccaa tccggacata gctttttgac tatgagacaa 277380
tcagtgcatg caaattgtcc cagttccttg gcctcactta atctctgccc agggcaaata 277440
cagataaatc acccatcatg atatttttat ttattcatga taattttatt tattcaaatt 277500
ctatttatgc aagtgtctgt attggaaact gttgagttcc tgcctactgc ataactttat 277560
aactatgagc agaattctga acagaattat caaatttgcc tttttttttt ttttgagacg 277620
gggtctcacc atatcaccca ggctggagtg cagtggcatg atctcagctt actgcagcct 277680
ccacctcctg ggctcaaaca agcctcccac ctcagccttc tcagtagctg ggaccacagg 277740
tcctcaccac cacgcctggc taatttctgt gtttgtttgt atttttgata gagatgggtt 277800
ttgccatgtt gcccaggctg gtcttgaact cctgagctca aggaatctgt ctgcctcacc 277860
ctcccaaagt gctgggatta caggtgtgag ccaccatgcc cagccggcct tttcttctta 277920

aaaaatgtgt gatcagagga tgcttaaaat tacttttttc tttcccatta gccttggaat 277980
tataattcta tactaattac tttatgtata ttttgttcta cagtattata aattgggaac 278040
tgctaagcat atttattaaa acagtagaag gggatatgta ttttaatatt tcaaaatgag 278100
aaaaatttct caaactttac cctgaaaaaa gcatagcttt tactacttaa tattctgttg 278160
atatttactt ggaatattat acttttttgt tttgacagga caatgtaact gcctacttta 278220
agcttgaatc catttgagag tcgaaatagc tttaggccag gcatgtggct catgccaata 278280
atcccagcac tttgggaggc caaggcaaga ggatcacctg agcccaggag tttgagacca 278340
gcctgggcag catagggaga ctctgtctct acaaataatt aaacaattag ccgggtgtgg 278400
tggcgtgcac ctatgatcct agccactcag gaggctgagt tgggcggatc gcctgagccc 278460
aagaggttga ggctgtcggg agccatgatt gcgccactat gctccagcct gggcaacaga 278520
gtgagagccc gtctcaaaga aacagagaga gaggtggaag aggagaagag aagagaaggg 278580
aaggaaggga aggagggaag gaaggtagga ggggaagaaa gaaaaagaag aaatagcttt 278640
atttcttacc ctgatccacc ctacatacag tcagcaagca atatgggcat gcacttggca 278700
tttcaaggga tggtctagta gataatggta ttttctaaaa cccctagatc ttactttcac 278760
tatgcccata ctgtatctcc catgccagag ttttcttttt tcctgctgta atctagatgt 278820
gttttctcca caggcatatt ttggtggtga agctcgctgc gatgctgagg ctggacgggg 278880
agatgattat gatcccagag agctcatttg tggtgcctgt tctgatgttt ccagggctca 278940
ggtaggcaga acattttatt agaaacaaca gtttagaaat gttaaatagt attatttttt 279000
aaacaataat atggtgggaa tgtaaatcag gcttttctcc tcagtatttt ctgatatttg 279060
ttcagtgttg tataattttt cacattattt catatatatt atttcctttg ggcgatattt 279120
ttattttata attttgtctt ccatgaaatg atcaccatag ttaaacaatg taataaattt 279180
tcttattttc ttagtttctt aaacgtgcag tgaaactttt aggttttccc ctcttcagtg 279240
aaatgttcac agtacatctg tgagattata agagaggtta atttcattaa ttttcagttt 279300
gaccagccat aaaacattca taaagaaatt aagtaccttg cttattaaat cagatagatt 279360
atgatggaaa tggaattaga acttggatct ttgtccagac cttttggaca ggccacatat 279420
ttatttgtta gttttcaaac atgtgtatca tcagtttttc aatcaatttt tagagttatg 279480
taggccctca aaatatgaat taaggagctg tctatgtata tgcctatctt aagtgtgatt 279540
gaaatagtct cactatgatc ttgtgtctgt ctcttctctt ttgctataga tgtgtcccaa 279600
acatggcaca gactttttgg aatataaatg tcgctactgc tgttcagtgg ctgttttttt 279660
ctgttttgga acaacacatt tttgtaatgc ttgtcatgat gattttcaaa gaatgactag 279720
cattcctaag gaagaactac cacactgtcc tgcaggtatg cttttaatat tttaaaatca 279780
cgattatgat ctatatacca tagttttatg taaacattat atgaaagctc tgtttcaagt 279840
gacagaaact caattcaaac tagcctaaag aagcaggagg aattccttga tccttgtaag 279900
cttatgactt tctgggtgaa ttaggaagca ggctcatcat catgagtgag aagttagctg 279960

tgacggagca gatctcatgg ggagtgagaa tgaagtctac taaggattgg tgaaaagatg 280020
gctgttcagc actaaaaagt catatgaagt tcaatattac agaaccattt aaaaggattc 280080
tgtgattttt tttttttttt aacgaagcct ggaaccgttg ccataaaatg aaccaagtga 280140
tttaatctag aatggctggg aatcagtagt gtagtgagca tcagaaagtc aagggaatta 280200
aagaaaccac tcagaataca ggagaaatta ttgtctatgg aaagcaggct gaggggaaag 280260
aagtatgaaa ccaaaaagaa gctgaaaata ctggaagagg acaggaggct agtggcaacg 280320
gtgaggtgga ataccaggct tcacggaagt gagagaagtg gaaggagagg tccgtgcaac 280380
cataggagag aattttacat aagatatatc tgaaattagt gaaaatacat gcacaagtac 280440
atacaacaca atataccgtg ttgtcaaagc tagaaaacac acactgccat ctgctgccta 280500
tcagagtgca agtgatagac ctctccggaa aacagcttaa tagtgcctct caaaattacc 280560
agcgtatcaa cccttcagca ctccctcttc agggatatag ctacaaatgt gaggacagta 280620
aaaaagggca aataaacaag gttattaact acagctttgc tatgataata aaaatgtata 280680
aacaatgcta gtgtccagta gggtagtggc taatattacg catcagttta aaaaaaaaaa 280740
agagtgagga tggtgtttct gtgtgctgat ttggaaagat cttcaggaga tagcatcaga 280800
tgaaaaaagt aaattaggcc aagtggtgtg actgacacct gtaatcccag cactatggga 280860
gaccgaagca ggtggatcac ttgaggtcag gagtacgaga ccagcctggc caacatggtg 280920
aaaccccatc tctactaaat gtacaaaaat tagccaggca tggtggcggg tacctgtaat 280980
ccgagctact cacgaggctg aggccagaga atcacttgag cctgagaggc ggaggttgca 281040
gtgagccaag attgcgccac tgcactccag cctgggcgac agagctgttt caaaaaaaaa 281100
aagcaaattg cagaacagag tatgcagtat gctgcctttt gtaagaggga aaatgagact 281160
atctagtcat atttgtttat atttacataa agaaacccct ggaaagatac tcaagaaact 281220
aatactattg attaactatt gggaaaagca agaaaaagat gagggtagag actagggaaa 281280
gttgcttcca attgtgttat catttttaac attttgattt taaaccatgt gaaacatata 281340
ctatccattt caaaataaag ataagtaggg ttttttcaag tatgactact ttttaccatt 281400
tgctcatgtc aaataaaaat tgttttactg tttctcctca aatactgttt ttttttaata 281460
gaagtagcag gctttgaaag aagcaaggcc agaatgatgt gaagccaggt tatggaggat 281520
aatggacaga acagagatgt gagccatgct tggagggagg gggtgatgtg ctggaggtca 281580
gcaagtgaag ggagtgtgga aagggcagta ttctagatga ctctagatga cttgagcttg 281640
gaggaagggc aattattgaa tgaaagttgt aggttgtggt ctggaaagaa ataggagcca 281700
agagaaaaat cagtcacacg ttgtagccat atgacctgga ggagcttggg agataaccag 281760
cctccagttg agaaagattt aaggcaagca gtatcttcag gggaaatcta gattttactt 281820
aaagtttcct ctatgttaaa aaacagaaat aagaatgcta aaaataagga agttaacaat 281880
gaaacacttt agagaactca gtggtgggta gtagctgaga gaggagggtc aggtttcctc 281940
acaggatcag ggcagccaac acaaaggaat cacaggaggg aggcggggga ggagccagag 282000

ttggcagaag gaaggaatga cctcggtcct gggcattttc acacatattt gctccatttt 282060
tacaatctgc gagtagatgg tattatccct atgttatcaa atggaaaatt gagggctagg 282120
gaggttaatg aactggccta agaacataca ctgagattga ggtatactct cccagcctca 282180
cacatccctc taactcatcc attcatttag agaactggaa tagaataaag gagatcccac 282240
agggatgtga gtagggaaga tggccatata cagtttactt agaaggacag aagaagcaat 282300
tctctaacca ctctcaaccc tgttaattct aatattttta gataatcagt cttcatccat 282360
tcaagctgct ataacaaaat actgtaaact gggtagctta taaacagtgg aaatttattc 282420
tcacagttct ggagaccagg aagtctaaga tctaggaatt agcagtcgag gtgtctggtg 282480
aggacccact ctcagcctca taggtggcac tttcttgctg tcgtggaagg gcagggggcc 282540
tctcctgggt ctcattgata ggaagagcac taatcccatt catggattcc accccatgac 282600
ctcatcacct cccacagctc cacctcctaa caccataaca ttggtgttaa tgttgtagtc 282660
tcagcagtgc accaagatat aacagtctct cattgtctga gataatgcca ggagttcttt 282720
gtcctacctc caagaagatt aaggagcaca gatacaaagg tgaggttaga gcgaaagttt 282780
aataagcaaa agaagaaagc tctctgccag cagagagggg ggcccaaaca ggatgctccc 282840
gtgaggctgg ggcccagggt ttttatggac tggaaagggg aaggaatgtg cttagtctgt 282900
gggctgtctt ggcccgcagc gtgactcagc ttggcccggg accctggccc aggaacccac 282960
tggagcccac tgtgcctatg cccacaaaag gagagagccc cctgactata caaaggacaa 283020
aggcatttct atcccaggtc ttgtccttta tctgattgaa ggtttttctg tctgtgcagc 283080
cgtgggcatg tctttaggca caatgccctg tgctagttcc ctcatcggtg cctgcagctt 283140
gacttttttt ccccaactgc tttttatgtt atatggggat gaggcactga cctgtggacc 283200
tggggctctc tggggaccct tcccttgcta tctacctaag gcaaactaac tcctttcatt 283260
aacacgtcag tatataaatt tggaggagca caaaaacatc cagaccatag cataatccct 283320
ctatccattt ctttctaaaa tatttattga ctaccttctc gtgccaggat acttaatctt 283380
cacagcaccc catgagttag gtattgctgt gtctatttta aagatgaggt atctaagaat 283440
cagaaaaaat aattttactt cagtttcata tctacaaaat gaggataatc gtttctgcct 283500
cctatgactg ttaaatggtt aagtagctga tatactagaa cagtgcctca gtagtaagca 283560
ctcagatgtt tgcaggtgtg ttttcactat ttttatttct caccaccatg ttggattgtg 283620
aacaccttga agatgggagc catgcacttt tcatctttaa tcccaagtac ctaagaatgc 283680
ccttcatata ttaggcatgc ggtaaatatt tgttgagtaa atgactcatt ttttccttga 283740
aaaaacccaa gggctgcagt ttgtgtcacc accattgata tgttgtgtat ctgaactaga 283800
tctgccattg agtgtcccca tcctgcgcac tctcccccac ccactggtgg ggtgggcacc 283860
aatctgcagt gtgatttgga gcaggagcta ctgaactact ttctaacagg aataattaac 283920
tttctagcaa acctctggca tcctcccatg ctgacccatc ctgaggtctg tgcctgcatt 283980
tgtctgaggt ggcactcatt gccactctca tgcctgttag ctctaacctg aagttgactt 284040

ctctgagatt agctgtcttc agtttgacac tctaatgtct tggtcttact cttgtacaga 284100
acttactttt tattcctcta gatcaggcct cagtttgtgg ttgttcagta atagtagtaa 284160
tagccaacat ttattgggta ttatactgtg cagtctaaga gctttataaa tgctagcctt 284220
ttatccatca tggtttcctt ctcttatgtc agaaatgttt ttaaacctag aaaaggtaaa 284280
gtaaatgaga cgtcattcct cattgatcag tgtaagctca ttccatccaa tctcgtgata 284340
tttttctctc aagcacaatc agaatttaga tttattattt tgaaaactag ctgtagaaaa 284400
ctaatgcaga gttcctattc cttcatctac tattaaatag ggactctgct ttcaagtata 284460
agcctagcgt gtttttctga caaagtaagc ctcctggttt ctaaaggaac taaaaatctt 284520
ttctaaatga tatgaaaccc atgacgtgaa gcacattaat ataataattt tcttttttaa 284580
aatactgttt ttggtacatt tgataggcct actttgatta ttatcaacaa attcatatct 284640
tccccaaagt gttttttcca cattgtattt tataacattg ttcatgtttt taacctgata 284700
actttcattt tcctgctaat atccttcagg tacttttaaa aattacagga atcattggcc 284760
ttcccatttt accagtgtgg gctttttcat cagtaaaaat aaaagggaaa acatggcttc 284820
tttaaactta aatgttttta cttatagaaa aacattctac acaaaaatct ccctgttttc 284880
tattcagaaa aataatgttc tgccagacta aattgagggg ttttgtgacc ttttttgttc 284940
attactctgt tttaaaatga tatatctgat gcatttatta atagaaaaag atctgtagct 285000
cgaagtgatc ctctctaggc aggatatagt gagtaaggga ccccatcact tcttccattg 285060
tacatatctg tgttgaattt tttatagtga acatttttta cttttgtgat cagaagaaaa 285120
atttctaaat gttatatttg tataaccata gtcatgttat ttctttgaat aacctatcag 285180
tgatttatat ttctgtgagg tttatatgtg tgtattctta aaacattatt ttcctttgcc 285240
tccttcaaat ttatacaggt cccaaaggca agcagttaga aggaactgaa tgtccactcc 285300
atgttgttca tccacccact ggggaagagt ttgctctggg atgtggagtg tgcagaaatg 285360
cccacacttt ttagaacacg cagatccttt gtctacagag agaaaaattg ccttcatccc 285420
ccaagaggat gcggtgaagt ttaaactctg ctcaggataa ggacgggacc attttacat 285480
ccatgaaaat gaaccattca cagtgcaaga aggataccaa ataccatgta cataattctt 285540
gctatgaaaa gtttccccat tattttggtt tatcttcttt tgaacaaatg acatcaaact 285600
tgtgaggtgt ttgcatgtgg ccattaccgt cattggcctg tgaagcattg gacatttata 285660
gataattgat ataaaagaat cgccatgccc atggactaag aacgatgctg gctttcaagc 285720
aaaaaagaaa aataatcatt gtttattgta tactgccttt ttgtaatcct gtacaattgc 285780
atcacgggtg gggataaaaa gaggaatatt ctggtttatt tcctagactg ttatttaaaa 285840
aaaaaaaaaa cattgtgtta ggacagcata taaatgtaat aagtatcaca ctgtatataa 285900
acatatcaat gtttgtcctg tataagaatt actaaattac aaatgcaatt tcatttaaac 285960
ttctaggtta agtttgagcc tgaaatttta atgaagtgca atactgagtg tgcctcatta 286020
tcttgcagct gtaaacatat tggaatgtac atgtcaataa aaccactgta catttttata 286080

cagtgataaa gtctaccact gtgggaggta ttgtttaaaa aacaaaattt gaacaccttt 286140
aaggtctaaa agtccagttt tcctcagaaa gaaatttact aacacaacac attcataatt 286200
ttcaaaactg ttagagaaaa taagaatagt aatgaagtgc aatgttggaa tcttaaaccc 286260
taagcagaag atcataaata tatatatata tattcgtagg taaatatatt cataccaaag 286320
acagaagaaa gcatttagga aattaacttc tattttaact agcagcattt ttaactttat 286380
ttatttattt atgagacagg gtcttacact gtcacccagg ctggagtaca gcggcgtgat 286440
ctcagctcac tgcagcctcc aactgggctc aagcgatcct ccagcctcag ccccctgagt 286500
agttgggact acaggcatgc accagcacca tgcccagcta attttgtaat ttttgtagag 286560
atggggtttt gccatgttgc cgaggctggt ctcaaactcc tagactcaag caatccccac 286620
tgctttggcc tcccaaagtg ctaggattac aggcgtgagc cattgcaccc agcccctttt 286680
tgaagttcat ataaagtaat aaaatcacta atttttattt cattttctat agacaacggt 286740
gtgtttaggt acatcttttt gacactcgag tcatgaaatt gccaaattat tgattgttga 286800
ctcatcatac atcagattag caaaactttt gttgttgttg ttgtatttga gacagtctca 286860
ctctgctgcc cacactggag tgcagtggca ccatcttggc tcactgcaac ctccacctcc 286920
caggttcaag cgattctcct gcctcagcct cccaaggagc tgggactaca ggcgtgtgcc 286980
acctcaccca gctaattttt gtatttttag tagggatggg gtttcaccag gctggtcttg 287040
aactcctgac ctcaggtgat ccgctcgcct ccggcctccc aaaattccgg tattacaggc 287100
gtgagccacc gcgcctggcc aagactagca aaagttttaa tcacattttg ttctaaattc 287160
caactgtggg aaatatagaa cttaatttgt caatatagaa ttactgacaa atttaatgac 287220
atttgtgtta agcatttatg agaaatttaa aacaatcttt gccttgaagg aacttaaaaa 287280
ataaagatca gatctgagca caggtaacta ttttacagaa tgttctaata agtttcataa 287340
aaatatccaa gccaaatgct tcaggattta ggaaaaatca ttattgtttt gtcgttggga 287400
gtgtagctaa gaggccattt aagctgcctt ttgaagaata agtagcattt caaggagcag 287460
agatgtggag aaaggcatga gaaataccta agtaggaaaa tctagaatat gatcaaagaa 287520
cagtaagtat gagtgttcaa gcttaggatt gactgaaaat gtttccagaa aacacaaagt 287580
acgtagagag actaaagctg ttacaagaaa actggagggg agaatgcagt gaggacactg 287640
tcagtatatt tgacttgaca ctgtcagtat atttgactta aattcacagg ccacaggaat 287700
gaaactaacc ttgagaatgc cacacagata agagacttta tggacactta atagctgcga 287760
agaataccat cttgcgtcag ttaactttgc tggcaaactg gtcagctcag tttgtaaatc 287820
gtagaggaaa agaaacaaac tattgagatg gcttggctaa aaaagccatc tcaagttatg 287880
aaagggataa aatcaacaaa gctgtgtagt gaatactcac aatgacttca acttatcttc 287940
atttatgagg aagtcacttt tagtcaggat ctctagcacc tcaaaaccag agaacataaa 288000
catcttagca ataaattatg acaaagcaca ggaaggagtg gctttggcag gcatttttag 288060
ctcaagcatc ctacacacaa ggaccatcct tcaaacactg catgtaacat ccaaggcagg 288120

agacagaaag cagaatcaca gtggtagatg tcagtgaatc ttcctggaaa gaaattgttt 288180
ttccgttata ttttcaagcc taggaaaagc catttttctt agtattatgg ctagactgtt 288240
taggcctaaa atctccctat tttatcaata agcttatttg gttttggttt ttggtttttt 288300
gagttttttg ggtttttttt ttttttttta agaaagggtc tcactctgtc gccccagctg 288360
gagtgcagtg gcgttatctc ggctcgcagc aacctctgcc ttttgggctc aagtgatcct 288420
cccacctcag ccccaagtag ctgggagtac aggcacgagc caccatgccc agctaatttt 288480
tgtaattgaa acagggtttc accatgttgg ccaggctggt ctcaaactcc tgagctgaag 288540
cgatctacct gcctcagcta ggattacagg tgtgagccac cgcacccaac ctcaataagc 288600
ttatttgata aaatatatgc aatgctccct ttattcactt ttcattcaga atgtttagta 288660
atttgtattg tttttcagat tttcagccca atatatctcc ctgcccactg tgtcactgta 288720
ttctacctat acatcatcac gtgtttctgc tattggctgt atgatggaac actgcggctc 288780
attttcctga aaactgccga tagtgcatag agtgctggga tggaaaccag aagctttgaa 288840
ttcaagcctt ggttctgcct tgtttttgct tgggtggcct tgagtcagcc acataccttt 288900
taaaatctca atttattaga aattattcca aatcaaaatc aaatgagaag gtatatacaa 288960
aagtgcttta tcccacaata aactattcaa gagagagcaa aggagaggac atttactcaa 289020
cacctcctaa aaggcagcca gtgaaattag gcattttatt taatcctcct ggcaactctg 289080
agagtaaagc attattaatc ccattttggc tgtttaaaga aattatttgc actagattcc 289140
agctgtagtt tagcttcaga aaaaaaaatc ctgagatgtg aattcacagc tttctgggtt 289200
taaagcccaa gctctatcac atcatgctat tattgttaca ttactgctag ttctatgaaa 289260
agaaatacta atttatgaaa tacatcttat ccaaaatggt tgggaccaga agtgtttctg 289320
atttcgggtt ttttcaggtt tgggaatatt tgcattacca gttgagcatc ccaaatctga 289380
aatccaaaat gctccagtga gcattttatt tgggtatcag gcatgtcagt actcaaagtt 289440
ttaaatttca ggacacttat cgtatggcca aggttgcaca ggggacaaga cagcatatgc 289500
ctccaagagt gttcagtgtc gtcaggaggt acctccaccc agatttccct tcaggggagg 289560
ccccacctca aggagtccag ttagctgaca gcctccagct ttaatcaccc ccacatatga 289620
cccaatgtca cactcctcca gggcagcccc agcctgtgat agagttcaga tggggttctc 289680
gggcctggcc atttctgccc aagcggcact cctgcgtgcg cggtcttgcg taagaactca 289740
ggccgactga gactgtctta cagctgccct gcagtcggag gcttttccta cccaatcttt 289800
ccttctctct tctttcctag atgccagacc tgcatcacag tctaatggct ctccctgccc 289860
actcctgctc cctcatcact ttatctttca tagggattac ccccaacaga ttttctgcac 289920
ttctaactta atcttggcat ctgcttccta gaggacctgc ctgacacagg agggtaatac 289980
attttaaatg acttacttat ggcttcattt ctcaaatgtc ttagggtggc ttagaaaaaa 290040

<210> 4
<211> 739

<212>  DNA
<213>  Rattus norvegicus

<400>  4
gcttgccaaa cgattcaggt gccaacaata ctaaattcac tacagagaag tgtacaggca      60

gtgttggttg gaaagattca agtccaggac tggtttagca atggcattaa gaaagcagct     120

ttaatgcaca agtggccatt aaaggaaata tctgttgatg aagatgacca gtgtctgctt     180

cagaatgatg gattttttct ttatttgtta tgcaaggatg gattatacaa aataggctct     240

ggatacagtg gaacagttag gggtcatata tataattcta catctcgtat cagaaacaga     300

aaagaaaaga agtcttggtt aggatatgcc cagggttact tgttatatcg ggatgtgaat     360

aaccacagta tgacagccat aagaataagc cccgaaacgc tggagcaaga cggcactgta     420

atgttaccag cttgcagatg ttgcttgaca cattcctgaa gctgctgtct tataatgtca     480

tacacgggta aagagcggac acgggaagtt gtcagtgtga cgccgaagga agacccagcc     540

ctcagcccca gccctcaagc ctgaagaagc ctgctgtcat ttcatcactt ctttgacacg     600

gactacagtt tcttcaataa aatcatttgc tttttccctg acaagggata ctacttctct     660

gtagcttttc atcaagaaga gaagtagtaa actgtcagat ttataggtgg ttactgaata     720

ccatgtttaa aatgaacat                                                  739

<210>  5
<211>  473
<212>  DNA
<213>  Rattus norvegicus

<400>  5
gcagcttcag agtgacagag gaactgtctc aacatcttca agaccagtgt ctacatcagc      60

aaagtcagag ctgccctcca agaacagcag atcagttaaa cctgatgggc gtgtgagccg     120

gactactgct gaccagaaga agccacgggg cacagaaggc ttatctgcta gtgaatccct     180

catgttaaaa tctgatgctg caaagttgag gtcagactcc catagtaggt cactgtcccc     240

taaccataac actttgcaga cactgaagtc tgatggaagg gtatcttcta gcttcagggc     300

tgaatcccca ggaccaggct ctaggtcatc ctctcctaag cccaagactc tgccgactcc     360

caggtctagc ccatctggtg ctagctctcc acgctcctcc tcaccgcagg ataaaaatct     420

acctcagaaa agcacagctc ctgctaagac aaaacttgac ccacctcggg agc            473

<210>  6
<211>  708
<212>  DNA
<213>  Rattus norvegicus

<220>
<221>  misc_feature
<222>  (615)..(615)
<223>  n is a, c, g, or t

<400>  6
tttttttttt ttttttttcc tttttcgttt ttttattctc tttctcacat tctttctttt      60

taaggactgc acaggaacct ggacttggaa aaatcatatt ctgggaagca gctttgattg 120

tagccaaaga gatgtcctcc cagaaggcca ctaagtgttg taatgttaag gggagcggag 180

acctagactt cactgagtga tgcatggaca tttcaaaagt ggcttccgat tttccgtctt 240

cacacttctc atgtagaggt ggctccttaa gcatagacag gaccttattt ttgttgatac 300

ttcccatctt ggatatgtct ggtccgtgag gtgaaatgtt gaacatattt agggcagatg 360

atatttccaa cgaatgtcta ttttttaact tgatttcctt ttcctctgtg ggtggctggc 420

tattcaaact acttcttatg ggagcatgtt ctttggaaag ttcaggatta aacttcagga 480

aggaagagca ggccattgca tcatgtacta tgccttcatg ccacagaaaa gaggcaaaga 540

cagctcgggc acactcagcc acggacgggg acatggcttg cttggctggc tctaaagatt 600

tggctccatc tcctnttgaa agaaagttag acttttcttt tttgggcctg gtgtgtctat 660

tagcacattt ccgacttatt ttggggtgat gctctcattt cctgttca 708

<210> 7
<211> 529
<212> DNA
<213> Rattus norvegicus

<400> 7
taagcttgct ctgtccctgc cccgtacatc tcagtaactc ctgatgcaag tcccaatgtc 60

tttgaagagc cggagagcaa tatgaagtcg atgccaccaa gtttggaaac gagcccgata 120

actgacaccg acctggctaa gagaactgtc ttccagaggt catactcagt tgtcgcttcg 180

gaatatgata aacaacactc cattttacct gcacgagtta aagccatccc tagaaggaga 240

gtgaacagtg gagacacgga agttgggtct tctctcttgc gacatccgtc accggagctt 300

tcccggctta tatcagccca cagctctctc tccaaaggag agcgaaactt ccagtggcca 360

gtcttagctt tcgtcataca gcatcatgat ttagaagggc tggaaatcgc aatgaagcag 420

gccttaagga agtcggcttg ccgtgtgttt gctatggagg cattcaactg gcttctctgt 480

aatgtcatcc aaacaacgtc tctgcatgac attctctggc actttgtgg 529

<210> 8
<211> 667
<212> DNA
<213> Rattus norvegicus

<400> 8
acgaagggct gcataagagt gaagcgatca cgacgccggg cgtcaggttt tacaatgatc 60

cagccggcta tgccatgaac agatacgcat attatgtttg ctacaaatgc agaaaggcat 120

attttggtgg tgaagctcgc tgtgatgctg aggctggaca aggagacgac tacgacccca 180

gagagctcat ctgtggagcc tgttctgatg tgtctagggc tcagatgtgt cccaaacatg 240

gaacagactt tctagaatac aaatgtcgct actgctgttc agtggctgtc ttcttctgtt 300

ttggaacaac acatttctgc aatgcttgtc atgatgattt tcaaagaatg accagcattc 360

ctaaggaaga gctcccacac tgtcctgcag gtcccaaagg caaacagcta gaaggaactg 420

aatgtccact ccatgttgtt catccgccca cgggggaaga gtttgctctt ggttgtggag 480

tgtgcagaaa tgctcacacg ttttagaact ttcagatcct ttgtctacaa agaggatagt 540

tgccttcatc ccctgggagg atgcagtgaa actttaaact ctgctcaagg ataaggaacg 600

gggaccattt ttacattctg aaaacgaacc attttccagt gccaggaagt gatgccccaa 660

atacctg 667

<210> 9
<211> 652
<212> DNA
<213> Rattus norvegicus

<220>
<221> misc_feature
<222> (605)..(605)
<223> n is a, c, g, or t

<400> 9
tttttttttt ttttttttga cgctctcact gtgtagagca cagtggttta ctgacgtaca 60

ttccaatatg ctgacagctg caaggtagtg aggcacaccc agtattgcac tgcattagag 120

cttcaggctc aaacgtaacc tacaggttta aatgagactg ctgtgtcatt tagtaattct 180

catacaggac aaacactgat agctttatat acagtctgat acttattaca attataggcc 240

ttactaacac aatttttttt tttaaataac agtctaggaa agaaaccaga atattcctct 300

ttttataccc acccgtgatg caattgtaca ggattacaaa aaggcagtat acaataaaca 360

gtgattattt ttcttttttt gcttgaaagc cagcatcatt cttagtccat gagtatggag 420

atccttttat atcaattatc tataaatgtc caatgctcca caggccagtg acggtaatgg 480

ccacatgcaa acacctcaca agtttgatgt cttggttcaa agatgataaa ccaaaacaat 540

ggggaaacgt tcgtagcaag aattatgtac acagtatttg gcatcactcc tgcactggaa 600

atggntcgtt ttcagaatgt aaaatggtcc cgttcttatc ctgagcagag tt 652

<210> 10
<211> 18
<212> DNA
<213> Artificial Sequence

<400> 10
gaagggtggg gccaaaag 18

<210> 11
<211> 20
<212> DNA
<213> Artificial Sequence

<400> 11
ggatgcaggg atgatgttct 20

<210> 12
<211> 24
<212> DNA
<213> Artificial Sequence

<400>  12
ggtggtgaag ctcgctgtga tgct                                              24

<210>  13
<211>  24
<212>  DNA
<213>  Artificial Sequence

<400>  13
cgtgtgagca tttctgcaca ctcc                                              24

<210>  14
<211>  18
<212>  DNA
<213>  Artificial Sequence

<400>  14
gactggttta gcaatggc                                                     18

<210>  15
<211>  18
<212>  DNA
<213>  Artificial Sequence

<400>  15
gccattgcta aaccagtc                                                     18

<210>  16
<211>  18
<212>  DNA
<213>  Artificial Sequence

<400>  16
gcaattgcta aatcagta                                                     18

<210>  17
<211>  1010
<212>  PRT
<213>  Homo sapiens

<400>  17

Asn Asn His Ser Met Thr Ala Ile Arg Ile Ser Pro Glu Thr Leu Glu
1               5                   10                  15

Gln Asp Gly Thr Val Met Leu Pro Asp Cys His Thr Glu Gly Gln Asn
            20                  25                  30

Ile Leu Phe Thr Asp Gly Glu Tyr Ile Asn Gln Ile Ala Ala Ser Arg
        35                  40                  45

Asp Asp Gly Phe Val Val Arg Ile Phe Ala Thr Ser Thr Glu Pro Val
    50                  55                  60

Leu Gln Gln Glu Leu Gln Leu Lys Leu Ala Arg Lys Cys Leu His Ala
65                  70                  75                  80

Cys Arg Ile Ser Leu Phe Asp Leu Glu Lys Asp Leu His Ile Ile Ser
                85                  90                  95

Thr Gly Phe Asp Glu Glu Ser Ala Ile Leu Gly Ala Gly Arg Glu Phe
            100                 105                 110

Ala Leu Met Lys Thr Ala Asn Gly Lys Ile Tyr Tyr Thr Gly Lys Tyr
        115                 120                 125

Gln Ser Leu Gly Ile Lys Gln Gly Gly Pro Ser Ala Gly Lys Trp Val
    130                 135                 140

Glu Leu Pro Ile Thr Lys Ser Pro Lys Ile Val His Phe Ser Val Gly
145                 150                 155                 160

His Asp Gly Ser His Ala Leu Leu Val Ala Glu Asp Gly Ser Ile Phe
                165                 170                 175

Phe Thr Gly Ser Ala Ser Lys Gly Glu Asp Gly Glu Ser Ile Lys Ser
            180                 185                 190

Arg Arg Gln Ser Lys Pro Tyr Lys Pro Lys Lys Ile Ile Lys Met Glu
        195                 200                 205

Gly Lys Ile Val Val Tyr Thr Ala Cys Asn Asn Gly Ser Ser Ser Val
    210                 215                 220

Ile Ser Lys Asp Gly Glu Leu Tyr Met Phe Gly Lys Asp Ala Ile Tyr
225                 230                 235                 240

Ser Asp Ser Ser Ser Leu Val Thr Asp Leu Lys Gly His Phe Val Thr
                245                 250                 255

Gln Val Ala Met Gly Lys Ala His Thr Cys Val Leu Met Lys Asn Gly
            260                 265                 270

Glu Val Trp Thr Phe Gly Val Asn Asn Lys Gly Gln Cys Gly Arg Asp
        275                 280                 285

Thr Gly Ala Met Asn Gln Gly Gly Lys Gly Phe Gly Val Glu Asn Met
    290                 295                 300

Ala Thr Ala Met Asp Glu Asp Leu Glu Glu Glu Leu Asp Glu Lys Asp
305                 310                 315                 320

Glu Lys Ser Met Met Cys Pro Pro Gly Met His Lys Trp Lys Leu Glu
                325                 330                 335

Gln Cys Met Val Cys Thr Val Cys Gly Asp Cys Thr Gly Tyr Gly Ala
            340                 345                 350

Ser Cys Val Ser Ser Gly Arg Pro Asp Arg Val Pro Gly Gly Ile Cys
        355                 360                 365

Gly Cys Gly Ser Gly Glu Ser Gly Cys Ala Val Cys Gly Cys Cys Lys
    370                 375                 380

Ala Cys Ala Arg Glu Leu Asp Gly Gln Glu Ala Arg Gln Arg Gly Ile
385                 390                 395                 400

Leu Asp Ala Val Lys Glu Met Ile Pro Leu Asp Leu Leu Leu Ala Val
                405                 410                 415

Pro Val Pro Gly Val Asn Ile Glu Glu His Leu Gln Leu Arg Gln Glu
            420                 425                 430

Glu Lys Arg Gln Arg Val Ile Arg Arg His Arg Leu Glu Glu Gly Arg
        435                 440                 445

Gly Pro Leu Val Phe Ala Gly Pro Ile Phe Met Asn His Arg Glu Gln
    450                 455                 460

Ala Leu Ala Arg Leu Arg Ser His Pro Ala His Val Lys His Lys Arg
465                 470                 475                 480

Asp Lys His Lys Asp Gly Ser Gly Glu Arg Gly Glu Lys Asp Ala Ser
                485                 490                 495

Lys Ile Thr Thr Tyr Pro Pro Gly Ser Val Arg Phe Asp Cys Glu Leu
            500                 505                 510

Arg Ala Val Gln Val Ser Cys Gly Phe His His Ser Val Val Leu Met
        515                 520                 525

Glu Asn Gly Asp Val Tyr Thr Phe Gly Tyr Gly Gln His Gly Gln Leu
    530                 535                 540

Gly His Gly Asp Val Asn Ser Arg Gly Cys Pro Thr Leu Val Gln Ala
545                 550                 555                 560

Leu Pro Gly Pro Ser Thr Gln Val Thr Ala Gly Ser Asn His Thr Ala
                565                 570                 575

Val Leu Leu Met Asp Gly Gln Val Phe Thr Phe Gly Ser Phe Ser Lys
            580                 585                 590

Gly Gln Leu Gly Arg Pro Ile Leu Asp Val Pro Tyr Trp Asn Ala Lys
        595                 600                 605

Pro Ala Pro Met Pro Asn Ile Gly Ser Lys Tyr Gly Arg Lys Ala Thr
    610                 615                 620

Trp Ile Gly Ala Ser Gly Asp Gln Thr Phe Leu Arg Ile Asp Glu Ala
625                 630                 635                 640

Leu Ile Asn Ser His Val Leu Ala Thr Ser Glu Ile Phe Ala Ser Lys
                645                 650                 655

His Ile Ile Gly Leu Val Pro Ala Ser Ile Ser Glu Pro Pro Pro Phe
            660                 665                 670

Lys Cys Leu Leu Ile Asn Lys Val Asp Gly Ser Cys Lys Thr Phe Asn
        675                 680                 685

Asp Ser Glu Gln Glu Asp Leu Gln Gly Phe Gly Val Cys Leu Asp Pro
    690                 695                 700

Val Tyr Asp Val Ile Trp Arg Phe Arg Pro Asn Thr Arg Glu Leu Trp
705                 710                 715                 720

Cys Tyr Asn Ala Val Val Ala Asp Ala Arg Leu Pro Ser Ala Ala Asp
                725                 730                 735

Met Gln Ser Arg Cys Ser Ile Leu Ser Pro Glu Leu Ala Leu Pro Thr
            740                 745                 750

Gly Ser Arg Ala Leu Thr Thr Arg Ser His Ala Ala Leu His Ile Leu
        755                 760                 765

Gly Cys Leu Asp Thr Leu Ala Ala Met Gln Asp Leu Lys Met Gly Val
    770                 775                 780

Ala Ser Thr Glu Glu Glu Thr Gln Ala Val Met Lys Val Tyr Ser Lys
785                 790                 795                 800

Glu Asp Tyr Ser Val Val Asn Arg Phe Glu Ser His Gly Gly Gly Trp
                805                 810                 815

Gly Tyr Ser Ala His Ser Val Glu Ala Ile Arg Phe Ser Ala Asp Thr
            820                 825                 830

Asp Ile Leu Leu Gly Gly Leu Gly Leu Phe Gly Gly Arg Gly Glu Tyr
        835                 840                 845

Thr Ala Lys Ile Lys Leu Phe Glu Leu Gly Pro Asp Gly Gly Asp His
    850                 855                 860

Glu Thr Asp Gly Asp Leu Leu Ala Glu Thr Asp Val Leu Ala Tyr Asp
865                 870                 875                 880

Cys Ala Ala Arg Glu Lys Tyr Ala Met Met Phe Asp Glu Pro Val Leu
                885                 890                 895

Leu Gln Ala Gly Trp Trp Tyr Val Ala Trp Ala Arg Val Ser Gly Pro
            900                 905                 910

Ser Ser Asp Cys Gly Ser His Gly Gln Ala Ser Ile Thr Thr Asp Asp
        915                 920                 925

Gly Val Val Phe Gln Phe Lys Ser Ser Lys Lys Ser Asn Asn Gly Thr
    930                 935                 940

Asp Val Asn Ala Gly Gln Ile Pro Gln Leu Leu Tyr Arg Leu Pro Thr
945                 950                 955                 960

Ser Asp Gly Ser Ala Ser Lys Gly Lys Gln Gln Thr Ser Glu Pro Val
                965                 970                 975

His Ile Leu Lys Arg Ser Phe Ala Arg Thr Val Ser Val Glu Cys Phe
            980                 985                 990

Glu Ser Leu Leu Ser Ile Leu His Trp Ser Trp Thr Thr Leu Val Leu
        995                 1000                1005

Gly Val
    1010

<210> 18
<211> 617
<212> PRT
<213> Homo sapiens

<400> 18

Ile Phe Ala Thr Ser Thr Glu Pro Val Leu Gln Gln Glu Leu Gln Leu
1               5                   10                  15

Lys Leu Ala Arg Lys Cys Leu His Ala Cys Arg Ile Ser Leu Phe Asp
            20                  25                  30

Leu Glu Lys Asp Leu His Ile Ile Ser Thr Gly Phe Asp Glu Glu Ser
        35                  40                  45

Ala Ile Leu Gly Ala Gly Arg Glu Phe Ala Leu Met Lys Thr Ala Asn
    50                  55                  60

Gly Lys Ile Tyr Tyr Thr Gly Lys Tyr Gln Ser Leu Gly Ile Lys Gln
65                  70                  75                  80

Gly Gly Pro Ser Ala Gly Lys Trp Val Glu Leu Pro Ile Thr Lys Ser
                85                  90                  95

Pro Lys Ile Val His Phe Ser Val Gly His Asp Gly Ser His Ala Leu
            100                 105                 110

Leu Val Ala Glu Asp Gly Ser Ile Phe Phe Thr Gly Ser Ala Ser Lys

115                 120                 125

Gly Glu Asp Gly Glu Ser Ile Lys Ser Arg Arg Gln Ser Lys Pro Tyr
    130                 135                 140

Lys Pro Lys Lys Ile Ile Lys Met Glu Gly Lys Ile Val Val Tyr Thr
145                 150                 155                 160

Ala Cys Asn Asn Gly Ser Ser Ser Val Ile Ser Lys Asp Gly Glu Leu
                165                 170                 175

Tyr Met Phe Gly Lys Asp Ala Ile Tyr Ser Asp Ser Ser Ser Leu Val
            180                 185                 190

Thr Asp Leu Lys Gly His Phe Val Thr Gln Val Ala Met Gly Lys Ala
        195                 200                 205

His Thr Cys Val Leu Met Lys Asn Gly Glu Val Trp Thr Phe Gly Val
    210                 215                 220

Asn Asn Lys Gly Gln Cys Gly Arg Asp Thr Gly Ala Met Asn Gln Gly
225                 230                 235                 240

Gly Lys Gly Phe Gly Val Glu Asn Met Ala Thr Ala Met Asp Glu Asp
                245                 250                 255

Leu Glu Glu Glu Leu Asp Glu Lys Asp Glu Lys Ser Met Met Cys Pro
            260                 265                 270

Pro Gly Met His Lys Trp Lys Leu Glu Gln Cys Met Val Cys Thr Val
        275                 280                 285

Cys Gly Asp Cys Thr Gly Tyr Gly Ala Ser Cys Val Ser Ser Gly Arg
    290                 295                 300

Pro Asp Arg Val Pro Gly Gly Ile Cys Gly Cys Gly Ser Gly Glu Ser
305                 310                 315                 320

Gly Cys Ala Val Cys Gly Cys Cys Lys Ala Cys Ala Arg Glu Leu Asp
                325                 330                 335

Gly Gln Glu Ala Arg Gln Arg Gly Ile Leu Asp Ala Val Lys Glu Met
            340                 345                 350

Ile Pro Leu Asp Leu Leu Leu Ala Val Pro Val Pro Gly Val Asn Ile
        355                 360                 365

Glu Glu His Leu Gln Leu Arg Gln Glu Glu Lys Arg Gln Arg Val Ile
    370                 375                 380

Arg Arg His Arg Leu Glu Glu Gly Arg Gly Pro Leu Val Phe Ala Gly

385                 390                 395                 400

Pro Ile Phe Met Asn His Arg Glu Gln Ala Leu Ala Arg Leu Arg Ser
                405                 410                 415

His Pro Ala His Val Lys His Lys Arg Asp Lys His Lys Asp Gly Ser
            420                 425                 430

Gly Glu Arg Gly Glu Lys Asp Ala Ser Lys Ile Thr Thr Tyr Pro Pro
        435                 440                 445

Gly Ser Val Arg Phe Asp Cys Glu Leu Arg Ala Val Gln Val Ser Cys
    450                 455                 460

Gly Phe His His Ser Val Val Leu Met Glu Asn Gly Asp Val Tyr Thr
465                 470                 475                 480

Phe Gly Tyr Gly Gln His Gly Gln Leu Gly His Gly Asp Val Asn Ser
                485                 490                 495

Arg Gly Cys Pro Thr Leu Val Gln Ala Leu Pro Gly Pro Ser Thr Gln
            500                 505                 510

Val Thr Ala Gly Ser Asn His Thr Ala Val Leu Leu Met Asp Gly Gln
        515                 520                 525

Val Phe Thr Phe Gly Ser Phe Ser Lys Gly Gln Leu Gly Arg Pro Ile
    530                 535                 540

Leu Asp Val Pro Tyr Trp Asn Ala Lys Pro Ala Pro Met Pro Asn Ile
545                 550                 555                 560

Gly Ser Lys Tyr Gly Arg Lys Ala Thr Trp Ile Gly Ala Ser Gly Asp
                565                 570                 575

Gln Thr Phe Leu Arg Ile Asp Glu Ala Leu Ile Asn Ser His Val Leu
            580                 585                 590

Ala Thr Ser Glu Ile Phe Ala Ser Lys His Ile Ile Gly Leu Val Pro
        595                 600                 605

Ala Ser Ile Ser Glu Pro Pro Pro Phe
    610                 615

<210>  19
<211>  567
<212>  PRT
<213>  Homo sapiens

<400>  19

Gly Arg Glu Phe Ala Leu Met Lys Thr Ala Asn Gly Lys Ile Tyr Tyr
1               5                   10                  15

Thr Gly Lys Tyr Gln Ser Leu Gly Ile Lys Gln Gly Gly Pro Ser Ala
            20                  25                  30

Gly Lys Trp Val Glu Leu Pro Ile Thr Lys Ser Pro Lys Ile Val His
        35                  40                  45

Phe Ser Val Gly His Asp Gly Ser His Ala Leu Leu Val Ala Glu Asp
    50                  55                  60

Gly Ser Ile Phe Phe Thr Gly Ser Ala Ser Lys Gly Glu Asp Gly Glu
65                  70                  75                  80

Ser Ile Lys Ser Arg Arg Gln Ser Lys Pro Tyr Lys Pro Lys Lys Ile
                85                  90                  95

Ile Lys Met Glu Gly Lys Ile Val Val Tyr Thr Ala Cys Asn Asn Gly
            100                 105                 110

Ser Ser Ser Val Ile Ser Lys Asp Gly Glu Leu Tyr Met Phe Gly Lys
        115                 120                 125

Asp Ala Ile Tyr Ser Asp Ser Ser Ser Leu Val Thr Asp Leu Lys Gly
    130                 135                 140

His Phe Val Thr Gln Val Ala Met Gly Lys Ala His Thr Cys Val Leu
145                 150                 155                 160

Met Lys Asn Gly Glu Val Trp Thr Phe Gly Val Asn Asn Lys Gly Gln
                165                 170                 175

Cys Gly Arg Asp Thr Gly Ala Met Asn Gln Gly Gly Lys Gly Phe Gly
            180                 185                 190

Val Glu Asn Met Ala Thr Ala Met Asp Glu Asp Leu Glu Glu Glu Leu
        195                 200                 205

Asp Glu Lys Asp Glu Lys Ser Met Met Cys Pro Pro Gly Met His Lys
    210                 215                 220

Trp Lys Leu Glu Gln Cys Met Val Cys Thr Val Cys Gly Asp Cys Thr
225                 230                 235                 240

Gly Tyr Gly Ala Ser Cys Val Ser Ser Gly Arg Pro Asp Arg Val Pro
                245                 250                 255

Gly Gly Ile Cys Gly Cys Gly Ser Gly Glu Ser Gly Cys Ala Val Cys
            260                 265                 270

Gly Cys Cys Lys Ala Cys Ala Arg Glu Leu Asp Gly Gln Glu Ala Arg
        275                 280                 285

Gln Arg Gly Ile Leu Asp Ala Val Lys Glu Met Ile Pro Leu Asp Leu
    290                 295                 300

Leu Leu Ala Val Pro Val Pro Gly Val Asn Ile Glu Glu His Leu Gln
305                 310                 315                 320

Leu Arg Gln Glu Glu Lys Arg Gln Arg Val Ile Arg Arg His Arg Leu
                325                 330                 335

Glu Glu Gly Arg Gly Pro Leu Val Phe Ala Gly Pro Ile Phe Met Asn
            340                 345                 350

His Arg Glu Gln Ala Leu Ala Arg Leu Arg Ser His Pro Ala His Val
        355                 360                 365

Lys His Lys Arg Asp Lys His Lys Asp Gly Ser Gly Glu Arg Gly Glu
    370                 375                 380

Lys Asp Ala Ser Lys Ile Thr Thr Tyr Pro Pro Gly Ser Val Arg Phe
385                 390                 395                 400

Asp Cys Glu Leu Arg Ala Val Gln Val Ser Cys Gly Phe His His Ser
                405                 410                 415

Val Val Leu Met Glu Asn Gly Asp Val Tyr Thr Phe Gly Tyr Gly Gln
            420                 425                 430

His Gly Gln Leu Gly His Gly Asp Val Asn Ser Arg Gly Cys Pro Thr
        435                 440                 445

Leu Val Gln Ala Leu Pro Gly Pro Ser Thr Gln Val Thr Ala Gly Ser
    450                 455                 460

Asn His Thr Ala Val Leu Leu Met Asp Gly Gln Val Phe Thr Phe Gly
465                 470                 475                 480

Ser Phe Ser Lys Gly Gln Leu Gly Arg Pro Ile Leu Asp Val Pro Tyr
                485                 490                 495

Trp Asn Ala Lys Pro Ala Pro Met Pro Asn Ile Gly Ser Lys Tyr Gly
            500                 505                 510

Arg Lys Ala Thr Trp Ile Gly Ala Ser Gly Asp Gln Thr Phe Leu Arg
        515                 520                 525

Ile Asp Glu Ala Leu Ile Asn Ser His Val Leu Ala Thr Ser Glu Ile
    530                 535                 540

Phe Ala Ser Lys His Ile Ile Gly Leu Val Pro Ala Ser Ile Ser Glu
545                 550                 555                 560

Pro Pro Pro Phe Lys Cys Leu
                565

<210>  20
<211>  301
<212>  PRT
<213>  Homo sapiens

<400>  20

Ala Pro Met Pro Asn Ile Gly Ser Lys Tyr Gly Arg Lys Ala Thr Trp
1               5                   10                  15

Ile Gly Ala Ser Gly Asp Gln Thr Phe Leu Arg Ile Asp Glu Ala Leu
            20                  25                  30

Ile Asn Ser His Val Leu Ala Thr Ser Glu Ile Phe Ala Ser Lys His
        35                  40                  45

Ile Ile Gly Leu Val Pro Ala Ser Ile Ser Glu Pro Pro Pro Phe Lys
    50                  55                  60

Cys Leu Leu Ile Asn Lys Val Asp Gly Ser Cys Lys Thr Phe Asn Asp
65                  70                  75                  80

Ser Glu Gln Glu Asp Leu Gln Gly Phe Gly Val Cys Leu Asp Pro Val
                85                  90                  95

Tyr Asp Val Ile Trp Arg Phe Arg Pro Asn Thr Arg Glu Leu Trp Cys
            100                 105                 110

Tyr Asn Ala Val Val Ala Asp Ala Arg Leu Pro Ser Ala Ala Asp Met
        115                 120                 125

Gln Ser Arg Cys Ser Ile Leu Ser Pro Glu Leu Ala Leu Pro Thr Gly
    130                 135                 140

Ser Arg Ala Leu Thr Thr Arg Ser His Ala Ala Leu His Ile Leu Gly
145                 150                 155                 160

Cys Leu Asp Thr Leu Ala Ala Met Gln Asp Leu Lys Met Gly Val Ala
                165                 170                 175

Ser Thr Glu Glu Glu Thr Gln Ala Val Met Lys Val Tyr Ser Lys Glu
            180                 185                 190

Asp Tyr Ser Val Val Asn Arg Phe Glu Ser His Gly Gly Gly Trp Gly
        195                 200                 205

Tyr Ser Ala His Ser Val Glu Ala Ile Arg Phe Ser Ala Asp Thr Asp
    210                 215                 220

Ile Leu Leu Gly Gly Leu Gly Leu Phe Gly Gly Arg Gly Glu Tyr Thr
225                 230                 235                 240

Ala Lys Ile Lys Leu Phe Glu Leu Gly Pro Asp Gly Gly Asp His Glu
                245                 250                 255

Thr Asp Gly Asp Leu Leu Ala Glu Thr Asp Val Leu Ala Tyr Asp Cys
            260                 265                 270

Ala Ala Arg Glu Lys Tyr Ala Met Met Phe Asp Glu Pro Val Leu Leu
        275                 280                 285

Gln Ala Gly Trp Trp Tyr Val Ala Trp Ala Arg Val Ser
    290                 295                 300

<210>  21
<211>  101
<212>  PRT
<213>  Homo sapiens

<400>  21

Ala Pro Met Pro Asn Ile Gly Ser Lys Tyr Gly Arg Lys Ala Thr Trp
1               5                   10                  15

Ile Gly Ala Ser Gly Asp Gln Thr Phe Leu Arg Ile Asp Glu Ala Leu
            20                  25                  30

Ile Asn Ser His Val Leu Ala Thr Ser Glu Ile Phe Ala Ser Lys His
        35                  40                  45

Ile Ile Gly Leu Val Pro Ala Ser Ile Ser Glu Pro Pro Pro Phe Lys
    50                  55                  60

Cys Leu Leu Ile Asn Lys Val Asp Gly Ser Cys Lys Thr Phe Asn Asp
65                  70                  75                  80

Ser Glu Gln Glu Asp Leu Gln Gly Phe Gly Val Cys Leu Asp Pro Val
                85                  90                  95

Tyr Asp Val Ile Trp
            100

<210>  22
<211>  204
<212>  PRT
<213>  Homo sapiens

<400>  22

Asp Glu Ala Leu Ile Asn Ser His Val Leu Ala Thr Ser Glu Ile Phe
1               5                   10                  15

Ala Ser Lys His Ile Ile Gly Leu Val Pro Ala Ser Ile Ser Glu Pro

            20                  25                  30

Pro Pro Phe Lys Cys Leu Leu Ile Asn Lys Val Asp Gly Ser Cys Lys
        35                  40                  45

Thr Phe Asn Asp Ser Glu Gln Glu Asp Leu Gln Gly Phe Gly Val Cys
    50                  55                  60

Leu Asp Pro Val Tyr Asp Val Ile Trp Arg Phe Arg Pro Asn Thr Arg
65                  70                  75                  80

Glu Leu Trp Cys Tyr Asn Ala Val Val Ala Asp Ala Arg Leu Pro Ser
                85                  90                  95

Ala Ala Asp Met Gln Ser Arg Cys Ser Ile Leu Ser Pro Glu Leu Ala
            100                 105                 110

Leu Pro Thr Gly Ser Arg Ala Leu Thr Thr Arg Ser His Ala Ala Leu
        115                 120                 125

His Ile Leu Gly Cys Leu Asp Thr Leu Ala Ala Met Gln Asp Leu Lys
    130                 135                 140

Met Gly Val Ala Ser Thr Glu Glu Glu Thr Gln Ala Val Met Lys Val
145                 150                 155                 160

Tyr Ser Lys Glu Asp Tyr Ser Val Val Asn Arg Phe Glu Ser His Gly
                165                 170                 175

Gly Gly Trp Gly Tyr Ser Ala His Ser Val Glu Ala Ile Arg Phe Ser
            180                 185                 190

Ala Asp Thr Asp Ile Leu Leu Gly Gly Leu Gly Leu [?]
195 200

<210> 23
<211> 38
<212> PRT
<213> Homo sapiens

<400> 23

Asp Glu Ala Leu Ile Asn Ser His Val Leu Ala Thr Ser Glu Ile Phe
1               5                   10                  15

Ala Ser Lys His Ile Ile Gly Leu Val Pro Ala Ser Ile Ser Glu Pro
            20                  25                  30

Pro Pro Phe Lys Cys Leu [?]
35

<210> 24
<211> 3030

<212>  DNA
<213>  Homo sapiens

<400>  24
aataaccaca gcatgacagc cataaggata agccctgaaa cactggagca agatggtact      60

gtgatgttac cagattgcca cactgaaggt caaaatattt tattcactga tggagaatat     120

attaatcaga tagctgcttc aagagatgat ggctttgttg tcagaatatt tgccacaagc     180

actgaacctg ttctacagca agaattgcaa cttaaactgg ctagaaaatg cttacatgcc     240

tgtcgtatct cattattcga tctggaaaag gacttgcata ttataagtac aggatttgat     300

gaggagtcag caattcttgg tgcaggacga gagtttgcgc taatgaaaac agcaaatgga     360

aagatatatt acactggcaa ataccagagt cttggaatca aacaaggtgg tccttcagca     420

ggaaaatggg ttgagctacc aattacaaaa tctccaaaga tagtacactt ctcagttgga     480

cacgatggct ctcacgccct tttagttgca gaagatggga gcatattctt tacaggatct     540

gctagtaaag gagaagatgg agaatcaatt aagagcagac ggcaatccaa accttataaa     600

cctaaaaaga taattaagat ggaaggaaag attgtggtat atacagcctg caataatgga     660

agtagttctg ttatttctaa agatggagaa ctctacatgt ttggaaaaga tgccatttac     720

tctgatagtt caagtttggt aactgatttg aagggccatt ttgtaactca ggtagctatg     780

ggcaaagctc acacttgtgt tttaatgaag aatggagagg tgtggacatt tggtgtaaat     840

aataaaggac agtgtggacg agatactggt gccatgaacc aaggtgggaa agggtttgga     900

gttgaaaata tggcaacagc aatggatgaa gacctggaag aagaactaga tgaaaaagat     960

gagaagtcta tgatgtgccc tccaggcatg cacaaatgga agctggagca gtgcatggtt    1020

tgcactgtct gtggagactg tacaggttat ggagccagct gtgtcagtag tggacggcca    1080

gacagagtcc ccggagggat ctgtggttgt ggttccggag aatctggttg tgctgtgtgt    1140

ggatgttgca aggcctgtgc aagagagtta gatggtcaag aggcaagaca aagaggaatt    1200

cttgatgcag tgaaagaaat gataccttta gatcttcttt tagctgtccc agtgcccggg    1260

gttaacattg aagaacacct tcagttacga caagaagaaa aacggcaacg tgtaatcaga    1320

aggcacagat tagaggaagg aagaggcccc cttgtatttg ctggtcctat ttttatgaac    1380

catcgagaac aggctctagc cagactcaga tcccatccag cacacgtaaa gcataaacgg    1440

gacaagcaca aagatggaag tggagaaaga ggcgaaaagg atgcaagcaa aatcacaaca    1500

taccctccag gctctgtgcg atttgactgt gagctccggg cagtccaagt cagctgtgga    1560

tttcaccatt cagtggtttt aatggaaaat ggagatgtct atacatttgg ttatgggcag    1620

catgggcagc taggacatgg agatgtcaac tccaggggat gtcccactct tgttcaagca    1680

ttgccaggcc ctagcacaca agtcactgca ggcagcaacc atacggcagt acttttaatg    1740

gatggacagg tcttcacatt tggaagtttt tctaaaggac aactgggcag accaattttg    1800

gatgtgccat attggaatgc aaagccagct cccatgccta acattggatc aaaatatgga    1860

agaaaagcta cttggatagg tgcaagtggg gaccaaactt ttttacgaat tgatgaagca    1920

cttattaatt ctcatgtact tgctacatca gaaatttttg ccagtaaaca cataataggc 1980

ttggtacctg cttctatatc agaacctcct ccatttaaat gccttctgat aaataaagtg 2040

gatgggagtt gtaaaacttt taatgactca gaacaagagg atctgcaagg atttggtgtg 2100

tgtcttgatc ctgtatatga tgtaatttgg aggtttcgac caaatactag agagctgtgg 2160

tgttacaatg cggtggttgc tgatgccagg cttccctctg cagcagacat gcagtccaga 2220

tgtagtatcc taagtcctga acttgcctta ccaacaggat caagggccct cactacccga 2280

tctcatgcag ctttgcacat tttaggttgt cttgatacct tggcagctat gcaggactta 2340

aaaatgggtg ttgcaagtac agaggaagag actcaagcag taatgaaggt ttattctaaa 2400

gaagattata gtgtggtaaa caggtttgaa agtcatggag gaggctgggg ttattctgcc 2460

cattcagtag aagctatacg tttcagtgcc gacactgata ttttacttgg tggtcttggt 2520

ctgtttggag gtagaggaga atatactgct aaaattaagc tgtttgaatt gggtcctgat 2580

ggaggagatc atgaaactga tggtgacctt cttgcagaga ctgatgtatt ggcttatgac 2640

tgtgctgcta gagaaaaata tgcaatgatg tttgatgagc ctgttctcct gcaagctggg 2700

tggtggtatg tggcatgggc ccgagtgtca ggacccagca gtgactgtgg atctcatgga 2760

caggcatcta ttaccacaga tgatggggtt gttttccagt tcaagagttc aaagaaatca 2820

aataatggta cagatgttaa tgcgggtcag atacctcagt tattatacag acttccaacc 2880

agtgatggca gtgcttcaaa aggcaaacag caaaccagtg aacctgtaca cattttaaag 2940

aggtcttttg caagaactgt ctcagtggaa tgttttgagt cattgttgag tattcttcac 3000

tggagctgga ccaccttagt cttaggagtt 3030

<210> 25
<211> 1851
<212> DNA
<213> Homo sapiens

<400> 25
atatttgcca caagcactga acctgttcta cagcaagaat tgcaacttaa actggctaga 60

aaatgcttac atgcctgtcg tatctcatta ttcgatctgg aaaaggactt gcatattata 120

agtacaggat ttgatgagga gtcagcaatt cttggtgcag gacgagagtt tgcgctaatg 180

aaaacagcaa atggaaagat atattacact ggcaaatacc agagtcttgg aatcaaacaa 240

ggtggtcctt cagcaggaaa atgggttgag ctaccaatta caaaatctcc aaagatagta 300

cacttctcag ttggacacga tggctctcac gcccttttag ttgcagaaga tgggagcata 360

ttctttacag gatctgctag taaaggagaa gatggagaat caattaagag cagacggcaa 420

tccaaacctt ataaacctaa aaagataatt aagatggaag gaaagattgt ggtatataca 480

gcctgcaata atggaagtag ttctgttatt tctaaagatg gagaactcta catgtttgga 540

aaagatgcca tttactctga tagttcaagt ttggtaactg atttgaaggg ccattttgta 600

actcaggtag ctatgggcaa agctcacact tgtgttttaa tgaagaatgg agaggtgtgg 660

acatttggtg taaataataa aggacagtgt ggacgagata ctggtgccat gaaccaaggt 720

gggaaagggt ttggagttga aaatatggca acagcaatgg atgaagacct ggaagaagaa 780
ctagatgaaa aagatgagaa gtctatgatg tgccctccag gcatgcacaa atggaagctg 840
gagcagtgca tggtttgcac tgtctgtgga gactgtacag gttatggagc cagctgtgtc 900
agtagtggac ggccagacag agtccccgga gggatctgtg gttgtggttc cggagaatct 960
ggttgtgctg tgtgtggatg ttgcaaggcc tgtgcaagag agttagatgg tcaagaggca 1020
agacaaagag gaattcttga tgcagtgaaa gaaatgatac ctttagatct tcttttagct 1080
gtcccagtgc ccggggttaa cattgaagaa caccttcagt tacgacaaga agaaaaacgg 1140
caacgtgtaa tcagaaggca cagattagag gaaggaagag gcccccttgt atttgctggt 1200
cctattttta tgaaccatcg agaacaggct ctagccagac tcagatccca tccagcacac 1260
gtaaagcata aacgggacaa gcacaaagat ggaagtggag aaagaggcga aaaggatgca 1320
agcaaaatca caacataccc tccaggctct gtgcgatttg actgtgagct ccgggcagtc 1380
caagtcagct gtggatttca ccattcagtg gttttaatgg aaaatggaga tgtctataca 1440
tttggttatg ggcagcatgg gcagctagga catggagatg tcaactccag gggatgtccc 1500
actcttgttc aagcattgcc aggccctagc acacaagtca ctgcaggcag caaccatacg 1560
gcagtacttt taatggatgg acaggtcttc acatttggaa gtttttctaa aggacaactg 1620
ggcagaccaa ttttggatgt gccatattgg aatgcaaagc cagctcccat gcctaacatt 1680
ggatcaaaat atggaagaaa agctacttgg ataggtgcaa gtggggacca aactttttta 1740
cgaattgatg aagcacttat taattctcat gtacttgcta catcagaaat ttttgccagt 1800
aaacacataa taggcttggt acctgcttct atatcagaac ctcctccatt t 1851

<210> 26
<211> 1701
<212> DNA
<213> Homo sapiens

<400> 26
ggacgagagt ttgcgctaat gaaaacagca aatggaaaga tatattacac tggcaaatac 60
cagagtcttg gaatcaaaca aggtggtcct tcagcaggaa aatgggttga gctaccaatt 120
acaaaatctc caaagatagt acacttctca gttggacacg atggctctca cgccctttta 180
gttgcagaag atgggagcat attctttaca ggatctgcta gtaaaggaga agatggagaa 240
tcaattaaga gcagacggca atccaaacct tataaaccta aaaagataat taagatggaa 300
ggaaagattg tggtatatac agcctgcaat aatggaagta gttctgttat ttctaaagat 360
ggagaactct acatgtttgg aaaagatgcc atttactctg atagttcaag tttggtaact 420
gatttgaagg gccattttgt aactcaggta gctatgggca aagctcacac ttgtgtttta 480
atgaagaatg gagaggtgtg gacatttggt gtaaataata aaggacagtg tggacgagat 540
actggtgcca tgaaccaagg tgggaaaggg tttggagttg aaaatatggc aacagcaatg 600
gatgaagacc tggaagaaga actagatgaa aaagatgaga agtctatgat gtgccctcca 660

ggcatgcaca aatggaagct ggagcagtgc atggtttgca ctgtctgtgg agactgtaca 720
ggttatggag ccagctgtgt cagtagtgga cggccagaca gagtccccgg agggatctgt 780
ggttgtggtt ccggagaatc tggttgtgct gtgtgtggat gttgcaaggc ctgtgcaaga 840
gagttagatg gtcaagaggc aagacaaaga ggaattcttg atgcagtgaa agaaatgata 900
cctttagatc ttcttttagc tgtcccagtg cccggggtta acattgaaga acaccttcag 960
ttacgacaag aagaaaaacg gcaacgtgta atcagaaggc acagattaga ggaaggaaga 1020
ggcccccttg tatttgctgg tcctattttt atgaaccatc gagaacaggc tctagccaga 1080
ctcagatccc atccagcaca cgtaaagcat aaacgggaca agcacaaaga tggaagtgga 1140
gaaagaggcg aaaaggatgc aagcaaaatc acaacatacc ctccaggctc tgtgcgattt 1200
gactgtgagc tccgggcagt ccaagtcagc tgtggatttc accattcagt ggttttaatg 1260
gaaaatggag atgtctatac atttggttat gggcagcatg ggcagctagg acatggagat 1320
gtcaactcca ggggatgtcc cactcttgtt caagcattgc caggccctag cacacaagtc 1380
actgcaggca gcaaccatac ggcagtactt ttaatggatg gacaggtctt cacatttgga 1440
agtttttcta aaggacaact gggcagacca attttggatg tgccatattg gaatgcaaag 1500
ccagctccca tgcctaacat tggatcaaaa tatggaagaa aagctacttg gataggtgca 1560
agtggggacc aaactttttt acgaattgat gaagcactta ttaattctca tgtacttgct 1620
acatcagaaa tttttgccag taaacacata ataggcttgg tacctgcttc tatatcagaa 1680
cctcctccat ttaaatgcct t 1701

<210> 27
<211> 903
<212> DNA
<213> Homo sapiens

<400> 27
gctcccatgc ctaacattgg atcaaaatat ggaagaaaag ctacttggat aggtgcaagt 60
ggggaccaaa cttttttacg aattgatgaa gcacttatta attctcatgt acttgctaca 120
tcagaaattt ttgccagtaa acacataata ggcttggtac ctgcttctat atcagaacct 180
cctccattta aatgccttct gataaataaa gtggatggga gttgtaaaac ttttaatgac 240
tcagaacaag aggatctgca aggatttggt gtgtgtcttg atcctgtata tgatgtaatt 300
tggaggtttc gaccaaatac tagagagctg tggtgttaca atgcggtggt tgctgatgcc 360
aggcttccct ctgcagcaga catgcagtcc agatgtagta tcctaagtcc tgaacttgcc 420
ttaccaacag gatcaagggc cctcactacc cgatctcatg cagctttgca cattttaggt 480
tgtcttgata ccttggcagc tatgcaggac ttaaaaatgg gtgttgcaag tacagaggaa 540
gagactcaag cagtaatgaa ggtttattct aaagaagatt atagtgtggt aaacaggttt 600
gaaagtcatg gaggaggctg gggttattct gcccattcag tagaagctat acgtttcagt 660
gccgacactg atattttact tggtggtctt ggtctgtttg gaggtagagg agaatatact 720
gctaaaatta agctgtttga attgggtcct gatggaggag atcatgaaac tgatggtgac 780

cttcttgcag agactgatgt attggcttat gactgtgctg ctagagaaaa atatgcaatg 840

atgtttgatg agcctgttct cctgcaagct gggtggtggt atgtggcatg ggcccgagtg 900

tca 903

<210> 28
<211> 303
<212> DNA
<213> Homo sapiens

<400> 28
gctcccatgc ctaacattgg atcaaaatat ggaagaaaag ctacttggat aggtgcaagt 60

ggggaccaaa cttttttacg aattgatgaa gcacttatta attctcatgt acttgctaca 120

tcagaaattt ttgccagtaa acacataata ggcttggtac ctgcttctat atcagaacct 180

cctccattta aatgccttct gataaataaa gtggatggga gttgtaaaac ttttaatgac 240

tcagaacaag aggatctgca aggatttggt gtgtgtcttg atcctgtata tgatgtaatt 300

tgg 303

<210> 29
<211> 612
<212> DNA
<213> Homo sapiens

<400> 29
gatgaagcac ttattaattc tcatgtactt gctacatcag aaatttttgc cagtaaacac 60

ataataggct tggtacctgc ttctatatca gaacctcctc catttaaatg ccttctgata 120

aataaagtgg atgggagttg taaaactttt aatgactcag aacaagagga tctgcaagga 180

tttggtgtgt gtcttgatcc tgtatatgat gtaatttgga ggtttcgacc aaatactaga 240

gagctgtggt gttacaatgc ggtggttgct gatgccaggc ttccctctgc agcagacatg 300

cagtccagat gtagtatcct aagtcctgaa cttgccttac caacaggatc aagggccctc 360

actacccgat ctcatgcagc tttgcacatt ttaggttgtc ttgatacctt ggcagctatg 420

caggacttaa aaatgggtgt tgcaagtaca gaggaagaga ctcaagcagt aatgaaggtt 480

tattctaaag aagattatag tgtggtaaac aggtttgaaa gtcatggagg aggctggggt 540

tattctgccc attcagtaga agctatacgt ttcagtgccg acactgatat tttacttggt 600

ggtcttggtc tg 612

<210> 30
<211> 117
<212> DNA
<213> Homo sapiens

<400> 30
gatgaagcac ttattaattc tcatgtactt gctacatcag aaatttttgc cagtaaacac 60

ataataggct tggtacctgc ttctatatca gaacctcctc catttaaatg ccttctg 117